# EUROPÄISCHE PATENTSCHRIFT

(11) **EP 3 429 355 B1**
(45) Veröffentlichungstag und Bekanntmachung des Hinweises auf die Patenterteilung: **05.02.2020**
(21) Anmeldenummer: 17708548.7
(22) Anmeldetag: 08.03.2017
(51) Int. Cl.: A01N 43/50, A01N 43/56, A01N 43/653, A01P 7/04, C07D 233/90, C07D 249/10, C07D 401/04, C07D 401/12, C07D 403/12, C07D 403/04, C07D 405/04, C07D 409/12, C07D 409/14, C07D 413/12, C07D 417/12, C07D 405/12, C07D 409/04

(54) **SUBSTITUIERTE SULFONYLAMIDE ZUR BEKÄMPFUNG TIERISCHER SCHÄDLINGE**
SUBSTITUTED SULFONYLAMIDE FOR COMBATING ANIMAL PESTS
SULFONYLAMIDES SUBSTITUES POUR LA LUTTE CONTRE LES RAVAGEURS

(30) Priorität: 15.03.2016 EP 16160293
(43) Veröffentlichungstag der Anmeldung: 23.01.2019
(73) Patentinhaber: Bayer CropScience AG, 40789 Monheim am Rhein (DE)
(72) Erfinder: FÜSSLEIN, Martin, 40225 Düsseldorf (DE); WROBLOWSKY, Heinz-Jürgen, 40764 Langenfeld (DE); KÜBBELER, Susanne, 40589 Düsseldorf (DE); HAGER, Dominik, 40789 Monheim (DE); KAUSCH-BUSIES, Nina, 51467 Bergisch Gladbach (DE); MÜLLER, Klaus-Helmut, 40593 Düsseldorf (DE); PORTZ, Daniela, 52391 Vettweiß (DE); ILG, Kerstin, 50670 Köln (DE); MALSAM, Olga, 51503 Rösrath (DE); EILMUS, Sascha, 42799 Leichlingen (DE); LÖSEL, Peter, 51371 Leverkusen (DE); GÖRGENS, Ulrich, 40882 Ratingen (DE); HERRMANN, Stefan, 40764 Langenfeld (DE); BECKER, Angela, 06650 Opio (FR)
(74) Vertreter: BIP Patents
(86) Internationale Anmeldenummer: PCT/EP2017/055405
(87) Internationale Veröffentlichungsnummer: WO 2017/157735

(56) Entgegenhaltungen:
- EP-A1- 2 092 824
- EP-A1- 2 540 163

## Beschreibung

Die vorliegende Anmeldung betrifft die Verwendung substituierter Sulfonylamide zur Bekämpfung von tierischen Schädlingen, ein Mittel enthaltend substituierte Sulfonylamide zur Bekämpfung von tierischen Schädlingen, ein Verfahren zur Bekämpfung von tierischen Schädlingen, eine agrochemische Formulierung enthaltend die substituierten Sulfonylamide, neue substituierte Sulfonylamide sowie ein Verfahren und Zwischenprodukte zur Herstellung der substituierten Sulfonylamide.

In der Literatur sind Sulfonylamide und deren Eignung als Wirkstoffe beispielsweise in den Patentanmeldungen WO 2005/099705, WO 2003/040107, WO 2014/077285 und WO 2014/023367 beschrieben.

Darüber hinaus ist z.B. aus den Dokumenten WO 2010/129500, WO 2012/054233, WO 2013/055584, WO 2014/109933, WO 2015/007668, WO 2015/011082 und WO 2015/169776 bekannt, das bestimmte Sulfonylamide als Nematizide verwendet werden können.

Aus der EP 2092824 sind zusätzlich auch Sulfonylamide bekannt, die als Insektizide verwendet werden können.

Moderne Insektizide müssen vielen Anforderungen genügen, beispielsweise in Bezug auf Höhe, Dauer und Breite ihrer Wirkung und möglichen Verwendung. Es spielen Fragen der Toxizität, der Nützling- und Bestäuberschonung, der Umwelteigenschaften, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Paramenter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen zur Verwendung zur Bekämpfung von tierischen Schädlingen bereitzustellen, durch die das Spektrum der Schädlingsbekämpfungsmittel unter verschiedenen Aspekten ergänzt wird.

Gelöst wird die Aufgabe, sowie weitere nicht explizit genannte Aufgaben, die aus den hierin diskutierten Zusammenhängen ableitbar oder erschließbar sind, durch die Verwendung einer Verbindung der Formel (I) 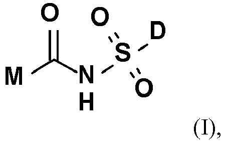 in der (Ausgestaltung 0-1)
M für einen Rest ausgewählt aus den Formeln (IIa-IIf) steht: 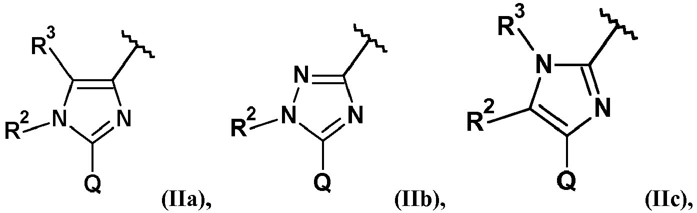 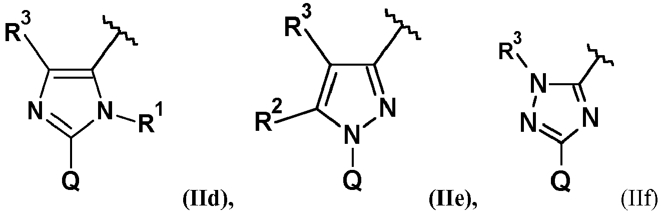 wobei
R¹, R², R³ jeweils unabhängig voneinander H oder ein substituierter oder unsubstituierter Alkyl, Cycloalkyl, Alkenyl, Cycloalkenyl, Cyloheteroalkyl, Aryl oder Heteroaryl Rest sind, wobei R² im Falle (IIc) und (IIe) zusätzlich ein Halogen Rest oder ein Alkoxy Rest sein kann, und R³ zusätzlich im Falle (IIa), (IId) und (IIe) ein Halogen Rest sein kann;
Q ein substituierter oder unsubstituierter Aryl oder Heteroaryl Rest, im Falle (IIe) aber nicht 2-Pyrimidinyl ist;
D ein substituierter oder unsubstituierter Alkyl, Heteroalkyl, gegebenenfalls teilweise ungesättigter Cycloalkyl, Cycloheteroalkyl, Heteroaryl, Aryl oder Phenylalkyl Rest oder ein substituierter oder unsubstituierter Stickstoff Rest ist,
zur Bekämpfung von tierischen Schädlingen.

### Bevorzugt ist eine Ausführungsform der Formel (I), bei der (Vorzugsbereich 1-1)

M für einen Rest ausgewählt aus den Formeln (IIa-IIf) steht, wobei R¹, R², R³ jeweils unabhängig voneinander H oder ein substituierter oder unsubstituierter (C₁-C₄)-Alkyl, (C₃-C₆)-Cycloalkyl, (C₂-C₄)-Alkenyl, Pyridyl oder Phenyl Rest sind, wobei R² im Falle (IIc) und (IIe) zusätzlich ein Halogen Rest oder ein Alkoxy Rest sein kann und wobei R³ zusätzlich im Falle (IIa), (IId) und (IIe) ein Halogen Rest sein kann;
Q ein substituierter oder unsubstituierter Phenyl, Naphthyl oder Heteroaryl Rest ist, der ein bis drei Heteroatome aus der Reihe Sauerstoff, Schwefel, Stickstoff enthalten kann; im Falle (IIe) aber nicht 2-Pyrimidinyl ist;
D ein substituierter oder unsubstituierter Alkyl, Heteroalkyl, Cycloalkyl, Heteroaryl, Aryl oder Phenyl-(C₁-C₈)alkyl Rest oder ein substituierter oder unsubstituierter Stickstoff Rest ist.

### Weiter bevorzugt ist eine Ausführungsform der Formel (I), bei der (Vorzugsbereich 2-1)

M für einen Rest ausgewählt aus den Formeln (IIa-IIf) steht, wobei R¹, R², R³ definiert sind wie in Ausgestaltung (0-1) oder Vorzugsbereich (1-1) und
Q ein unsubstituierter oder mit einem oder mehreren Resten R⁴ substituierter Phenyl, Naphthyl, Pyridyl, Pyrimidinyl, Thiophen, Benzthiophen, Isochinolin, Benzdioxol oder Pyrazol Rest ist, im Falle (IIe) aber nicht 2-Pyrimidinyl ist,
wobei der oder die Substituenten R⁴ jeweils unabhängig voneinander ausgewählt sind aus:
Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino) und/oder
gegebenenfalls einfach oder mehrfach, gleich oder verschieden substituiertes Aryl, Aryloxy oder Hetaryl, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino und
D ein unsubstituierter oder mit einem oder mehreren Resten R⁵ substituierter (C₁-C₆)-Alkyl, Phenyl, Phenyl-(C₁-C₂)alkyl oder Heteroaryl Rest ist, der ein bis drei Heteroatome aus der Reihe Sauerstoff, Schwefel, Stickstoff enthalten kann oder ein NR⁶R⁷ Rest ist,
wobei der oder die Substituenten R⁵ jeweils unabhängig voneinander ausgewählt sind aus:
Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)alkyl
und /oder
einfach oder mehrfach, gleich oder verschieden substituiertes Aryl oder Hetaryl, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino
und wobei R⁶ und R⁷ jeweils unabhängig voneinander H, (C₁-C₆)-Alkyl oder ein substituierter oder unsubstituierter Phenyl Rest sind oder R⁶ und R⁷ gemeinsam einen unsubstituierten oder substituierten 4- bis 8- gliedrigen, gesättigten oder gegebenenfalls ganz oder teilweise ungesättigten Ring bilden können der von 1 bis 3 Heteroatomen aus der Reihe Sauerstoff, Schwefel, Stickstoff unterbrochen sein kann und der einfach oder mehrfach mit einer der Definition von R⁵ entsprechenden Substitution versehen sein kann.

### Noch weiter bevorzugt ist, wenn (Vorzugsbereich 3-1)

M für einen Rest ausgewählt aus den Formeln (IIa-IIf) steht, wobei R¹, R², R³ definiert sind wie in Ausgestaltung (0-1) oder Vorzugsbereich (1-1) und
Q ein unsubstituierter oder mit einem oder mehreren Resten R⁴ substituierter Phenyl, Naphthyl, Pyridyl, Pyrimidinyl, Thiophen, Benzthiophen, Isochinolin, Benzdioxol oder Pyrazol Rest ist, im Falle (IIe) aber nicht 2-Pyrimidinyl ist,
wobei der oder die Substituenten R⁴ jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(Ci-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, Phenyl, Halogenphenyl, Phenoxy oder Halogenphenoxy und
D ein unsubstituierter oder mit einem oder mehreren Resten R⁵ substituierter (C₁-C₆)-Alkyl, Phenyl, Phenyl-(C₁-C₂)alkyl oder Heteroaryl Rest ist, der ein bis drei Heteroatome aus der Reihe Sauerstoff, Schwefel oder Stickstoff enthalten kann oder ein NR⁶R⁷ Rest ist,
wobei der oder die Substituenten R⁵ jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)Alkyl und
R⁶ und R⁷ jeweils unabhängig voneinander H, ein (C₁-C₆)-Alkyl, (C₁-C₆)Halogenalkyl, (C₁-C₆)Alkoxy-(C₁-C₆)alkyl Rest oder ein unsubstituierter Phenyl oder mit Halogen, (C₁-C₆)Alkyl, (C₁-C₆)-Alkoxy, (C₁-C₆)Halogenalkyl, (C₁-C₆)Alkoxy-(C₁-C₆)alkyl substituierter Phenyl Rest sind,
oder
R⁶ und R⁷ gemeinsam einen unsubstituierten oder substituierten 5- bis 6- gliedrigen , gesättigten oder gegebenenfalls ganz oder teilweise ungesättigten Ring, bilden können der von 1 bis 3 Heteroatomen aus der Reihe Sauerstoff, Schwefel oder Stickstoff unterbrochen sein kann, und der einfach oder mehrfach mit einer der Definition von R⁵ entsprechenden Substitution versehen sein kann.

### Insbesonders bevorzugt ist eine Ausführungsform der Formel (I), bei der (Vorzugsbereich 4-1)

M für einen Rest ausgewählt aus den Formeln (IIa-IIf) steht, wobei
im Falle (IIa) R², R³ jeweils unabhängig voneinander H oder ein substituierter oder unsubstituierter (C₁-C₄)-Alkyl, (C₃-C₆)-Cycloalkyl oder Phenyl Rest sind, wobei R³ zusätzlich ein Halogen Rest sein kann,
im Falle (IIb) R² H oder ein substituierter oder unsubstituierter (C₁-C₄)-Alkyl oder (C₃-C₆)-Cycloalkyl Rest ist,
im Falle (IIc) R², R³ jeweils unabhängig voneinander H oder ein substituierter oder unsubstituierter (C₁-C₄)-Alkyl, (C₃-C₆)-Cycloalkyl oder Phenyl Rest sind und R² zusätzlich ein Halogen oder (C₁-C₄)-Alkoxy Rest sein kann,
im Falle (IId) R¹, R³ jeweils unabhängig voneinander H oder ein substituierter oder unsubstituierter (C₁-C₄)-Alkyl oder (C₃-C₆)-Cycloalkyl Rest sind, wobei R³ zusätzlich ein Halogen Rest sein kann,
im Falle (IIe) R², R³ jeweils unabhängig voneinander H, Halogen oder ein substituierter oder unsubstituierter (C₁-C₄)-Alkyl, (C₃-C₆)-Cycloalkyl, Pyridyl oder Phenyl Rest sind und R² zusätzlich ein (C₁-C₄)-Alkoxy Rest sein kann und
im Falle (IIf) R³ H oder ein substituierter oder unsubstituierter (C₁-C₄)-Alkyl, oder (C₃-C₆)-Cycloalkyl Rest ist
und Q definiert ist wie in Ausgestaltung (0-1), Vorzugsbereich (1-1), Vorzugsbereich (2-1) oder Vorzugsbereich (3-1) und
D ein unsubstituierter oder mit einem oder mehreren Resten R⁵ substituierter (C₁-C₆)-Alkyl, Phenyl, Naptht-2-yl, Pyridin, Pyrimidin, Pyrazol, Triazol, Thiazol, Oxazol, Thiadiazol, Oxadiazol, Thiophen, Pyrrol, Furan, Tetrahydrofuran, Dioxan, Isoxazol, Benzyl, 2,3-Dihydro-1,4-benzodioxin-5-yl, 2,3-Dihydro-1-benzofuran-7-yl, Chinoxalin-5-yl oder Indol-7-yl Rest oder ein NR⁶R⁷ Rest ist,
wobei der oder die Substituenten R⁵ jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, 1-Pyrazolyl-(C₁-C₃)Alkyl und
R⁶ und R⁷ jeweils unabhängig voneinander H, ein (C₁-C₆)-Alkyl, (C₁-C₆)Halogenalkyl, (C₁-C₆)Alkoxy-(C₁-C₆)alkyl Rest oder ein unsubstituierter Phenyl oder mit Halogen, (C₁-C₆)-Alkyl, (C₁-C₆)-Alkoxy, (C₁-C₆)Halogenalkyl oder (C₁-C₆)Alkoxy-(C₁-C₆)alkyl substituierter Phenyl Rest sind,
oder einen Ring aus der Reihe Pyrrolidin, Morpholin, Piperidin bilden.

### Noch weiter bevorzugt ist eine Ausführungsform der Formel (I), bei der (Vorzugsbereich 5-1)

M ein Rest ausgewählt aus den Resten der Formeln (IIa) bis (IIf) ist, wobei
im Falle (IIa) R² H, Methyl oder Ethyl oder gegebenenfalls mit Halogen substituiertes Phenyl ist, und R³ H, Methyl, Ethyl, iso-Propyl oder Halogen ist,
im Falle (IIb) R² H, Methyl oder Ethyl ist,
im Falle (IIc) R² H oder Methyl und R³ H, Methyl oder Ethyl ist,
im Falle (IId) R¹ H oder Methyl und R³ H oder Halogen ist,
im Falle (IIe) R² H, Methyl, Methoxy, mit Halogen substituiertes Phenyl oder mit Halogen substituiertes Pyridyl und R³ H ist,
im Falle (IIf) R³ H, Methyl oder Ethyl ist
und Q definiert ist wie in Ausgestaltung (0-1), Vorzugsbereich (1-1), Vorzugsbereich (2-1) oder Vorzugsbereich (3-1).

### Ganz besonders bevorzugt ist eine Ausführungsform der Formel (I), bei der (Vorzugsbereich 6-1)

M ausgewählt ist aus einer der Formeln (IIa) bis (IIf) oder (IVa) bis (IVf), wobei R¹ bis R³ definiert sind wie in Ausgestaltung (0-1), Vorzugsbereich (1-1), Vorzugsbereich (4-1) oder Vorzugsbereich (5-1) und
Q ein unsubstituierter oder mit einem oder mehreren Resten R⁴ substituierter Phenyl, Napht-1-yl, Pyridyl, Pyrimidinyl, Thiophen-2-yl, Benzthiophen-2-yl, Benzthiophen-3-yl, Isochinolin-1-yl, Benzdioxol-4-yl oder Pyrazol-5-yl Rest ist, im Falle (IVe) aber nicht 2-Pyrimidinyl ist,
wobei der oder die Substituenten R⁴ unabhängig voneinander sind:
Cyano, Halogen, Nitro, Acetyl, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino, Phenyl, Halogenphenyl, Phenoxy oder Halogenphenoxy und
D ein unsubstituierter oder mit einem oder mehreren Resten R⁵ substituierter (C₁-C₆)-Alkyl, Phenyl, Naptht-2-yl, Pyridin, Pyrimidin, Pyrazol, Triazol, Thiazol, Oxazol, Thiadiazol, Oxadiazol, Thiophen, Pyrrol, Furan, Tetrahydrofuran, Dioxan, Isoxazol, Benzyl, 2,3-Dihydro-1,4-benzodioxin-5-yl, 2,3-Dihydro-1-benzofuran-7-yl, Chinoxalin-5-yl oder Indol-7-yl Rest oder ein NR⁶R⁷ Rest ist,
wobei der oder die Substituenten R⁵ unabhängig voneinander sind:
   Cyano, Halogen, Nitro, Acetyl, Hydroxy, Carboxy, Amino, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino, 1-Pyrazolyl-(C₁-C₃)Alkyl,
R⁶ und R⁷ jeweils unabhängig voneinander H, ein (C₁-C₆)-Alkyl, Phenyl,Alkoxyphenyl oder Halogenphenyl sind oder einen Ring aus der Reihe
Pyrrolidin, Morpholin, Piperidin bilden.

### Noch weiter bevorzugt ist eine Ausgestaltung der Formel (I), wobei (Vorzugsbereich 7-1)

M ein Rest ausgewählt aus den Resten der Formeln (Va-Vz) ist: 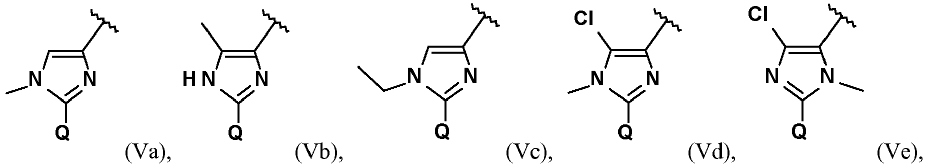 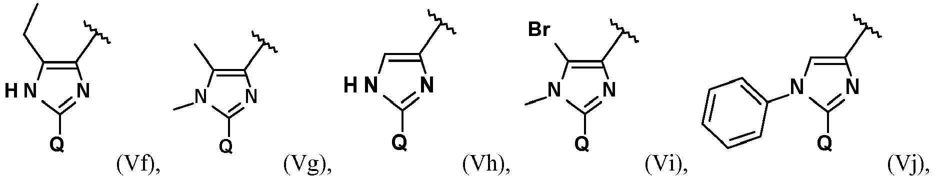 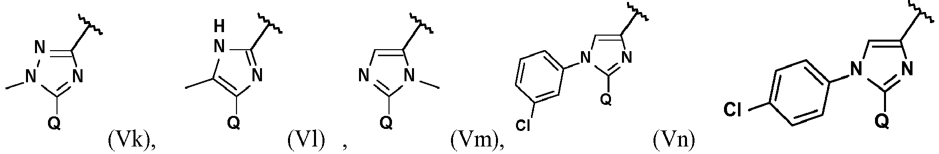 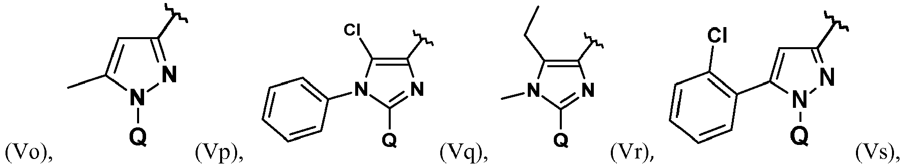 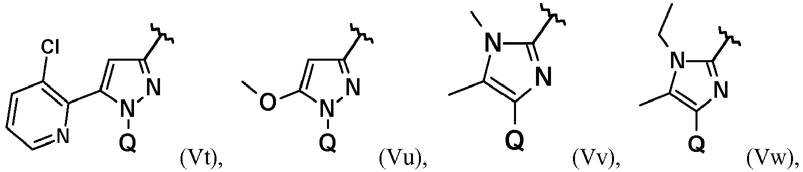 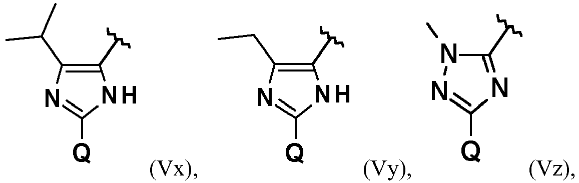 Q ein Rest ausgewählt aus den Resten der Formeln (VIa-VIz und VIa1 - VIa30) ist: 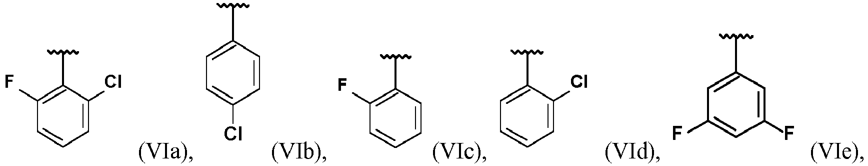 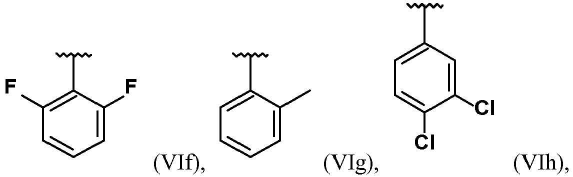 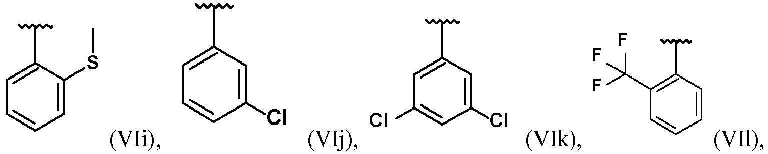 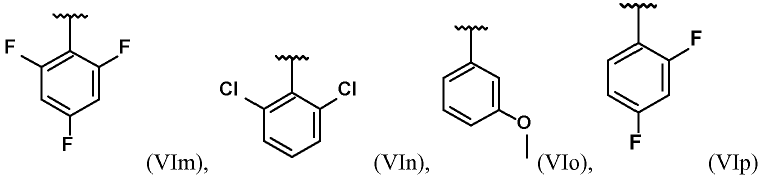 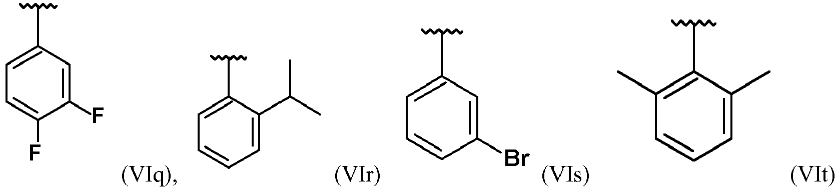 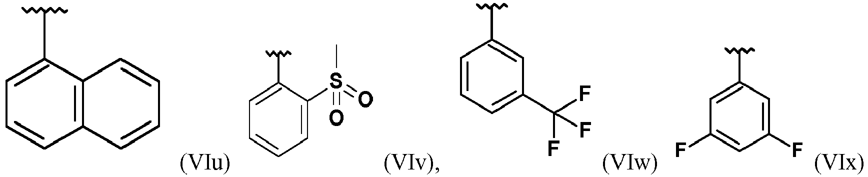 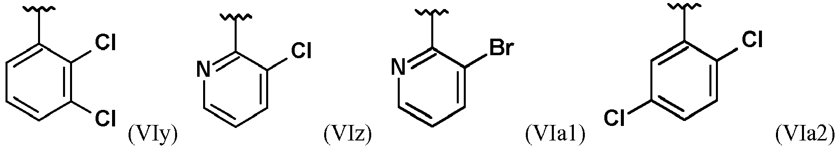 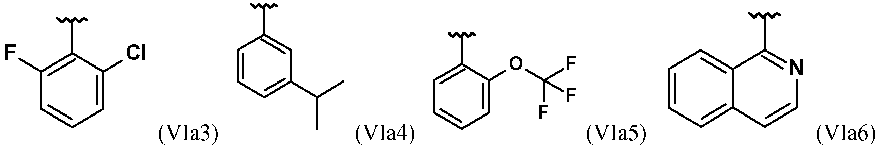 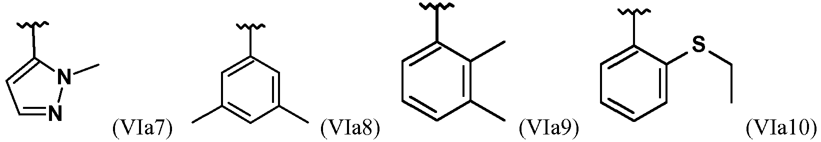 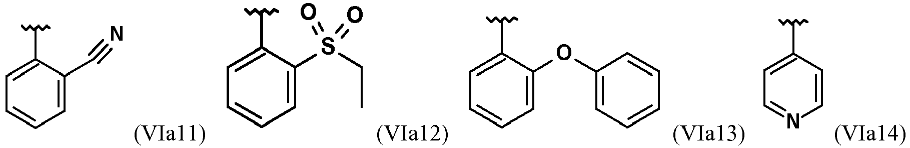 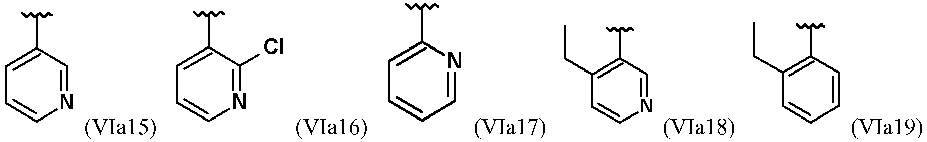 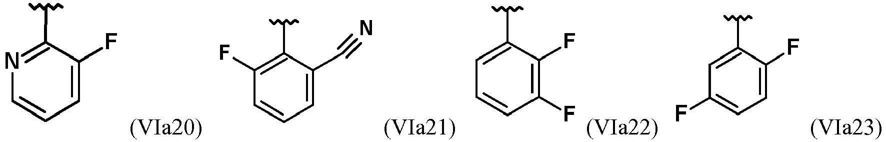 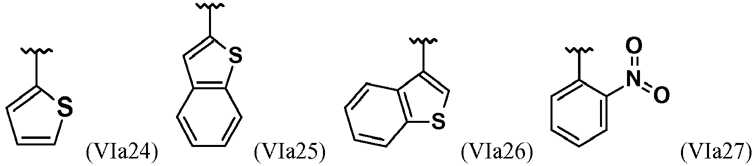 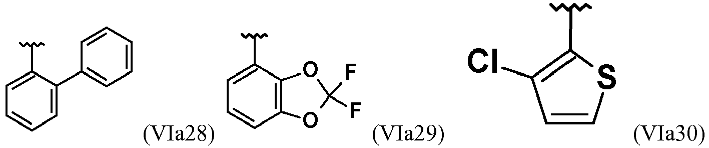 und D ein Rest ausgewählt aus den Resten der Formeln (VIII-VII192) ist

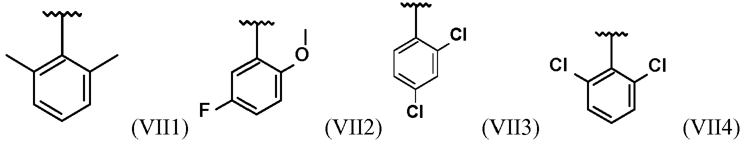

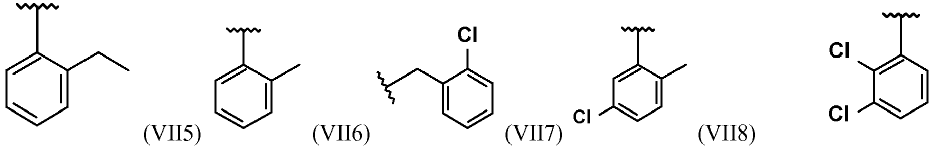

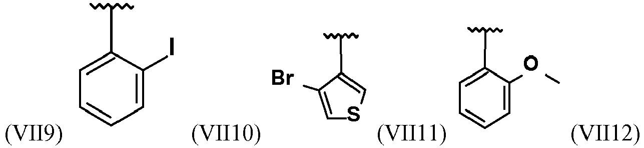

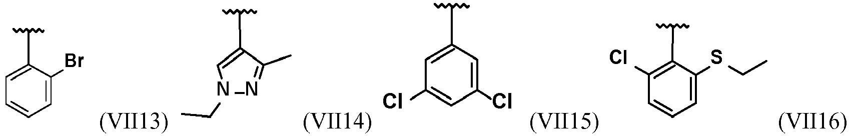

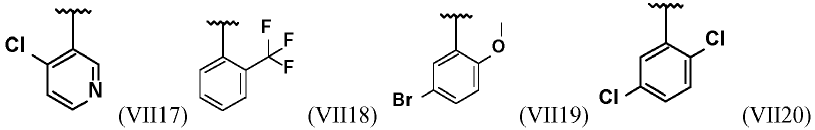

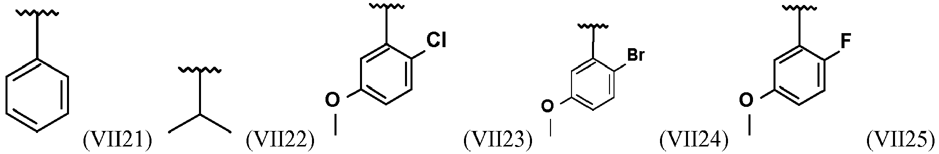

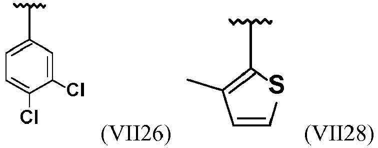

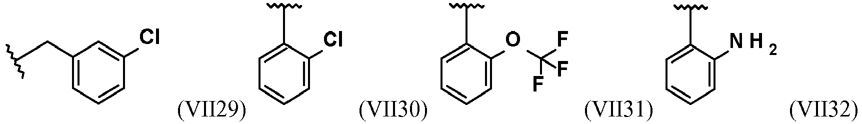

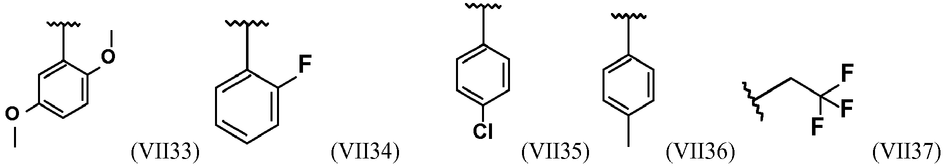

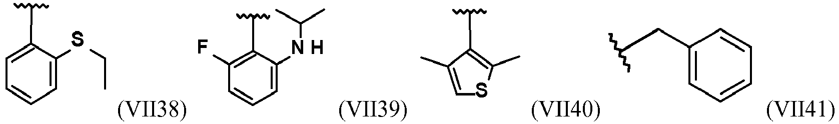

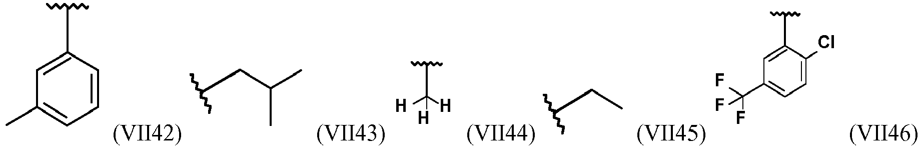

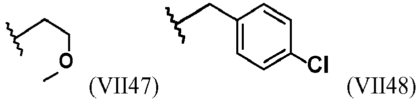

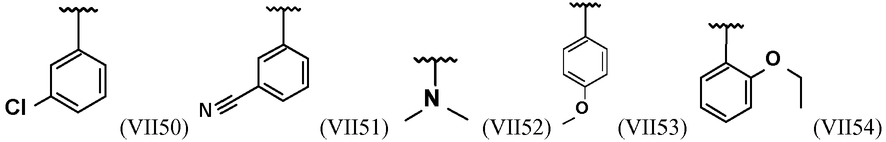

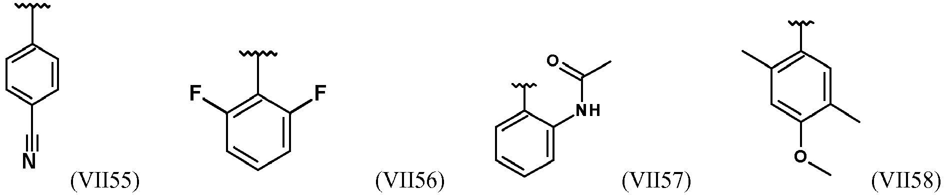

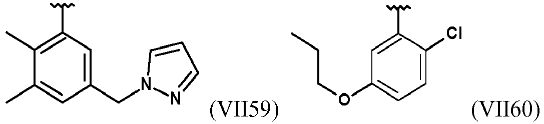

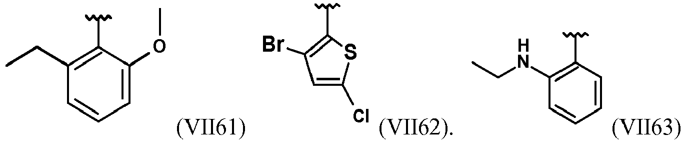

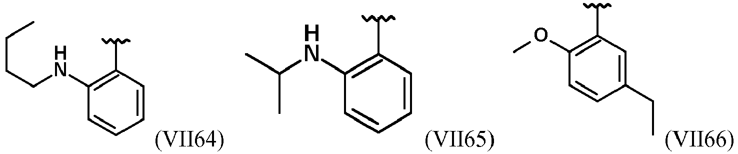

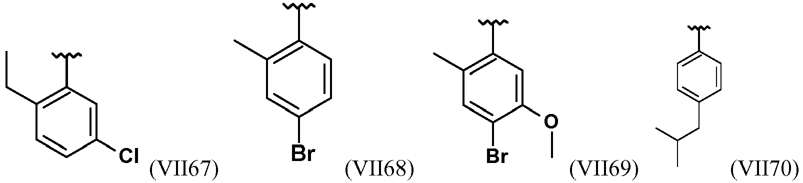

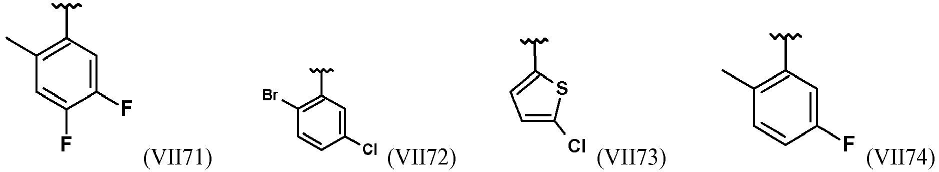

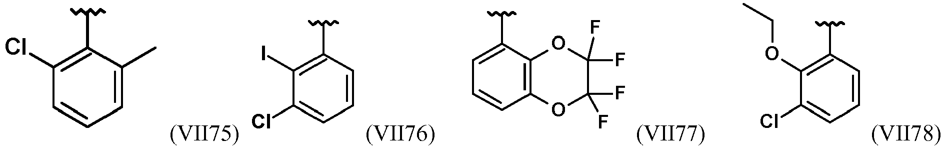

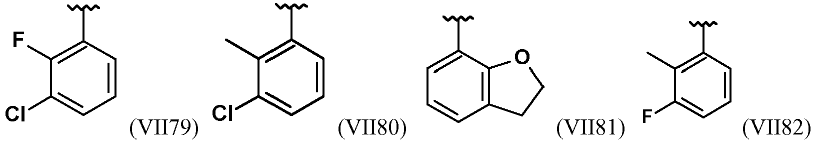

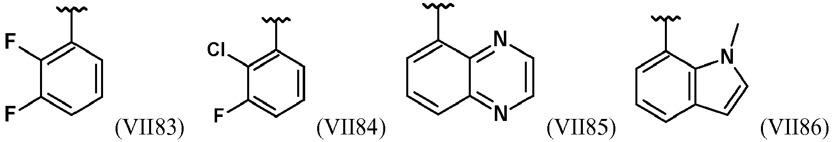

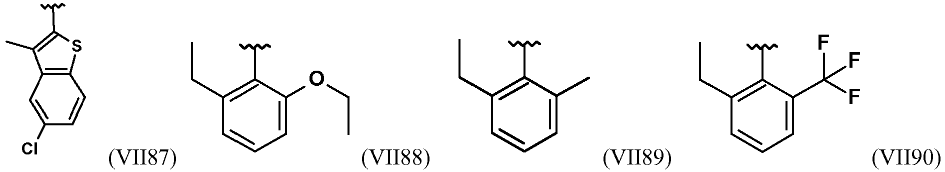

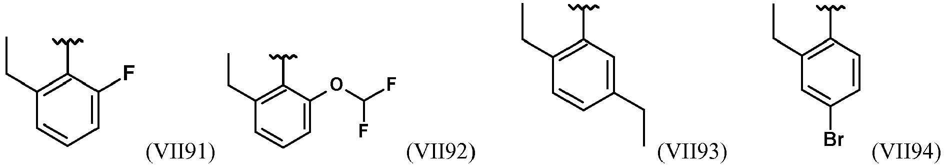

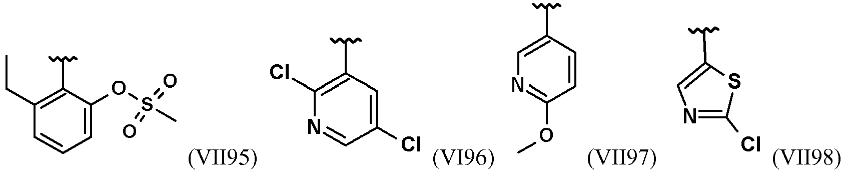

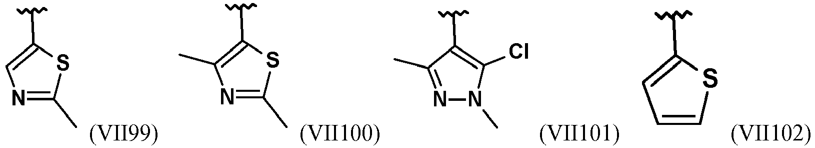

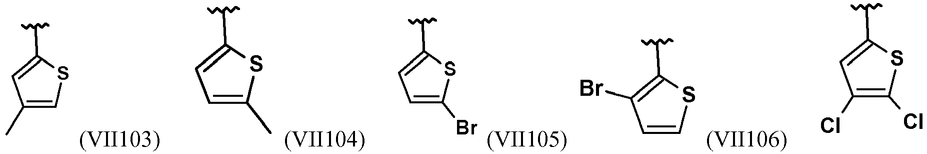

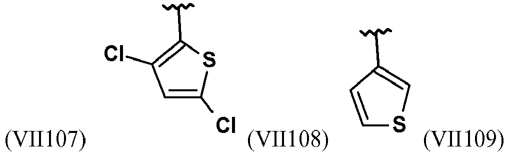

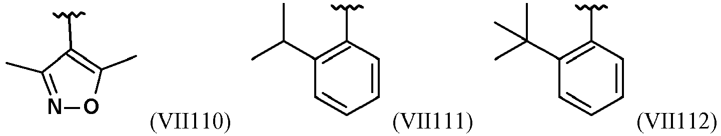

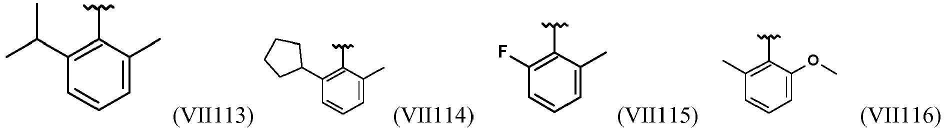

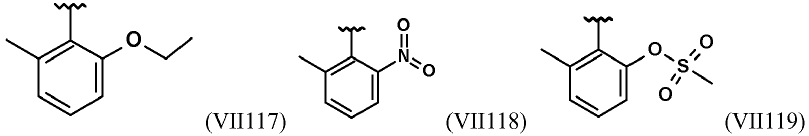

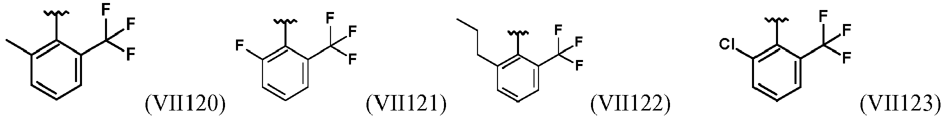

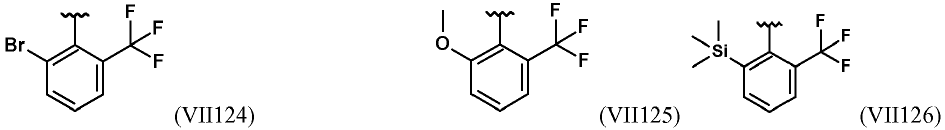

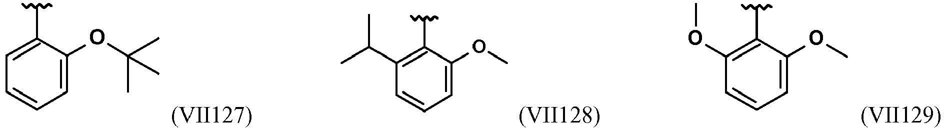

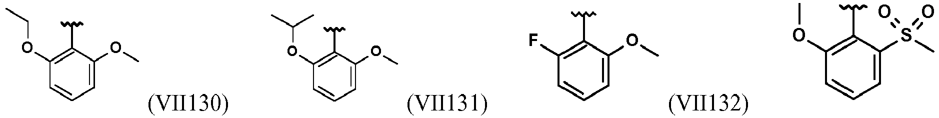

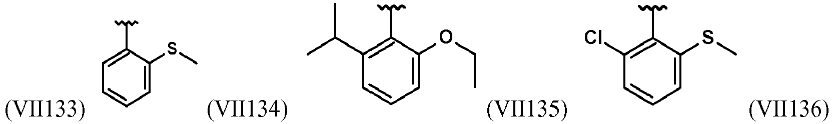

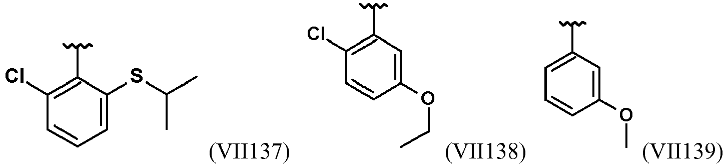

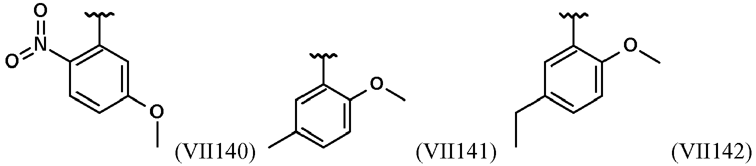

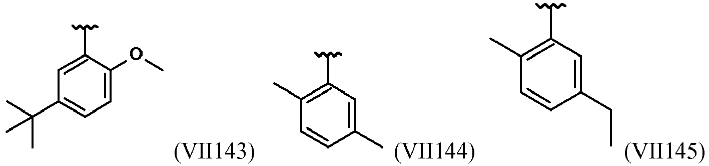

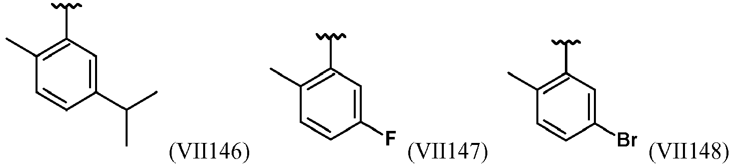

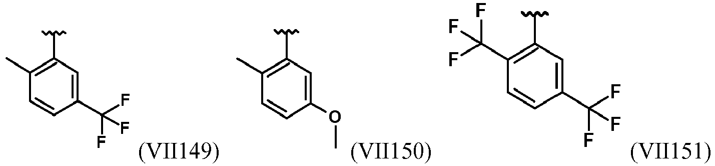

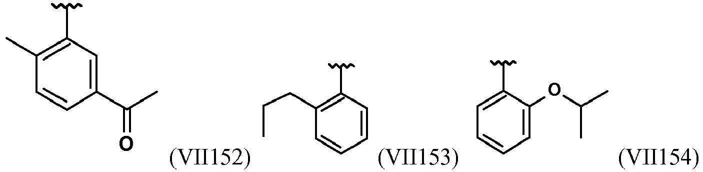

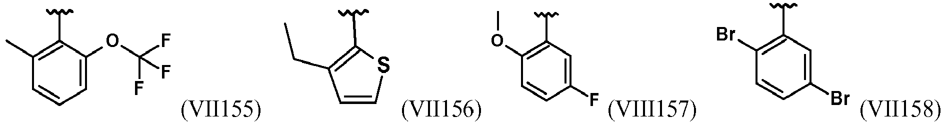

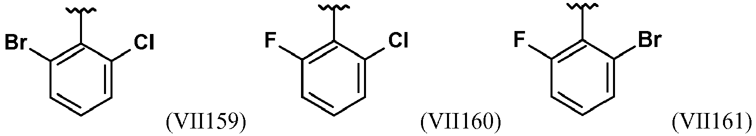

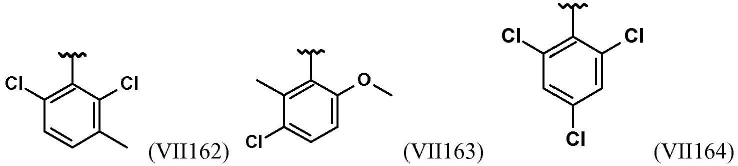

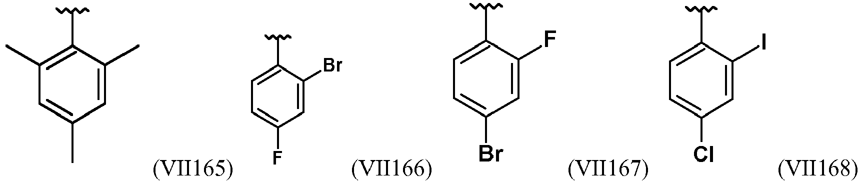

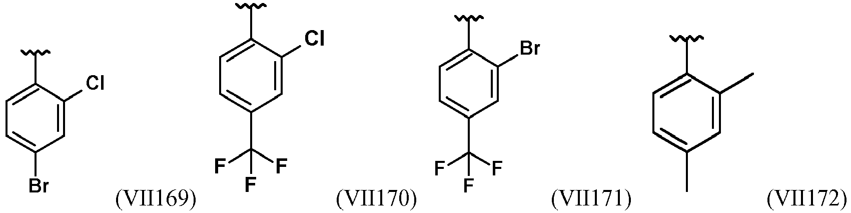

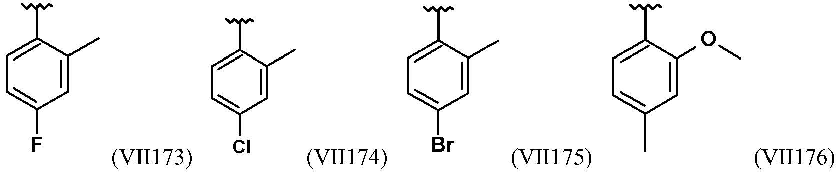

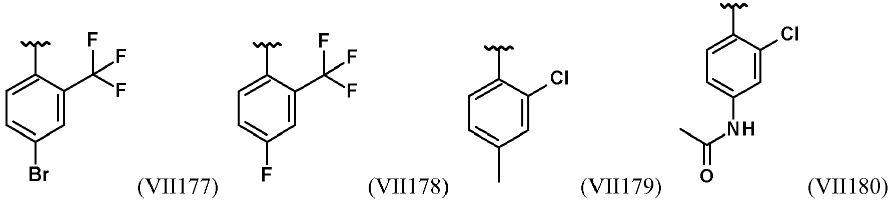

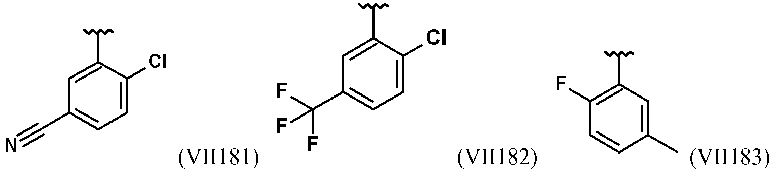

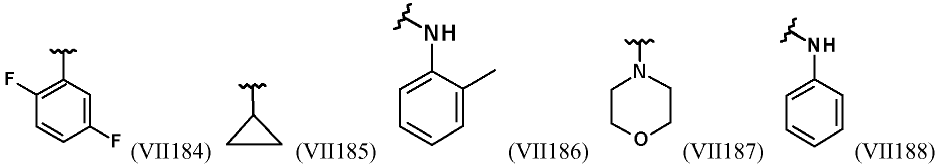

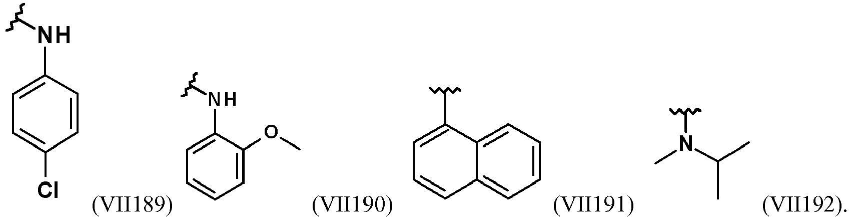

Gemäß einer weiteren bevorzugten Ausführungsform der Erfindung ist die Verwendung einer Verbindung gemäß Formel (I) zum Schutz des Vermehrungsmaterials von Pflanzen vorgesehen.

Gegenstand der Erfindung ist auch ein Mittel, mit einem Gehalt von mindestens einer Verbindung gemäß Formel (I) und üblichen Streckmitteln und/oder oberflächenaktiven Substanzen insbesondere zur Bekämpfung von tierischen Schädlingen.

Ein weiterer Gegenstand der Erfindung ist ein Verfahren zur Bekämpfung von tierischen Schädlingen, bei dem man wenigstens eine Verbindung gemäß Formel (I) oder ein erfindungsgemäßes Mittel auf die tierischen Schädlinge und/oder ihren Lebensraum einwirken lässt, wobei die chirurgische, therapeutische und diagnostische Behandlung des menschlichen oder tierischen Körpers ausgeschlossen ist.

Noch weiterer Gegenstand der Erfindung ist eine agrochemische Formulierung enthaltend wenigstens eine erfindungsgemäße Verbindung gemäß Formel (I) in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

Eine bevorzugte Ausgestaltung der erfindungsgemäßen Formulierung enthält zusätzlich einen weiteren agrochemischen Wirkstoff.

Ebenfalls Gegenstand der Erfindung sind Verbindungen der Formel (VIII) 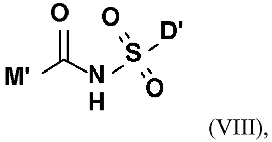 in der
M' für einen Rest der Formel (II) steht, ausgewählt aus: 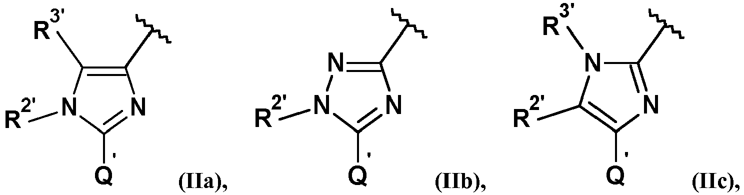 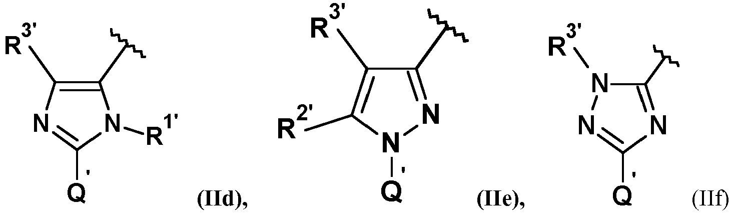 in der (Ausgestaltung 0-2)
R^{1'}, R^{2'}, R^{3'} jeweils unabhängig voneinander H oder ein substituierter oder unsubstituierter Alkyl, Cycloalkyl, Alkenyl, Cycloalkenyl, Cyloheteroalkyl, Aryl oder Heteroaryl Rest sind, wobei R^{2'} im Falle (IIc) und (IIe) zusätzlich ein Halogen Rest oder ein Alkoxy Rest sein kann und R^{2'} im Falle (IIa) und (IIb) nur H oder ein substituierter oder unsubstituierter Alkyl oder Cycloalkyl Rest sein kann, R^{3'} zusätzlich im Falle (IIa), (IId) und (IIe) ein Halogen Rest sein kann,
Q' ein substituierter oder unsubstituierter Aryl oder Heteroaryl Rest, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl, nicht 3,4-Dichlorphenyl und nicht 3,5-bis-tert-Butyl ist;
D' ein substituierter oder unsubstituierter Alkyl, Heteroalkyl, gegebenenfalls teilweise ungesättigter Cycloalkyl, Cycloheteroalkyl, Heteroaryl, Aryl oder Phenylalkyl Rest oder im Falle, daß Q' mindestens einen Substituenten in 2-Stellung trägt, ein substituierter oder unsubstituierter Stickstoff Rest ist.

Gemäß einer ersten bevorzugten Ausführungsform (Vorzugsbereich 1-2) der erfindungsgemäßen Verbindungen ist vorgesehen, dass
M' für einen Rest gemäß einer der Formeln (IIa) bis (IIf) steht und
im Falle (IIa) R^{2'}, R^{3'} jeweils unabhängig voneinander H oder ein substituierter oder unsubstituierter (C₁-C₄)-Alkyl, (C₃-C₆)-Cycloalkyl oder Phenyl Rest sind, wobei R^{3'} zusätzlich ein Halogen Rest sein kann;
im Falle (IIb) R^{2'} H oder ein substituierter oder unsubstituierter (C₁-C₄)-Alkyl oder (C₃-C₆)-Cycloalkyl Rest ist,
im Falle (IIc) R^{2'}, R^{3'} jeweils unabhängig voneinander H oder ein substituierter oder unsubstituierter (C₁-C₄)-Alkyl, (C₃-C₆)-Cycloalkyl oder Phenyl Rest sind, wobei R^{2'} zusätzlich ein Halogen Rest oder ein (C₁-C₄)-Alkoxy Rest sein kann,
im Falle (IId) R^{1'}, R^{3'} jeweils unabhängig voneinander H oder ein substituierter oder unsubstituierter (C₁-C₄)-Alkyl oder (C₃-C₆)-Cycloalkyl Rest sind und R^{3'} zusätzlich ein Halogen Rest sein kann;
im Falle (IIe) R^{2'}, R^{3'} jeweils unabhängig H, Halogen oder ein substituierter oder unsubstituierter (C₁-C₄)-Alkyl, (C₃-C₆)-Cycloalkyl, Pyridyl oder Phenyl Rest sind und R² zusätzlich ein (C₁-C₄)-Alkoxy Rest sein kann und
im Falle (IIf) R³ H oder ein substituierter oder unsubstituierter (C₁-C₄)-Alkyl oder (C₃-C₆)-Cycloalkyl Rest ist,
Q' ein substituierter oder unsubstituierter Phenyl, Naphthyl oder Heteroaryl Rest ist, der ein bis drei Heteroatome aus der Reihe Sauerstoff, Schwefel oder Stickstoff enthalten kann, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist,
D' ein substituierter oder unsubstituierter Alkyl, Heteroalkyl, Cycloalkyl, Heteroaryl, Aryl oder Phenyl-(C₁-₈)alkyl Rest oder im Falle, daß Q' mindestens einen Substituenten in 2-Stellung trägt, ein substituierter oder unsubstituierter Stickstoff Rest ist.

### Weiter bevorzugt gilt für die erfindungsgemäßen Verbindungen der Formel (VIII), dass (Vorzugsbereich 2-2)

M' für einen Rest ausgewählt aus den Formeln (IIa-IIf) steht, wobei R^{1'}, R^{2'}, R^{3'} definiert sind wie in Ausgestaltung (0-2) oder Vorzugsbereich (1-2) und
Q' ein unsubstituierter oder mit einem oder mehreren Resten R^{4'} substituierter Phenyl, Naphthyl, Pyridyl, Pyrimidinyl, Thiophen, Benzthiophen, Isochinolin, Benzdioxol oder Pyrazol-Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl und
wobei der oder die Substituenten R^{4'} jeweils unabhängig voneinander ausgewählt sind aus:
Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und/oder
gegebenenfalls einfach oder mehrfach, gleich oder verschieden substituiertes Aryl, Aryloxy oder Hetaryl, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino und
D' ein unsubstituierter oder mit einem oder mehreren Resten R^{5'} substituierter (C₁-C₆)-Alkyl, Phenyl oder Heteroaryl Rest ist, der ein bis drei Heteroatome aus der Reihe Sauerstoff, Schwefel oder Stickstoff enthalten kann oder oder im Falle, daß Q' mindestens einen Substituenten in 2-Stellung trägt, ein NR^{6'}R^{7'} Rest ist,
wobei der oder die Substituenten R^{5'} jeweils unabhängig voneinander ausgewählt sind aus:
   Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)alkyl
   und /oder
   einfach oder mehrfach, gleich oder verschieden substituiertes Aryl oder Hetaryl, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino,
   und wobei R^{6'} und R^{7'} jeweils unabhängig voneinander H, ein (C₁-C₆)-Alkyl, (C₁-C₆)Halogenalkyl, (C₁-C₆)Alkoxy-(C₁-C₆)alkyl Rest oder ein unsubstituierter Phenyl oder mit Halogen, (C₁-C₆)-Alkyl, (C₁-C₆)-Alkoxy, (C₁-C₆)Halogenalkyl, (C₁-C₆)Alkoxy-(C₁-C₆)alkyl substituierter Phenyl Rest sind oder
   R^{6'} und R^{7'} gemeinsam einen unsubstituierten oder substituierten 5- bis 6- gliedrigen, gesättigten oder gegebenenfalls ganz oder teilweise ungesättigten Ring bilden können der von 1 bis 3 Heteroatomen aus der Reihe Sauerstoff, Schwefel, Stickstoff unterbrochen sein kann, und der einfach oder mehrfach mit einer der Definition von R^{5'} entsprechenden Substitution versehen sein kann.

### Noch weiter bevorzugt ist, wenn (Vorzugsbereich 3-2)

M' für einen Rest ausgewählt aus den Formeln (IIa-IIf) steht, wobei R^{1'}, R^{2'}, R^{3'} definiert sind wie in Ausgestaltung (0-2) oder Vorzugsbereich (1-2) und
Q' ein unsubstituierter oder mit einem oder mehreren Resten R^{4'} substituierter Phenyl, Naphthyl, Pyridyl, Pyrimidinyl, Thiophen, Benzthiophen, Isochinolin, Benzdioxol oder Pyrazol-Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist
und der oder die Substituenten R^{4'} jeweils unabhängig voneinander ausgewählt sind aus:Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, Phenyl, Halogenphenyl, Phenoxy oder Halogenphenoxy und
D' ein unsubstituierter oder mit einem oder mehreren Resten R^{5'} substituierter Phenyl, Pyridin, Pyrimidin, Pyrazol, Triazol, Thiazol, Oxazol, Thiadiazol, Oxadiazol, Thiophen, Pyrrol, Furan, Tetrahydrofuran, Dioxan oder (C₁-C₆)Alkyl Rest oder ein NR^{6'}R^{7'} Rest ist,
wobei der oder die Substituenten R^{5'} jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und (1-Pyrazolyl)-(C₁-C₃)alkyl,
und wobei R^{6'} und R^{7'} jeweils unabhängig voneinander H, ein (C₁-C₄)-Alkyl, (C₁-C₄)Halogenalkyl, (C₁-C₄)Alkoxy-(C₁-C₄)alkyl Rest oder ein unsubstituierter Phenyl oder mit Halogen, (C₁-C₄)-Alkyl, (C₁-C₄)-Alkoxy, (C₁-C₄)Halogenalkyl, (C₁-C₄)Alkoxy-(C₁-C₄)alkyl substituierter Phenyl Rest sind oder
R^{6'} und R^{7'} gemeinsam einen unsubstituierten oder substituierten 5- bis 6- gliedrigen, gesättigten oder gegebenenfalls ganz oder teilweise ungesättigten Ring bilden können der von 1 bis 3 Heteroatomen aus der Reihe Sauerstoff, Schwefel, Stickstoff unterbrochen sein kann, und der einfach oder mehrfach mit einer der Definition von R^{5'} entsprechenden Substitution versehen sein kann.

### Insbesonders bevorzugt ist eine Ausführungsform der Formel (VIII), bei der (Vorzugsbereich 4-2)

D' ein unsubstituierter oder mit einem oder mehreren Resten R^{5'} substituierter (C₁-C₆)-Alkyl Rest, Phenyl Rest, Pyridin, Pyrimidin, Pyrazol, Triazol, Thiazol, Oxazol, Thiadiazol, Oxadiazol, Thiophen, Pyrrol, Furan, Tetrahydrofuran, Dioxan, Isoxazol, Benzyl, 2,3-Dihydro-1,4-benzodioxin-5-yl, 2,3-Dihydro-1-benzofuran-7-yl, Chinoxalin-5-yl oder Indol-7-yl Rest oder ein NR^{6'}R^{7'} Rest ist, wobei der oder die Substituenten R^{5'} jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)Alkyl und
R^{6'} und R^{7'} jeweils unabhängig voneinander H, ein (C₁-C₆)-Alkyl, (C₁-C₆)Halogenalkyl, (C₁-C₆)Alkoxy-(C₁-C₆)alkyl Rest oder unsubstituierter Phenyl oder mit Halogen, (C₁-C₆)-Alkyl, (C₁-C₆)Halogenalkyl oder (C₁-C₆)Alkoxy-(C₁-C₆)alkyl substituierter Phenyl Rest sind
oder einen Ring aus der Reihe Pyrrolidin, Morpholin, Piperidin bilden.

### Ganz besonders bevorzugt ist eine Ausführungsform der Formel (VIII), bei der (Vorzugsbereich 5-2)

M' ein Rest ausgewählt aus den Resten der Formeln (IIa) bis (IIf) ist, wobei
im Falle (IIa) R^{2'} H, Methyl oder Ethyl oder gegebenenfalls mit Halogen substituiertes Phenyl ist und R^{3'} H, Methyl, Ethyl, iso-Propyl oder Halogen ist,
im Falle (IIb) R^{2'} H, Methyl oder Ethyl ist,
im Falle (IIc) R^{2'} H oder Methyl und R^{3'} H, Methyl oder Ethyl ist,
im Falle (IId) R^{1'} H oder Methyl und R^{3'} H oder Halogen ist,
im Falle (IIe) R^{2'} H, Methyl, Methoxy, mit Halogen substituiertes Phenyl oder Pyridyl und R^{3'} H ist,
im Falle (IIf) R^{3'} H, Methyl oder Ethyl ist und
Q' ein unsubstituierter oder mit einem oder mehreren Resten R^{4'} substituierter Phenyl, Napht-1-yl, Pyridyl, Pyrimidinyl, Thiophen-2-yl, Benzthiophen-2-yl, Benzthiophen-3-yl, Isochinolin-1-yl, Benzdioxol-4-yl oder Pyrazol-5-yl Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist und
wobei die Substituenten R^{4'} definiert sind wie in Vorzugsbereich (2-2) oder (3-2).

### Noch weiter bevorzugt ist eine Ausgestaltung der Formel (VIII), wobei (Vorzugsbereich 6-2)

Q' ein unsubstituierter oder mit einem oder mehreren Resten R^{4'} substituierter Phenyl, Napht-1-yl, Pyridyl, Pyrimidinyl, Thiophen-2-yl, Benzthiophen-2-yl, Benzthiophen-3-yl, Isochinolin-1-yl, Benzdioxol-4-yl oder Pyrazol-5-yl Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist und
wobei der oder die Substituenten R^{4'} unabhängig voneinander ausgewählt sind aus:
Cyano, Halogen, Nitro, Acetyl, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino, Phenyl, Halogenphenyl, Phenoxy oder Halogenphenoxy;
D' ein unsubstituierter oder mit einem oder mehreren Resten R^{5'} substituierter (C₁-C₆)-Alkyl, Phenyl, Pyridin, Pyrimidin, Pyrazol, Triazol, Thiazol, Oxazol, Thiadiazol, Oxadiazol, Thiophen, Pyrrol, Furan, Tetrahydrofuran oder Dioxan Rest ist,
wobei der oder die Substituenten R^{5'} unabhängig voneinander ausgewählt sind aus:
   Cyano, Halogen, Nitro, Acetyl, Hydroxy, Carboxy, Amino, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino oder 1-Pyrazolyl-(C₁-C₃)alkyl und
   R^{6'} und R^{7'} jeweils unabhängig voneinander H, ein (C₁-C₆)-Alkyl, Phenyl,Alkoxyphenyl oder Halogenphenyl sind oder einen Ring aus der Reihe Pyrrolidin, Morpholin, Piperidin bilden.

### Schließlich besonders bevorzugt ist eine Ausgestaltung der Formel (VIII), wobei (Vorzugsbereich 7-2)

M' ein Rest ausgewählt aus den Resten der Formeln (Va-Vz) ist: 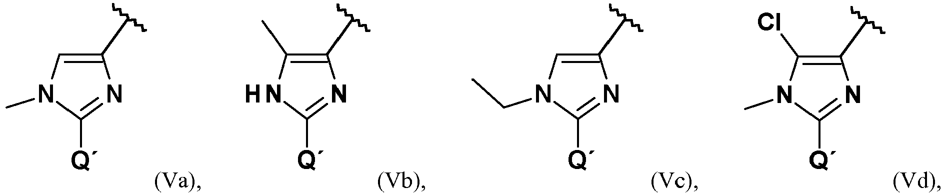 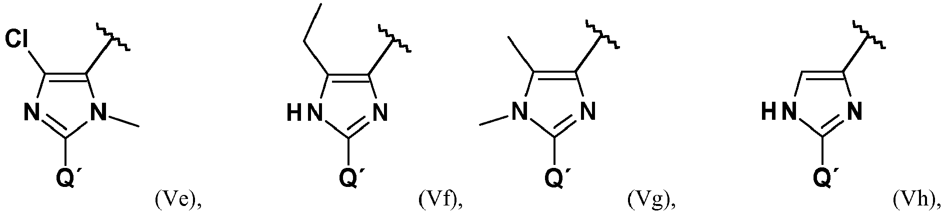 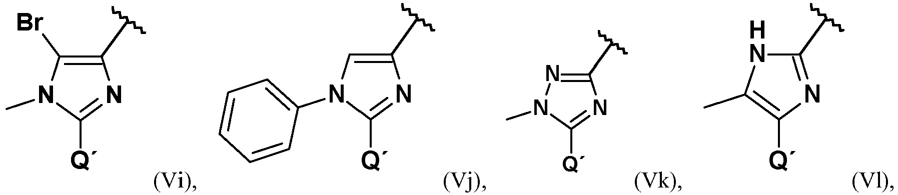 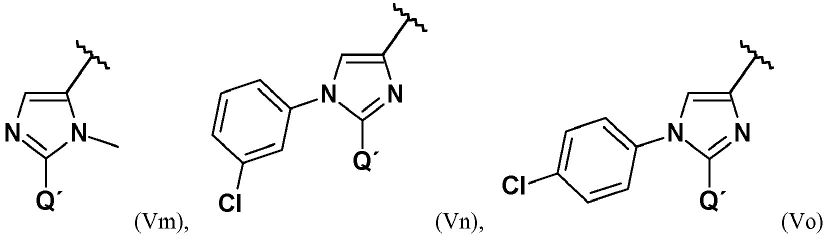 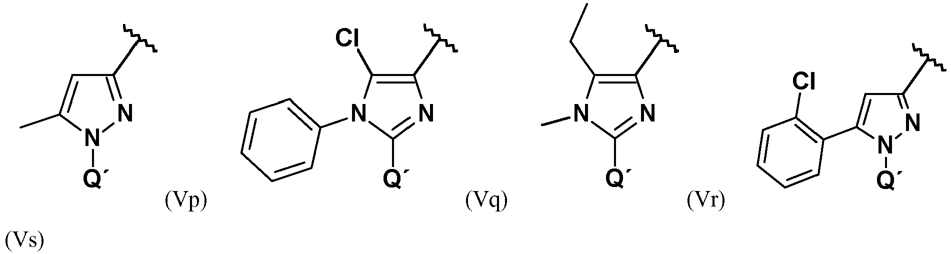 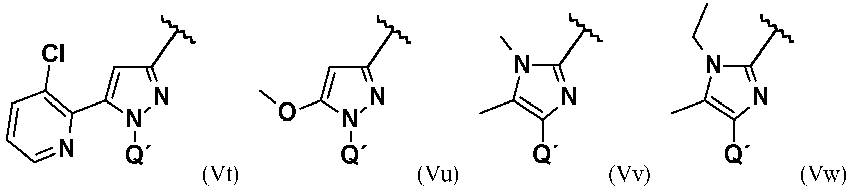 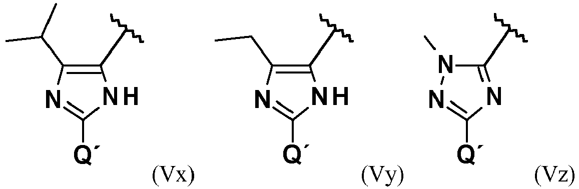 Q' ein Rest ausgewählt aus den Resten der Formeln (VIa-VIz und VIa1-VIa30) ist: 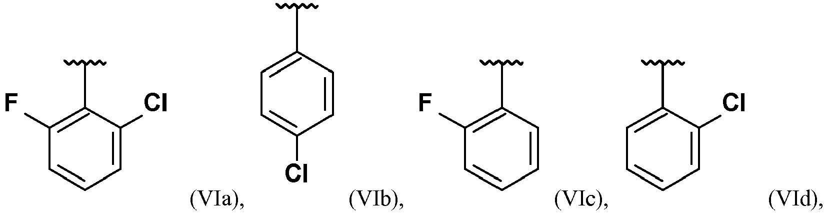 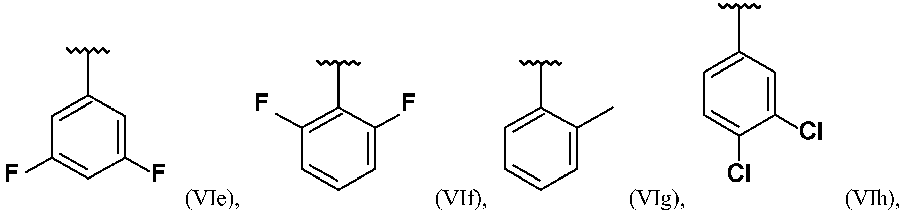 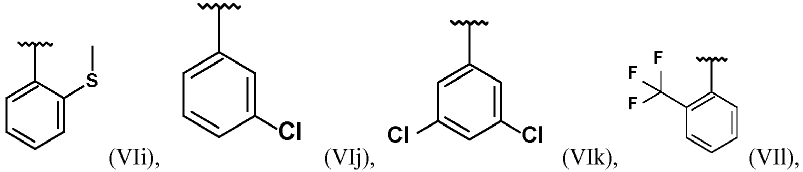 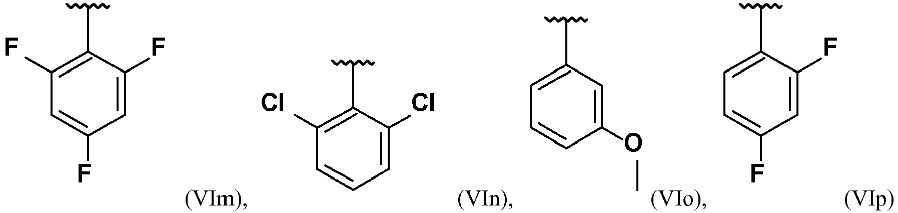 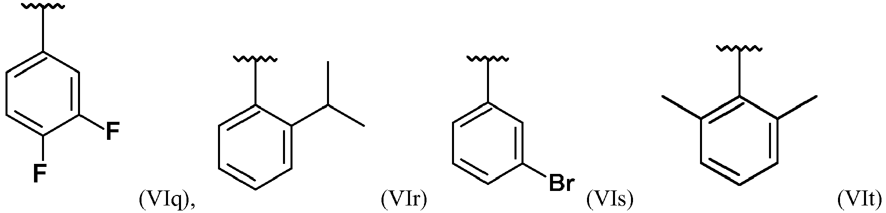 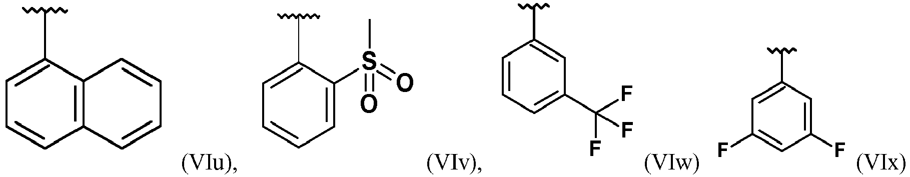 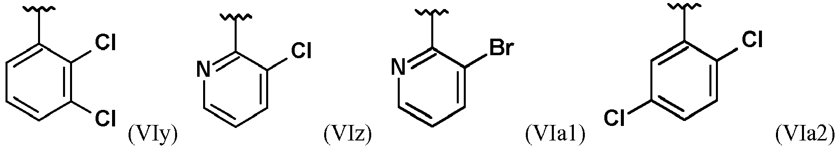 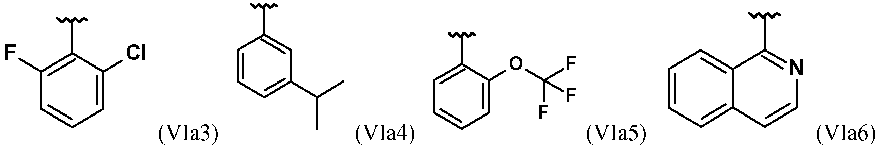 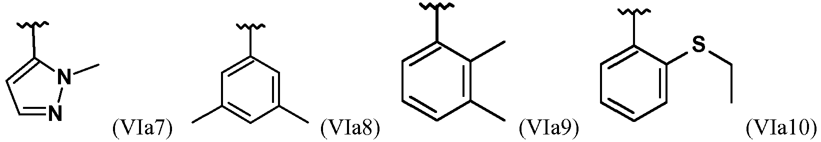 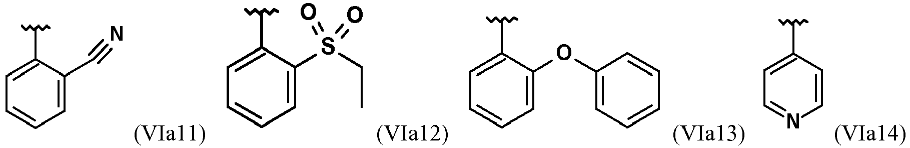 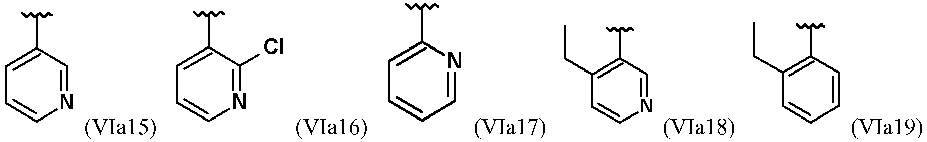 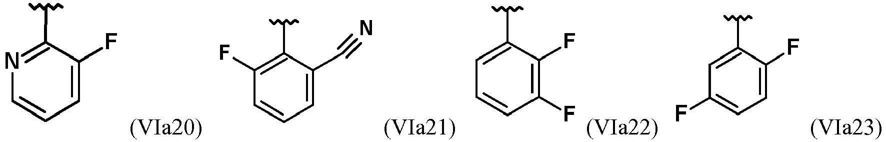 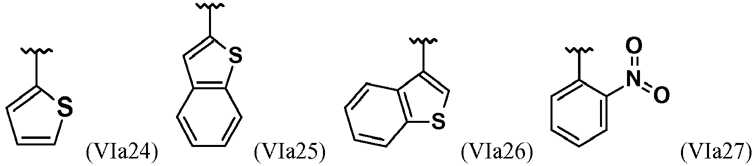 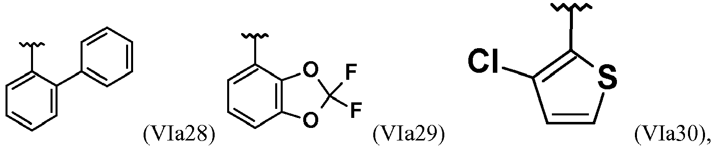 D' ein Rest ausgewählt aus den Resten der Formeln (VII1-VII192) ist

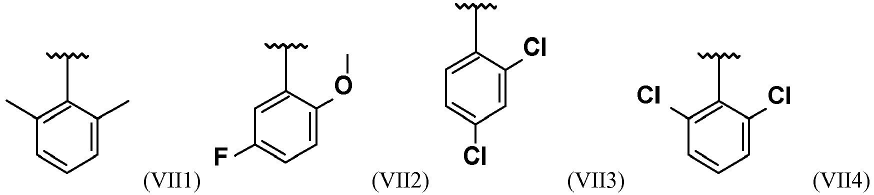

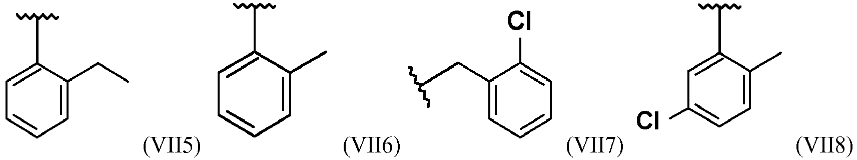

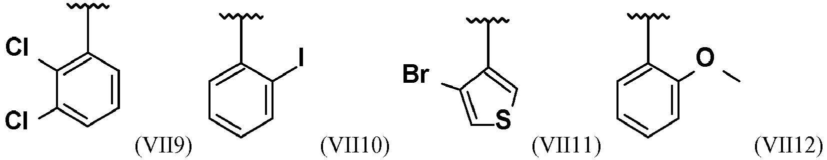

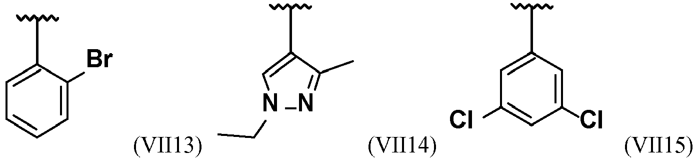

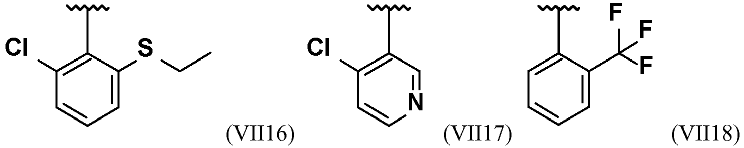

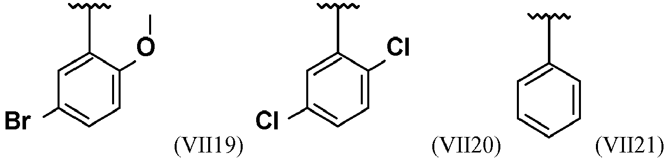

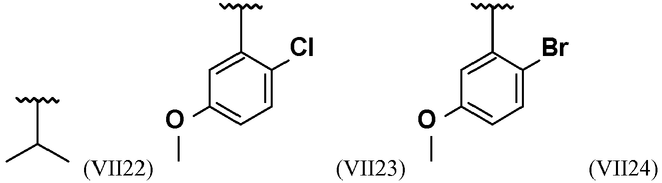

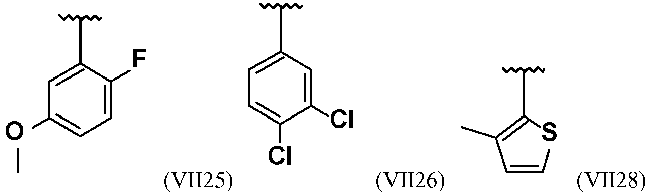

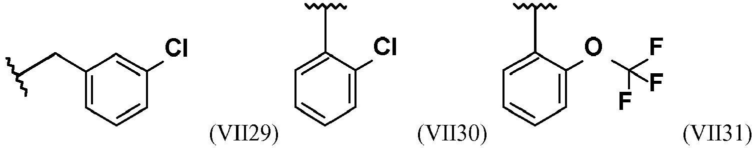

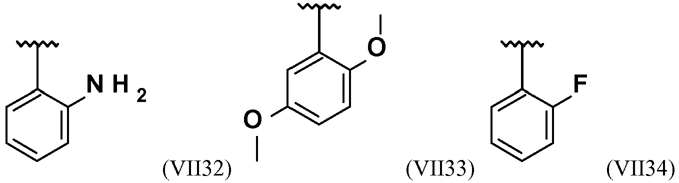

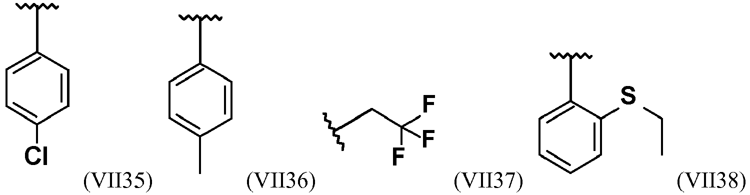

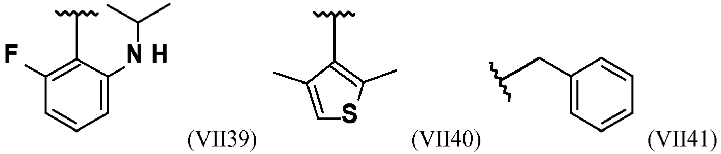

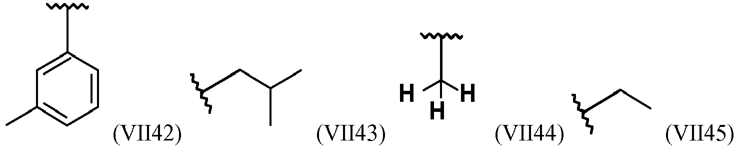

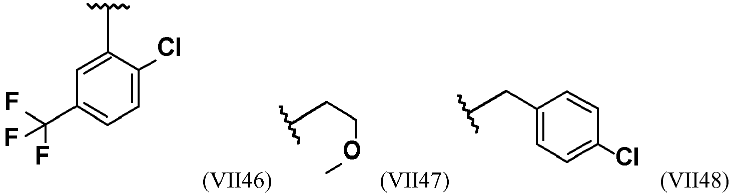

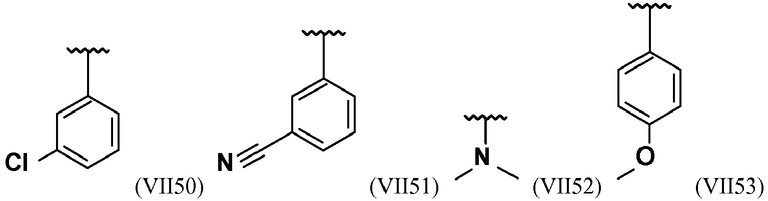

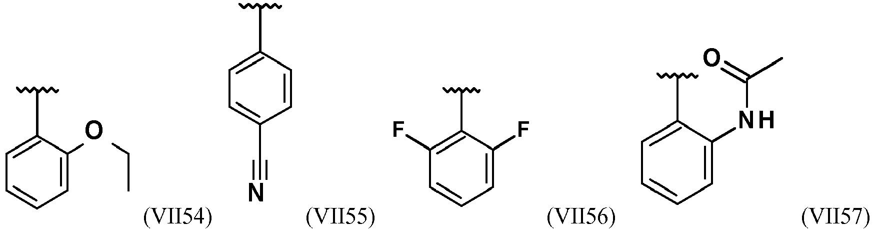

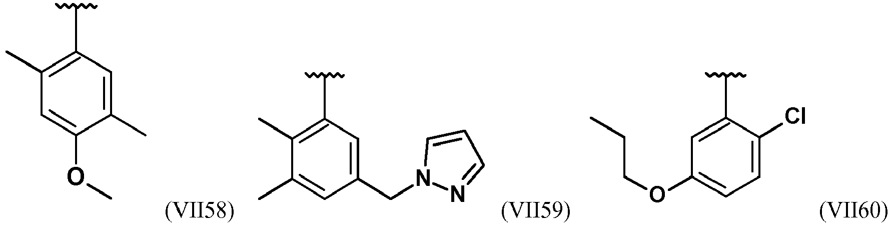

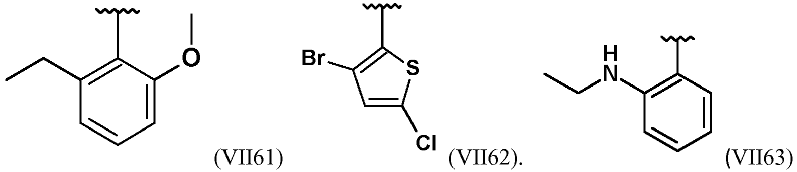

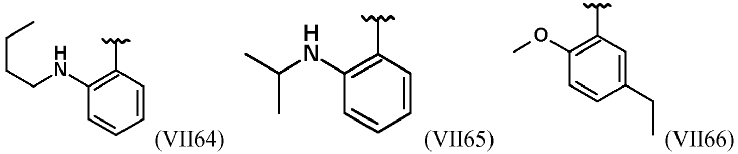

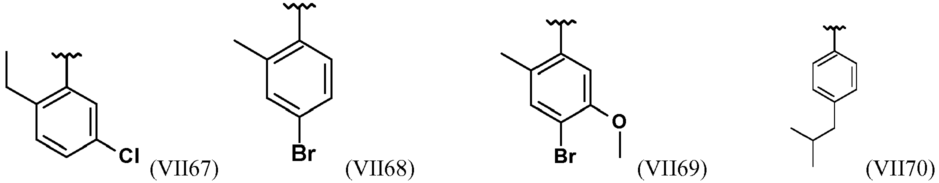

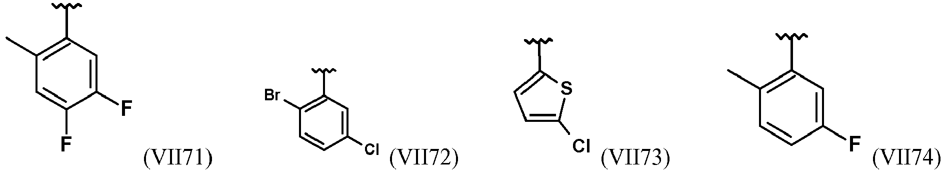

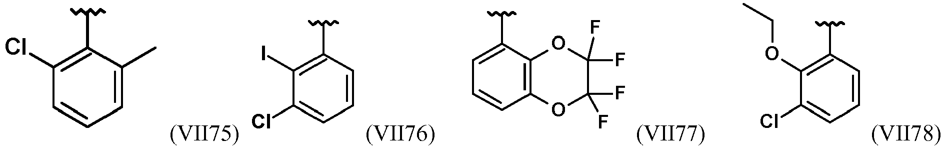

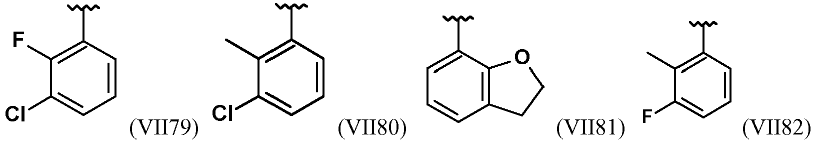

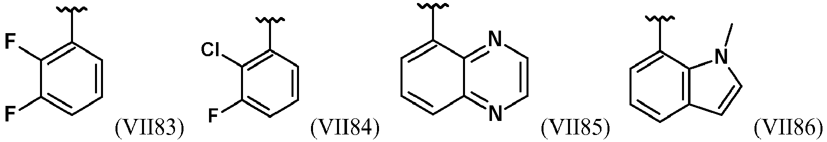

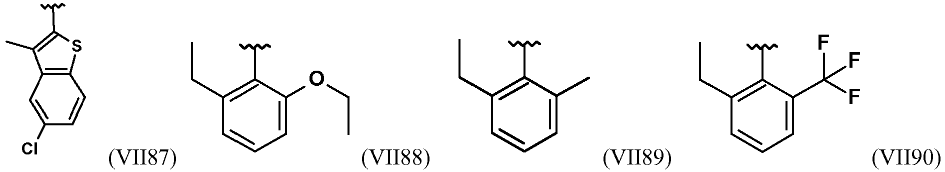

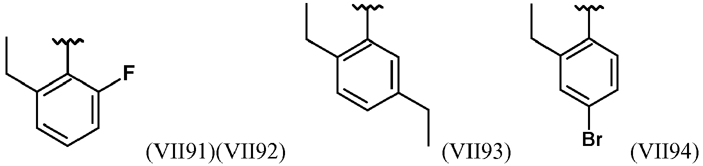

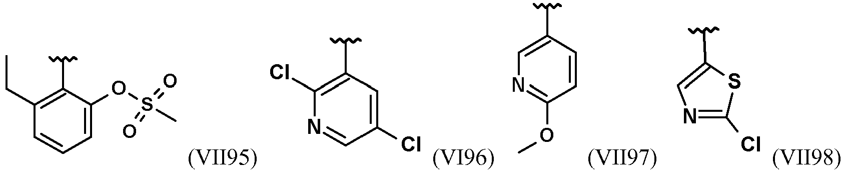

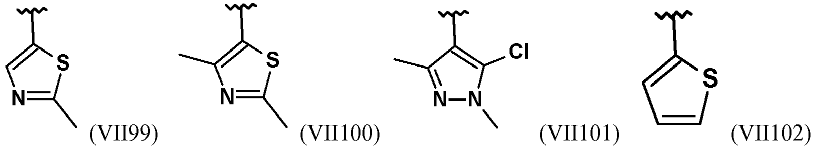

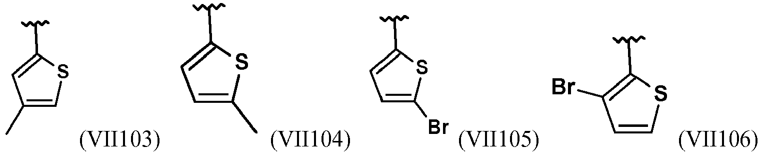

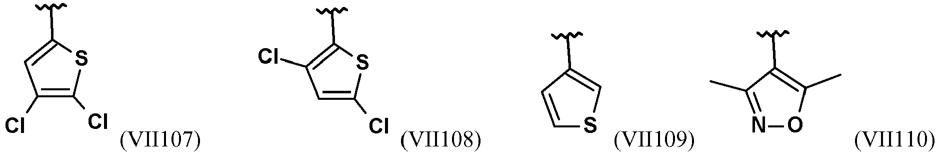

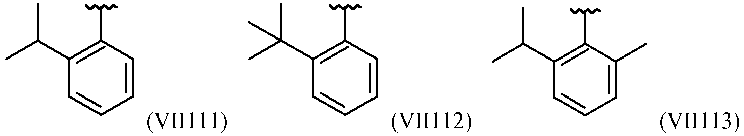

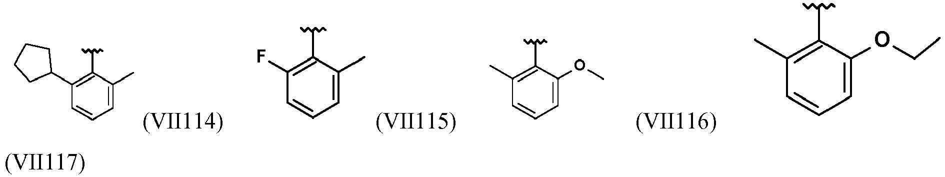

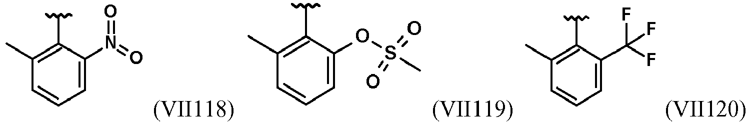

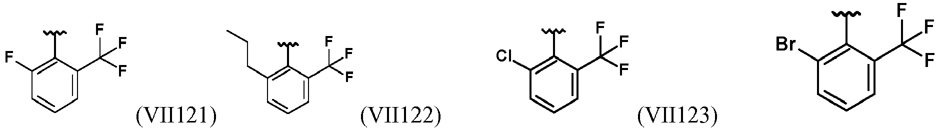

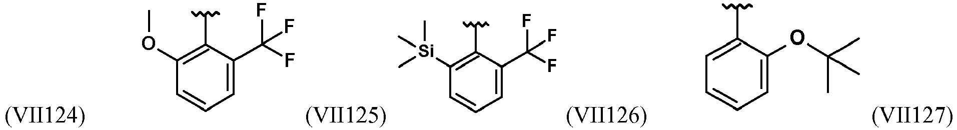

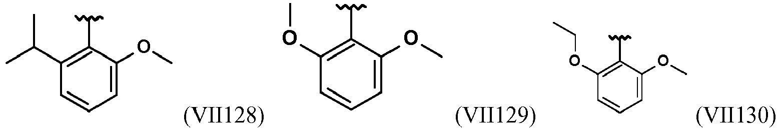

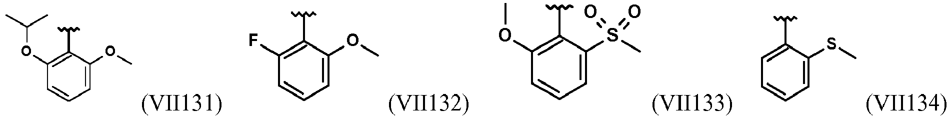

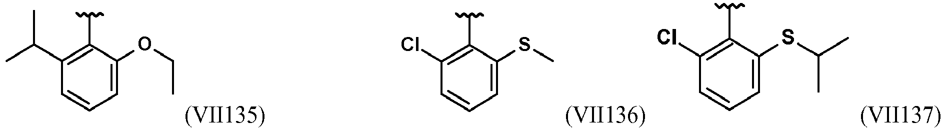

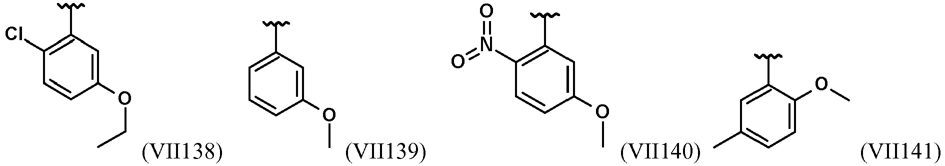

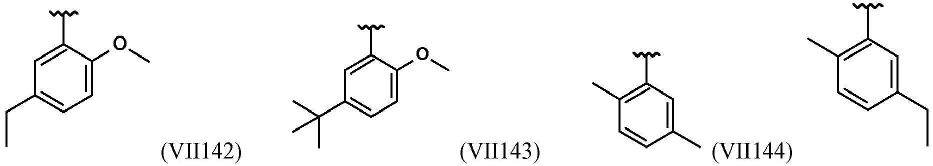

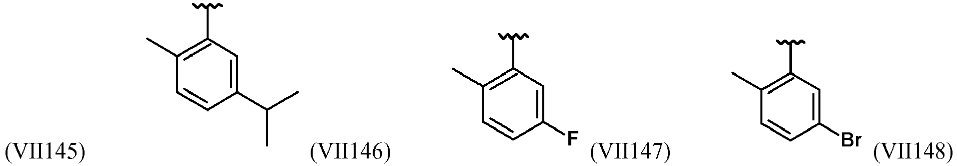

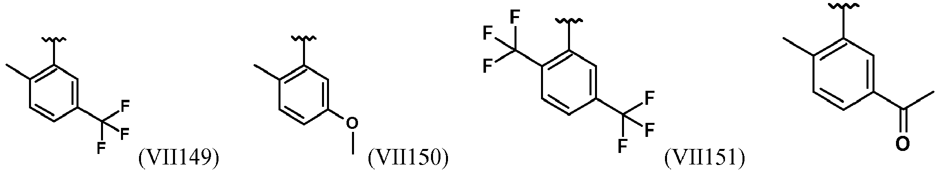

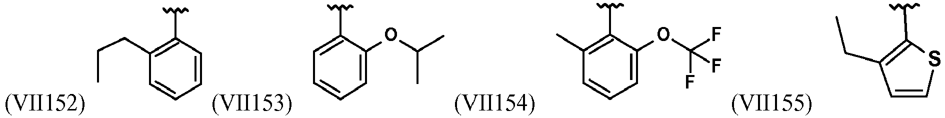

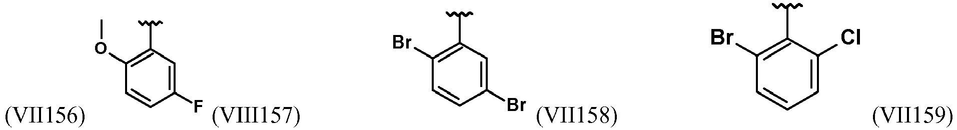

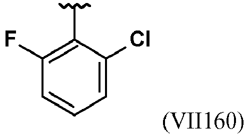

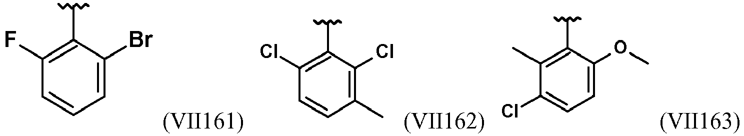

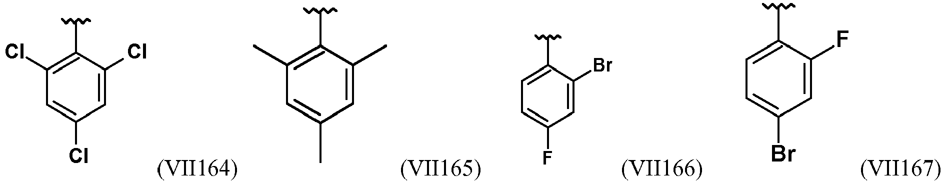

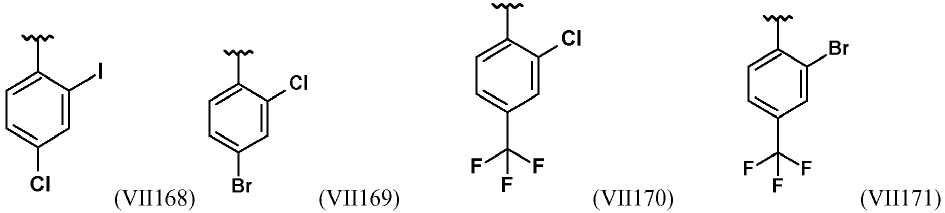

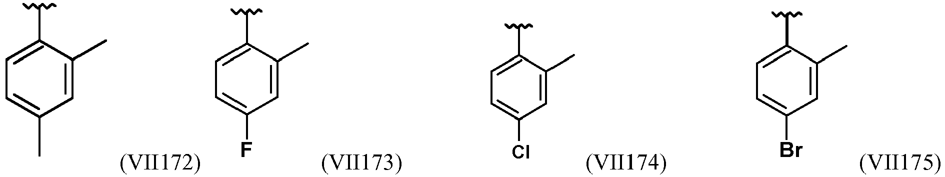

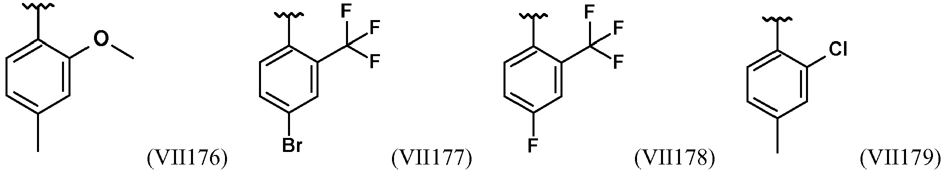

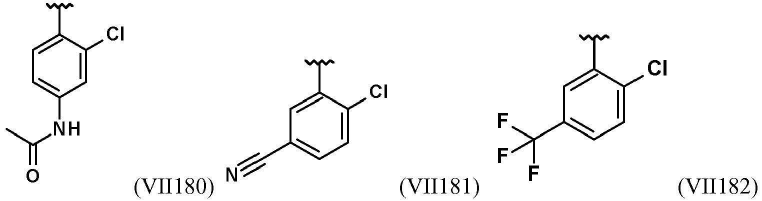

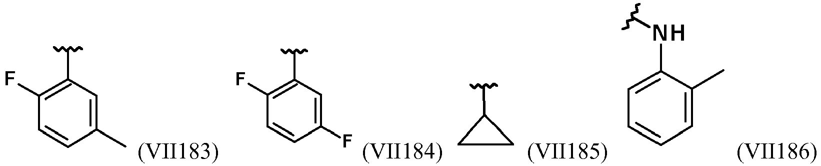

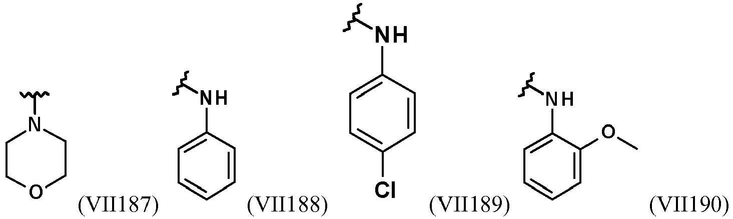

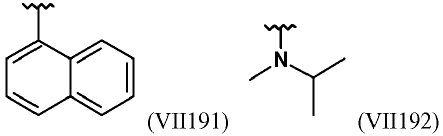

### Noch ein weiterer Gegenstand der Erfindung sind Zwischenprodukte der Formeln XIa - XIq:

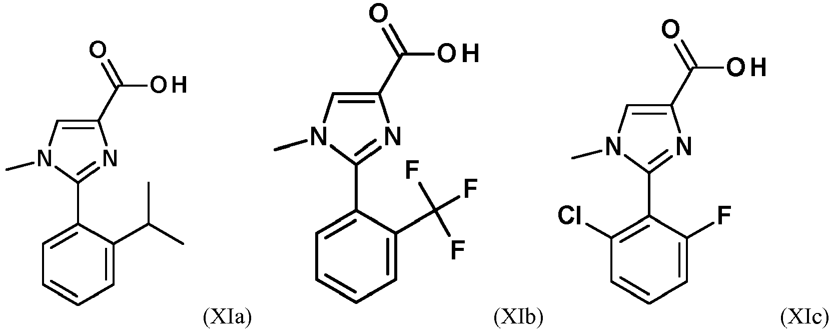

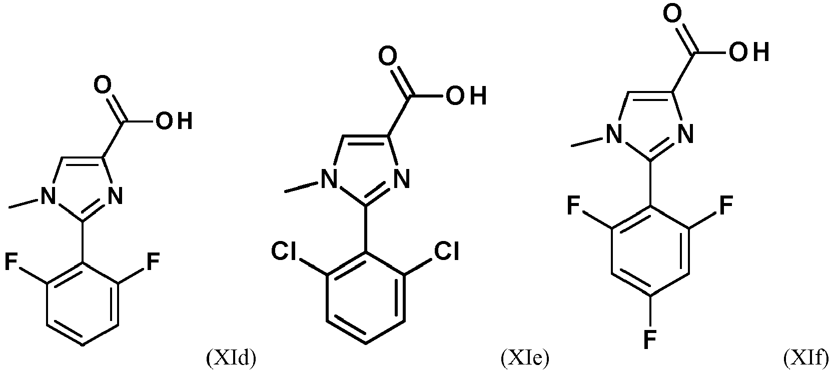

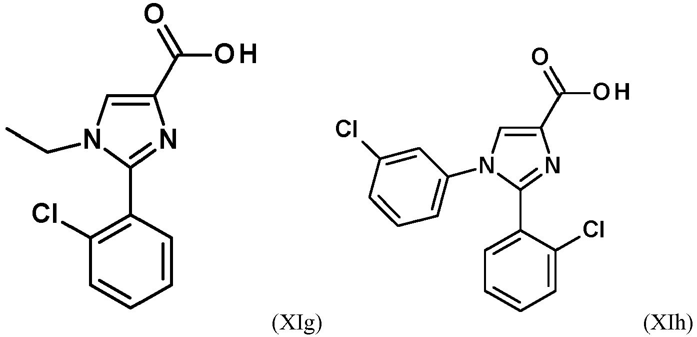

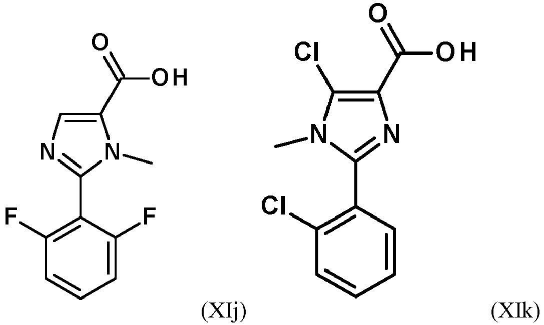

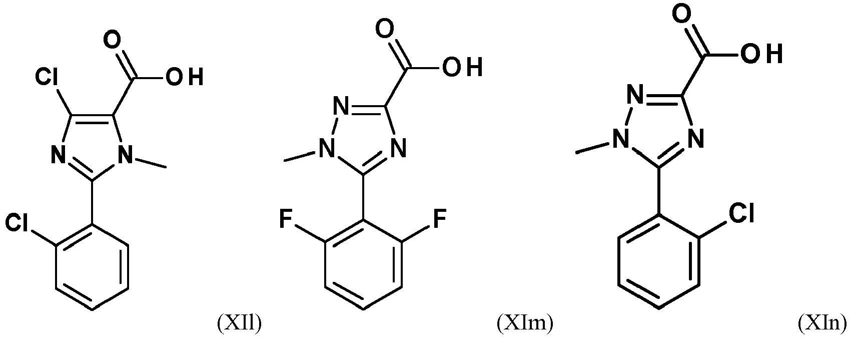

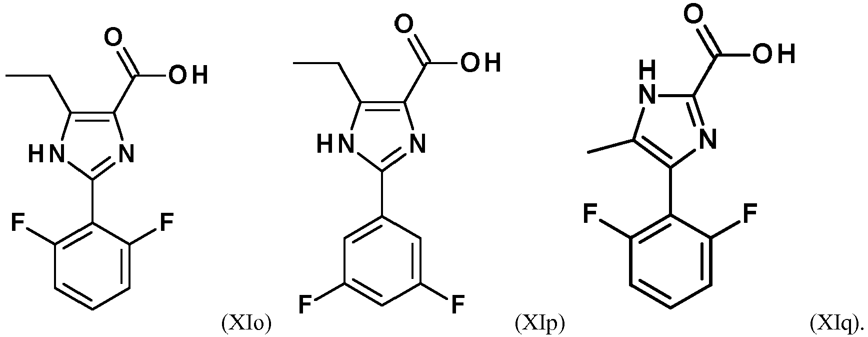

Weitere Vorzugsbereiche der Erfindung sind im Folgenden aufgeführt:
Verwendung einer Verbindung der Formel (I) 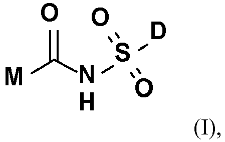 in der
M für einen Rest ausgewählt aus den Formeln (IIa-IIe) steht: 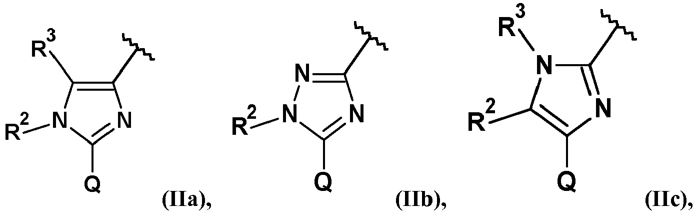 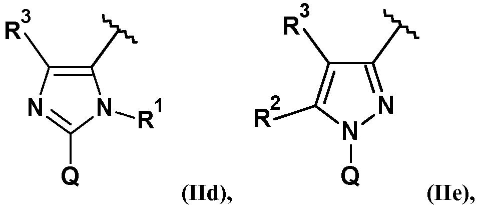 wobei
R¹, R², R³ jeweils unabhängig voneinander H oder ein substituierter oder unsubstituierter Alkyl, Cycloalkyl, Alkenyl, Cycloalkenyl, Cyloheteroalkyl, Aryl, Heteroaryl Rest sind, wobei R² im Falle (IIc) und (IIe) zusätzlich ein Halogen Rest sein kann, und R³ zusätzlich im Falle (IIa), (IId) und (IIe) ein Halogen Rest sein kann;
Q ein substituierter oder unsubstituierter Aryl oder Heteroaryl Rest, im Falle (IIe) aber nicht 2-Pyrimidinyl ist;
D ein substituierter oder unsubstituierter Alkyl, Heteroalkyl, gegebenenfalls teilweise ungesättigter Cycloalkyl, Cycloheteroalkyl, Heteroaryl, Aryl oder Phenylalkyl Rest oder ein substituierter oder unsubstituierter Stickstoff Rest ist,
zur Bekämpfung von tierischen Schädlingen.

Bevorzugt ist eine Ausführunsgform, bei der
R¹, R², R³ jeweils unabhängig voneinander H oder ein substituierter oder unsubstituierter C₁-C₄-Alkyl, C₃-C₆-Cycloalkyl, C₂-C₄-Alkenyl oder Phenyl Rest sind, wobei R² im Falle (IIc) und (IIe) zusätzlich ein Halogen Rest sein kann und wobei R³ zusätzlich im Falle (IIa), (IId) und (IIe) ein Halogen Rest sein kann;
Q ein substituierter oder unsubstituierter Phenyl, Naphthyl, oder Heteroaryl Rest ist, der ein bis drei Heteroatome aus der Reihe Sauerstoff, Schwefel, Stickstoff enthalten kann; im Falle (IIe) aber nicht 2-Pyrimidinyl ist;
D ein substituierter oder unsubstituierter Alkyl, Heteroalkyl, Cycloalkyl, Heteroaryl, Aryl oder Phenyl-(C₁-C₈)alkyl Rest oder ein substituierter oder unsubstituierter Stickstoff Rest ist.
Weiter bevorzugt ist eine Ausführunsgform, bei der
Q ein unsubstituierter oder mit einem oder mehreren Resten R⁴ substituierter Phenyl, Naphthyl, Pyridyl, Pyrimidinyl, Thiophen oder Pyrazol Rest ist, im Falle (IIe) aber nicht 2-Pyrimidinyl ist;
wobei der oder die Substituenten R⁴ jeweils unabhängig voneinander sind:
Wasserstoff, Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino); und/oder
gegebenenfalls einfach oder mehrfach, gleich oder verschieden substituiertes Aryl oder Hetaryl, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino;
D ein unsubstituierter oder mit einem oder mehreren Resten R⁵ substituierter C₁-C₆-Alkyl, Phenyl, Phenyl-(C₁-C₂)alkyl oder Heteroaryl Rest ist, der ein bis drei Heteroatome aus der Reihe Sauerstoff, Schwefel, Stickstoff enthalten kann oder ein NR⁶R⁷ Rest ist,
wobei der oder die Substituenten R⁵ jeweils unabhängig voneinander sind:
   Wasserstoff, Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, 1-Pyrazolyl-(C₁-C₃)Alkyl,
   und /oder
   einfach oder mehrfach, gleich oder verschieden substituiertes Aryl oder Hetaryl, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino;
   und wobei R⁶ und R⁷ jeweils unabhängig voneinander H, ein C₁-C₆-Alkyl, substituierter oder unsubstituierter Phenyl Rest sind, oder R⁶ und R⁷ gemeinsam einen unsubstituierten oder substituierten 4-bis 8- gliedrigen , gesättigten oder gegebenenfalls ganz oder teilweise ungesättigten Ring, bilden können der von 1 bis 3 Heteroatomen aus der Reihe Sauerstoff, Schwefel, Stickstoff unterbrochen sein kann, und der einfach oder mehrfach mit einer der Definition von R⁵ entsprechenden Substitution versehen sein kann.

Noch weiter bevorzugt ist, wenn der oder die Substituenten R⁴ jeweils unabhängig voneinander sind:
Wasserstoff, Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino;
der oder die Substituenten R⁵ jeweils unabhängig voneinander sind:
Wasserstoff, Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, 1-Pyrazolyl-(C₁-C₃)Alkyl;
R⁶ und R⁷ jeweils unabhängig voneinander H, ein C₁-C₆-Alkyl, (C₁-C₆)Halogenalkyl, (C₁-C₆)Alkoxy-(C₁-C₆)alkyl Rest oder ein unsubstituierter Phenyl oder mit Halogen, C₁-C₆-Alkyl, (C₁-C₆)Halogenalkyl, (C₁-C₆)Alkoxy-(C₁-C₆)alkyl substituierter Phenyl Rest sind,
oder
R⁶ und R⁷ gemeinsam einen unsubstituierten oder substituierten 5- bis 6- gliedrigen , gesättigten oder gegebenenfalls ganz oder teilweise ungesättigten Ring, bilden können der von 1 bis 3 Heteroatomen aus der Reihe Sauerstoff, Schwefel, Stickstoff unterbrochen sein kann, und der einfach oder mehrfach mit einer der Definition von R⁵ entsprechenden Substitution versehen sein kann.

Insbesonders bevorzugt ist eine Ausführunsgform, bei der
im Falle (IIa), R², R³ jeweils unabhängig voneinander H oder ein substituierter oder unsubstituierter C₁-C₄-Alkyl, C₃-C₆-Cycloalkyl oder Phenyl Rest sind, wobei R³ zusätzlich ein Halogen Rest sein kann,
im Falle (IIb) R² H oder ein substituierter oder unsubstituierter C₁-C₄-Alkyl, oder C₃-C₆-Cycloalkyl Rest ist,
im Falle (IIc) R², R³ jeweils unabhängig voneinander H oder ein substituierter oder unsubstituierter C₁-C₄-Alkyl, C₃-C₆-Cycloalkyl oder Phenyl Rest sind,
im Falle (IId) R¹, R³ jeweils unabhängig voneinander H oder ein substituierter oder unsubstituierter C₁-C₄-Alkyl, C₃-C₆-Cycloalkyl Rest sind,
im Falle (IIe) R², R³ jeweils unabhängig voneinander H oder Halogen oder ein substituierter oder unsubstituierter C₁-C₄-Alkyl, C₃-C₆-Cycloalkyl, oder Phenyl Rest sind,
D ein unsubstituierter oder mit einem oder mehreren Resten R^{5'} substituierter C₁-C₆-Alkyl Rest oder Phenyl Rest oder Pyridin, Pyrimidin, Pyrazol, Triazol, Thiazol, Oxazol, Thiadiazol, Oxadiazol, Thiophen, Pyrrol, Furan, Tetrahydrofuran, Dioxan Rest oder ein NR^{6'}R^{7'} Rest ist, wobei
R⁶ und R⁷ jeweils unabhängig voneinander H, ein C₁-C₆-Alkyl, (C₁-C₆)Halogenalkyl, (C₁-C₆)Alkoxy-(C₁-C₆)alkyl Rest oder ein unsubstituierter Phenyl oder mit Halogen, C₁-C₆-Alkyl, (C₁-C₆)Halogenalkyl, (C₁-C₆)Alkoxy-(C₁-C₆)alkyl substituierter Phenyl Rest sind,
oder einen Ring aus der Reihe Pyrrolidin, Morpholin, Piperidin bilden.

Noch weiter bevorzugt ist eine Ausführungsform, bei der
M ein Rest ausgewählt aus den Resten der Formeln (IVa), (IVb), (IVc) (IVd), (IVe) 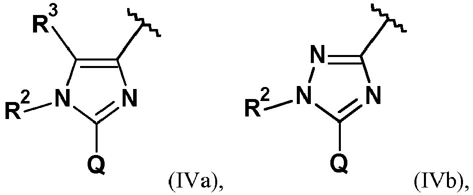 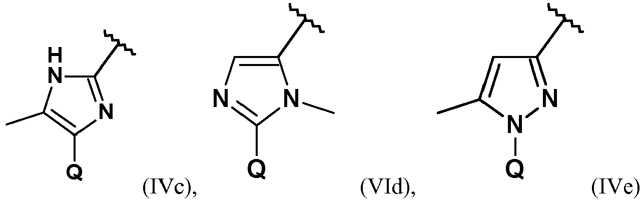 ist,
wobei
im Falle (IVa) R² H, Methyl oder Ethyl oder mit Halogen substituiertes Phenyl ist, undR³ H, Methyl, Ethyl oder Halogen ist, und
im Falle (IVb) R² H, Methyl oder Ethyl ist.
Ganz besonders bevorzugt ist eine Ausführungsform, bei der
Q ein unsubstituierter oder mit einem oder mehreren Resten R⁴ substituierter Phenyl, Naphthyl, Pyridyl, Pyrimidinyl, Thiophen oder Pyrazol-Rest ist; im Falle (IVe) aber nicht 2-Pyrimidinyl ist,
wobei der oder die Substituenten R⁴ unabhängig voneinander sind:
Wasserstoff, Cyano, Halogen, Nitro, Acetyl, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino,
wobei der oder die Substituenten R⁵ unabhängig voneinander sind:
   Wasserstoff, Cyano, Halogen, Nitro, Acetyl, Hydroxy, Carboxy, Amino, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino, 1-Pyrazolyl-(C₁-C₃)Alkyl,
   R⁶ und R⁷ jeweils unabhängig voneinander H, ein C₁-C₆-Alkyl sind oder einen Ring aus der Reihe
   Pyrrolidin, Morpholin, Piperidin bilden.

Noch weiter bevorzugt ist, wenn
M ein Rest ausgewählt aus den Resten der Formeln (Va-Vr) ist: 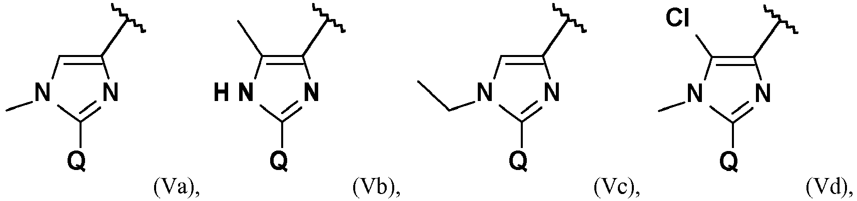 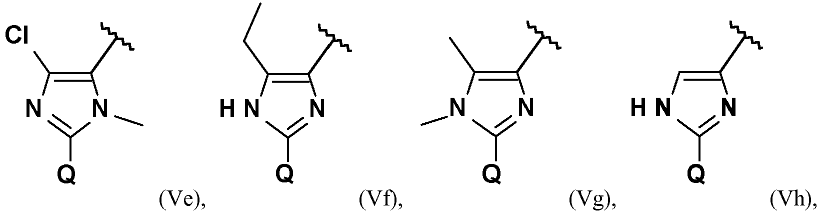 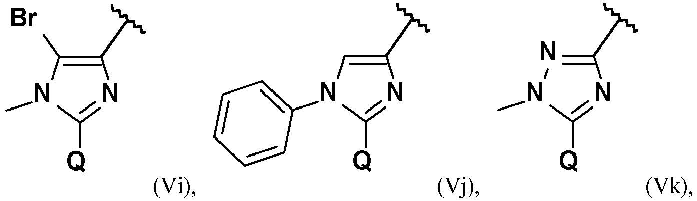 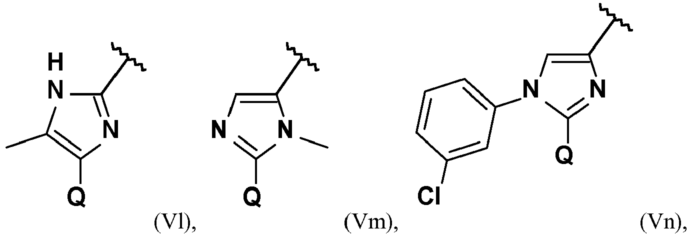 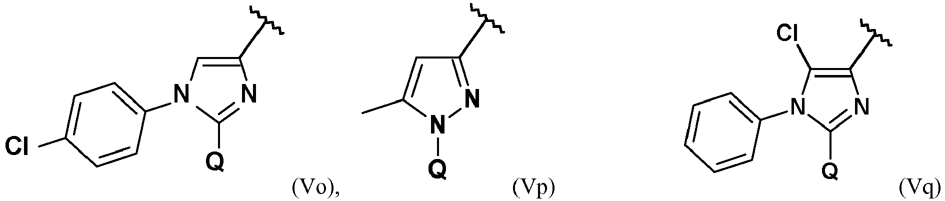 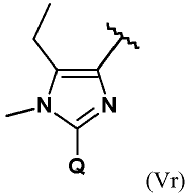 Q ein Rest ausgewählt aus den Resten der Formeln (VIa-VIv) ist: 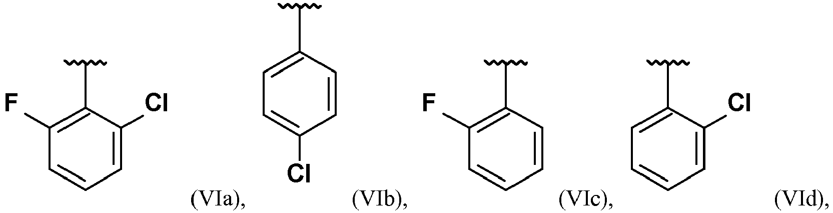 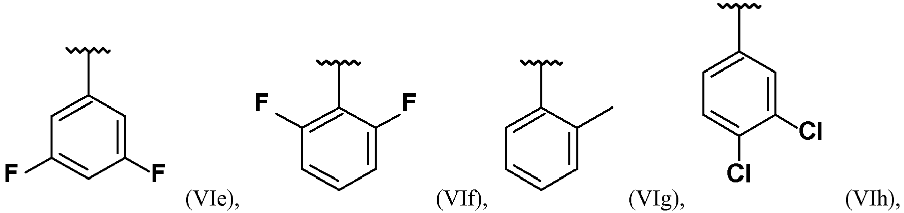 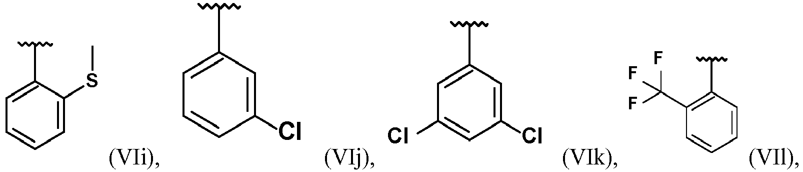 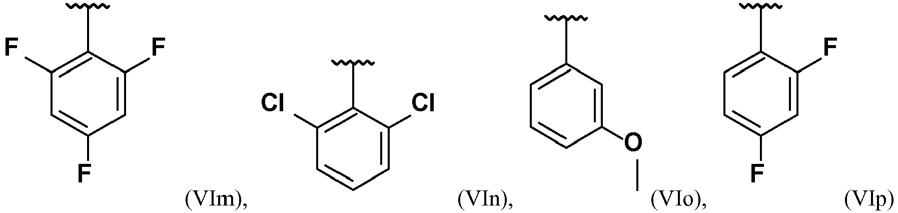 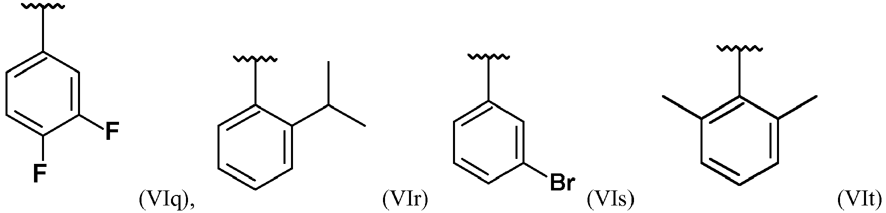 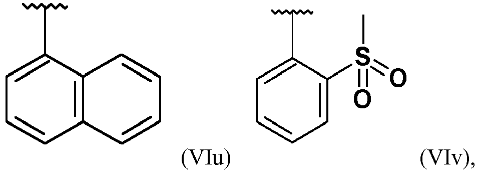 D ein Rest ausgewählt aus den Resten der Formeln (VII1-VII62) ist

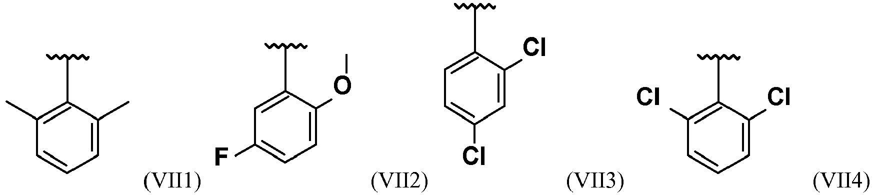

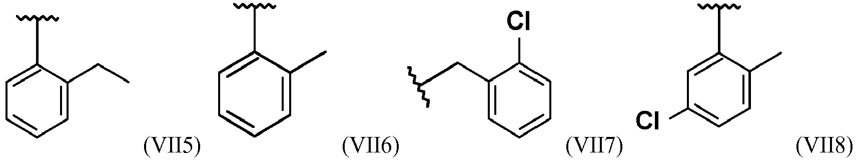

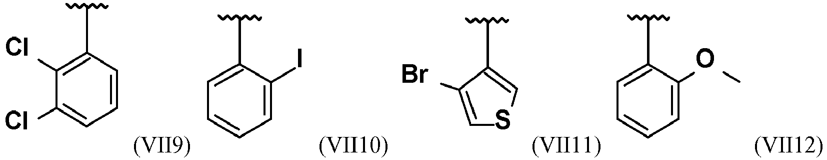

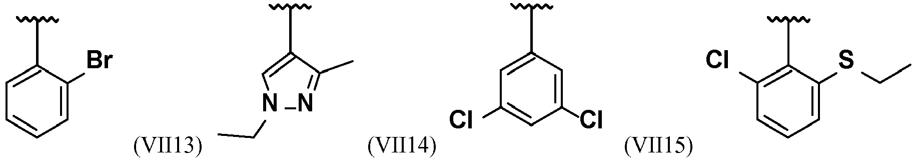

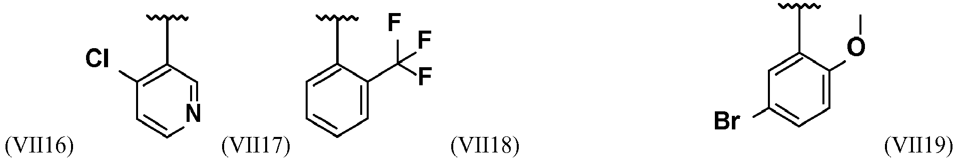

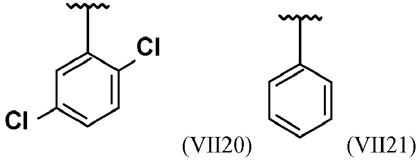

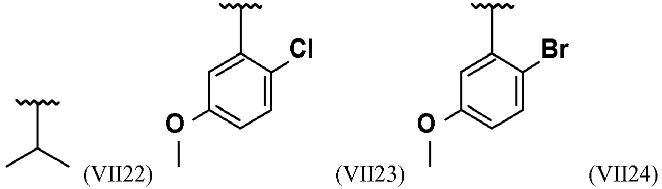

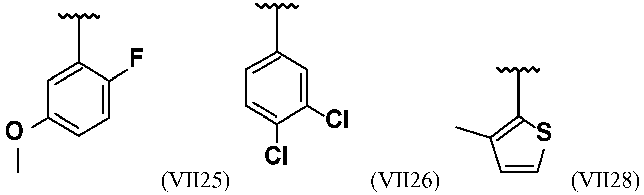

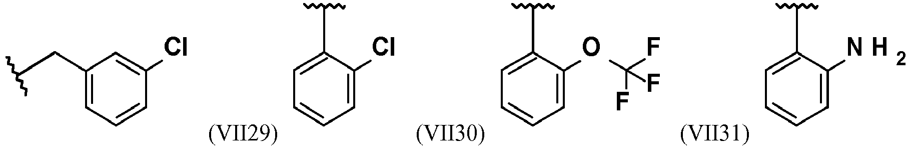

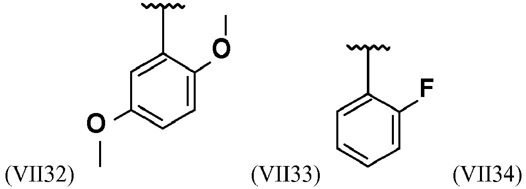

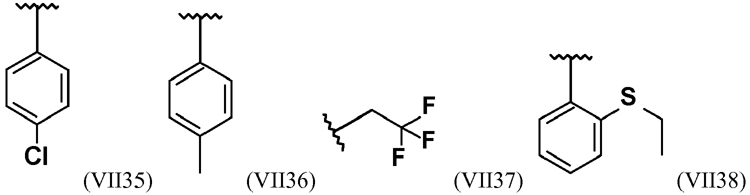

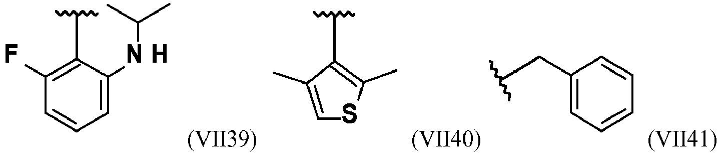

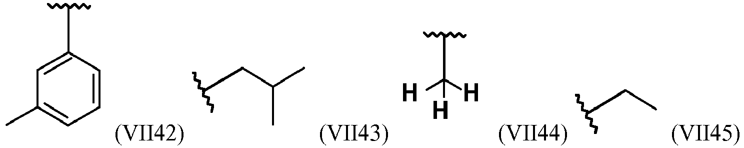

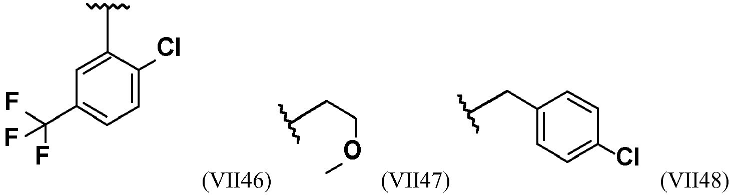

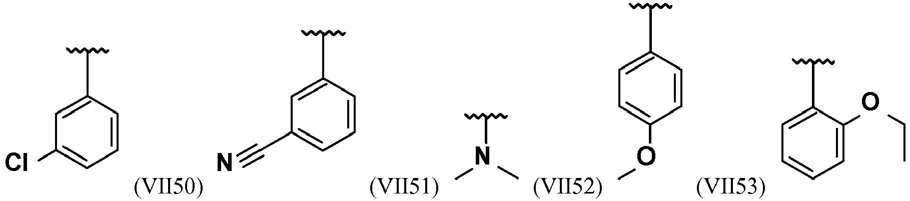

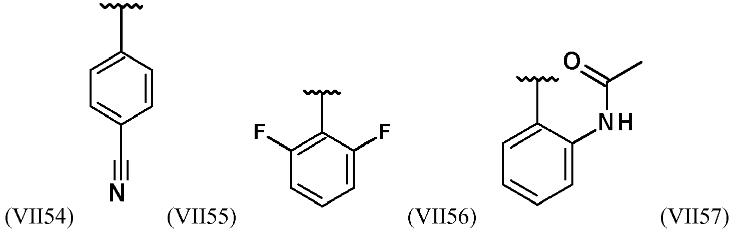

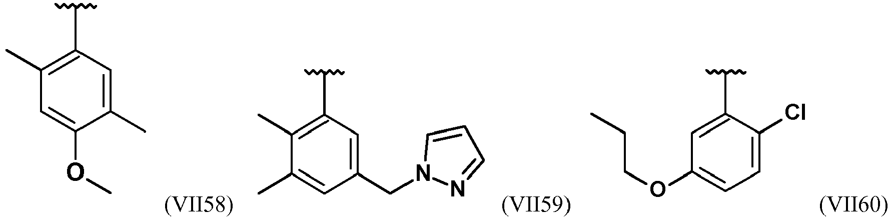

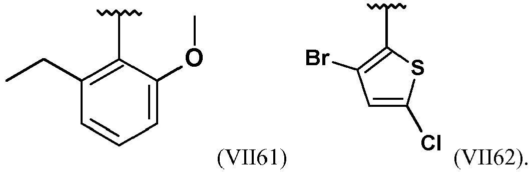

Gemäß einer weiteren bevorzugten Ausführungsform der Erfindung ist die Verwendung einer Verbindung gemäß Formel (I) zum Schutz des Vermehrungsmaterials von Pflanzen vorgesehen.

Gegenstand der Erfindung ist auch ein Mittel, mit einem Gehalt von mindestens einer Verbindung gemäß Formel (I) und üblichen Streckmitteln und/oder oberflächenaktiven Substanzen insbesondere zur Bekämpfung von tierischen Schädlingen.

Ein weiterer Gegenstand der Erfindung ist ein Verfahren zur Bekämpfung von tierischen Schädlingen, bei dem man wenigstens eine Verbindung gemäß Formel (I) oder ein erfindungsgemäßes Mittel auf die tierischen Schädlinge und/oder ihren Lebensraum einwirken lässt, wobei die chirurgische, therapeutische und diagnostische Behandlung des menschlichen oder tierischen Körpers ausgeschlossen ist.

Noch weiterer Gegenstand der Erfindung ist eine agrochemische Formulierung enthaltend wenigstens eine Verbindung gemäß Formel (I) nach einem der Ansprüche 1 bis 8 in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

Eine bevorzugte Asugestaltung der erfindungsgemäßen Fromulierung enthält zusätzlich einen weiteren agrochemischen Wirkstoff.

Ebenfalls Gegenstand der Erfinungs sind Verbindungen der Formel (VIII) 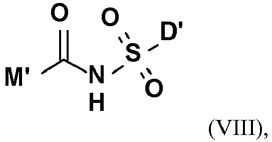 in der
M' für einen Rest der Formel (II) steht, ausgewählt aus: 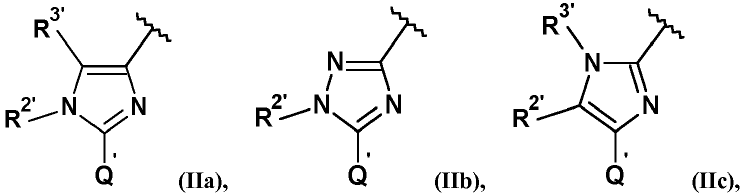 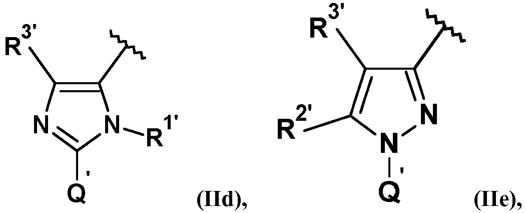 in der
R^{1'}, R^{2'}, R^{3'} jeweils unabhängig voneinander H oder ein substituierter oder unsubstituierter Alkyl, Cycloalkyl, Alkenyl, Cycloalkenyl, Cyloheteroalkyl, Aryl, Heteroaryl Rest sind, wobei R^{2'} im Falle (IIc) und (IIe) zusätzlich ein Halogen Rest sein kann, und R^{2'} im Falle (IIa) und (IIb) nur H oder ein substituierter oder unsubstituierter Alkyl, Cycloalkyl Rest sein kann, R^{3'} zusätzlich im Falle (IIa), (IId) und (IIe) ein Halogen Rest sein kann;
Q' ein substituierter oder unsubstituierter Aryl oder Heteroaryl Rest, aber im Falle (IIa), (IId) im Falle R^{1'}=H und R^{3'} =Methyl nicht 3-Methoxyphenyl ist, im Falle (IId) nicht 3-Pyridyl ist, im Falle (IIe) nicht 2-Pyrimidinyl, nicht unsubstituiertes Phenyl, nicht 3,4-Dichlorphenyl und nicht 3,5-bis-tert-Butyl ist;
D' ein substituierter oder unsubstituierter Alkyl, Heteroalkyl, gegebenenfalls teilweise ungesättigter Cycloalkyl, Cycloheteroalkyl, Heteroaryl, Aryl oder Phenylalkyl Rest oder im Falle, daß Q' mindestens einen Substituenten in 2-Stellung trägt, ein substituierter oder unsubstituierter Stickstoff Rest ist.
Gemäß einer ersten bevorzugten Ausführungsfrom der erfindungsgemäßen Verbindungen ist vorgesehen, dass
Q' ein substituierter oder unsubstituierter Phenyl, Naphthyl, Heteroaryl Rest ist, der ein bis drei Heteroatome aus der Reihe Sauerstoff, Schwefel, Stickstoff enthalten kann;
D' ein substituierter oder unsubstituierter Alkyl, Heteroalkyl, Cycloalkyl, Heteroaryl, Aryl oder Phenyl-(C₁-C₈)alkyl Rest oder im Falle, daß Q' mindestens einen Substituenten in 2-Stellung trägt, ein substituierter oder unsubstituierter Stickstoff Rest ist;
im Falle (IIa) R^{2'}, R^{3'} jeweils unabhängig voneinander H oder ein substituierter oder unsubstituierter C₁-C₄-Alkyl, C₃-C₆-Cycloalkyl Rest sind, wobei R³ zusätzlich ein Halogen Rest sein kann;
im Falle (IIb) R^{2'} H oder ein substituierter oder unsubstituierter C₁-C₄-Alkyl, oder C₃-C₆-Cycloalkyl Rest ist,
im Falle (IIc) R^{2'}, R^{3'} jeweils unabhängig voneinander H oder ein substituierter oder unsubstituierter C₁-C₄-Alkyl, C₃-C₆-Cycloalkyl oder Phenyl Rest sind, im Falle (IId) R^{1'}, R^{3'} jeweils unabhängig voneinander H oder ein substituierter oder unsubstituierter C₁-C₄-Alkyl, C₃-C₆-Cycloalkyl Rest sind und R³ zusätzlich ein Halogen Rest sein kann;
im Falle (IIe) R^{2'}, R^{3'} jeweils unabhängig voneinander H oder Halogen oder ein substituierter oder unsubstituierter C₁-C₄-Alkyl, C₃-C₆-Cycloalkyl Rest sind.

Weiter bevorzugt ist, wenn
Q' ein unsubstituierter oder mit einem oder mehreren Resten R^{4'} substituierter Phenyl, Naphthyl, Pyridyl, Pyrimidinyl, Thiophen oder Pyrazol-Rest ist; wobei der oder die Substituenten R^{4'} jeweils unabhängig voneinander sind:
Wasserstoff, Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino; und/oder
gegebenenfalls einfach oder mehrfach, gleich oder verschieden substituiertes Aryl oder Hetaryl, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino;
D' ein unsubstituierter oder mit einem oder mehreren Resten R^{5'} substituierter C₁-C₆-Alkyl, Phenyl oder Heteroaryl Rest ist, der ein bis drei Heteroatome aus der Reihe Sauerstoff, Schwefel, Stickstoff enthalten kann oder oder im Falle, daß Q mindestens einen Substituenten in 2-Stellung trägt, ein NR^{6'}R^{7'} Rest ist,
wobei der oder die Substituenten R^{5'} jeweils unabhängig voneinander sind:
   Wasserstoff, Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, 1-Pyrazolyl-(C₁-C₃)Alkyl,
   und /oder
   einfach oder mehrfach, gleich oder verschieden substituiertes Aryl oder Hetaryl, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino,
   und wobei R^{6'} und R^{7'} jeweils unabhängig voneinander H, ein C₁-C₆-Alkyl, substituierter oder ein unsubstituierter Phenyl Rest sind, oder R^{6'} und R^{7'} gemeinsam einen unsubstituierten oder substituierten 5- bis 6- gliedrigen, gesättigten oder gegebenenfalls ganz oder teilweise ungesättigten Ring bilden können der von 1 bis 3 Heteroatomen aus der Reihe Sauerstoff, Schwefel, Stickstoff unterbrochen sein kann, und der einfach oder mehrfach mit einer der Definition von R^{5'} entsprechenden Substitution versehen sein kann.

Noch weiter bevorzugt ist, wenn
der oder die Substituenten R^{4'} jeweils unabhängig voneinander sind:
Wasserstoff, Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, -(C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, -(C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, -(C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino;
D' ein unsubstituierter oder mit einem oder mehreren Resten R^{5'} substituierter Phenyl, Pyridin, Pyrimidin, Pyrazol, Triazol, Thiazol, Oxazol, Thiadiazol, Oxadiazol, Thiophen, Pyrrol, Furan, Tetrahydrofuran, Dioxan oder C₁-C₆-Alkyl Rest oder ein NR^{6'}R^{7'} Rest ist,
wobei der oder die Substituenten R^{5'} jeweils unabhängig voneinander sind:
   Wasserstoff, Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, 1-Pyrazolyl-(C₁-C₃)Alkyl,
   und wobei R^{6'} und R^{7'} jeweils unabhängig voneinander H, ein C₁-C₆-Alkyl, (C₁-C₆)Halogenalkyl, (C₁-C₆)Alkoxy-(C₁-C₆)alkyl Rest oder ein unsubstituierter Phenyl oder mit Halogen, C₁-C₆-Alkyl, (C₁-C₆)Halogenalkyl, (C₁-C₆)Alkoxy-(C₁-C₆)alkyl substituierter Phenyl Rest sind.

Insbesonders bevorzugt ist eine Ausführunsgform, bei der
M ein Rest ausgewählt aus den Resten der Formeln (IIa), (IIb), (IIc) (IId), (IIe) 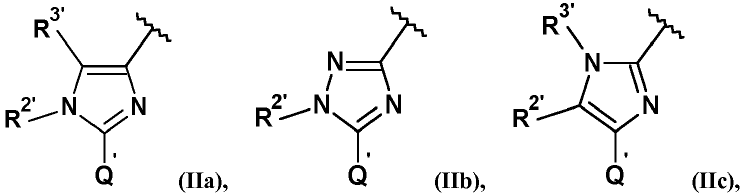 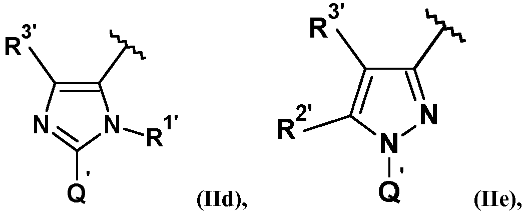 ist,
wobei
im Falle (IIa) R^{2'}, R^{3'} jeweils unabhängig voneinander H oder ein substituierter oder unsubstituierter C₁-C₄-Alkyl, C₃-C₆-Cycloalkyl Rest sind, wobei R³ zusätzlich ein Halogen Rest sein kann,
im Falle (IIb) R^{2'} H oder ein substituierter oder unsubstituierter C₁-C₄-Alkyl, oder C₃-C₆-Cycloalkyl Rest ist,
im Falle (IIc) R^{2'}, R^{3'} jeweils unabhängig voneinander H oder ein substituierter oder unsubstituierter C₁-C₄-Alkyl, C₃-C₆-Cycloalkyl oder Phenyl Rest sind,
im Falle (IId) R^{1'}, R^{3'} jeweils unabhängig voneinander H oder ein substituierter oder unsubstituierter C₁-C₄-Alkyl, C₃-C₆-Cycloalkyl Rest sind und R³ zusätzlich ein Halogen Rest sein kann,
im Falle (IIe) R^{2'}, R^{3'} jeweils unabhängig voneinander H oder Halogen oder ein substituierter oder unsubstituierter C₁-C₄-Alkyl, C₃-C₆-Cycloalkyl Rest sind;
D' ein unsubstituierter oder mit einem oder mehreren Resten R^{5'} substituierter C₁-C₆-Alkyl Rest oder Phenyl Rest oder Pyridin, Pyrimidin, Pyrazol, Triazol, Thiazol, Oxazol, Thiadiazol, Oxadiazol, Thiophen, Pyrrol, Furan, Tetrahydrofuran, Dioxan Rest oder ein NR^{6'}R^{7'} Rest ist, wobei
R^{6'} und R^{7'} jeweils unabhängig voneinander H, ein C₁-C₆-Alkyl, (C₁-C₆)Halogenalkyl, (C₁-C₆)Alkoxy-(C₁-C₆)alkyl Rest oder unsubstituierter Phenyl oder mit Halogen, C₁-C₆-Alkyl, (C₁-C₆)Halogenalkyl, (C₁-C₆)Alkoxy-(C₁-C₆)alkyl substituierter Phenyl Rest sind,
oder einen Ring aus der Reihe Pyrrolidin, Morpholin, Piperidin bilden.

Ganz besonders bevorzugt ist, wenn
M' ein Rest ausgewählt aus den Resten der Formeln (IVa), (IVb), (IVc), (IVd), (IVe) ist, 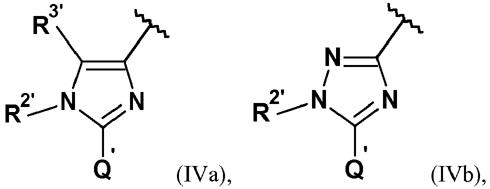 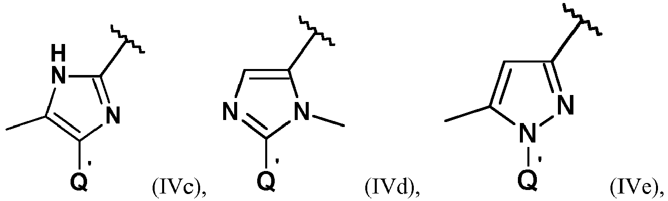 wobei
im Falle (IVa) R^{2'}' H, Methyl oder Ethyl ist, R^{3'} H, Methyl, Ethyl oder Halogen ist;
im Falle (IVb) R^{2'} H, Methyl oder Ethyl ist;
Q' ein unsubstituierter oder mit einem oder mehreren Resten R^{4'} substituierter Phenyl, Naphthyl, Pyridyl, Pyrimidinyl, Thiophen oder Pyrazol-Rest ist;
D' ein unsubstituierter oder mit einem oder mehreren Resten R^{5'} substituierter Phenyl, Pyridin, Pyrimidin, Pyrazol, Triazol, Thiazol, Oxazol, Thiadiazol, Oxadiazol, Thiophen, Pyrrol, Furan, Tetrahydrofuran, Dioxan oder C₁-C₆-Alkyl Rest oder ein NR^{6'}R^{7'} Rest ist.

Noch weiter bevorzugt ist, wenn
Q' ein unsubstituierter oder mit einem oder mehreren Resten R^{4'} substituierter Phenyl, Naphthyl, Pyridyl, Pyrimidinyl, Thiophen oder Pyrazol-Rest ist;
wobei der oder die Substituenten R^{4'} unabhängig voneinander sind:
Wasserstoff, Cyano, Halogen, Nitro, Acetyl, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloa!kyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenaₗkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino;
D' ein unsubstituierter oder mit einem oder mehreren Resten R^{5'} substituierter C₁-C₆-Alkyl Rest oder Phenyl Rest oder Pyridin, Pyrimidin, Pyrazol, Triazol, Thiazol, Oxazol, Thiadiazol, Oxadiazol, Thiophen, Pyrrol, Furan, Tetrahydrofuran, Dioxan Rest ist,
wobei der oder die Substituenten R^{5'} unabhängig voneinander sind:
   Wasserstoff, Cyano, Halogen, Nitro, Acetyl, Hydroxy, Carboxy, Amino, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino, 1-Pyrazolyl- (C₁-C₃)Alkyl,
   R^{6'} und R^{7'} jeweils unabhängig voneinander
   H, ein C₁-C₆-Alkyl, (C₁-C₆)Halogenalkyl, (C₁-C₆)Alkoxy-(C₁-C₆)alkyl Rest oder unsubstituierter Phenyl oder mit Halogen, C₁-C₆-Alkyl, (C₁-C₆)Halogenalkyl, (C₁-C₆)Alkoxy-(C₁-C₆)alkyl substituierter Phenyl Rest sind.

Schließlich besonders bevorzugt ist auch, wenn
M' ein Rest ausgewählt aus den Resten der Formeln (Va-Vr) ist: 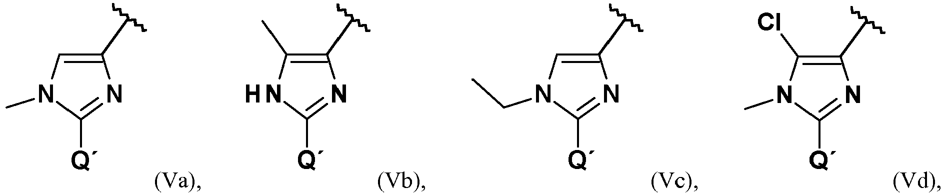 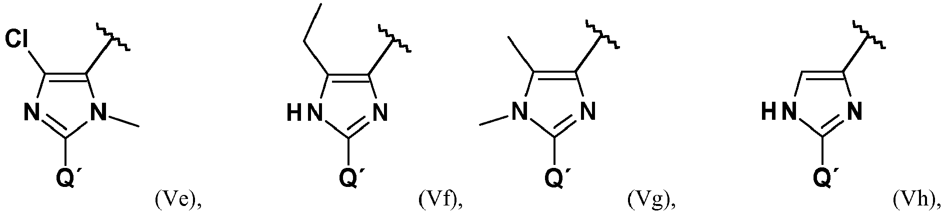 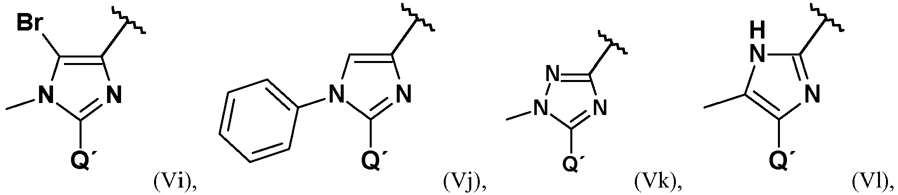 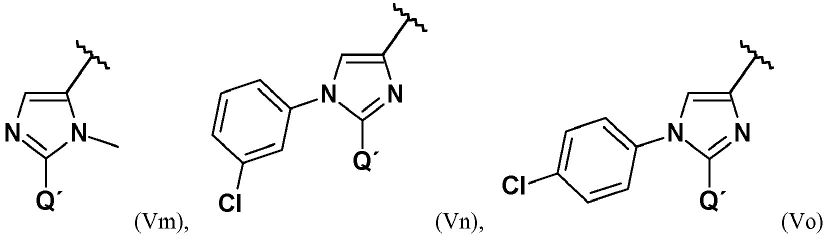 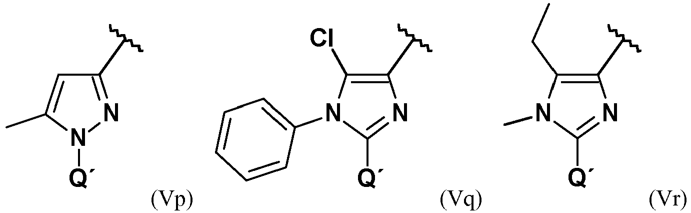 Q' ein Rest ausgewählt aus den Resten der Formeln (VIa-VIv) ist: 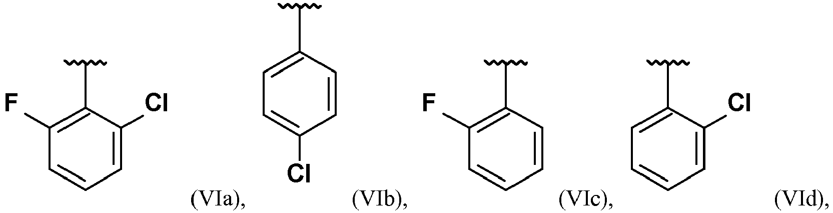 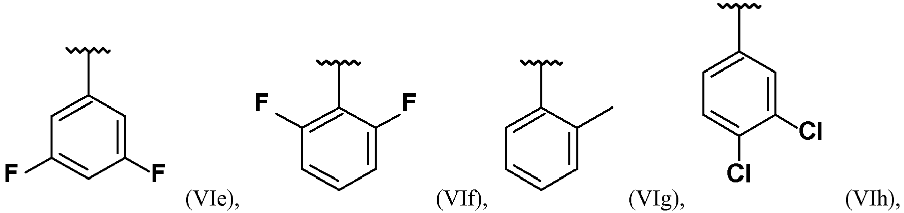 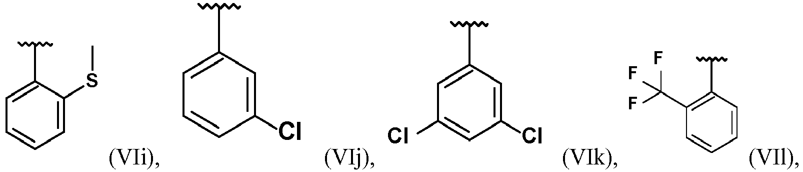 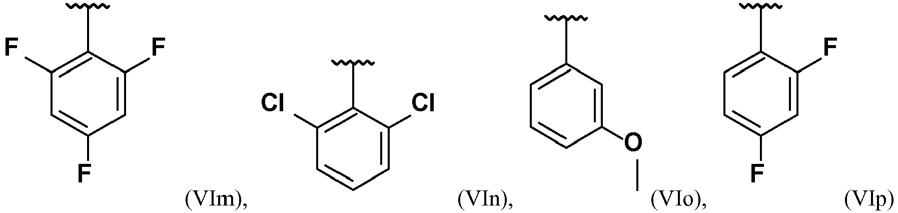 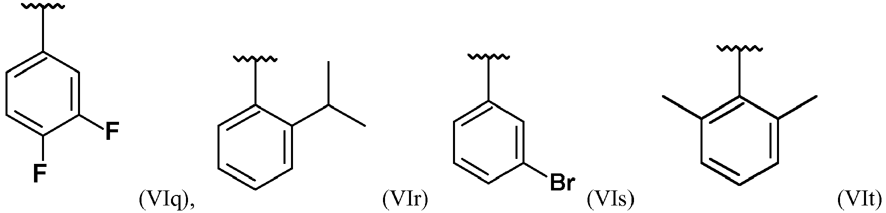 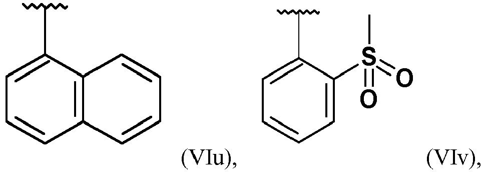 D' ein Rest ausgewählt aus den Resten der Formeln (VII1-VII62) ist

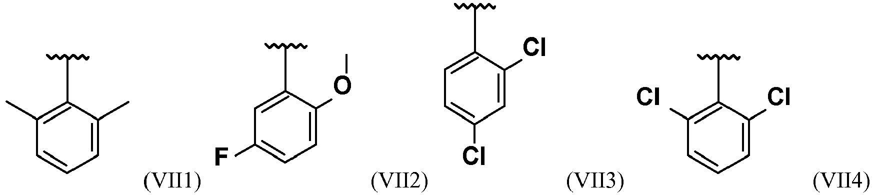

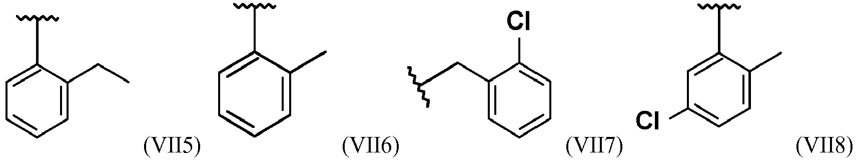

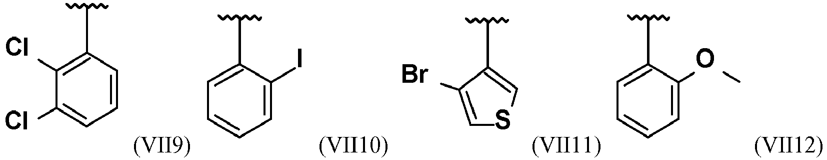

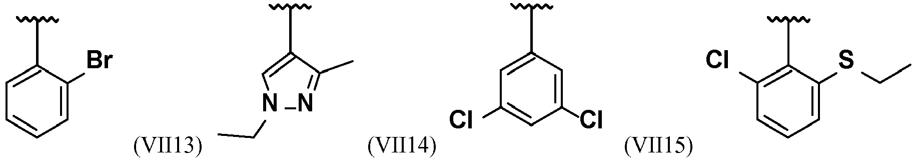

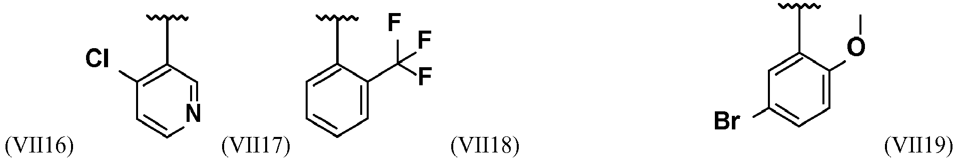

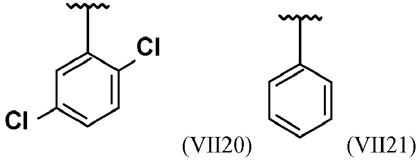

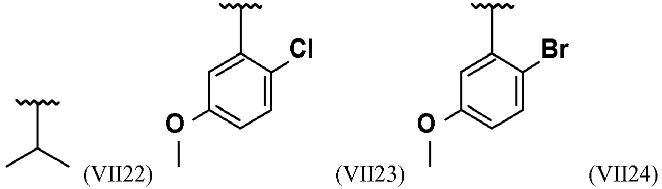

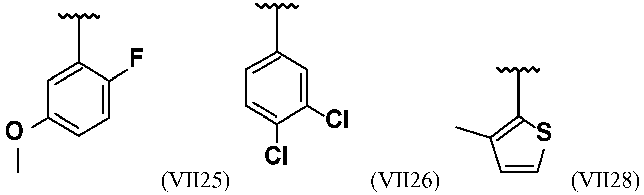

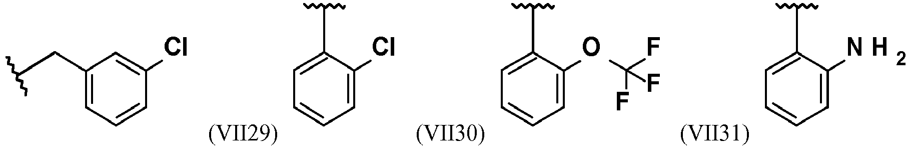

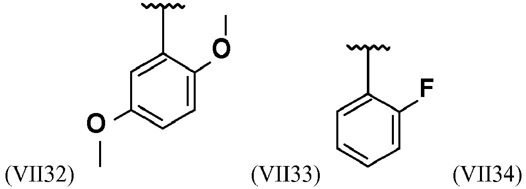

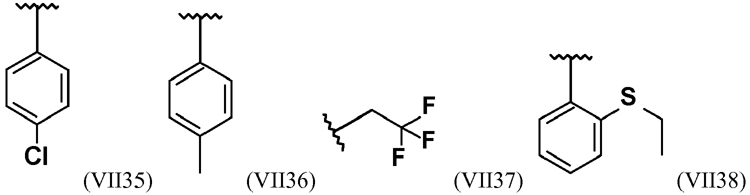

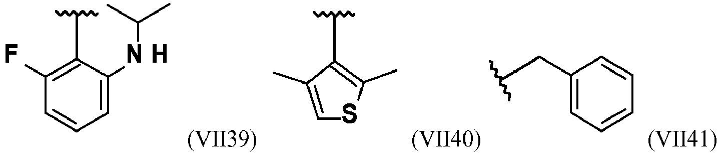

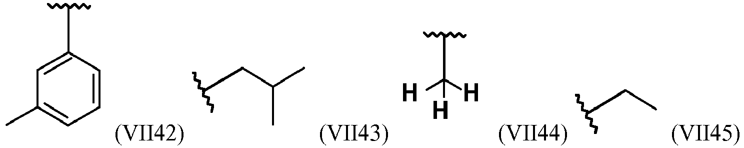

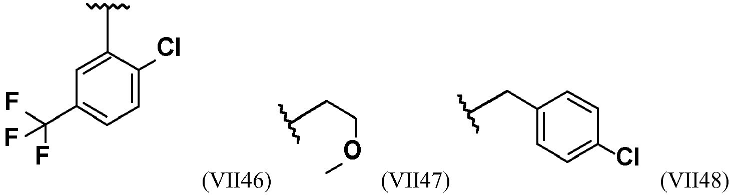

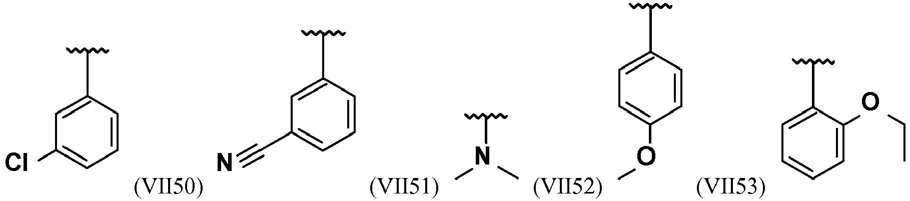

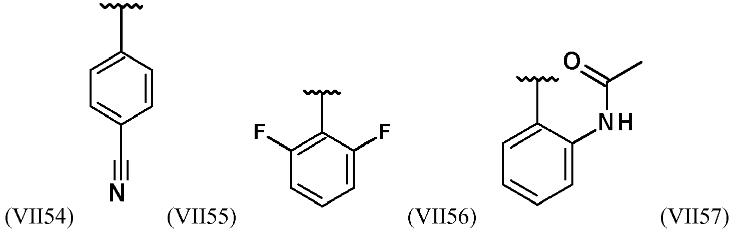

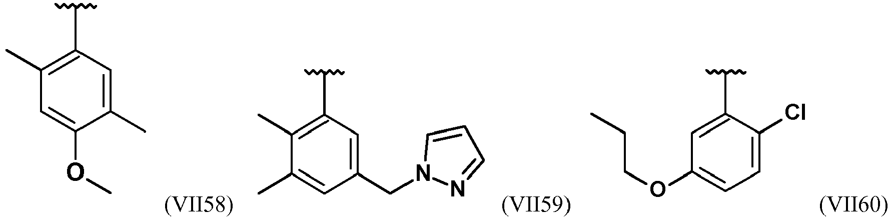

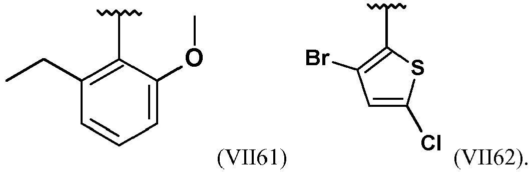

Im Folgenden ist der Ausdruck *Formel (I)* gleichbedeutend mit *Formel (I) oder Formel (VIII),* das heißt alle Ausführungen gelten analog auch für Verbindungen der Formel (VIII).

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit sowohl reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Verfahren zur chirurgischen oder therapeutischen Behandlung des menschlichen oder tierischen Körpers und Diagnostizierverfahren, die am menschlichen oder tierischen Körper vorgenommen werden, sind ausgeschlossen.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Vorzugsweise ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt alle Gebiete, technischen Felder und industriellen Anwendungen ab, bei denen diese Hygienemaßnahmen, -vorschriften und -verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z. B. Geophilus spp., Scutigera spp.;
aus der Ordnung oder der Klasse der Collembola z. B. Onychiurus armatus; Sminthurus viridis;
aus der Klasse der Diplopoda z. B. Blaniulus guttulatus;
aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;
aus der Ordnung der Coleoptera z. B. Acalymma vittatum, Acanthoscelides obtectus, Adoretus spp., Aethina tumida, Agelastica alni, Agriotes spp., z. B. Agriotes linneatus, Agriotes mancus, Alphitobius diaperinus, Amphimallon solstitialis, Anobium punctatum, Anoplophora spp., Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Lissorhoptrus oryzophilus, Listronotus (=Hyperodes) spp., Lixus spp., Luperodes spp., Luperomorpha xanthodera, Lyctus spp., Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;
aus der Ordnung der Dermaptera z. B. Anisolabis maritime, Forficula auricularia, Labidura riparia;
aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;
aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;
aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., Solenopsis invicta, Tapinoma spp., Technomyrmex albipes, Urocerus spp., Vespa spp., z. B. Vespa crabro, Wasmannia auropunctata, Xeris spp.;
aus der Ordnung der Isopoda z. B. Armadillidium vulgare, Oniscus asellus, Porcellio scaber;
aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;
aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;
aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;
aus der Ordnung der Phthiraptera z. B. Damalinia spp., Haematopinus spp., Linognathus spp., Pediculus spp., Phylloxera vastatrix, Phthirus pubis, Trichodectes spp.;
aus der Ordnung der Psocoptera z. B. Lepinotus spp., Liposcelis spp.;
aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;
aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z. B. Scutigerella spp., z. B. Scutigerella immaculata;
Schädlinge aus dem Stamm der Mollusca, z. B. aus der Klasse der Bivalvia, z. B. Dreissena spp.;
sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;
Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus, Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella ornata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanol-alkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einer oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (I) mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamineethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

### Insektizide/Akarizide/Nematizide

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.
(1) Acetylcholinesterase(AChE)-Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder Organophosphate, z. B. Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Blocker, wie beispielsweise Cyclodien-organochlorine, z. B. Chlordan und Endosulfan oder Phenylpyrazole (Fiprole), z. B. Ethiprol und Fipronil.
(3) Natrium-Kanal-Modulatoren, wie beispielsweise Pyrethroide, z. B. Acrinathrin, Allethrin, d-cistrans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin oder DDT oder Methoxychlor.
(4) Kompetitive Modulatoren des nicotinischen Acetylcholin-Rezeptors (nAChR), wie beispielsweise Neonicotinoide, z. B. Acetamiprid, Clothianidin, Dinotefuran, Imidacloprid, Nitenpyram, Thiacloprid und Thiamethoxam oder Nicotin oder Sulfoxaflor oder Flupyradifurone.
(5) Allosterische Modulatoren des nicotinischen Acetylcholin-Rezeptors (nAChR), wie beispielsweise Spinosyne, z. B. Spinetoram und Spinosad.
(6) Allosterische Modulatoren des Glutamat-abhängigen Chloridkanals(GluCl), wie beispielsweise Avermectine/Milbemycine, z. B. Abamectin, Emamectin-benzoat, Lepimectin und Milbemectin.
(7) Juvenilhormon-Mimetika, wie beispielsweise Juvenilhormon-Analoge, z. B. Hydropren, Kinopren und Methopren oder Fenoxycarb oder Pyriproxyfen.
(8) Verschiedene nicht spezifische (multi-site) Inhibitoren, wie beispielsweise Alkylhalogenide, z. B. Methylbromid und andere Alkylhalogenide; oder Chloropicrin oder Sulfurylfluorid oder Borax oder Brechweinstein oder Methylisocyanaterzeuger, z. B. Diazomet und Metam.
(9) Modulatoren chordotonaler Organe, z. B. Pymetrozin oder Flonicamid.
(10) Milbenwachstumsinhibitoren, wie z. B. Clofentezin, Hexythiazox und Diflovidazin oder Etoxazol.
(11) Mikrobielle Disruptoren der Insektendarmmembran, wie z. B. *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und *B.t*.-Pflanzenproteine: Cry1Ab, Cry1Ac, Cry1Fa, CrylA.105, Cry2Ab, VIP3A, mCry3A, Cry3Ab, Cry3Bb, Cry34Ab1/35Ab1.
(12) Inhibitoren der mitochondrialen ATP-Synthase, wie ATP-Disruptoren, wie beispielsweise Diafenthiuron oder Organozinnverbindungen, z. B. Azocyclotin, Cyhexatin und Fenbutatin-oxid oder Propargit oder Tetradifon.
(13) Entkoppler der oxidativen Phoshorylierung durch Störung des Protonengradienten, wie beispielsweise Chlorfenapyr, DNOC und Sulfluramid.
(14) Blocker des nicotinischen Acetylcholinrezeptorkanals, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsdisruptor (insbesondere bei Dipteren, d. h. Zweiflüglern), wie beispielsweise Cyromazin.
(18) Ecdyson-Rezeptor-Agonisten, wie beispielsweise Chromafenozid, Halofenozid, Methoxyfenozid und Tebufenozid.
(19) Oktopamin-Rezeptor-Agonisten, wie beispielsweise Amitraz.
(20) Mitochondriale Komplex-III-Elektronentransportinhibitoren, wie beispielsweise Hydramethylnon oder Acequinocyl oder Fluacrypyrim.
(21) Mitochondriale Komplex-I-Elektronentransportinhibitoren, wie beispielsweise METI-Akarizide, z. B. Fenazaquin, Fenpyroximat, Pyrimidifen, Pyridaben, Tebufenpyrad und Tolfenpyrad oder Rotenon (Derris).
(22) Blocker des spannungsabhängigen Natriumkanals, wie z. B. Indoxacarb oder Metaflumizone.
(23) Inhibitoren der Acetyl-CoA-Carboxylase, wie beispielsweise Tetron- und Tetramsäurederivate, z. B. Spirodiclofen, Spiromesifen und Spirotetramat.
(24) Inhibitoren des mitochondrialen Komplex-IV-Elektronentransports, wie beispielsweise Phosphine, z. B. Aluminiumphosphid, Calciumphosphid, Phosphin und Zinkphosphid oder Cyanide, Calciumcyanid, Kaliumcyanid und Natriumcyanid.
(25) Inhibitoren des mitochondrialen Komplex-II-Elektronentransports, wie beispielsweise beta-Ketonitrilderivate, z. B. Cyenopyrafen und Cyflumetofen und Carboxanilide, wie beispielsweise Pyflubumid.
(28) Ryanodinrezeptor-Modulatoren, wie beispielsweise Diamide, z. B. Chlorantraniliprol, Cyantraniliprol und Flubendiamid,
weitere Wirkstoffe wie beispielsweise Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tioxazafen, Thiofluoximat, Triflumezopyrim und Iodmethan; des Weiteren Präparate auf Basis von *Bacillus firmus* (I-1582, BioNeem, Votivo), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457) (CAS 637360-23-7), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl] -2-(3 -chlor-2-pyridyl)pyrazol-3 -carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-1-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e][1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8) und N-[3-Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9).

### Fungizide

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.
1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-1-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl]-1-(1H-1,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-yl-thiocyanat, (1.040) 1-{[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2-[4-(4-Chlorphenoxy)-2-(trifluormethyl)phenyl]-1-(1H-1,2,4-triazol-1-yl)pentan-2-ol, (1.055) 2-[4-(4-Chlorphenoxy)-2-(trifluormethyl)phenyl]-1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1-{[3-(2-chlorphenyl)-2-(2,4-difluorphenyl)oxiran-2-yl]methyl}-1H-1,2,4-triazol, (1.061) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4-{[3-(2,2,3,3-tetrafluorpropoxy)phenyl]sulfanyl}phenyl)-N-ethyl-N-methylimidoformamid, (1.066) N'-(2,5-Dimethyl-4-{[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068) N'-(2,5-Dimethyl-4- {3-[(2,2,2-trifluorethyl)sulfanyl]phenoxy}phenyl)-N-ethyl-N-methylimidoformamid, (1.069) N'-(2,5-Dimethyl-4- {3-[(2,2,3,3-tetrafluorpropyl)sulfanyl]phenoxy}phenyl)-N-ethyl-N-methylimidoformamid, (1.070) N'-(2,5-Dimethyl-4-{3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4- {[3-(Difluormethoxy)phenyl]sulfanyl} -2,5-dimethylphenyl)-N-ethyl-N-methylimidoformamid, (1.073) N'-(4-{3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-{4-[(4,5-Dichlor-1,3-thiazol-2-yl)oxy]-2,5-dimethylphenyl} -N-ethyl-N-methylimidoformamid, (1.076) N'-{5-Brom-6-[(1R)-1-(3,5-difluorophenyl)ethoxy]-2-methylpyridin-3-yl}-N-ethyl-N-methylimidoformamid, (1.077) N'-{5-Brom-6-[(1S)-1-(3,5-difluorphenyl)ethoxy]-2-methylpyridin-3-yl} -N-ethyl-N-methylimidoformamid, (1.078) N'- {5-Brom-6-[(cis-4-isopropylcyclohexyl)oxy]-2-methylpyridin-3-yl}-N-ethyl-N-methylimidoformamid, (1.079) N'-{5-Brom-6-[(trans-4-isopropylcyclohexyl)oxy]-2-methylpyridin-3-yl}-N-ethyl-N-methylimidoformamid, (1.080) N'-{5-Bromo-6-[1-(3,5-difluorphenyl)ethoxy]-2-methylpyridin-3-yl}-N-ethyl-N-methylimidoformamid.
2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des anti-epimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4-{[4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1H-pyrazol-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-[1-(2,4-Dichlorphenyl)-1 -methoxypropan-2-yl] -3 -(difluormethyl)-1 -methyl-1 H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.
3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{[1-(4-Chlorphenyl)-1H-pyrazol-3-yl]oxy}-2-(methoxyimino)-N,3-dimethylpent-3-enamid, (3.023) (2R)-2-{2-[(2,5-Dimethylphenoxy)methyl]phenyl}-2-methoxy-N-methylacetamid, (3.024) (2S)-2-{2-[(2,5-Dimethylphenoxy)methyl]phenyl}-2-methoxy-N-methylacetamid, (3.025) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl-2-methylpropanoat, (3.026) 2-{2-[(2,5-Dimethylphenoxy)methyl]phenyl}-2-methoxy-N-methylacetamid, (3.027) N-(3-Ethyl-3,5,5-trimethylcyclohexyl)-3-formamido-2-hydroxybenzamid, (3.028) (2E,3Z)-5-{[1-(4-Chlor-2-fluorphenyl)-1H-pyrazol-3-yl]oxy}-2-(methoxyimino)-N,3-dimethylpent-3-enamid.
4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.
5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram.
6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.
7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.
(8) Inhibitoren der ATP-Produktion, beispielsweise (8.001) Silthiofam.
9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Buty|phenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.
10) Inhibitoren der Lipid- und Membran-Synthese, beispielsweise (10.001) Propamocarb, (10.002) Propamocarbhydrochlorid, (10.003) Tolclofos-Methyl.
11) Inhibitoren der Melanin-Biosynthese, beispielsweise (11.001) Tricyclazol, (11.002) 2,2,2-Trifluorethyl-{3-methyl-1-[(4-methylbenzoyl)amino]butan-2-yl}carbamat.
12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).
13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.
14) Verbindungen, die als Entkoppler wirken können, beispielsweise (14.001) Fluazinam, (14.002) Meptyldinocap.
15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarb-fosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-l-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2-{2-Fluor-6-[(8-fluor-2-methylquinolin-3-yl)oxy]phenyl}propan-2-ol, (15.043) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1-yl)quinolin, (15.047) 3-(4,4-Difluor-3,3-dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(quinolin-3-yl)-2,3-dihydro-1,4-benzoxazepin, (15.055) But-3-yn-1-yl{6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl{6-[({[(1-methyl-1H-tetrazol-5-yl)(phenyl)methylene]amino}oxy)methyl]pyridin-2-yl}carbamat.

### Biologische Schädlingsbekämpfungsmittel als Mischungskomponenten

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens*, Stamm FZB42 (DSM 231179), oder *Bacillus cereus,* insbesondere *B. cereus* Stamm CNCM I-1562 oder *Bacillus firmus*, Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans*, *Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana*, insbesondere Stamm ATCC 74040, *Coniothyrium minitans*, insbesondere Stamm CON/M/91-8 (Accession No. DSM-9660), *Lecanicillium spp*., insbesondere Stamm HRO LEC 12, *Lecanicillium lecanii* (ehemals bekannt als *Verticillium lecanii*), insbesondere Stamm KV01, *Metarhizium anisopliae*, insbesondere Stamm F52 (DSM3884/ ATCC 90448), *Metschnikowia fructicola*, insbesondere Stamm NRRL Y-30752, *Paecilomyces fumosoroseus (*neu: *Isaria fumosorosea)*, insbesondere Stamm IFPC 200613, oder Stamm Apopka 97 (Accesion No. ATCC 20874), *Paecilomyces lilacinus*, insbesondere *P. lilacinus* Stamm 251 (AGAL 89/030550), *Talaromyces flavus*, insbesondere Stamm V117b, *Trichoderma atroviride*, insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp*., *Burkholderia spp.,* insbesondere *Burkholderia cepacia* (ehemals bekannt als *Pseudomonas cepacia*), *Gigaspora spp.,* oder *Gigaspora monosporum*, *Glomus spp., Laccaria spp., Lactobacillus buchneri, Paraglomus spp., Pisolithus tinctorus*, *Pseudomonas spp*., *Rhizobium spp*., insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischungskomponenten

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d. h. die Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) getränkt, oder durch die Bodenapplikation, d. h. die erfindungsgemäßen Verbindungen der Formel (I) werden in fester Form (z. B. in Form eines Granulats) in den Standort der Pflanzen eingebracht. Bei Wasserreiskulturen kann das auch durch Zudosieren der Verbindung der Formel (I) in einer festen Anwendungsform (z. B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und einer Mischungskomponente behandelt wird. Es umfasst ferner auch ein Verfahren, in dem das Saatgut zu unterschiedlichen Zeiten mit einer Verbindung der Formel (I) und einer Mischungskomponente behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) behandelt wurde. Die Erfindung bezieht sich auch auf Saatgut, welches zur gleichen Zeit mit einer Verbindung der Formel (I) und einer Mischungskomponente behandelt wurde. Die Erfindung bezieht sich weiterhin auf Saatgut, welches zu unterschiedlichen Zeiten mit einer Verbindung der Formel (I) und einer Mischungskomponente behandelt wurde. Bei Saatgut, welches zu unterschiedlichen Zeiten mit einer Verbindung der Formel (I) und einer Mischungskomponente behandelt wurde, können die einzelnen Substanzen in unterschiedlichen Schichten auf dem Saatgut vorhanden sein. Dabei können die Schichten, die eine Verbindung der Formel (I) und Mischungskomponenten enthalten, gegebenenfalls durch eine Zwischenschicht getrennt sein. Die Erfindung bezieht sich auch auf Saatgut, bei dem eine Verbindung der Formel (I) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating-Verfahren unterzogen wird, um Staubabrieb am Saatgut zu vermeiden.

Einer der auftretenden Vorteile, wenn eine Verbindung der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d. h. dem Gebiet der Tiermedizin, sind die Verbindungen der Formel (I) gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische.

Gemäß einer bestimmten Ausführungsform werden die Verbindungen der Formel (I) an Säugetiere verabreicht.

Gemäß einer weiteren bestimmten Ausführungsform werden die Verbindungen der Formel (I) an Vögel, nämlich Stubenvögel oder insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (I) wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (I) den jeweiligen Parasiten abtöten, sein Wachstum verhindern oder seine Vermehrung verhindern.

Zu den Arthropoden zählen beispielsweise, ohne hierauf beschränkt zu sein,
aus der Ordnung Anoplurida zum Beispiel Haematopinus spp., Linognathus spp., Pediculus spp., Phtirus spp., Solenopotes spp.;
aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;
aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., O-dagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;
aus der Ordnung Siphonapterida, zum Beispiel Ceratophyllus spp., Ctenocephalides spp., Pulex spp., Tunga spp., Xenopsylla spp.;
aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Sternostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:
Metamonada: aus der Ordnung Diplomonadida zum Beispiel Giardia spp., Spironucleus spp.

Parabasala: aus der Ordnung Trichomonadida zum Beispiel Histomonas spp., Pentatrichomonas spp., Tetratrichomonas spp., Trichomonas spp., Tritrichomonas spp.

Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z. B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:
Monogenea: z. B.: Dactylogyrus spp., Gyrodactylus spp., Microbothrium spp., Polystoma spp., Troglecephalus spp.;
Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.

Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.

Nematoden: aus der Ordnung Trichinellida zum Beispiel: Capillaria spp., Trichinella spp., Trichomosoides spp., Trichuris spp.

Aus der Ordnung Tylenchida zum Beispiel: Micronema spp., Parastrangyloides spp., Strongyloides spp.

Aus der Ordnung Rhabditina zum Beispiel: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.

Aus der Ordnung Spirurida zum Beispiel: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acanthocephala: aus der Ordnung Oligacanthorhynchida z.B: Macracanthorhynchus spp., Prosthenorchis spp.; aus der Ordnung Moniliformida zum Beispiel: Moniliformis spp.,
Aus der Ordnung Polymorphida zum Beispiel: Filicollis spp.; aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen.

So bezieht sich eine Ausführungsform der vorliegenden Erfindung auf die Verbindungen der Formel (I) zur Verwendung als Arzneimittel.

Ein weiterer Aspekt bezieht sich auf die Verbindungen der Formel (I) zur Verwendung als Antiendoparasitikum.

Ein weiterer spezieller Aspekt der Erfindung betrifft die Verbindungen der Formel (I) zur Verwendung als Antihelminthikum, insbesondere zur Verwendung als Nematizid, Platymelminthizid, Acanthocephalizid oder Pentastomizid.

Ein weiterer spezieller Aspekt der Erfindung betrifft die Verbindungen der Formel (I) zur Verwendung als Antiprotozoikum.

Ein weiterer Aspekt betrifft die Verbindungen der Formel (I) zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (I) und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (I) mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder herkömmlicherweise in veterinärmedizinischen Formulierungen verwendeten Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe nematizider, platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (I) bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (I) bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (I) mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alanwood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akkarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren;
Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;
Verbindungen aus anderen Klassen, z.B. Butacarb, Dimetilan, Cloethocarb, Phosphocarb, Pirimiphos(-ethyl), Parathion(-ethyl), Methacrifos, Isopropyl-o-salicylat, Trichlorfon, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;
Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;
Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),
Neonicotinoide, z. B. Nithiazin
Dicloromezotiaz, Triflumezopyrim
makrocyclische Lactone, z. B. Nemadectin, Ivermectin, Latidectin, Moxidectin, Selamectin, Eprinomectin, Doramectin, Emamectinbenzoat; Milbemycinoxim
Tripren, Epofenonan, Diofenolan;
Biologicals, Hormone oder Pheromone, zum Beispiel natürliche Produkte, z.B. Thuringiensin, Codlemon oder Neem-Komponenten
Dinitrophenole, z. B. Dinocap, Dinobuton, Binapacryl;
Benzoylharnstoffe, z. B. Fluazuron, Penfluron,
Amidinderivate, z. B. Chlormebuform, Cymiazol, Demiditraz
Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.

Zu den anthelmintischen Wirkstoffen zählen, ohne hierauf beschränkt zu sein, die folgenden nematiziden, trematiziden und/oder cestoziden Wirkstoffe:
aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;
aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;
aus der Klasse der Depsipeptide, vorzugsweise cyclischen Depsipetide, insbesondere 24-gliedrigen cyclischen Depsipeptide, zum Beispiel: Emodepsid, PF1022A;
aus der Klasse der Tetrahydropyrimidine zum Beispiel: Morantel, Pyrantel, Oxantel;
aus der Klasse der Imidazothiazole zum Beispiel: Butamisol, Levamisol, Tetramisol;
aus der Klasse der Aminophenylamidine zum Beispiel: Amidantel, deacyliertes Amidantel (dAMD), Tribendimidin;
aus der Klasse der Aminoacetonitrile zum Beispiel: Monepantel;
aus der Klasse der Paraherquamide zum Beispiel: Paraherquamid, Derquantel;
aus der Klasse der Salicylanilide zum Beispiel: Tribromsalan, Bromoxanid, Brotianid, Clioxanid, Closantel, Niclosamid, Oxyclozanid, Rafoxanid;
aus der Klasse der substituierten Phenole zum Beispiel: Nitroxynil, Bithionol, Disophenol, Hexachlorophen, Niclofolan, Meniclopholan;
aus der Klasse der Organophosphate zum Beispiel: Trichlorfon, Naphthalofos, Dichlorvos/DDVP, Crufomat, Coumaphos, Haloxon;
aus der Klasse der Piperazinone/Chinoline zum Beispiel: Praziquantel, Epsiprantel;
aus der Klasse der Piperazine zum Beispiel: Piperazin, Hydroxyzin;
aus der Klasse der Tetracycline zum Beispiel: Tetracyclin, Chlorotetracyclin, Doxycyclin, Oxytetracyclin, Rolitetracyclin;
aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:
aus der Klasse der Triazine zum Beispiel: Diclazuril, Ponazuril, Letrazuril, Toltrazuril;
aus der Klasse Polyletherionophor zum Beispiel: Monensin, Salinomycin, Maduramicin, Narasin;
aus der Klasse der makrocyclischen Lactone zum Beispiel: Milbemycin, Erythromycin;
aus der Klasse der Chinolone zum Beispiel: Enrofloxacin, Pradofloxacin;
aus der Klasse der Chinine zum Beispiel: Chloroquin;
aus der Klasse der Pyrimidine zum Beispiel: Pyrimethamin;
aus der Klasse der Sulfonamide zum Beispiel: Sulfachinoxalin, Trimethoprim, Sulfaclozin;
aus der Klasse der Thiamine zum Beispiel: Amprolium;
aus der Klasse der Lincosamide zum Beispiel: Clindamycin;
aus der Klasse der Carbanilide zum Beispiel: Imidocarb;
aus der Klasse der Nitrofurane zum Beispiel: Nifurtimox;
aus der Klasse der Chinazolinonalkaloide zum Beispiel: Halofuginon;
aus diversen anderen Klassen zum Beispiel: Oxamniquin, Paromomycin;
aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

### Vektorbekämpfung

Die Verbindungen der Formel (I) können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von anderen Würmern;
   - Aedes: Gelbfieber, Dengue-Fieber, weitere virale Erkrankungen, Filariasis;
   - Simulien: Übertragung von Würmern, insbesondere Onchocerca volvulus;
   - Psychodidae: Übertragung von Leishmaniose
2) Läuse: Hautinfektionen, epidemisches Fleckfieber;
3) Flöhe: Pest, endemisches Fleckfieber, Bandwürmer;
4) Fliegen: Schlafkrankheit (Trypanosomiasis); Cholera, weitere bakterielle Erkrankungen;
5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;
6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) Resistenz-brechend sind.

Verbindungen der Formel (I) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) zur Vektorbekämpfung, z. B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Beispielhaft und ergänzend wird die Herstellung von Verbindungen der Formel (I) in den folgenden Formelschemata erläutert. An dieser Stelle sei auch auf die Herstellungsbeispiele verwiesen.

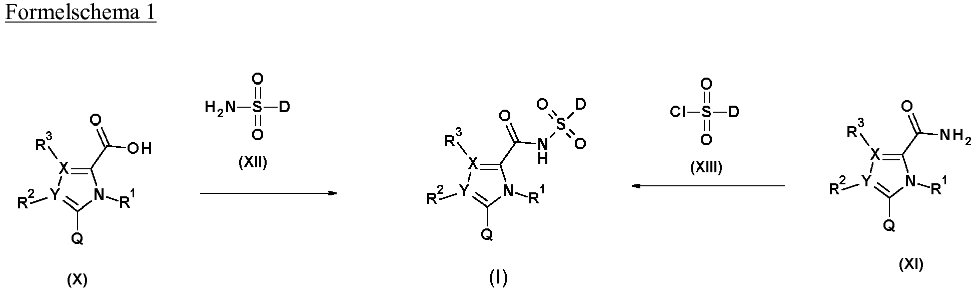

Die Herstellung von erfindungsgemäßen Verbindungen der Formel (I) erfolgt entsprechend Formelschema 1 ausgehend von Carbonsäuren der Formel (X) durch Umsetzung mit einem Kupplungsreagenz und Sulfonamiden der Formel (XII), siehe dazu beispielsweise WO2012/80447, WO2006/114313, WO2015/11082, WO2010/129500, US2008/227769 und WO2009/67108. Alternativ können die Verbindungen der Formel (I) auch durch Umsetzung eines Carbonsäureamids der Formel (XI) mit einem Sulfochlorid der Formel (XIII) in Gegenwart einer Base wie beispielsweise Natriumhydrid hergestellt werden, siehe dazu beispielsweise US2004/6143. Die benötigten Amide der Formel (XI) können aus den Säuren der Formel (X) beispielsweise durch Umsetzung mit einem Kupplungsreagenz und Ammoniumacetat erhalten werden, siehe dazu beispielsweise US5300498.

Die benötigten Sulfonamide und Sulfochloride der Formel (XII) und (XIII) sind bekannt oder lassen sich nach im Allgemeinen bekannten Methoden herstellen. Dabei können die Sulfonamide aus den Sulfochloriden durch Umsetzung mit Ammoniak erhalten werden, siehe dazu WO2014/146490, Eur. J. Med. Chem. 2013, 62, 597-604; Bioorg. Med. Chem. 2005, 13, 7, 2459-2468.

### Weitere Beispiele sind:

3-Chlorbenzolsulfonylamid: Coll. Czech. Chem. Comm. 1984, 49, 5, 1182-1192
2-Chlorbenzolsulfonsäurechlorid: US5099025
2-Chlor-5-Methoxy-benzolsulfonylamid: WO2010/129500
Isopropylsulfonylamid: US542803

Die benötigten Carbonsäuren der Formel (X) sind bekannt oder können in Analogie zu im Allgemeinen bekannten Verfahren oder nach bekannten Verfahren oder entsprechend der im Folgenden beschriebenen Verfahren A bis G hergestellt werden.

Beispiele für bekannte Säuren der Formel (X):
Für 5-Methyl-1-[2-(trifluormethyl)phenyl]-1H-pyrazol-3-carbonsäure siehe Bioorg. Med. Chem. Lett. 2001, Vol 11, #17, 287-2290
Für 2-(2-Chlorphenyl)-5-methyl-1H-imidazol-4-carbonsäure siehe WO2004/60870 A1 Seite 50
Für 2-(2-Chlorphenyl)-1-(4-chlorphenyl)-1H-imidazol-4-carbonsäure siehe Bioorg. Med. Chem. Lett. 2007, Vol 17, #10, 2706-2711

### Verfahren A

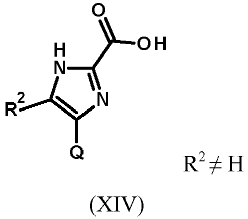

Die Herstellung von Verbindungen der Formel (XIV) erfolgt entsprechend Formelschema 1 ausgehend von Bromiden der Formel (XV) durch Umsetzung mit einer Boronsäure oder einem Boronsäureester der Formel (XVI), einem Palladiumkatalysator wie zum Beispiel Tetrakis(triphenylphosphin)palladium oder (1,1'-bis(diphenylphosphino)ferrocen)palladium Dichlorid und einer Base wie beispielsweise Kaliumcarbonat, in einem hochsiedenden Lösemittel wie zum Beispiel DMF. Siehe dazu beispielsweise WO2011/149874 A2 oder EP2518054 A1.

In Formelschema 1 kann R^{x} zum Beispiel H oder Alkyl (auch cyclisch) sein. R^{y} kann beispielsweise Methyl, Ethyl, Propyl oder tert-Butyl sein.

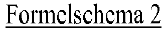

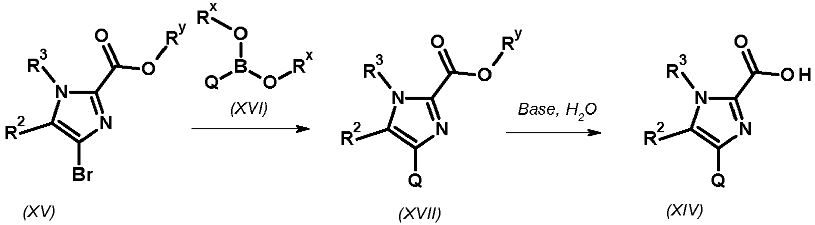

Die Hydrolyse des Esters zur Zielverbindung XIV erfolgt gemäß allgemein bekannter Bedingungen (LiOH, H₂O, THF oder NaOH, EtOH). Im Falle von R^{y} = tert-Butyl wird der Ester unter sauren Bedingungen hydrolysiert, beispielsweise mit Trifluoressigsäure in Dichlormethan.

Boronsäuren bzw. Boronsäureester der Formel (XVI) sind entweder kommerziell erhältlich oder können nach bekannten Methoden hergestellt werden.

Die Herstellung der benötigten Bromide der Formel XV erfolgt gemäß Schema 2. Das Ausgangsmaterial der allgemeinen Formel XVIII ist entweder kommerziell erhältlich oder kann nach bekannten Methoden hergestellt werden.

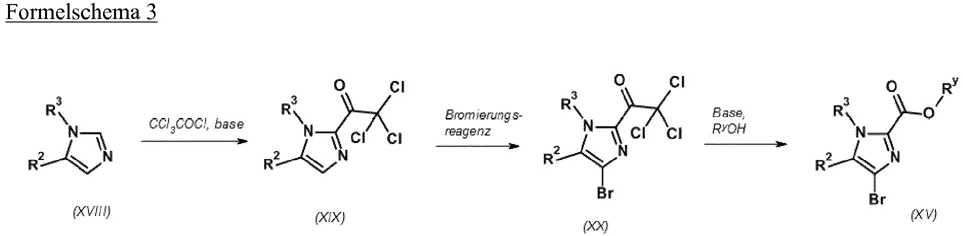

Durch Umsetzung eines Imidazols XVIII mit Trichloracetylchlorid wie in Bioorganic and Medicinal Chemistry Letters, 2008, 18, 4325 oder EP2518054 A1 beschrieben, wird Zwischenverbindung (XIX) erhalten. Diese wird wie beispielsweise in WO2008/85302 A1 oder WO2015/25025 A1 publiziert, durch Reaktion mit einem Bromierungsreagenz (N-Bromsuccinimid, Br₂/AcOH o.a.) bromiert. Die Zielverbindung (XV) wird durch Alkoholyse in Gegenwart einer Base erhalten (siehe z.B. WO2007/45096 A1, Chemistry - A European Journal, 2003, 9, 3353).

### Verfahren B

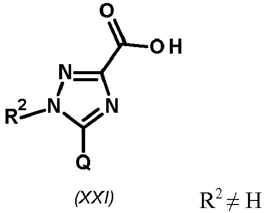

Die Herstellung von Verbindungen der Formel (XXI) erfolgt entsprechend Formelschema 4 wie allgemein in Tetrahedron Letters, 2012, 53, 6078 beschrieben. In Formelschema 4 kann R^{y} beispielsweise Methyl, Ethyl, Propyl oder tert-Butyl sein.

Das Ausgangsmaterial der Formel (XXII) ist entweder kommerziell erhältlich oder kann nach bekannten Methoden hergestellt werden.

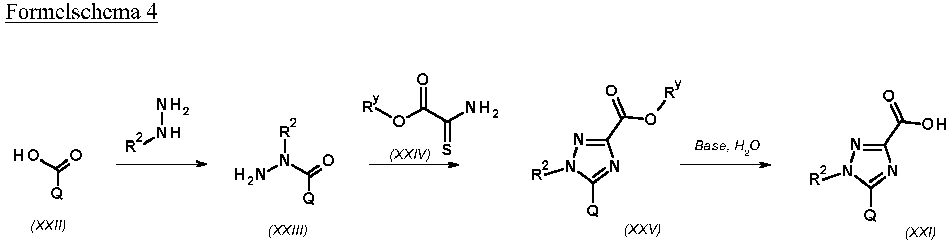

Durch Umsetzung der Säure XXII mit einem Alkylhydrazin und einem geeigneten Kupplungsreagenz/Katalysator-System (z.B. 2,3,4,5,6-Pentafluorophenol / 1-Ethyl-(3-(3-dimethylamino)propyl)-carbodiimid hydrochlorid) wird Zwischenstufe (XXIII) erhalten, welche mit Ethyl-amino(thioxo)acetat in Gegenwart von Essigsäure in Toluol cyclisiert wird.

Die Hydrolyse des Esters zur Zielverbindung XXI erfolgt gemäß allgemein bekannter Bedingungen (LiOH, H₂O, THF oder NaOH, EtOH). Im Falle von R^{y} = tert-Butyl wird der Ester unter sauren Bedingungen hydrolysiert, beispielsweise mit Trifluoressigsäure in Dichlormethan.

### Verfahren C:

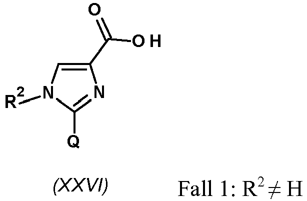

Die Herstellung von Verbindungen der Formel (XXVI) erfolgt entsprechend Formelschema 5. In Formelschema 5 kann R^{x} zum Beispiel H oder Alkyl (auch cyclisch) sein. R^{y} kann beispielsweise Methyl, Ethyl, Propyl oder tert-Butyl sein.

Die als Ausgangsmaterial benötigten Ester XXVII sind entweder kommerziell erhältlich oder können durch Veresterung der entsprechenden Säure nach bekannten Methoden hergestellt werden.

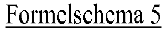

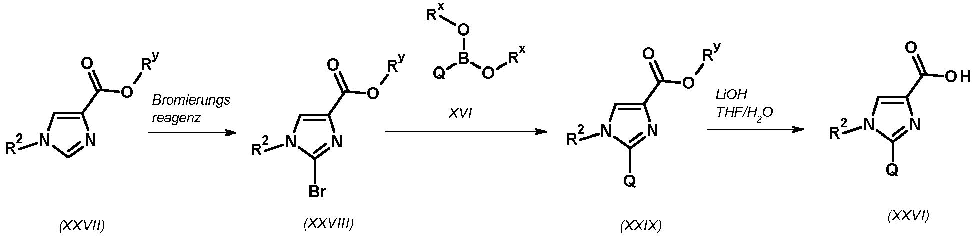

Die Bromierung wird Mit Hilfe eines Bromierungsreagenzes wie beispielsweise N-Bromsuccinimid analog WO2014/191894 A1, 2014 durchgeführt.

Bromide der Formel (XXVIII) können anschließend durch Umsetzung mit einer Boronsäure oder einem Boronsäureester der Formel (XVI), einem Palladiumkatalysator wie zum Beispiel Tetrakis(triphenylphosphin)palladium oder Chloro(2-dicyclohexylphosphino-2',4',6'-triisopropyl-1,1'-biphenyl)[2-(2'-amino-1,1'-biphenyl)]palladium(II) (XPhos Pd G2) und einer Base wie beispielsweise Kaliumphosphat, in einem Lösemittel wie zum Beispiel 1,4-Dioxan oder THF, unter Rückfluss in Verbindungen der Formel XXIX überführt werden. Siehe dazu beispielsweise WO2014/115077 A1, 2014 oder J. Am. Chem. Soc., 2010, 132, 14073.

Boronsäuren bzw. Boronsäureester der Formel (XVI) sind entweder kommerziell erhältlich oder können nach bekannten Methoden hergestellt werden.

Die Hydrolyse des Esters zur Zielverbindung XXVI erfolgt gemäß allgemein bekannter Bedingungen (LiOH, H₂O, THF oder NaOH, EtOH). Im Falle von R^{y} = tert-Butyl wird der Ester unter sauren Bedingungen hydrolysiert, beispielsweise mit Trifluoressigsäure in Dichlormethan.

### Verfahren D

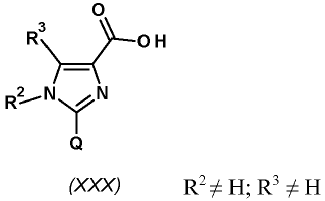

Die Herstellung von erfindungsgemäßen Verbindungen der Formel (XXX) erfolgt entsprechend Formelschema 6 ausgehend von Bromiden der Formel (XXXI) durch Umsetzung mit einer kommerziell erhältlichen oder unter bekannten Bedingungen herstellbaren Boronsäure oder einem Boronsäureester der Formel (XVI), einem Palladiumkatalysator wie zum Beispiel [1,1'-bis(diphenylphosphino)-ferrocen]palladium(II)chlorid Dichlormethan Komplex und einer Base wie beispielsweise Cäsiumcarbonat in einem hochsiedenden Lösemittel wie zum Beispiel Toluol unter Rückfluss. Siehe dazu beispielsweise Journal of Organic Chemistry, 2004, 69, 8829. In Formelschema 6 kann R^{x} zum Beispiel H oder Alkyl (auch cyclisch) sein. R^{y} kann beispielsweise Methyl, Ethyl, Propyl oder tert-Butyl sein.

Die Hydrolyse des Esters zur Zielverbindung XXX erfolgt gemäß allgemein bekannter Bedingungen (LiOH, H₂O, THF oder NaOH, EtOH). Im Falle von R^{y} = tert-Butyl wird der Ester unter sauren Bedingungen hydrolysiert, beispielsweise mit Trifluoressigsäure in Dichlormethan.

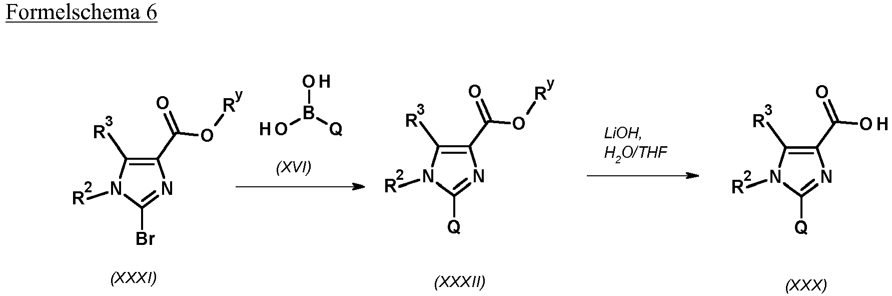

Boronsäuren der Formel (XVI) sind entweder kommerziell erhältlich oder können nach bekannten Methoden hergestellt werden.

Der benötigte bromierte Baustein XXXI wird gemäß Schema 7 hergestellt.

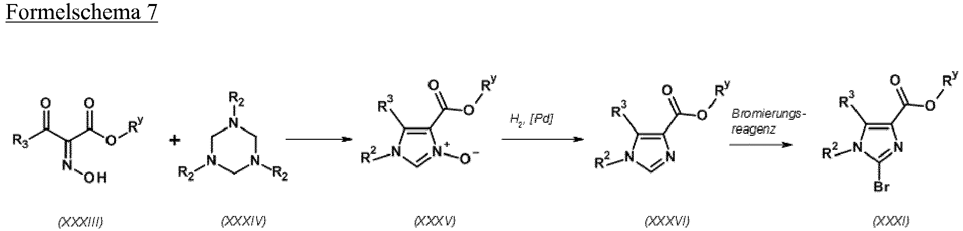

Die Cyclisierung eines 1,3,5-Trialkyl-1,3,5-triazinans XXXIV mit einem 2-(hydroxyimino)-3-oxocarbonsäureester XXXIII ist beispielsweise in Helvetica Chimica Acta, 2008, 91, 1916 beschrieben. Die nachfolgende Reduktion kann durch Zugabe eines geeigneten heterogenen Katalysators wie beispielsweise Raney Nickel in einem geeigneten Lösemittel wie beispielsweise Ethanol erfolgen. Bevorzugt wird die Reaktion bei Raumtemperatur unter erhöhtem Druck (beispielsweise 2 bar) durchgeführt. Analoge Reaktionen sind zum Beispiel in Tetrahedron Asymmetry, 2013, 24, 958 beschrieben.

Das Imidazol der Formel XXXVI kann beispielsweise analog EP2518054 A1, 2012 durch Bromierung mit N-Bromsuccinimid in Acetonitril zum benötigten Baustein XXXI umgesetzt werden.

### Verfahren E

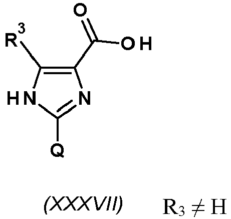

Die Herstellung von erfindungsgemäßen Verbindungen der Formel (XXXVII) erfolgt entsprechend Formelschema 8. In Formelschema 8 kann R^{y} beispielsweise Methyl, Ethyl, Propyl oder tert-Butyl sein.

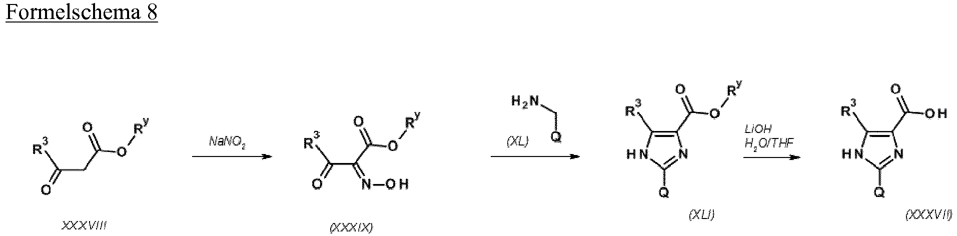

Gemäß WO2011/119777 A2, 2011 oder US2012/238599 A1, 2012 wird ein kommerziell erhältlicher oder nach allgemein bekannten Bedingungen herstellbarer α-Ketoster XXXVIII durch Umsetzung mit Natriumnitrit und Essigsäure in Wasser in ein Oxim der allgemeinen Formel XXXIX umgesetzt. Dieses kann durch Erhitzen mit einem Amin in einem geeigneten Lösemittel (beispielsweise Acetonitril oder Toluol) analog WO2005/99705 A2, 2005 oder US6288061 B1, 2001 zu einem Imidazol der Formel XLI cyclisiert werden.

Die Hydrolyse des Esters zur Zielverbindung XXXVII erfolgt gemäß allgemein bekannter Bedingungen (LiOH, H₂O, THF oder NaOH, EtOH). Im Falle von R^{y} = tert-Butyl wird der Ester unter sauren Bedingungen hydrolysiert, beispielsweise mit Trifluoressigsäure in Dichlormethan. **Verfahren F:**

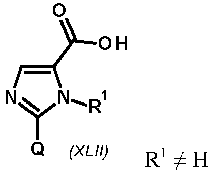

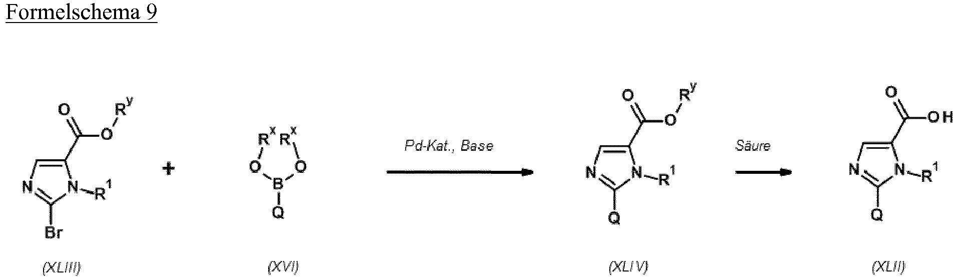

Die Herstellung von erfindungsgemäßen Verbindungen der Formel (XLII) erfolgt ausgehend von 2-Halogenimidazolen der Formel (XLIII) durch Umsetzung mit einer Boronsäure oder Boronsäureester der Formel (XVI). In Formelschema 9 kann R^{x} zum Beispiel H oder Alkyl (auch cyclisch) sein. R^{y} kann beispielsweise Methyl, Ethyl, Propyl oder tert-Butyl sein.

Beispielsweise erhält man durch Umsetzung eines Bromides der Formel (XLIII) mit einem Boronsäureester der Formel (XVI) in Gegenwart eines Palladium-Katalysators wie zum Beispiel Tetrakis(triphenylphosphin)palladium oder [1,1'-Bis(diphenylphosphino)ferrocen]dichlorpalladium und einer Hilfsbase wie Kalium- oder Caesiumcarbonat die Verbindungen der Formel (XLIV). Siehe beispielsweise für 2-Brom-3-methyl-3H-imidazol-4-carbonsäuremethylester: US2009/23707 A1. Aus diesen Estern der Formel (XLIV) kann im Falle, dass R^{y} = tButy, durch Umsetzung mit einer Säure wie zum Beispiel Trifluoressigsäure die Verbindungen der Formel (XLII) freigesetzt werden. Siehe beispielsweise für tert-Butyl 2-(2-chlorphenyl)-5-methyl-1-(4-nitrophenyl)-1H-imidazol-4-carboxylat: WO2005/99705 A2.

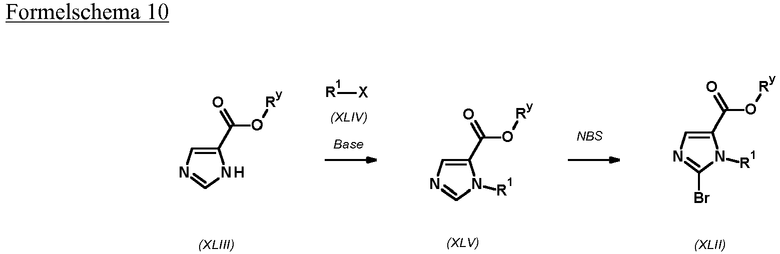

Die Herstellung von erfindungsgemäßen Verbindungen der Formel (XLII) erfolgt ausgehend von Imidazolen der Formel (XLIII) durch Umsetzung mit Halogeniden der Formel (XLIV) und einer starken oder schwachen Base wie zum Beispiel Natriumhydrid oder Kaliumcarbonat. Siehe beispielsweise für Imidazol-5-carbonsaeure-ethylester: Green Chemistry 2013, 15, 2740-2746. Imidazole der Formel (XLV) können mit N-Bromsuccinimid in Verbindungen der Formel (XLII) überführt werden. Siehe beispielsweise für tert-Butyl-1-methyl-1H-imidazol-5-carboxylat: Journal of Chemical Research - Part S, 2000, 5, 230-231.

### Verfahren G

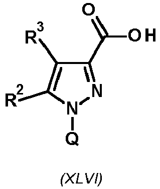

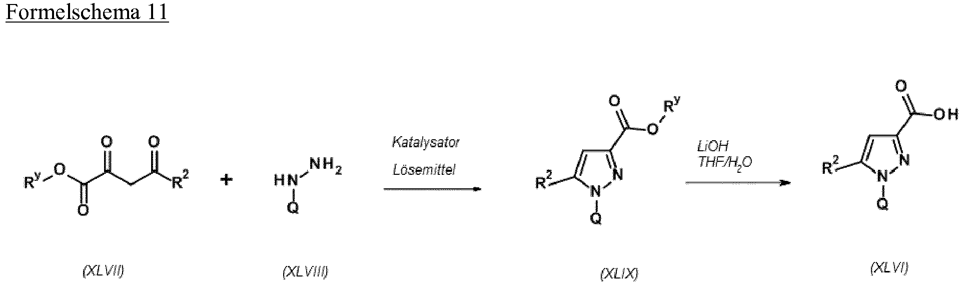

Die Herstellung von erfindungsgemäßen Verbindungen der Formel (XLVI) erfolgt ausgehend von einem entsprechenden Arylhydrazin XLVII und einem Diketon XLVII. Die Cyclisierung kann basen- oder säurekatalysiert erfolgen wie beispielsweise in US2007/287734 A1, 2007 (Base: Natriumhydroxid in Ethanol) oder US6020357 A1, 2000 (Säure: para-Toluolsulfonsäure, in Ethanol) beschrieben.

Die Hydrolyse des Esters zur Zielverbindung XLVI erfolgt gemäß allgemein bekannter Bedingungen (LiOH, H₂O, THF oder NaOH, EtOH).

Die Herstellung von denjenigen erfindungsgemäßen Verbindungen der Formel (XLVI) mit R²= Alkoxy erfolgt ausgehend der Ester (XLIXb), die gemäß Formelschema 11a erhalten werden. Dazu wird ein Arylhydrazin der Formel (XLVIII) mit einem Diazodicarbonsäureester umgesetzt, siehe dazu beispielsweise US2004/248881 Seite 25-26. Der erhaltene Ester (XLIXa) wird dann mit einem Alkylierungsmittel wie Methyliodid in Gegenwart einer Base wie Kaliumcarbonat zum Ester der Formel (XLIXb) alkyliert; siehe dazu beispielsweise US2014/315934 §0919, der dann entsprechend dem oben beschriebenen Weg weiter umgesetzt werden kann.

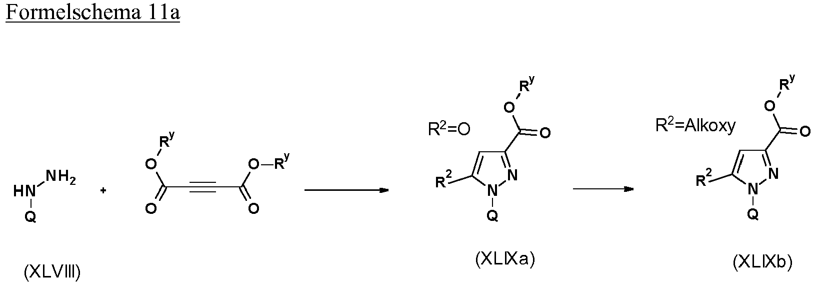

### Verfahren H

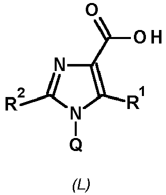

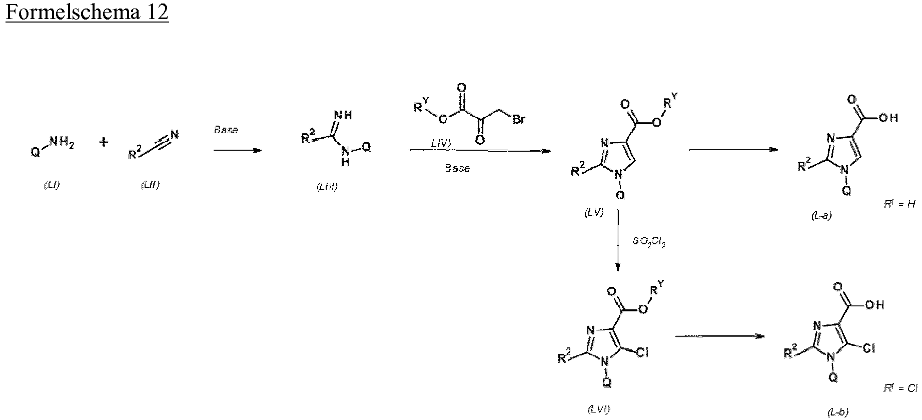

Die Herstellung von erfindungsgemäßen Verbindungen der Formel (L) erfolgt ausgehend von einem entsprechenden Nitril LII und einem Amin LI, die unter Zuhilfenahme einer Base wie beispielsweise NaHMDS zu einem Amidin LIII verknüpft werden. Siehe hierzu z.B. Journal of Medicinal Chemistry, 2005, (48), 1823. Das Amidin kann zum Beispiel basenkatalysiert (z.B. mit NaHCO3) in einem polar-protischen Lösemittel wie Isopropanol mit einem α-Bromoketon LIV zum Imidazol LV cyclisiert werden. Siehe hierzu US2004/122074 A1, 2004.

Das erhaltene Imidazol des Typs LV kann entweder direkt basisch zu L-a verseift werden (wie bereits oben mehrfach beschrieben), oder es wird durch Umsetzung mit SO₂Cl₂ oder Oxalylchlorid zuvor zu einer Vorstufe des Typs LVI chloriert, die ihrerseits zu L-b verseift werden kann. Zur Chlorierung siehe beispielsweise WO2005/99705 A2, 2005 oder EP2196459 A1, 2010.

### Verfahren I

Handelt es sich bei Q um ein Pyridinderivat wie zum Beispiel im Fall Q = (VIz), (VIa1), (VIa6), (VIa17) so erfolgt die Herstellung von erfindungsgemäßen Verbindungen der Formel (XLII, R¹ ≠ H) entsprechend Formelschema 13 und Formelschema 14 nach zwei möglichen Verfahren.

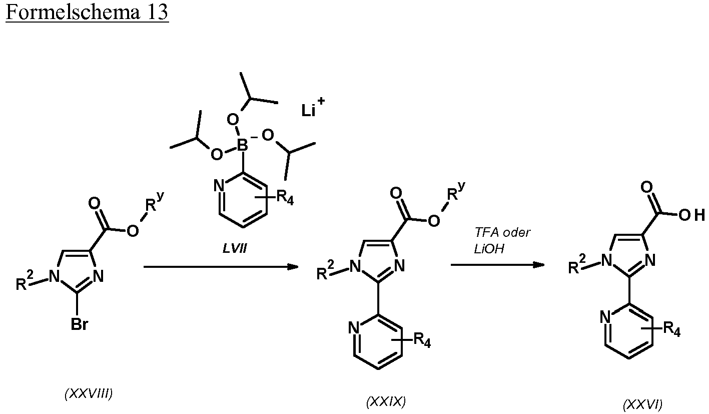

Verbindungen der Formel (XXVIII) können ähnlich Verfahren C statt mit einer Boronsäure mit einem Triisopropoxyborat der Formel (LVII) in Gegenwart eines Palladiumkatalysators (bestehend aus Palladiumsalz und gegebenenfalls einem weiteren Liganden), eines Kupfersalzes und einer Base zu Reaktion gebracht werden. Ein geeignetes Reaktionssystem ist beispielsweise die Kombination aus Palladiumacetat, 1,1'-Bis(diphenylphosphino)ferrocen, Kupfer(I)iodid und Cäsiumcarbonat wie in Org. Lett., 2009, (11), 345 beschrieben. Die Reaktion kann in einem unter den Reaktionsbedingungen inerten Lösemittel, wie zum Beispiel Dimethylformamid, erfolgen. Die Reaktion erfolgt üblicherweise in einem Temperaturbereich von 50 - 150°C.

Die allgemeine Herstellung von Triisopropoxyboraten der Formel (LVII) ist in Tet. Lett. 2012 (53), 4873 oder WO2011103435A2 beschrieben.

Die Hydrolyse des Esters zur Zielverbindung XXVI erfolgt gemäß allgemein bekannter Bedingungen (LiOH, Wasser/THF oder NaOH, EtOH). Im Falle von Ry = tert-Butyl wird der Ester unter sauren Bedingungen hydrolysiert, beispielsweise mit Trifluoressigsäure in Dichlormethan.

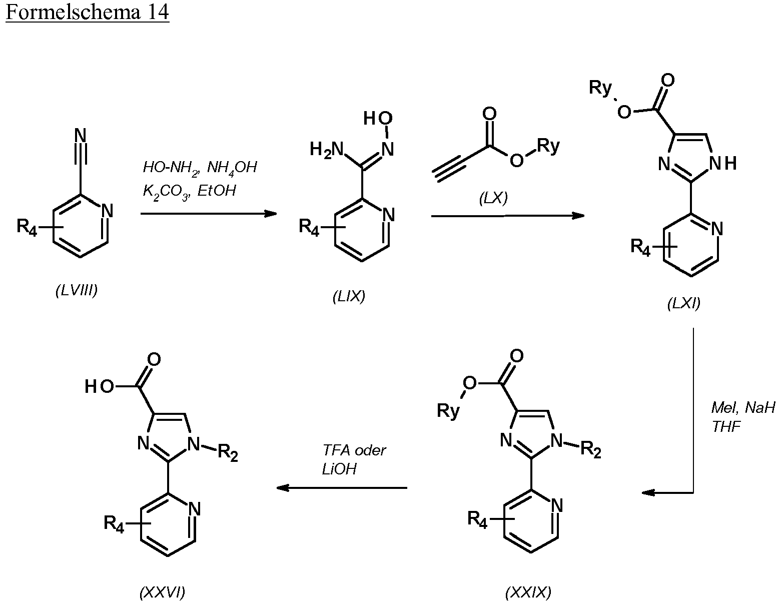

Alternativ zu dem oben beschriebenen Verfahren lassen sich Verbindungen der Formel (XLII, R¹ ≠ H) ausgehend von Nitrilen der Formel (LVIII) herstellen.

Im ersten Schritt wird gemäß Bioorganic and Medicinal Chemistry Letters, 2012, vol. 22, # 22 p. 6974 - 6979,6 oder WO2007/75749 A2, 2007 Verbindung (LVIII) durch Umsetzung mit Hydroxylamin in Gegenwart einer Base zum Amidoxim (LIX) überführt. Geeignete Basen sind beispielseweise K2CO3 oder NaOH. Die Reaktion kann in einem Lösemittel wie beispielsweise Ethanol, Methanol oder Wasser durchgeführt werden.

In einem zweiten Schritt erfolgt die Cyclisierung von (LIX) mit einem Propiolat (LX) zum Imidazol (LXI), siehe hierzu beispielsweise US4853383 A1, 1989, US6492516 B1, 2002. Die Reaktion erfolgt üblicherweise bei 50-200°C. Geeignete Lösemittel sind Alkohole wie Methanol oder hochsiedende Lösemittel wie Diphenylether.

Im dritten Schritt wird aus Verbindung (LXI) mit Hilfe eines Alkylierungsreagenzes und in Gegenwart einer Base Imidazol (XXIX) hergestellt. Geeignete Alkylierungsmittel sind beispielsweise Alkylhalogenide wie Methyliodid. Als Basen eignen sich Natriumhydrid, Kaliumcarbonat, Kaliumterbutanolat. Siehe hierzu WO2008/84218 A1, 2008, WO2007/113276 A1, 2007 WO2016/46230 A1, 2016. Die Reaktion kann in einem unter den Reaktionsbedingungen inerten Lösemittel wie beispielsweise THF erfolgen.

Die Hydrolyse des Esters (XXIX) zur Zielverbindung (XXVI) erfolgt gemäß allgemein bekannter Bedingungen (LiOH, Wasser/THF oder NaOH, EtOH). Im Falle von Ry = tert-Butyl wird der Ester unter sauren Bedingungen hydrolysiert, beispielsweise mit Trifluoressigsäure in Dichlormethan.

### Herstellungsbeispiele

Die folgenden Herstellungs- und Verwendungsbeispiele illustrieren die Erfindung, ohne sie zu beschränken.

### Methoden

Die Bestimmung der logP-Werte erfolgte gemäß EEC Directive 79/831 Annex V.A8 durch HPLC (High Performance Liquid Chromatography) an einer Phasenumkehrsäule (C18). Temperatur 43 °C. Die Eichung erfolgt mit unverzweigten Alkan-2-onen (mit 3 bis 16 Kohlenstoffatomen), deren logP Werte bekannt sind.

Die Bestimmung des M⁺ mit der LC-MS im sauren Bereich erfolgte bei pH 2,7 mit 0,1 % wässriger Ameisensäure und Acetonitril (enthält 0,1% Ameisensäure) als Eluenten; linearer Gradient von 10% Acetonitril bis 95% Acetonitril, Gerät: Agilent 1100 LC-System, Agilent MSD System, HTS PAL.

Die Bestimmung des M⁺ mit der LC-MS im neutralen Bereich erfolgte bei pH 7.8 mit 0,001 molarer wässriger Ammoniumhydrogencarbonat-Lösung und Acetonitril als Eluenten; linearer Gradient von 10 % Acetonitril bis 95 % Acetonitril.

In den Tabellen und Herstellungsbeispielen wurden die logP Werte für den sauren Bereich (als logP [a]) und/oder für den neutralen Bereich (als logP [n]) angegeben.

b) Die Bestimmung der ¹H-NMR-Daten erfolgte mit einem Bruker Avance 400 ausgestattet mit einem Durchflussprobenkopf (60 µl Volumen), mit Tetramethylsilan als Referenz (0.0) und den Lösungsmitteln CD₃CN, CDCl₃ oder D₆-DMSO oder mit einem Bruker Avance III HD 300MHz Digital NMR mit einem 5mm Probenkopf.

Die NMR-Daten ausgewählter Beispiele werden entweder in klassischer Form (δ-Werte, Multiplettaufspaltung, Anzahl der H-Atome) oder als NMR-Peak-Listen aufgeführt.

### NMR-Peak-Listenverfahren

Die ¹H-NMR-Daten ausgewählter Beispiele werden in Form von ¹H-NMR-Peaklisten notiert. Zu jedem Signalpeak wird erst der δ -Wert in ppm und dann die Signalintensität in runden Klammern aufgeführt. Die δ -Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensitätᵢ);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

### Herstellungsbeispiel zu Verfahren C:

### Herstellung von tert-Butyl-1-methyl-1H-imidazol-4-carboxylat

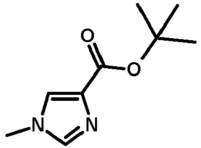

Unter einer Argon-Atmosphäre wurden 1-Methyl-1H-Imidazol-carbonsäure (100.0 g, 792 mmol) in 1.1 L Dichlormethan gelöst und es wurden 0.5 ml DMF zugegeben. Bei Raumtemperatur wurde Oxalylchlorid (120.8 g, 951 mmol) zugegeben und es wurde für 12 h bei Raumtemperatur gerührt. Das Lösemittel wurde entfernt und der Rückstand wurde in 795 ml THF gelöst und auf -30°C gekühlt. Langsam wurde zu dem Gemisch Lithium-tert-Butoxid-Lösung (2.2 M in THF, 896.4 ml, 1972 mmol) getropft. Nach Abschluss der Addition wurde das Reaktionsgemisch langsam auf Raumtemperatur erwärmt. Es wurde für 12 h bei Raumtemperatur gerührt und dann gesättigte NaHCO₃-Lösung zugegeben. Das Zwei-Phasengemisch wurde für 1 h gerührt, dann wurde das Lösemittel weitgehend am Rotationsverdampfer entfernt. Der Rückstand wurde mit Ethylacetat aufgenommen und die organische Phase wurde mit Wasser gewaschen. Die wässrige Phase wurde mit Ethylacetat extrahiert. Die vereinigten organischen Extrakte wurden mit gesättigter NaCl-Lösung gewaschen, über Na₂SO₄ getrocknet und das Lösemittel wurde am Rotationsverdampfer entfernt. Das Rohprodukt (86.7 g, 72%) wurde in der folgenden Reaktion ohne weitere Aufreinigung umgesetzt.
logP (sauer): 0.63; MH+: 183; 1H-NMR (400MHz, D6-DMSO) δ ppm: 7.71 (s, 1H), 7.64 (s, 1H), 3.67 (s, 3H), 1.49 (s, 9H)

### Herstellung von tert-Butyl-2-brom-1-methyl-1H-imidazol-4-carboxylat

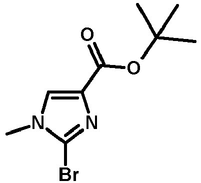

Unter Argon-Atmosphäre wurden tert-Butyl-1-methyl-1H-imidazol-4-carboxylat (64.0 g, 351 mmol, 1 eq.) und 1,2-Dibrom-1,1,2,2-tetrachlorethan (114.4 g, 351 mmol, 1 eq.) in 1200 ml THF gelöst, und es wurde bei -10 - 0°C portionsweise Lithium-tert-butoxid (84.3 g, 1.05 mmol, 3 eq.) zugegeben. Das Reaktionsgemisch wurde für 12 h bei Raumtemperatur gerührt. Nach entfernen des Lösemittels wurde der Rückstand in Ethylacetat aufgenommen. Die organische Lösung wurde mit Wasser gewaschen, über Na₂SO₄ getrocknet und am Rotationsverdampfer vom Lösemittel befreit. Das Rohprodukt wurde chromatographisch aufgereinigt (Laufmittel: Cyclohexan/Ethylacetat). Es wurden 45.8 g (50%) des gewünschten Produktes erhalten.
logP (sauer): 2.41; MH+: 261; 1H-NMR (400MHz, D6-DMSO) δ ppm: 7.94 (s, 1H), 3.63 (s, 3H), 1.49 (s, 9H).

### Herstellung von tert-Butyl-2-(2,6-diftuorphenyl)-1-methyl-1H-imidazol-4-carboxylat

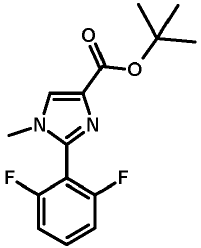

Zu einer Lösung von tert-Butyl-2-brom-1-methyl-1H-imidazol-4-carboxylat (100 mg, 0,38 mmol) in THF (15 mL) wurde eine Lösung von Kaliumphosphat (152 mg, 0,71 mmol) in Wasser (2,5 mL), Xphos Pd G2 Katalysator (15 mg, 0,01 mmol) und 2-(2,6-Difluorphenyl)-4,4,5,5-tetramethyl-1,3,2-dioxaborolan (276 mg, 1,14 mmol) gegeben. Das Reaktionsgemisch wurde 6 h bei 80 °C gerührt. Anschließend wurde eine zweite Portion 2-(2,6-Difluorphenyl)-4,4,5,5-tetramethyl-1,3,2-dioxaborolan (183 mg) zugegeben und das Gemisch weitere 15 h bei 65 °C gerührt. Die Reaktionsmischung wurde mit Wasser (50 mL) verdünnt und mit Ethylacetat (3 x 50 mL) extrahiert. Die vereinte organische Phase wurde über Na₂SO₄ getrocknet und das Lösungsmittel im Vakuum entfernt. Aufreinigung des Rohproduktes mittels HPLC lieferte das gewünschte Produkt (58,1 mg, 52%).
logP (neutral): 2,3; MH+: 295,1; 1H-NMR (400MHz, D6-DMSO) δ ppm: 8.00 (s, 1H), 7.67 (m, 1H), 7.32 (m, 2H), 3.54 (s, 3H), 1.51 (s, 9H).

### Herstellung von 2-(2,6-Difluorphenyl)-1-methyl-1H-imidazol-4-carbonsäure

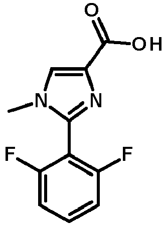

Tert-Butyl-2-(2,6-difluorphenyl)-1-methyl-1H-imidazol-4-carboxylat (58,0 mg, 0,19 mmol) wurde in Dichlormethan (0,5 mL) vorgelegt und Trifluoressigsäure (0,11 mL) zugegeben. Das Reaktionsgemisch wurde 15 h bei Raumtemperatur gerührt. Das Lösungsmittel wurde im Vakuum entfernt und der Rückstand mit Dichlormethan codestilliert.
logP (neutral): 2,3; MH+: 239,1; 1H-NMR (400MHz, D6-DMSO) δ ppm: 8.01 (s, 1H), 7.70 (m, 1H), 7.33 (m, 2H), 3.57 (s, 3H).

### Herstellungsbeispiel Verfahren D

### Herstellung von Ethyl-1,5-dimethyl-1H-imidazol-4-carboxylat

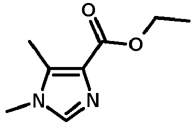

Ethyl-1,5-dimethyl-1H-imidazol-4-carboxylat-3-oxid (19.0 g, 103.1 mmol, 1 eq.; Herstellung analog European Journal of Organic Chemistry (2011), (13), 2542-2547,S2542/1-S2542/8. Helvetica Chimica Acta (2011), 94(10), 1764-1777. Helvetica Chimica Acta (2008), 91(10), 1916-1933) wurde in 93 ml Ethanol gelöst. Es wurde Raney-Nickel (Ra-Ni 400, 4 g) zugegeben und unter 5 bar WasserstoffAtmosphäre für 1 h bei Raumtemperatur gerührt. Nach Entfernen des Katalysators durch Filtration über Kieselgel wurde die Lösung eingeengt und das Rohprodukt (17.8 g, 91%) ohne weitere Aufreinigung in der nächsten Reaktion eingesetzt.
logP (neutral): 0.85; MH+: 169;

### Herstellung von Ethyl-2-brom-1,5-dimethyl-1H-imidazol-4-carboxylat

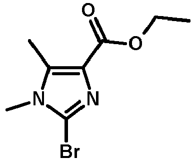

Ethyl-1,5-dimethyl-1H-imidazol-4-carboxylat (1.00 g; 5.49 mmol) und N-Bromsuccinimid (0.98 g; 5.49 mmol) wurde in 100 ml Acetonitril gelöst und für 4 h bei Raumtemperatur gerührt. Das Lösemittel wurde am Rotationsverdampfer entfernt und der Rückstand wurde in Ethylacetat aufgenommen. Die organische Phase wurde mit NaHCO3-Lösung und NaCl-Lösung gewaschen, über MgSO4 getrocknet und vom Lösemittel befreit. Der Rückstand wurde chromatographisch aufgereinigt. Es wurden 616 mg (43%) des gewünschten Produktes erhalten.
logP (neutral): 1.69; MH+: 263;

### Herstellung von Ethyl-2-(2,6-difluorphenyl)-1,5-dimethyl-1H-imidazol-4-carboxylat

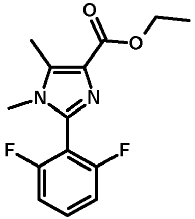

Ethyl-2-(2,6-difluorphenyl)-1,5-dimethyl-1H-imidazol-4-carboxylat kann in Analogie zu tert-Butyl-2-(2,6-difluorphenyl)-1 -methyl-1 H-imidazol-4-carboxylat hergestellt werden.
logP (neutral): 2,0; MH+: 281,1; 1H-NMR (400MHz, D6-DMSO) δ ppm: 7.68 (m, 1H), 7.32 (m, 2H), 4.22 (m, 2H), 3.41 (s, 3H), 2.49 (s, 3H), 1.28 (m, 3H).

### Herstellung von 2-(2,6-Difluorphenyl)-1,5-dimethyl-1H-imidazol-4-carbonsäure

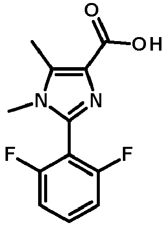

Eine Lösung von Ethyl-2-(2,6-difluorphenyl)-1,5-dimethyl-1H-imidazol-4-carboxylat (779 mg, 2.77 mmol) und Lithiumhydroxid (79.9 mg, 3.33 mmol) in THF/Wasser (3:1, 15 mL) wurde 15 h bei 60 °C gerührt. Das Lösungsmittel wurde im Vakuum entfernt.
logP (neutral): 2,0; MH+: 253,1; 1H-NMR (400MHz, D6-DMSO) δ ppm: 8.32 (s, 1H), 7.63 (m, 1H), 7.29 (m, 2H), 3.45 (s, 3H), 2.46 (s, 3H).

### Herstellungsbeispiel Verfahren E

### Herstellung von Ethyl-2-(2,6-difluorphenyl)-5-ethyl-1H-imidazol-4-carboxylat

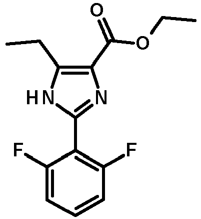

Ethyl-(2Z)-2-(hydroxyimino)-3-oxopentanoat (4.33 g, 25 mmol, 1 eq.: Herstellung siehe Chemical & Pharmaceutical Bulletin, 2013, 61 (12), 1248) und 2,6-Difluobenzylamin (3.94 g, 27.5 mmol, 1.1 eq.) wurden in 30 ml Acetonitril gelöst und für 12 h zum Rückfluss erhitzt. Das Reaktionsgemisch wurde auf konzentriert und der Rückstand chromatographisch aufgereinigt. Es wurden 1.82 g (24%) des gewünschten Produktes erhalten.
logP (sauer): 1.73; MH+: 281; 1H-NMR (400MHz, D6-DMSO) δ ppm: 13.10 (2 s, 1H), 7.58 (m, 1H), 7.26 (m, 2H), 4.26 (m, 2H), 2.98 (m, 2H), 1.29 (m, 3H), 1.21 (m, 3H).

### Herstellung von 2-(2,6-Difluorphenyl)-5-ethyl-1H-imidazol-4-carbonsäure

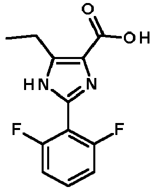

Analog der Herstellung von 2-(2,6-Difluorphenyl)-1,5-dimethyl-1H-imidazol-4-carbonsäure (xx) wurde Ethyl-2-(2,6-difluorphenyl)-5-ethyl-1H-imidazol-4-carboxylat (1.7 g, 6.07 mmol) mit Natronlauge in EtOH zur freien Säure 2-(2,6-Difluorphenyl)-5-ethyl-1H-imidazol-4-carbonsäure (1.3 g, 68%) verseift.
logP (neutral): 0.45; MH+: 253;

### Herstellungsbeispiel Verfahren F:

### Herstellung von tert-Butyl-1-methyl-1H-imidazol-5-carboxylat

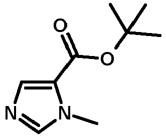

1-Methyl-1H-imidazol-5-carbonsäure (18.0 g; 142.7 mmol; 1 eq.) wurde in 106 g tert-Butanol gelöst und es wurden Pyridin (79.0 g, 999 mmol, 7 eq.) und Tosylchlorid (54.4 g, 285.4 mmol, 2 eq.) zugegeben. Das Reaktionsgemisch wurde für 12 h bei Raumtemperatur gerührt und dann aufkonzentriert. Der Rückstand wurde in Dichlormethan aufgenommen und mit NaHCO₃-Lösung versetzt bis ein pH-Wert von 8-9 erreicht wurde. Die wässrige Phase wurde mehrfach mit Dichlormethan extrahiert und die vereinigten organischen Extrakte wurden über MgSO₄ getrocknet. Nach Entfernen des Lösemittels wurde der Rückstand chromatographisch aufgereinigt (Laufmittel: Cyclohexan/Ethylacetat). Es wurden 14.7 g (56%) des gewünschten Produktes erhalten.
logP (neutral): 1.71; MH+: 183; 1H-NMR (400MHz, D6-DMSO) δ ppm: 7.84 (s, 1H), 7.51 (s, 1H), 3.80 (s, 3H), 1.51 (s, 9H).

### Herstellung von tert-Butyl-2-brom-1-methyl-1H-imidazol-5-carboxylat

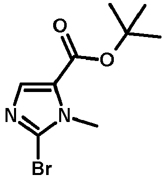

In einem ausgeheizten Schlenkgefäß wurde unter Argonatmosphäre tert-Butyl-1-methyl-1H-imidazol-5-carboxylat (1.00 g, 5.38 mmol, 1 eq.) in 30 ml trockenem THF gelöst. Die Lösung wurde auf -90° gekühlt. Über 20 min wurden 1.05 eq. n-Butyllithium (in Hexan) zugetropft. Nach Rühren für 0.5 h wurde 1,2-Dibrom-1,1,2,2-tetrachlorethan (1.75 g, 5.38 mmol, 1 eq.; gelöst in 15 ml THF) über einen Zeitraum von 20 min zugetropft. Es wurde für 2.5 h bei -80°C gerührt, dann wurde über 30 min auf Raumtemperatur erwärmt. Nach Zugabe von Kieselgur wurde das Gemisch aufkonzentriert und mehrfach chromatographisch aufgereinigt (Laufmittel: Dichlormethan/Methanol). Es wurden 438 mg (30%) des gewünschten Produktes erhalten
logP (neutral): 2.54; MH+: 263; 1H-NMR (400MHz, D6-DMSO) δ ppm: 7.56 (m, 1H), 3.79 (m, 3H), 1.52 (s, 9H).

### Herstellung von tert-Butyl-2-(2,6-difluorphenyl)-1-methyl-1H-imidazol-5-carboxylat

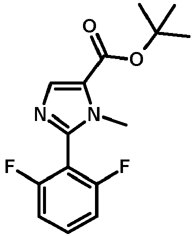

Tert-Butyl-2-(2,6-difluorphenyl)-1-methyl-1H-imidazol-5-carboxylat kann in Analogie zu tert-Butyl-2-(2,6-difluorphenyl)-1 -methyl-1 H-imidazol-4-carboxylat hergestellt werden.
logP (neutral): 3,0; MH+: 295,1; 1H-NMR (400MHz, D6-DMSO) δ ppm: 7.74 (s, 1H), 7.71 (m, 1H), 7.34 (m, 2H), 3.66 (s, 3H), 1.55 (s, 9H).

### Herstellung von 2-(2,6-Difluorphenyl)-1-methyl-1H-imidazol-5-carbonsäure

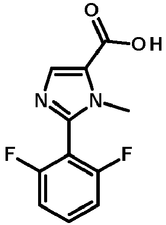

2-(2,6-Difluorphenyl)-1-methyl-1H-imidazol-5-carbonsäure kann in Analogie zu 2-(2,6-Difluorphenyl)-1-methyl-1H-imidazol-4-carbonsäure hergestellt werden.
logP (sauer): 0,46; MH+: 239,1; 1H-NMR (400MHz, D6-DMSO) δ ppm: 7.83 (s, 1H), 7.72 (m, 1H), 7.35 (m, 2H), 3.70 (s, 3H).

### Herstellung von Ethyl-1-(2-chlorphenyl)-5-hydroxy-1H-pyrazol-3-carboxylat

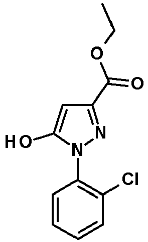

16,9g (94,3mmol) 2-Chlorphenylhydrazin-Hydrochlorid wurden mit 16,15g (95mmol) Acetylendicarbonsäurediethylester in ca 250 ml absolutem Ethanol mit 26,5g (191mmol) Kalimcarbonat über Nacht unter Rückfluß erhitzt. Nach Abdampfen des Lösungsmittel wurde mit Wasser versetzt, durch Sand/Celite abgesaugt, mit wäßriger Zitronensäure bis pH=4 versetzt, 1h gerührt, abgesaugt und der Rückstand getrocknet. Ausbeute 20,68g .
logP (sauer): 1,75; MH+: 267,1; 1H-NMR (400MHz, D6-DMSO) δ ppm: 1,3 (t, 3H), 4,25 (q, 2H), 5,9 (s, 1H), 7,5-7,6 (m, 3H), 7,7 (m, 1H)

### Herstellung von Ethyl-1-(2-chlorphenyl)-5-methoxy-1H-pyrazol-3-carboxylat

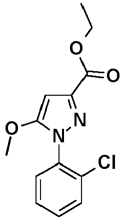

4,75g (17,8mmol) Ethyl-1-(2-chlorphenyl)-5-hydroxy-1H-pyrazol-3-carboxylat wurden in ca 200 ml Aceton sukzessive in 2 Chargen mit insgesamt 16,5 g (120,5mmol) Methyliodid und 22,5g (162mmol) Kaliumcarbonat gerührt. Man saugte ab, dampfte das Lösungsmittel ab, löste den Rückstand in wäßrigem Natrimchlorid, Ethylacetat, Na-EDTA-Puffer bei pH=9, extrahierte zweimal mit Ethylacetat, trocknete die vereinigten organischen Phasen mit Natriumsulfat und dampfte das Lösungsmittel ab. Der Rückstand wurde durch Chromatographie an Kieselgel (Petrolether/Aceton) gereinigt.
Ausbeute 2,2g.
logP (sauer): 2,61; MH+: 281,1; 1H-NMR (400MHz, D6-DMSO) δ ppm: 1,3 (t, 3H), 3,9 (s, 3H), 4,3 (q, 2H), 6,3 (s, 1H), 7,5-7,6 (m, 3H), 7,7 (m, 1H)

### Herstellung von 1-(2-Chlorphenyl)-5-methoxy-1H-pyrazol-3-carbonsäure

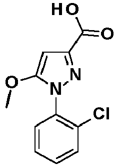

2,1g (7,5mmol) Ethyl-1-(2-chlorphenyl)-5-methoxy-1H-pyrazol-3-carboxylat wurden in Ethanol/Wasser mit Natronlauge versetzt und über Nacht bei 70°C gerührt. Das Lösungsmittel wurde abgedampft, der Rückstand mit Wasser versetzt, bei Eisbadkühlung mit verdünnter Salzsäure versetzt, über Nacht gerührt, abgesaugt, mit Wasser gewaschen und getrocknet.
Ausbeute 2g
logP (sauer): 1,63; MH+: 253,0; 1H-NMR (400MHz, D6-DMSO) δ ppm: 3,9 (s, 3H), 6,3 (s, 1H), 7,5-7,6 (m, 3H), 7,7 (m, 1H), 13 (s, 1H)

### Herstellung von Lithium (3-chloropyridin-2-yl)[tris(propan-2-olato)]borat(1-)

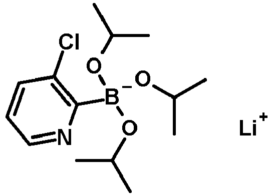

Unter Argon wurde 2-Brom-3-chlorpyridin (50.0 g, 260 mmol) in trockenem THF (200 ml) und trockenem Toluol (800 ml) gelöst und auf -78°C gekühlt. Langsam wurde eine 2.5 M Lösung n-BuLi in Hexan (100 mL, 250 mmol) zugetropft. Das Reaktionsgemisch wurde 2 h bei -78°C gerührt , dann wurde Triisopropyl borat (244 g, 1300 mmol) zugegeben. Nach weiteren 2 h bei -78°C wurde das Gemisch auf Raumtemperatur erwärmt. Es wurde Isopropanol (100 ml) zugegeben und die resultierende Lösung wurde bei 40°C aufkonzentriert. Zu dem Rückstand wurde Aceton (300 ml) addiert und es wurde für 30 min gerührt. Der ausgefallene Feststoff wurde durch Filtration isoliert, mit Aceton(2 x 100 ml) gewaschen und getrocknet.
1H-NMR (400MHz, D2O) δ ppm: 8.30 (b-s, 1H), 7.80 (b-s, 1H), 7.30 (b-s, 1H), 3.85 (b-s, 3H), 1.00 (b-s, 18H).

### Herstellung von tert-Butyl-2-(3-chlorpyridin-2-yl)-1-methyl-1H-imidazol-4-carboxylat

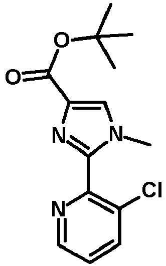

Unter Argon wurden Lithium (3-chloropyridin-2-yl)[tris(propan-2-olato)]borat(1-) (487 mg, 1.50 mmol, 1 eq), tert-Butyl-2-brom-1-methyl-1H-imidazol-4-carboxylat (196 mg, 0.75 mmol, 0.5 eq), Kupfer(I)chlorid (74 mg, 0.75 mmol, 0.5 eq.), Cäsiumcarbonat (677 mg, 3 mmol, 2 eq.), 1,1'-Bis(diphenylphosphino)ferrocen (42 mg, 0.07 mmol, 0.05 eq.), in DMF (15 ml) gelöst. Die Reaktionslösung wurde einige Minuten mit Argon gespült, dann wurde Palladiumacetat (8 mg, 0.03 mmol, 0.025 eq) zugegeben. Das Reaktionsgemisch wurde für 12 h auf 100°C erhitzt. Nach Abkühlen wurde das Lösemittel abdestilliert und der Rückstand in Ethylacetat aufgenommen. Die organische Phase wurde zuerst mit gesättigter Ammoniumchloridlösung, dann mit Wasser gewaschen, über Natriumsulfat getrocknet und am Rotationsverdampfer vom Lösemittel befreit. Der Rückstand würde chromatographisch aufgereinigt (SiO₂, Laufmittel: Cyclohexan/Ethylacetat 1:1).
MH+: 294; 1H-NMR (400MHz, D6-DMSO) δ ppm: 8.68 - 8.67 (m, 1H); 8.16 - 8.14 (m, 1H), 7.95 (s, 1H), 7.61 - 7.58 (m, 1H), 3.64 (s, 3H), 1.52 (s, 9H).

### Herstellung von 2-(3-chloropyridin-2-yl)-1-methyl-1H-imidazole-4-carboxylic acid

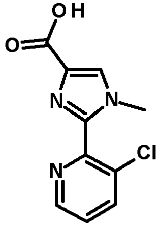

tert-Butyl-2-(3-chlorpyridin-2-yl)-1-methyl-1H-imidazol-4-carboxylat (1.53 g, 5.07 mmol, 1 eq.) wurde in Dichlormethan (15 ml) gelöst und mit Trifluoressigsäure (5.8 g, 50.7 mmol, 10 eq.) versetzt. Die Reaktionslösung wurde für 48 h bei Raumtemperatur gerührt, dann wurde das Lösemittel entfernt und der Rückstand chormatographisch aufgereinigt (SiO₂, Laufmittel: Ethylacetat).
MH+: 238; 1H-NMR (400MHz, D6-DMSO) δ ppm: DMSO 8.68 - 8.67 (m, 1H), 8.16 - 8.14 (m, 1H), 8.01 (s, 1H), 7.60 - 7.57 (m, 1H), 3.66 (s, 3H).

### Herstellung von 2-(3-Brompyridin-2-yl)-1-methyl-1H-imidazol-4-carbonsäure

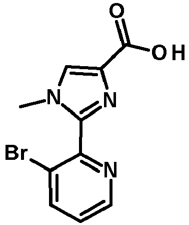

Ethyl-2-(3-brompyridin-2-yl)-1-methyl-1H-imidazol-4-carboxylat (2.11 g, 6.8 mmol, 1 eq.) und Lithiumhydroxid (0.244 g, 10.2 mmol, 1.5 eq.) wurden in einem Gemisch von THF (31 ml) und Wasser (31 ml) gelöst. Die Reaktionsmischung wurde über Nacht zum Rückfluss erhitzt. Nach Aufkonzentrieren wurde verdünnte Salzsäure zugegeben, bis ein pH-wert von 4 erreicht wurde und für 1 h bei Raumtemperatur gerührt. Der entstehende Niederschlag wurde durch Filtration isoliert, mit wenig Wasser gewaschen und getrocknet.
MH+: 284; 1H-NMR (400MHz, D6-DMSO) δ ppm: 8.71 - 8.70 (m, 1H), 8.30 - 8.28 (m, 1H), 8.00 (s, 1H), 7.52 - 7.48 (m, 1H), 3.05 (s, 3H).

### Herstellung von Ethyl-2-(3-brompyridin-2-yl)-1-methyl-1H-imidazol-4-carboxylat

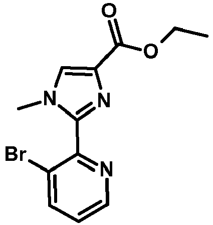

Unter Argon-Atmosphäre wurde Ethyl-2-(3-brompyridin-2-yl)-1H-imidazol-4-carboxylat (3.25 g, 10.3 mmol, 1 eq.) in trockenem THF (50 ml) gelöst. Die Lösung wurde auf -5°C gekühlt und es wurde Natriumhydrid (0.289 g, 11.4 mmol, 1.1 eq.) zugegeben. Nach 30 min Rühren wurde bei -5°C Iodmethan (1.61 g, 11.4 mmol, 1.1 eq.) gelöst in THF (10 ml) zu getropft und weitere 3 h bei 0°C gerührt. Das Gemisch wurde auf Raumtemperatur erwärmt und weitere 12 h gerührt. Nach Zugabe von verdünnter Salzsäure wurde die Lösung auf konzentriert. Der Rückstand wurde in Wasser aufgenommen und mit Triethylamin versetzt. Die wässrige Phase wurde mit Ethylacetat extrahiert. Die vereinigten organischen Extrakte wurden über Natriumsulfat getrocknet und vom Lösemittel befreit. Der Rückstand wurde chromatographisch aufgereinigt (SiO₂, Laufmittel Cyclohexan Essigester).
MH+: 312; 1H-NMR (400MHz, D6-DMSO) δ ppm: 8.72 - 8.70 (m, 1H), 8.31 - 8.29 (m, 1H), 8.07 (s, 1H), 7.53 - 7.50 (m, 1H), 4.27-4.22 (q, 2H), 3.62 (s, 3H), 1.30 - 1.27 (t, 3H).

### Herstellung von Ethyl-2-(3-brompyridin-2-yl)-1H-imidazol-4-carboxylat

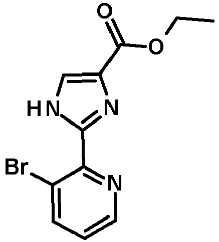

3-Brom-N'-hydroxypyridin-2-carboximidamid (9.81 g, 45.4 mmol, 1 eq.) und Ethylpropiolat (4.46 g, 45.4 mmol, 1 eq.) wurden in Ethanol (44 ml) gelöst und für 12 h zum Rückfluß erhitzt. Ethanol wurde abdestilliert, der Rückstand mit Toluol versetzt und das Lösemittel ein weiteres Mal abdestilliert. Der Rückstand wurde in Diphenylether (27 ml) aufgenommen und für 2.5 h auf 195°C erhitzt. Nach Abkühlen auf 70°C wurde Hexan (200 ml) zugegeben und das Gemisch 3 h gerührt. Die Lösung wurde abdekantiert, der erhaltene Feststoff wurde chromatographisch gereinigt (reversed phase; Lösemittel: Acetonitril, Wasser). Die überstehende Hexanphase wurde aufkonzentriert und ebenfalls chromatographisch gereinigt (SiO₂, Laufmittel: Cyclohexan Essigester; Anschließend Reversed Phase, Laufmittel Acetonitril/Wasser).
MH+: 298; 1H-NMR (400MHz, D6-DMSO) δ ppm: 8.68 - 8.66 (m, 1H), 8.26 - 8.24 (m, 1H), 7.93 - 7.92 (m, 1H), 7.42 - 7.39 (m, 1H), 4.29 - 4.24 (q, 2H), 1.31 - 1.27 (t, 3H).

### Herstellung von 3-Brom-N'-hydroxypyridin-2-carboximidamid

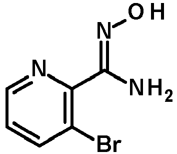

3-Brompyridin-2-carbonitril (13.2 g, 72.1 mmol, 1 eq.), Hydroxylammoniumchlorid (11.0 g, 158 mmol, 2.2 eq.) und Kaliumcarbonat (21.9 g, 158 mmol, 2.2 eq.) wurden in Ethanol (100 ml) gelöst und für 12 h zum Rückfluß erhitzt. Das Reaktionsgemisch wurde aufkonzentriert und in Wasser gelöst. Durch Zugabe von verdünnter Salzsäure wurde ein pH von 4 eingestellt und 1 h gerührt. Der entstehende Feststoff wurde durch Filtration isoliert, gewaschen und getrocknet.
MH+: 217; 1H-NMR (400MHz, D6-DMSO) δ ppm: 9.72 (s, 1H), 8.58 - 8.57 (s, 1H), 8.14 - 8.12 (s, 1H), 7.38 - 7.35 (m, 1H), 5.81 (s, 2H).

### Herstellungsbeispiel zur Herstellung eines Sulfonylamides

### Herstellung von 5-Chlor-2-(2-chlorphenyl)-1-methyl-N-[(2-methylphenyl)sulfonyl]-1H-imidazol-4-carboxamid

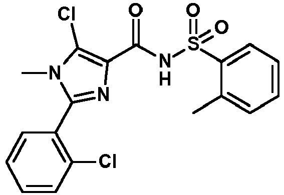

5-Chlor-2-(2-chlorphenyl)-1-methyl-1H-imidazol-4-carbonsäure (163 mg; 0.60 mmol; 1 eq.) wurde in 10 ml Dichlormethan gelöst. Nach Zugabe von 1-Ethyl-3-(3-dimethylaminopropyl)carbodiimid hydrochlorid (345 mg, 1.8 mmol, 3 eq.) und DMAP (220 mg, 1.8 mmol, 3 eq.) wurde das Gemisch für 1 h bei Raumtemperatur gerührt. Dann wurde 2-Methylbenzolsulfonamid (103 mg; 0.6 mmol, 1 eq.) zugegeben und anschließend für 48 h bei Raumtemperatur gerührt. Das Lösemittel wurde am Rotationsverdampfer entfernt und der Rückstand chromatographisch aufgereinigt (reversed Phase; Laufmittel: Acetonitril, H₂O). Es wurden 134 mg (52%) des gewünschten Produktes erhalten.
logP (sauer): 3.17; MH+: 424; 1H-NMR (400MHz, D6-DMSO) δ ppm: 8.04 (d, 1H); 7.65 - 7.52 (m, 5H), 7.46 - 7.38 (m, 2H), 3.41 (s, 3H); 2.61 (s, 3H).

Tabelle 1 führt weitere Verbindungen der Formel (I) auf, die analog zu den oben aufgeführten Beispielen hergestellt wurden. Die Synthese der Säurevorstufen erfolgte entweder wie oben beschrieben, oder die Säuren waren kommerziell erhältlich.

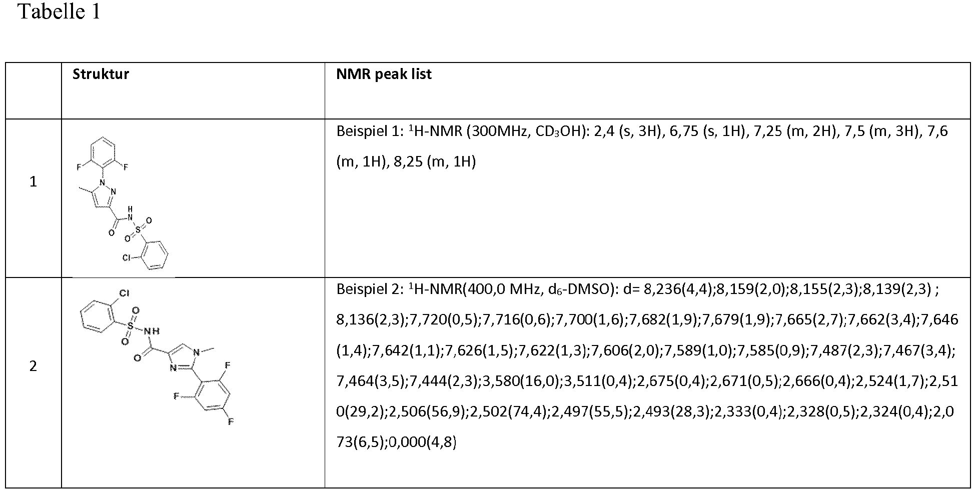

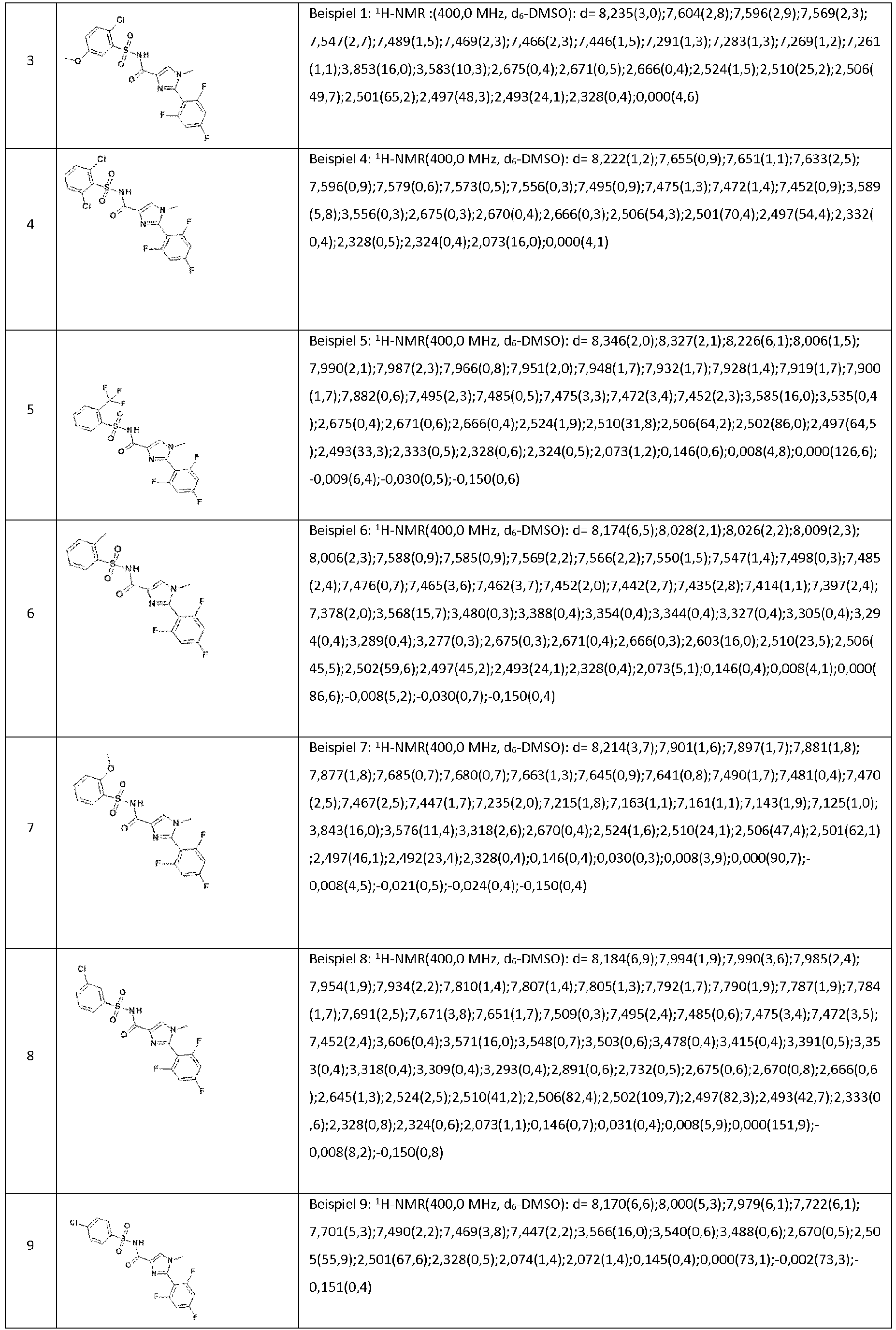

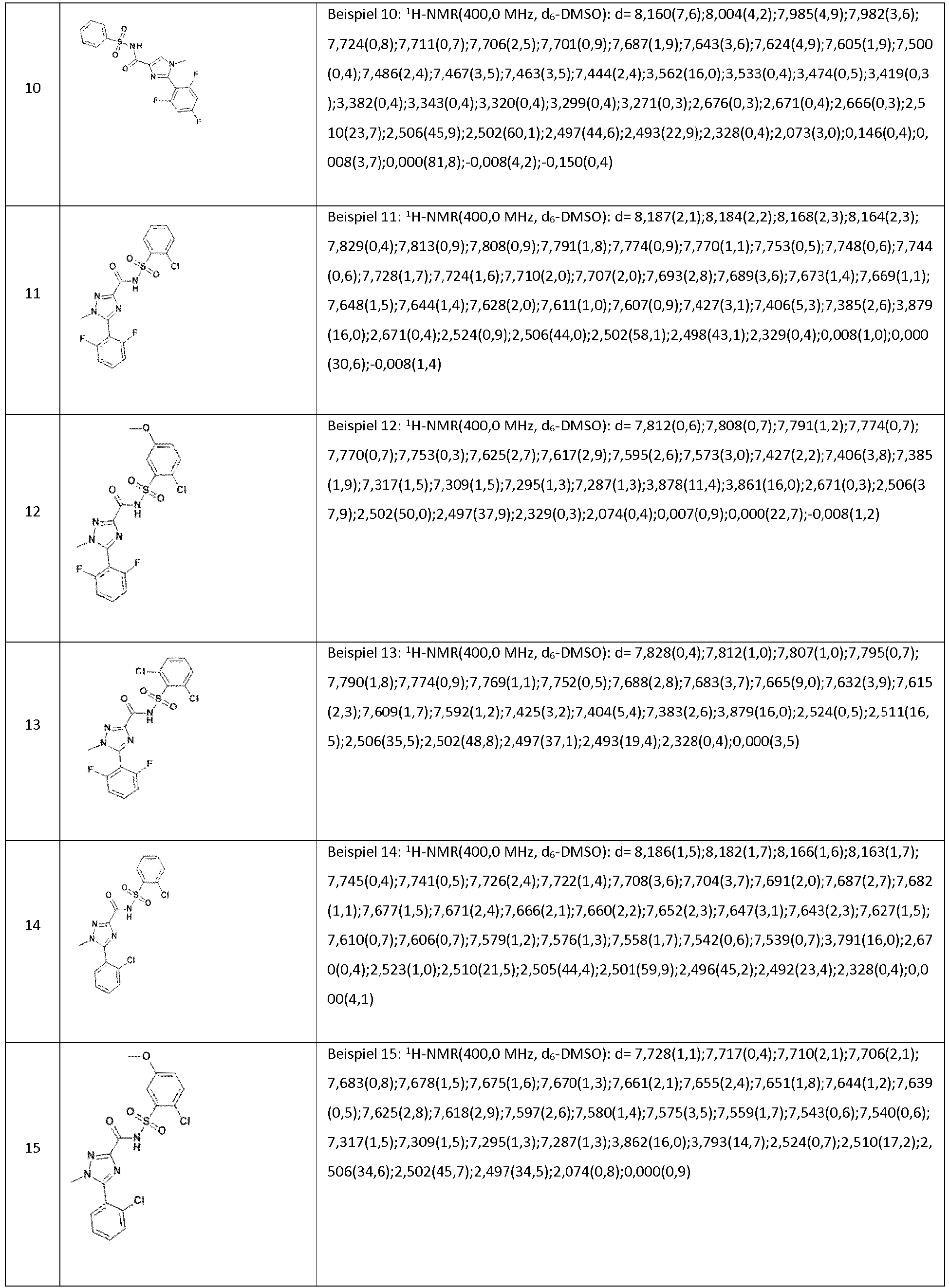

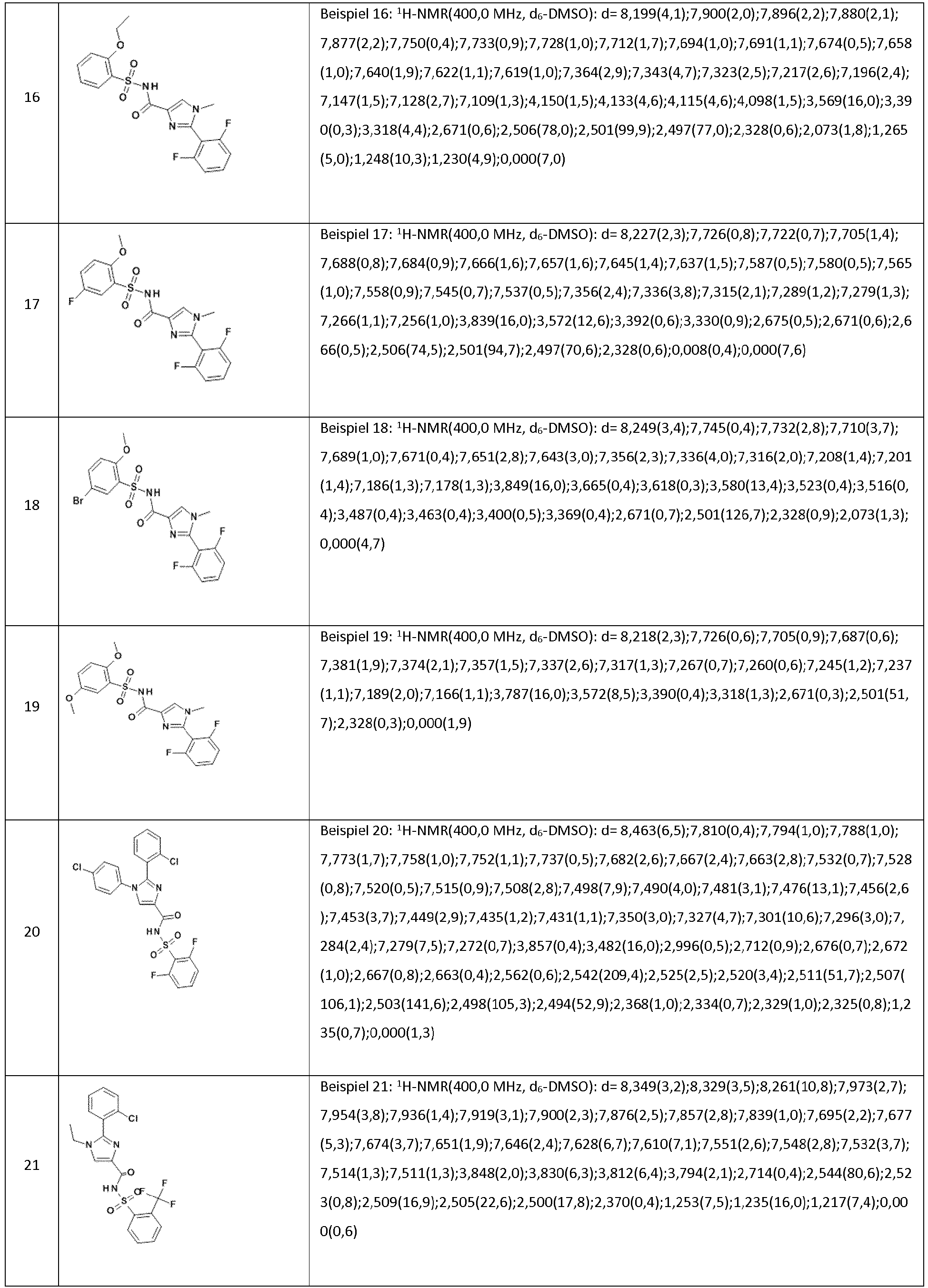

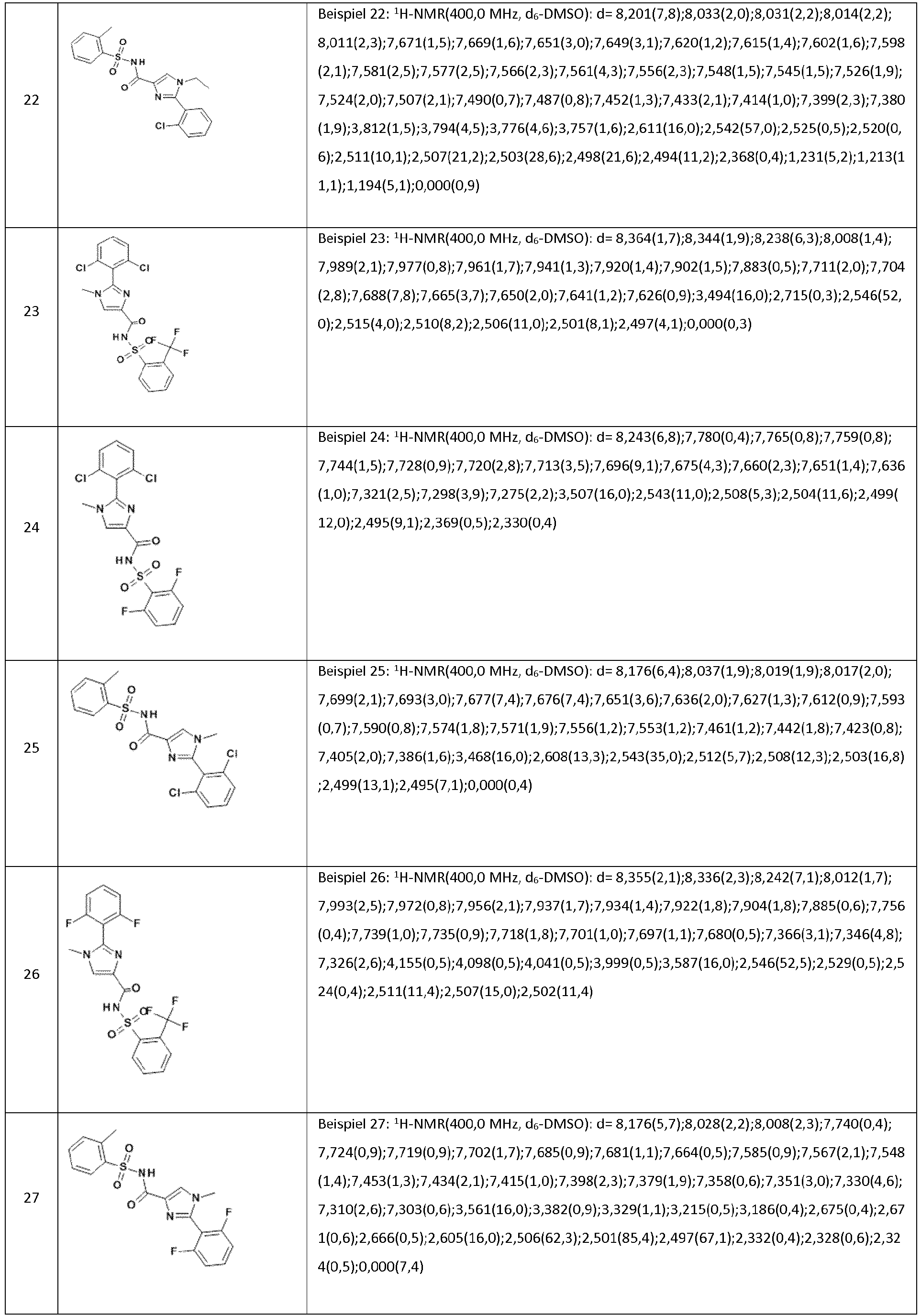

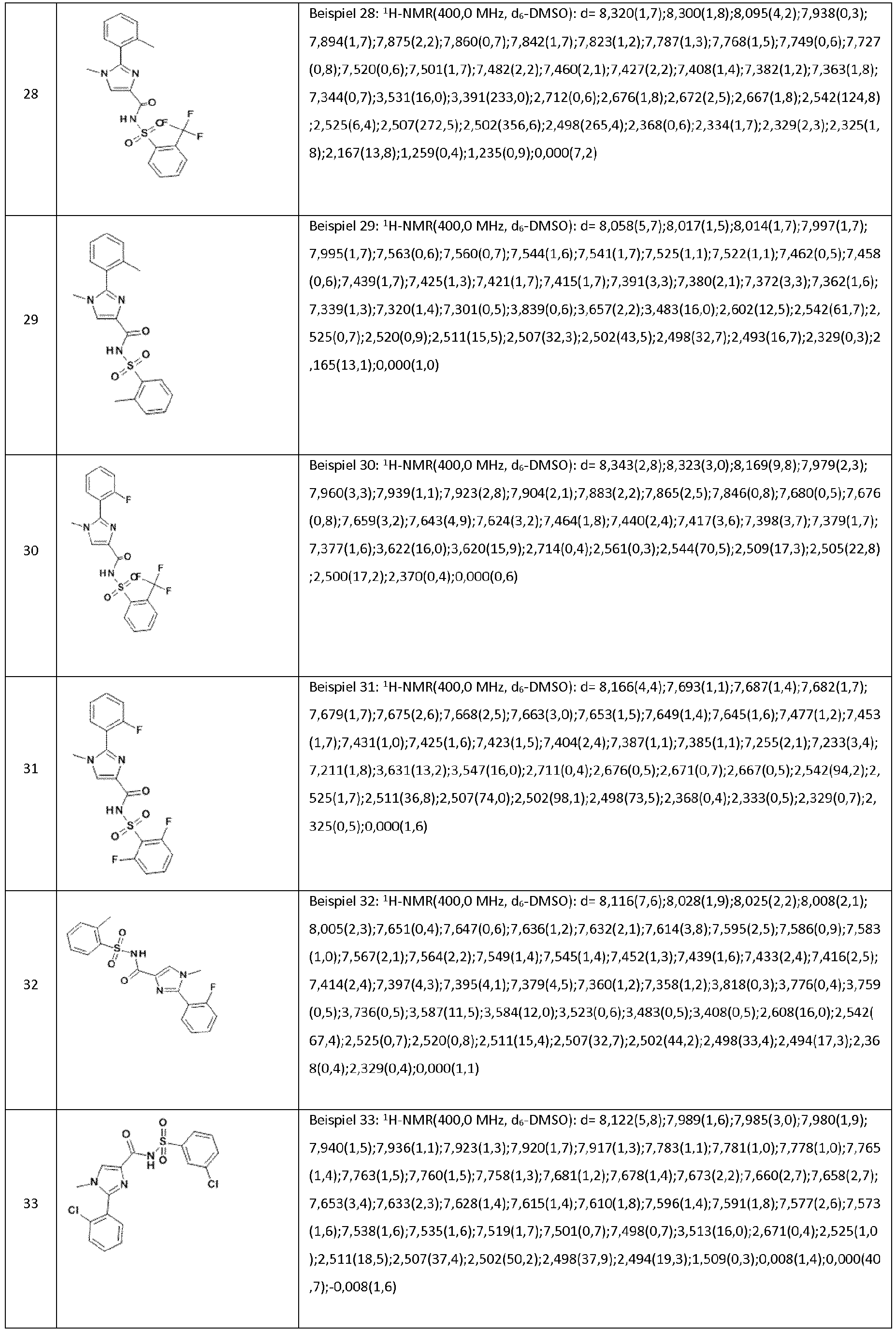

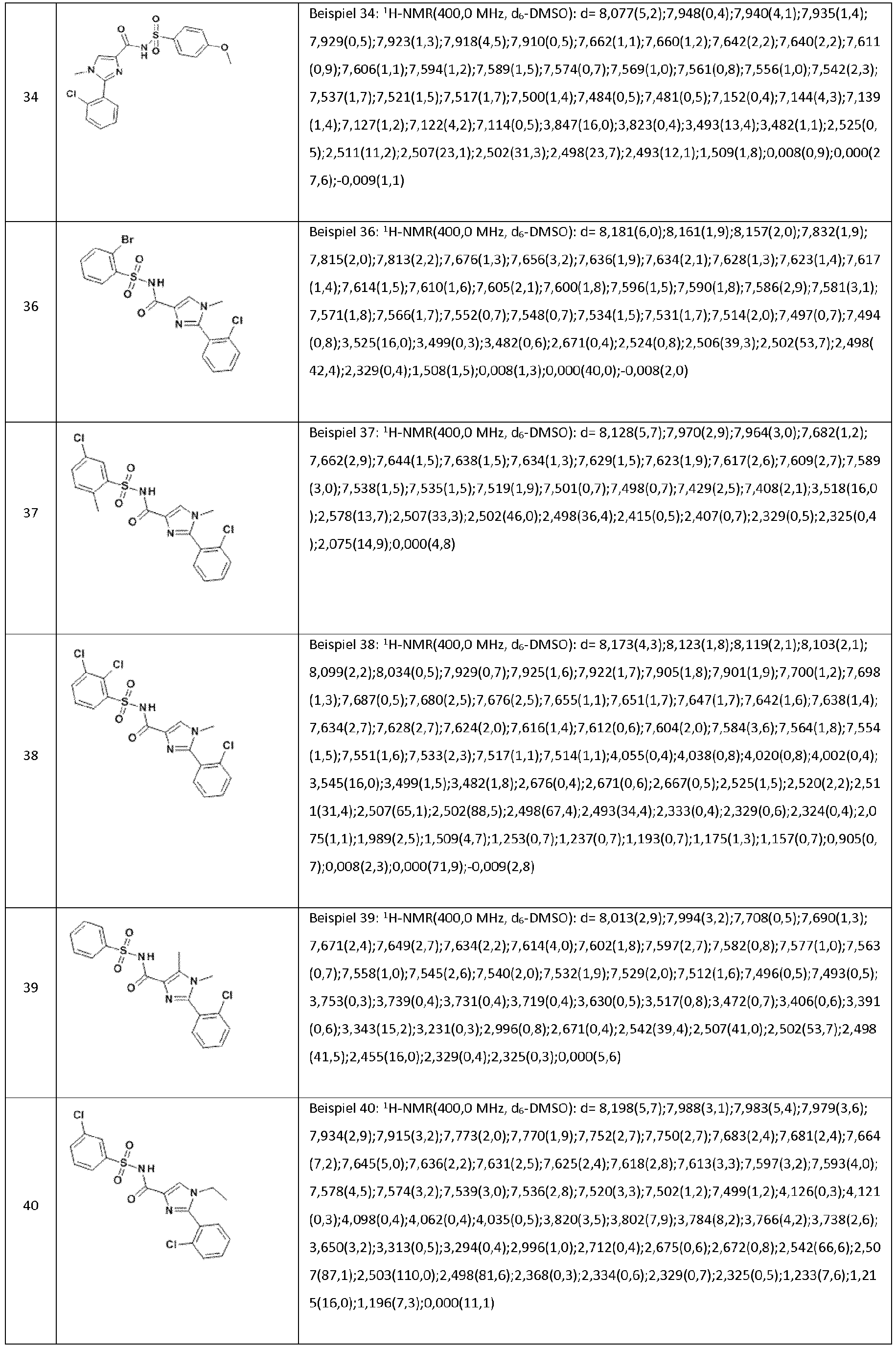

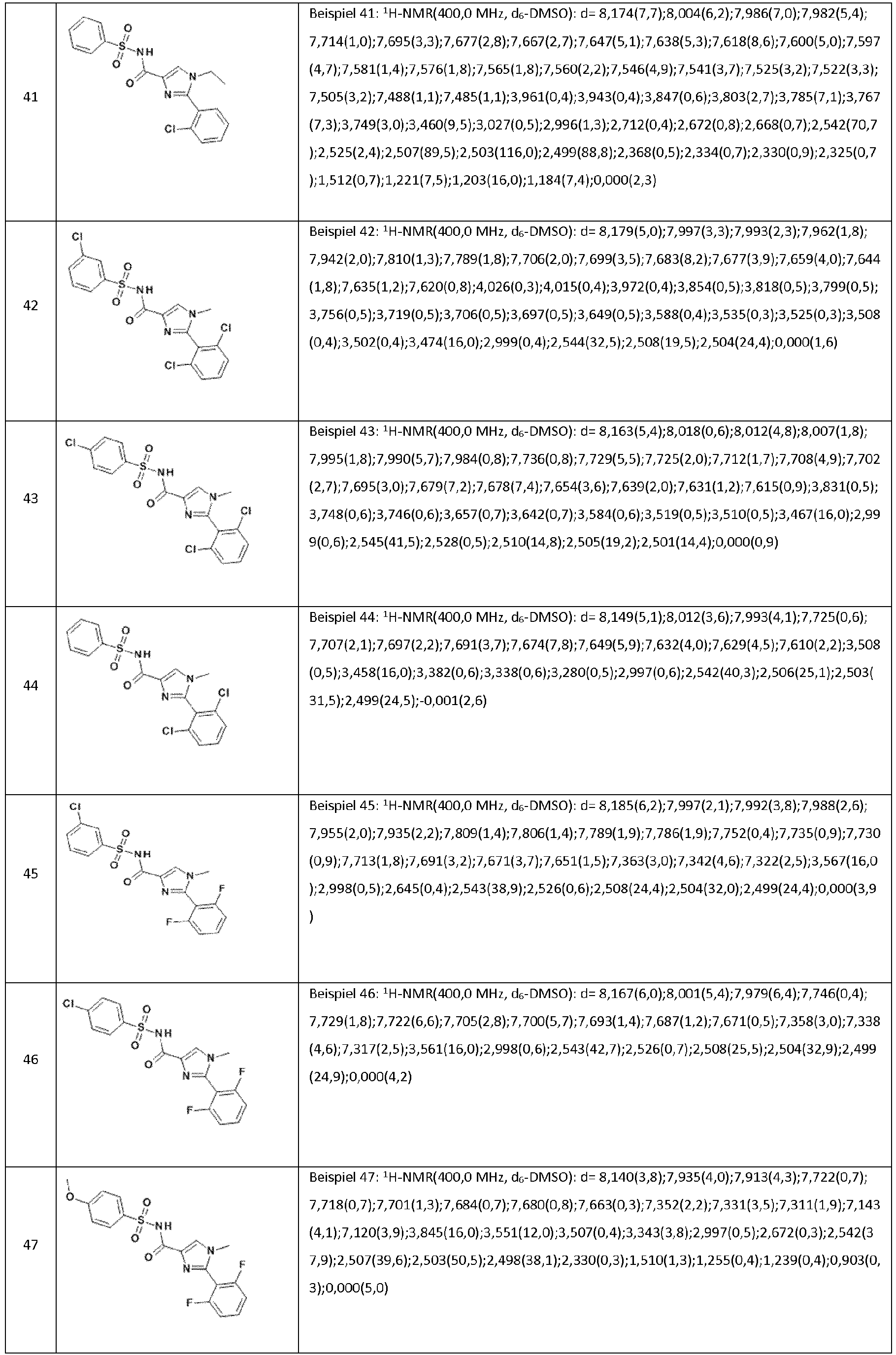

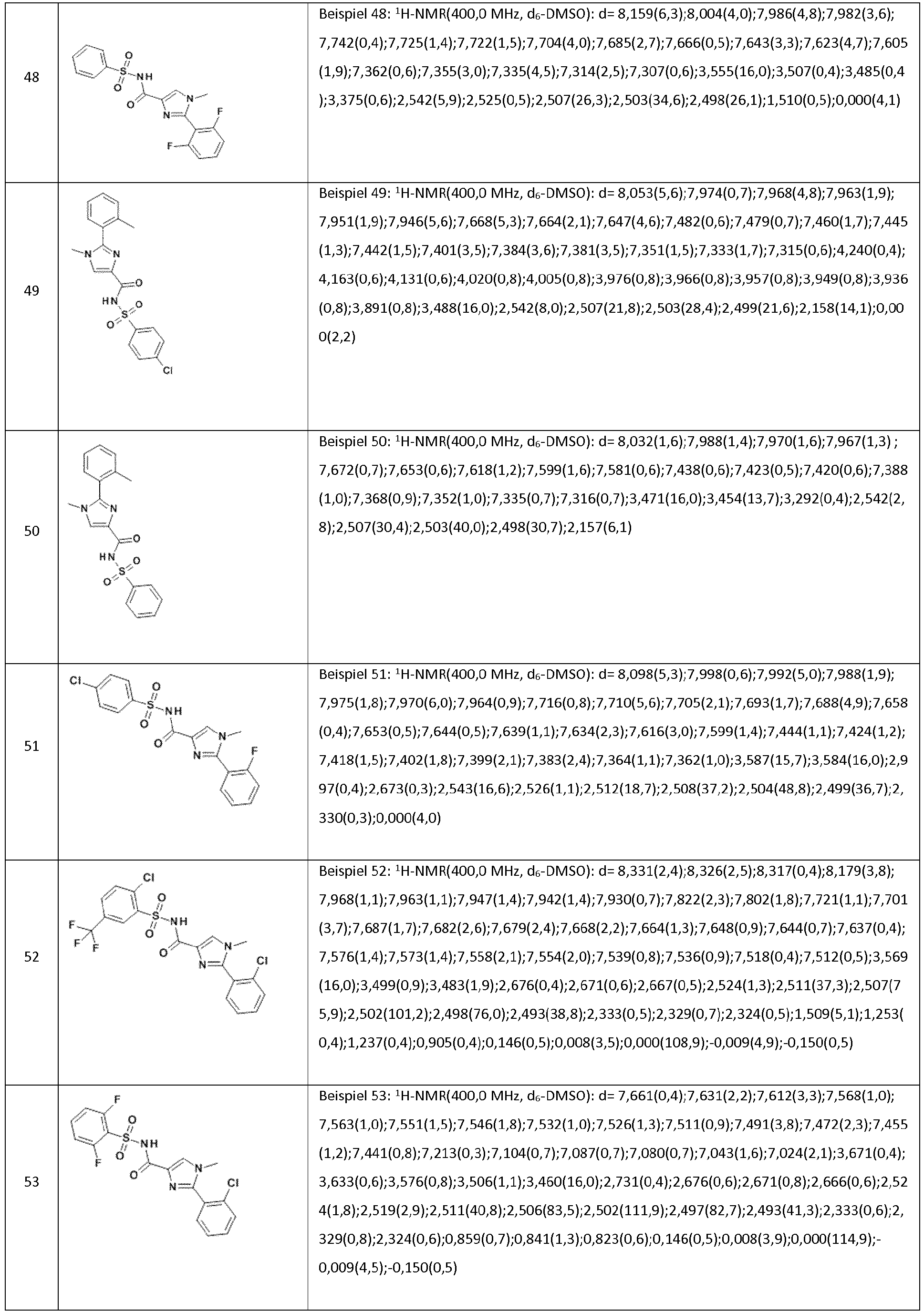

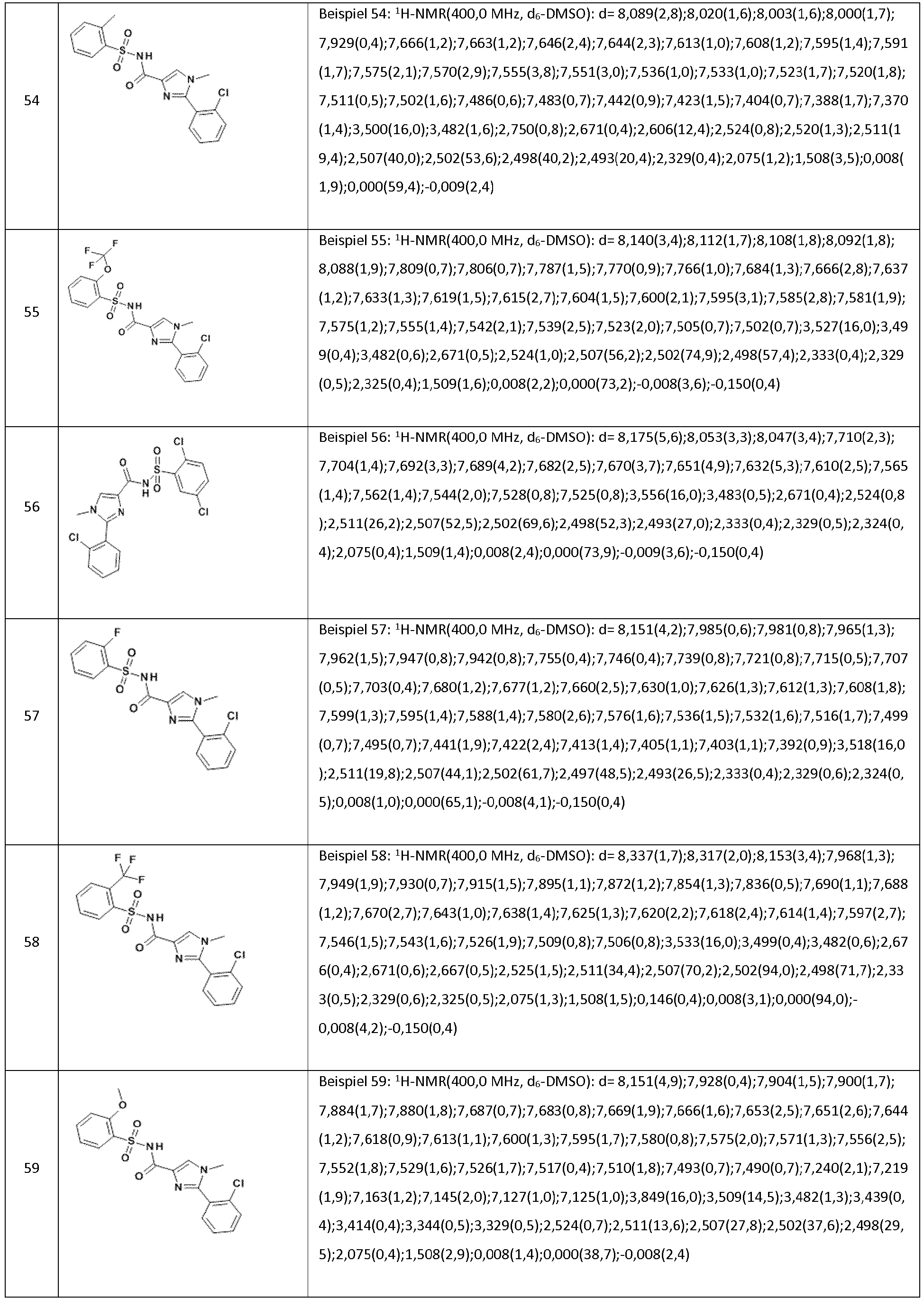

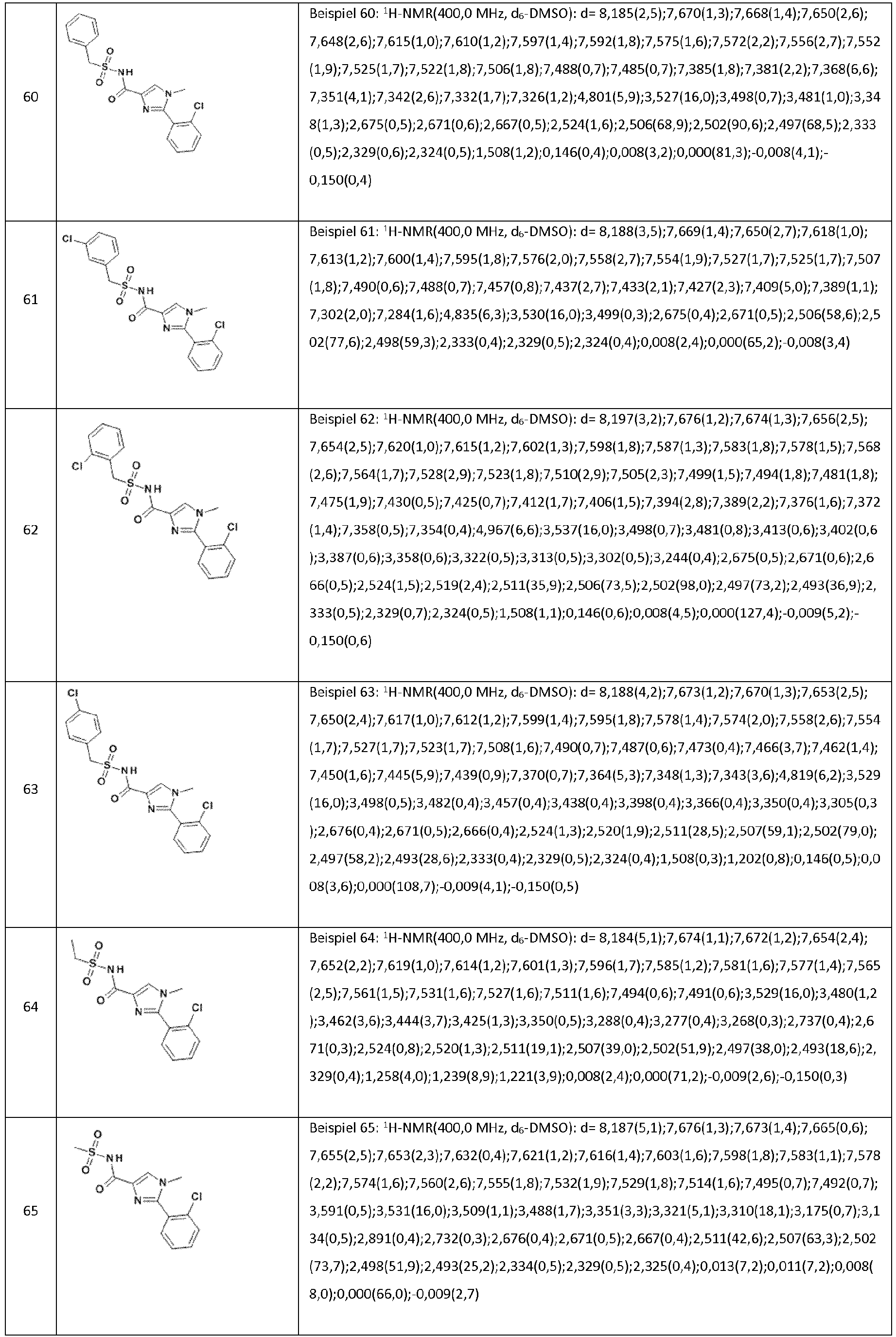

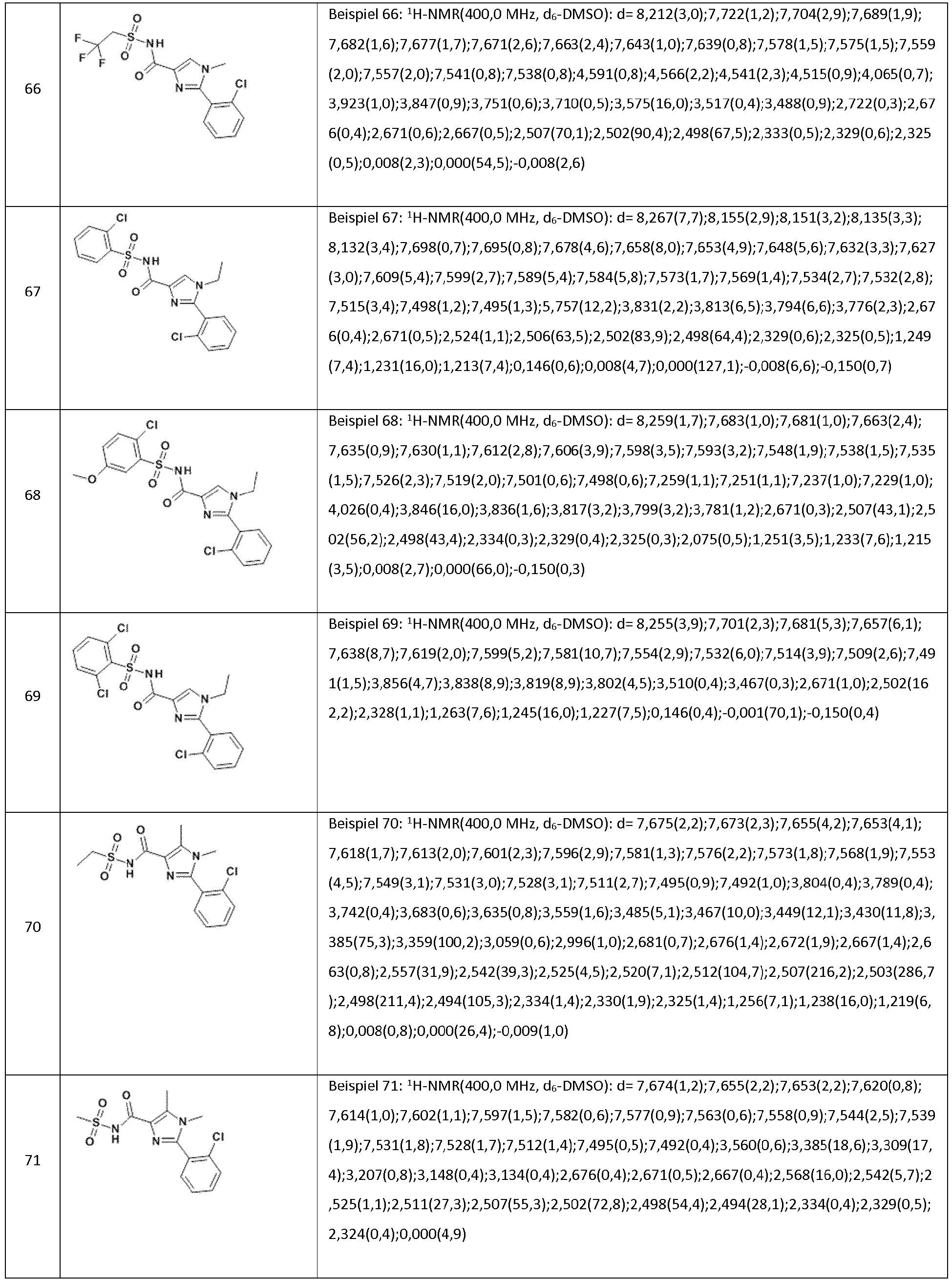

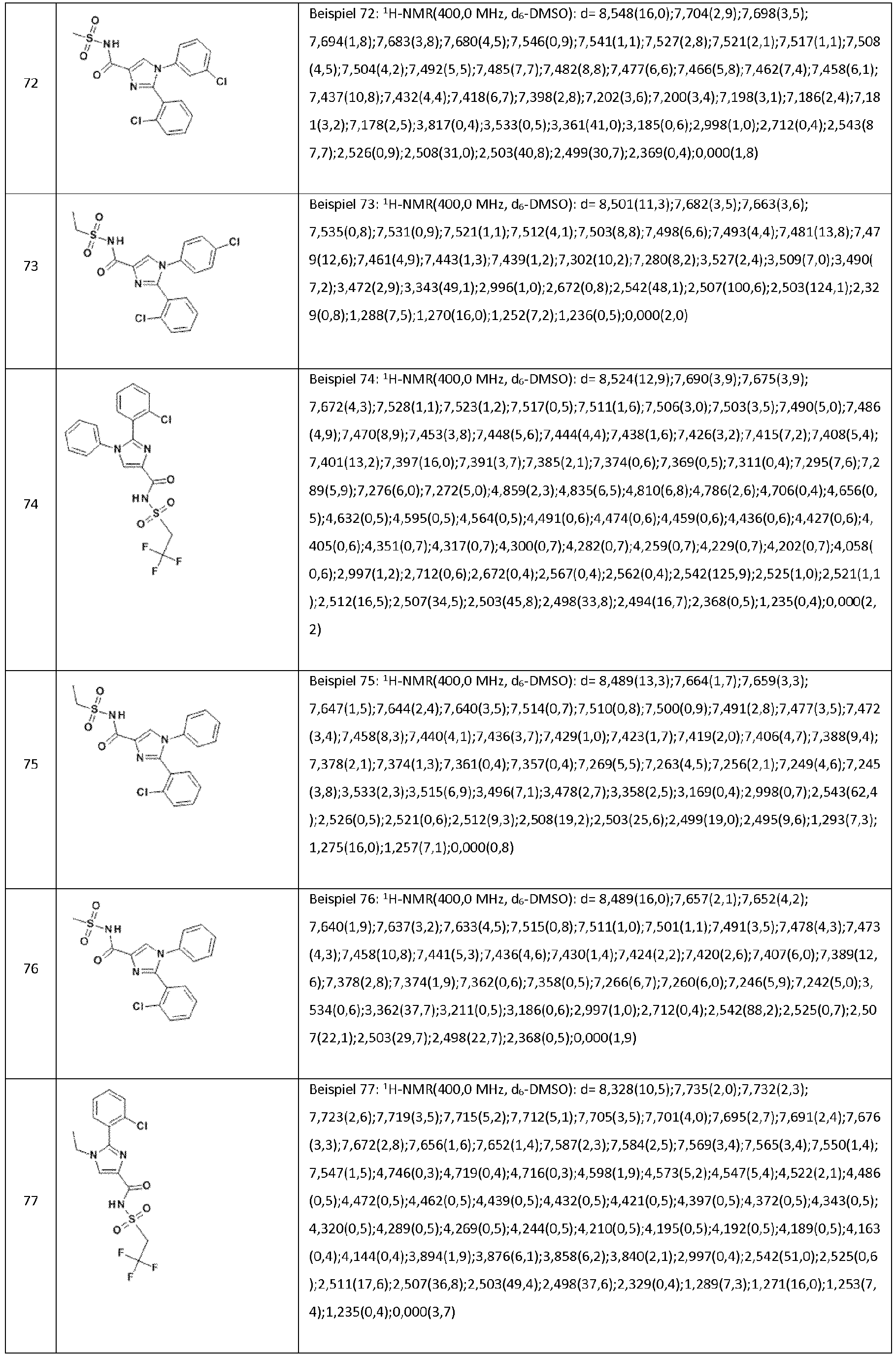

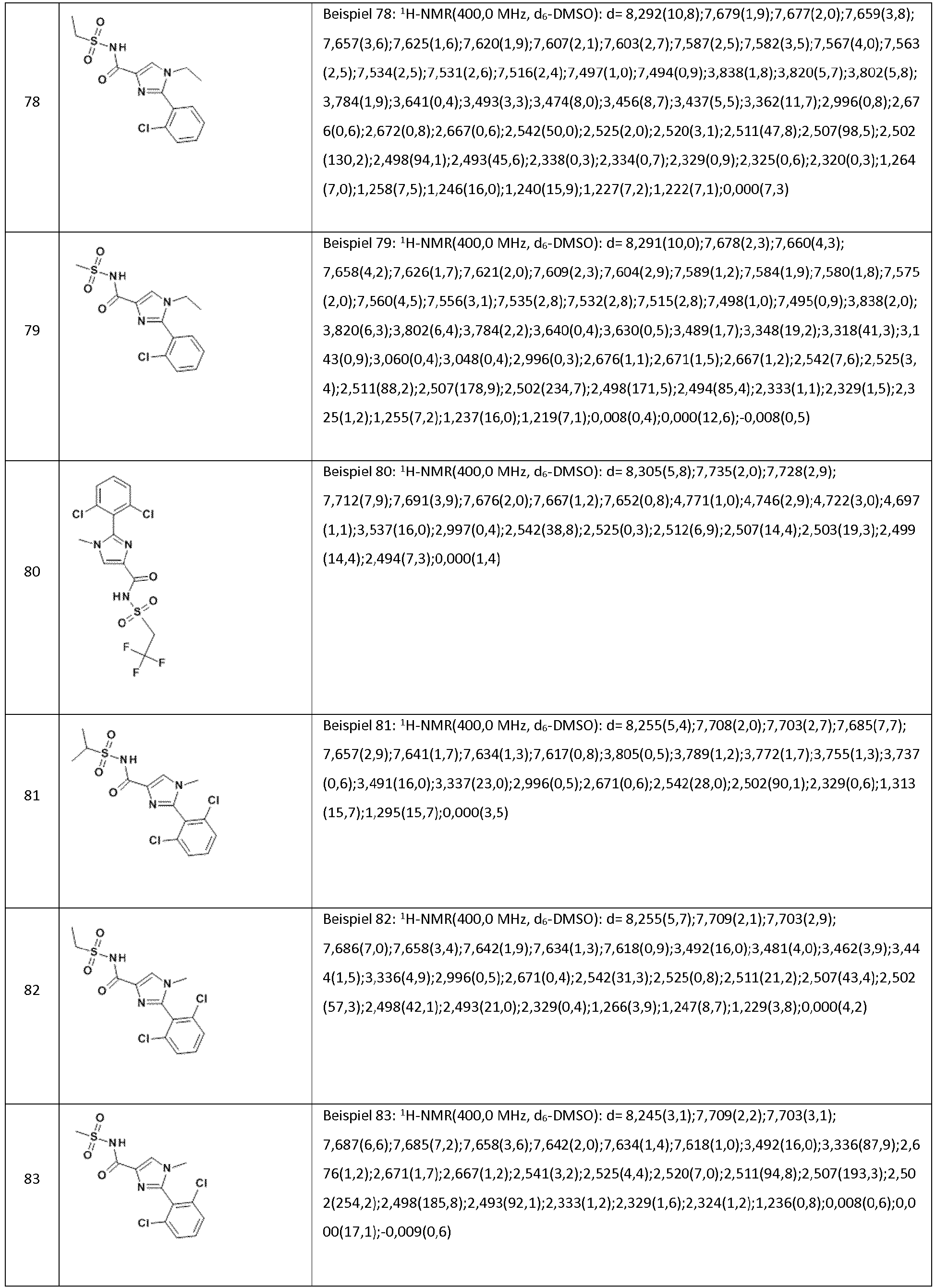

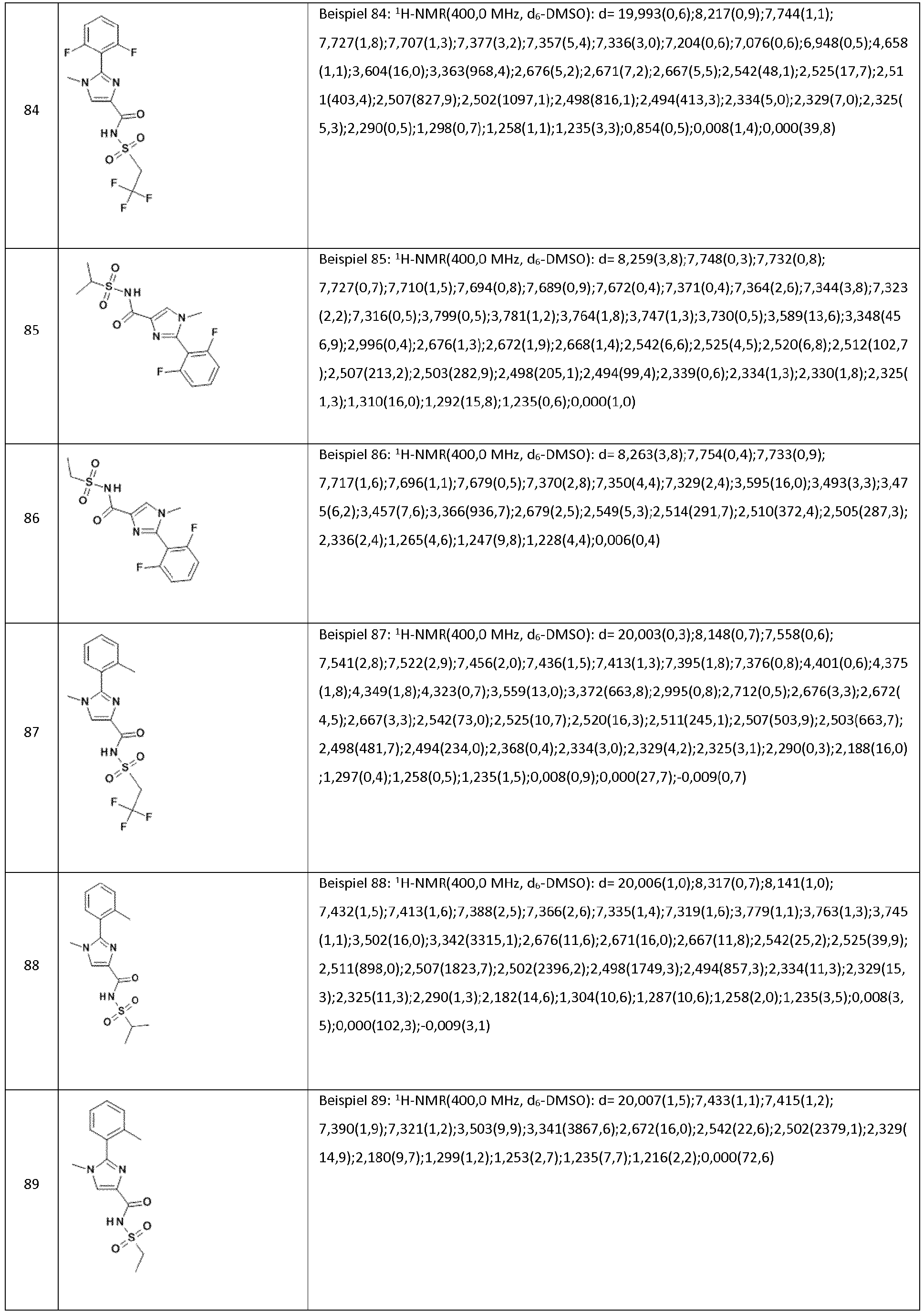

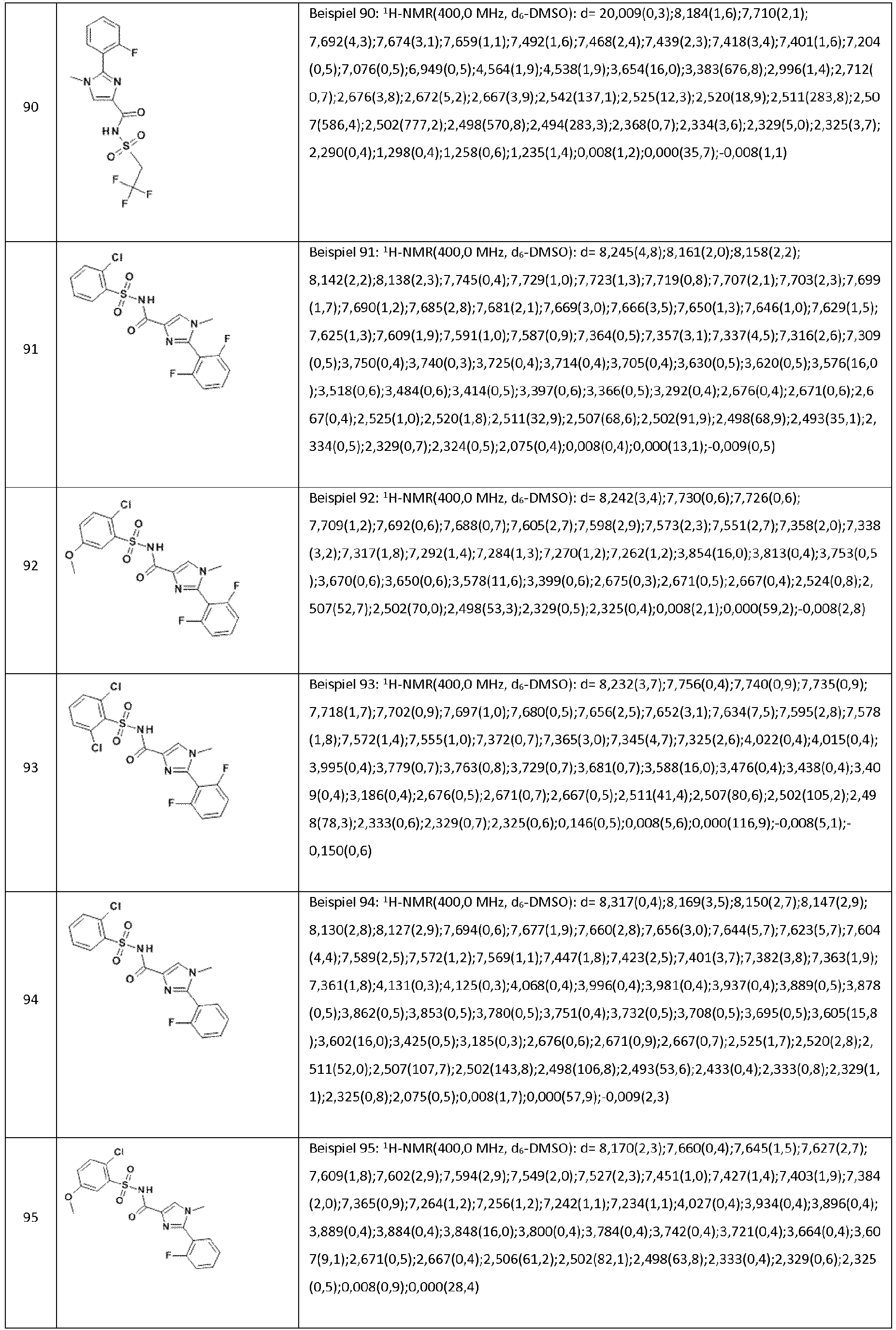

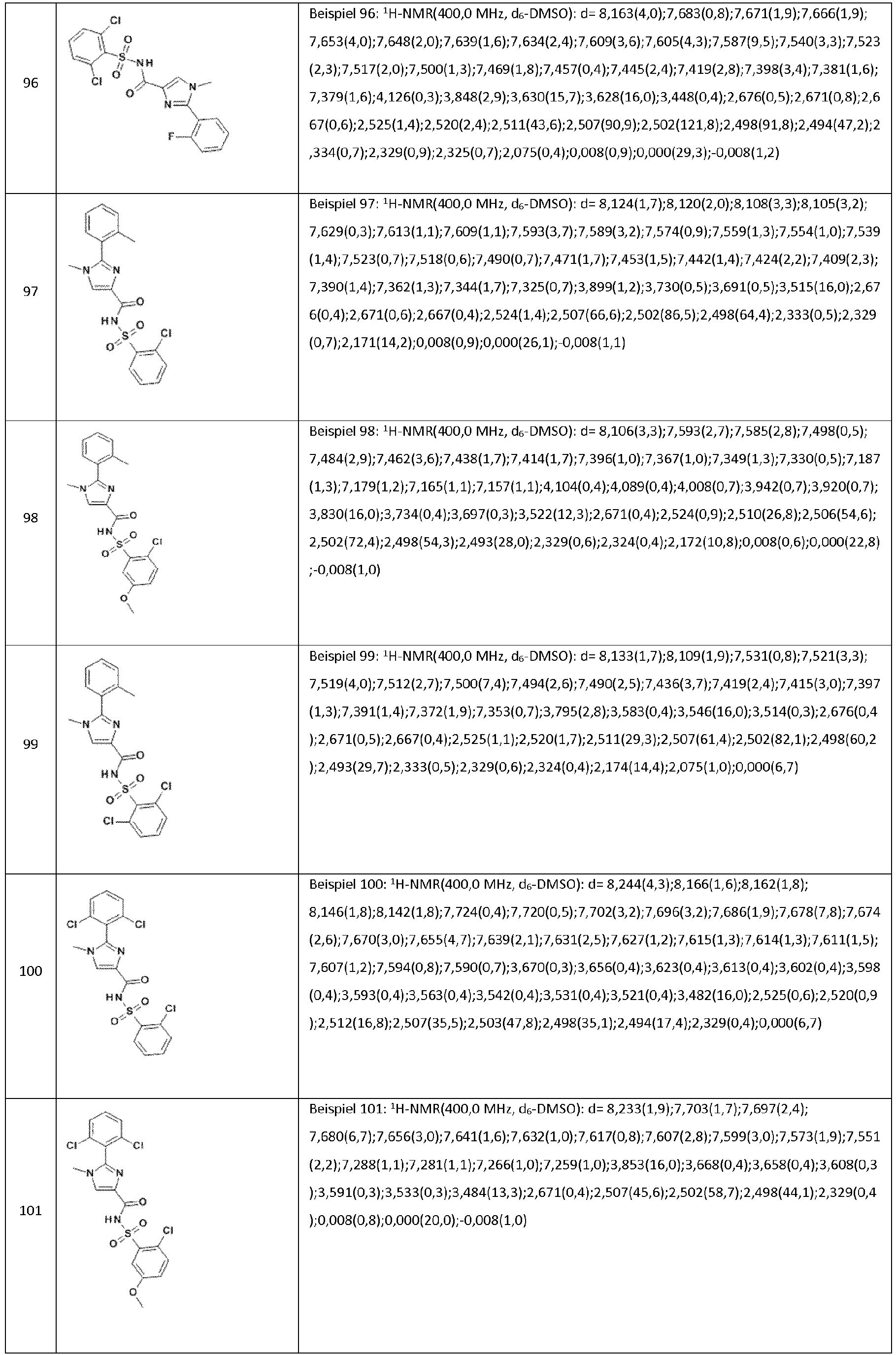

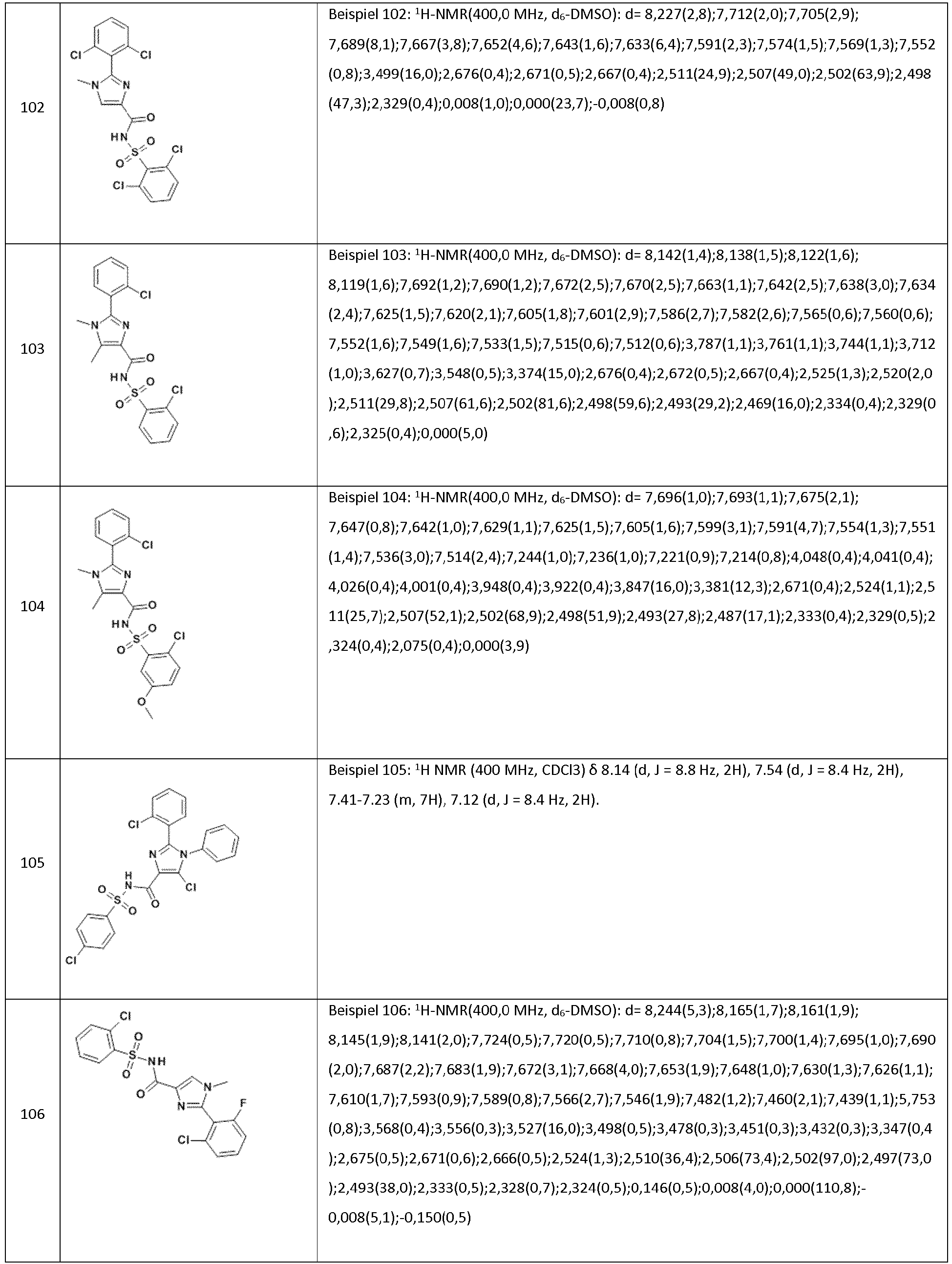

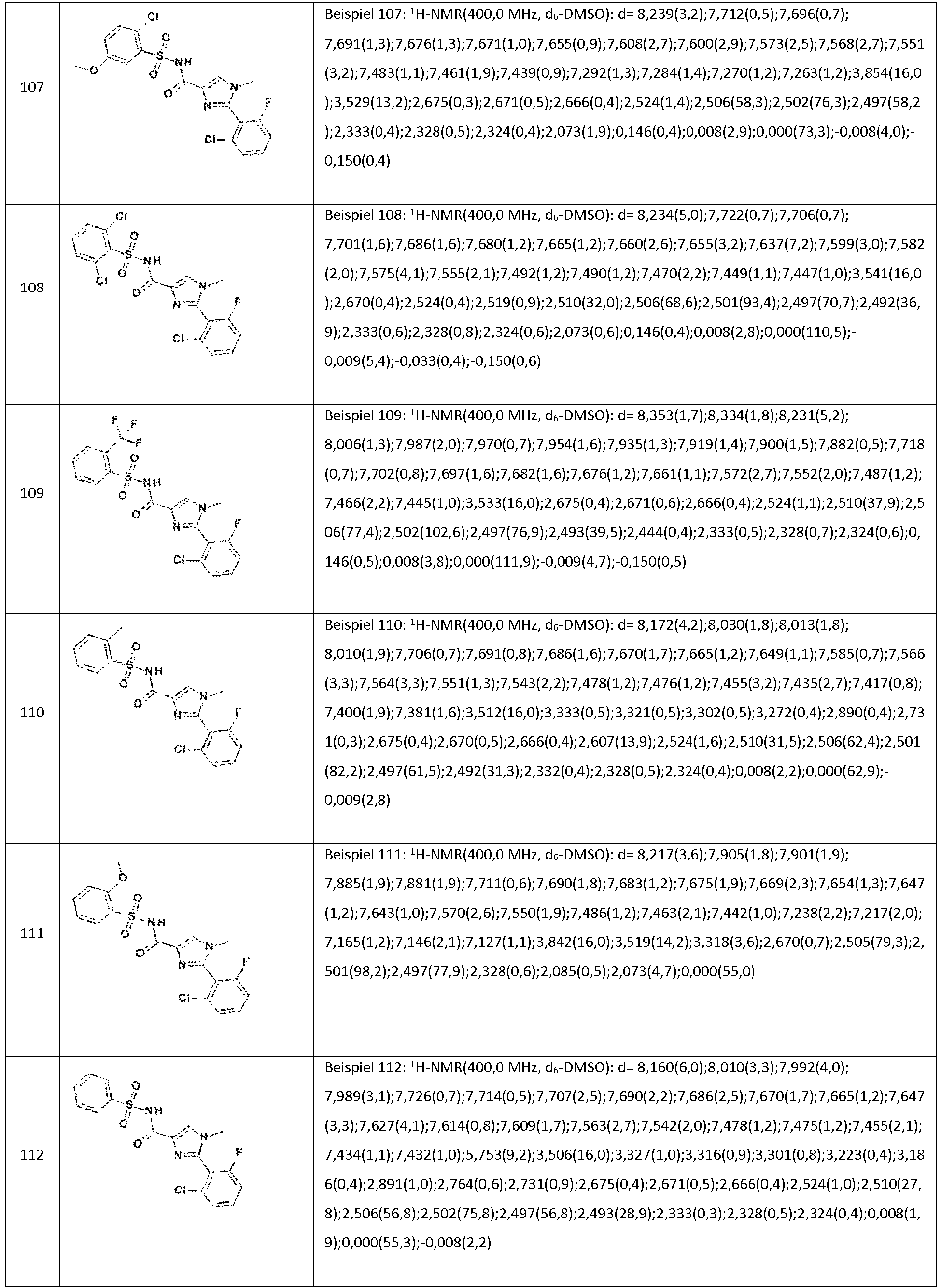

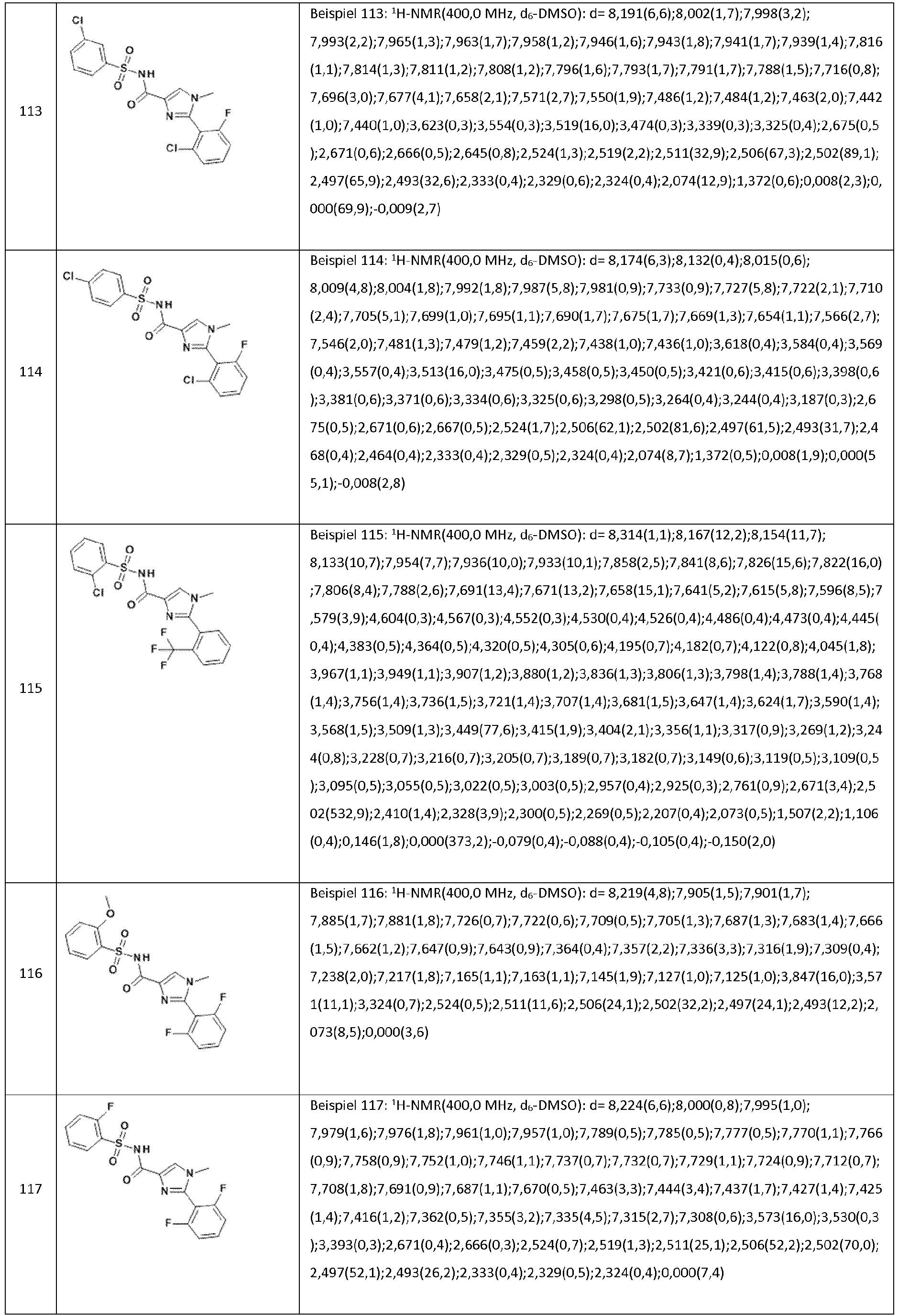

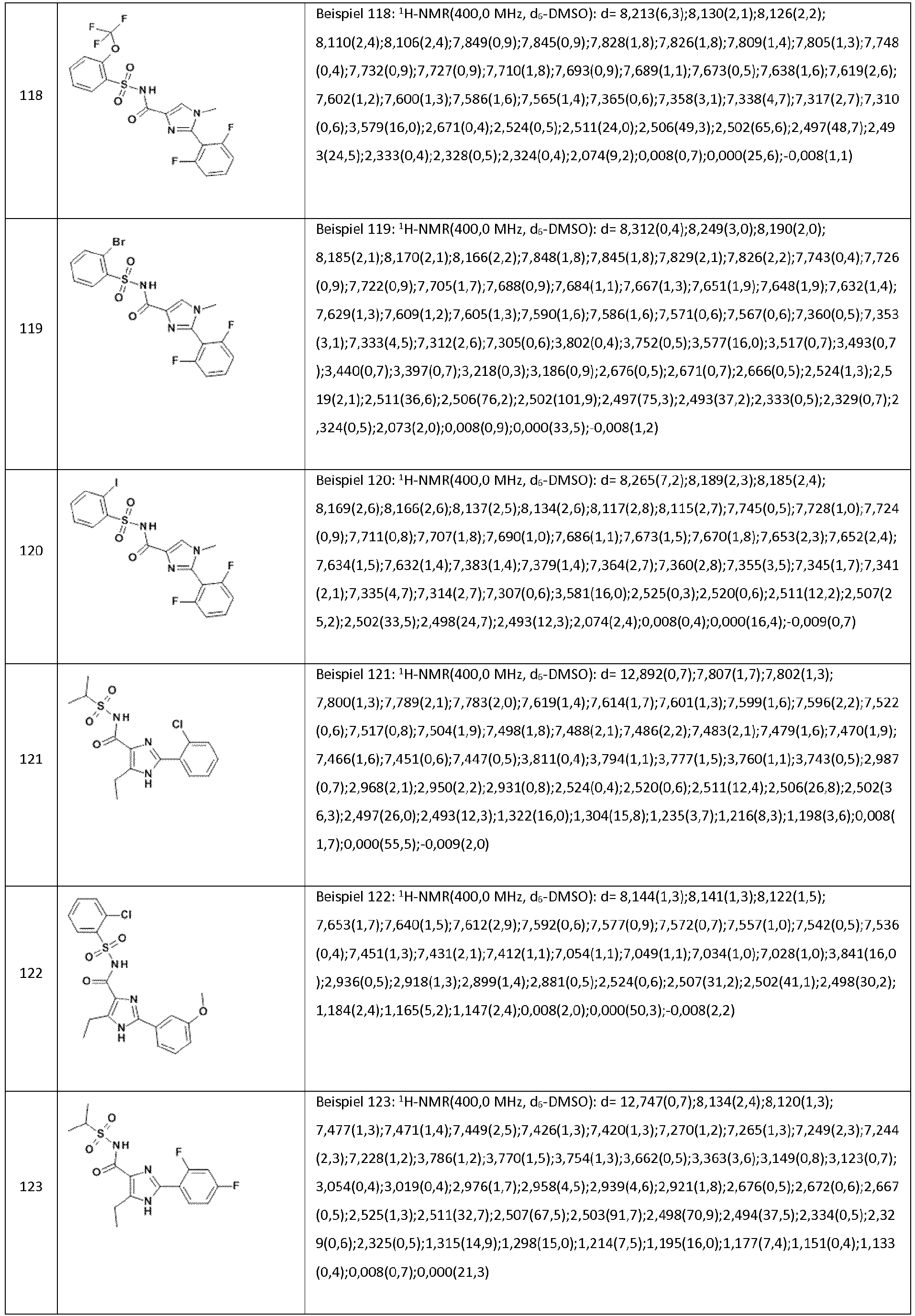

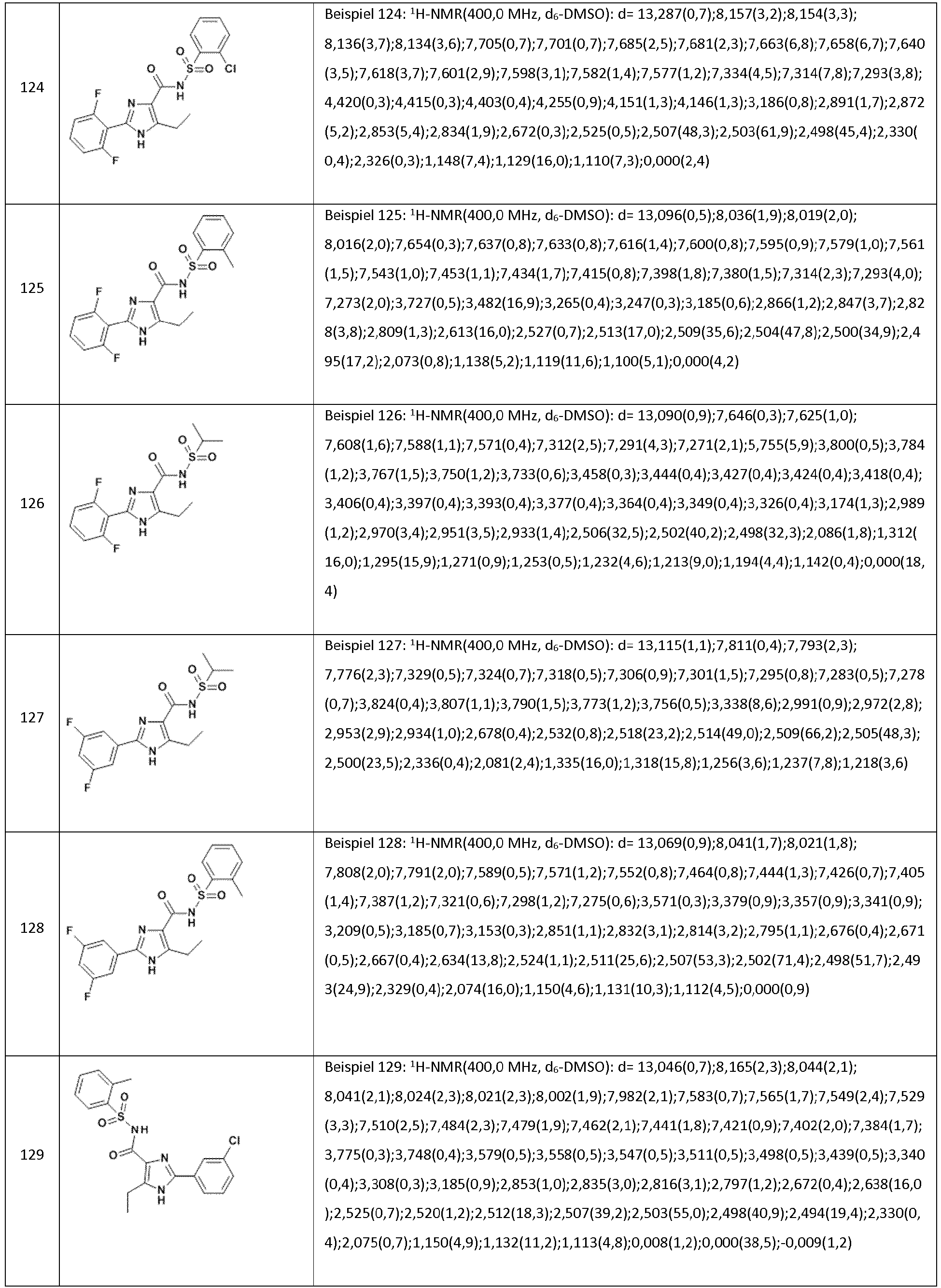

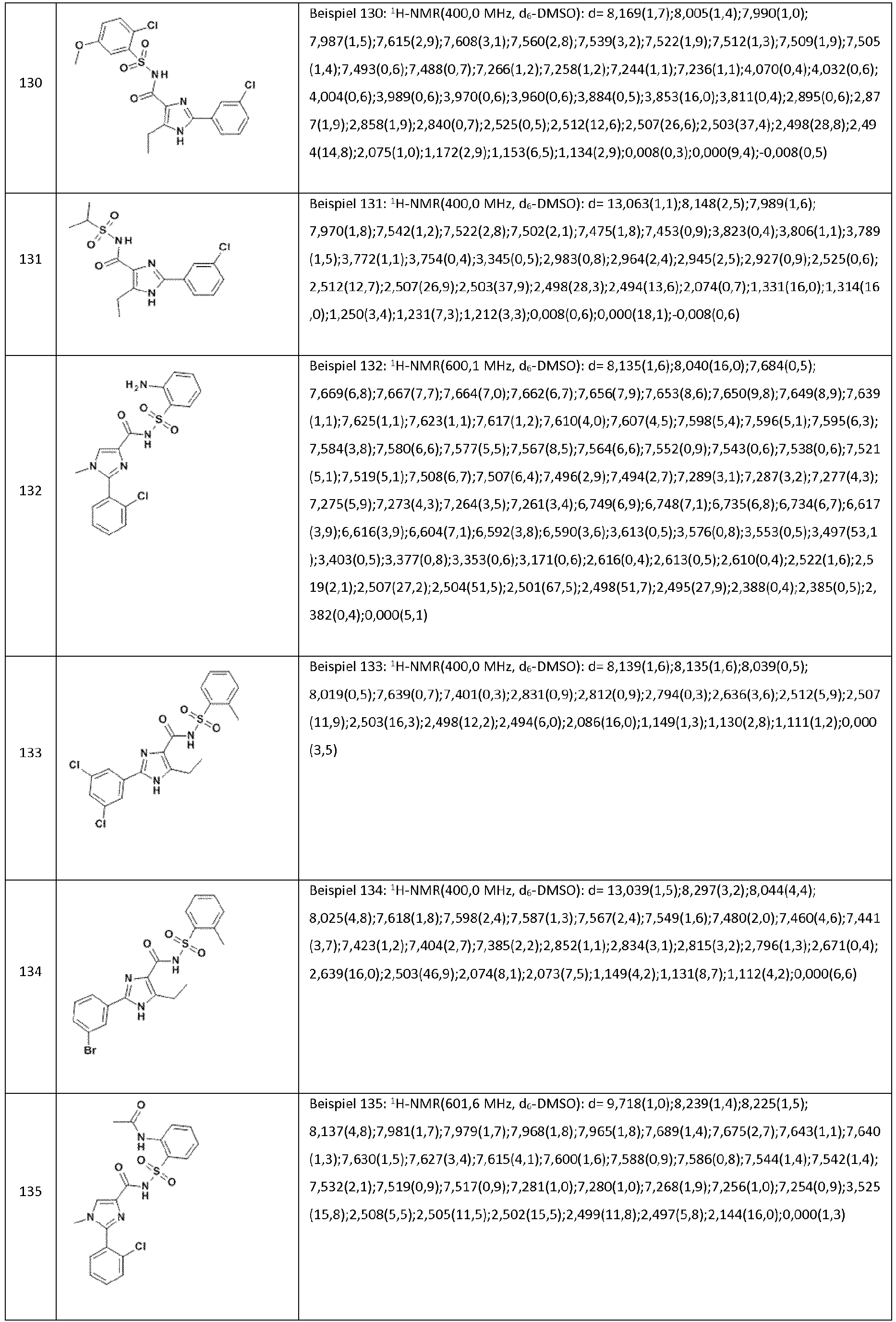

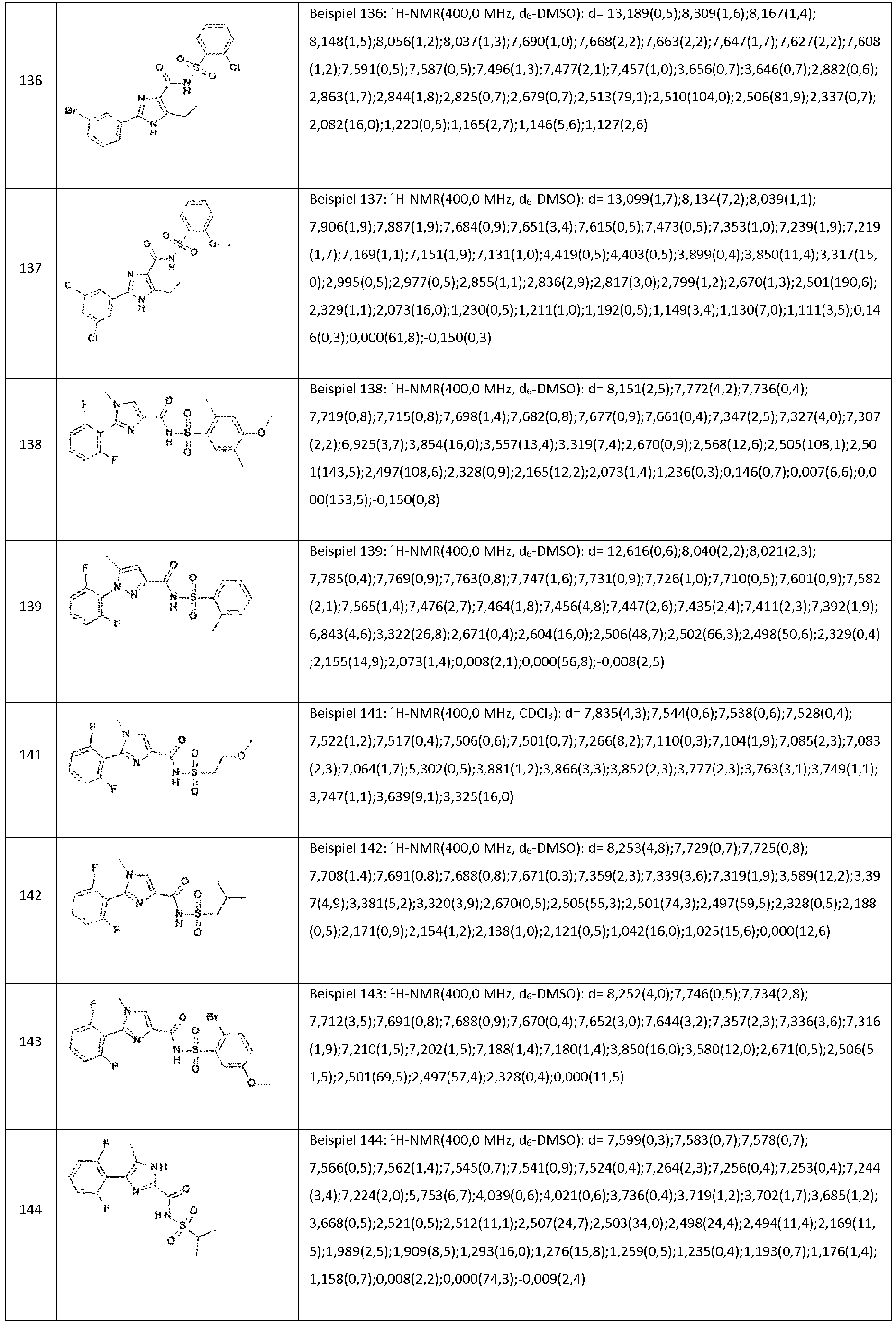

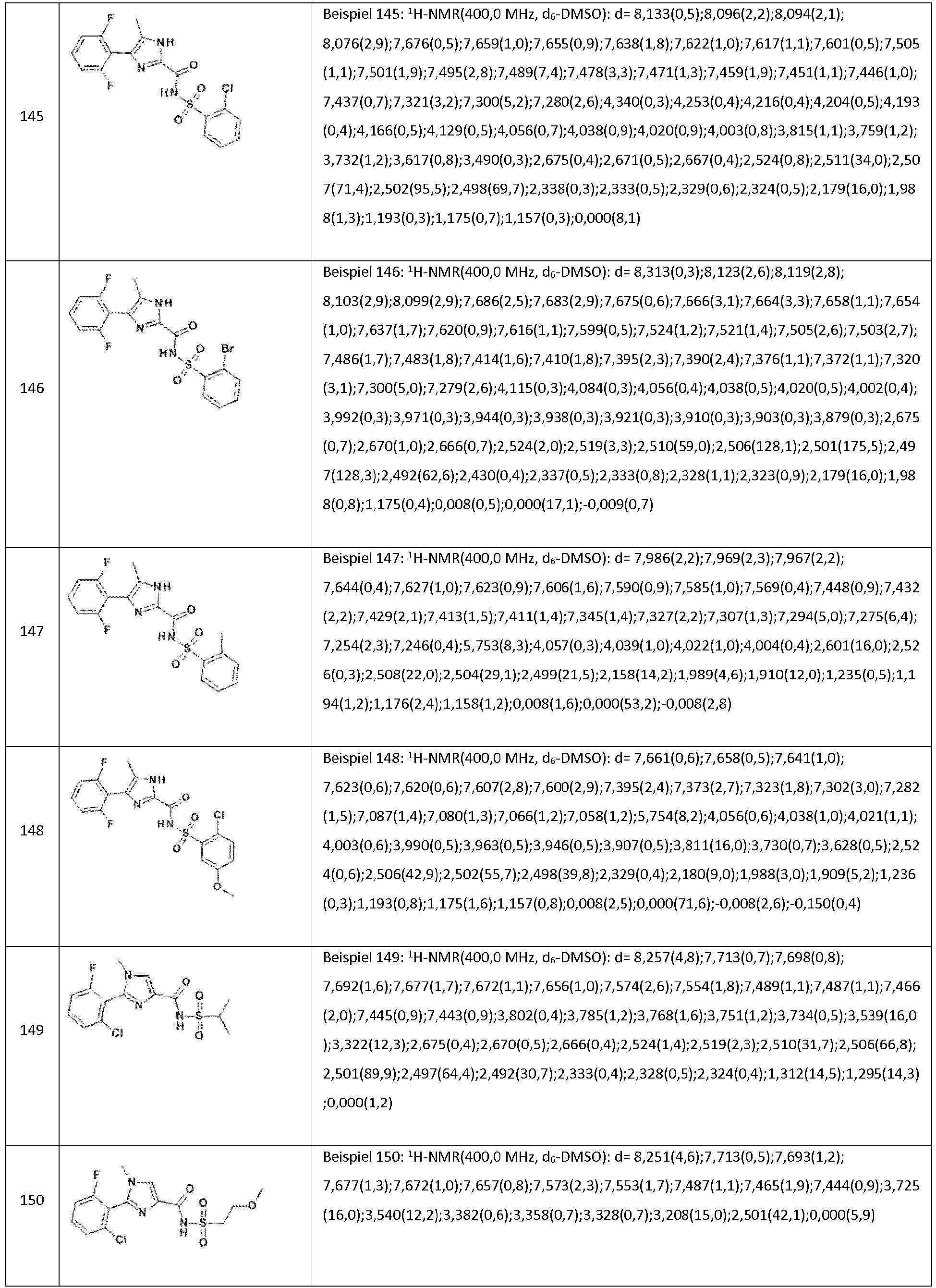

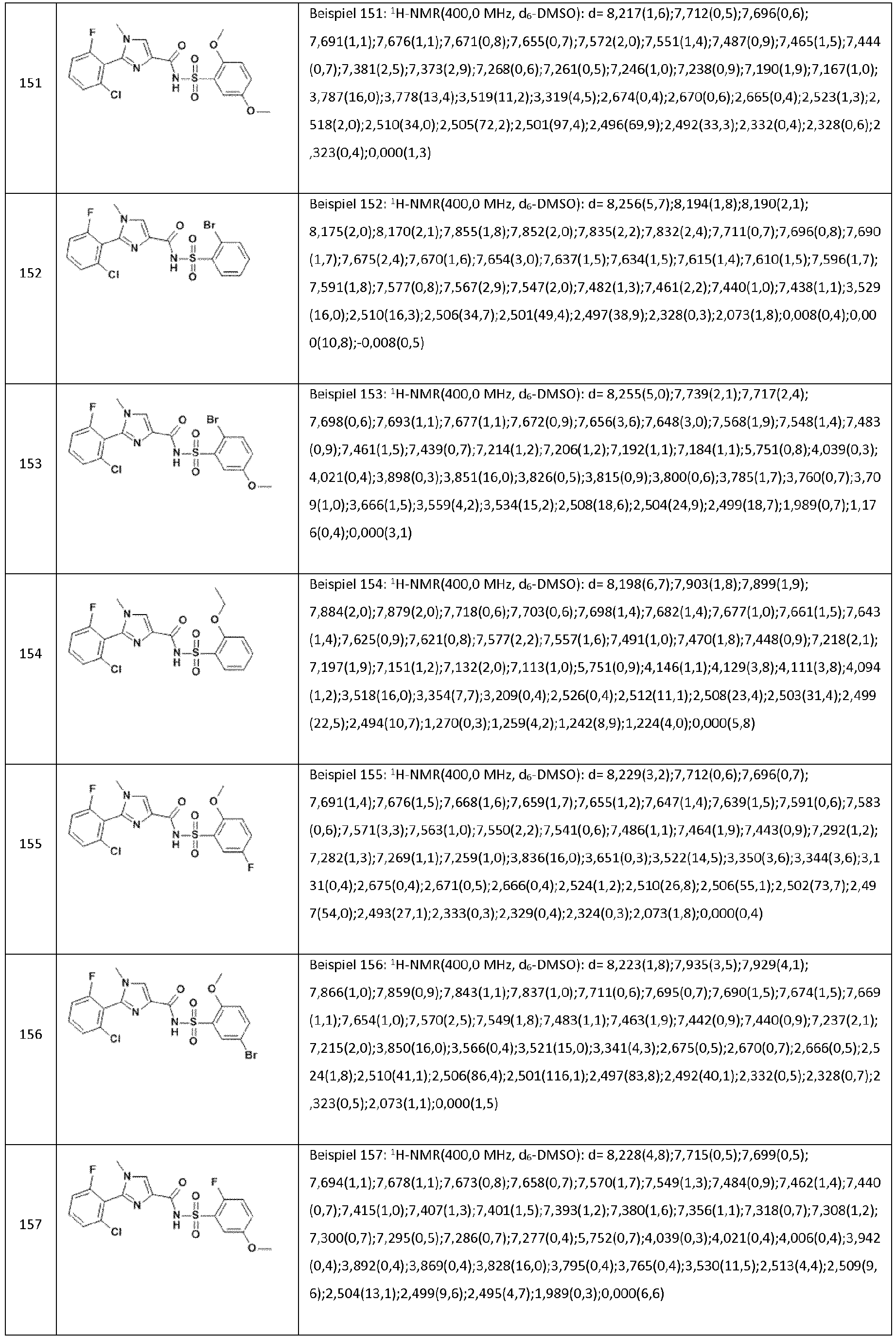

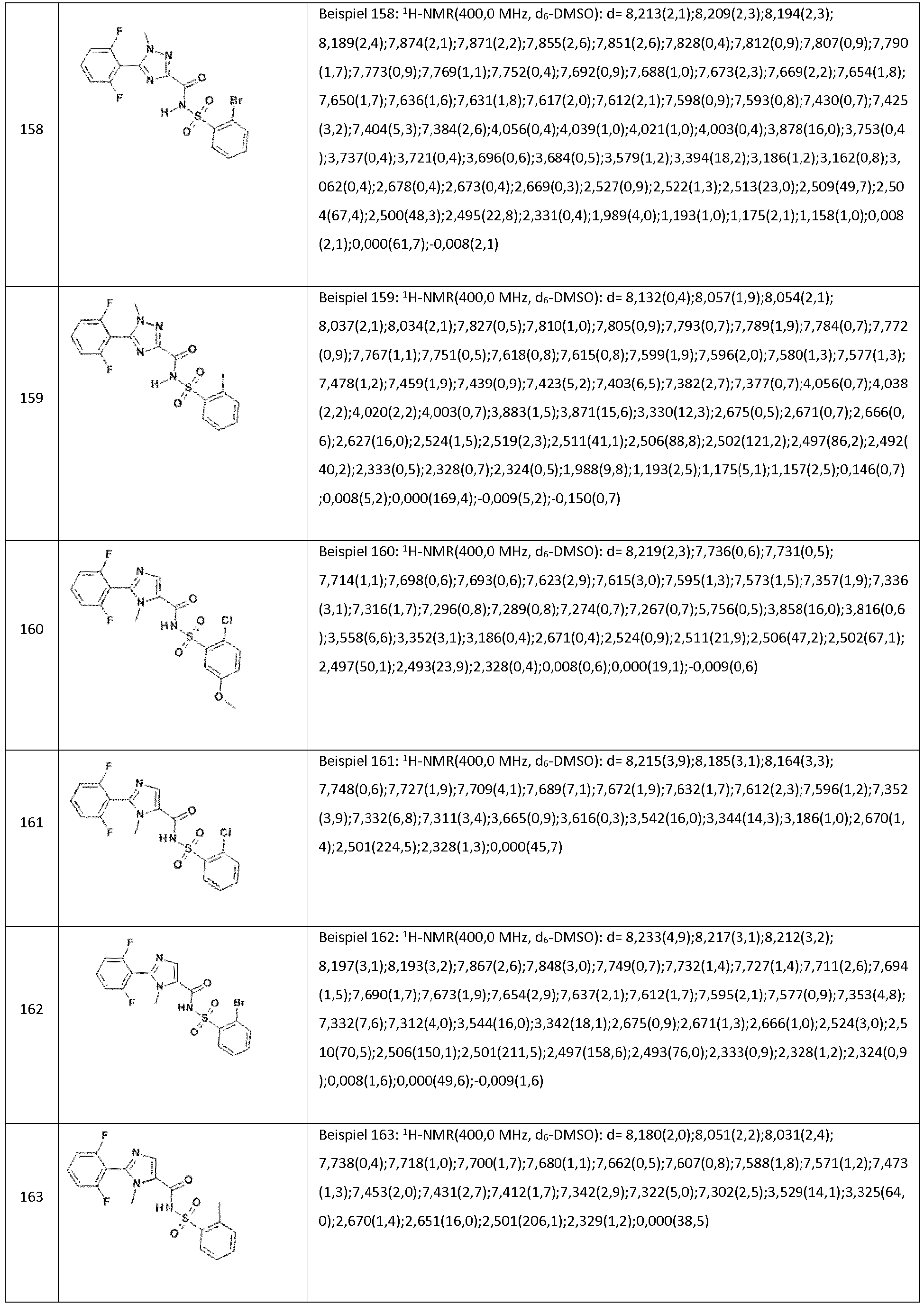

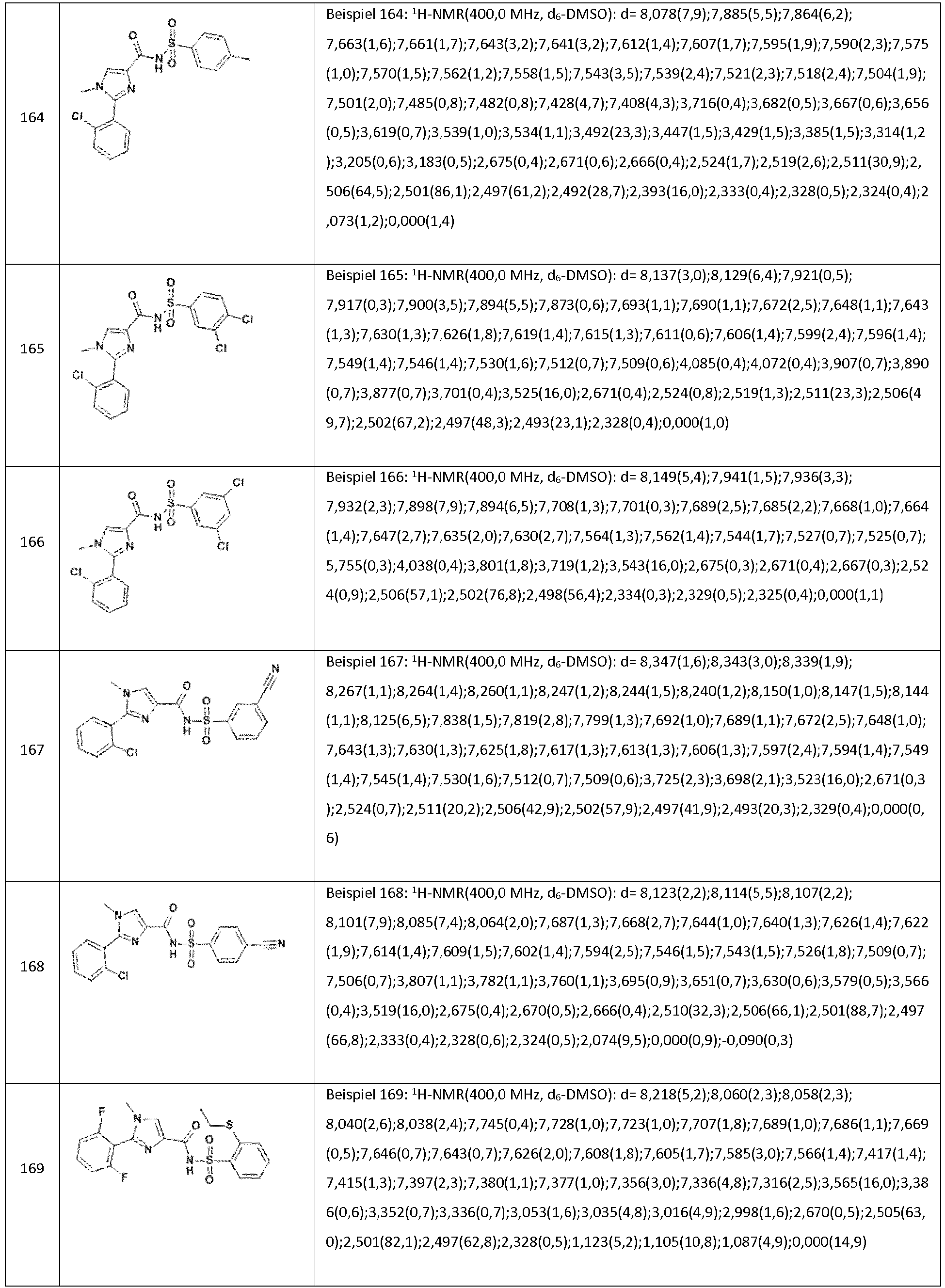

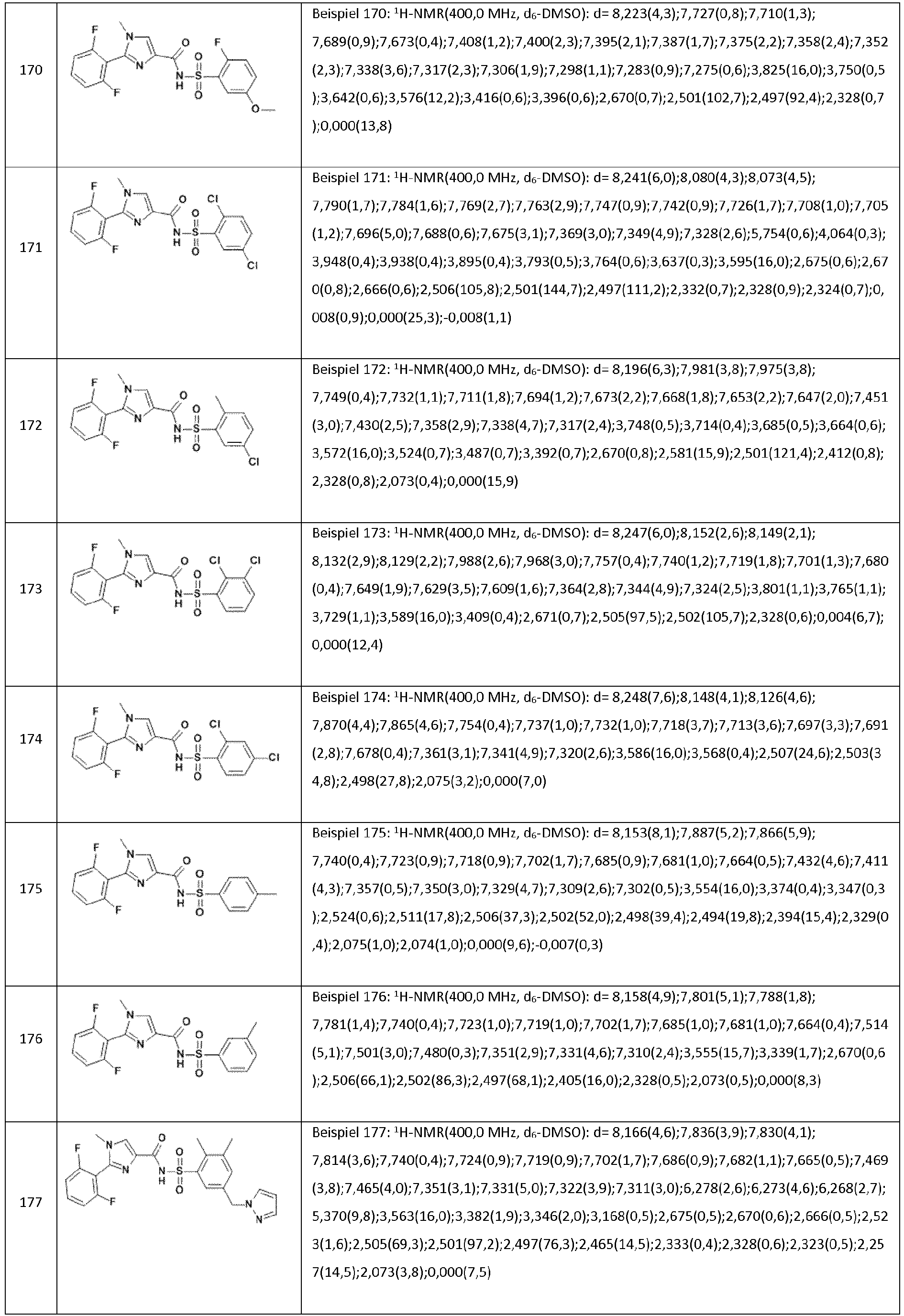

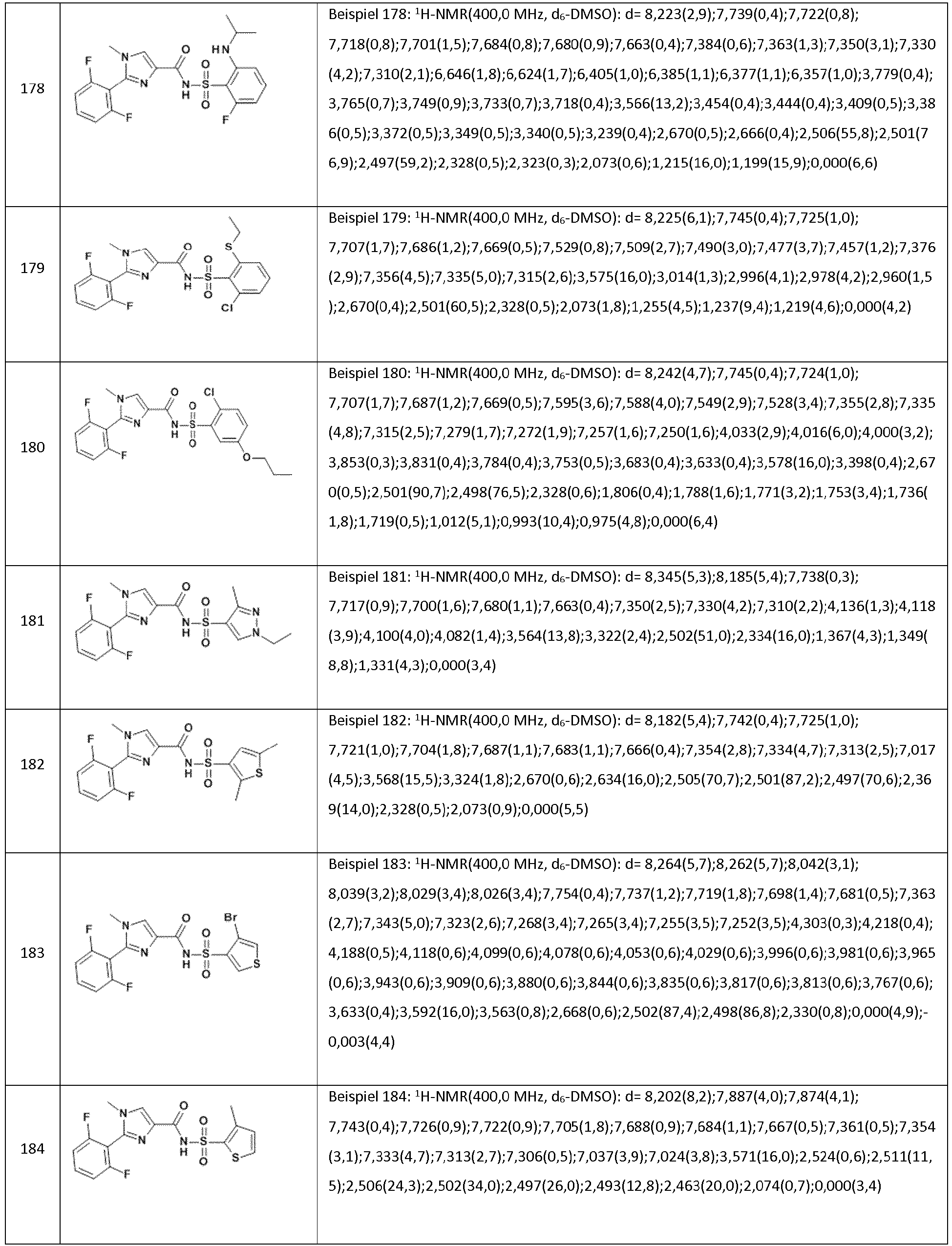

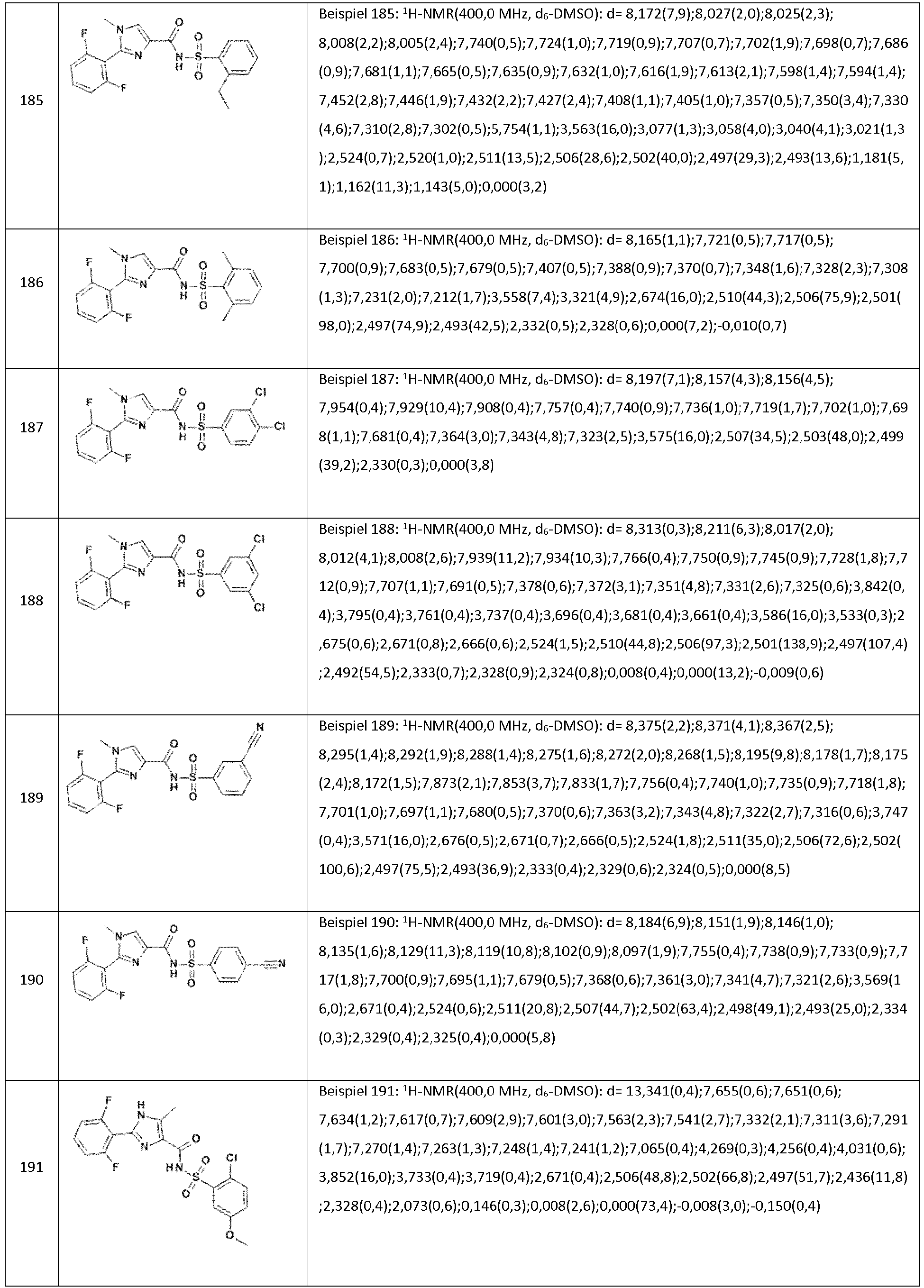

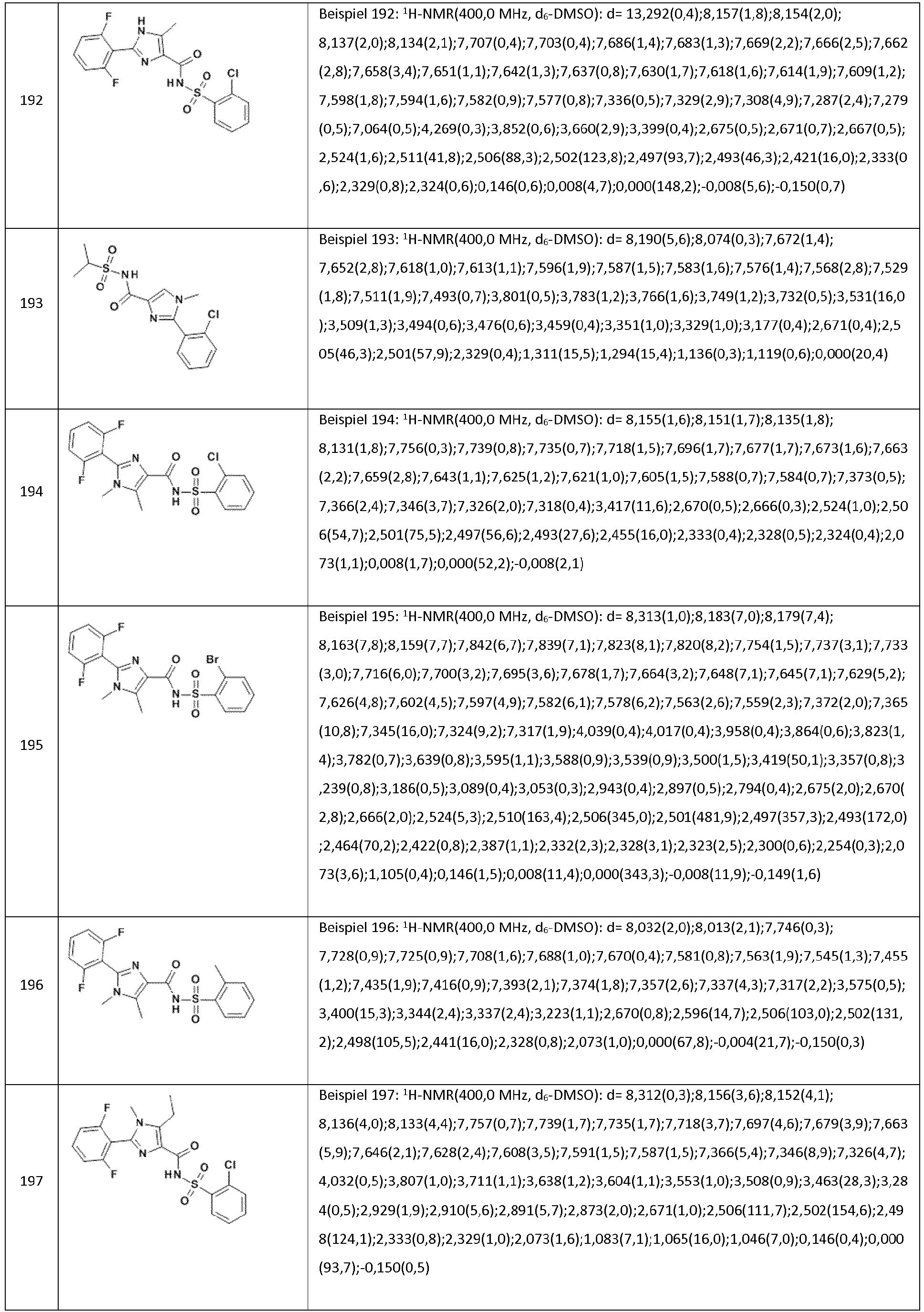

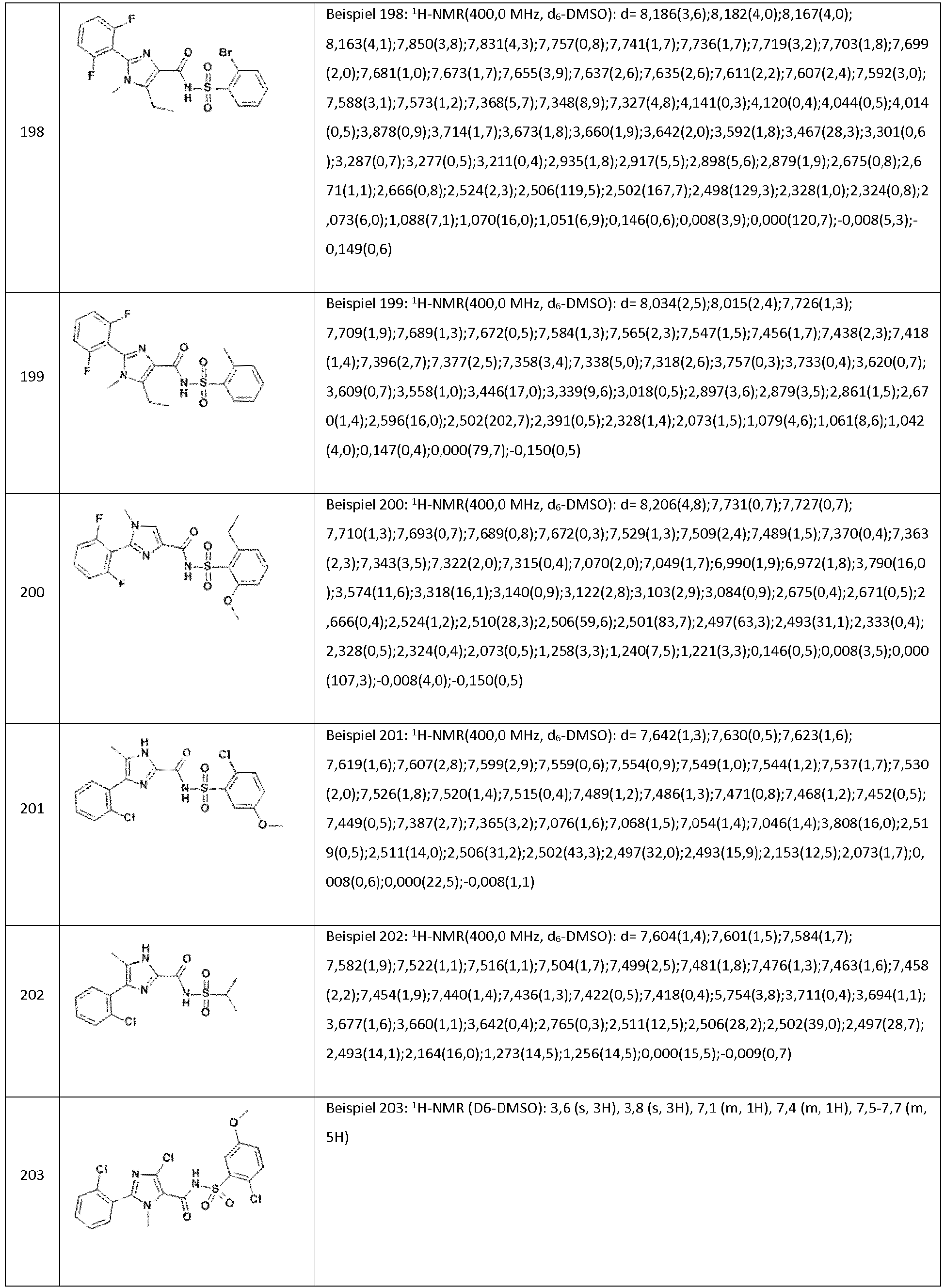

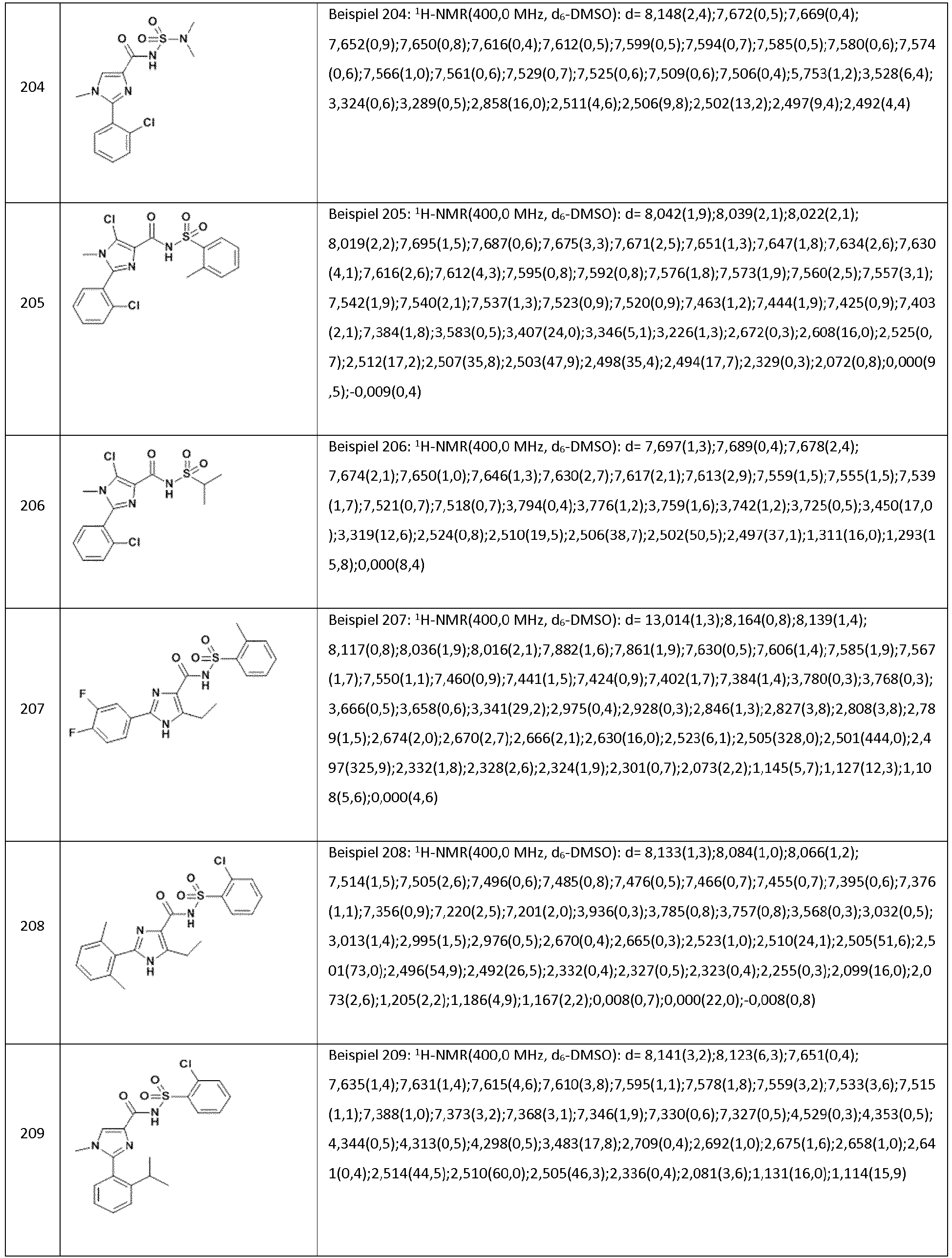

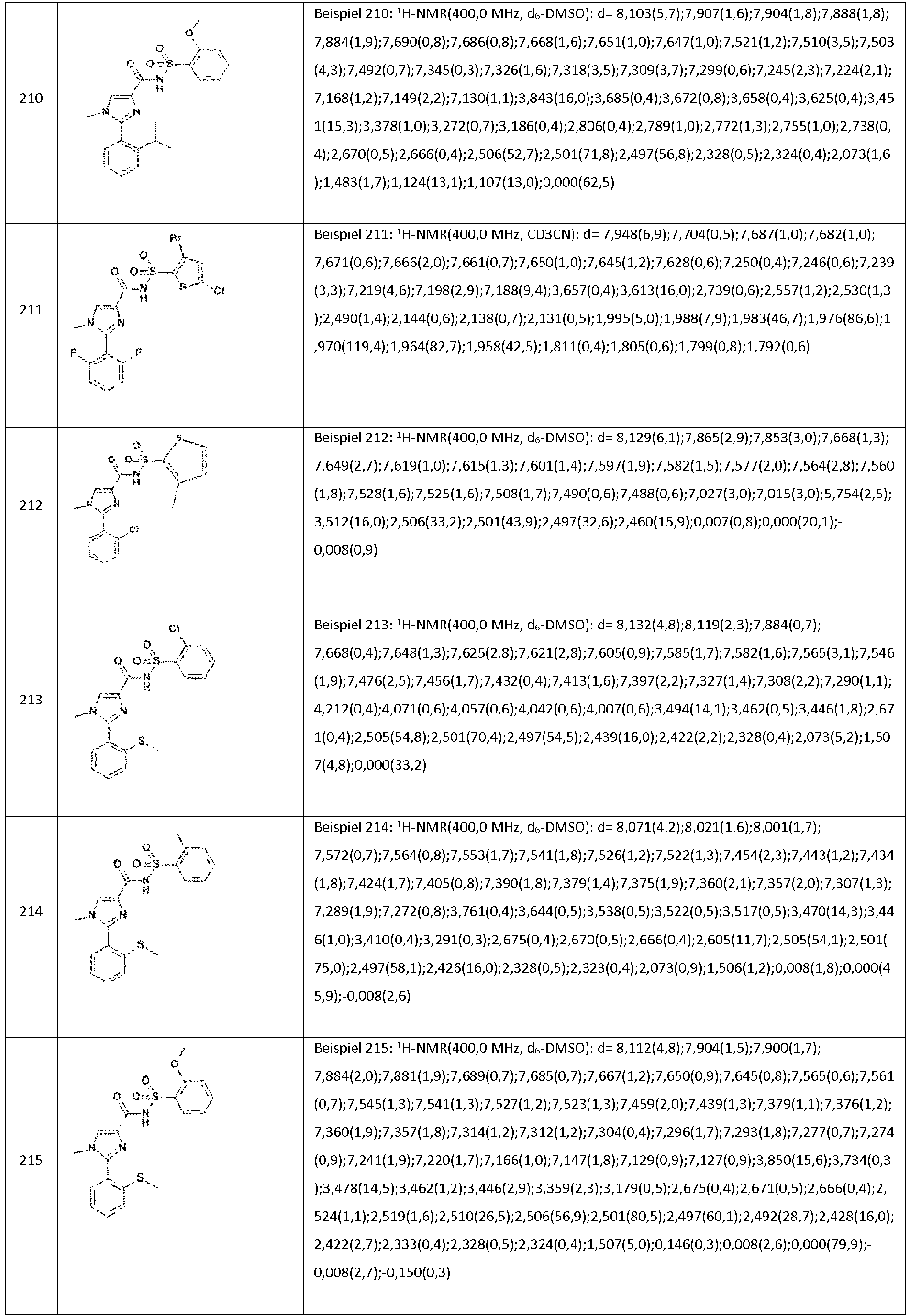

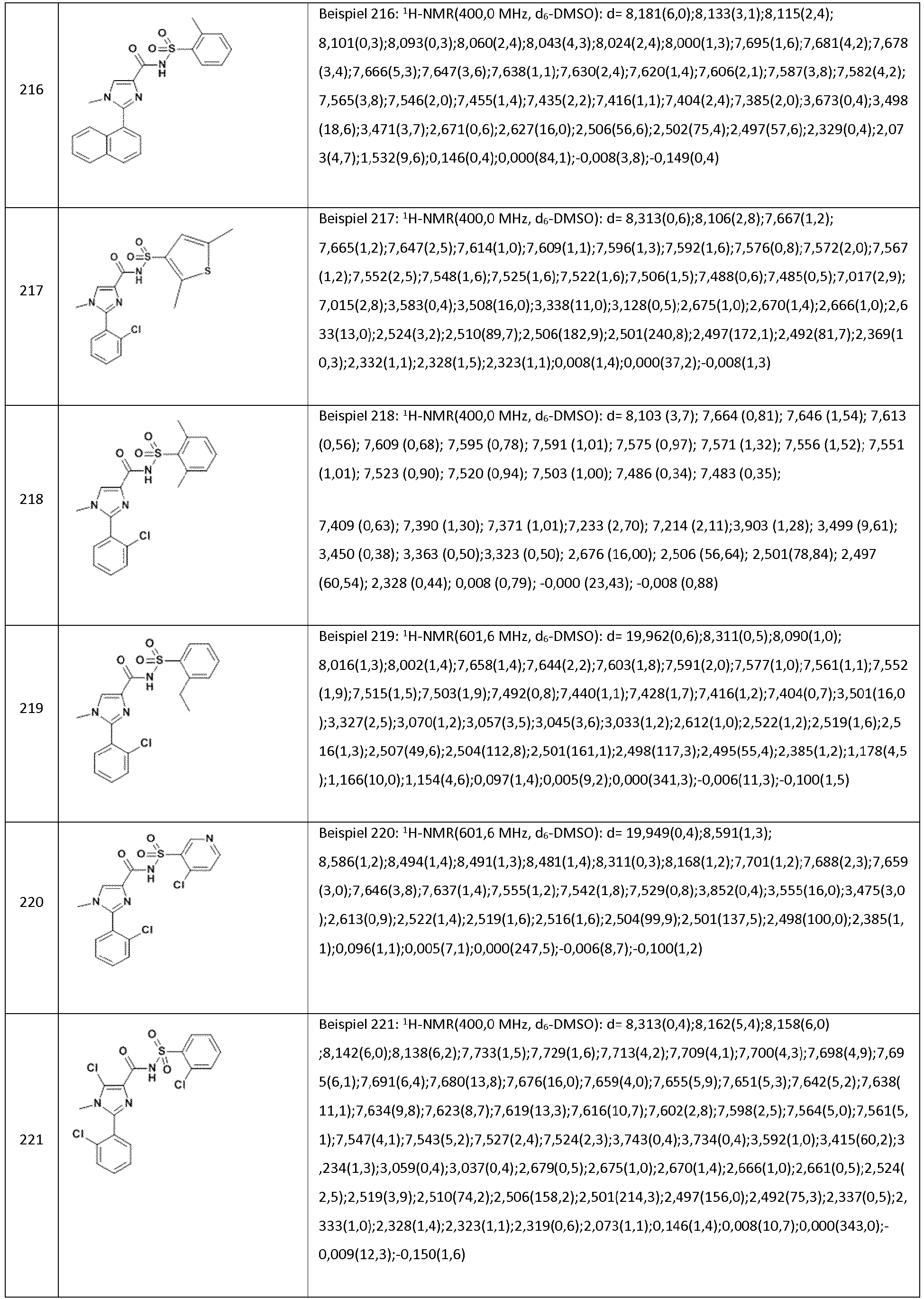

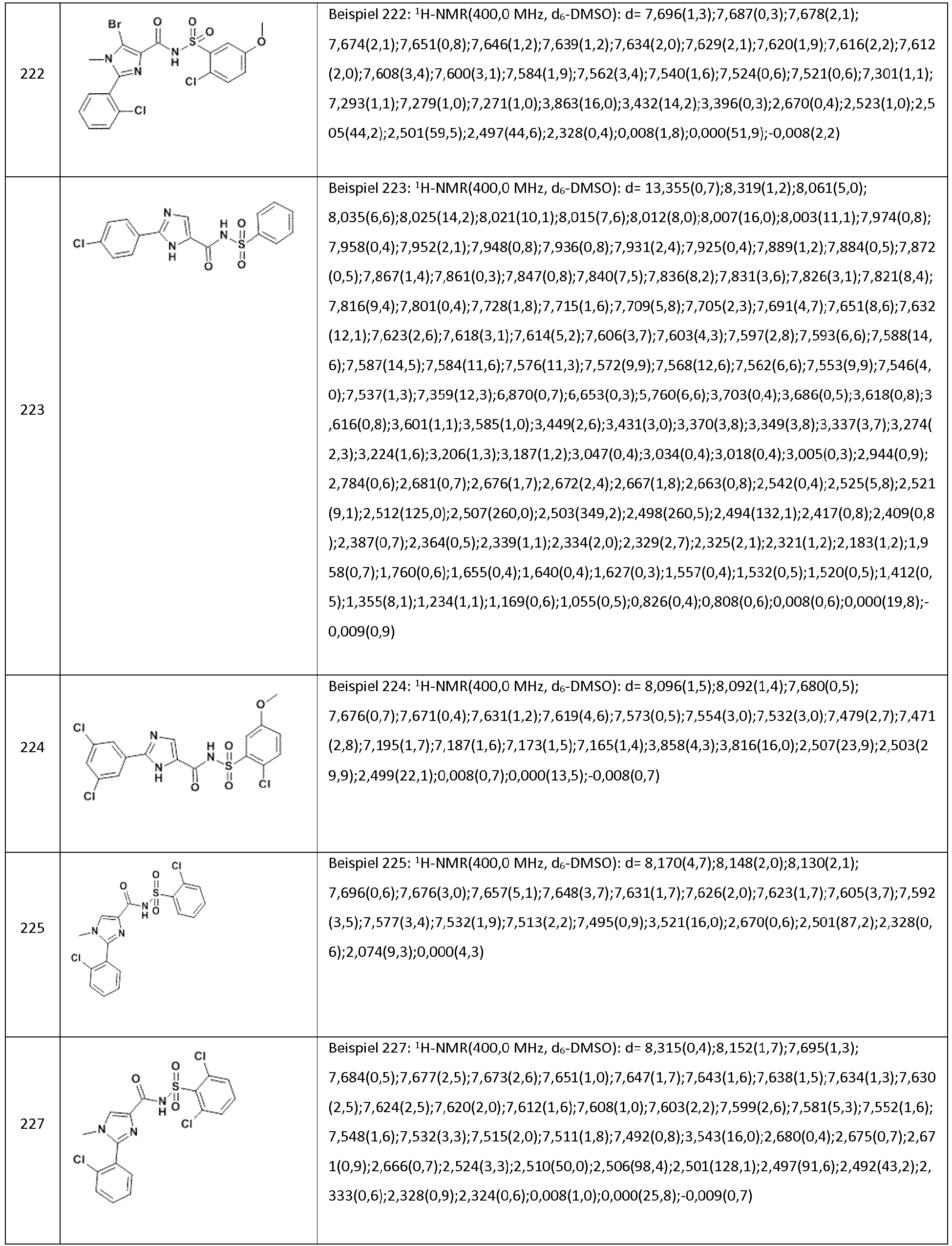

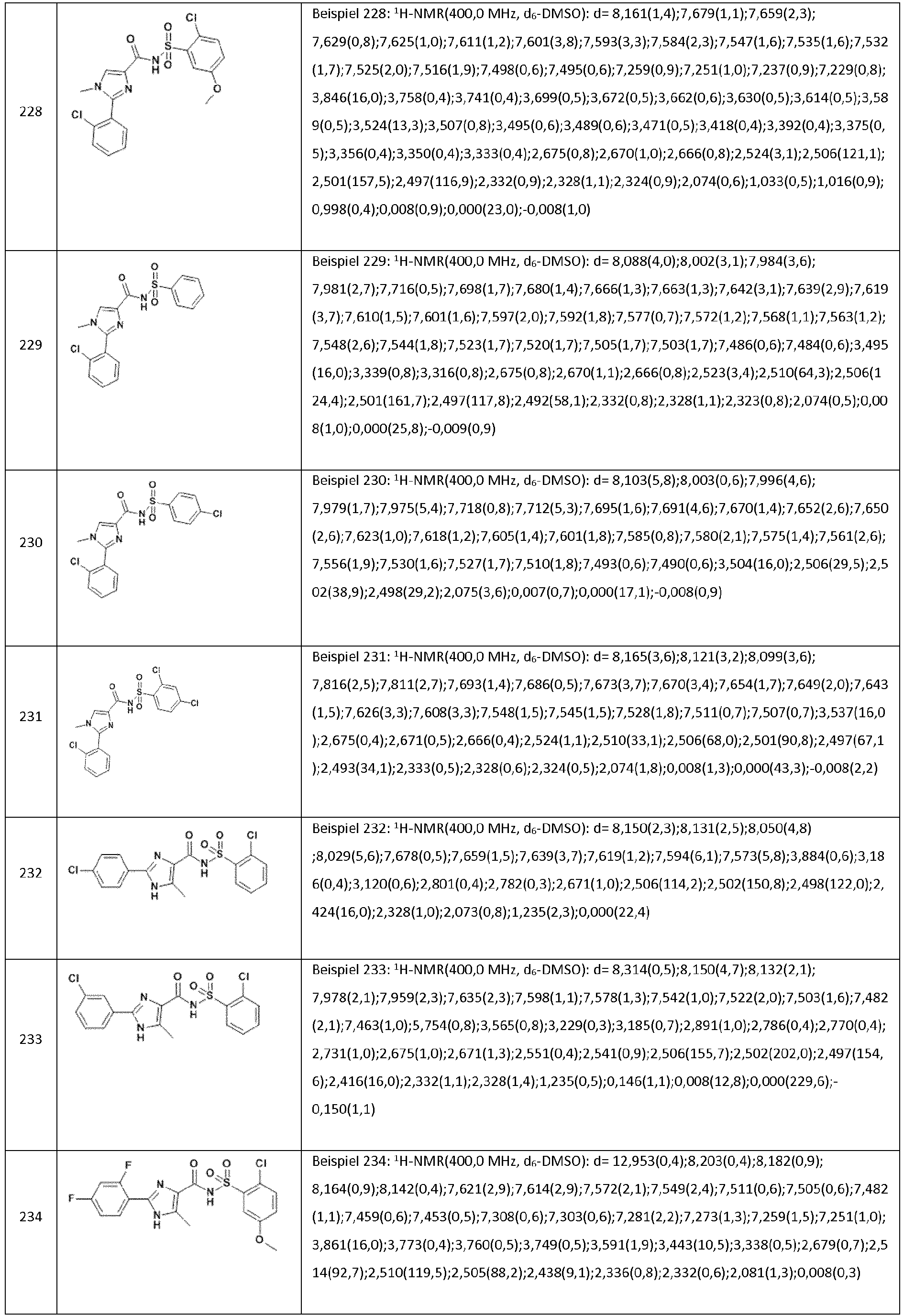

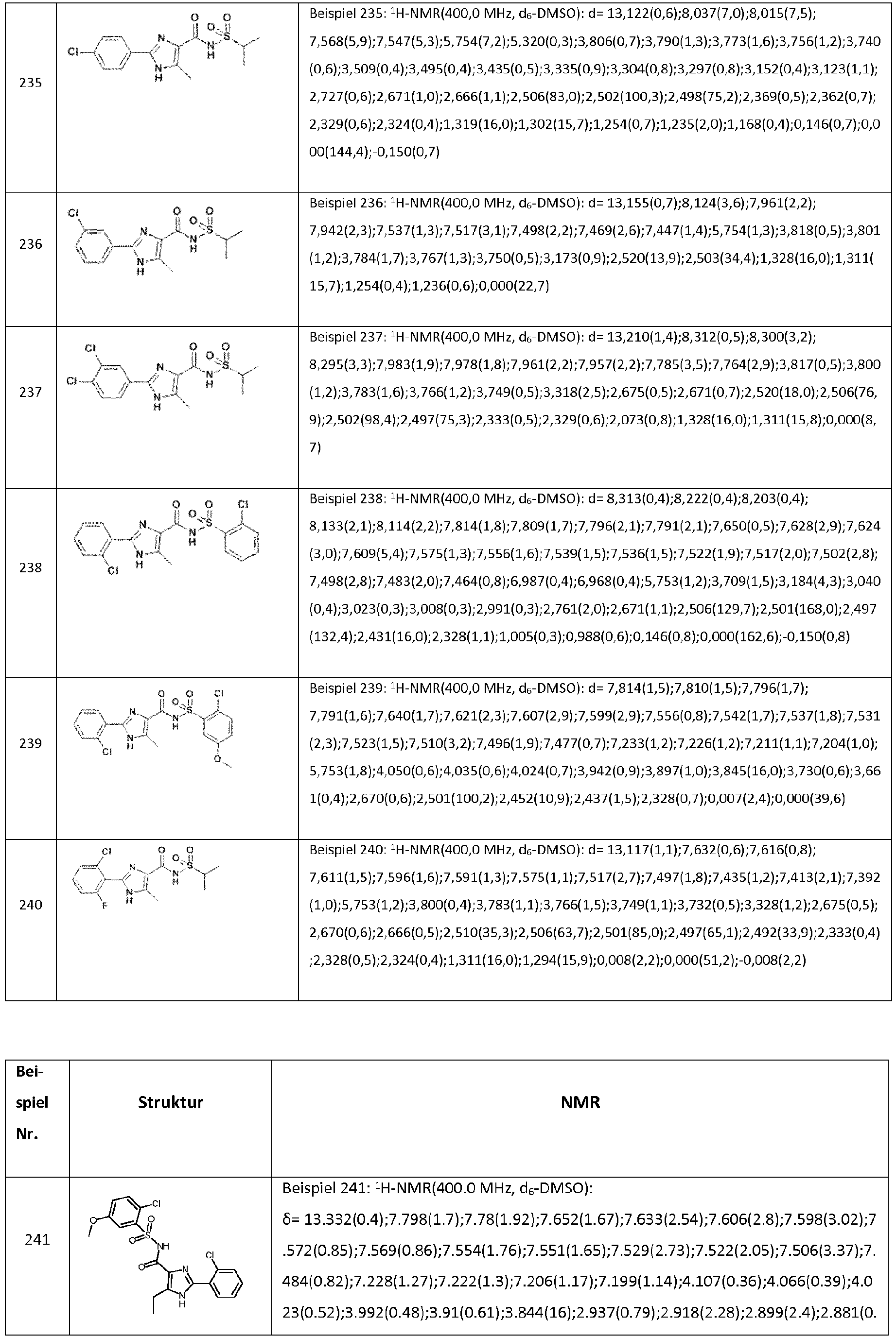

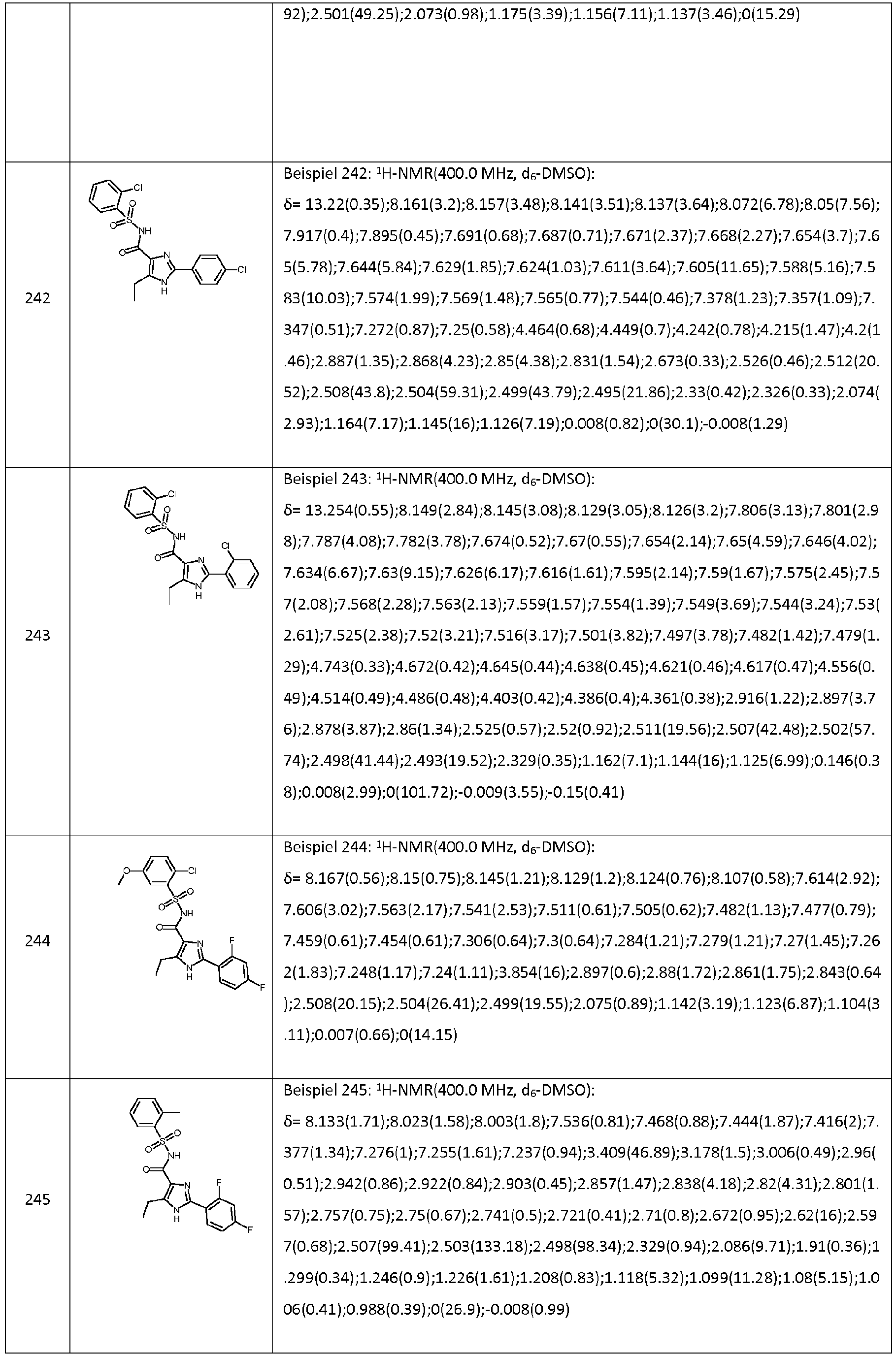

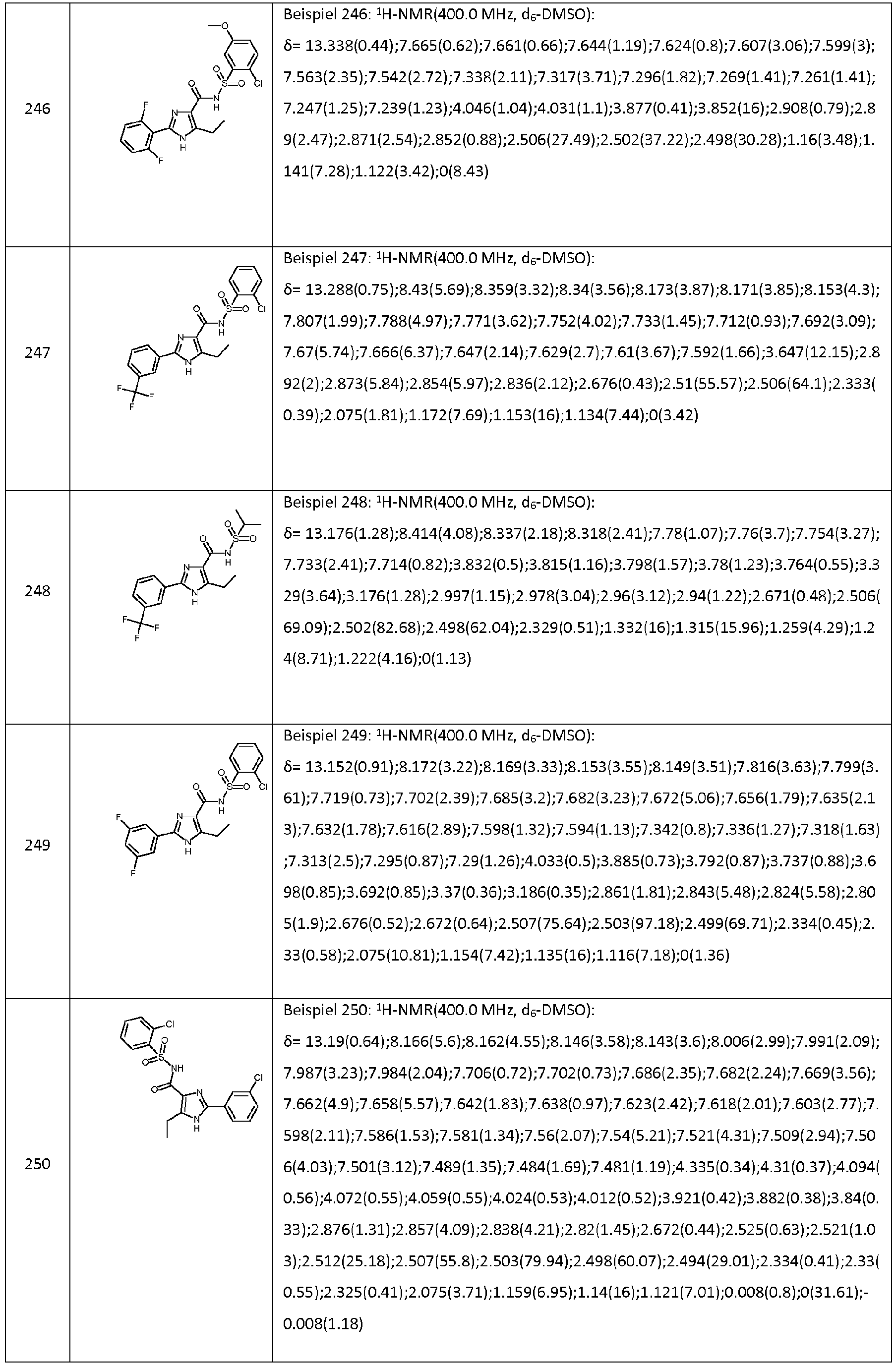

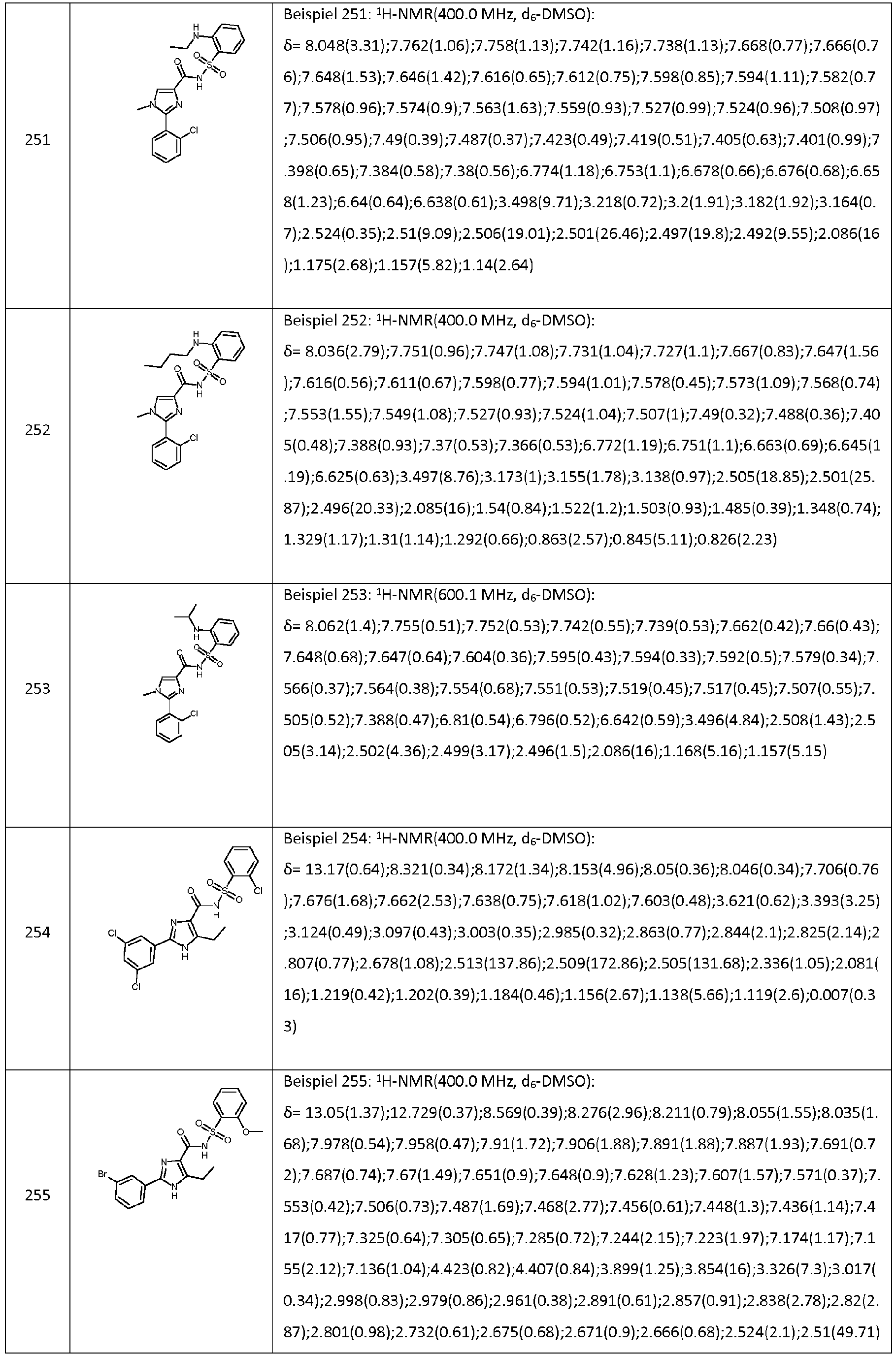

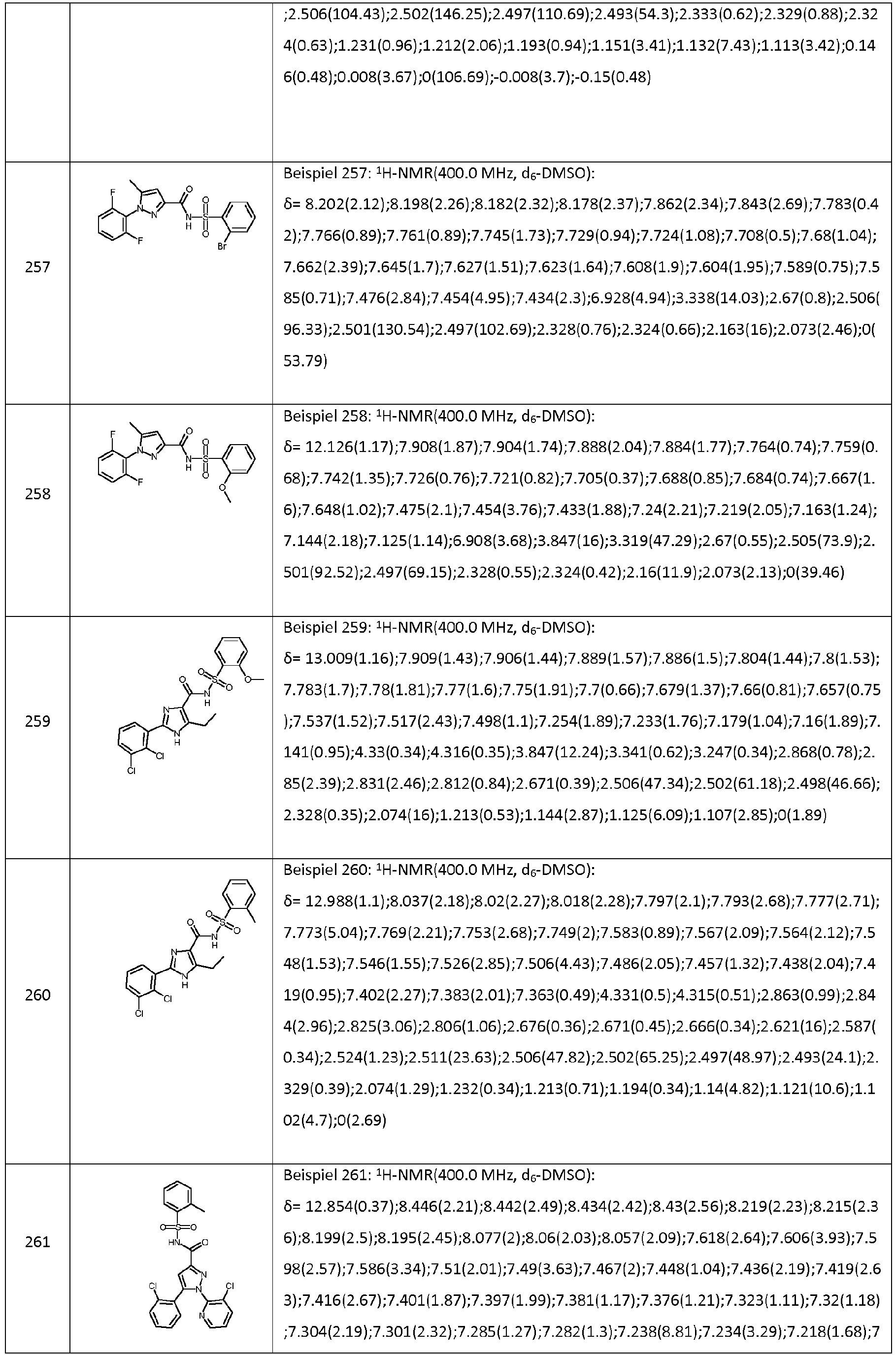

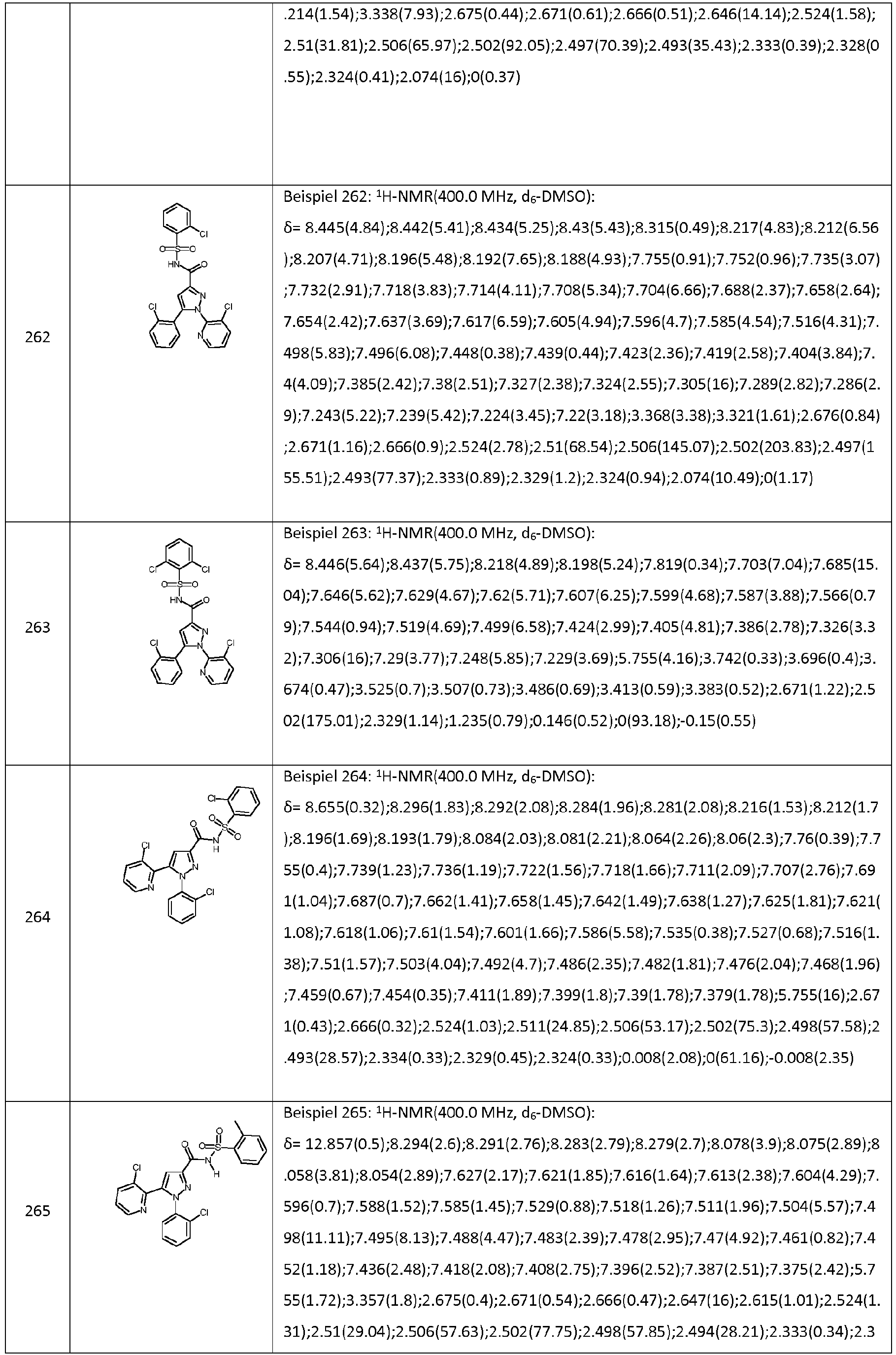

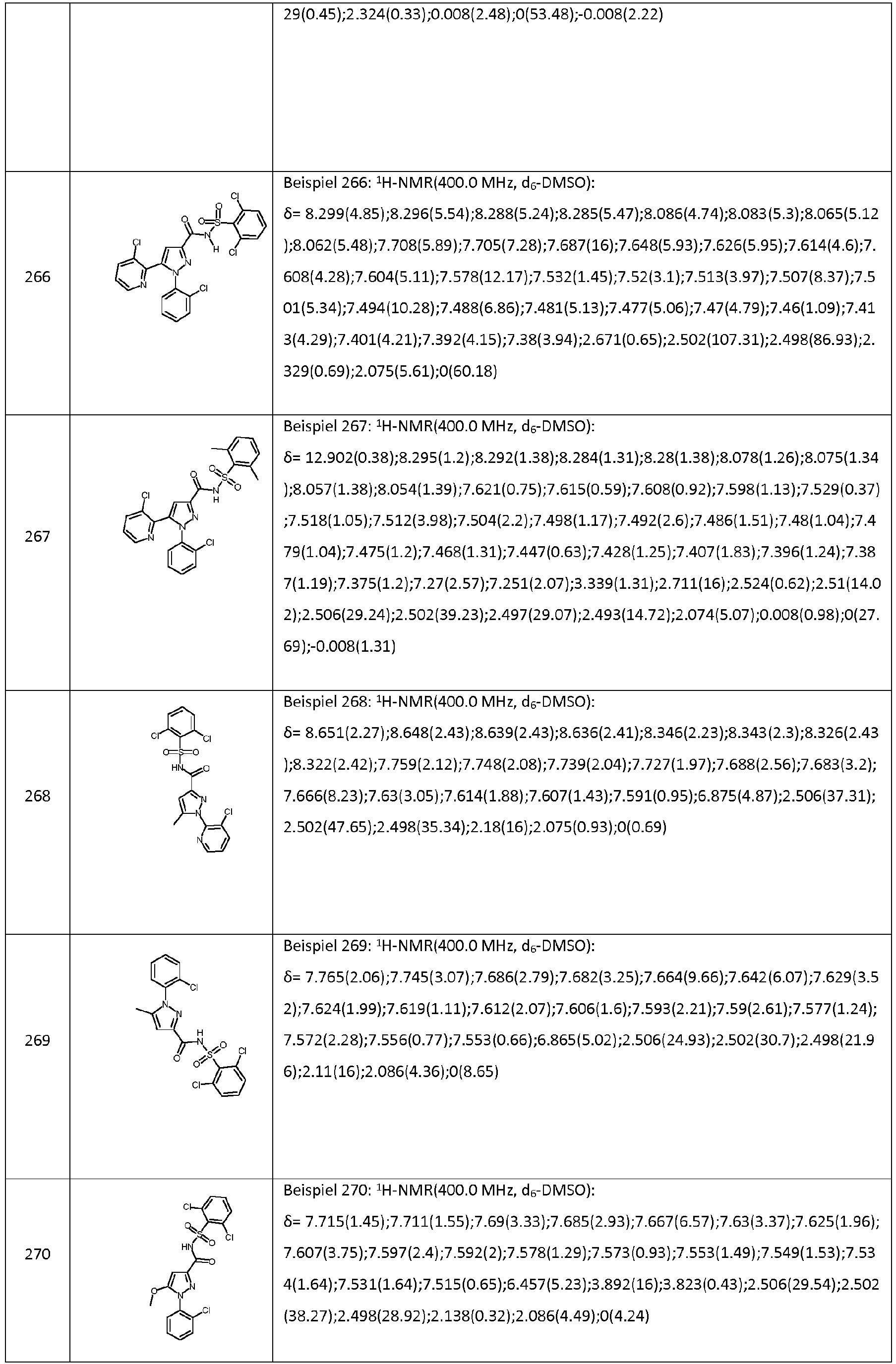

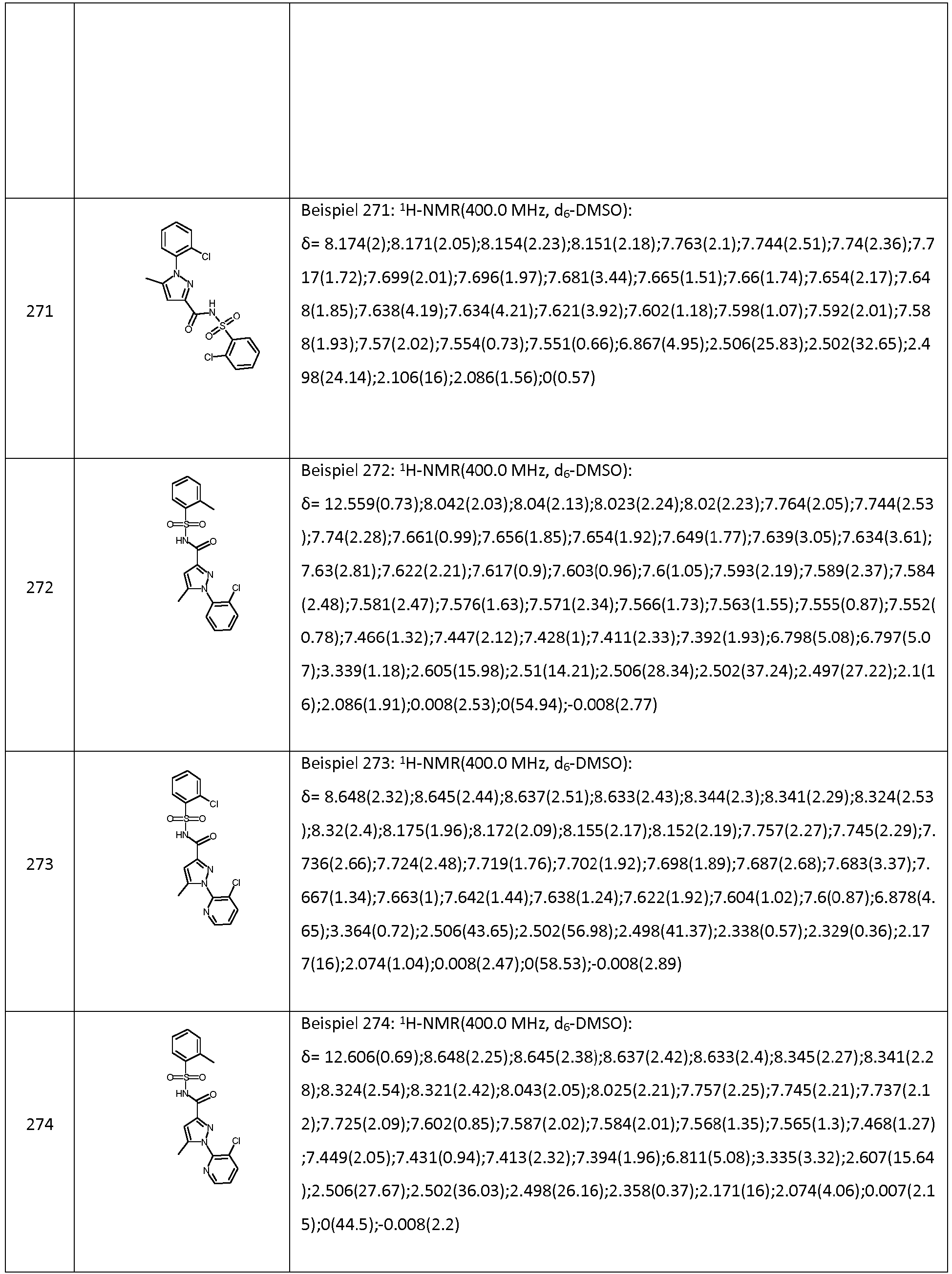

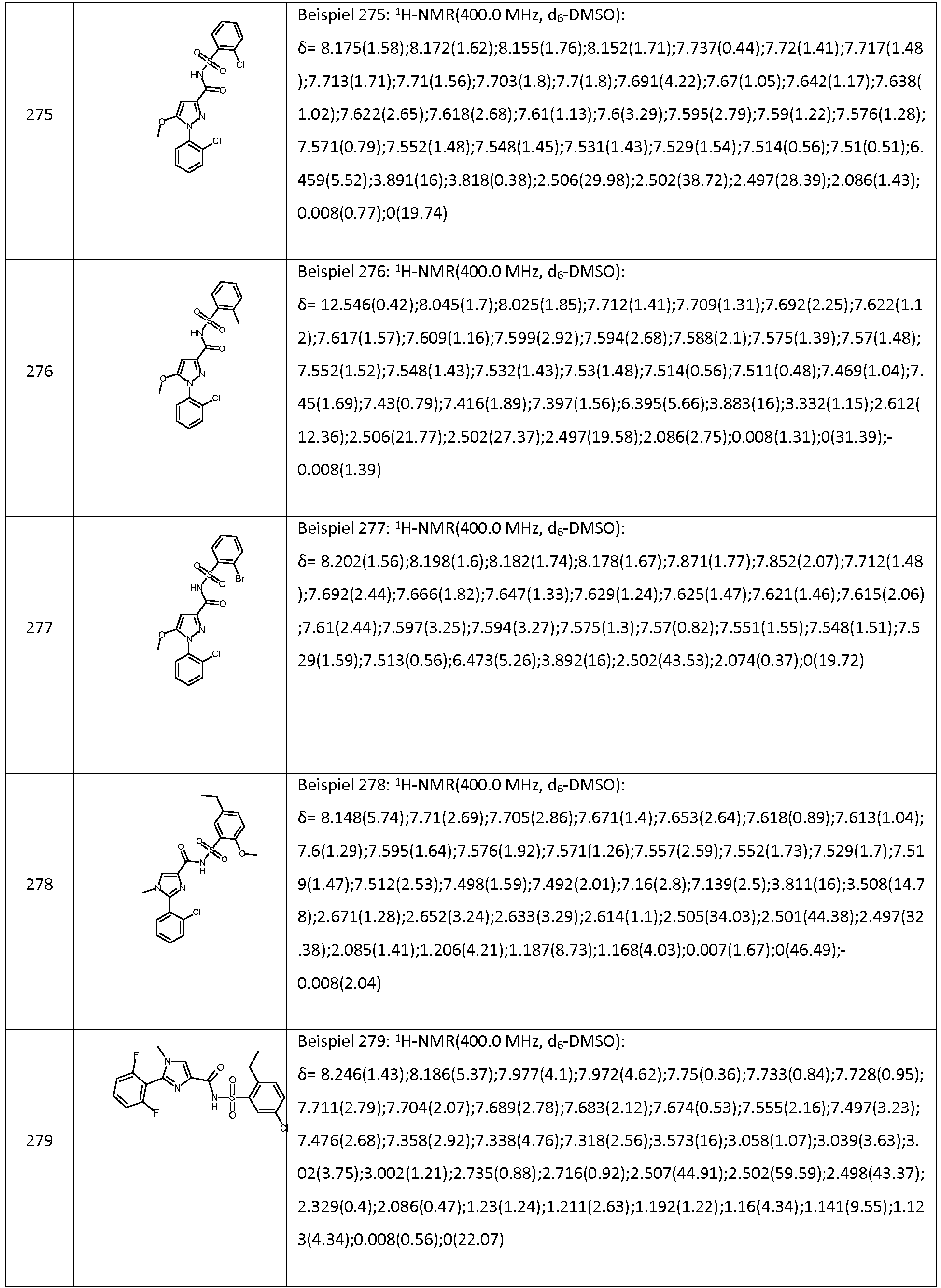

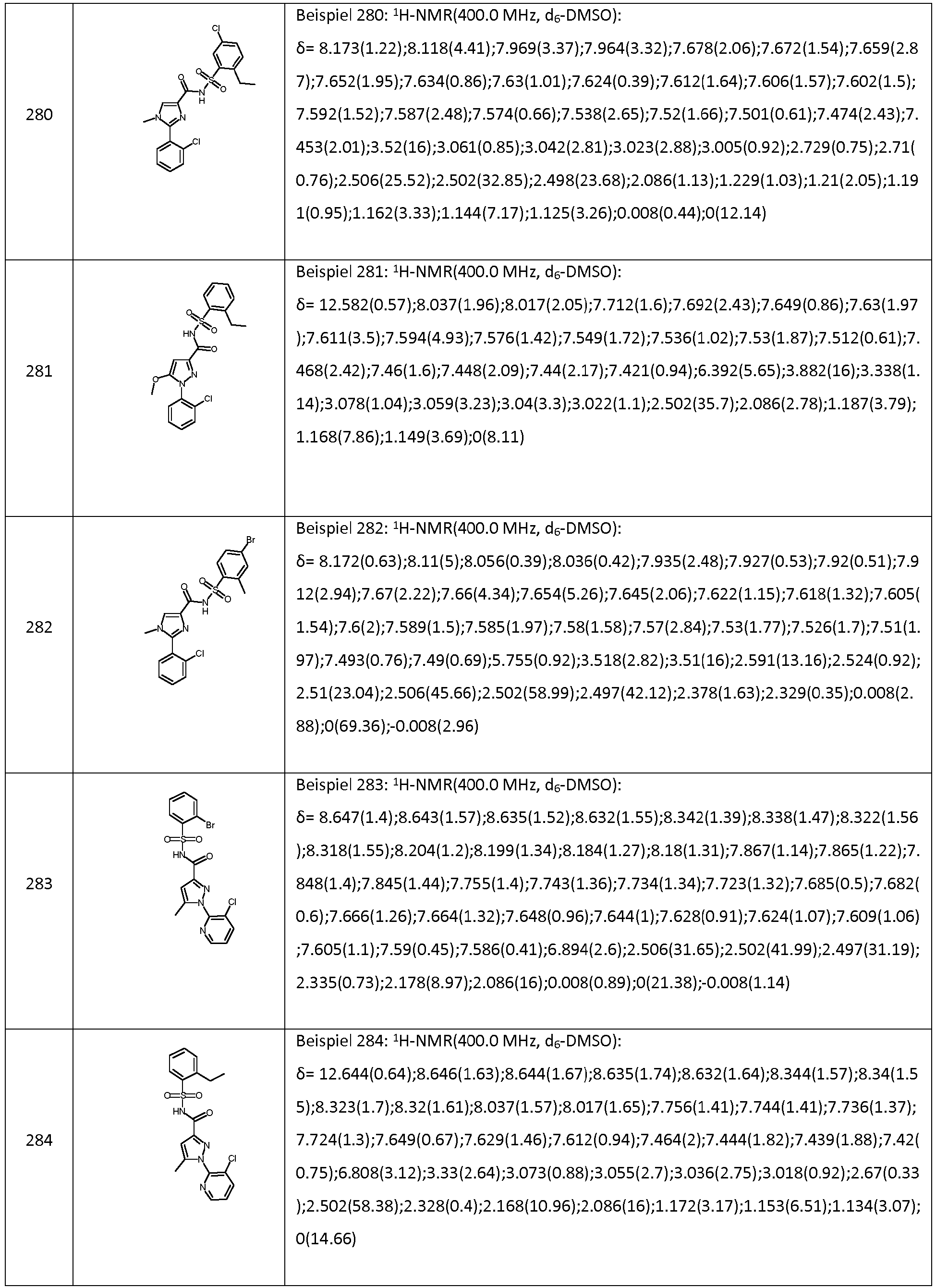

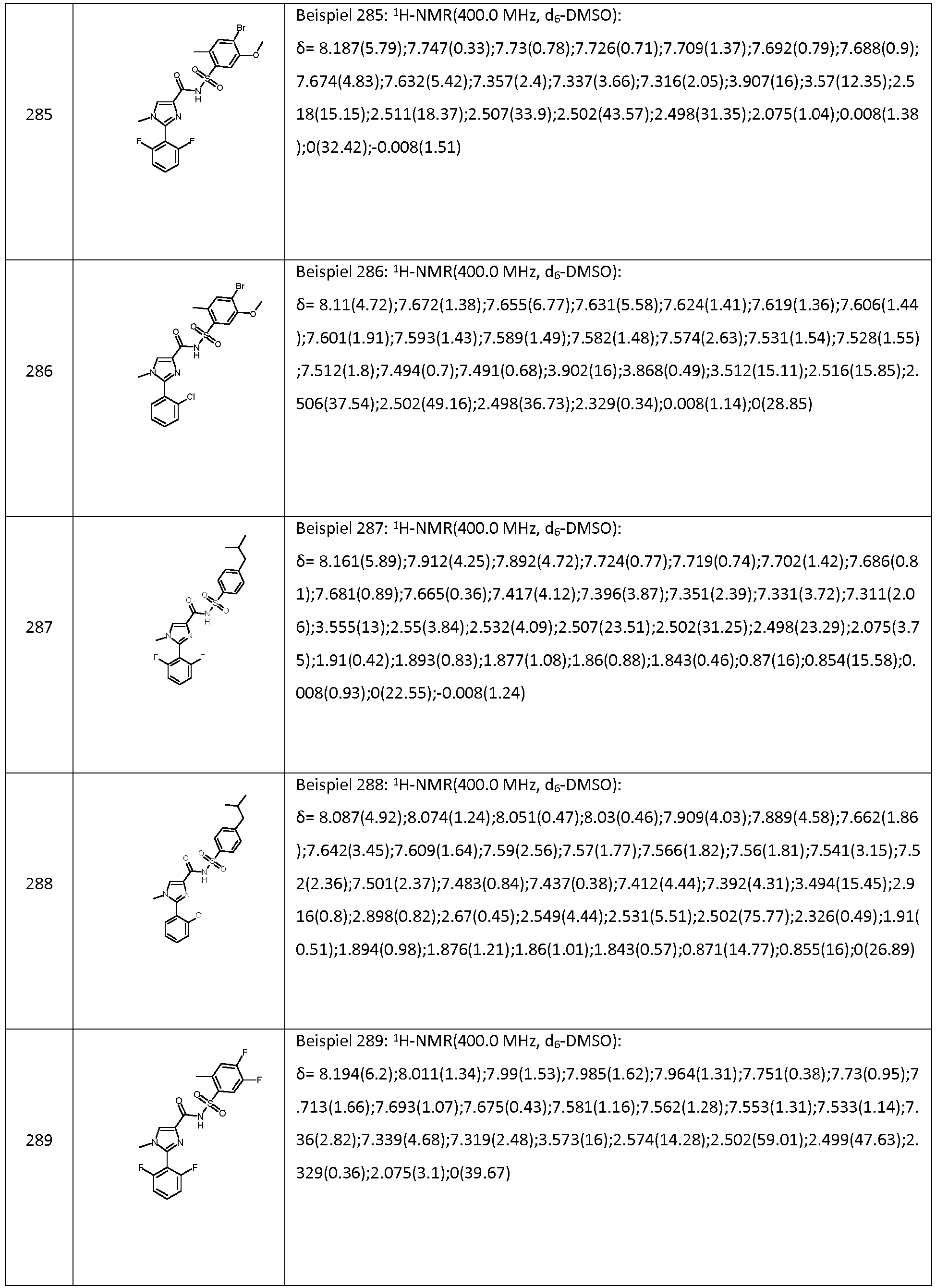

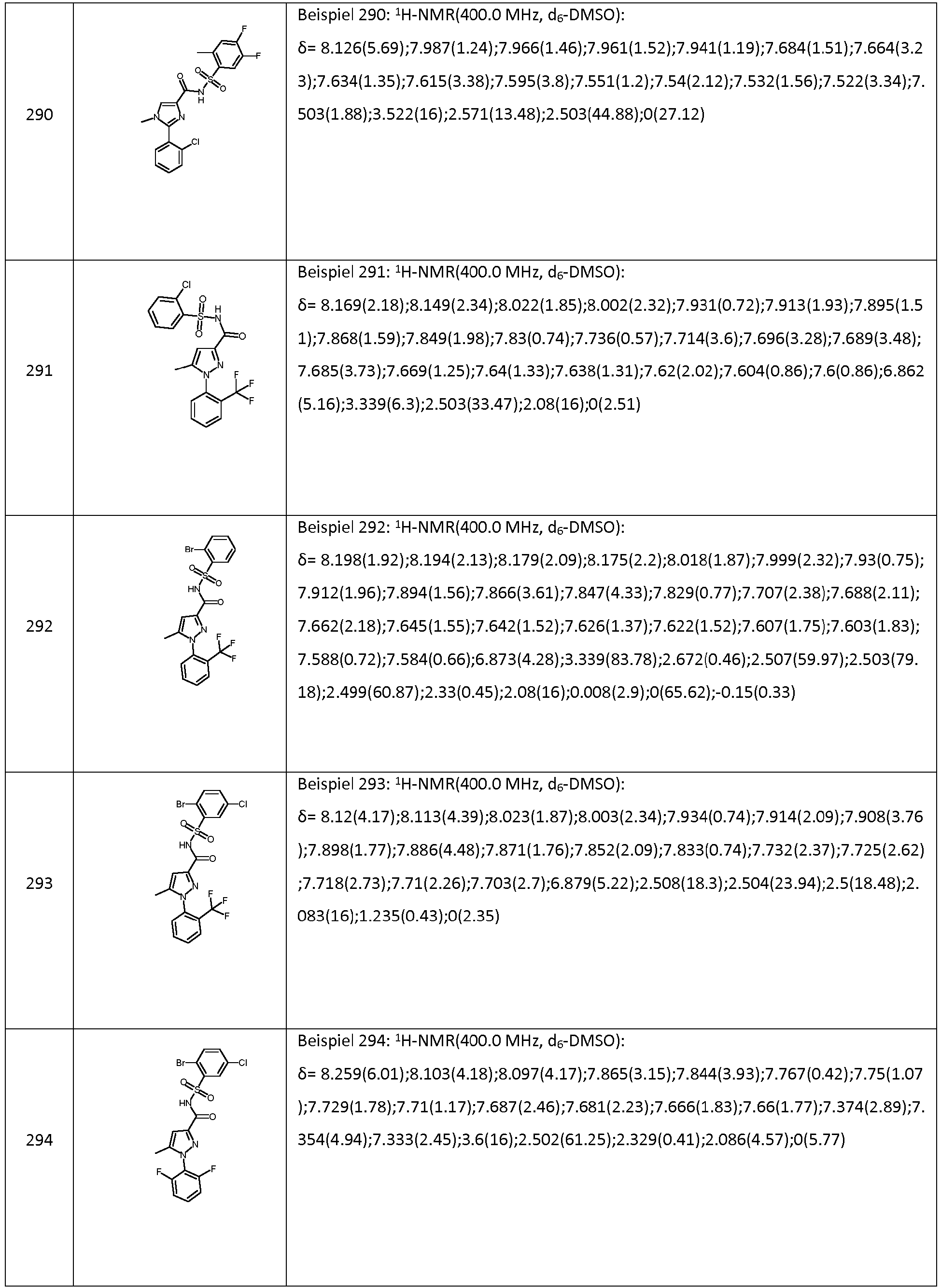

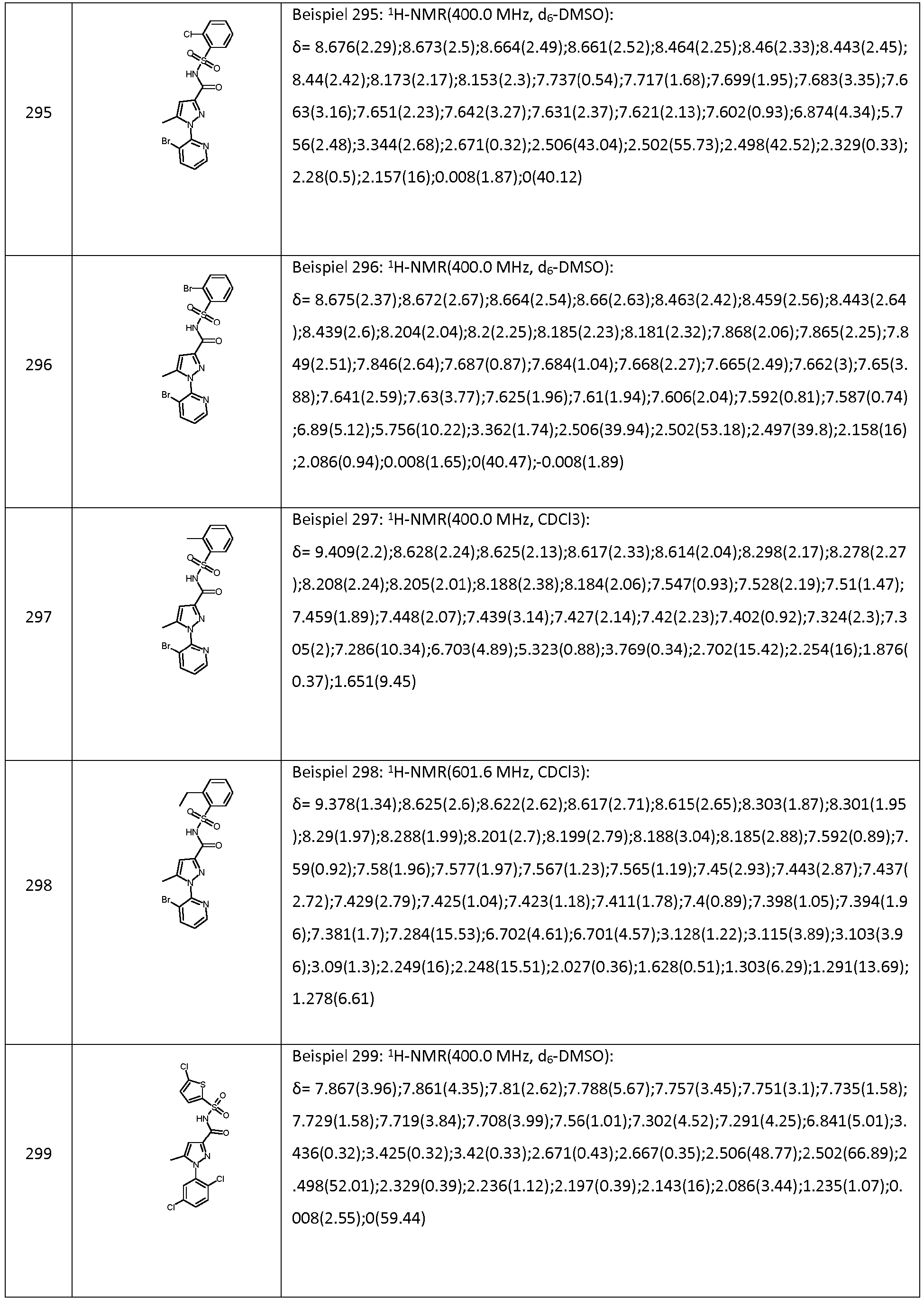

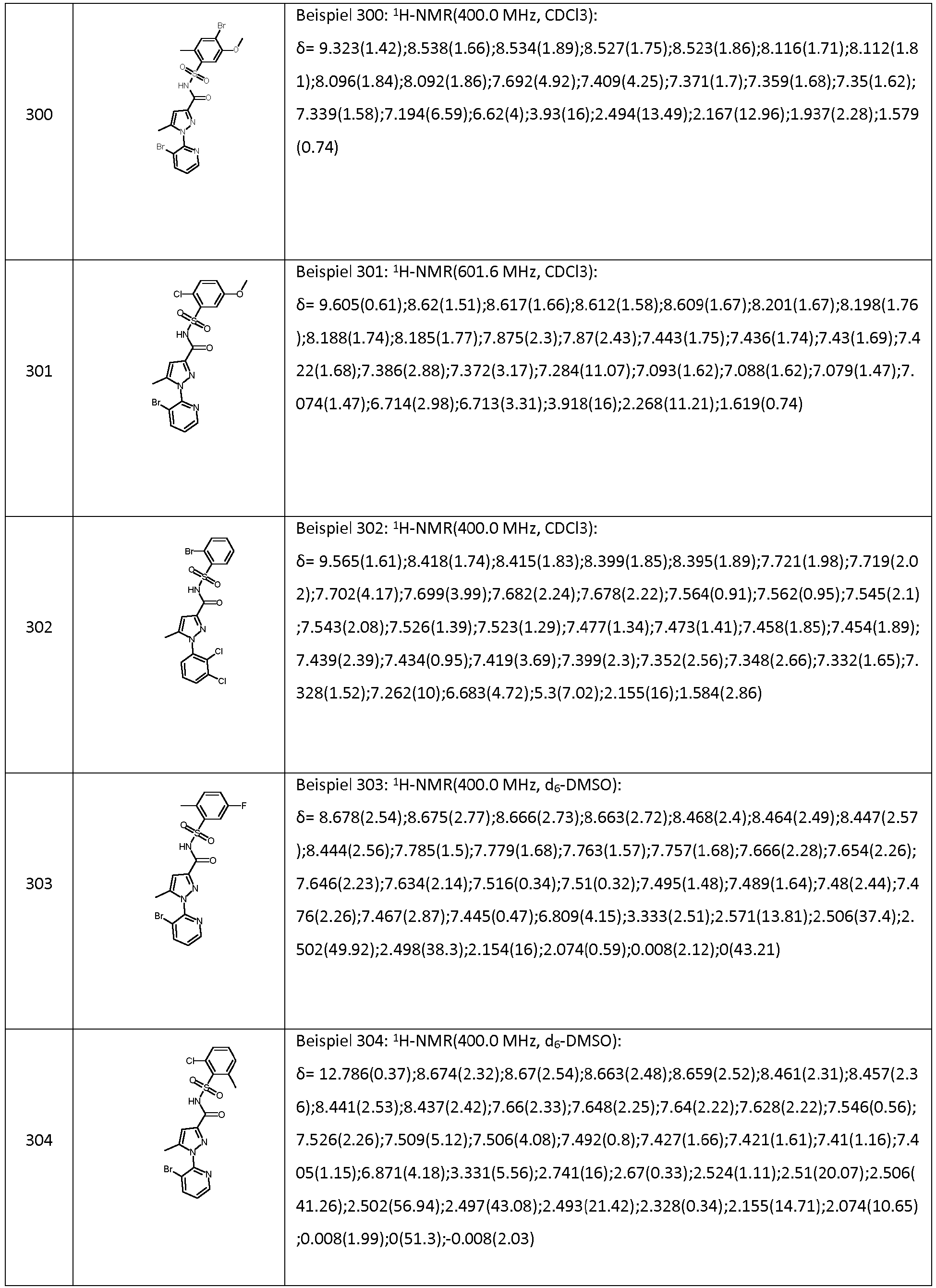

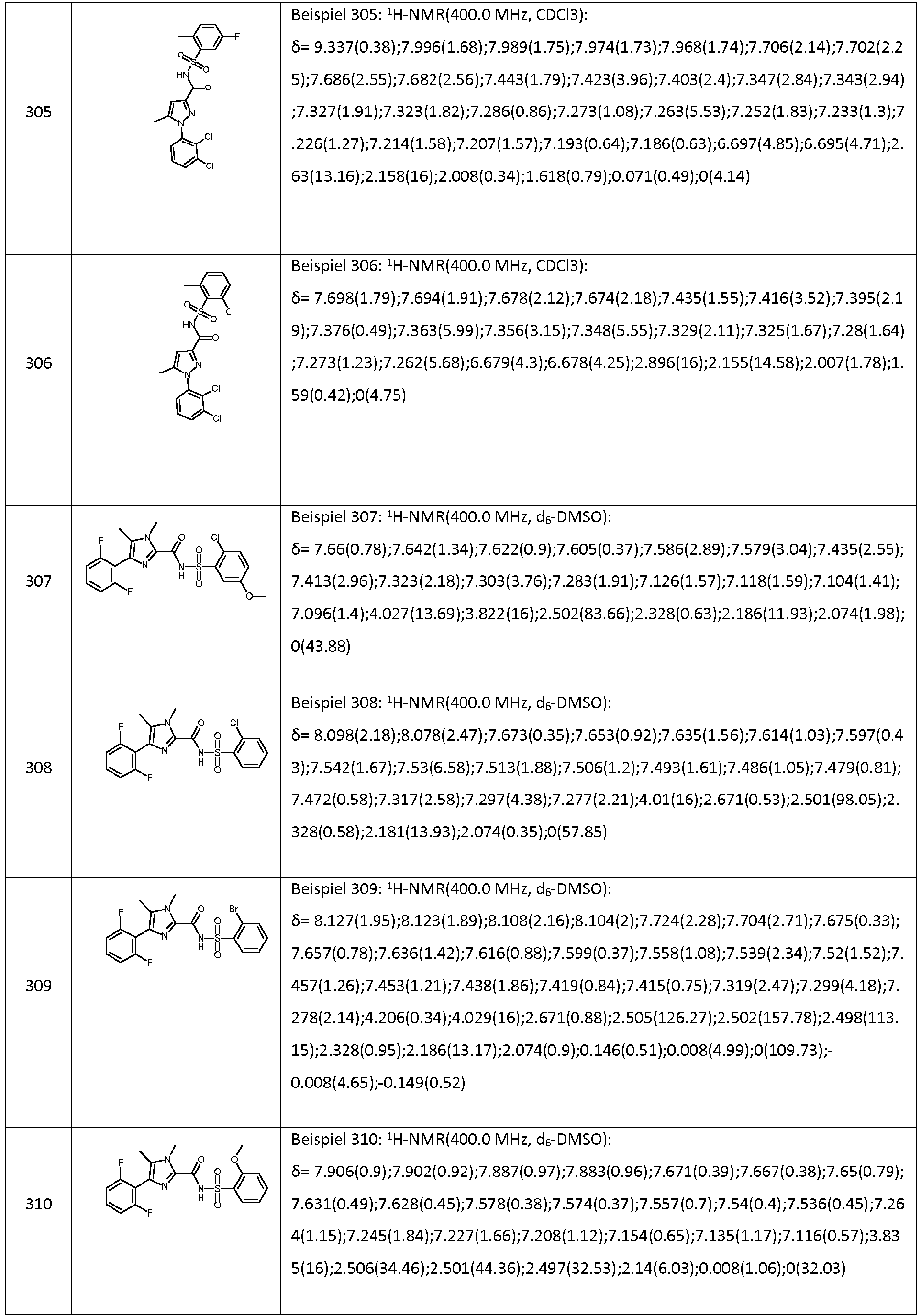

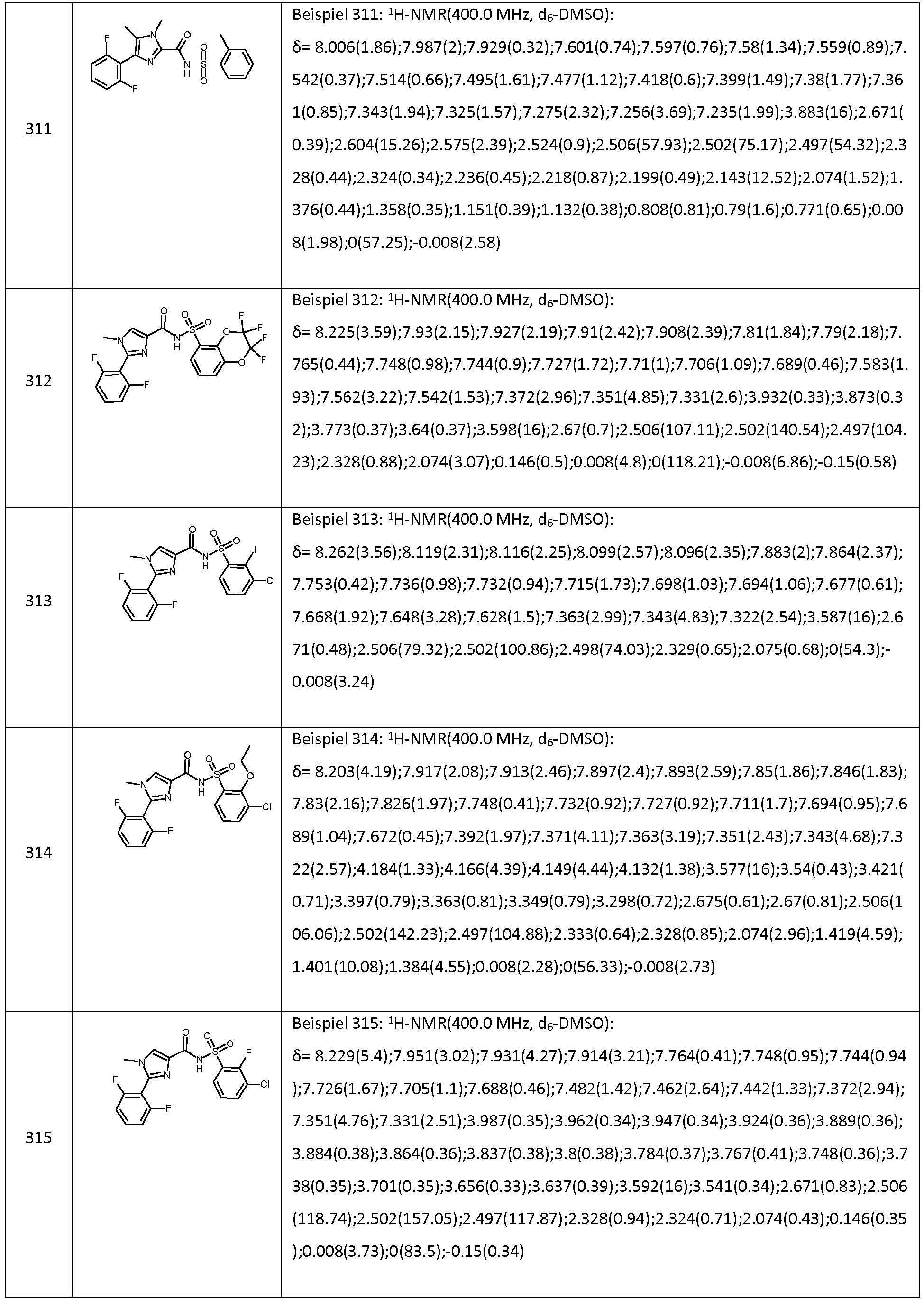

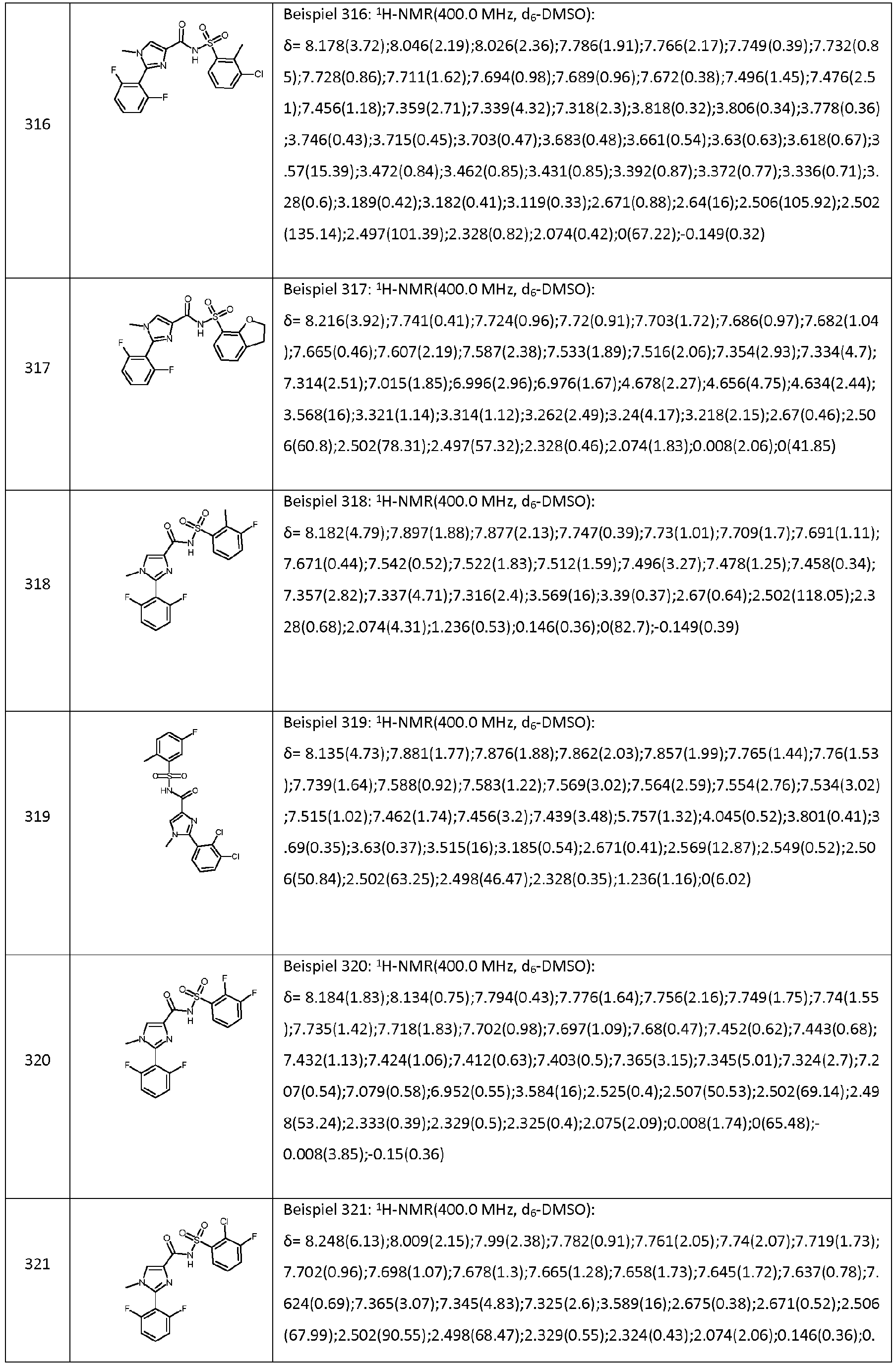

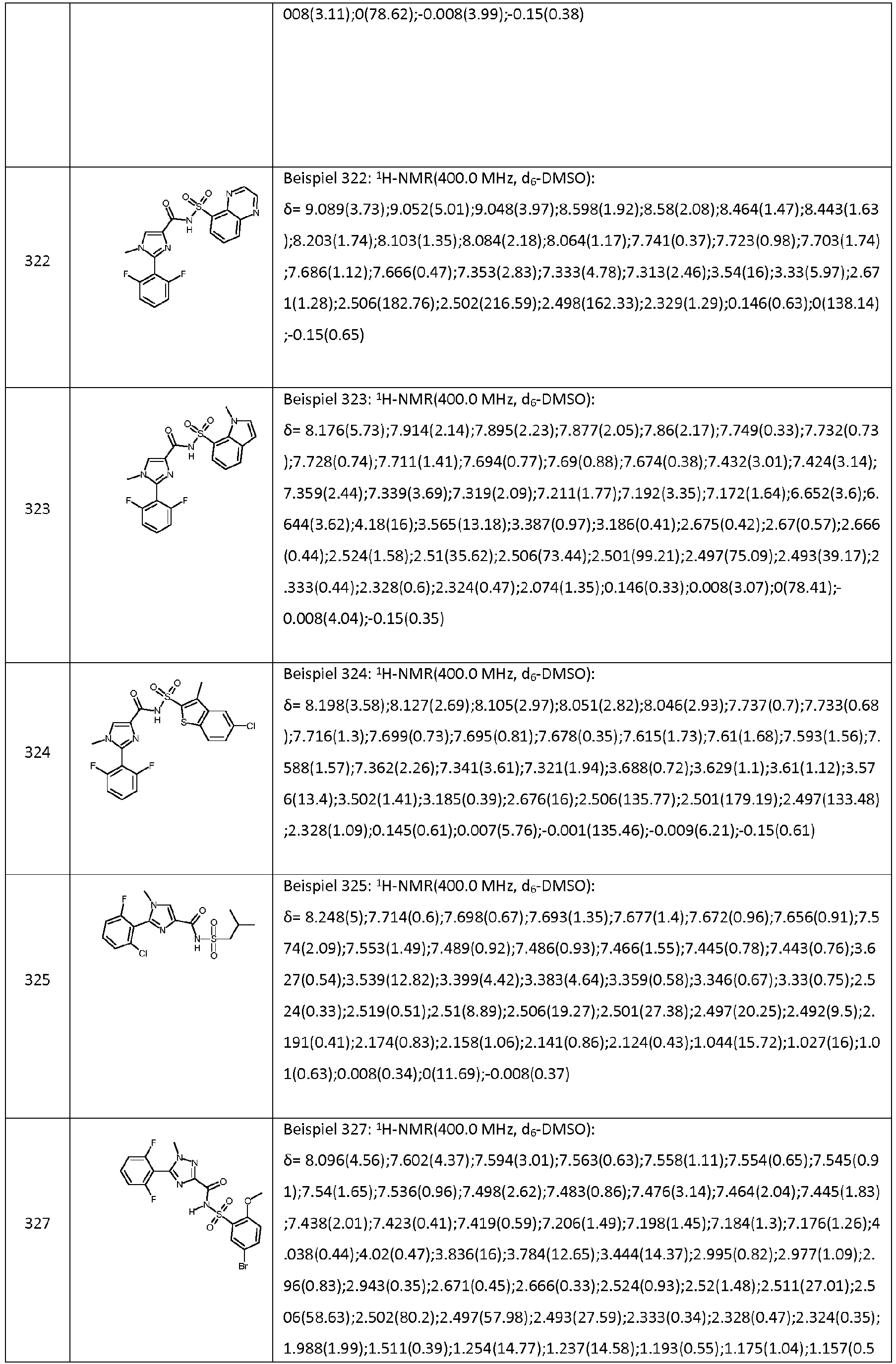

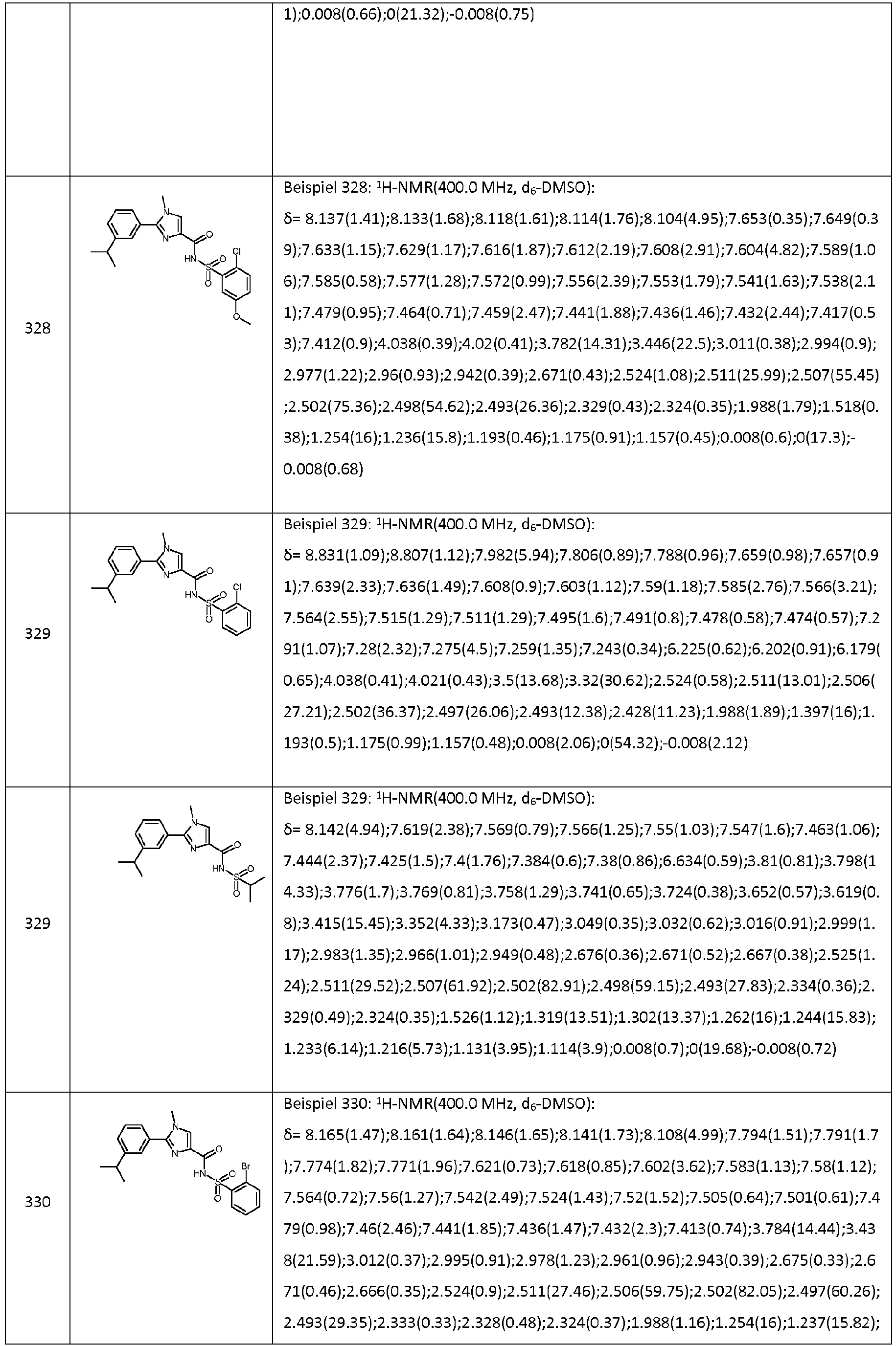

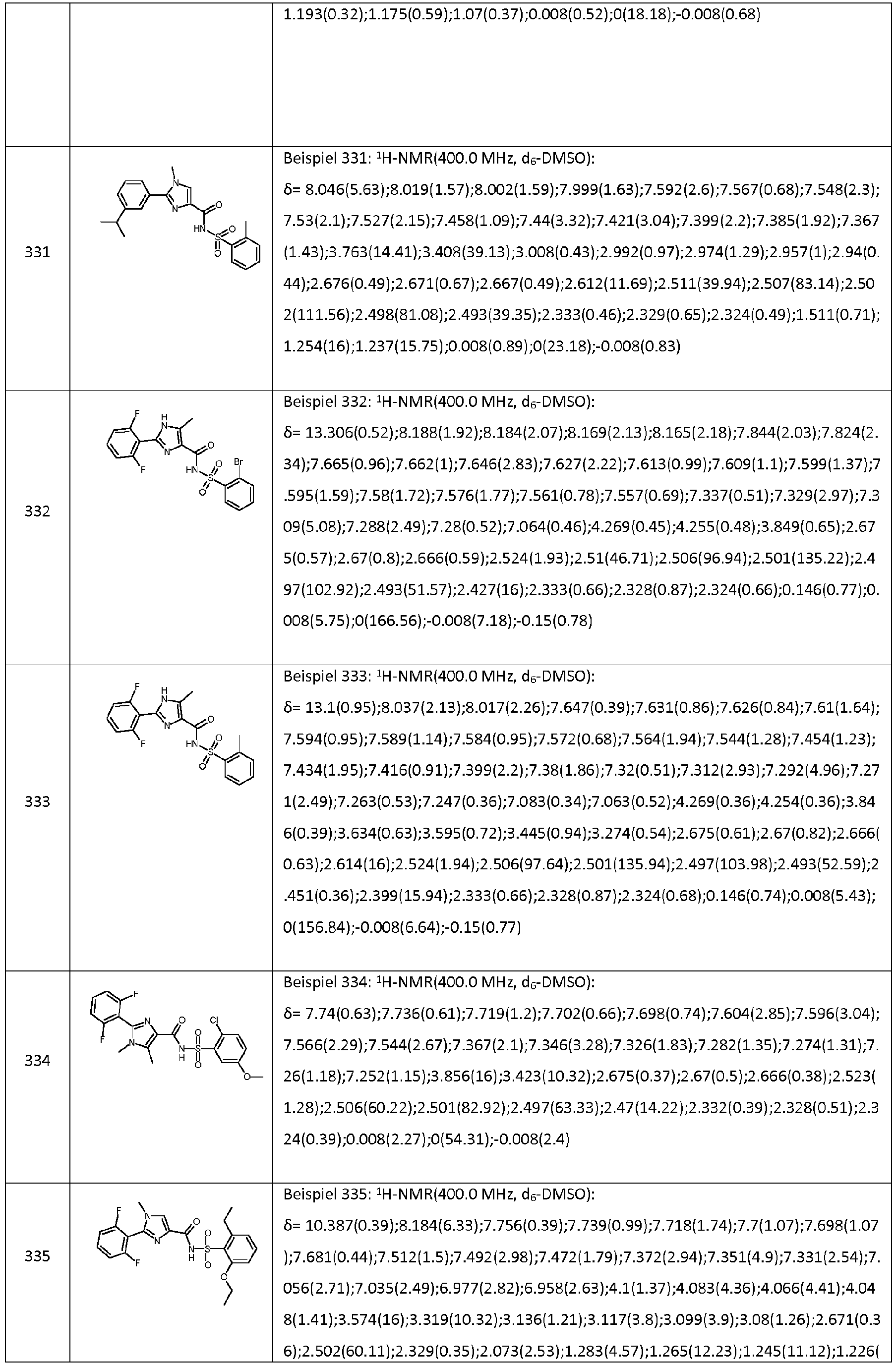

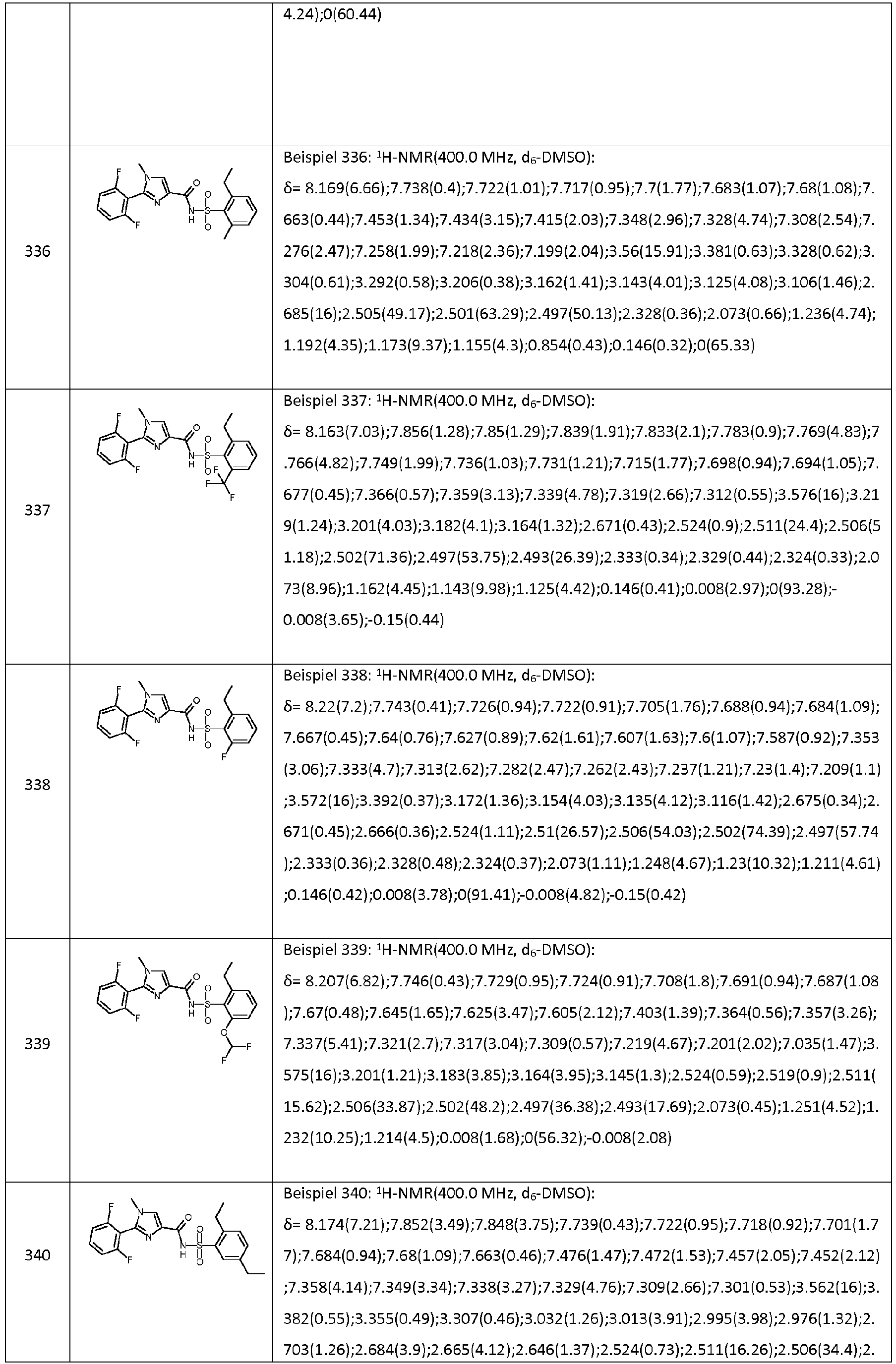

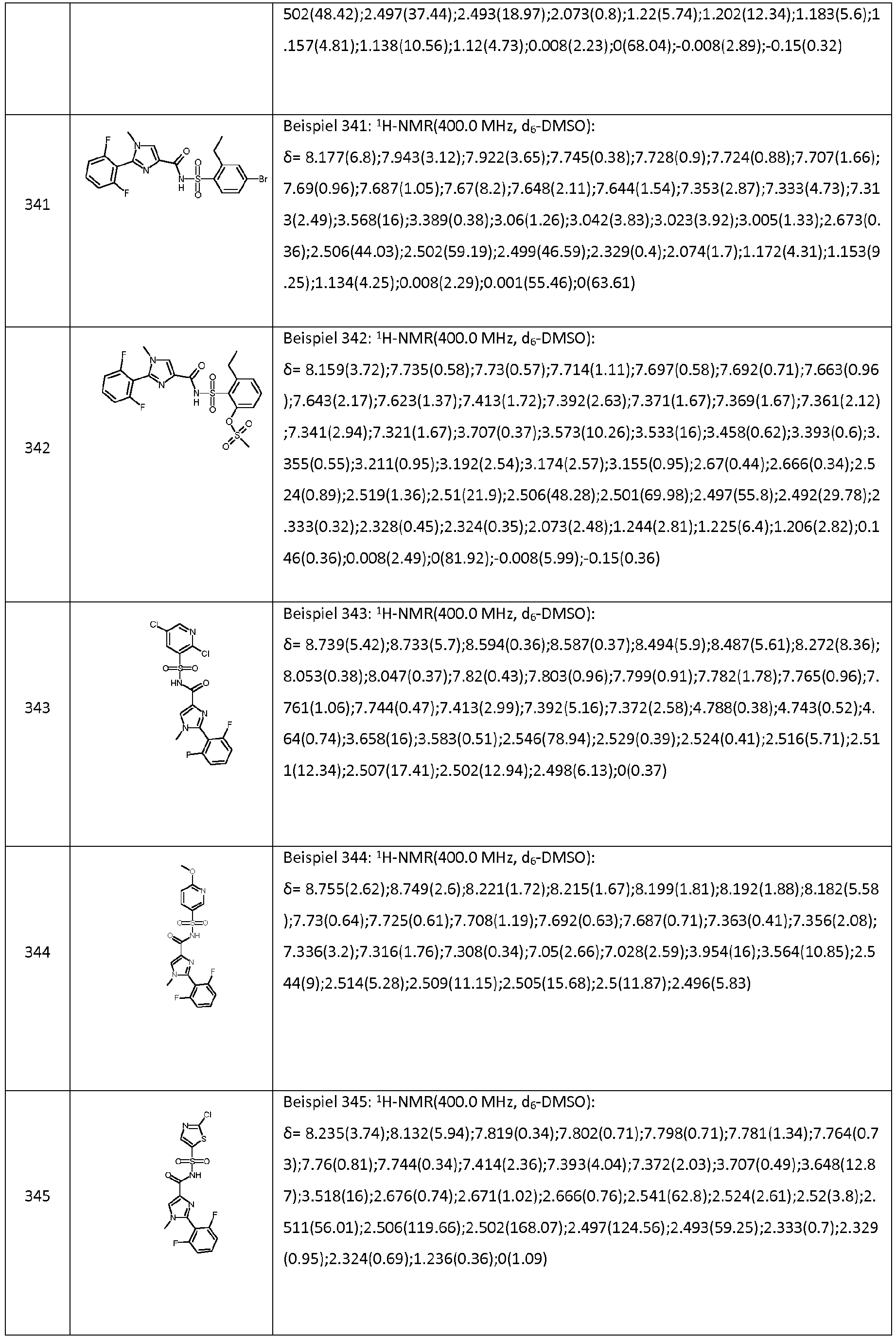

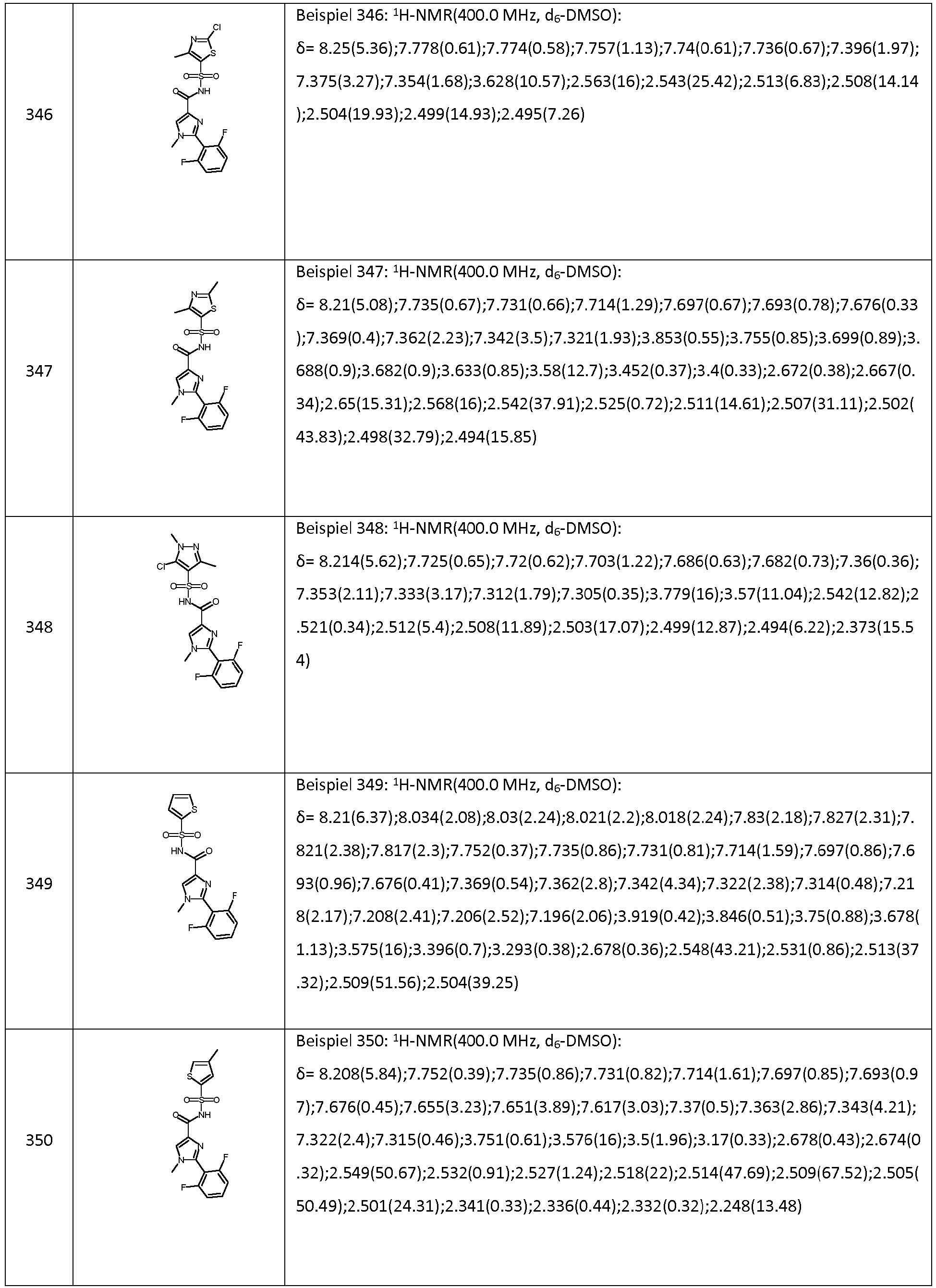

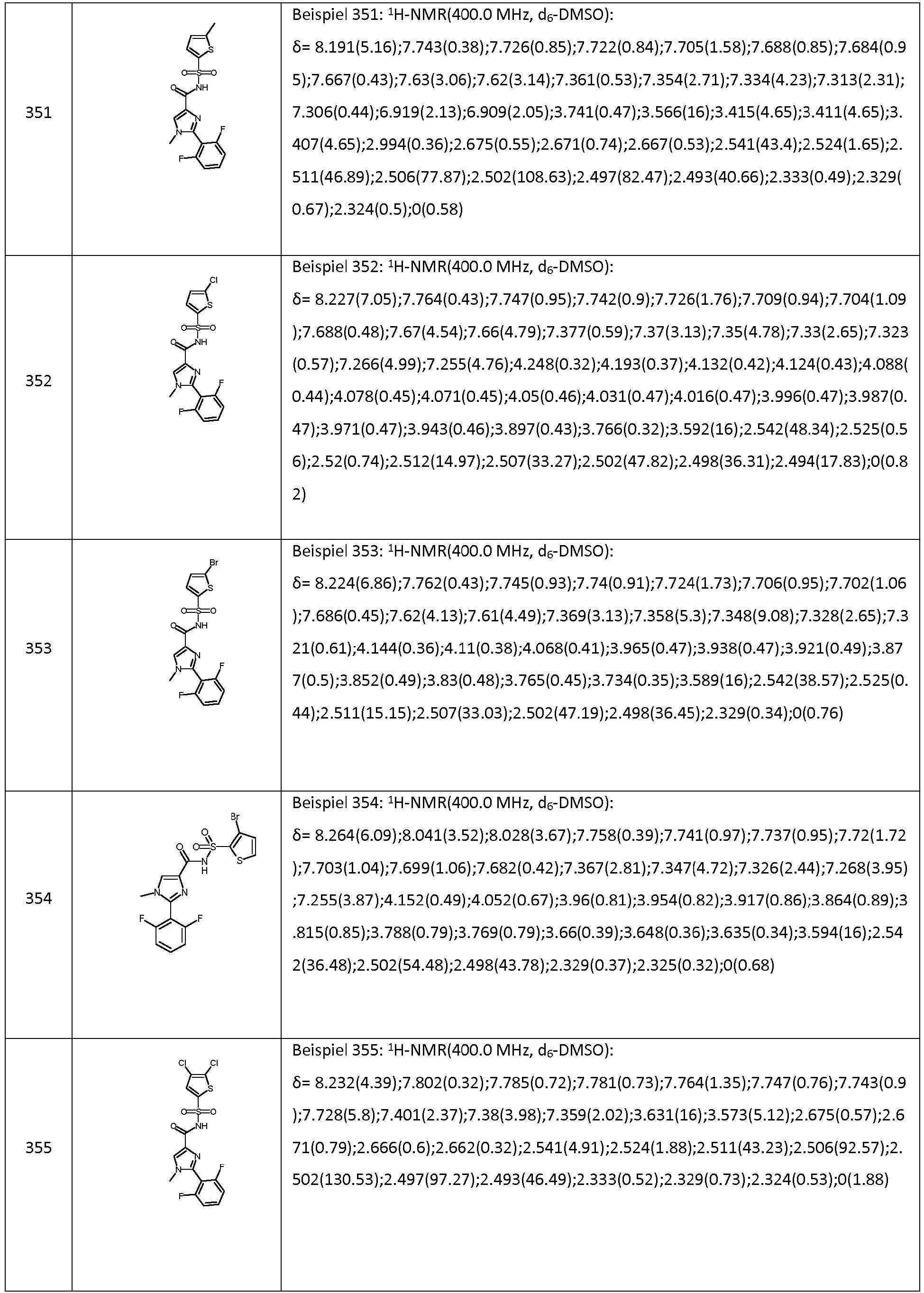

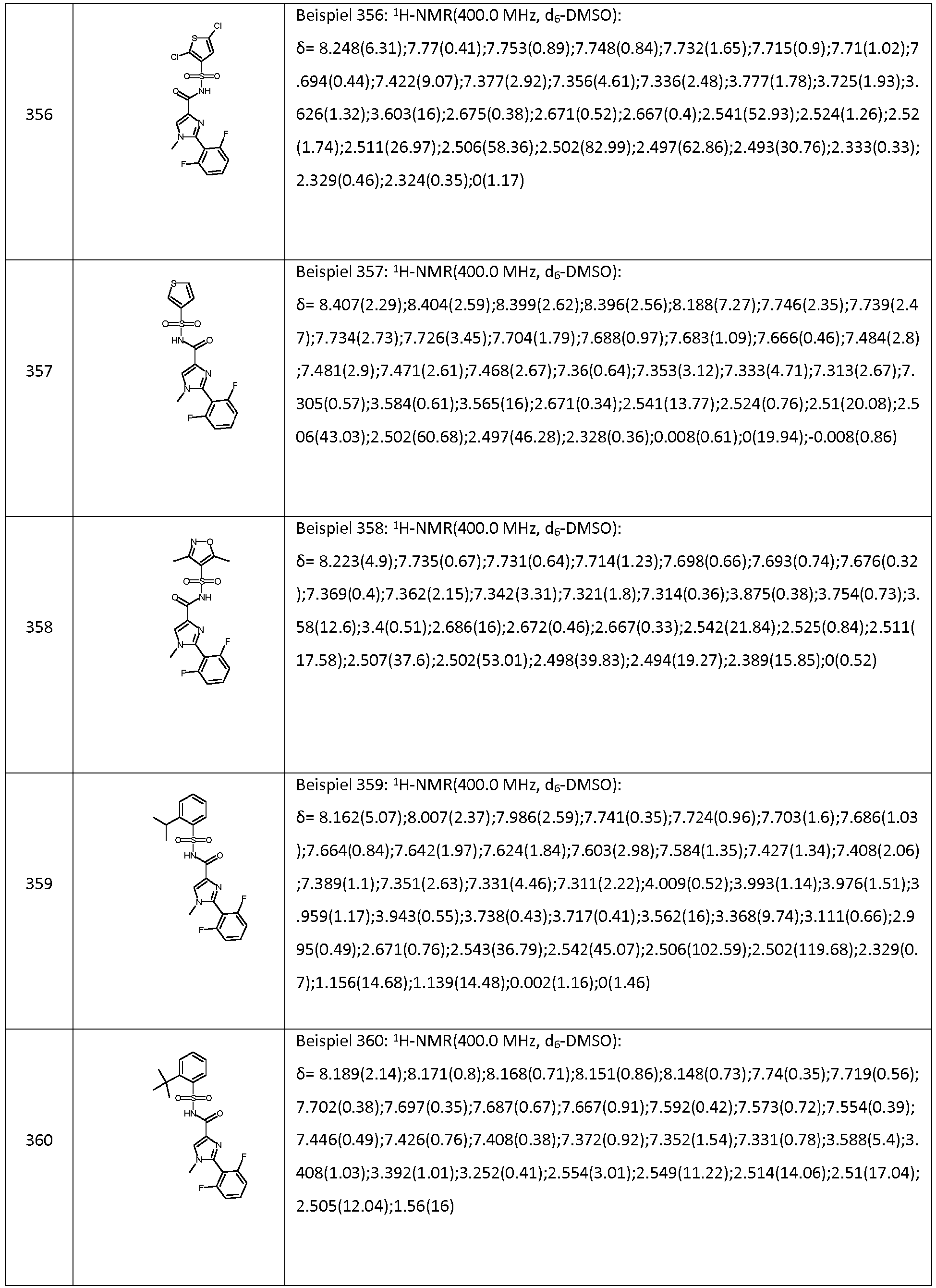

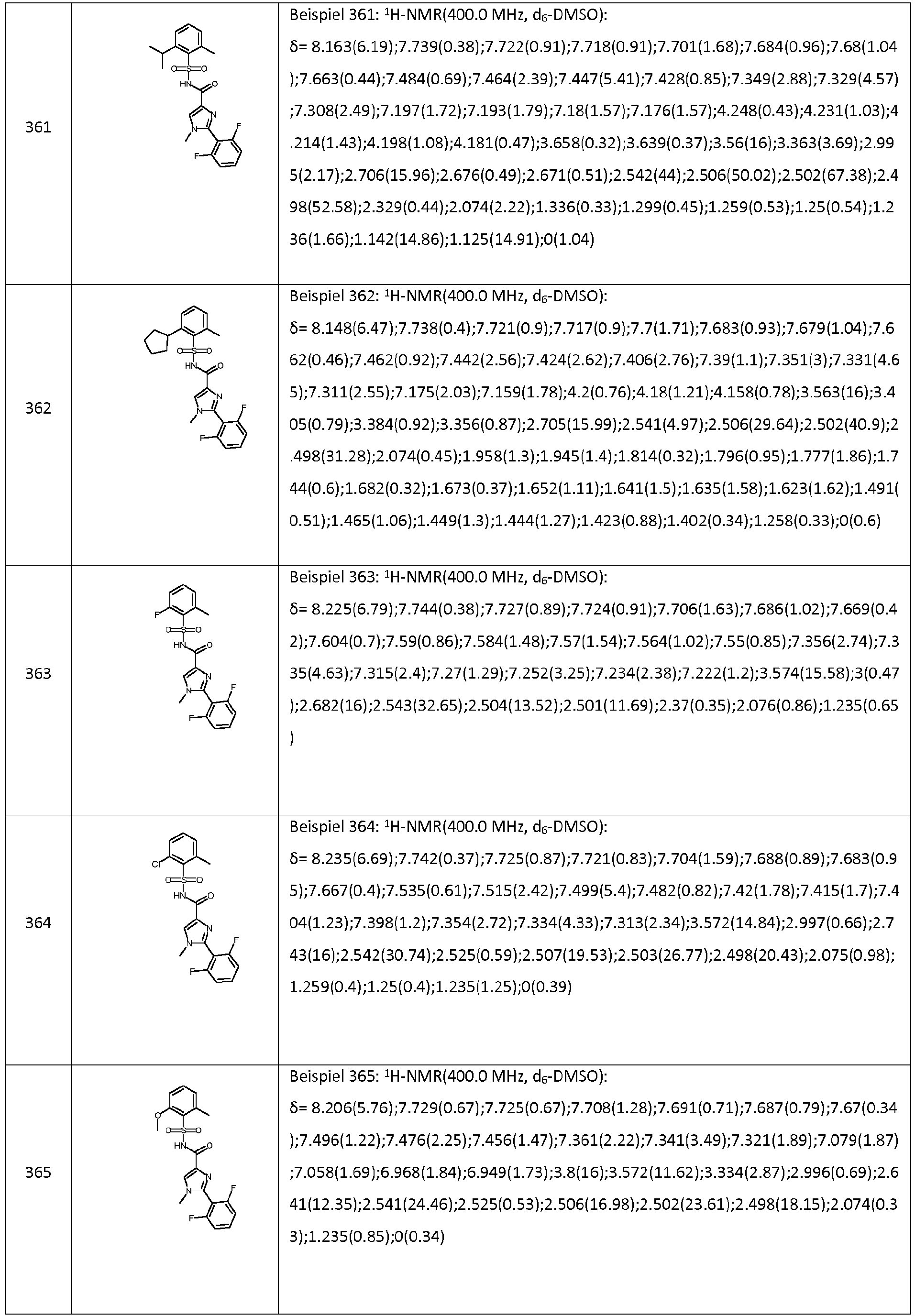

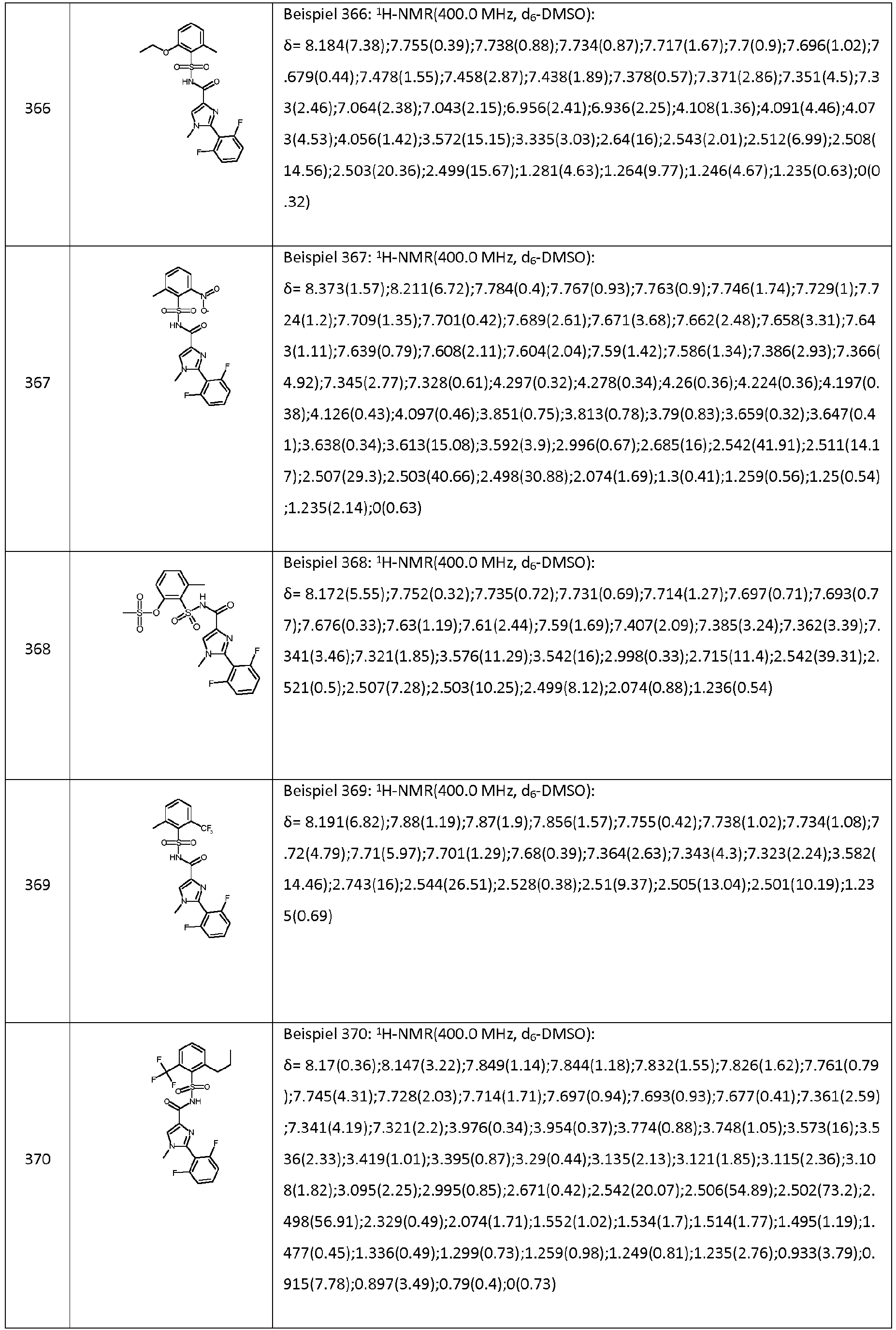

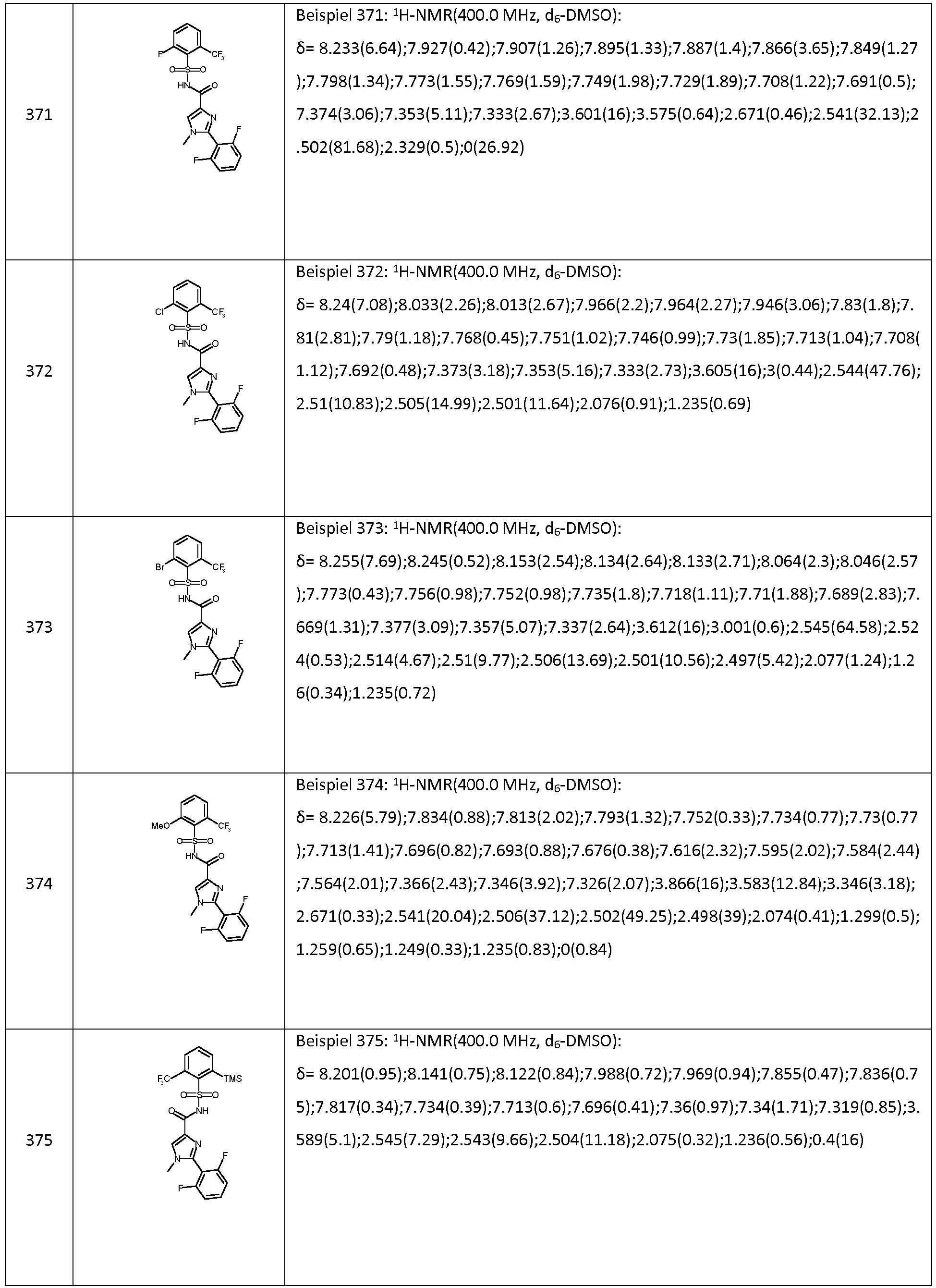

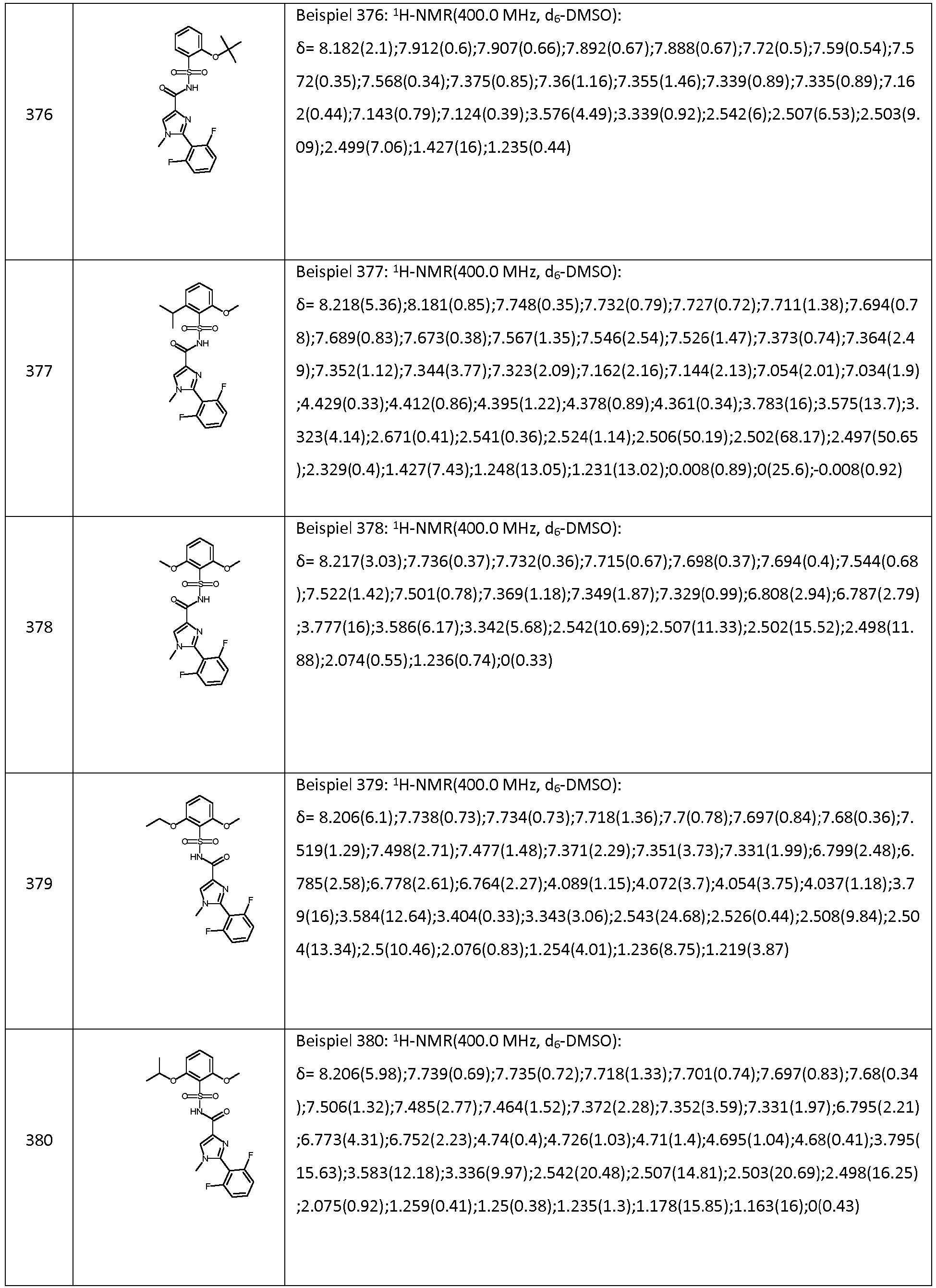

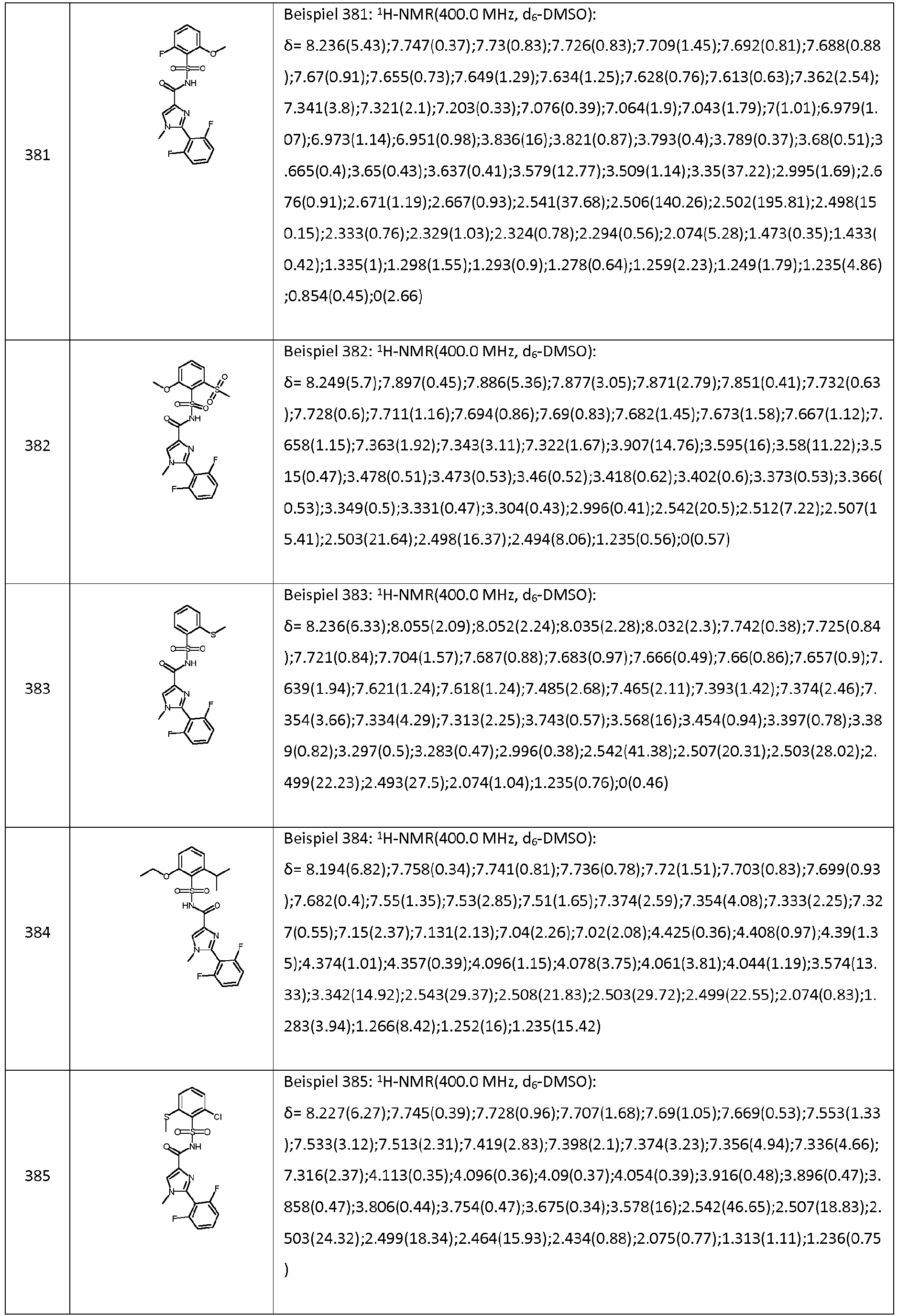

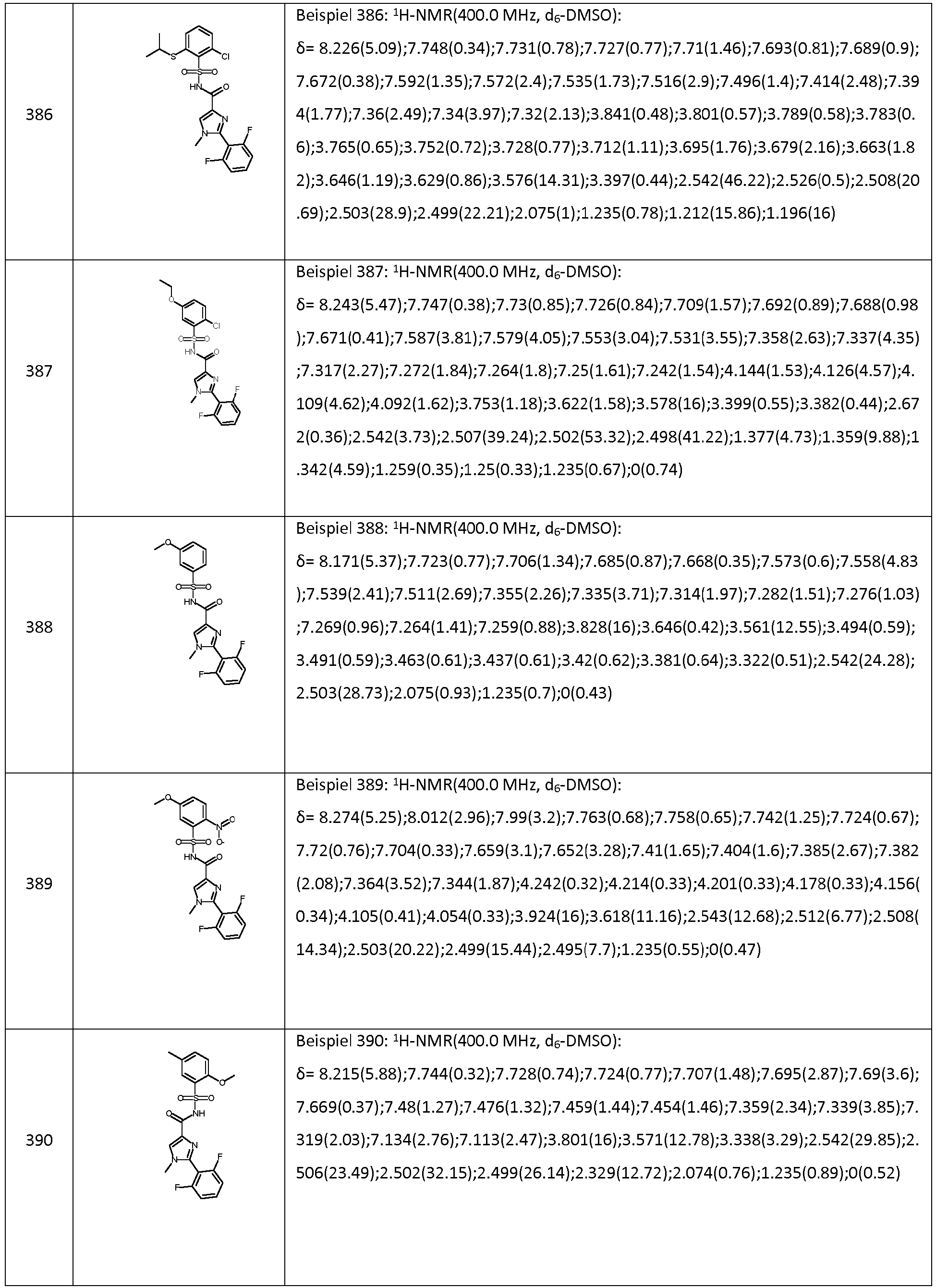

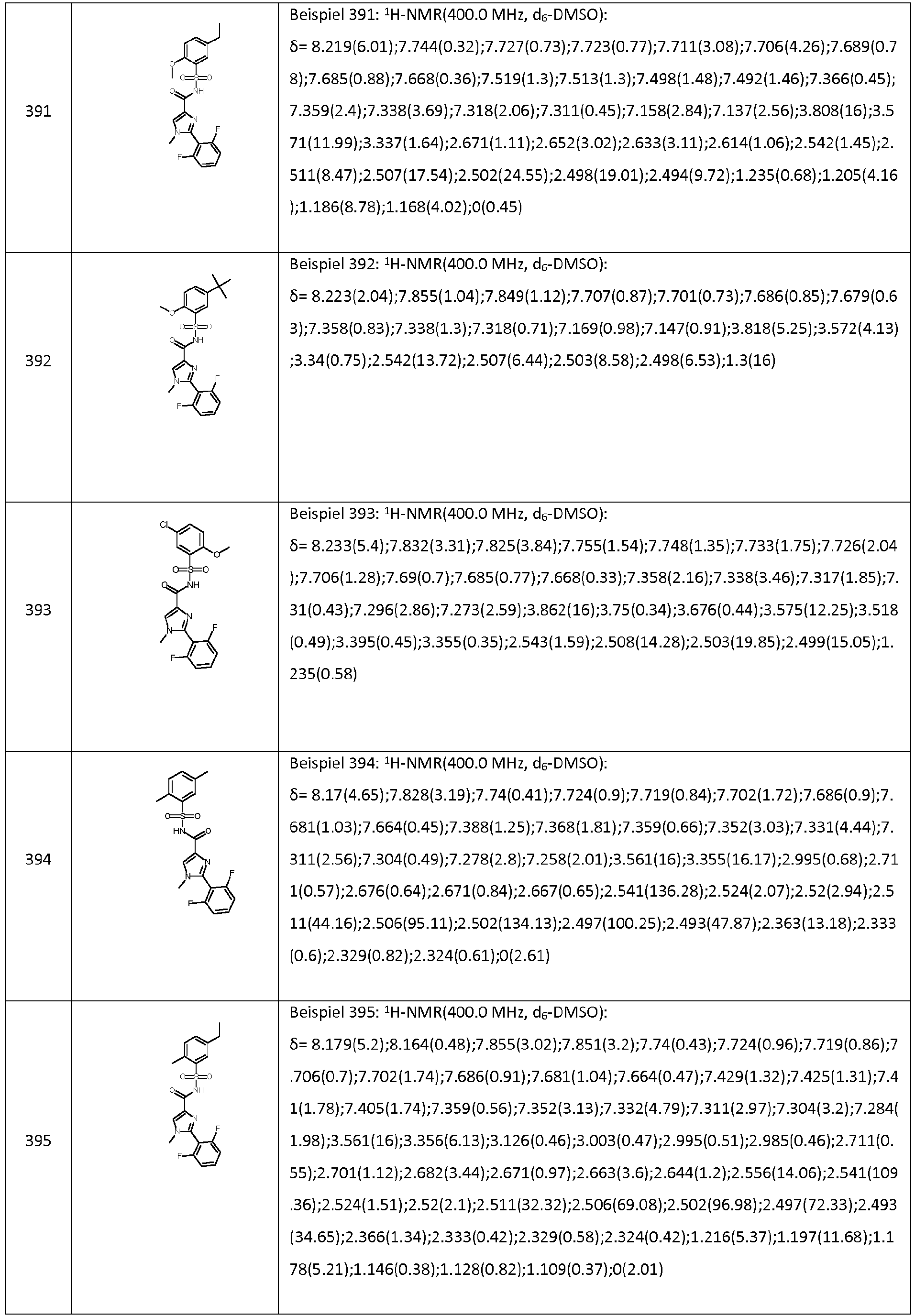

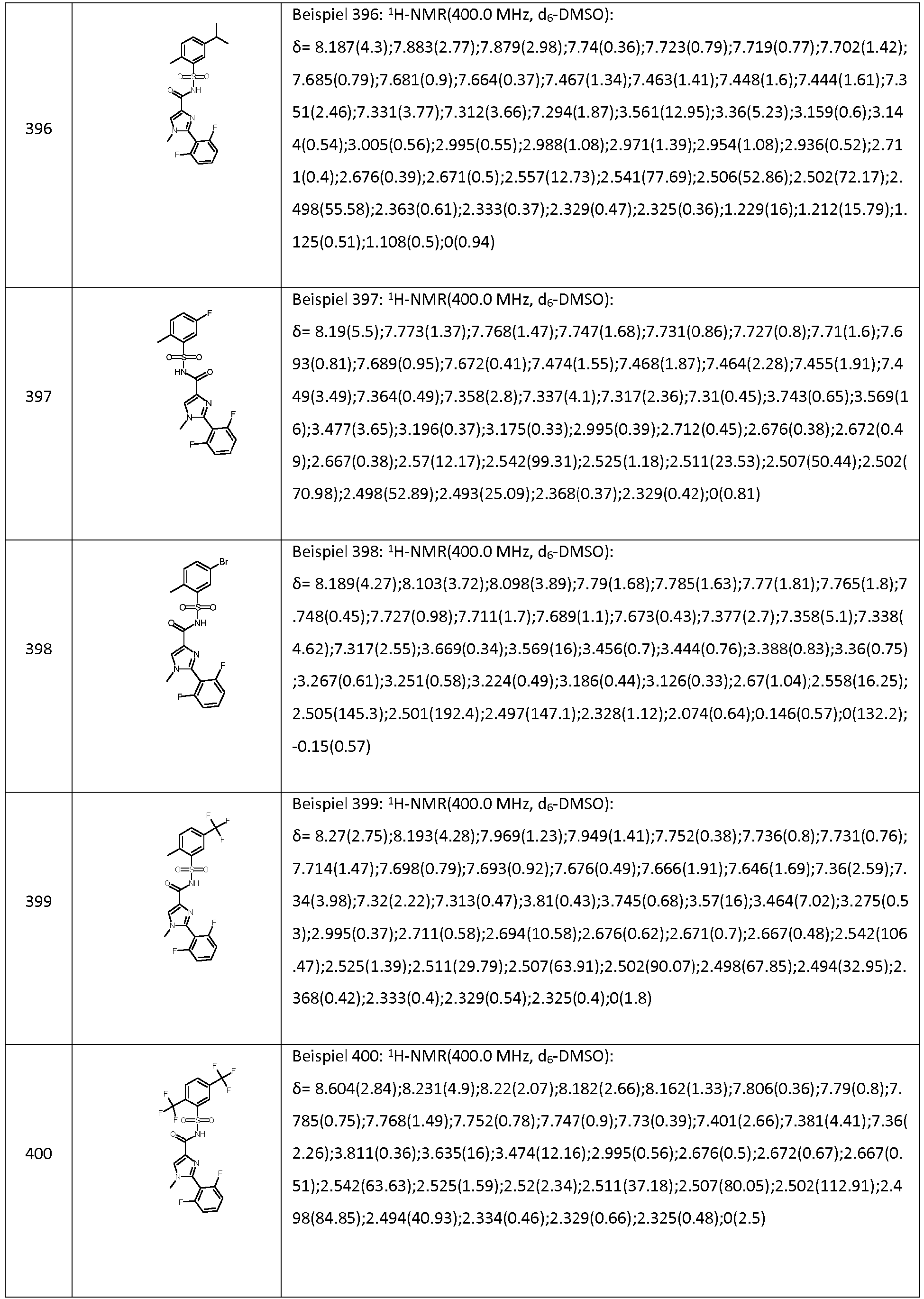

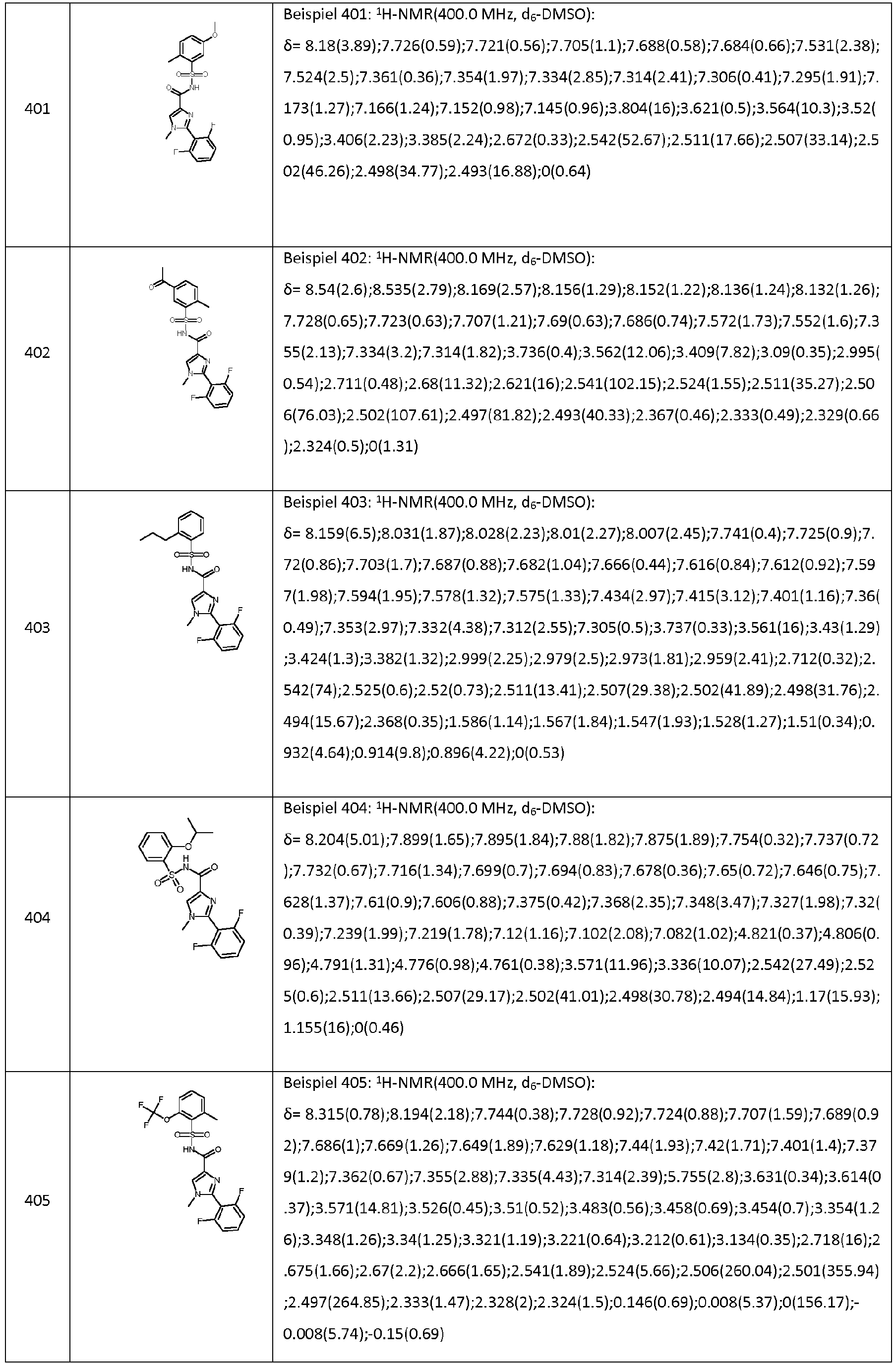

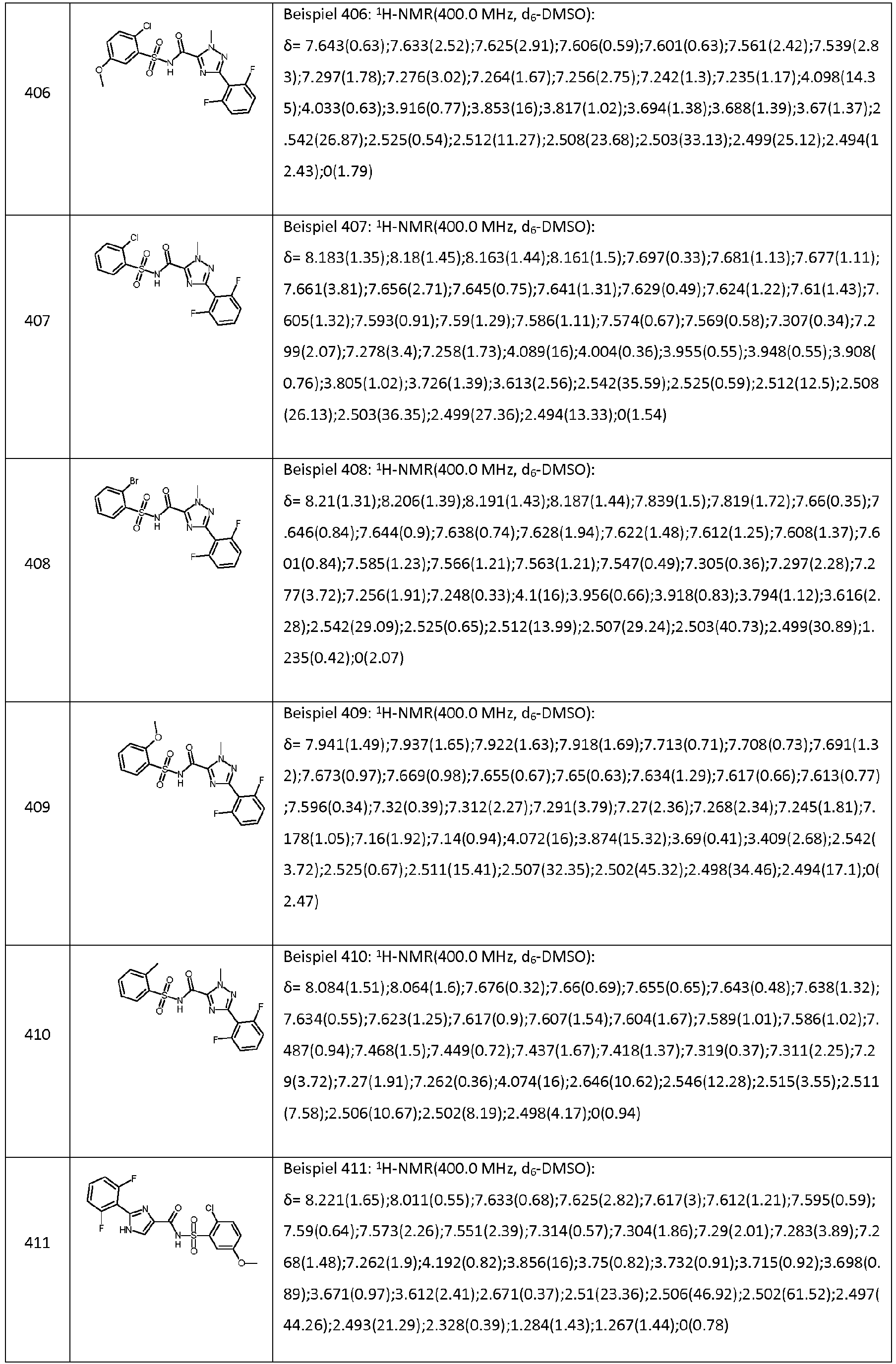

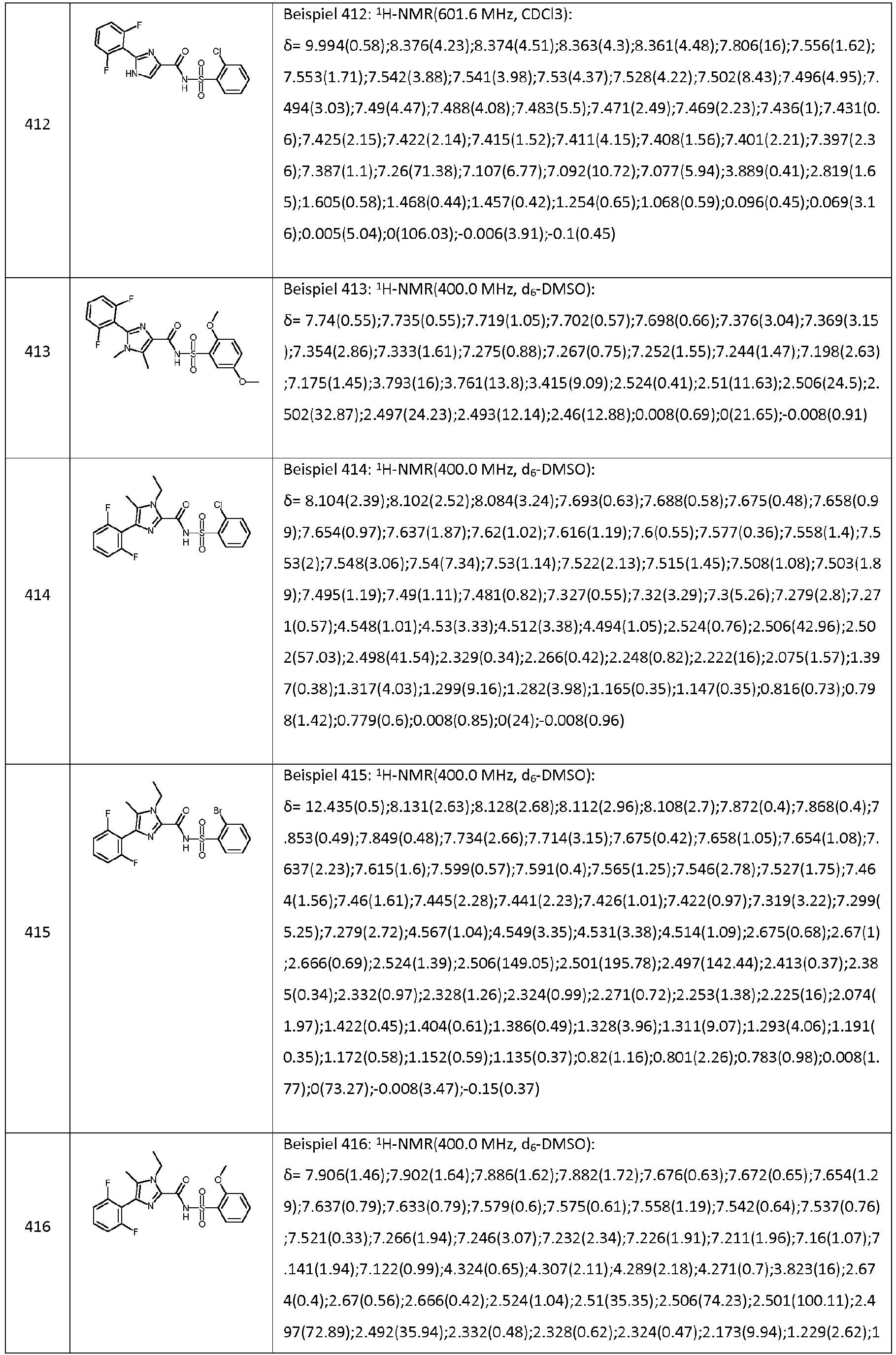

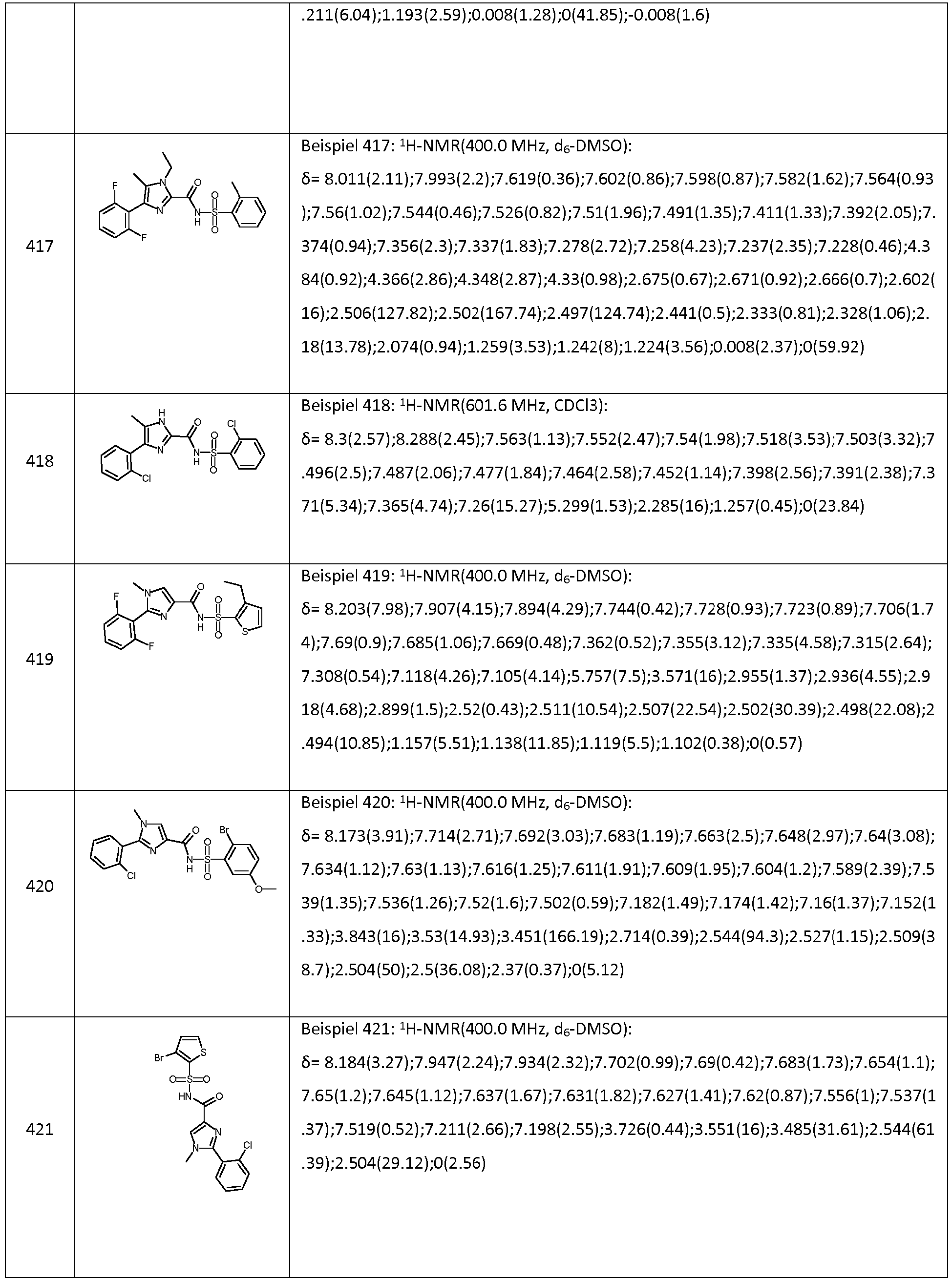

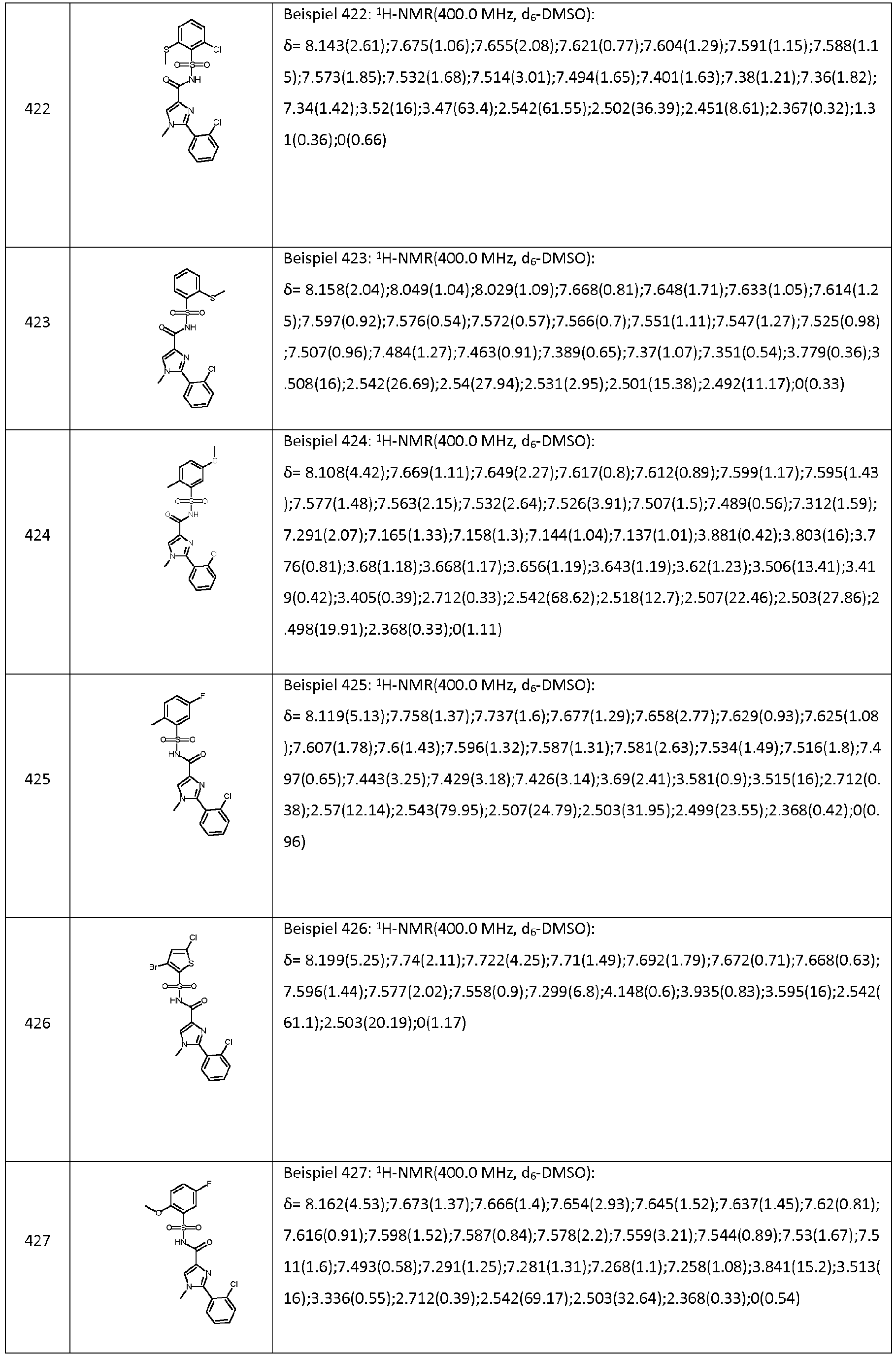

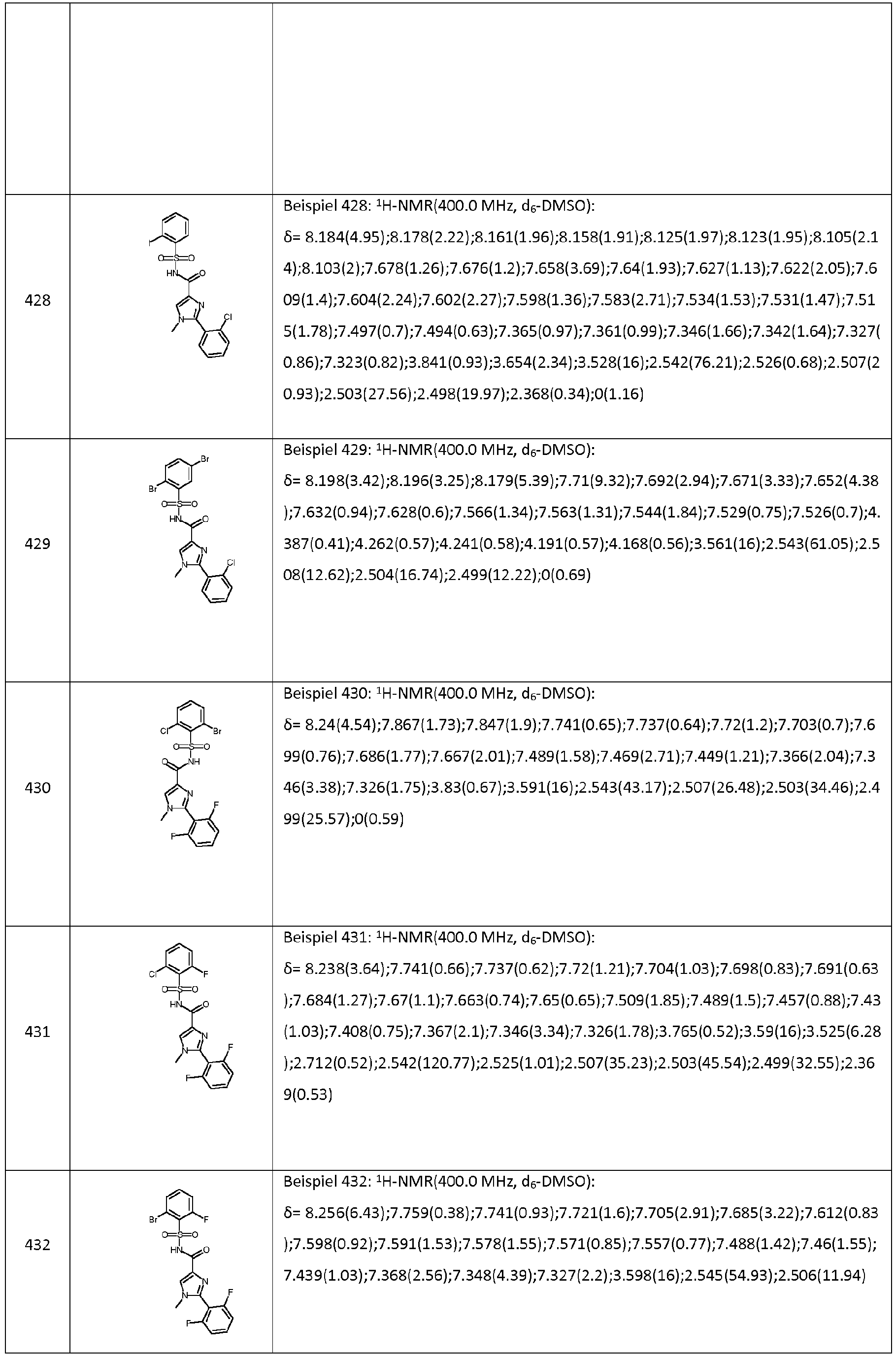

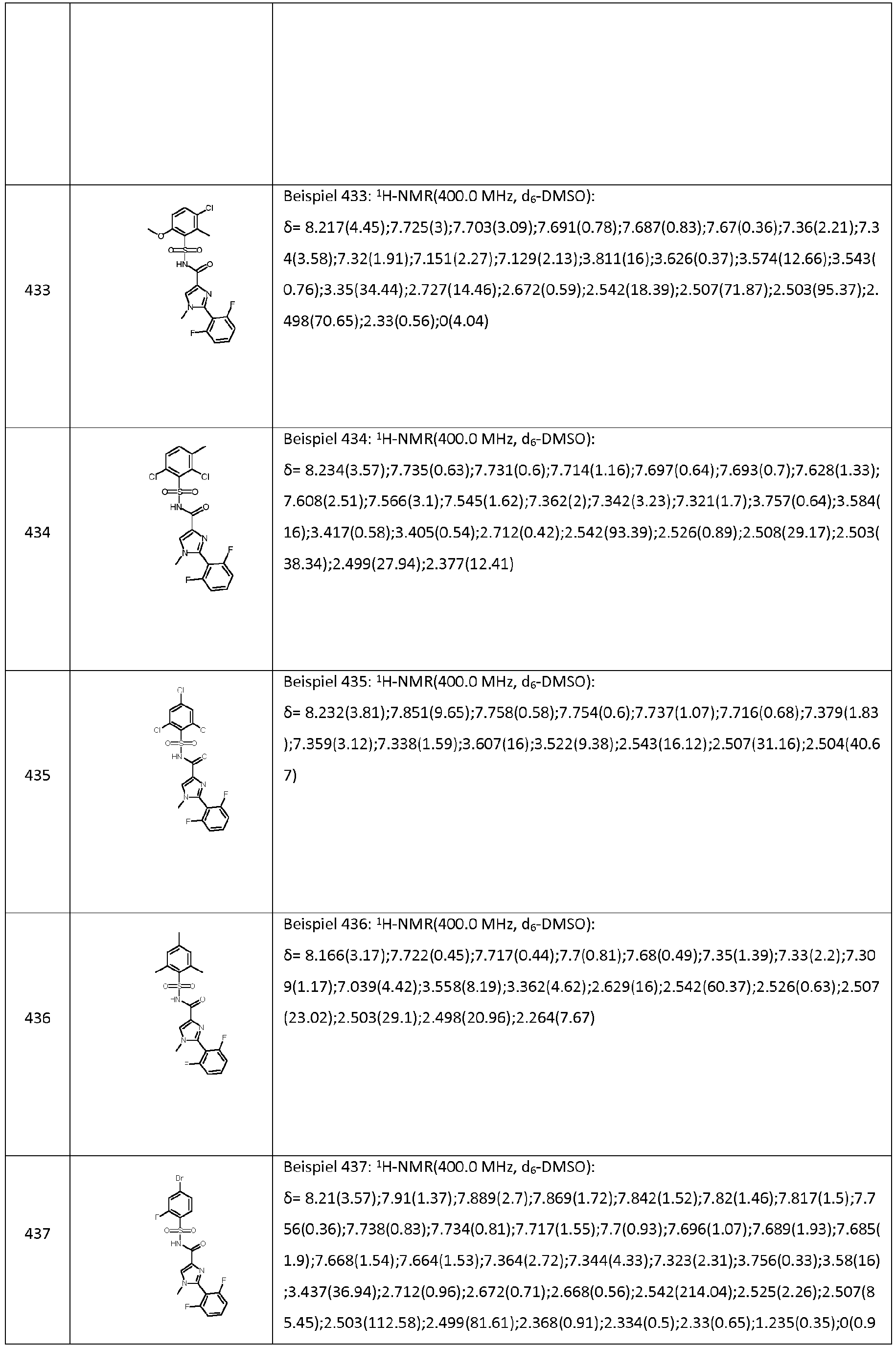

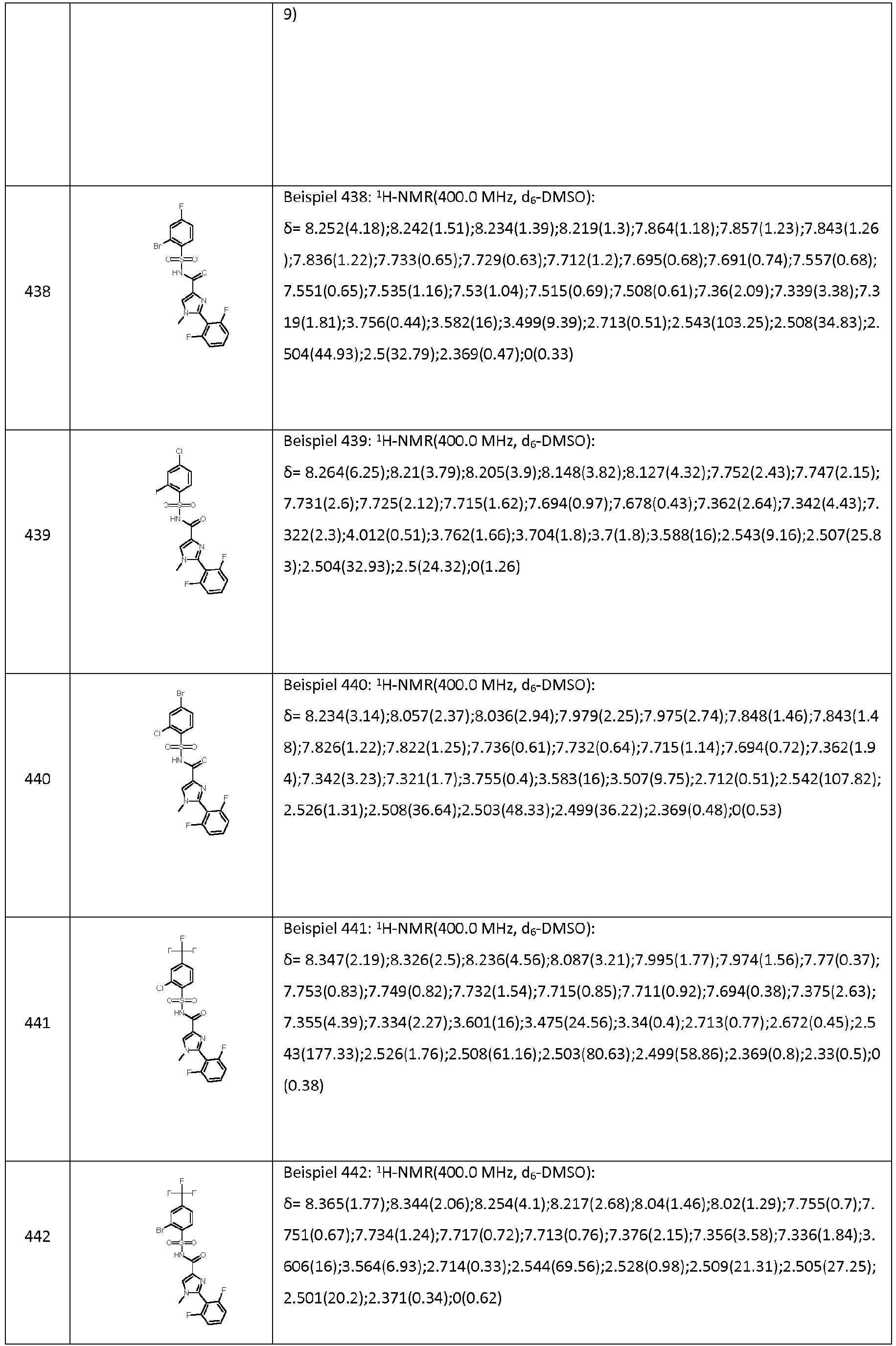

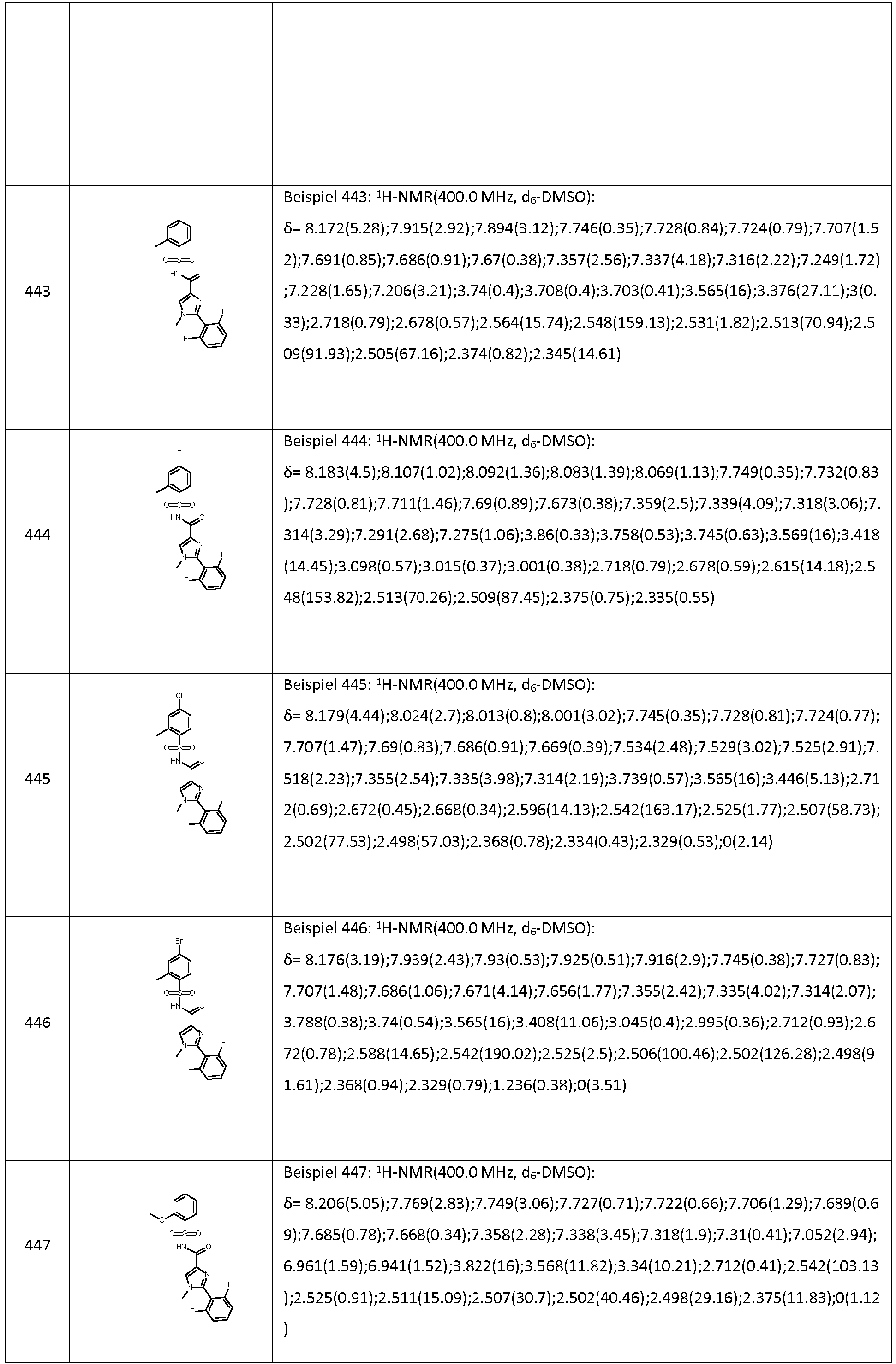

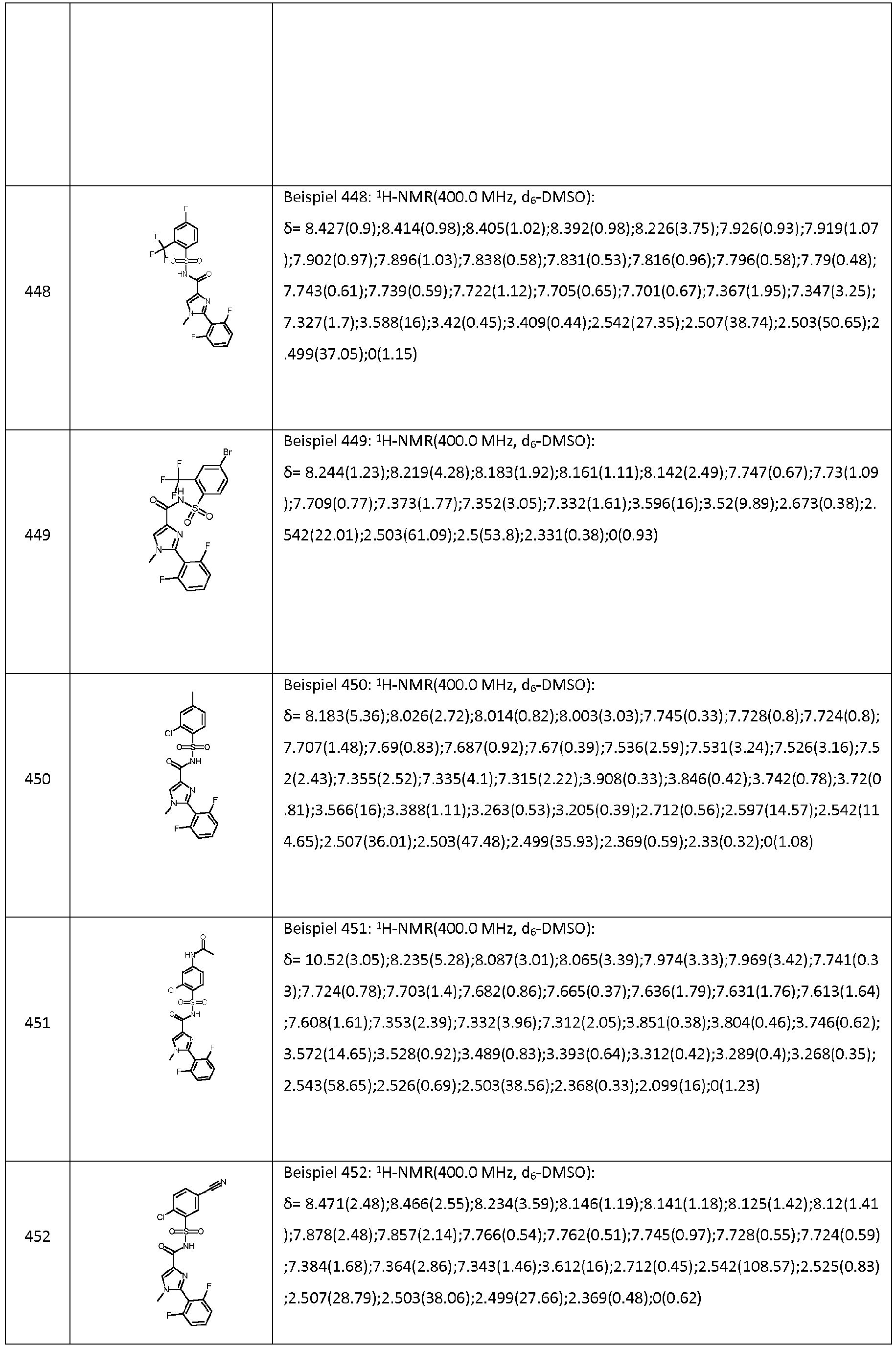

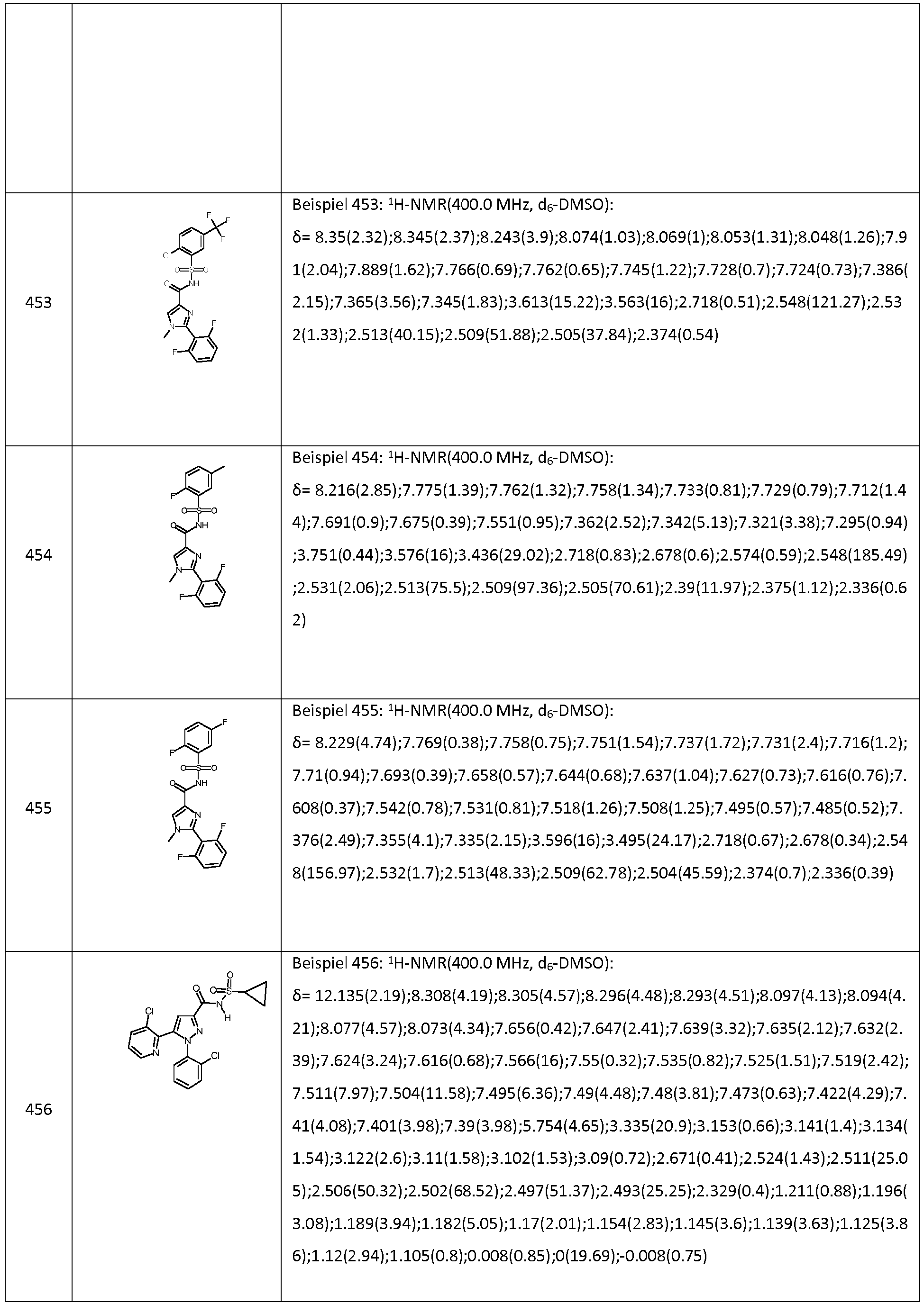

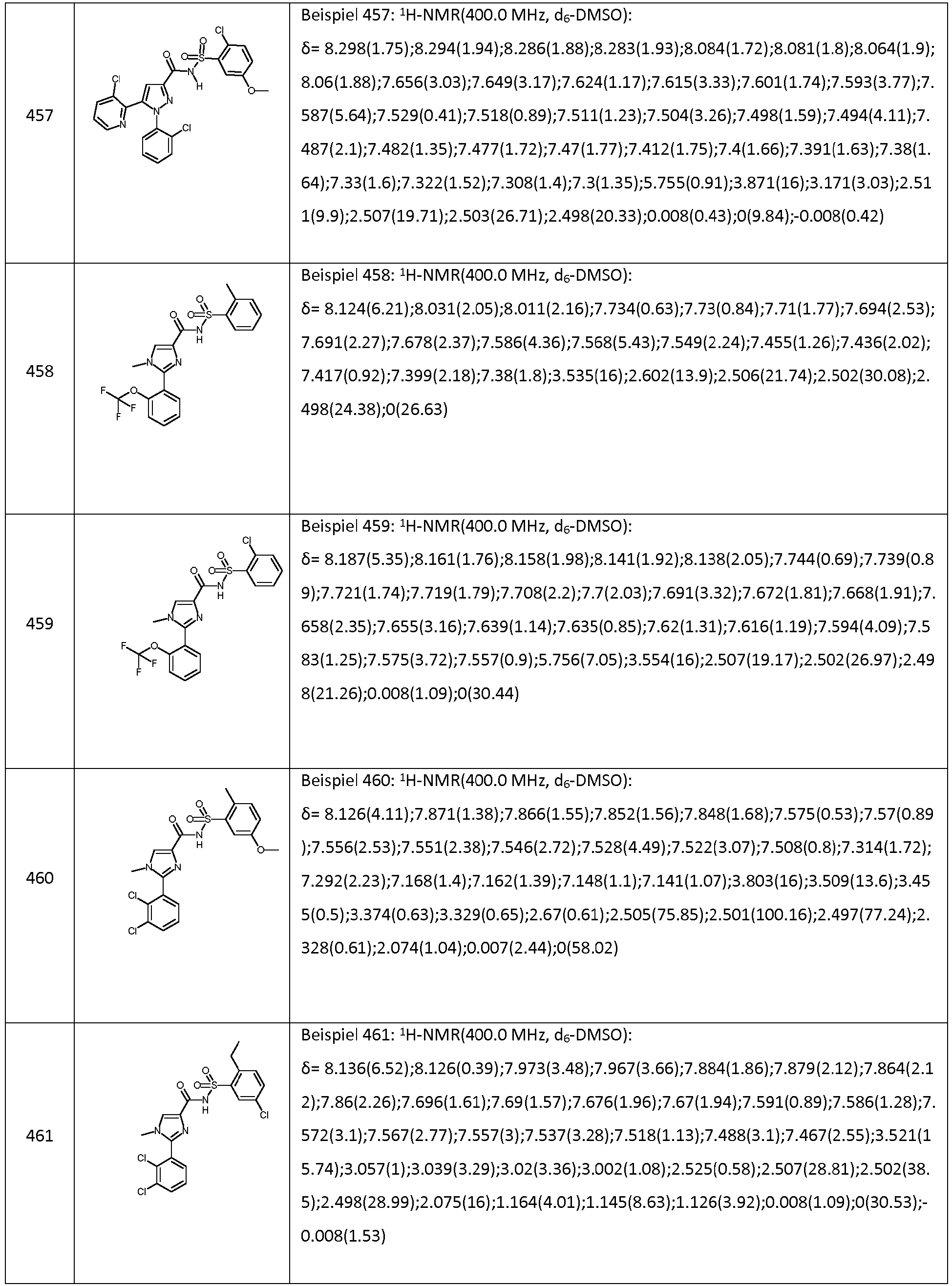

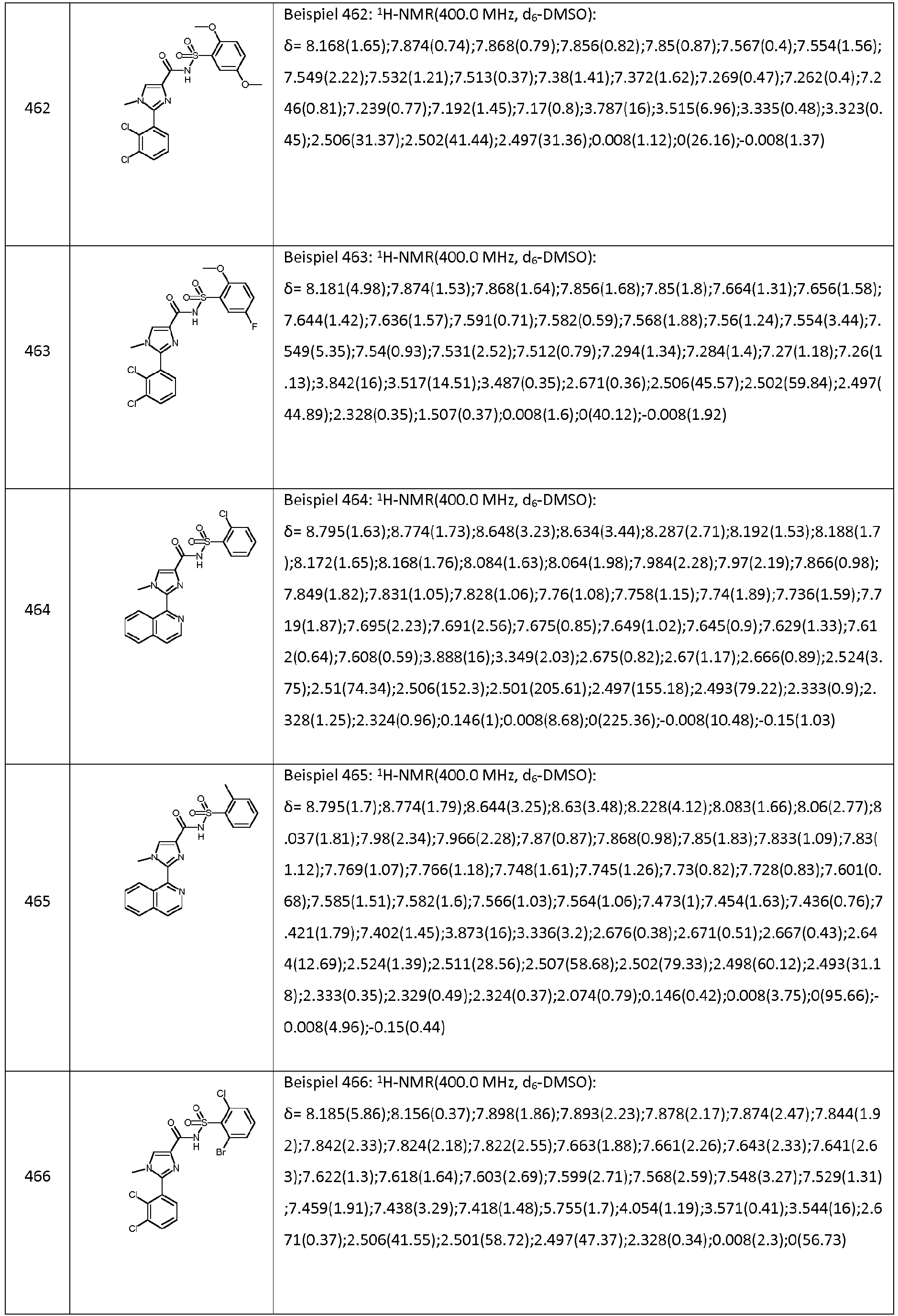

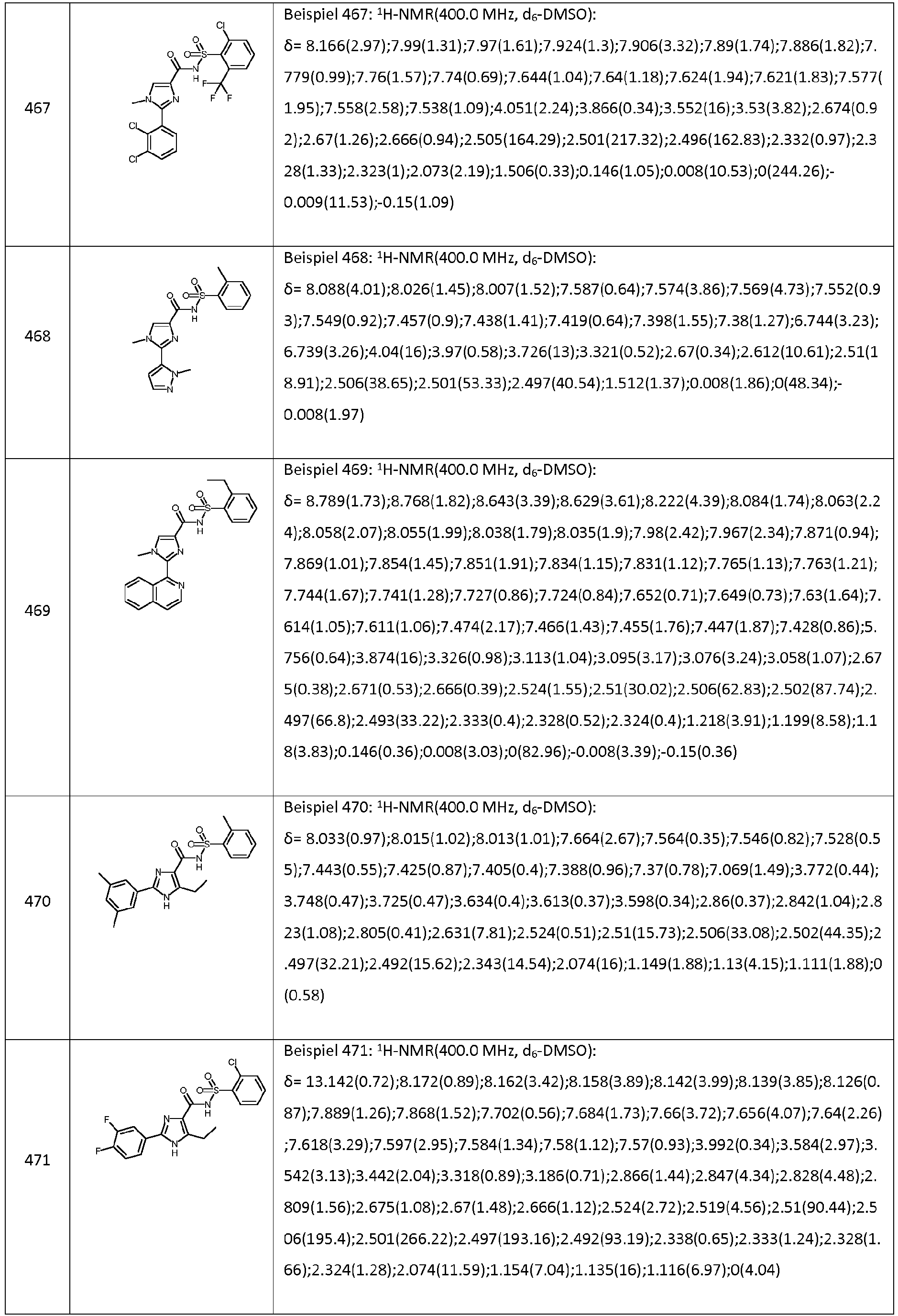

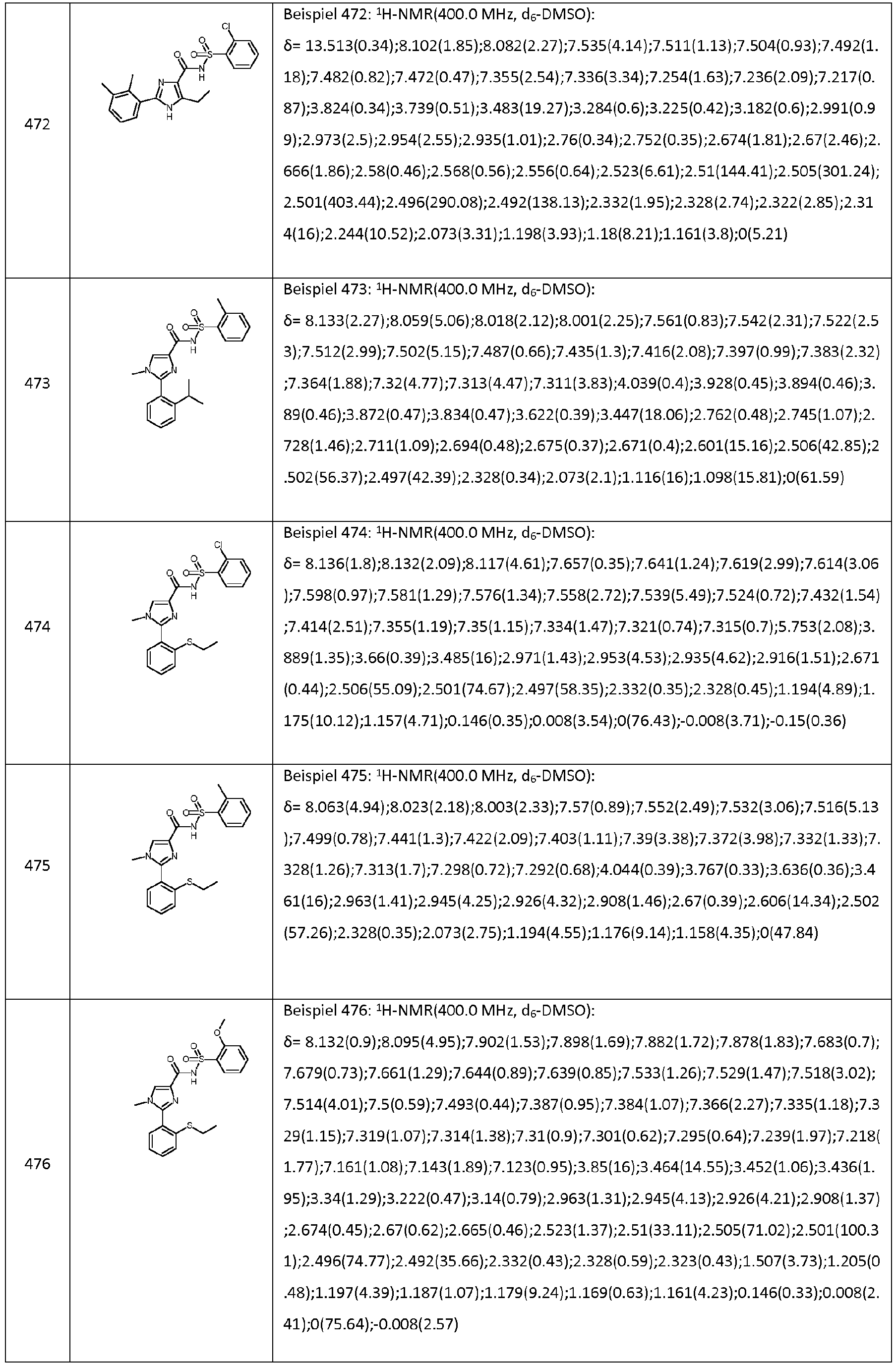

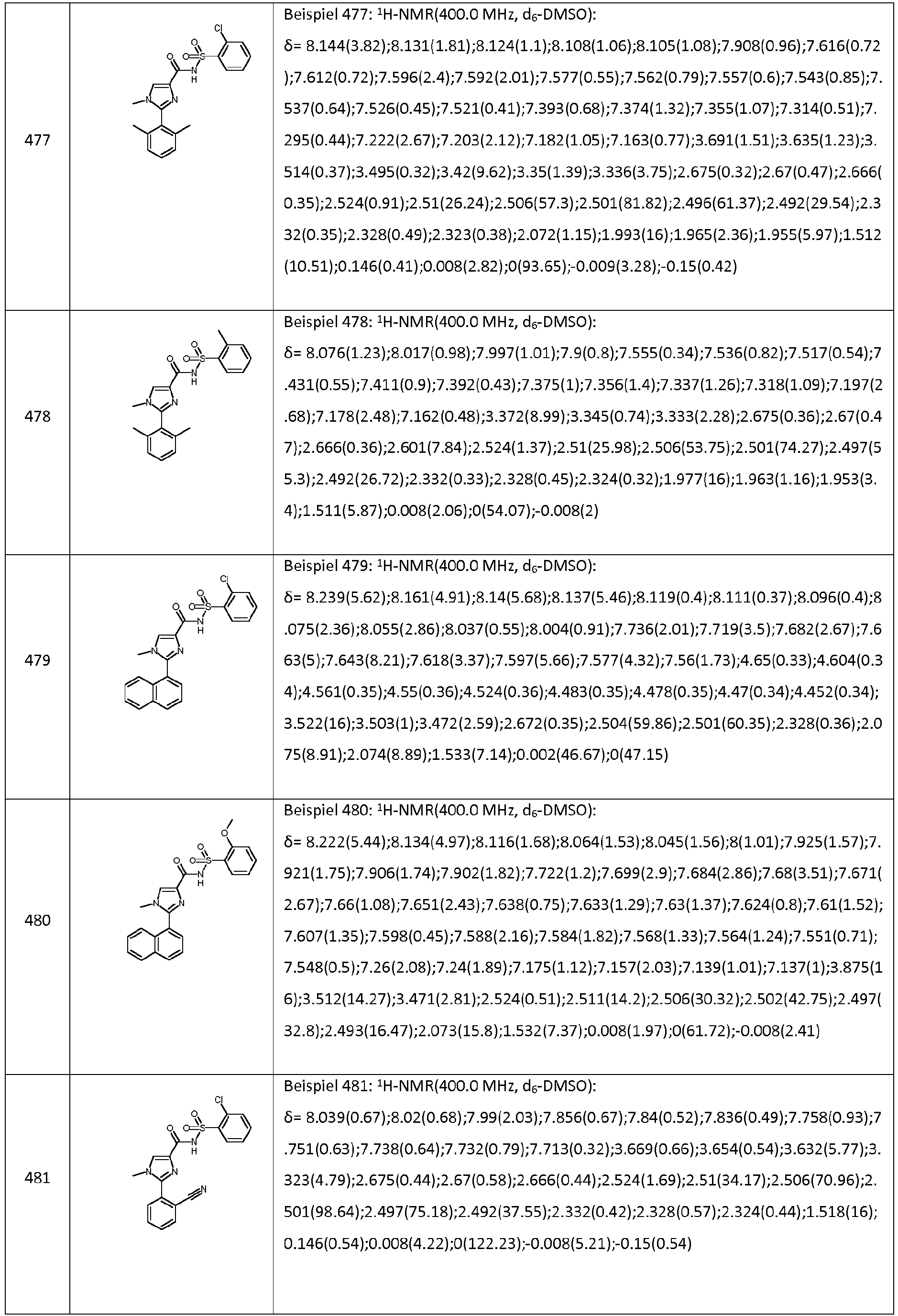

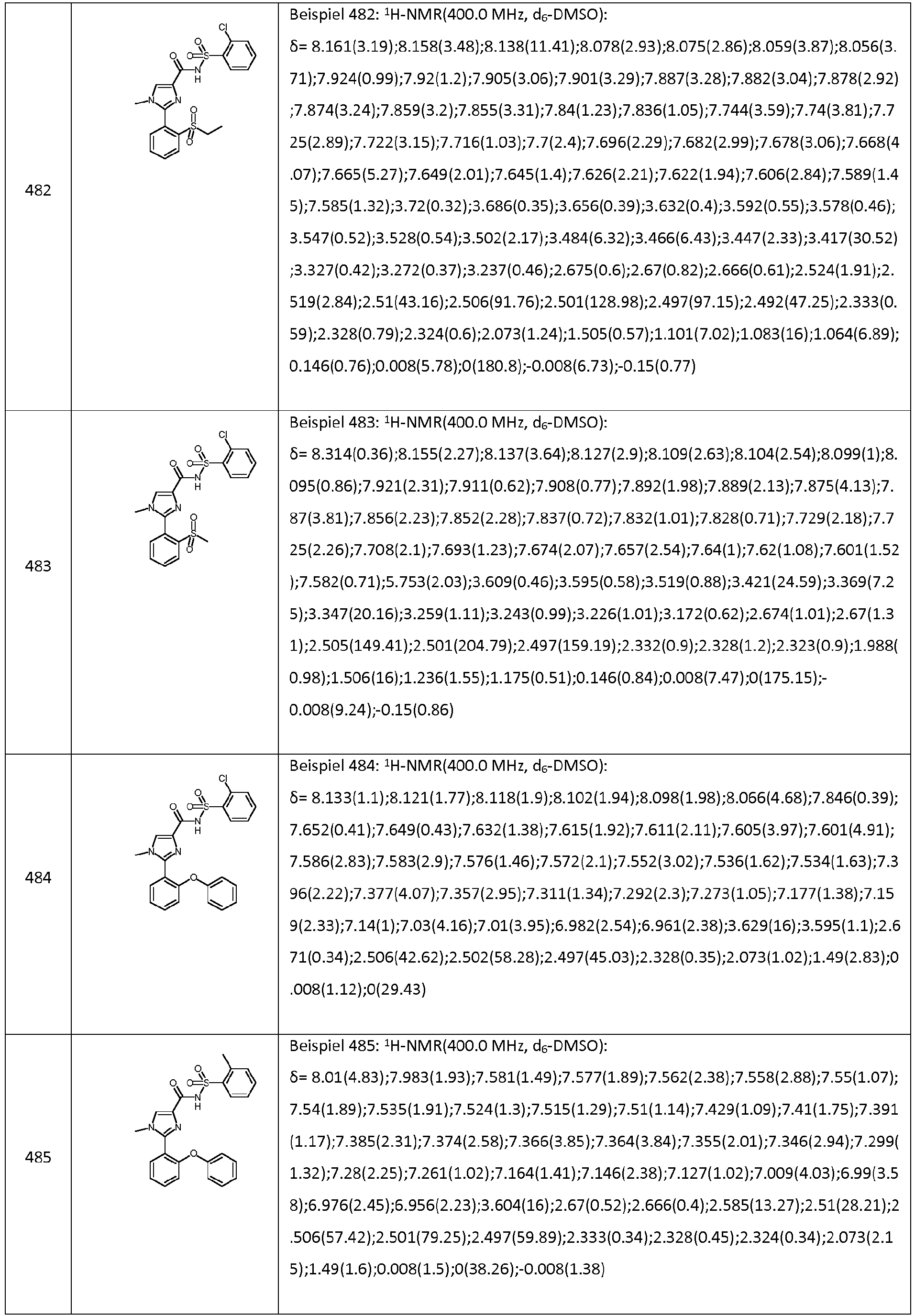

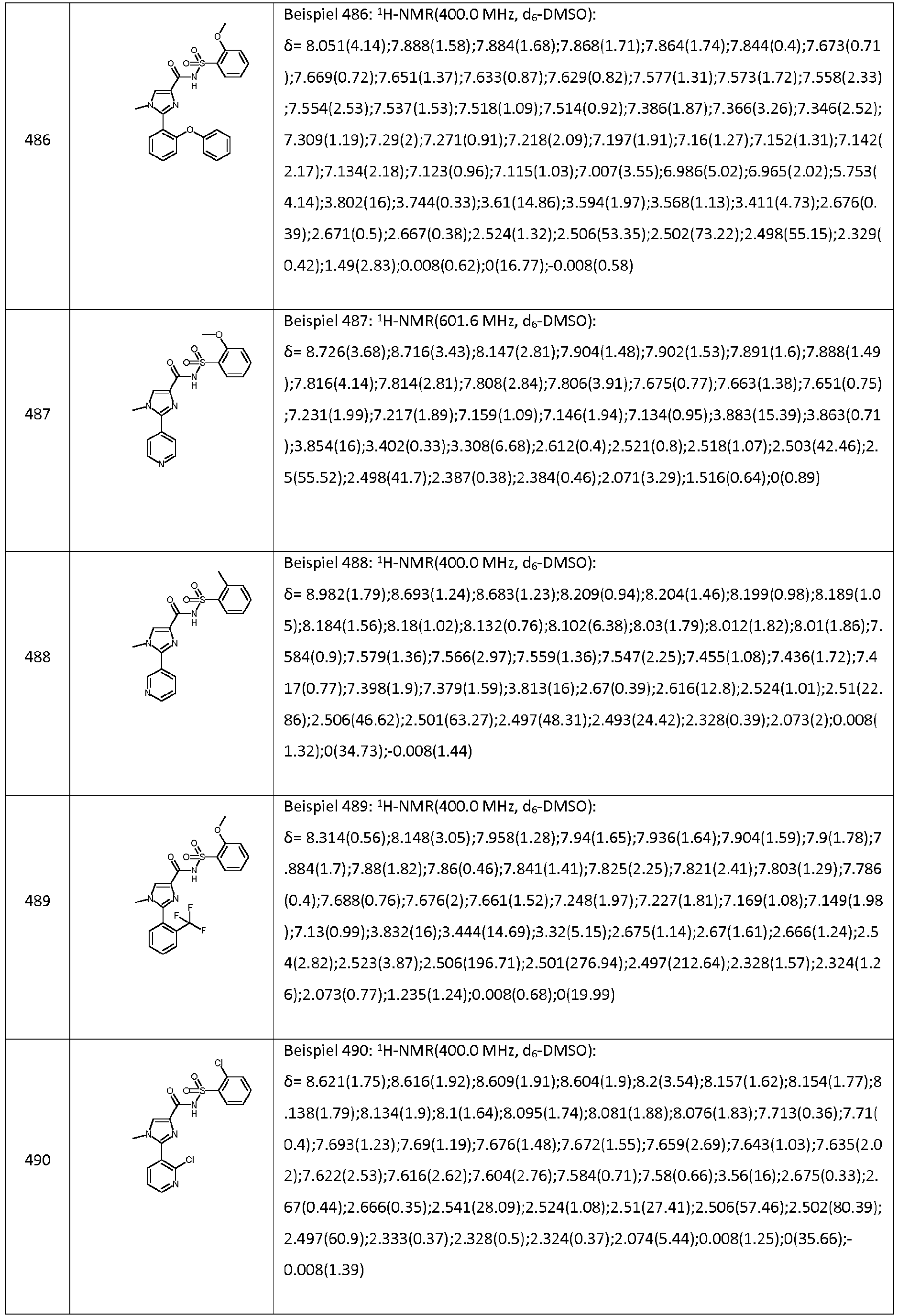

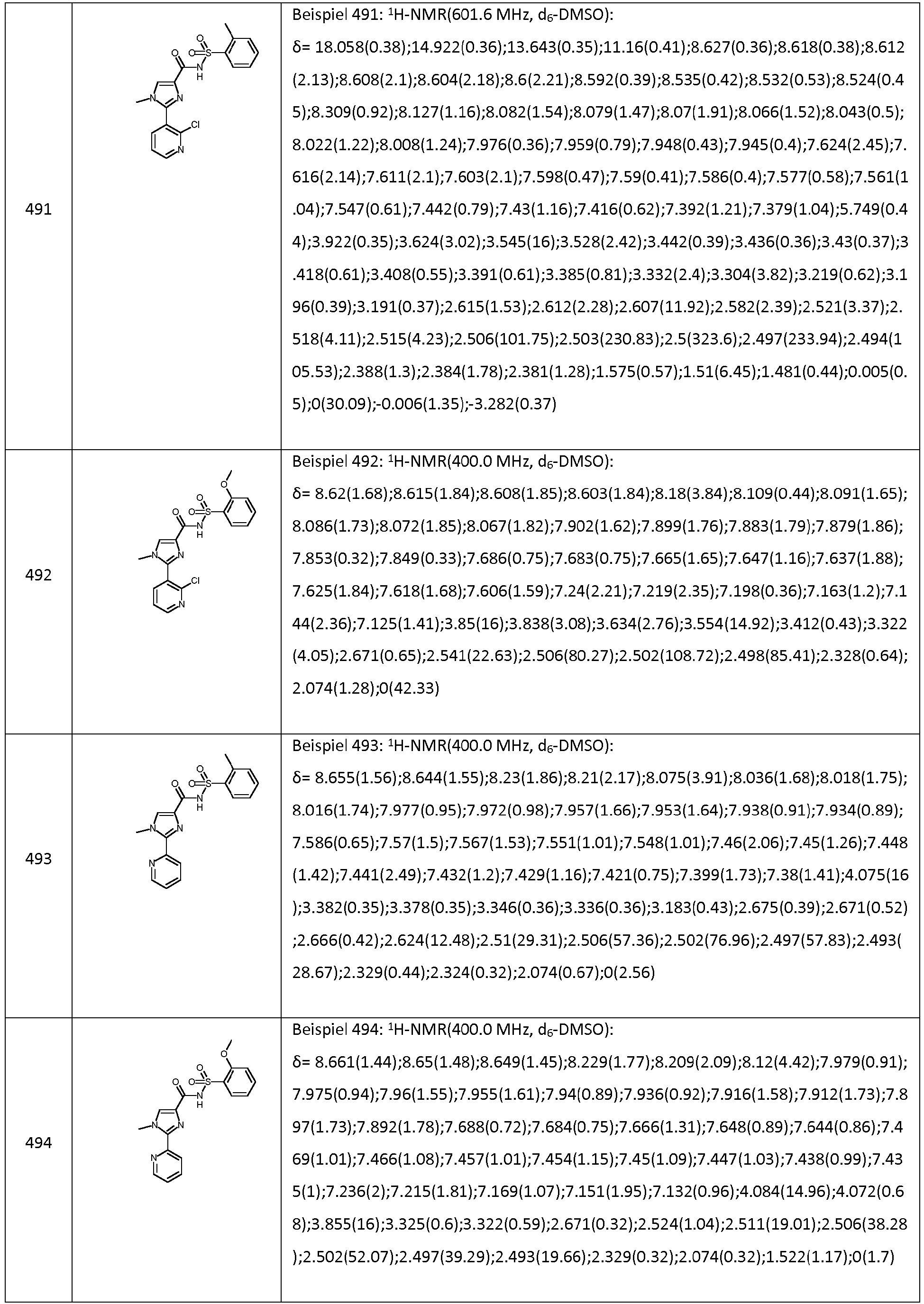

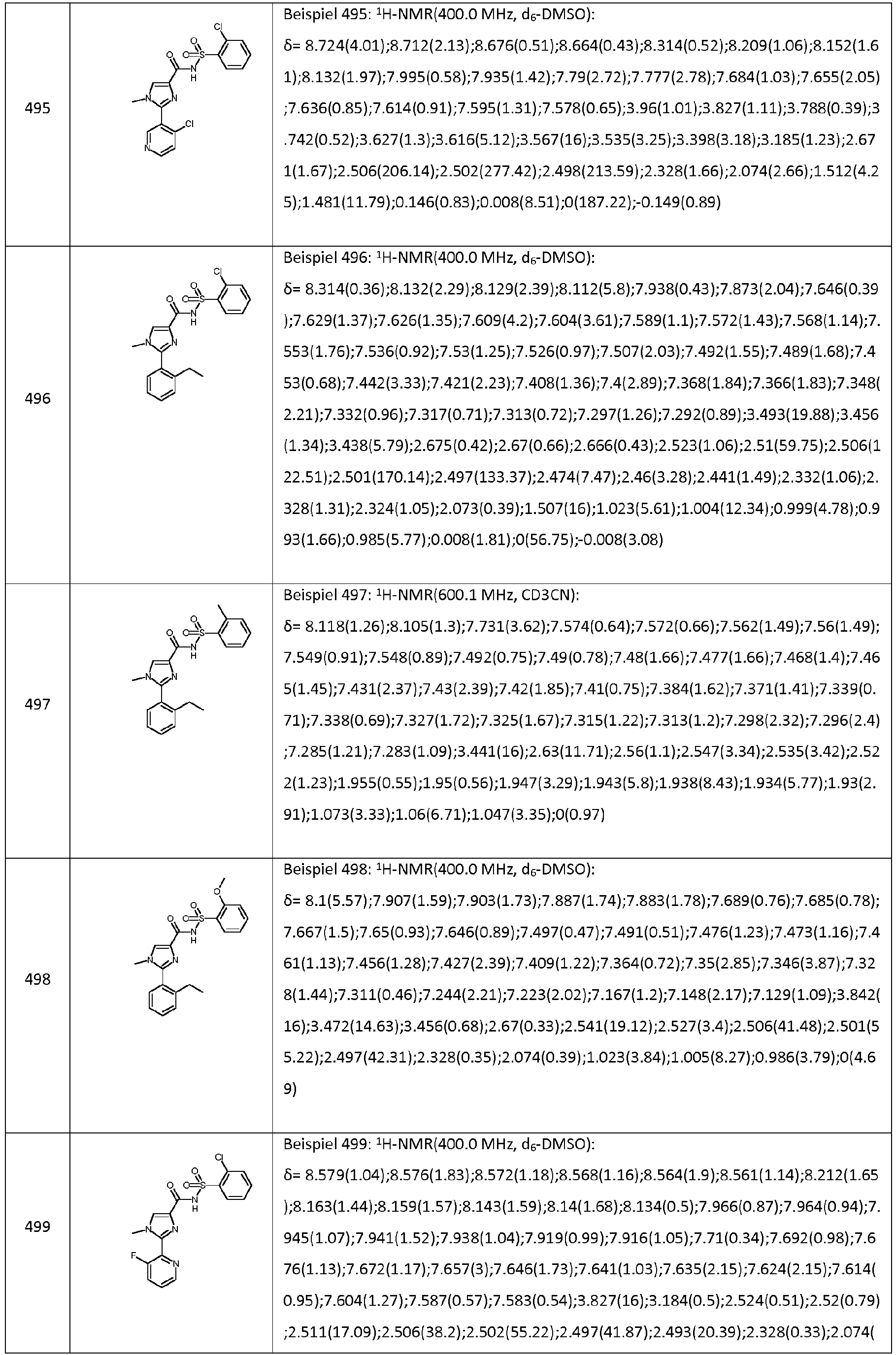

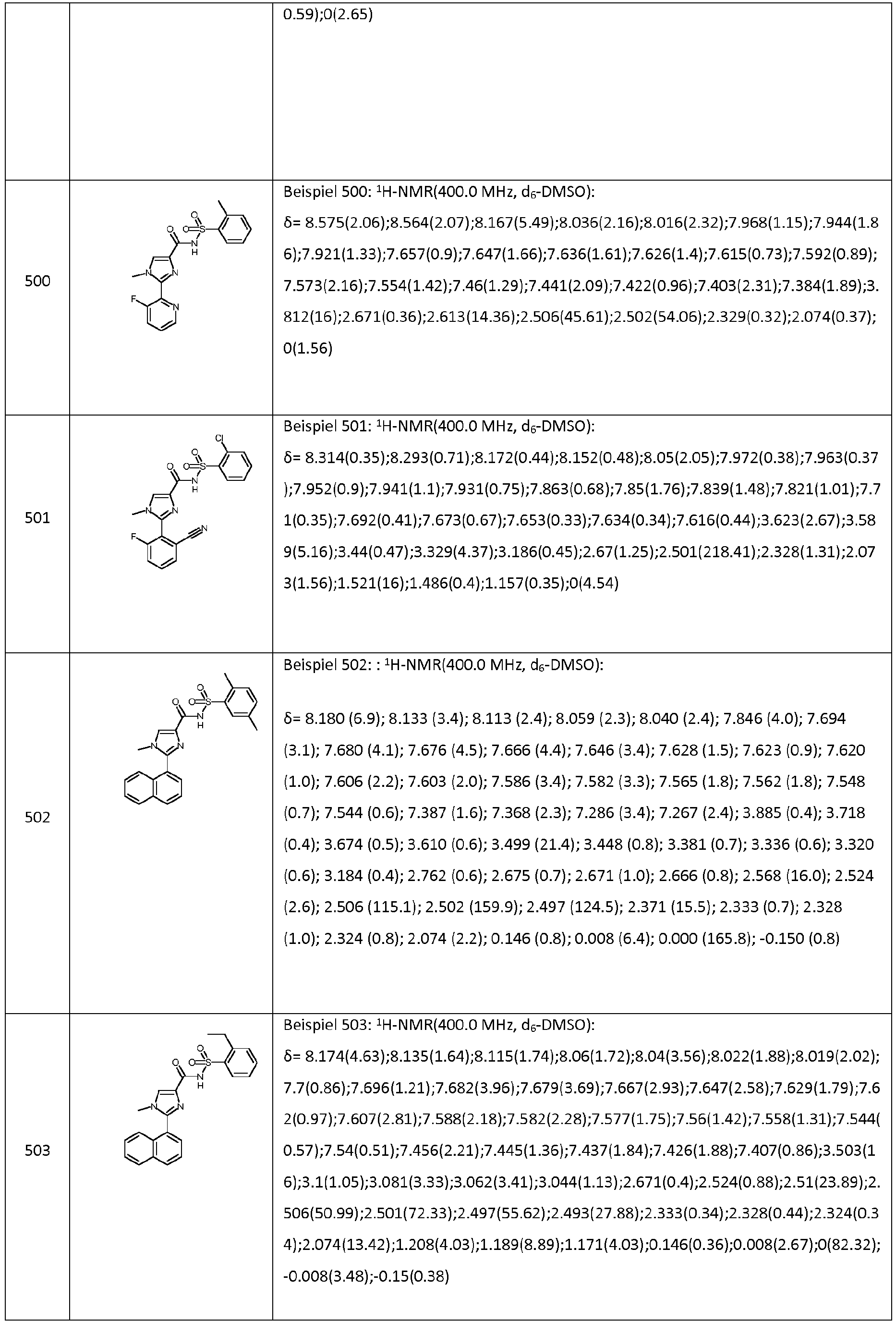

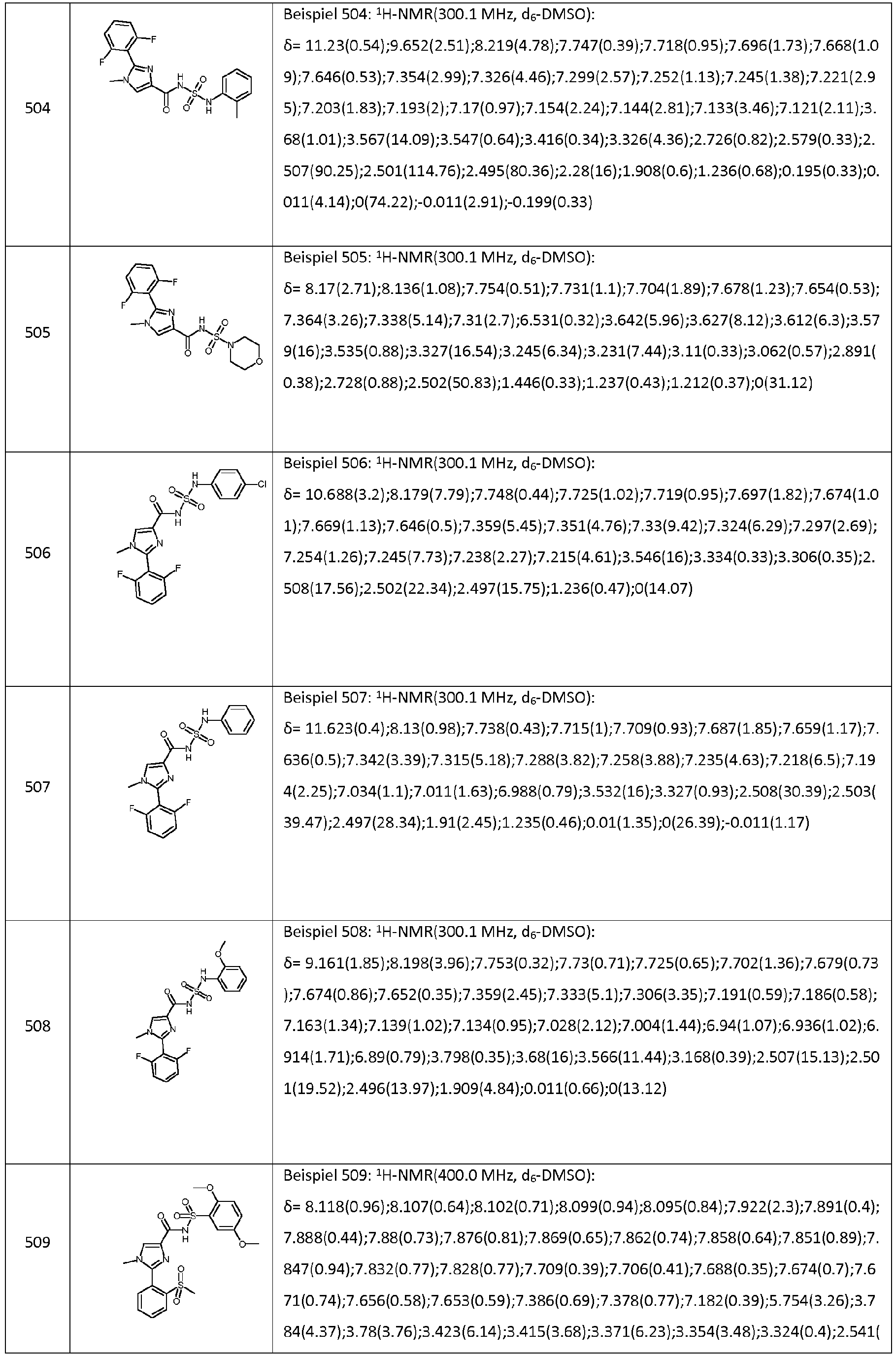

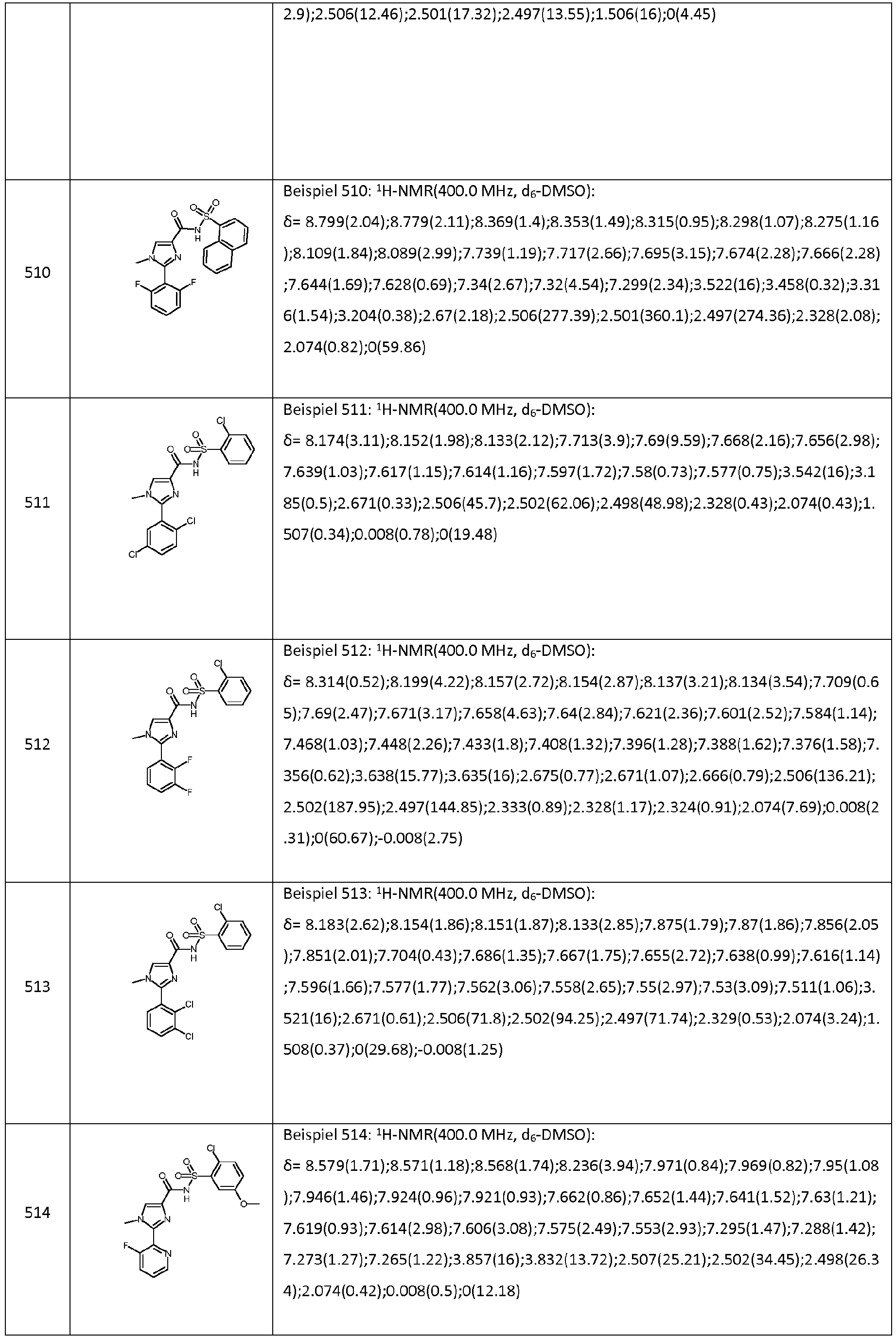

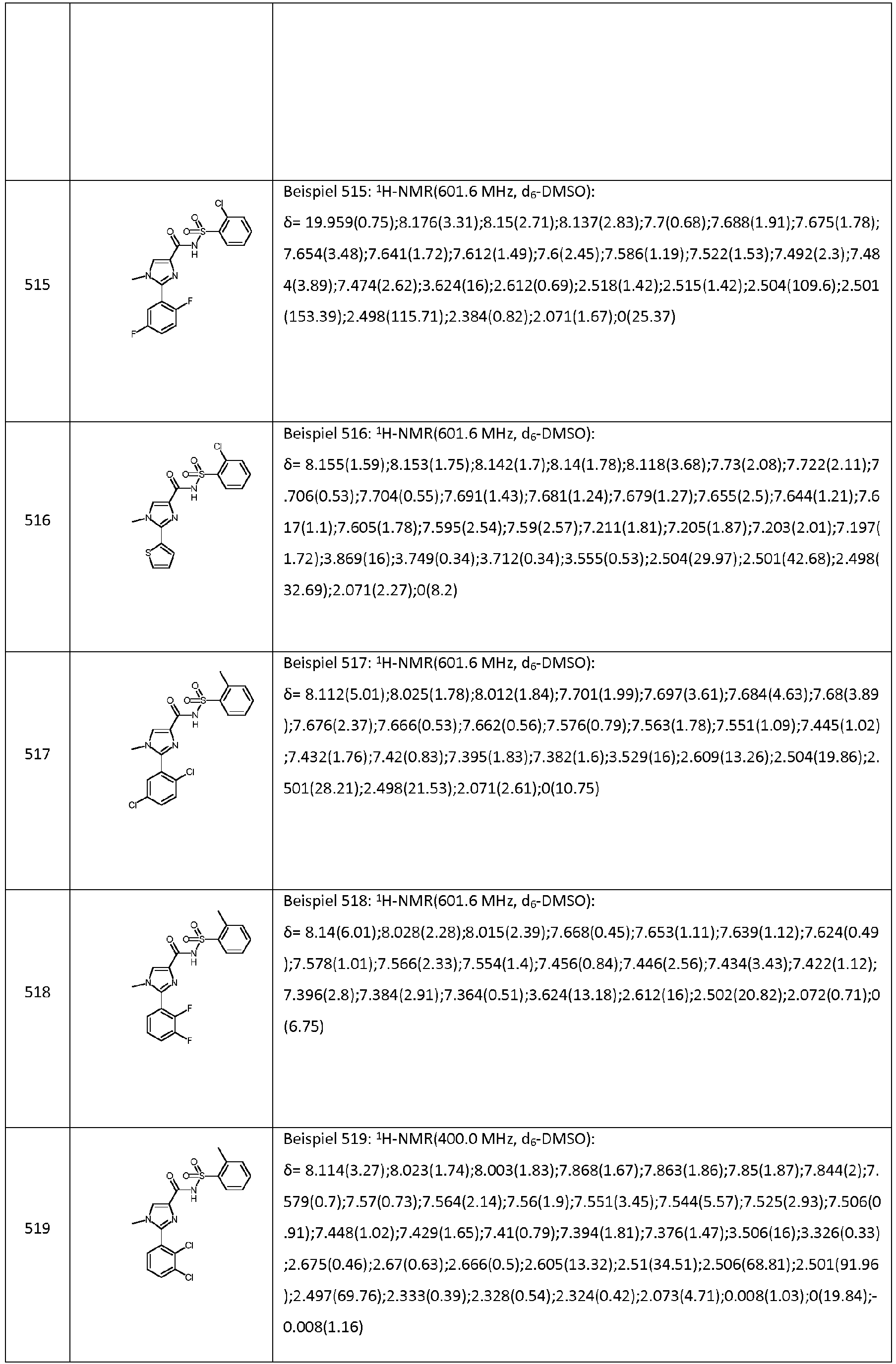

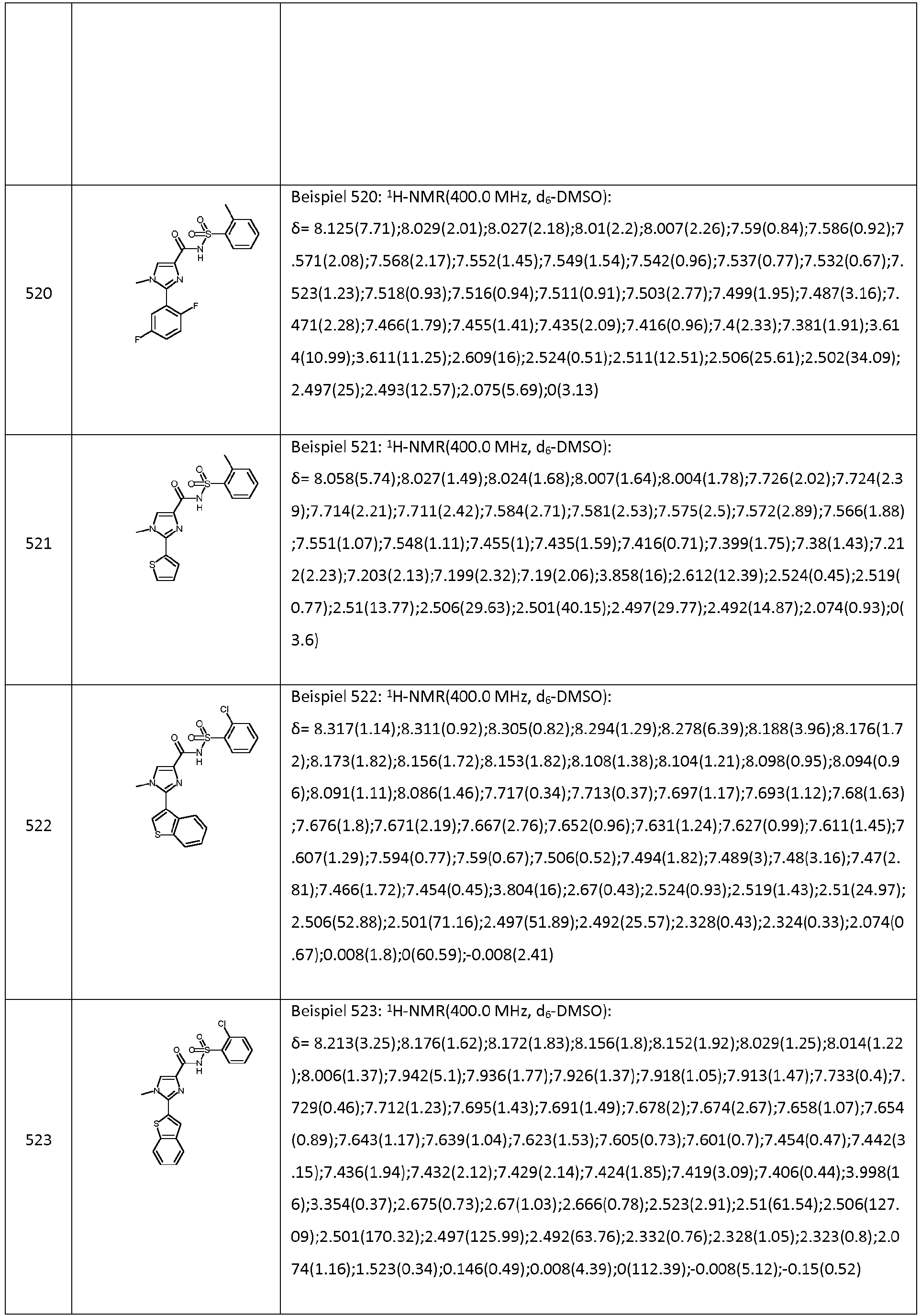

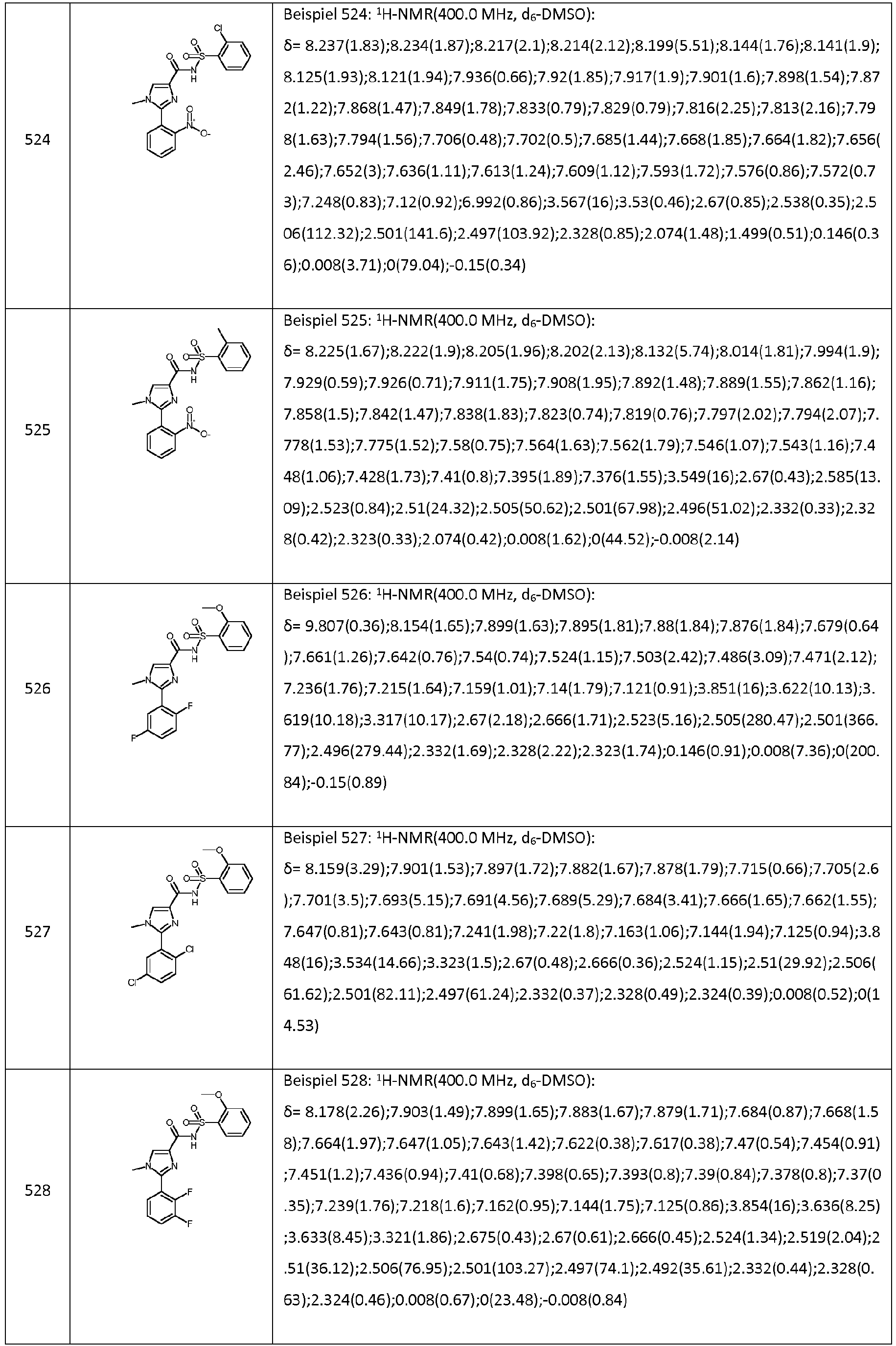

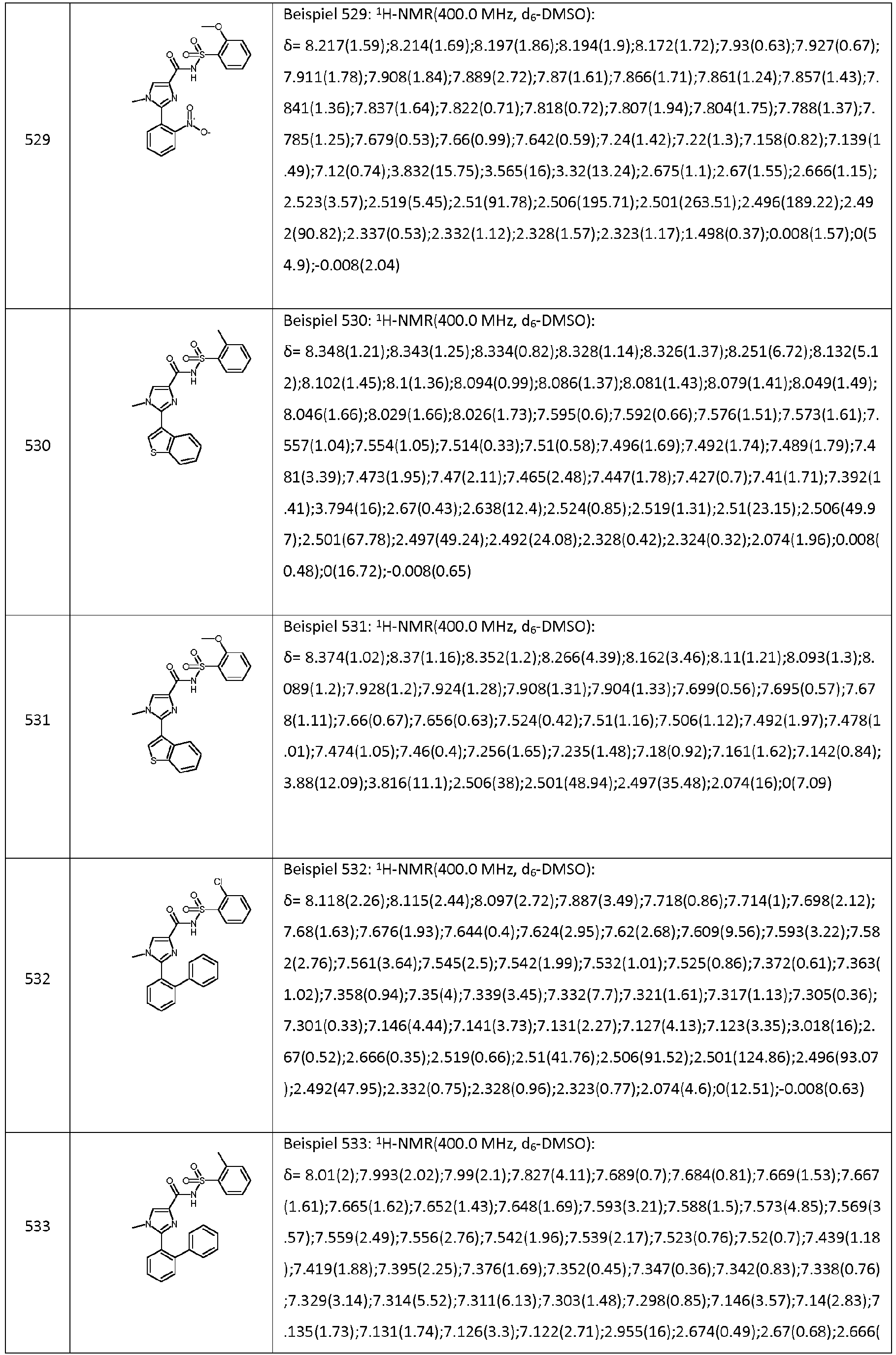

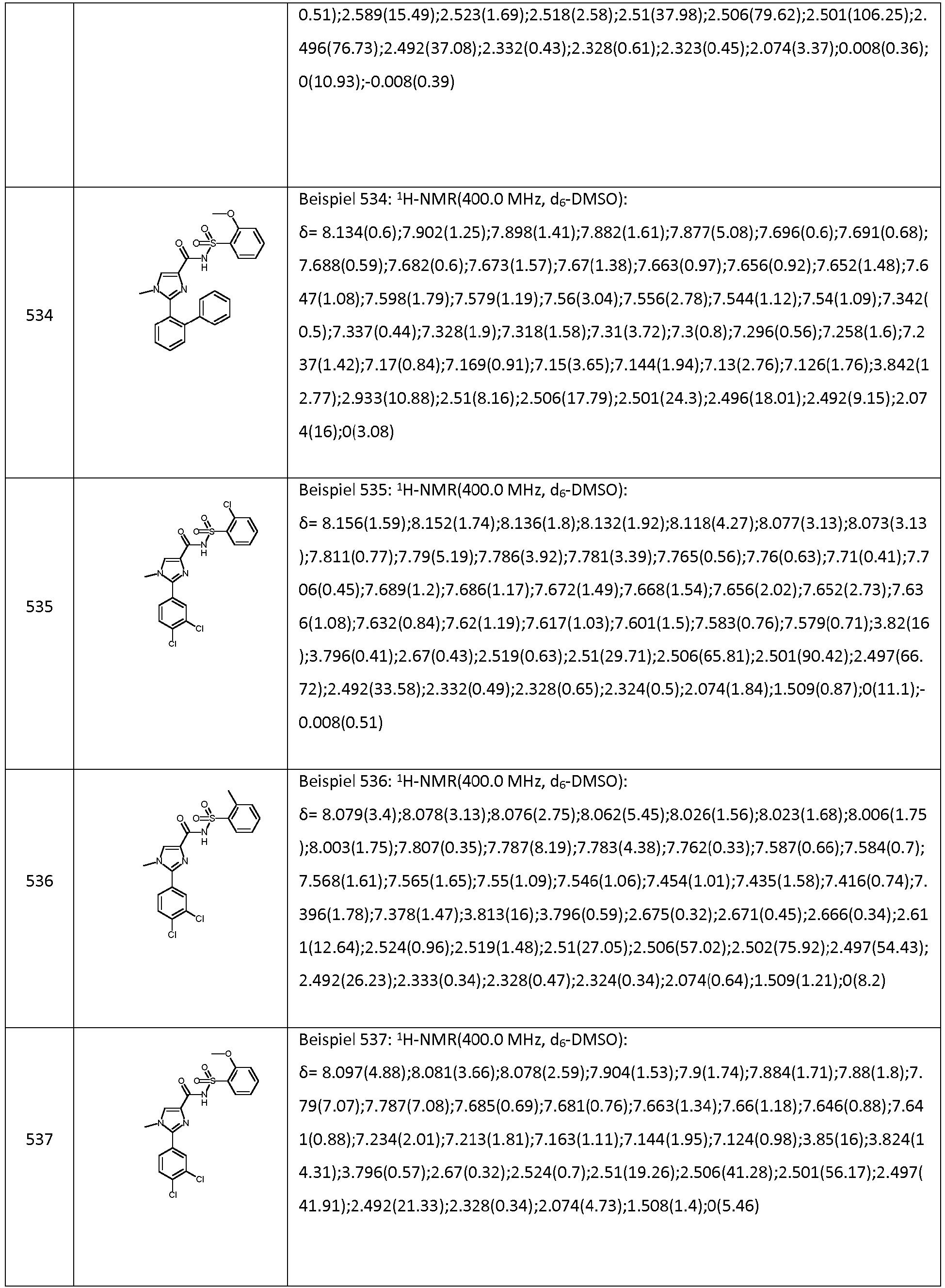

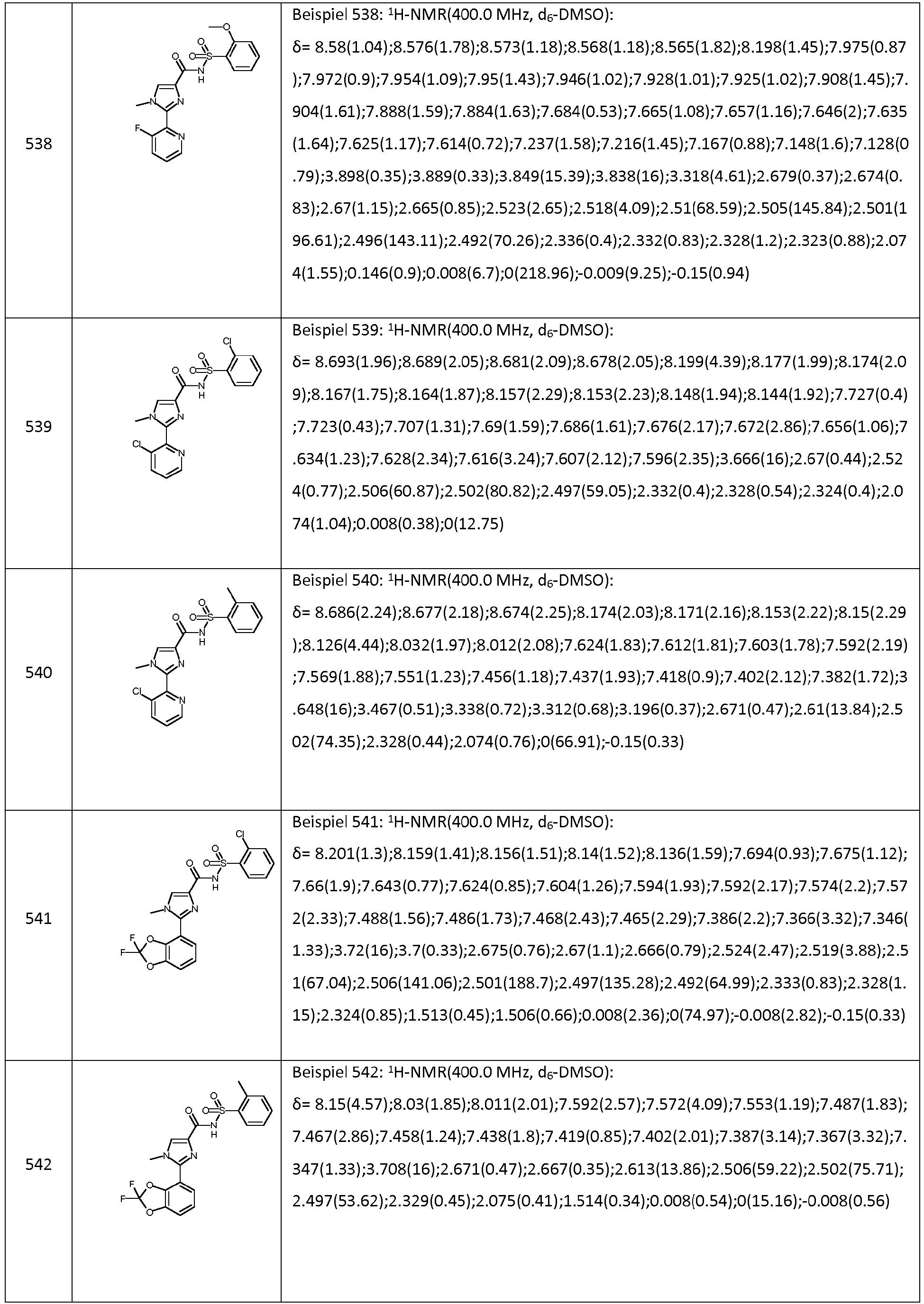

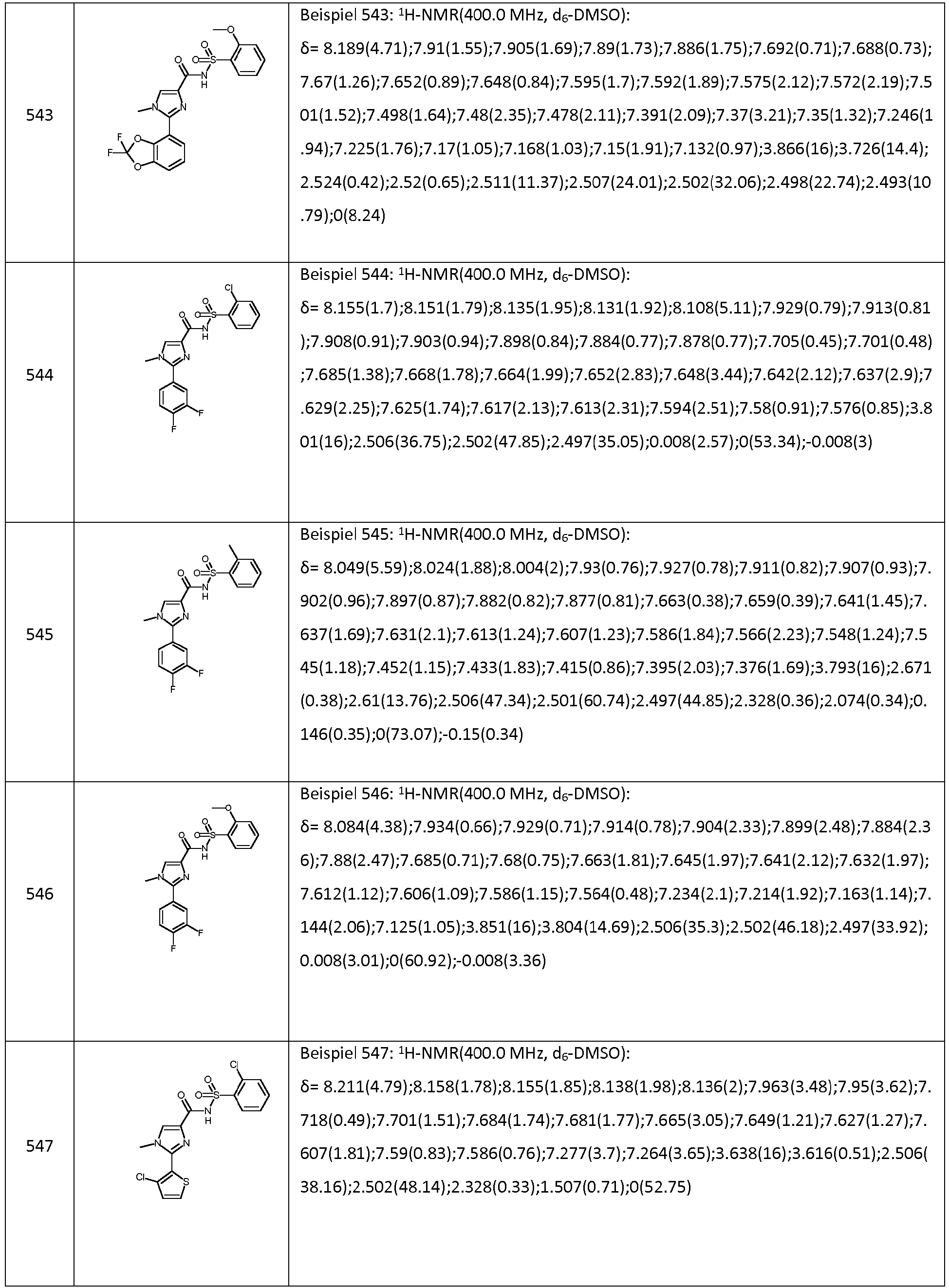

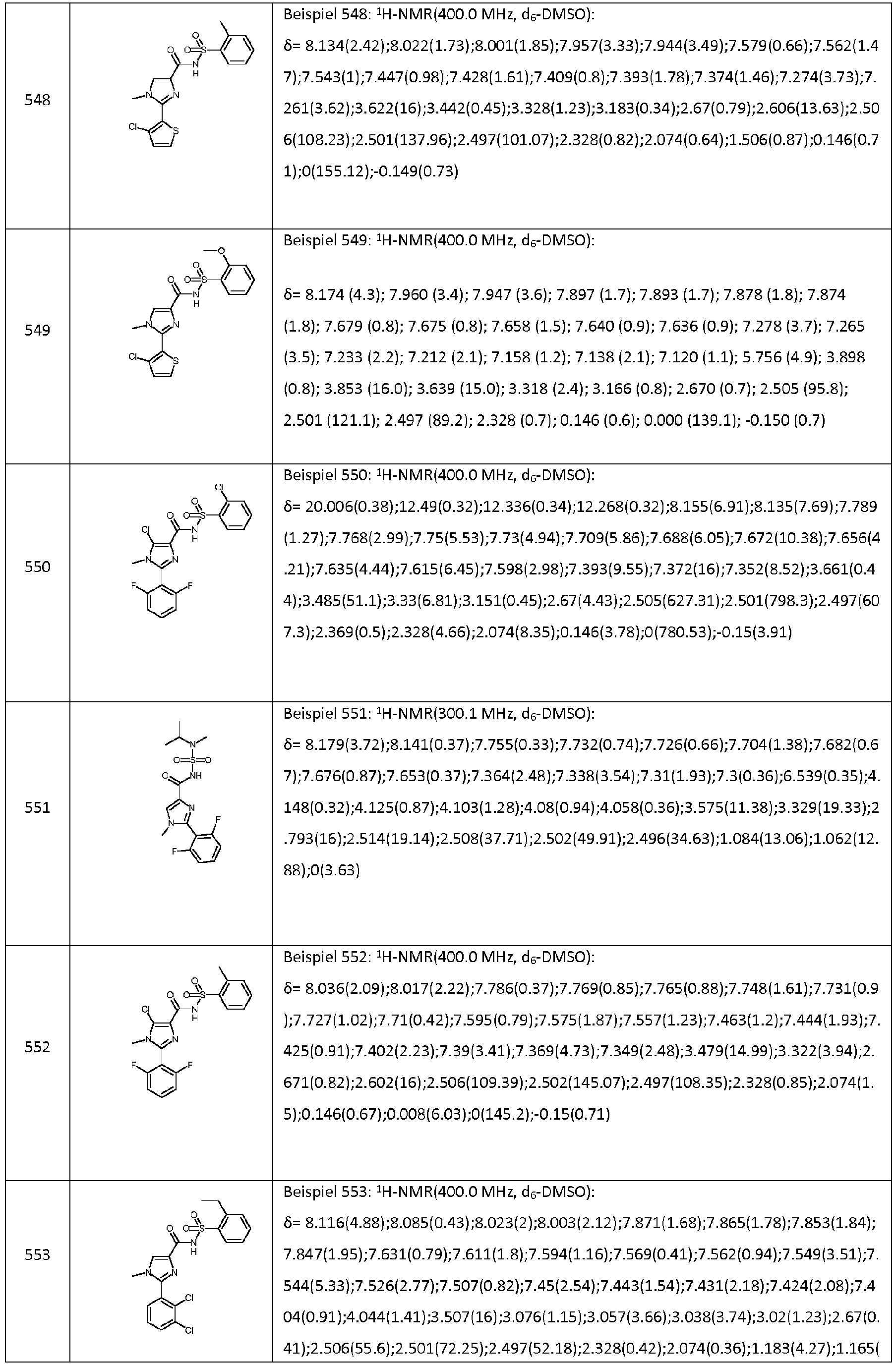

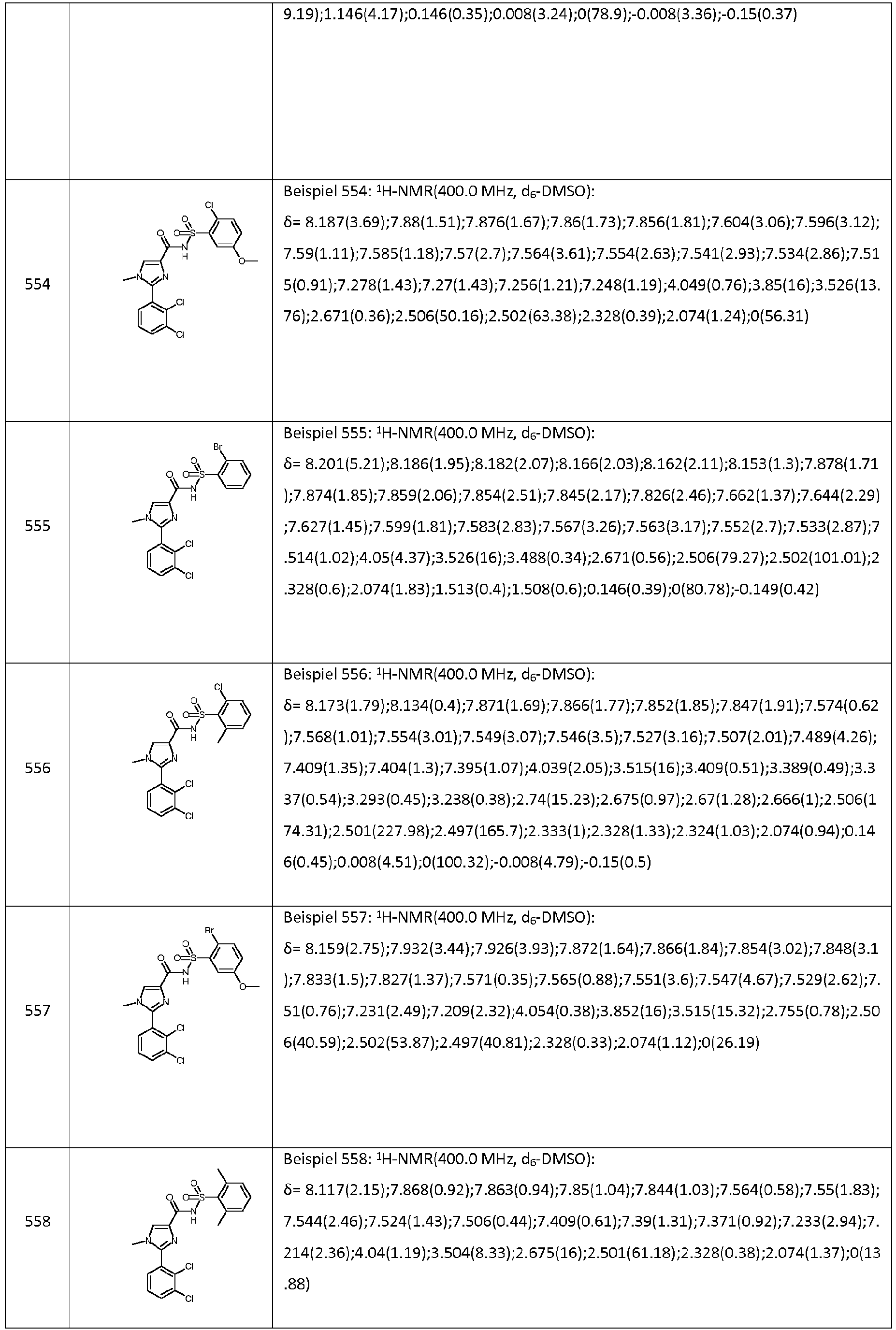

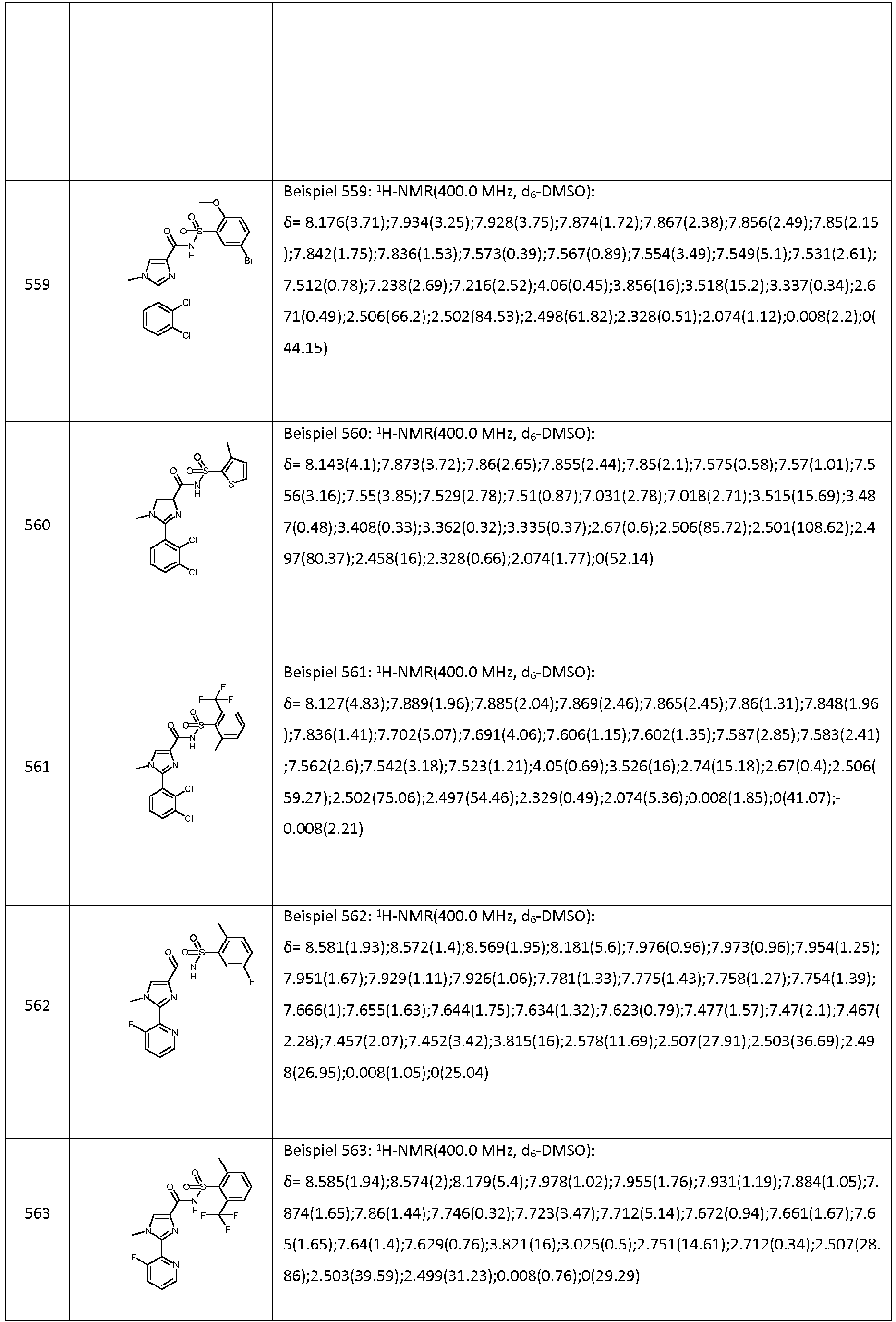

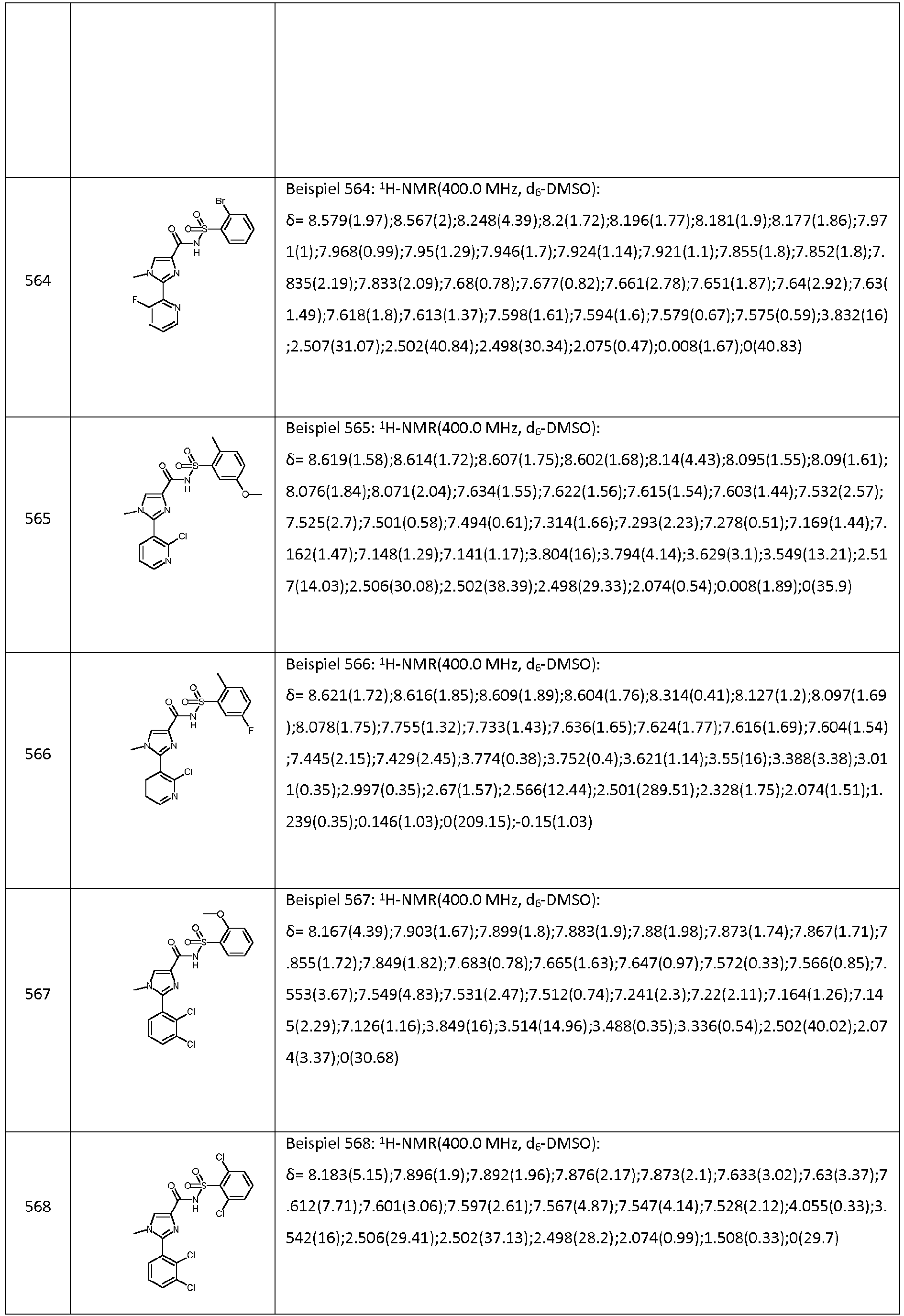

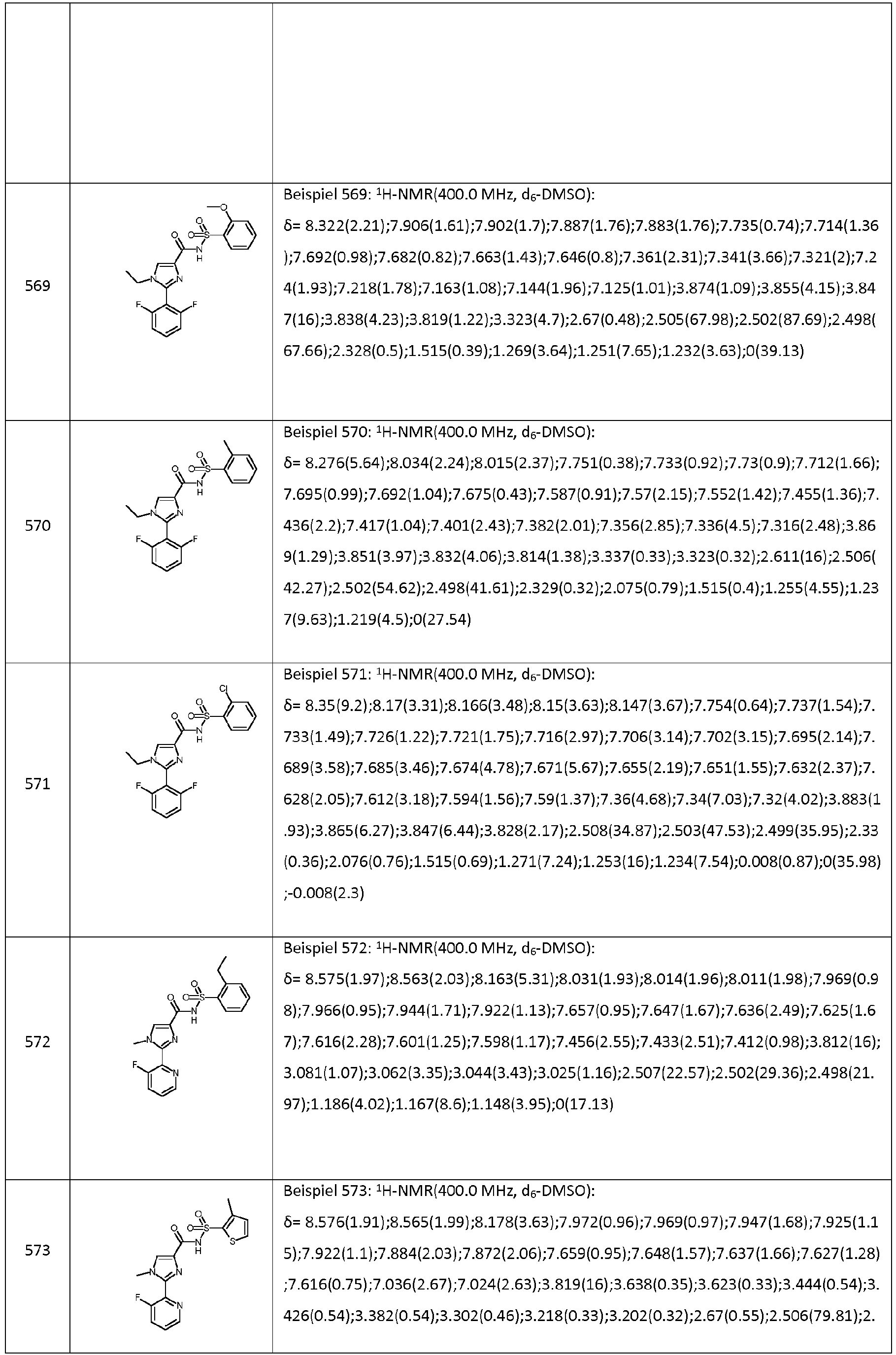

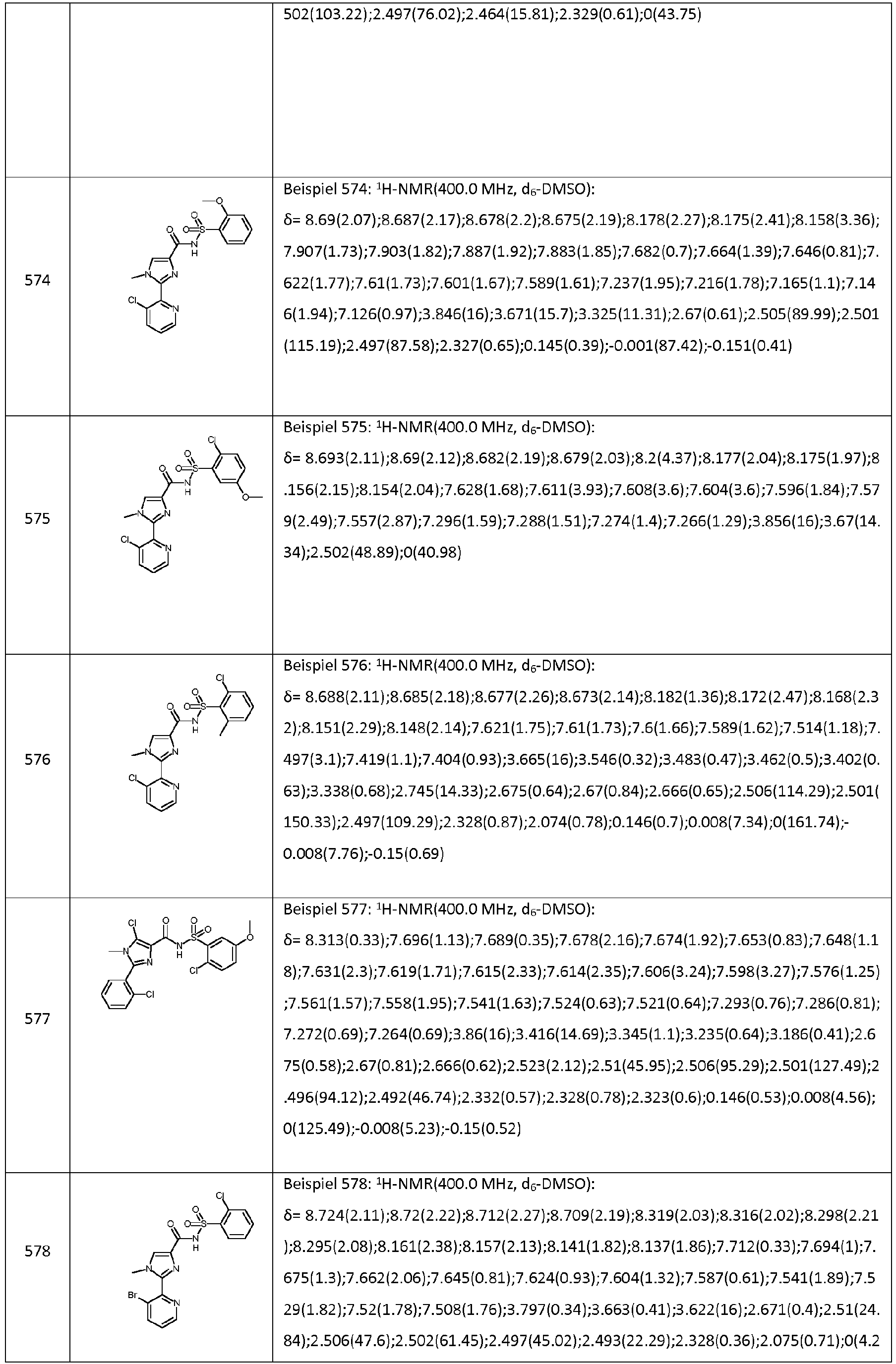

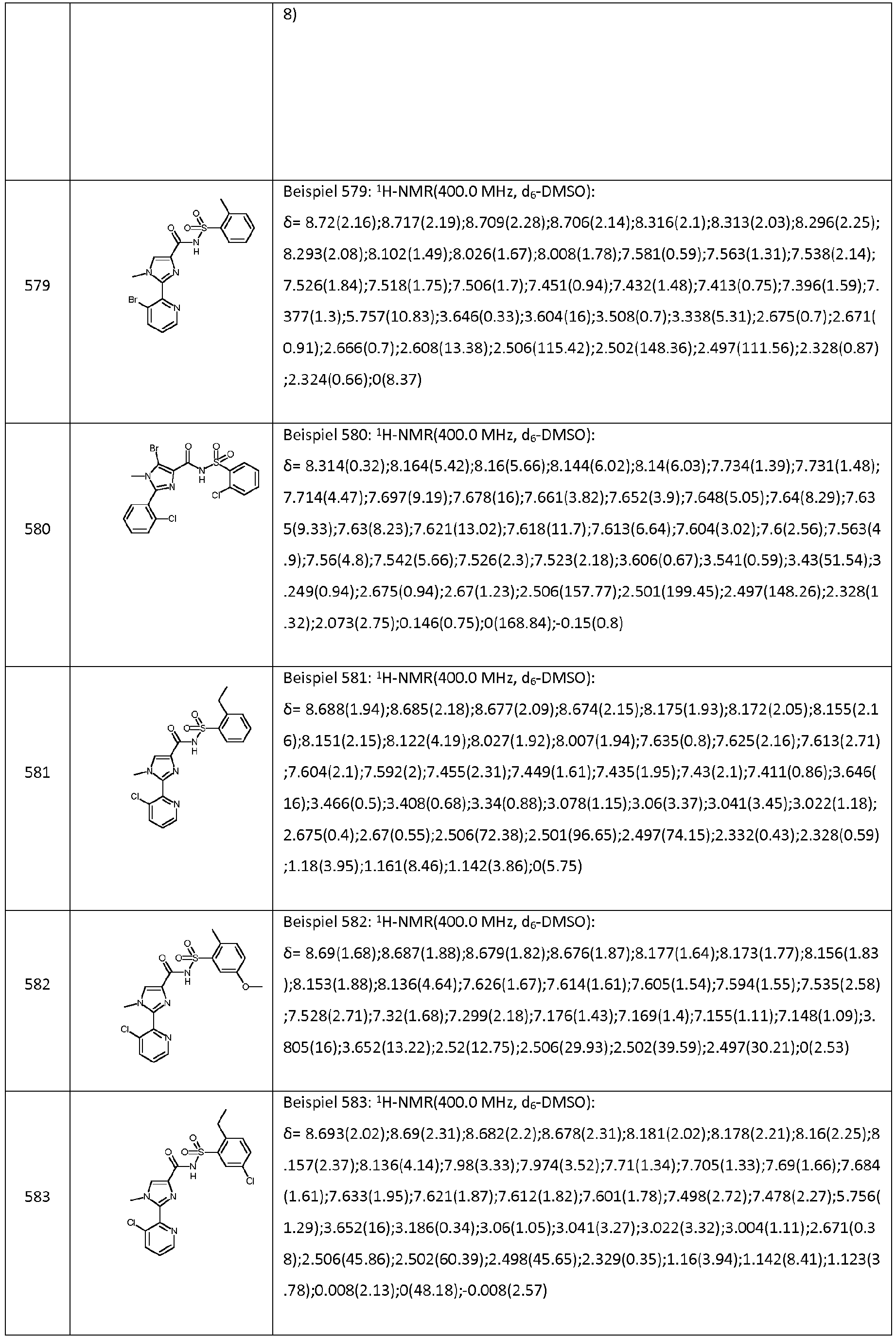

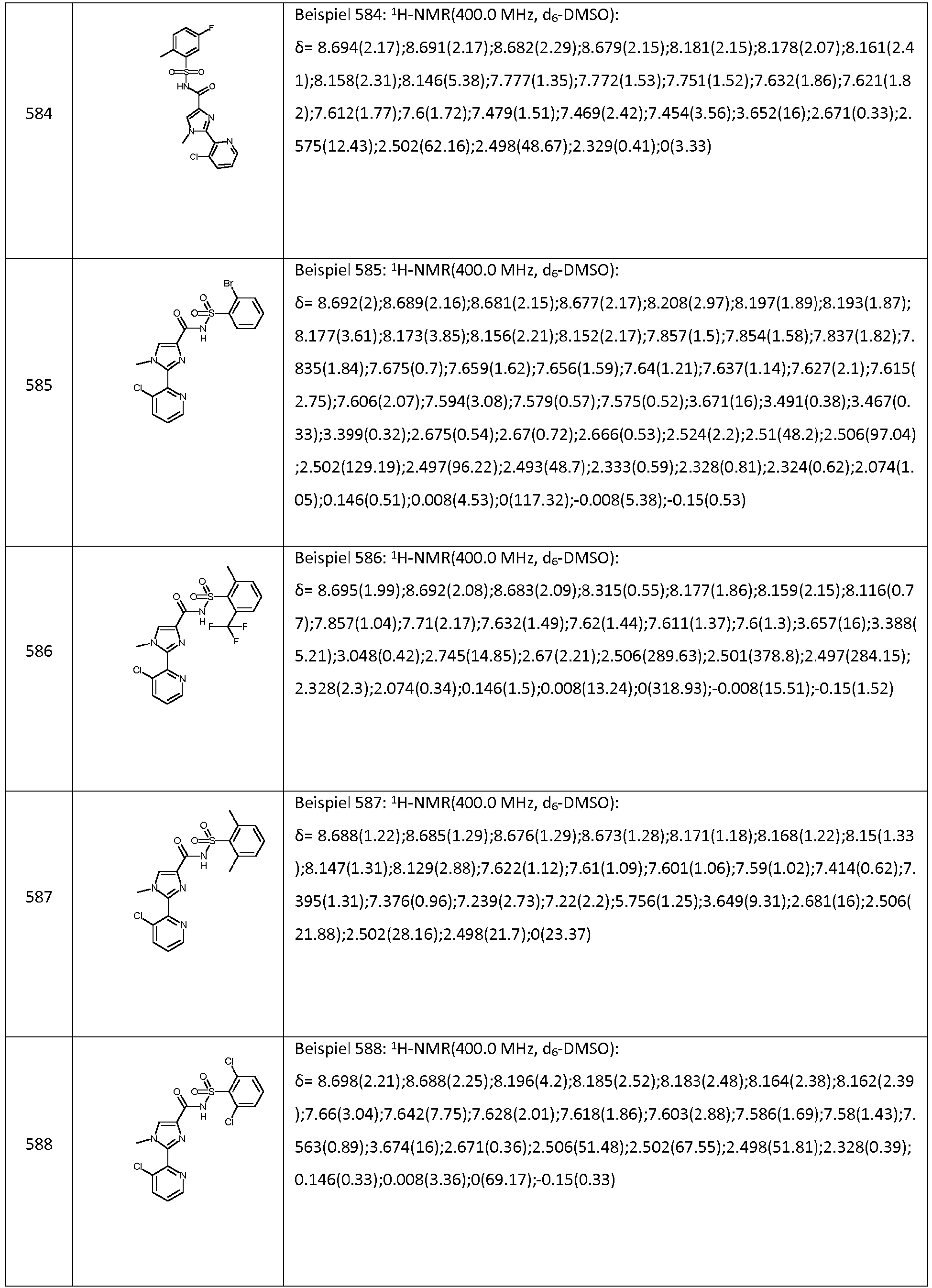

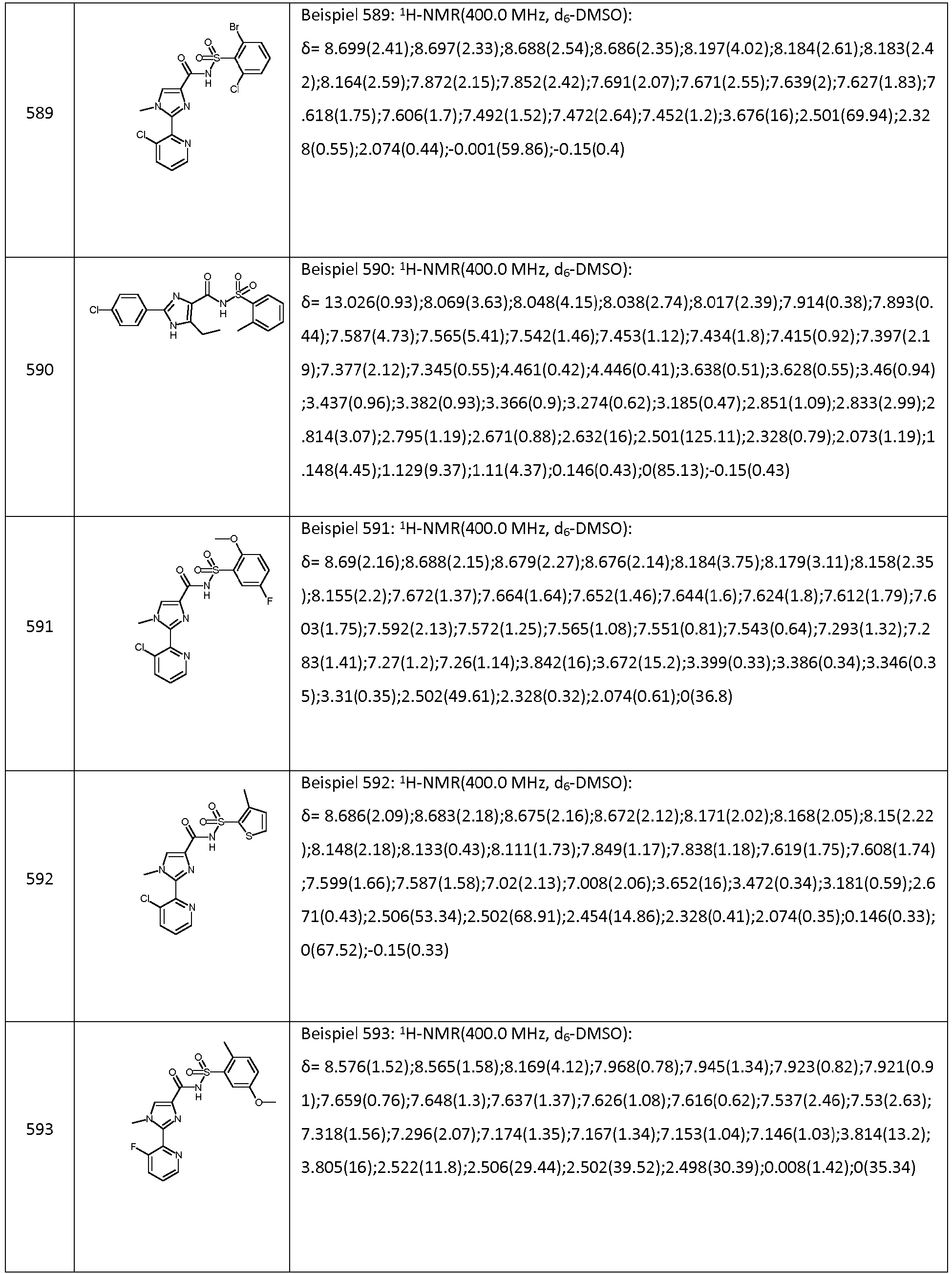

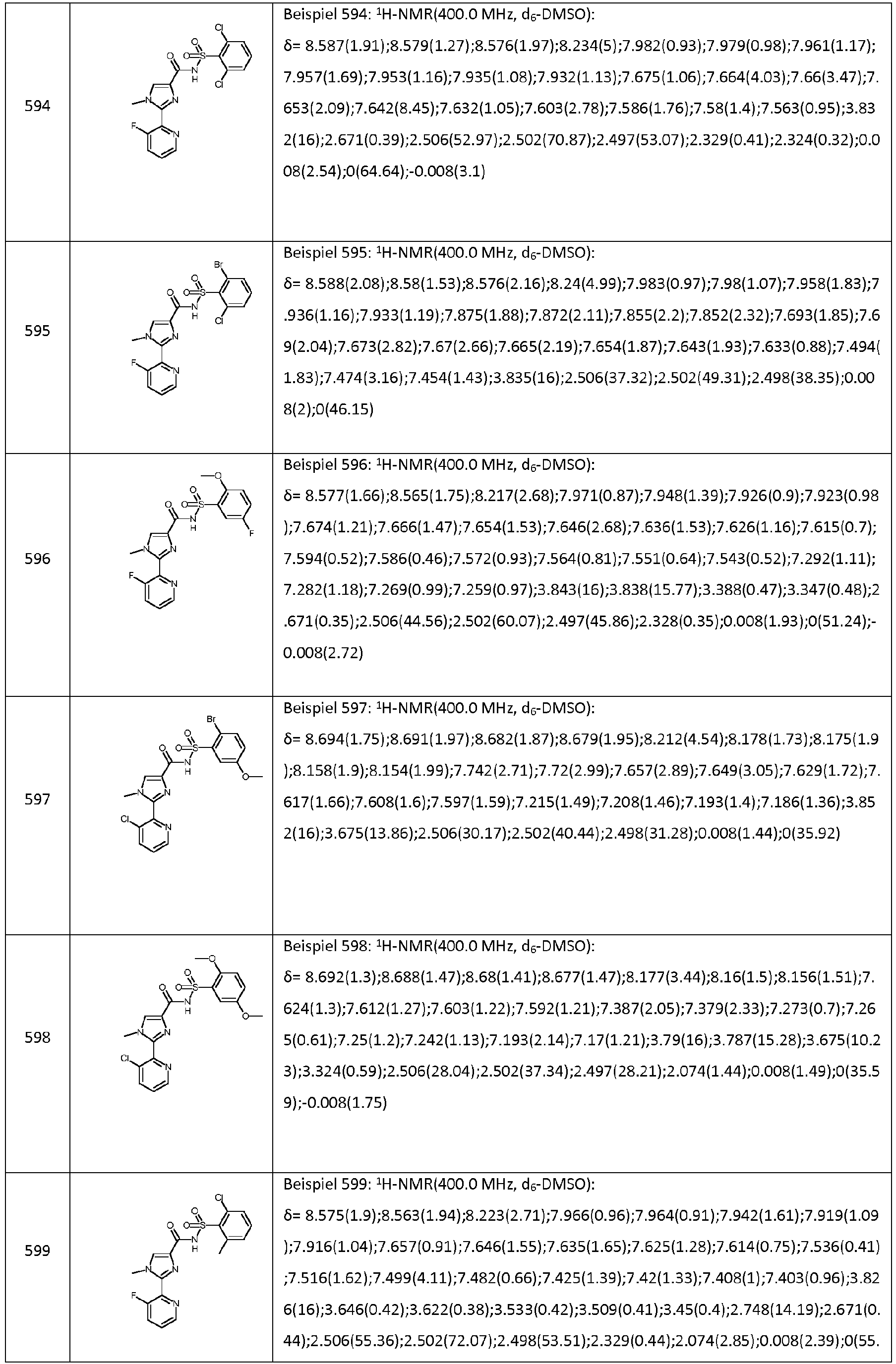

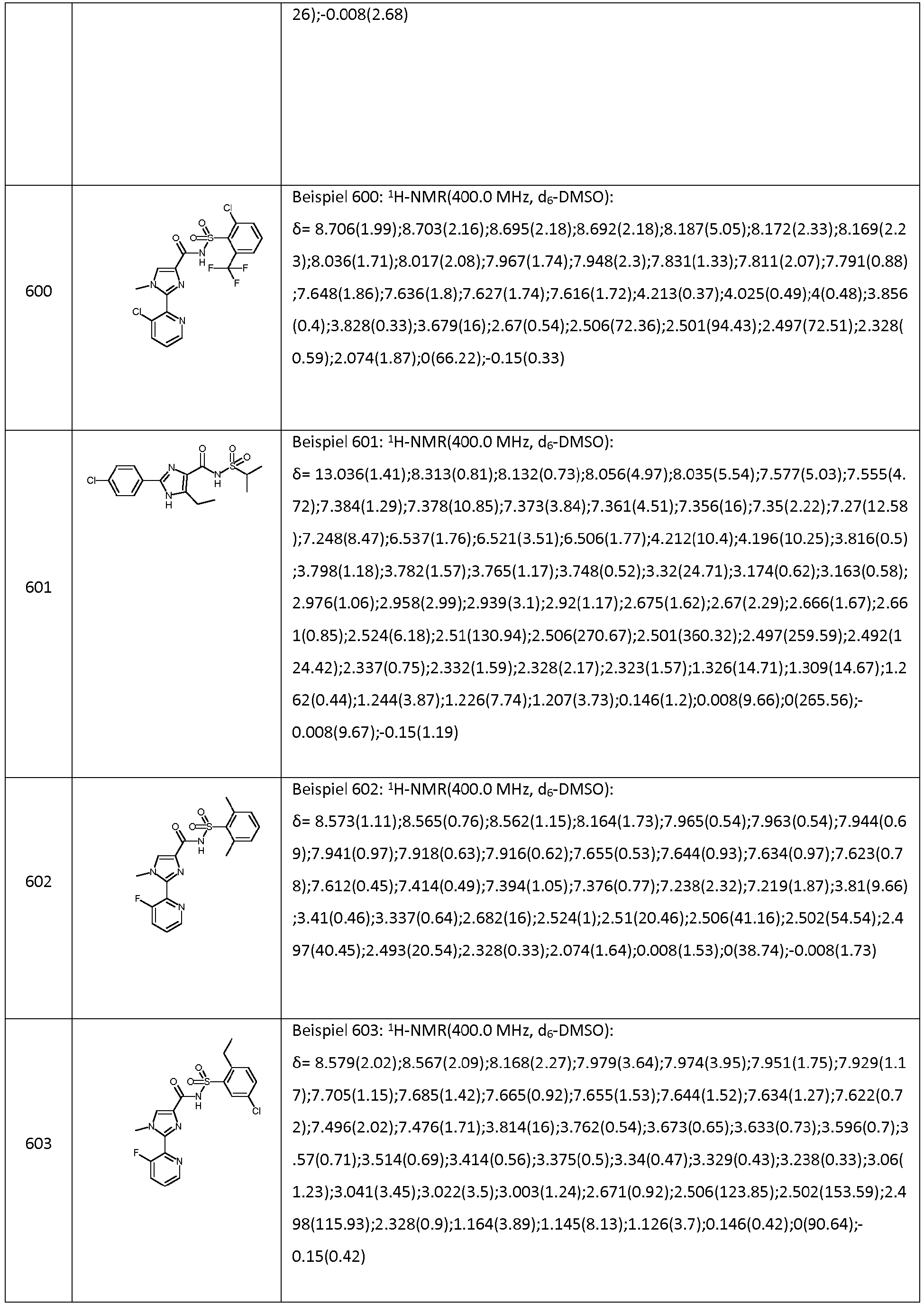

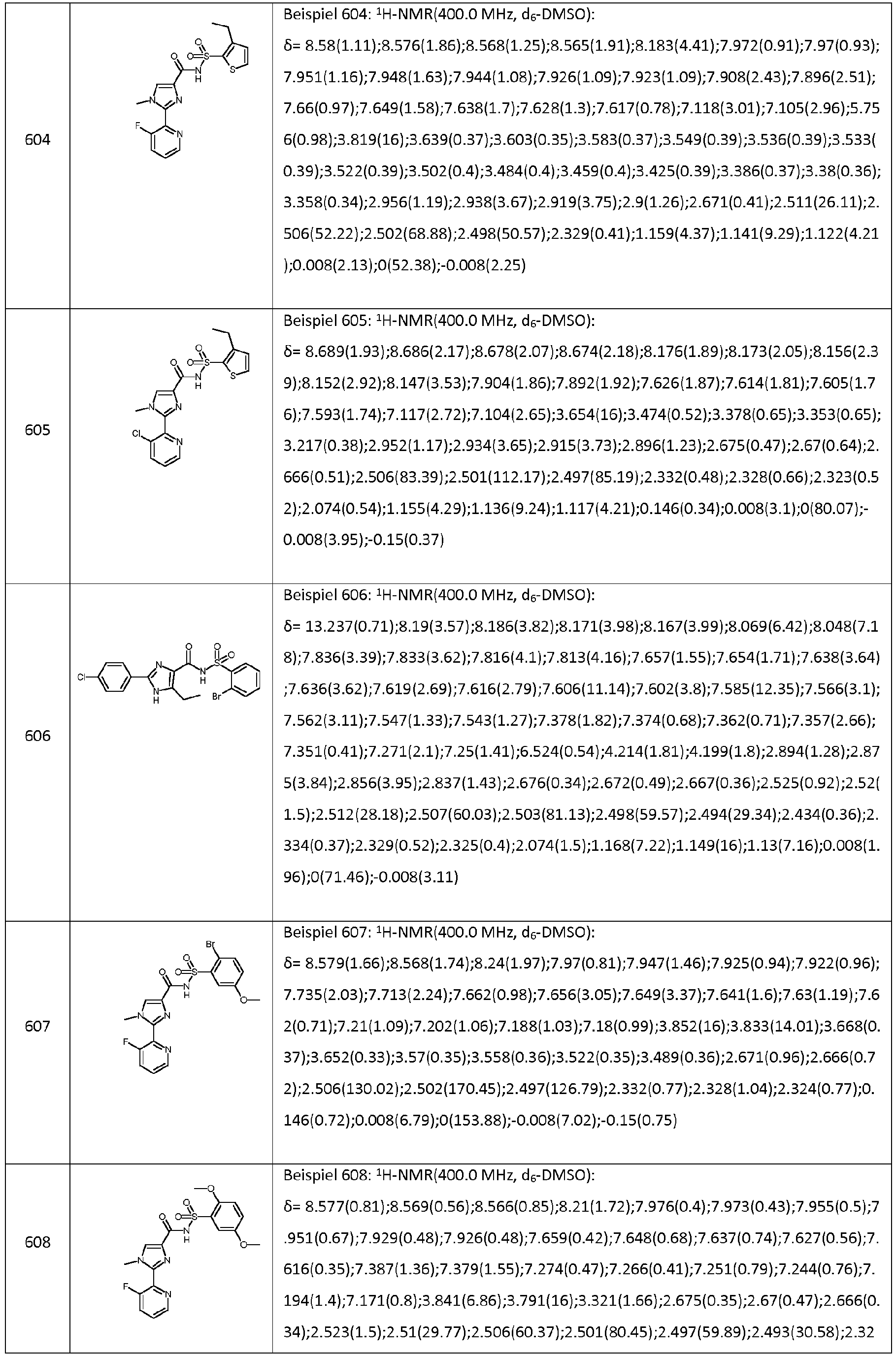

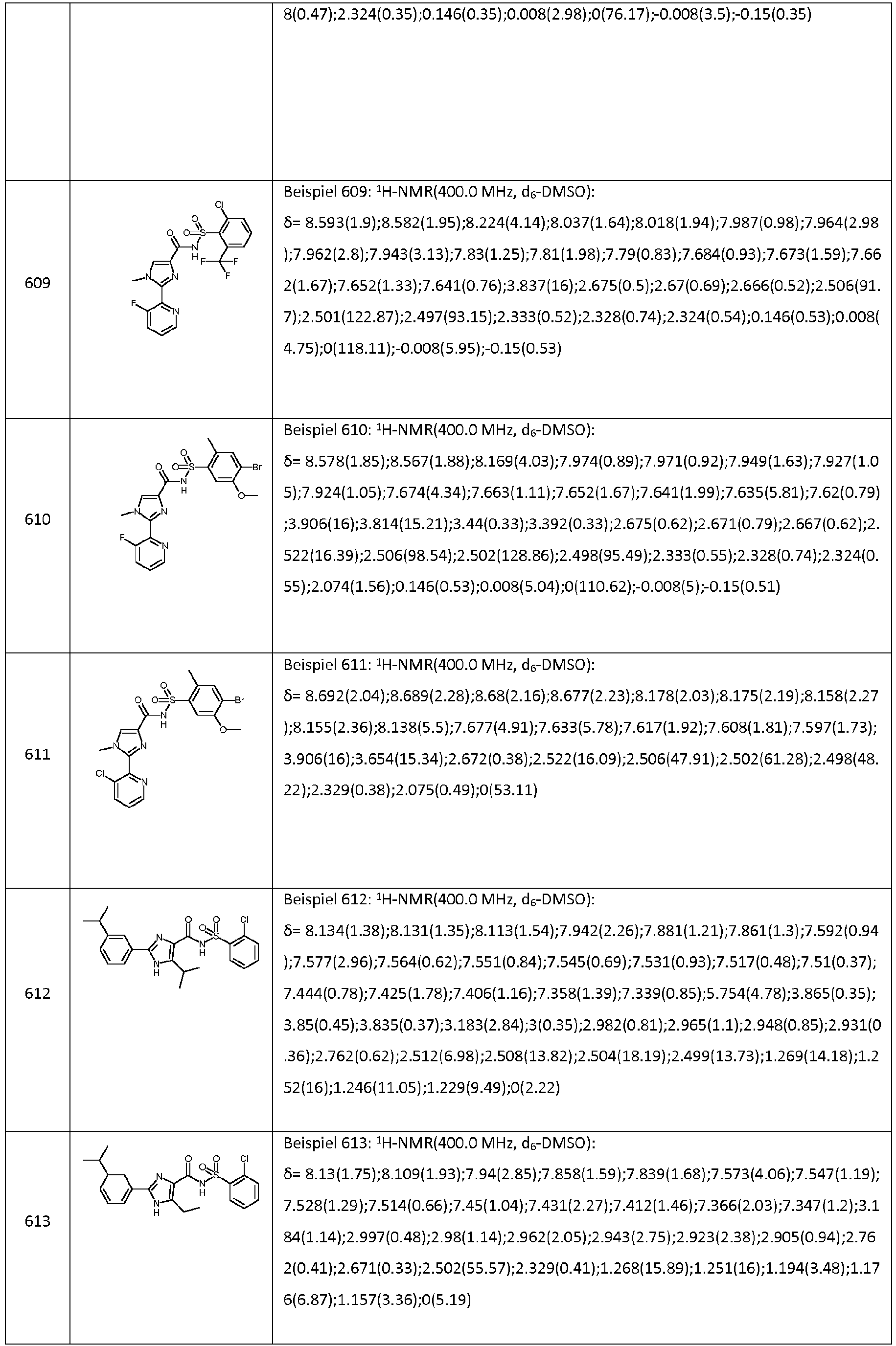

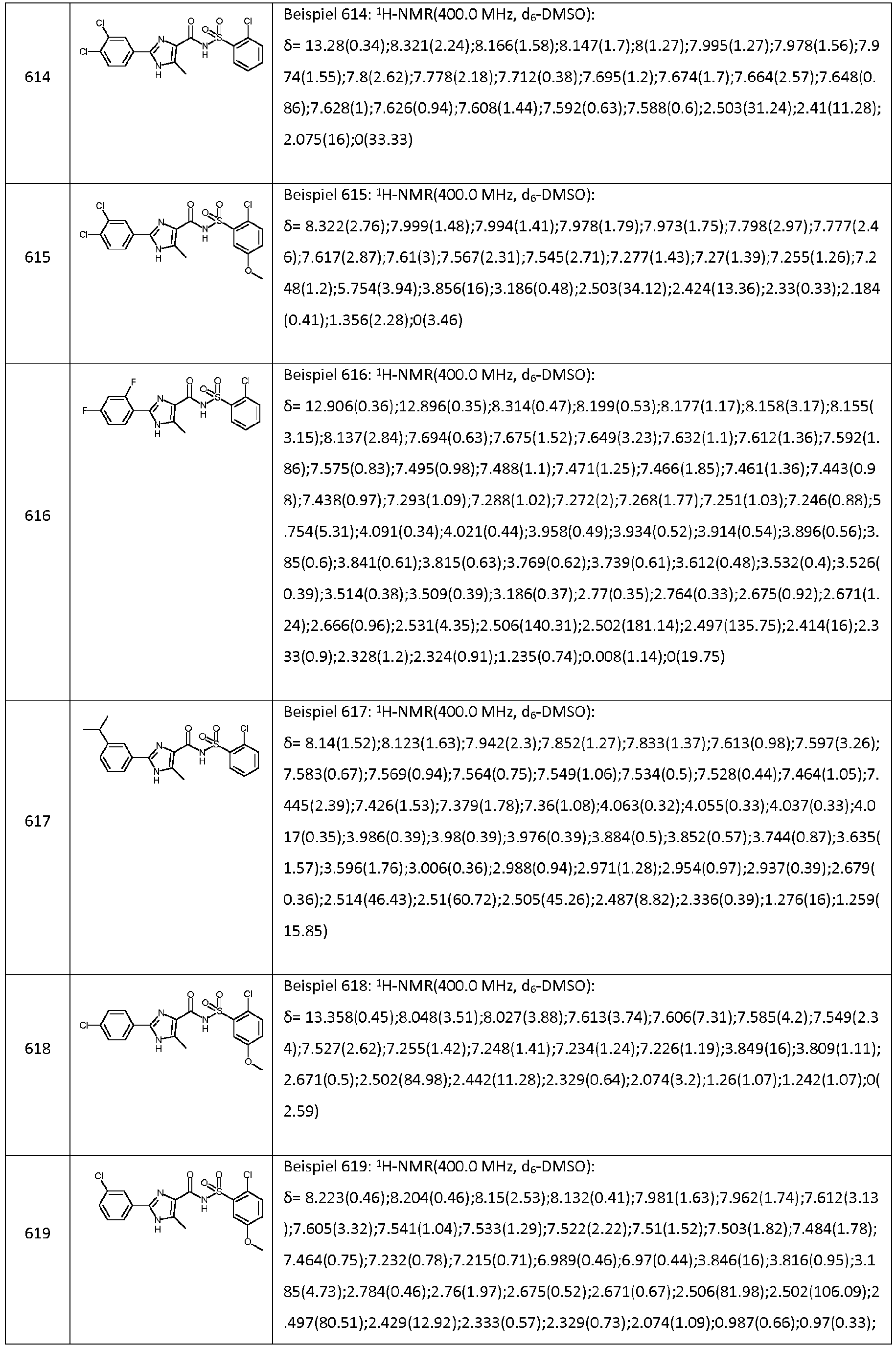

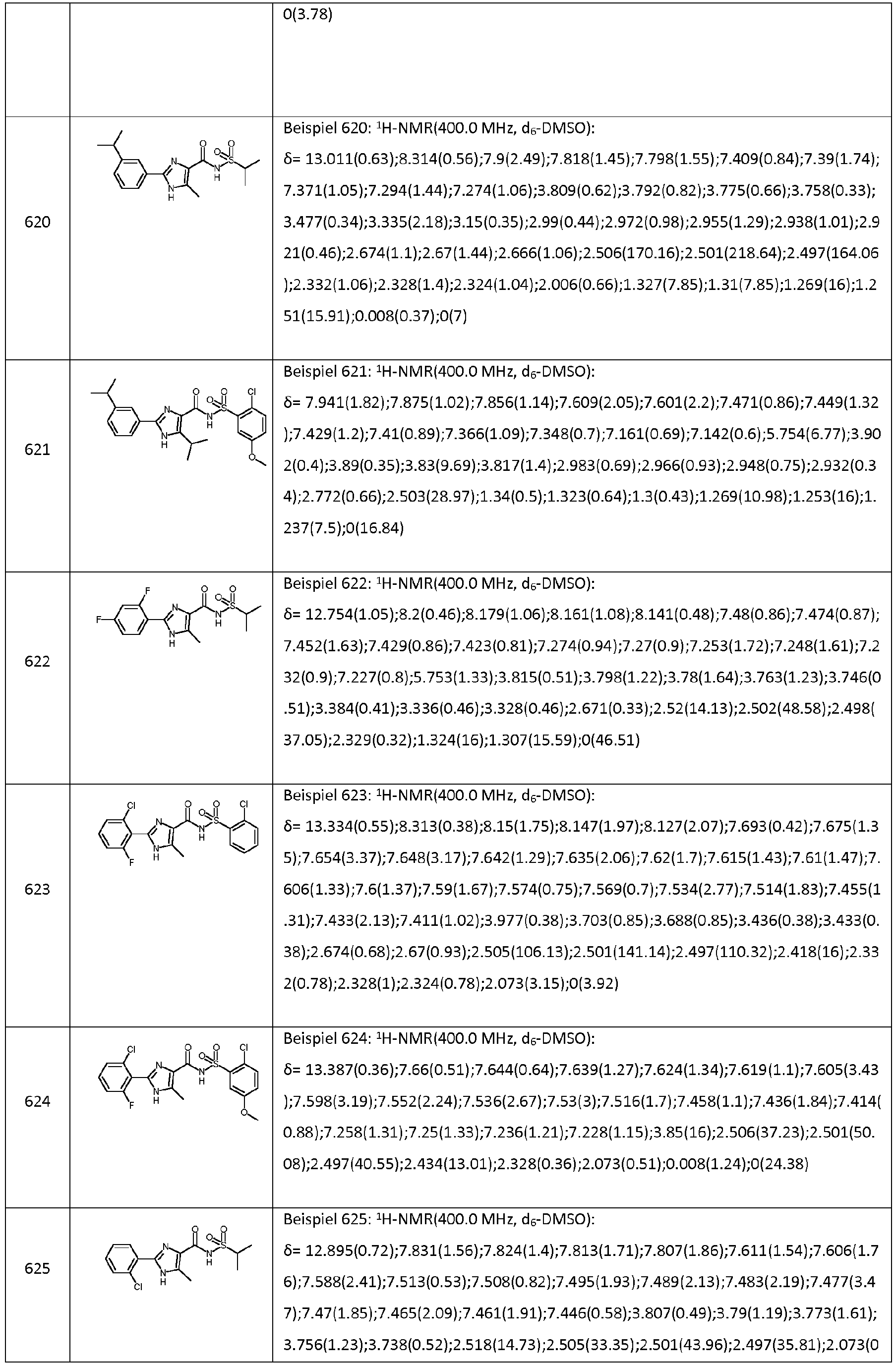

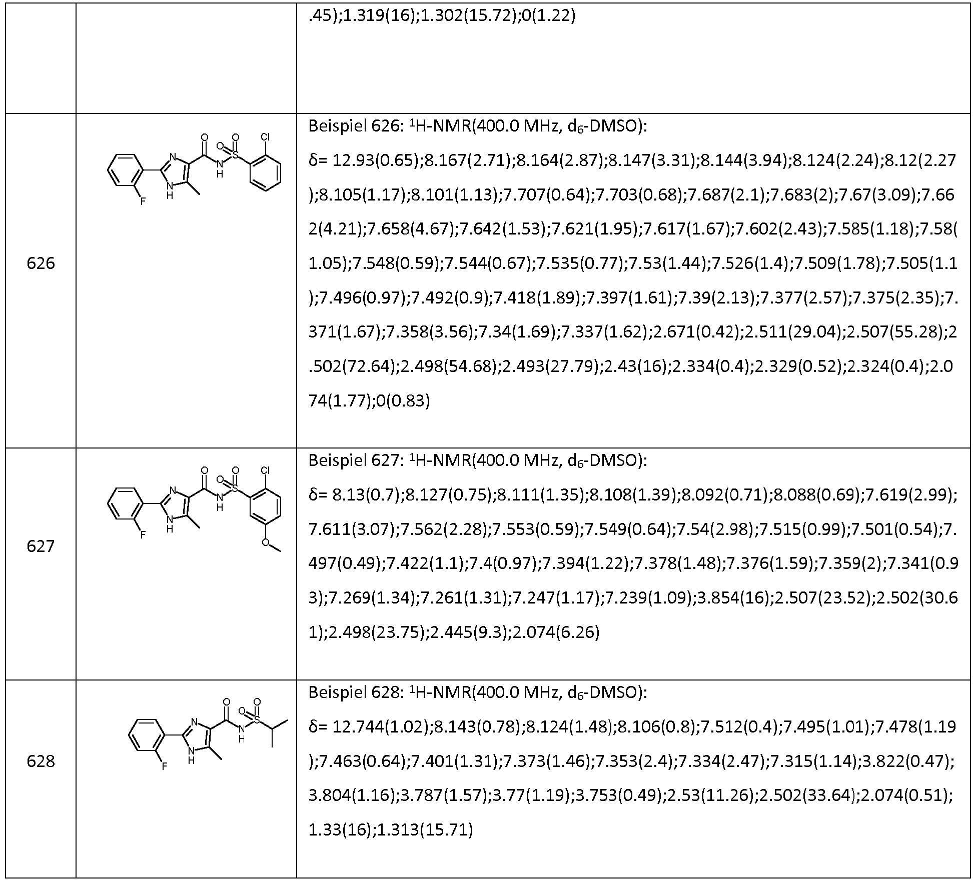

### Anwendungsbeispiele

### Boophilus microplus -Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken (Boophilus microplus) injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 80% bei einer Aufwandmenge von 20µg/Tier: 219

### Lucilia cuprina - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (Lucilia cuprina) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: 18, 23, 25, 27, 36, 37, 54, 56, 68, 101, 102, 107, 109, 120, 186, 218, 219, 231, 285, 341, 364, 383, 397, 401, 519, 539, 558, 572, 576, 584

Bei diesem Test zeigen z.B. die folgenden Verbindungen der Herstellungsbeispiele eine Wirkung von 95% bei einer Aufwandmenge von 100ppm: 92, 108, 274, 336, 394

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen (Musca domestica) besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100ppm: 107, 151, 231, 394, 576

Bei diesem Test zeigen z.B. die folgenden Verbindungen der Herstellungsbeispiele eine Wirkung von 90% bei einer Aufwandmenge von 100ppm: 172, 174

Bei diesem Test zeigen z.B. die folgenden Verbindungen der Herstellungsbeispiele eine Wirkung von 85% bei einer Aufwandmenge von 100ppm: 186, 219

Bei diesem Test zeigen z.B. die folgenden Verbindungen der Herstellungsbeispiele eine Wirkung von 80% bei einer Aufwandmenge von 100ppm: 32, 52, 68, 152, 153, 155, 185, 337

### Meloidogyne incognita- Test

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (Meloidogyne incognita) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 100% bei einer Aufwandmenge von 20ppm: 1, 6, 8, 9, 33, 34, 36, 37, 38, 40, 42, 43, 44, 45, 46, 47, 49, 51, 54, 60, 61, 63, 81, 96, 103, 106, 113, 114, 140, 152, 171, 172, 173, 174, 175, 176, 178, 182, 183, 184, 188, 197, 200, 203, 205, 206, 221, 222, 227, 228, 230, 231, 238, 260, 263,264, 265, 267, 269, 270, 271, 272, 275, 276, 279, 281, 282, 285, 286, 294, 307, 309, 310, 311, 315, 329, 336, 338, 339, 341, 352, 362, 364, 380, 414, 415, 417, 429, 434, 440, 441, 443, 444, 445, 449, 450, 456, 457, 484, 496, 502, 511, 513, 517, 519, 520, 525, 535, 537, 541, 542, 544, 545, 546, 547, 560, 561, 577, 580, 581, 599, 600, 614, 616, 618, 623, 625

Bei diesem Test zeigen z. B. die folgenden Verbindungen der Herstellungsbeispiele Wirkung von 90% bei einer Aufwandmenge von 20ppm: 2, 4, 10, 13, 14, 15, 16, 18, 21, 27, 29, 32, 35, 39, 41, 48, 50, 52, 55, 56, 58, 62, 64, 67, 68, 69, 71, 77, 78, 79, 82, 85, 93, 98, 99, 100, 101, 102, 104, 108, 109, 118, 119, 120, 123, 129, 134, 139, 141, 142, 149, 153, 156, 158, 164, 169, 179, 185, 186, 187, 192, 193,194, 195, 199, 202, 207, 209, 212, 216, 217, 218, 219, 232, 233, 235, 239, 243, 244, 246, 251, 253, 254, 257, 261, 262, 266, 273, 273, 274, 277, 280, 283, 284, 287, 288, 289, 291, 292, 293, 295, 296, 308, 312, 313, 314, 316, 317, 318, 319, 320, 321, 323, 324, 325, 330, 331, 334, 335, 337, 340, 343, 344, 350, 351, 353, 354, 355, 356, 357, 358, 359, 360, 361, 363, 365, 369, 371, 372, 373, 375, 376, 377, 378, 382, 384, 385, 386, 392, 393, 394, 395, 396, 397, 398, 399, 401, 403, 405, 406, 407, 408, 409, 410, 411, 419, 416, 420, 421, 422, 423, 424, 425, 426, 427, 428, 431, 435, 436, 437, 438, 439, 442, 446, 448, 453, 471, 472, 473, 475, 477, 478, 479, 480, 481, 485, 486, 487, 495, 497, 498, 499, 500, 503, 504, 505, 506, 507, 508, 510, 512, 514, 515, 516, 518, 522, 527, 528, 530, 532, 534, 536, 538, 543, 548, 550, 551, 552, 553, 554, 555, 556, 557, 558, 567, 568, 575, 579, 582, 583, 584, 586, 587, 588, 590, 593, 594, 595, 596, 597, 602, 603, 605, 613, 621, 622, 624, 626, 627

Bei diesem Test zeigen z. B. die folgenden Verbindungen der Herstellungsbeispiele Wirkung von 80% bei einer Aufwandmenge von 20ppm: 225, 226

### Myzus persicae - Sprühtest

| | | |
|---|---|---|
| Lösungsmittel: | 78 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt.

Nach 5-6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha: 1, 6, 11, 13, 18, 22, 23, 25, 26, 27, 28, 29, 36, 42, 53, 54, 56, 57, 66, 75, 79, 84, 86, 87, 90, 92, 94, 96, 97, 100, 102, 107, 108, 109, 111, 115, 116, 117, 118, 119, 120, 138, 140, 142, 147, 151, 153, 156, 157, 158, 164, 169, 173, 174, 181, 183, 185, 186, 193, 200, 204, 206, 211, 212, 218, 219, 220, 225, 226, 227, 228, 229, 230, 235, 238, 269, 271, 272, 273, 274, 277, 278, 283, 284, 290, 294, 295, 298, 301, 303, 304,315, 318, 319, 320, 321, 323, 324, 342, 343, 349, 350, 354, 358, 363, 364, 365, 369, 372, 373, 374, 381, 383, 385, 387, 390, 393, 394, 395, 397, 399, 401, 403, 419, 420, 421, 423, 425, 426, 427, 428, 430, 432, 434, 435, 437, 438, 440, 445, 448, 449, 450, 460, 461, 463, 464, 465, 467, 491, 492, 493, 499, 500, 501, 510, 513, 514, 528, 529, 540, 549, 553, 559, 560, 562, 563, 564, 565, 566, 567, 568, 569, 570, 571, 572, 574, 576, 578, 579, 581, 582, 583, 584, 585, 586, 589, 591, 592, 593, 596, 597, 599, 600, 605, 608, 610

Bei diesem Test zeigen z. B. die folgenden Verbindungen der Herstellungsbeispiele Wirkung von 90% bei einer Aufwandmenge von 500g/ha: 2, 5, 7, 8, 9, 10, 12, 14, 15, 16, 17, 19, 24, 30, 32, 33, 34, 37, 38, 39, 41, 43, 44, 45, 46, 47, 48, 51, 52, 55, 58, 59, 62, 64, 65, 67, 69, 70, 71, 72, 78, 80, 81, 82, 83, 89, 91, 93, 95, 98, 99, 101, 106, 110, 112, 113, 114, 121, 122, 123, 124, 125, 126, 127, 139, 143, 145, 148, 149, 150, 152, 154, 155, 159, 160, 162, 165, 166, 167, 168, 170, 171, 172, 175, 176, 178, 179, 182, 184, 188, 197, 207, 217, 224, 231, 257, 266, 268, 270, 275, 279, 280, 281, 282, 285, 289, 296, 297, 300, 310, 312, 313, 316, 317, 335, 336, 337, 338, 339, 341, 346, 352, 356, 357, 367, 370, 371, 388, 389, 391, 398, 400, 413, 422, 424, 429, 431, 433, 436, 439, 441, 442, 443, 444, 446, 447, 453, 454, 455, 458, 459, 462, 466, 469, 483, 488, 490, 497, 503, 512, 515, 517, 519, 520, 524, 526, 539, 542, 547, 548, 550, 552, 554, 555, 556, 557, 558, 561, 573, 575, 587, 588, 594, 595, 598, 602, 603, 604, 607, 609, 611

Bei diesem Test zeigen z. B. die folgenden Verbindungen der Herstellungsbeispiele Wirkung von 90% bei einer Aufwandmenge von 100g/ha: 351, 378, 613

### Myzus persicae - Sprühtest

| | |
|---|---|
| Lösungsmittel: | 14 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (Capsicum annuum), die stark von der Grünen Pfirsichblattlaus (Myzus persicae) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigen z. B. die folgenden Verbindungen der Herstellungsbeispiele Wirkung von 100% bei einer Aufwandmenge von 100ppm: 68, 415, 538
Bei diesem Test zeigen z. B. die folgenden Verbindungen der Herstellungsbeispiele Wirkung von 99% bei einer Aufwandmenge von 100ppm: 478
Bei diesem Test zeigen z. B. die folgenden Verbindungen der Herstellungsbeispiele Wirkung von 97% bei einer Aufwandmenge von 100ppm: 616
Bei diesem Test zeigen z. B. die folgenden Verbindungen der Herstellungsbeispiele Wirkung von 95% bei einer Aufwandmenge von 100ppm: 518
Bei diesem Test zeigen z. B. die folgenden Verbindungen der Herstellungsbeispiele Wirkung von 90% bei einer Aufwandmenge von 100ppm: 146, 237, 347, 533
Bei diesem Test zeigen z. B. die folgenden Verbindungen der Herstellungsbeispiele Wirkung von 85% bei einer Aufwandmenge von 100ppm: 60
Bei diesem Test zeigen z. B. die folgenden Verbindungen der Herstellungsbeispiele Wirkung von 80% bei einer Aufwandmenge von 100ppm: 536
Bei diesem Test zeigen z. B. die folgenden Verbindungen der Herstellungsbeispiele Wirkung von 80% bei einer Aufwandmenge von 20ppm: 195

### Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (Brassica pekinensis) werden mit einer Wirkstoffzubereitung der gewünschten Konzentration gespritzt und nach dem Abtrocknen mit Larven des Meerrettichblattkäfers (Phaedon cochleariae) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: 2, 18, 23, 27, 36, 37, 42, 56, 58, 100, 101, 102, 104, 105, 106, 107, 108, 120, 143, 152, 153, 164, 165, 172, 186, 188, 195, 196, 205, 216, 218, 221, 222, 231, 279, 280, 282, 285, 286, 289, 290, 294, 300, 304, 307, 308, 309, 313, 316, 318, 319, 324, 334, 336, 337, 338, 339, 340, 341, 361, 363, 364, 369, 370, 373, 383, 385, 387, 394, 397, 398, 399, 401, 405, 423, 424, 425, 428, 429, 430, 434, 436, 439, 445, 446, 448, 449, 450, 461, 464, 465, 466, 467, 469, 479, 502, 510, 517, 519, 540, 550, 556, 558, 561, 576, 577, 579, 581, 583, 584, 586, 587, 597, 599, 603, 610, 611

Bei diesem Test zeigen z. B. die folgenden Verbindungen der Herstellungsbeispiele Wirkung von 83% bei einer Aufwandmenge von 500g/ha: 171, 174, 176, 217, 227, 269, 303, 311, 413, 420, 460, 548, 552, 568, 572, 575, 582, 602

### Tetranychus urticae - Sprühtest, OP-resistent

| | | |
|---|---|---|
| Lösungsmittel: | 78,0 | GewichtsteileAceton |
| | 1,5 | Gewichtsteile Dimethylformamid |
| Emulgator: | | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben (Phaseolus vulgaris), die von allen Stadien der Gemeinen Spinnmilbe (Tetranychus urticae) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: 74, 217, 293

Bei diesem Test zeigen z. B. die folgenden Verbindungen der Herstellungsbeispiele eine Wirkung von 90% bei einer Aufwandmenge von 500g/ha: 46, 70, 78, 82, 87, 101, 104, 173, 195, 212, 291, 315, 318, 351, 406, 407, 495, 568, 573, 582

### Aphis gossypii - Sprühtest

| | |
|---|---|
| Lösungsmittel: | 14 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Baumwollpflanzen (Gossypium hirsutum), die stark von der Baumwollblattlaus (Aphis gossypii) befallen sind, werden mit einer Wirkstoffzubereitung der gewünschten Konzentration gespritzt.

Nach 6 Tagen wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele Wirkung von 100% bei einer Aufwandmenge von 100ppm: 194
Bei diesem Test zeigt z. B. die folgende Verbindungen der Herstellungsbeispiele Wirkung von 98% bei einer Aufwandmenge von 100ppm: 103
Bei diesem Test zeigt z. B. die folgende Verbindungen der Herstellungsbeispiele Wirkung von 95% bei einer Aufwandmenge von 100ppm: 502

### Diabrotica balteata - Sprühtest

| | | |
|---|---|---|
| Lösungsmittel: | 78 | Gewichtsteile Aceton |
| | 1,5 | Gewichtsteile Dimethylformamid |
| Emulgator: | | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Vorgequollene Weizenkörner (Triticum aestivum) werden in einer mit Agar und etwas Wasser gefüllten Multiwell-Platte für einen Tag inkubiert (5 Saatkörner pro Kavität). Die gekeimten Weizenkörner werden mit einer Wirkstoffzubereitung der gewünschten Konzentration gespritzt. Anschließend wird jede Kavität mit 10-20 Käferlarven von Diabrotica balteata infiziert.

Nach 7 Tagen wird die Wirkung in % bestimmt. Dabei bedeutet 100 %, dass alle Maispflanzen wie in der unbehandelten, nicht infizierten Kontrolle gewachsen sind; 0 % bedeutet, dass keine Maispflanze gewachsen ist.

Bei diesem Test zeigen z. B. die folgenden Verbindungen der Herstellungsbeispiele Wirkung von 100% bei einer Aufwandmenge von 160µg/Kavität: 102, 218, 279, 336, 337, 339, 341, 364, 369, 394, 429, 518, 519, 558

Bei diesem Test zeigen z. B. die folgenden Verbindungen der Herstellungsbeispiele Wirkung von 80% bei einer Aufwandmenge von 160µg/Kavität: 280, 282, 361, 398, 399, 401, 405, 423, 424, 446, 450, 510

Bei diesem Test zeigen z. B. die folgenden Verbindungen der Herstellungsbeispiele Wirkung von 80% bei einer Aufwandmenge von 80µg/Kavität: 363, 397

### Myzus persicae - Oraltest

| | | |
|---|---|---|
| Lösungsmittel: | 100 | Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus (Myzus persicae), die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 20ppm: 1, 11, 13, 14, 15, 17, 18, 19, 21, 22, 23, 25, 26, 27, 28, 29, 30, 32, 33, 36, 37, 38, 40, 49, 52, 53, 54, 55, 56, 57, 58, 59, 60, 61, 62, 63, 66, 67, 68, 69, 70, 71, 91, 92, 93, 94, 95, 96, 97, 98, 99, 100, 101, 102, 103, 104, 106, 107, 108, 109, 110, 111, 112, 113, 114, 116,118, 119, 120, 124, 125, 132, 134, 140, 147, 148, 152, 153, 155, 156, 157, 158, 159, 162, 163, 169, 170, 171, 172, 173, 174, 178, 179, 180, 182, 183, 184, 185, 186, 187, 188, 192, 194, 195, 196, 198, 199, 201, 202, 205, 211, 212, 213, 214, 216, 216, 217, 218, 219, 220, 221, 225, 226, 233, 235, 237, 238, 239, 245, 247, 248, 257, 265, 266, 268, 269, 270, 271, 272, 273, 274, 275, 276, 277, 278, 279, 280, 281, 282, 283, 284, 285, 286, 289, 290, 294, 295, 297, 298, 300, 302, 303, 304, 306, 311, 312, 313, 314, 315, 316, 317, 318, 319, 320, 323, 324, 325, 331, 336, 337, 338, 339, 341, 342, 348, 352, 354, 358, 363, 364, 367, 369, 370, 371, 372, 373, 378, 381, 383, 385, 386, 387, 388, 389, 390, 393, 394, 397, 398, 399, 401, 403, 405, 411, 414, 415, 419, 420, 421, 422, 423, 424, 425, 426, 427, 428, 429, 430, 431, 432, 433, 434, 435, 436, 437, 439, 440, 443, 444, 446, 447, 448, 449, 452, 453, 454, 456, 459, 461, 463, 465, 466, 467, 469, 470, 478, 483, 490, 491, 492, 497, 498, 499, 500, 501, 502, 503, 509, 510, 511, 512, 513, 514, 515, 517, 518, 519, 520, 524, 525, 528, 532, 533, 536, 538, 539, 540, 549, 553, 554, 555, 556, 557, 558, 559, 560, 561, 562, 563, 564, 565, 566, 567, 568, 569, 570, 571, 572, 573, 574, 575, 576, 578, 579, 583, 584, 585, 587, 588, 589, 590, 591, 592, 593, 594, 595, 596, 597, 598, 599, 600, 602, 603, 605, 607, 609, 610, 611, 612, 613, 615, 616, 617, 618, 619
Bei diesem Test zeigen z. B. die folgenden Verbindungen der Herstellungsbeispiele Wirkung von 90% bei einer Aufwandmenge von 20ppm: 7, 12, 39, 41, 43, 44, 45, 51, 75, 77, 81, 115, 117, 126, 130, 131, 136, 137, 141, 143, 150, 165, 176, 181, 191, 206, 208, 209, 215, 236, 296, 299, 301, 305, 327, 330, 343, 345, 347, 350, 353, 368, 395, 412, 416, 418, 438, 445, 446, 450, 462, 464, 477, 496, 531, 547, 548, 601, 604, 620, 625
Bei diesem Test zeigen z. B. die folgenden Verbindungen der Herstellungsbeispiele Wirkung von 100% bei einer Aufwandmenge von 4ppm: 581, 582, 586

## Patentansprüche

1. Verwendung einer Verbindung der Formel (I) 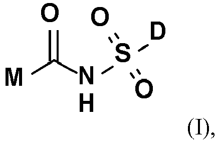 in der
M für einen Rest ausgewählt aus den Formeln (IIa-IIf) steht: 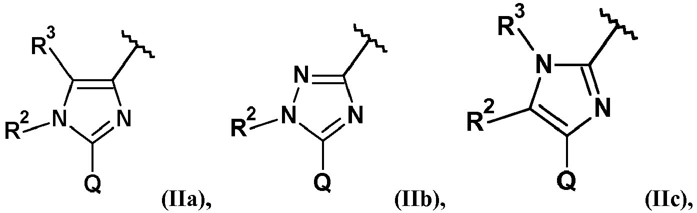 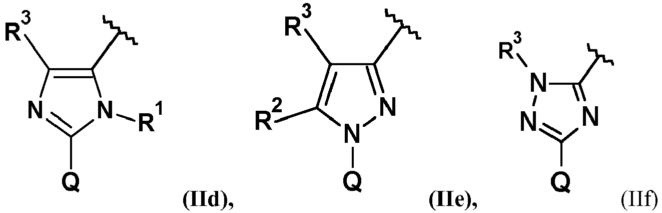 wobei
R¹, R², R³ jeweils unabhängig voneinander H oder ein substituierter oder unsubstituierter Alkyl, Cycloalkyl, Alkenyl, Cycloalkenyl, Cyloheteroalkyl, Aryl oder Heteroaryl Rest sind, wobei R² im Falle (IIc) und (IIe) zusätzlich ein Halogen Rest oder ein Alkoxy Rest sein kann, und R³ zusätzlich im Falle (IIa), (IId) und (IIe) ein Halogen Rest sein kann;
Q ein substituierter oder unsubstituierter Aryl oder Heteroaryl Rest, im Falle (IIe) aber nicht 2-Pyrimidinyl ist;
D ein substituierter oder unsubstituierter Alkyl, Heteroalkyl, gegebenenfalls teilweise ungesättigter Cycloalkyl, Cycloheteroalkyl, Heteroaryl, Aryl oder Phenylalkyl Rest oder ein substituierter oder unsubstituierter Stickstoff Rest ist,
zur Bekämpfung von tierischen Schädlingen, wobei die chirurgische, therapeutische oder diagnostische Behandlung des menschlichen oder tierischen Körpers ausgeschlossen ist.

2. Verwendung nach Anspruch 1, bei der
M für einen Rest ausgewählt aus den Formeln (IIa-IIf) steht, wobei R¹, R², R³ jeweils unabhängig voneinander H oder ein substituierter oder unsubstituierter (C₁-C₄)-Alkyl, (C₃-C₆)-Cycloalkyl, (C₂-C₄)-Alkenyl, Pyridyl oder Phenyl Rest sind, wobei R² im Falle (IIc) und (IIe) zusätzlich ein Halogen Rest oder ein Alkoxy Rest sein kann und wobei R³ zusätzlich im Falle (IIa), (IId) und (IIe) ein Halogen Rest sein kann;
Q ein substituierter oder unsubstituierter Phenyl, Naphthyl oder Heteroaryl Rest ist, der ein bis drei Heteroatome aus der Reihe Sauerstoff, Schwefel, Stickstoff enthalten kann; im Falle (IIe) aber nicht 2-Pyrimidinyl ist;
D ein substituierter oder unsubstituierter Alkyl, Heteroalkyl, Cycloalkyl, Heteroaryl, Aryl oder Phenyl-(C₁-C₈)alkyl Rest oder ein substituierter oder unsubstituierter Stickstoff Rest ist.

3. Verwendung nach Anspruch 1 oder 2, bei der
M für einen Rest ausgewählt aus den Formeln (IIa-IIf) steht, wobei R¹, R², R³ definiert sind wie in Anspruch 1 oder 2 und
Q ein unsubstituierter oder mit einem oder mehreren Resten R⁴ substituierter Phenyl, Naphthyl, Pyridyl, Pyrimidinyl, Thiophen, Benzthiophen, Isochinolin, Benzdioxol oder Pyrazol Rest ist, im Falle (IIe) aber nicht 2-Pyrimidinyl ist,
wobei der oder die Substituenten R⁴ jeweils unabhängig voneinander ausgewählt sind aus:
Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino) und/oder
gegebenenfalls einfach oder mehrfach, gleich oder verschieden substituiertes Aryl, Aryloxy oder Hetaryl, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino und
D ein unsubstituierter oder mit einem oder mehreren Resten R⁵ substituierter (C₁-C₆)-Alkyl, Phenyl, Phenyl-(C₁-C₂)alkyl oder Heteroaryl Rest ist, der ein bis drei Heteroatome aus der Reihe Sauerstoff, Schwefel, Stickstoff enthalten kann oder ein NR⁶R⁷ Rest ist,
wobei der oder die Substituenten R⁵ jeweils unabhängig voneinander ausgewählt sind aus:
Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkylaminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)alkyl
und /oder
einfach oder mehrfach, gleich oder verschieden substituiertes Aryl oder Hetaryl, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino
und wobei R⁶ und R⁷ jeweils unabhängig voneinander H, (C₁-C₆)-Alkyl oder ein substituierter oder unsubstituierter Phenyl Rest sind oder R⁶ und R⁷ gemeinsam einen unsubstituierten oder substituierten 4- bis 8- gliedrigen, gesättigten oder gegebenenfalls ganz oder teilweise ungesättigten Ring bilden können der von 1 bis 3 Heteroatomen aus der Reihe Sauerstoff, Schwefel, Stickstoff unterbrochen sein kann und der einfach oder mehrfach mit einer der Definition von R⁵ entsprechenden Substitution versehen sein kann.

4. Verwendung nach einem der Ansprüche 1 bis 3, bei der
M für einen Rest ausgewählt aus den Formeln (IIa-IIf) steht, wobei R¹, R², R³ definiert sind wie in Anspruch 1 oder 2 und
Q ein unsubstituierter oder mit einem oder mehreren Resten R⁴ substituierter Phenyl, Naphthyl, Pyridyl, Pyrimidinyl, Thiophen, Benzthiophen, Isochinolin, Benzdioxol oder Pyrazol Rest ist, im Falle (IIe) aber nicht 2-Pyrimidinyl ist,
wobei der oder die Substituenten R⁴ jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, Phenyl, Halogenphenyl, Phenoxy oder Halogenphenoxy und
D ein unsubstituierter oder mit einem oder mehreren Resten R⁵ substituierter (C₁-C₆)-Alkyl, Phenyl, Phenyl-(C₁-C₂)alkyl oder Heteroaryl Rest ist, der ein bis drei Heteroatome aus der Reihe Sauerstoff, Schwefel oder Stickstoff enthalten kann oder ein NR⁶R⁷ Rest ist,
wobei der oder die Substituenten R⁵ jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino oder 1-Pyrazolyl-(C₁-C₃)Alkyl und
R⁶ und R⁷ jeweils unabhängig voneinander H, ein (C₁-C₆)-Alkyl, (C₁-C₆)Halogenalkyl, (C₁-C₆)Alkoxy-(C₁-C₆)alkyl Rest oder ein unsubstituierter Phenyl oder mit Halogen, (C₁-C₆)Alkyl, (C₁-C₆)-Alkoxy, (C₁-C₆)Halogenalkyl, (C₁-C₆)Alkoxy-(C₁-C₆)alkyl substituierter Phenyl Rest sind
oder
R⁶ und R⁷ gemeinsam einen unsubstituierten oder substituierten 5- bis 6- gliedrigen , gesättigten oder gegebenenfalls ganz oder teilweise ungesättigten Ring, bilden können der von 1 bis 3 Heteroatomen aus der Reihe Sauerstoff, Schwefel oder Stickstoff unterbrochen sein kann, und der einfach oder mehrfach mit einer der Definition von R⁵ entsprechenden Substitution versehen sein kann.

5. Verwendung nach einem der Ansprüche 1 bis 4, bei der
M für einen Rest ausgewählt aus den Formeln (IIa-IIf) steht, wobei
im Falle (IIa) R², R³ jeweils unabhängig voneinander H oder ein substituierter oder unsubstituierter (C₁-C₄)-Alkyl, (C₃-C₆)-Cycloalkyl oder Phenyl Rest sind, wobei R³ zusätzlich ein Halogen Rest sein kann,
im Falle (IIb) R² H oder ein substituierter oder unsubstituierter (C₁-C₄)-Alkyl oder (C₃-C₆)-Cycloalkyl Rest ist,
im Falle (IIc) R², R³ jeweils unabhängig voneinander H oder ein substituierter oder unsubstituierter (C₁-C₄)-Alkyl, (C₃-C₆)-Cycloalkyl oder Phenyl Rest sind und R² zusätzlich ein Halogen oder (C₁-C₄)-Alkoxy Rest sein kann,
im Falle (IId) R¹, R³ jeweils unabhängig voneinander H oder ein substituierter oder unsubstituierter (C₁-C₄)-Alkyl oder (C₃-C₆)-Cycloalkyl Rest sind, wobei R³ zusätzlich ein Halogen Rest sein kann,
im Falle (IIe) R², R³ jeweils unabhängig voneinander H, Halogen oder ein substituierter oder unsubstituierter (C₁-C₄)-Alkyl, (C₃-C₆)-Cycloalkyl, Pyridyl oder Phenyl Rest sind und R² zusätzlich ein (C₁-C₄)-Alkoxy Rest sein kann und
im Falle (IIf) R³ H oder ein substituierter oder unsubstituierter (C₁-C₄)-Alkyl oder (C₃-C₆)-Cycloalkyl Rest ist
und Q definiert ist wie in einem der Ansprüche 1 bis 4 und
D ein unsubstituierter oder mit einem oder mehreren Resten R⁵ substituierter (C₁-C₆)-Alkyl, Phenyl, Naptht-2-yl, Pyridin, Pyrimidin, Pyrazol, Triazol, Thiazol, Oxazol, Thiadiazol, Oxadiazol, Thiophen, Pyrrol, Furan, Tetrahydrofuran, Dioxan, Isoxazol, Benzyl, 2,3-Dihydro-1,4-benzodioxin-5-yl, 2,3-Dihydro-1-benzofuran-7-yl, Chinoxalin-5-yl oder Indol-7-yl Rest oder ein NR⁶R⁷ Rest ist,
wobei der oder die Substituenten R⁵ jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino oder 1-Pyrazolyl-(C₁-C₃)Alkyl und
R⁶ und R⁷ jeweils unabhängig voneinander H, ein (C₁-C₆)-Alkyl, (C₁-C₆)Halogenalkyl, (C₁-C₆)Alkoxy-(C₁-C₆)alkyl Rest oder ein unsubstituierter Phenyl oder mit Halogen, (C₁-C₆)-Alkyl, (C₁-C₆)-Alkoxy, (C₁-C₆)Halogenalkyl oder (C₁-C₆)Alkoxy-(C₁-C₆)alkyl substituierter Phenyl Rest sind,
oder einen Ring aus der Reihe Pyrrolidin, Morpholin, Piperidin bilden.

6. Verwendung nach einem der Ansprüche 1 bis 5, bei der
M ein Rest ausgewählt aus den Resten der Formeln (IIa) bis (IIf) ist, wobei
im Falle (IIa) R² H, Methyl oder Ethyl oder gegebenenfalls mit Halogen substituiertes Phenyl ist, und R³ H, Methyl, Ethyl, iso-Propyl oder Halogen ist,
im Falle (IIb) R² H, Methyl oder Ethyl ist,
im Falle (IIc) R² H oder Methyl und R³ H, Methyl oder Ethyl ist,
im Falle (IId) R¹ H oder Methyl und R³ H oder Halogen ist,
im Falle (IIe) R² H, Methyl, Methoxy, mit Halogen substituiertes Phenyl oder mit Halogen substituiertes Pyridyl und R³ H ist,
im Falle (IIf) R³ H, Methyl oder Ethyl ist
und Q definiert ist wie in einem der Ansprüche 1 bis 4.

7. Verwendung nach einem der Ansprüche 1 bis 6, bei der
M ausgewählt ist aus einer der Formeln (IIa) bis (IIf) oder (IVa) bis (IVf), wobei R¹ bis R³ definiert sind wie in einem der Ansprüche 1, 2, 5 oder 6 und
Q ein unsubstituierter oder mit einem oder mehreren Resten R⁴ substituierter Phenyl, Napht-1-yl, Pyridyl, Pyrimidinyl, Thiophen-2-yl, Benzthiophen-2-yl, Benzthiophen-3-yl, Isochinolin-1-yl, Benzdioxol-4-yl oder Pyrazol-5-yl Rest ist, im Falle (IVe) aber nicht 2-Pyrimidinyl ist,
wobei der oder die Substituenten R⁴ unabhängig voneinander sind:
Cyano, Halogen, Nitro, Acetyl, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino, Phenyl, Halogenphenyl, Phenoxy oder Halogenphenoxy und
D ein unsubstituierter oder mit einem oder mehreren Resten R⁵ substituierter (C₁-C₆)-Alkyl, Phenyl, Naptht-2-yl, Pyridin, Pyrimidin, Pyrazol, Triazol, Thiazol, Oxazol, Thiadiazol, Oxadiazol, Thiophen, Pyrrol, Furan, Tetrahydrofuran, Dioxan, Isoxazol, Benzyl, 2,3-Dihydro-1,4-benzodioxin-5-yl, 2,3-Dihydro-1-benzofuran-7-yl, Chinoxalin-5-yl oder Indol-7-yl Rest oder ein NR⁶R⁷ Rest ist,
wobei der oder die Substituenten R⁵ unabhängig voneinander sind:
Cyano, Halogen, Nitro, Acetyl, Hydroxy, Carboxy, Amino, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino oder 1-Pyrazolyl-(C₁-C₃)alkyl und
R⁶ und R⁷ jeweils unabhängig voneinander H, ein (C₁-C₆)-Alkyl, Phenyl,Alkoxyphenyl oder Halogenphenyl sind oder einen Ring aus der Reihe Pyrrolidin, Morpholin, Piperidin bilden.

8. Verwendung nach einem der Ansprüche 1 bis 7, bei der
M ein Rest ausgewählt aus den Resten der Formeln (Va-Vz) ist: 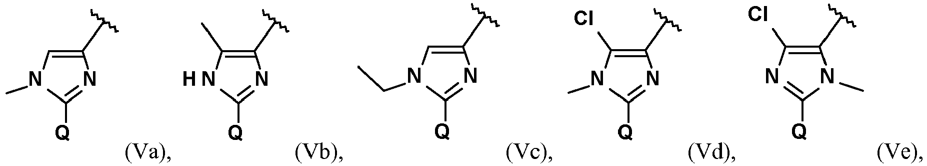 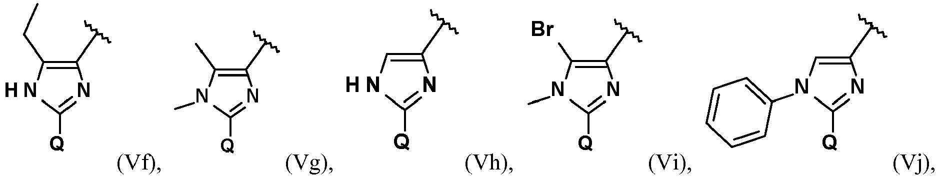 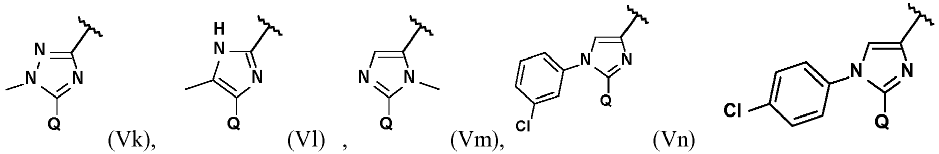 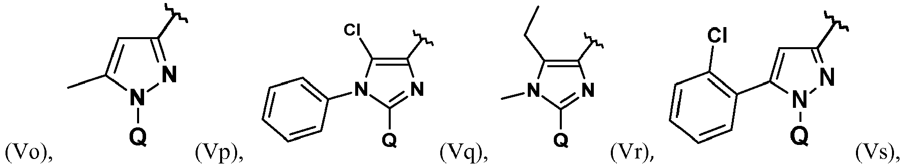 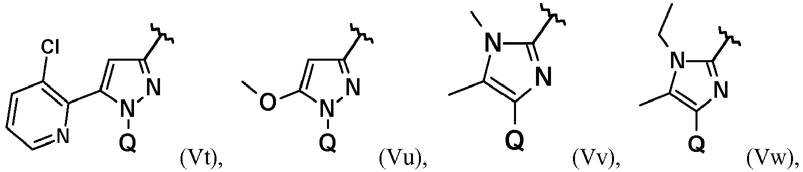 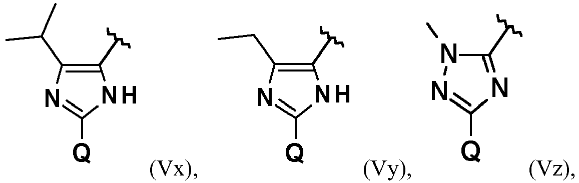 Q ein Rest ausgewählt aus den Resten der Formeln (VIa-VIz und VIa1 - VIa30) ist: 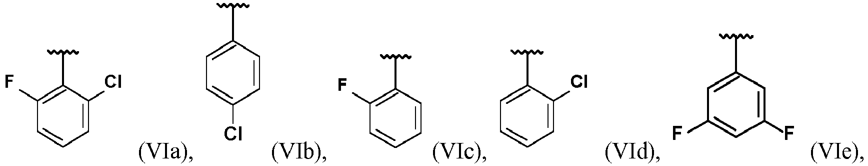 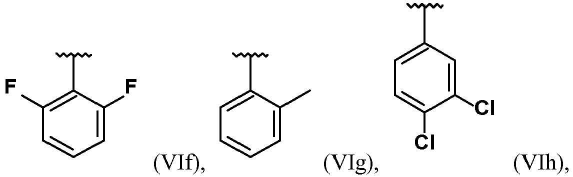 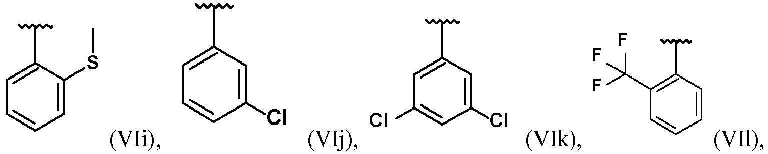 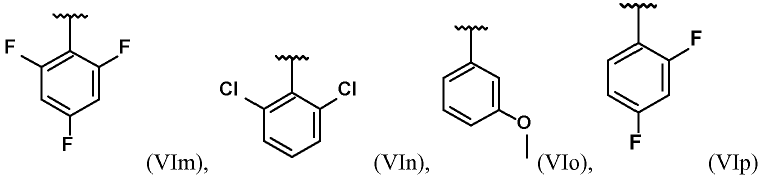 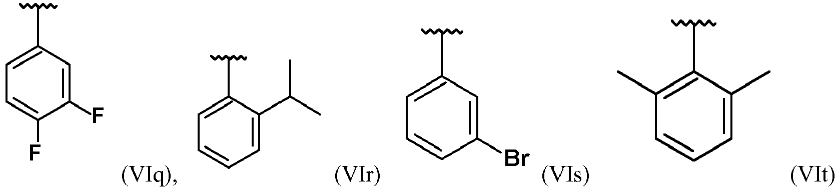 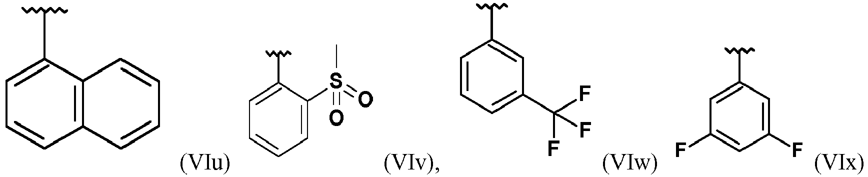 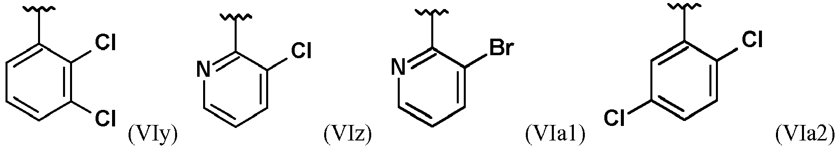 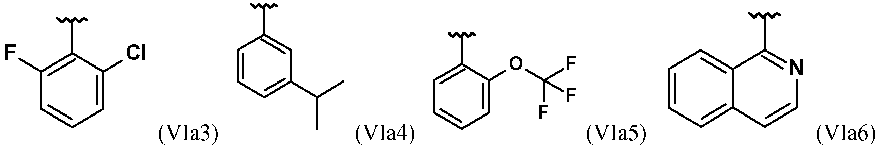 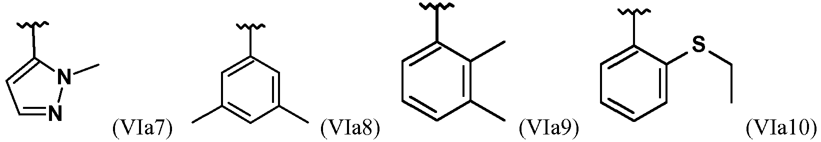 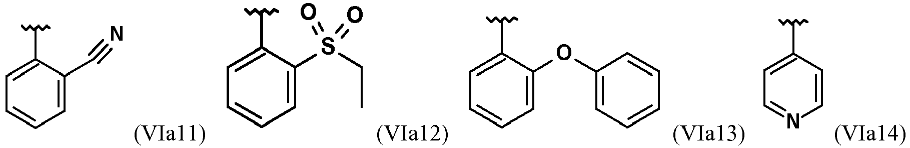 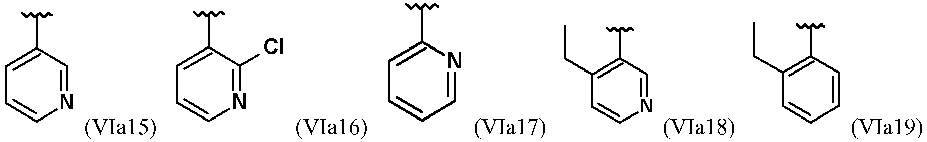 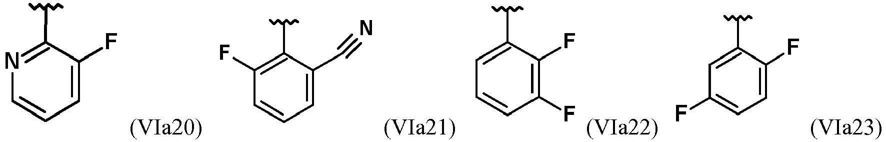 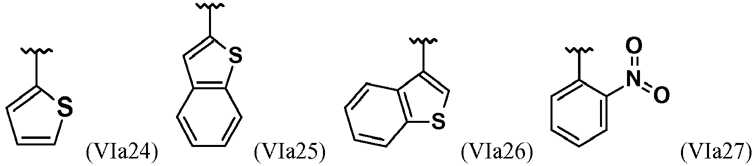 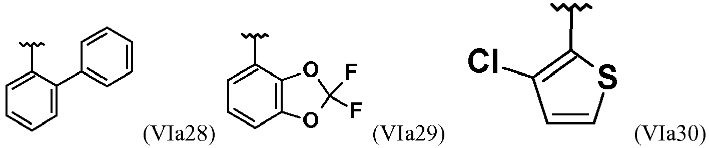 und
D ein Rest ausgewählt aus den Resten der Formeln (VII1-VII192) ist

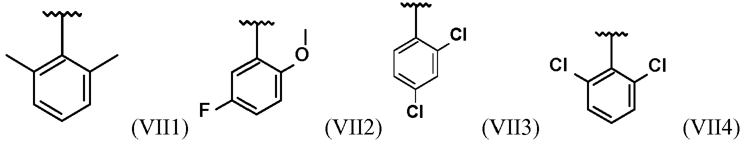

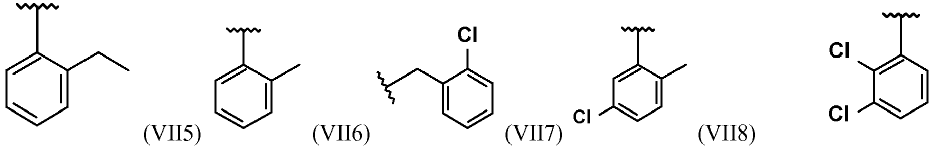

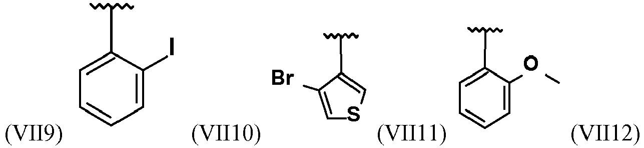

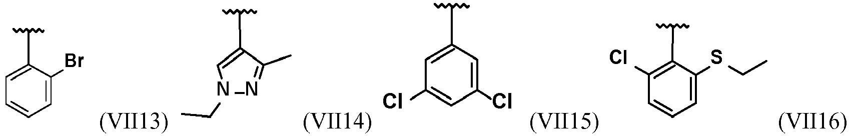

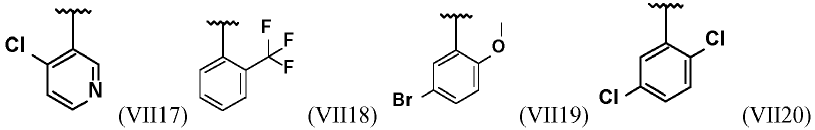

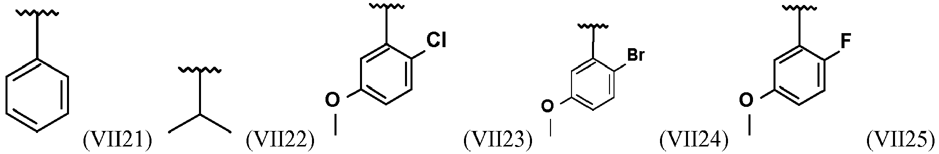

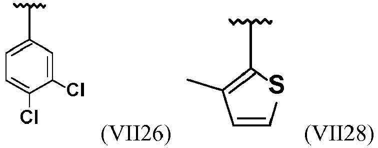

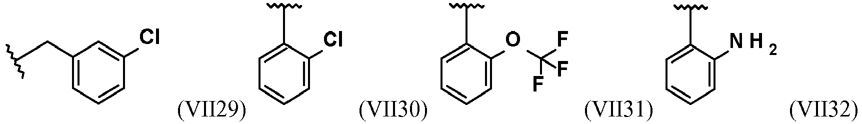

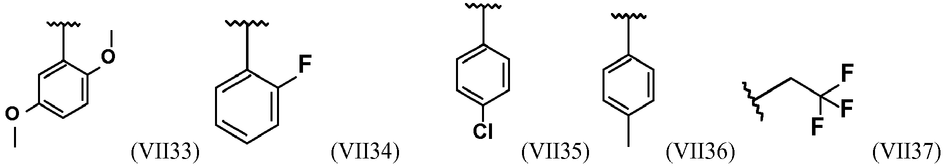

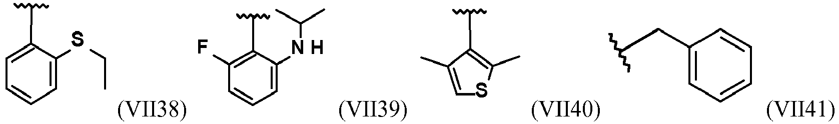

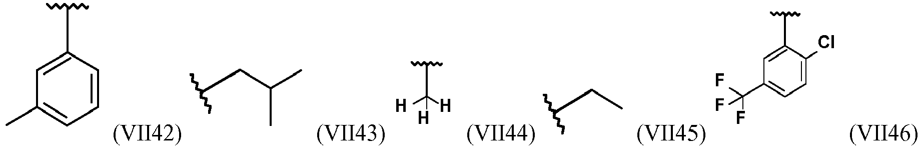

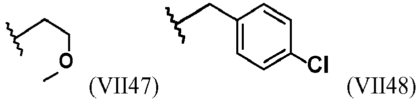

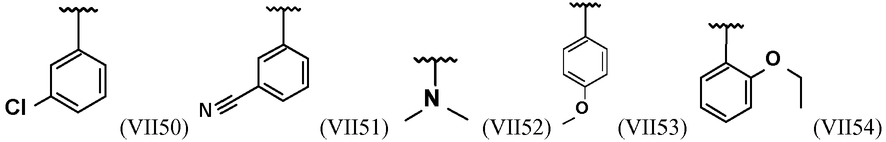

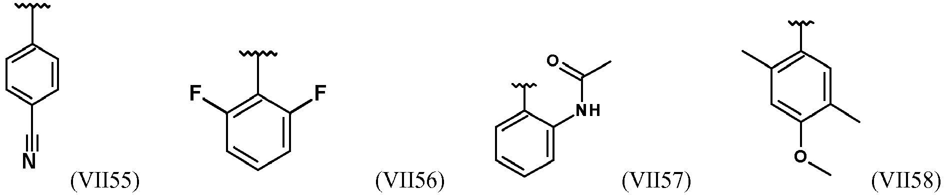

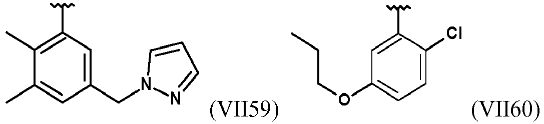

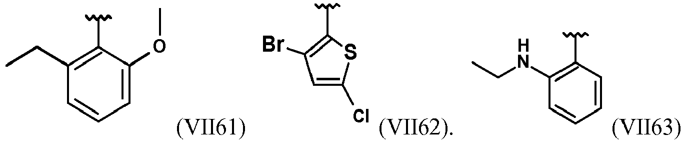

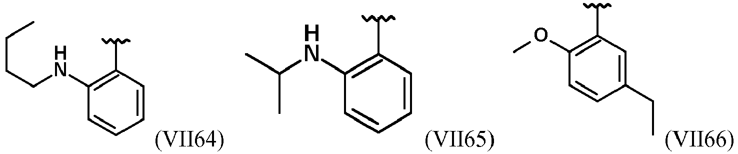

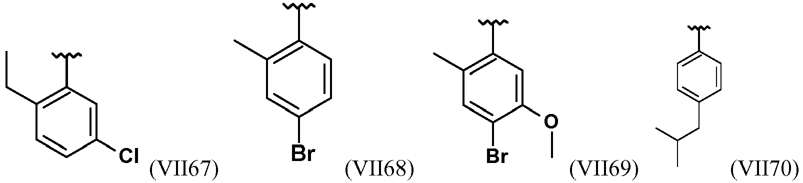

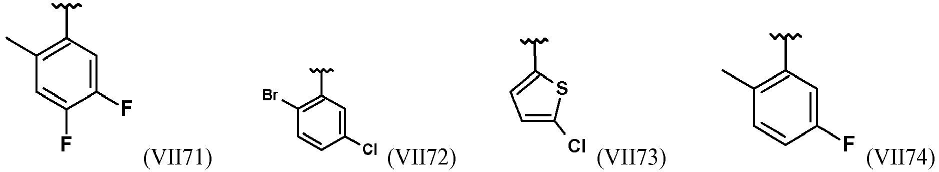

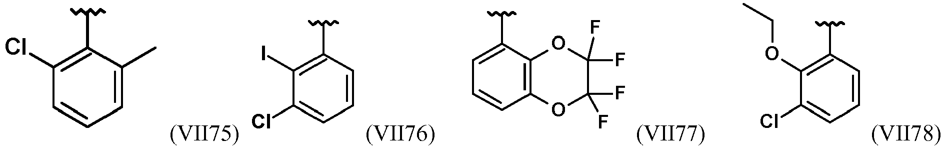

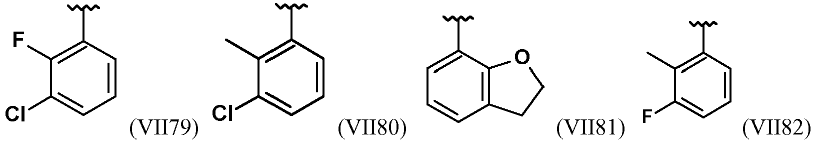

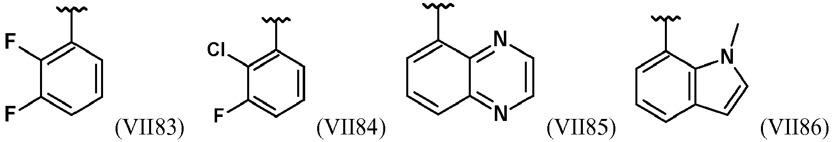

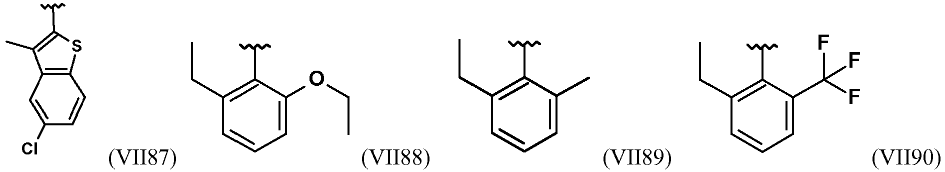

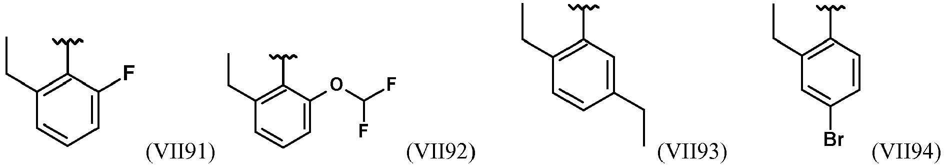

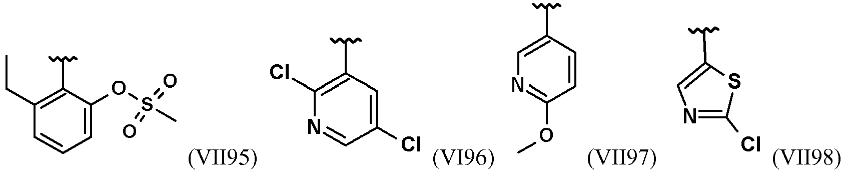

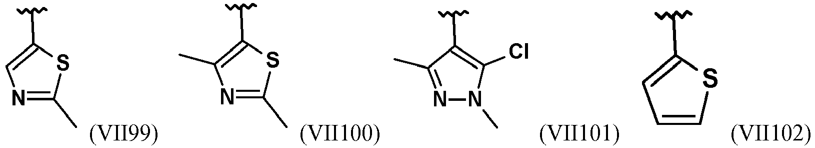

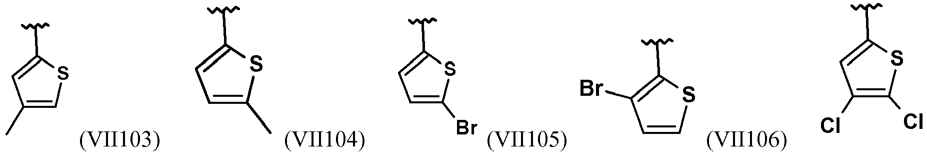

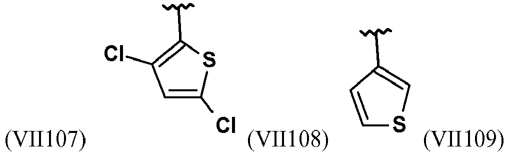

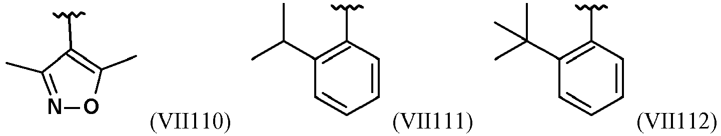

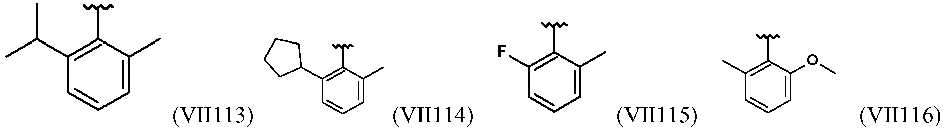

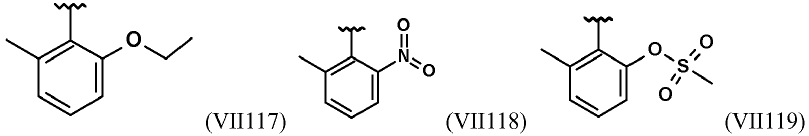

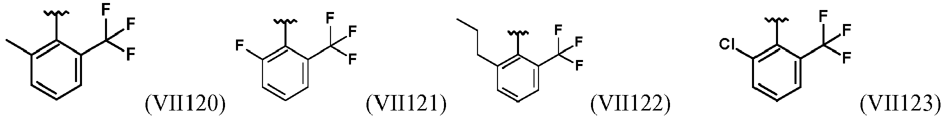

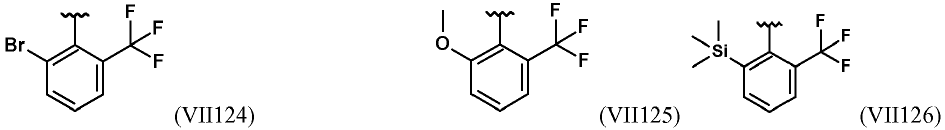

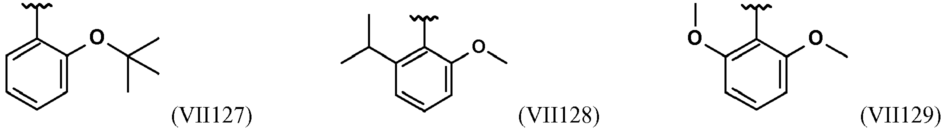

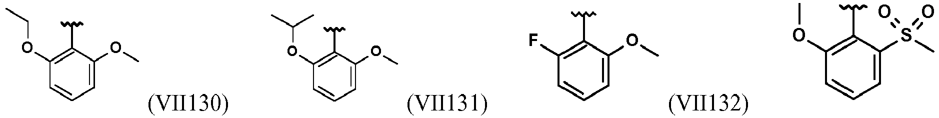

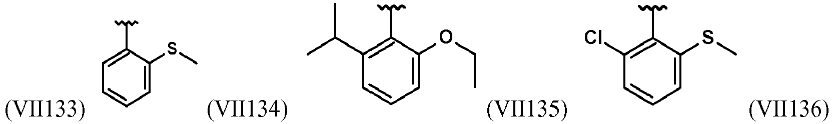

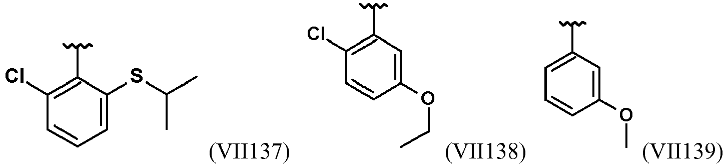

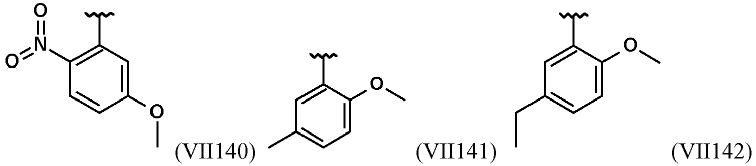

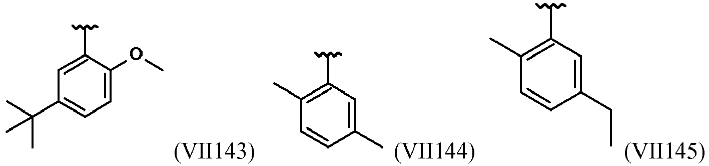

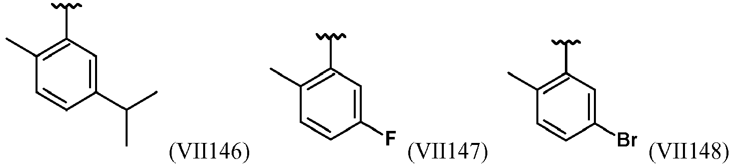

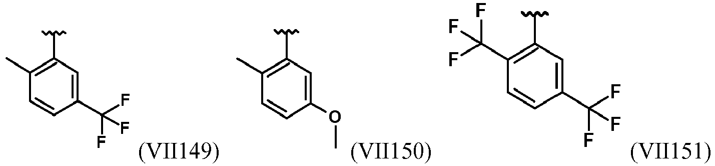

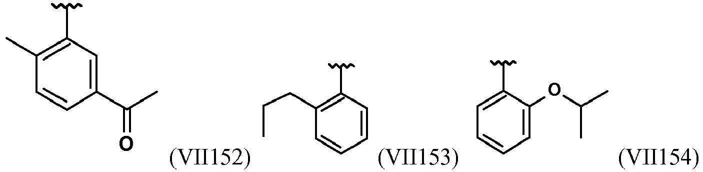

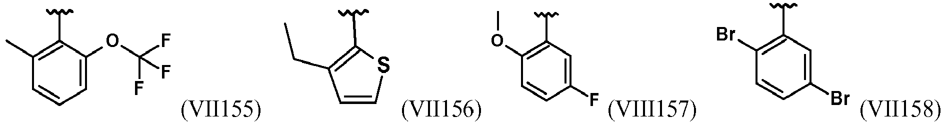

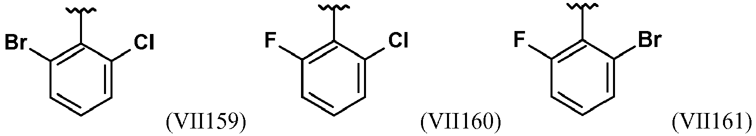

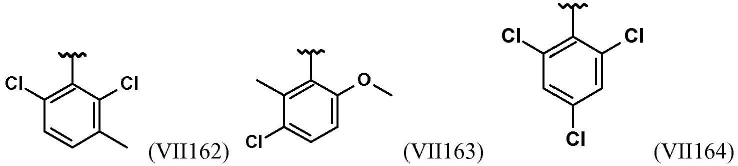

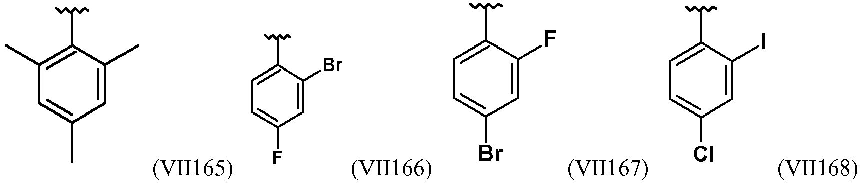

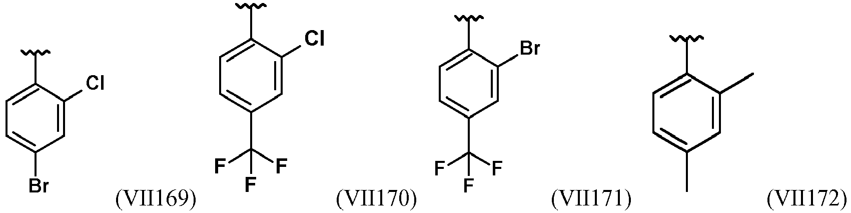

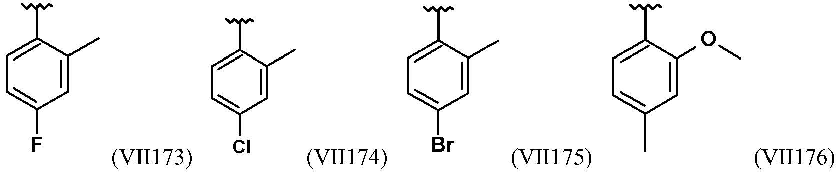

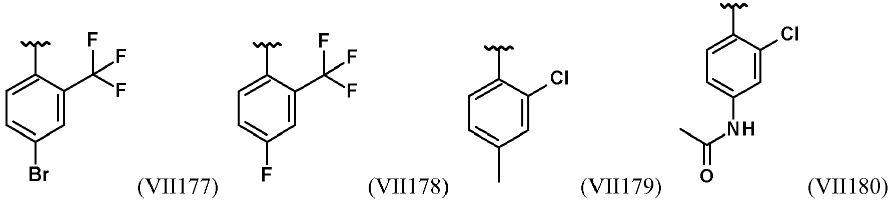

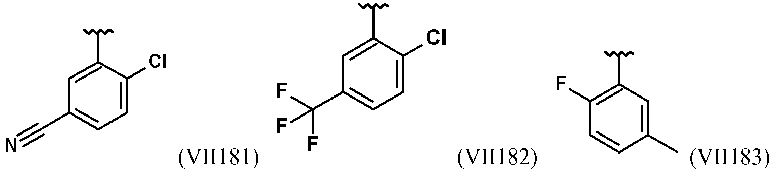

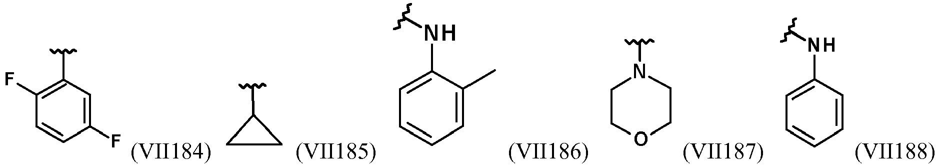

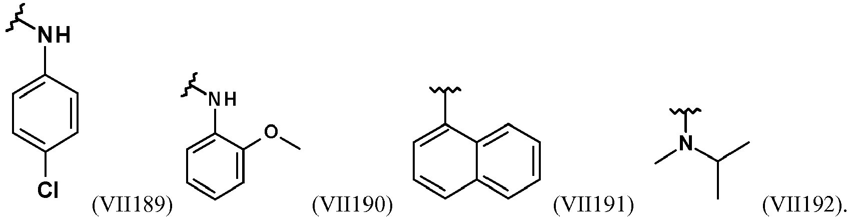

9. Verwendung nach einem der Ansprüche 1 bis 8 zum Schutz des Vermehrungsmaterials von Pflanzen.

10. Mittel, mit einem Gehalt von mindestens einer Verbindung gemäß Formel (I) nach einem der Ansprüche 1 bis 8 und üblichen Streckmitteln und/oder oberflächenaktiven Substanzen insbesondere zur Bekämpfung von tierischen Schädlingen.

11. Verfahren zur Bekämpfung von tierischen Schädlingen, bei dem man wenigstens eine Verbindung gemäß Formel (I) nach einem der Ansprüche 1 bis 8 oder ein Mittel gemäß Anspruch 10 auf die tierischen Schädlinge und/oder ihren Lebensraum einwirken lässt, bei dem die chirurgische, therapeutische und diagnostische Behandlung des menschlichen oder tierischen Körpers ausgeschlossen ist.

12. Agrochemische Formulierung enthaltend wenigstens eine Verbindung gemäß Formel (I) nach einem der Ansprüche 1 bis 8 in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

13. Agrochemische Formulierung gemäß Anspruch 12 zusätzlich enthaltend wenigstens einen weiteren agrochemischen Wirkstoff.

14. Verbindungen der Formel (VIII) 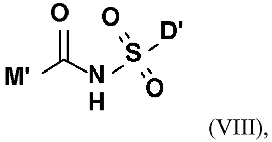 in der
M' für einen Rest der Formel (II) steht, ausgewählt aus: 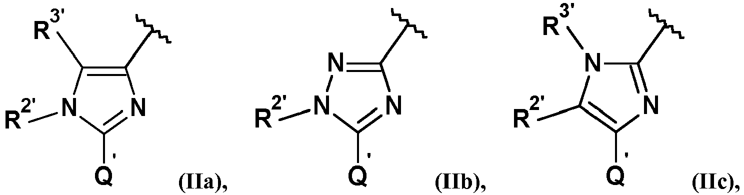 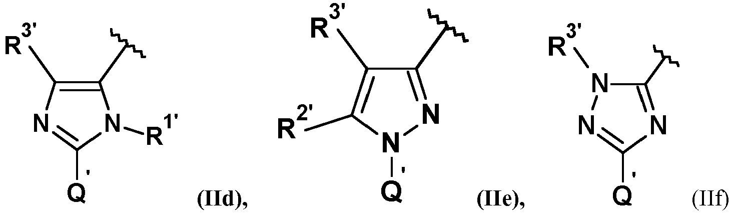 in der
R^{1'}, R^{2'}, R^{3'} jeweils unabhängig voneinander H oder ein substituierter oder unsubstituierter Alkyl, Cycloalkyl, Alkenyl, Cycloalkenyl, Cyloheteroalkyl, Aryl oder Heteroaryl Rest sind, wobei R^{2'} im Falle (IIc) und (IIe) zusätzlich ein Halogen Rest oder ein Alkoxy Rest sein kann und R^{2'} im Falle (IIa) und (IIb) nur H oder ein substituierter oder unsubstituierter Alkyl oder Cycloalkyl Rest sein kann, R^{3'} zusätzlich im Falle (IIa), (IId) und (IIe) ein Halogen Rest sein kann,
Q' ein substituierter oder unsubstituierter Aryl oder Heteroaryl Rest, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl, nicht 3,4-Dichlorphenyl und nicht 3,5-bis-tert-Butyl ist;
D' ein substituierter oder unsubstituierter Alkyl, Heteroalkyl, gegebenenfalls teilweise ungesättigter Cycloalkyl, Cycloheteroalkyl, Heteroaryl, Aryl oder Phenylalkyl Rest oder im Falle, daß Q' mindestens einen Substituenten in 2-Stellung trägt, ein substituierter oder unsubstituierter Stickstoff Rest ist.

15. Verbindungen nach Anspruch 14, bei der
M' für einen Rest gemäß einer der Formeln (IIa) bis (IIf) steht und
im Falle (IIa) R^{2'}, R^{3'} jeweils unabhängig voneinander H oder ein substituierter oder unsubstituierter (C₁-C₄)-Alkyl, (C₃-C₆)-Cycloalkyl oder Phenyl Rest sind, wobei R^{3'} zusätzlich ein Halogen Rest sein kann;
im Falle (IIb) R^{2'} H oder ein substituierter oder unsubstituierter (C₁-C₄)-Alkyl oder (C₃-C₆)-Cycloalkyl Rest ist,
im Falle (IIc) R^{2'}, R^{3'} jeweils unabhängig voneinander H oder ein substituierter oder unsubstituierter (C₁-C₄)-Alkyl, (C₃-C₆)-Cycloalkyl oder Phenyl Rest sind, wobei R^{2'} zusätzlich ein Halogen Rest oder ein (C₁-C₄)-Alkoxy Rest sein kann,
im Falle (IId) R^{1'}, R^{3'} jeweils unabhängig voneinander H oder ein substituierter oder unsubstituierter (C₁-C₄)-Alkyl oder (C₃-C₆)-Cycloalkyl Rest sind und R^{3'} zusätzlich ein Halogen Rest sein kann;
im Falle (IIe) R^{2'}, R^{3'} jeweils unabhängig H, Halogen oder ein substituierter oder unsubstituierter (C₁-C₄)-Alkyl, (C₃-C₆)-Cycloalkyl, Pyridyl oder Phenyl Rest sind und R² zusätzlich ein (C₁-C₄)-Alkoxy Rest sein kann und
im Falle (IIf) R³ H oder ein substituierter oder unsubstituierter (C₁-C₄)-Alkyl oder (C₃-C₆)-Cycloalkyl Rest ist,
Q' ein substituierter oder unsubstituierter Phenyl, Naphthyl oder Heteroaryl Rest ist, der ein bis drei Heteroatome aus der Reihe Sauerstoff, Schwefel oder Stickstoff enthalten kann, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist,
D' ein substituierter oder unsubstituierter Alkyl, Heteroalkyl, Cycloalkyl, Heteroaryl, Aryl oder Phenyl-(C₁-C₈)alkyl Rest oder im Falle, daß Q' mindestens einen Substituenten in 2-Stellung trägt, ein substituierter oder unsubstituierter Stickstoff Rest ist.

16. Verbindungen nach Anspruch 14 oder 15, bei der
M' für einen Rest ausgewählt aus den Formeln (IIa-IIf) steht, wobei R^{1'}, R^{2'}, R^{3'} definiert sind wie in Anspruch 14 oder 15 und
Q' ein unsubstituierter oder mit einem oder mehreren Resten R^{4'} substituierter Phenyl, Naphthyl, Pyridyl, Pyrimidinyl, Thiophen, Benzthiophen, Isochinolin, Benzdioxol oder Pyrazol-Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl und
wobei der oder die Substituenten R^{4'} jeweils unabhängig voneinander ausgewählt sind aus:
Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino; und/oder
gegebenenfalls einfach oder mehrfach, gleich oder verschieden substituiertes Aryl, Aryloxy oder Hetaryl, wobei (im Fall von Hetaryl) gegebenenfalls mindestens eine Carbonylgruppe ent-halten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino und
D' ein unsubstituierter oder mit einem oder mehreren Resten R^{5'} substituierter (C₁-C₆)-Alkyl, Phenyl oder Heteroaryl Rest ist, der ein bis drei Heteroatome aus der Reihe Sauerstoff, Schwefel oder Stickstoff enthalten kann oder im Falle, daß Q' mindestens einen Substituenten in 2-Stellung trägt, ein NR^{6'}R^{7'} Rest ist,
wobei der oder die Substituenten R^{5'} jeweils unabhängig voneinander ausgewählt sind aus:
Cyano, Halogen, Nitro, Acetyl, Hydroxy, Carboxy, Amino, SCN, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)alkyl,
und /oder
einfach oder mehrfach, gleich oder verschieden substituiertes Aryl oder Hetaryl, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und (C₁-C₆)Alkylcarbonylamino,
und wobei R^{6'} und R^{7'} jeweils unabhängig voneinander H, ein (C₁-C₆)-Alkyl, (C₁-C₆)Halogenalkyl, (C₁-C₆)Alkoxy-(C₁-C₆)alkyl Rest oder ein unsubstituierter Phenyl oder mit Halogen, (C₁-C₆)-Alkyl, (C₁-C₆)-Alkoxy, (C₁-C₆)Halogenalkyl, (C₁-C₆)Alkoxy-(C₁-C₆)alkyl substituierter Phenyl Rest sind oder
R^{6'} und R^{7'} gemeinsam einen unsubstituierten oder substituierten 5- bis 6- gliedrigen, gesättigten oder gegebenenfalls ganz oder teilweise ungesättigten Ring bilden können der von 1 bis 3 Heteroatomen aus der Reihe Sauerstoff, Schwefel, Stickstoff unterbrochen sein kann, und der einfach oder mehrfach mit einer der Definition von R^{5'} entsprechenden Substitution versehen sein kann.

17. Verbindungen nach einem der Ansprüche 14 bis 16, bei der
M' für einen Rest ausgewählt aus den Formeln (IIa-IIf) steht, wobei R^{1'}, R^{2'}, R^{3'} definiert sind wie in Anspruch 14 oder 15 und
Q' ein unsubstituierter oder mit einem oder mehreren Resten R^{4'} substituierter Phenyl, Naphthyl, Pyridyl, Pyrimidinyl, Thiophen, Benzthiophen, Isochinolin, Benzdioxol oder Pyrazol-Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist
und der oder die Substituenten R^{4'} jeweils unabhängig voneinander ausgewählt sind aus:Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, Phenyl, Halogenphenyl, Phenoxy oder Halogenphenoxy und
D' ein unsubstituierter oder mit einem oder mehreren Resten R^{5'} substituierter Phenyl, Pyridin, Pyrimidin, Pyrazol, Triazol, Thiazol, Oxazol, Thiadiazol, Oxadiazol, Thiophen, Pyrrol, Furan, Tetrahydrofuran, Dioxan oder (C₁-C₆)Alkyl Rest oder ein NR^{6'}R^{7'} Rest ist,
wobei der oder die Substituenten R^{5'} jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)Alkyl,
und wobei R^{6'} und R^{7'} jeweils unabhängig voneinander H, ein (C₁-C₄)-Alkyl, (C₁-C₄)Halogenalkyl, (C₁-C₄)Alkoxy-(C₁-C₄)alkyl Rest oder ein unsubstituierter Phenyl oder mit Halogen, (C₁-C₄)-Alkyl, (C₁-C₄)-Alkoxy, (C₁-C₄)Halogenalkyl, (C₁-C₄)Alkoxy-(C₁-C₄)alkyl substituierter Phenyl Rest sind oder
R^{6'} und R^{7'} gemeinsam einen unsubstituierten oder substituierten 5- bis 6- gliedrigen, gesättigten oder gegebenenfalls ganz oder teilweise ungesättigten Ring bilden können der von 1 bis 3 Heteroatomen aus der Reihe Sauerstoff, Schwefel, Stickstoff unterbrochen sein kann, und der einfach oder mehrfach mit einer der Definition von R^{5'} entsprechenden Substitution versehen sein kann.

18. Verbindungen nach einem der Ansprüche 14 bis 17, bei der
D' ein unsubstituierter oder mit einem oder mehreren Resten R^{5'} substituierter (C₁-C₆)-Alkyl Rest, Phenyl Rest, Pyridin, Pyrimidin, Pyrazol, Triazol, Thiazol, Oxazol, Thiadiazol, Oxadiazol, Thiophen, Pyrrol, Furan, Tetrahydrofuran, Dioxan, Isoxazol, Benzyl, 2,3-Dihydro-1,4-benzodioxin-5-yl, 2,3-Dihydro-1-benzofuran-7-yl, Chinoxalin-5-yl oder Indol-7-yl Rest oder ein NR^{6'}R^{7'} Rest ist,
wobei der oder die Substituenten R^{5'} jeweils unabhängig voneinander ausgewählt sind aus:
Cyano, Halogen, Nitro, Acetyl, Hydroxy, Carboxy, Amino, Tri-(C₁-C₆)alkylsilyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino und 1-Pyrazolyl-(C₁-C₃)Alkyl und
R^{6'} und R^{7'} jeweils unabhängig voneinander H, ein (C₁-C₆)-Alkyl, (C₁-C₆)Halogenalkyl, (C₁-C₆)Alkoxy-(C₁-C₆)alkyl Rest oder unsubstituierter Phenyl oder mit Halogen, (C₁-C₆)-Alkyl, (C₁-C₆)Halogenalkyl oder (C₁-C₆)Alkoxy-(C₁-C₆)alkyl substituierter Phenyl Rest sind
oder einen Ring aus der Reihe Pyrrolidin, Morpholin, Piperidin bilden.

19. Verbindungen nach einem der Ansprüche 14 bis 18, bei der
M' ein Rest ausgewählt aus den Resten der Formeln (IIa) bis (IIf) ist, wobei
im Falle (IIa) R^{2'} H, Methyl oder Ethyl oder gegebenenfalls mit Halogen substituiertes Phenyl ist und R^{3'} H, Methyl, Ethyl, iso-Propyl oder Halogen ist,
im Falle (IIb) R^{2'} H, Methyl oder Ethyl ist,
im Falle (IIc) R^{2'} H oder Methyl und R^{3'} H, Methyl oder Ethyl ist,
im Falle (IId) R^{1'} H oder Methyl und R^{3'} H oder Halogen ist,
im Falle (IIe) R^{2'} H, Methyl, Methoxy, mit Halogen substituiertes Phenyl oder Pyridyl und R^{3'} H ist,
im Falle (IIf) R^{3'} H, Methyl oder Ethyl ist und
Q' ein unsubstituierter oder mit einem oder mehreren Resten R^{4'} substituierter Phenyl, Napht-1-yl, Pyridyl, Pyrimidinyl, Thiophen-2-yl, Benzthiophen-2-yl, Benzthiophen-3-yl, Isochinolin-1-yl, Benzdioxol-4-yl oder Pyrazol-5-yl Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist und
wobei die Substituenten R^{4'} definiert sind wie in Anspruch 17 oder 18.

20. Verbindungen nach einem der Ansprüche 14 bis 19, bei der
Q' ein unsubstituierter oder mit einem oder mehreren Resten R^{4'} substituierter Phenyl, Napht-1-yl, Pyridyl, Pyrimidinyl, Thiophen-2-yl, Benzthiophen-2-yl, Benzthiophen-3-yl, Isochinolin-1-yl, Benzdioxol-4-yl oder Pyrazol-5-yl Rest ist, aber im Falle (IIa), (IId) im Falle R^{1'}=H und R^{3'}=Methyl nicht 3-Methoxyphenyl ist, im Falle (IId) nicht 3-Pyridyl ist, im Falle (IIe) nicht 2-Pyrimidinyl, nicht unsubstituiertes Phenyl und nicht 3,4-Dichlorphenyl ist und
wobei der oder die Substituenten R^{4'} unabhängig voneinander ausgewählt sind aus:
Cyano, Halogen, Nitro, Acetyl, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino, Phenyl, Halogenphenyl, Phenoxy oder Halogenphenoxy;
D' ein unsubstituierter oder mit einem oder mehreren Resten R^{5'} substituierter (C₁-C₆)-Alkyl, Phenyl, Pyridin, Pyrimidin, Pyrazol, Triazol, Thiazol, Oxazol, Thiadiazol, Oxadiazol, Thiophen, Pyrrol, Furan, Tetrahydrofuran oder Dioxan Rest ist,
wobei der oder die Substituenten R^{5'} unabhängig voneinander ausgewählt sind aus:
Cyano, Halogen, Nitro, Acetyl, Hydroxy, Carboxy, Amino, (C₃-C₆)Cycloalkyl, (C₃-C₆)Cycloalkyloxy, (C₃-C₆)Cycloalkyl-(C₃-C₆)Cycloalkyl, (C₁-C₆)Alkyl-(C₃-C₈)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Halogenalkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkylsulfonyl, (C₁-C₆)Halogenalkylsulfonyl, (C₁-C₆)Alkylsulfonyloxy, (C₁-C₆)Alkylcarbonyl, (C₁-C₆)Alkylamino, Di-(C₁-C₆)Alkylamino, (C₁-C₆)Alkylcarbonylamino oder 1-Pyrazolyl-(C₁-C₃)alkyl und
R^{6'} und R^{7'} jeweils unabhängig voneinander H, ein (C₁-C₆)-Alkyl, Phenyl, Alkoxyphenyl oder Halogenphenyl sind oder einen Ring aus der Reihe Pyrrolidin, Morpholin, Piperidin bilden.

21. Verbindungen nach einem der Ansprüche 14 bis 20, bei der
M' ein Rest ausgewählt aus den Resten der Formeln (Va-Vz) ist: 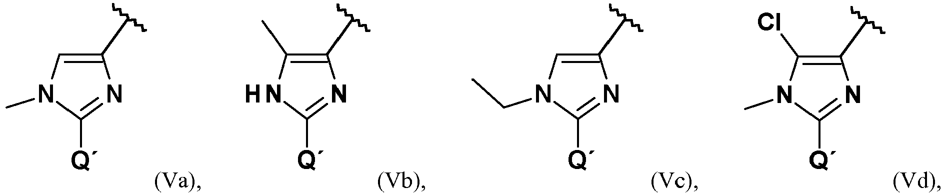 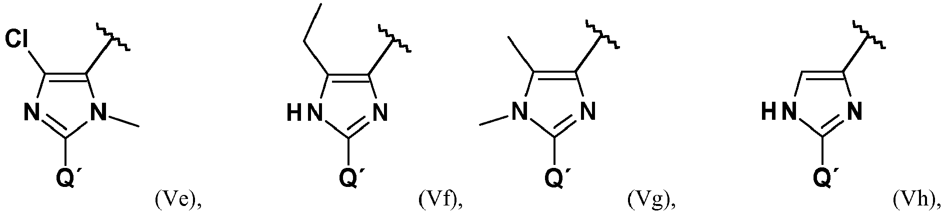 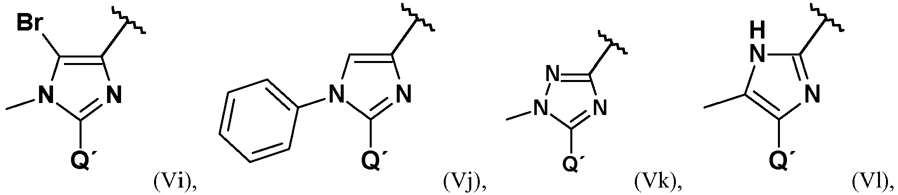 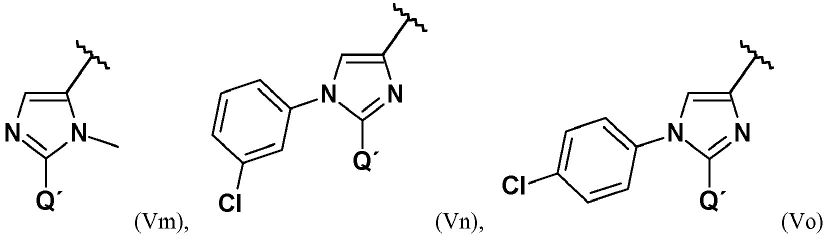 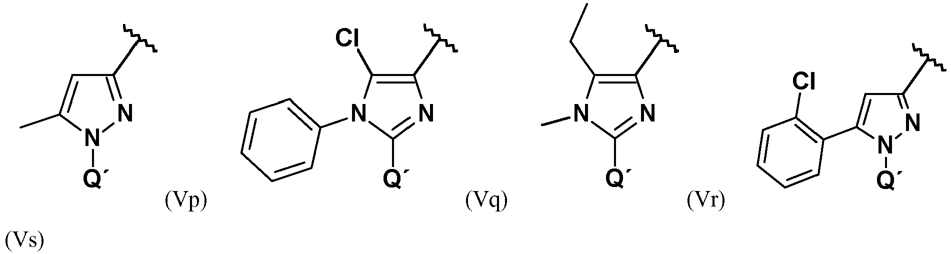 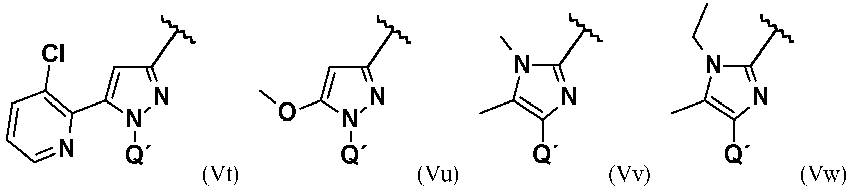 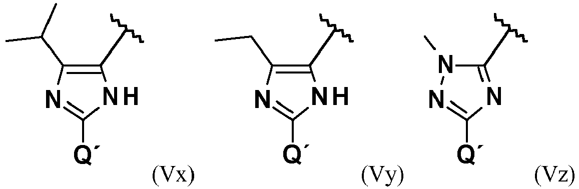 Q' ein Rest ausgewählt aus den Resten der Formeln (VIa-VIz und VIa1-VIa30) ist: 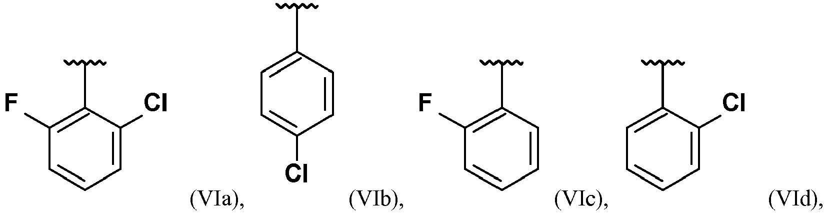 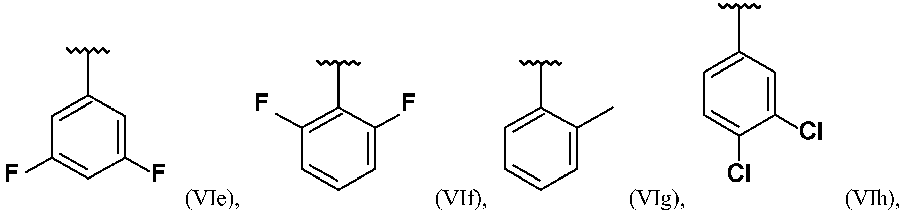 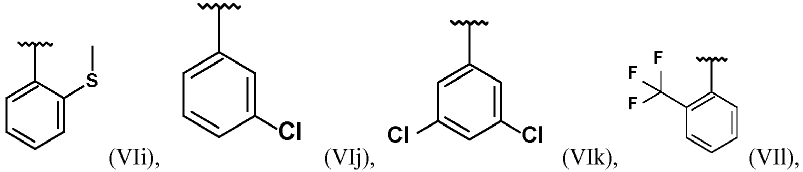 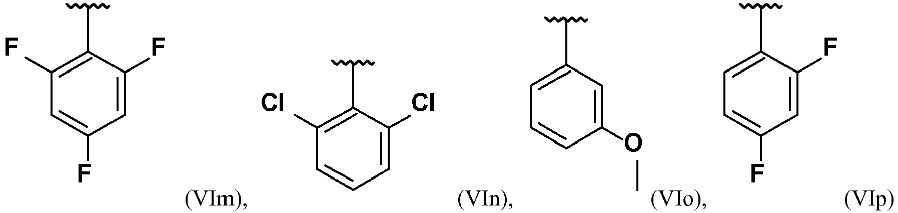 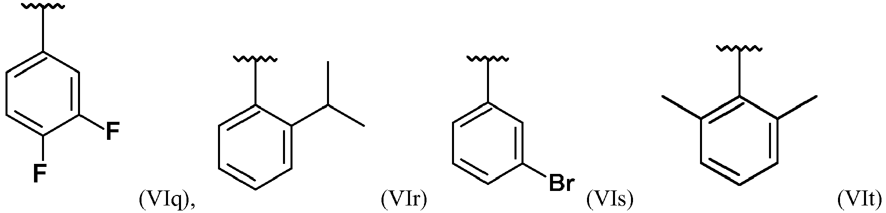 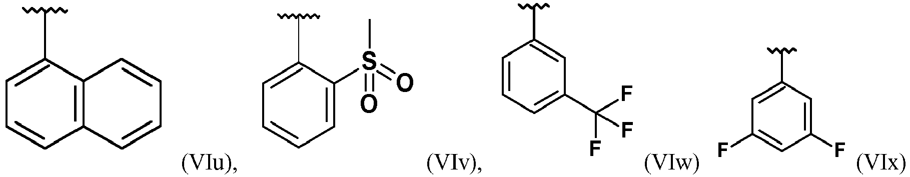 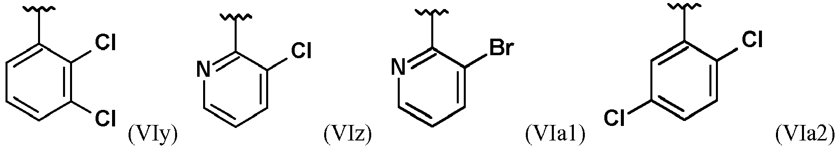 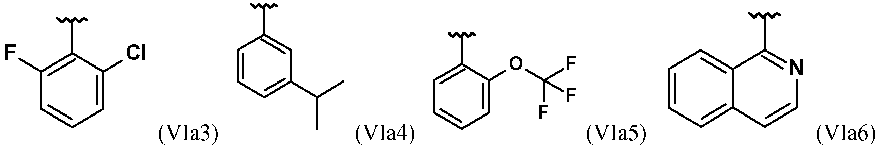 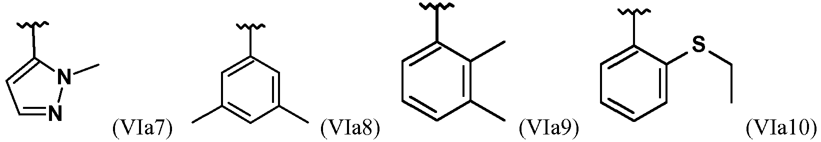 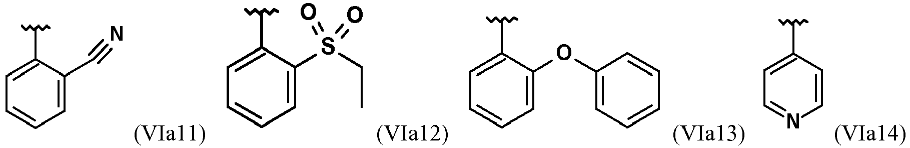 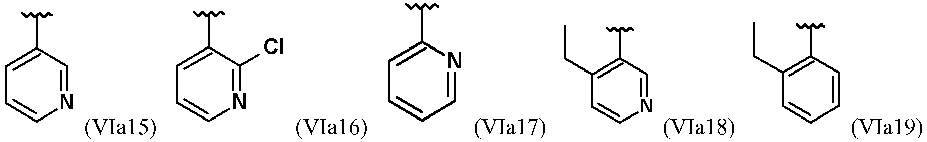 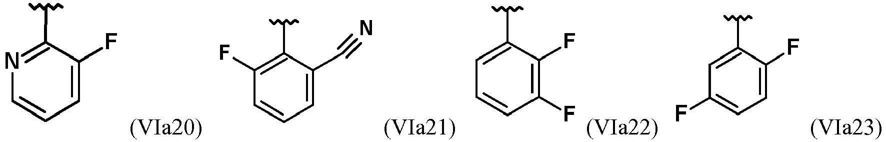 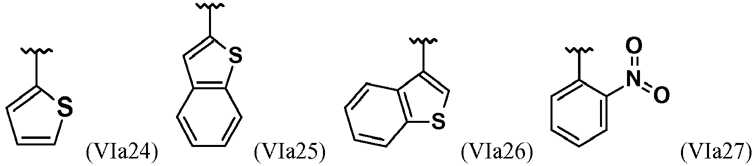 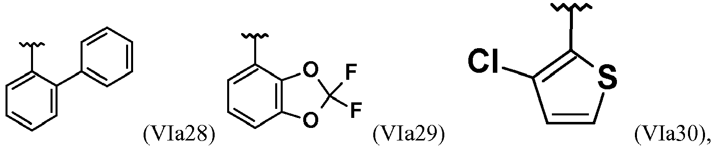 D' ein Rest ausgewählt aus den Resten der Formeln (VII1-VII192) ist

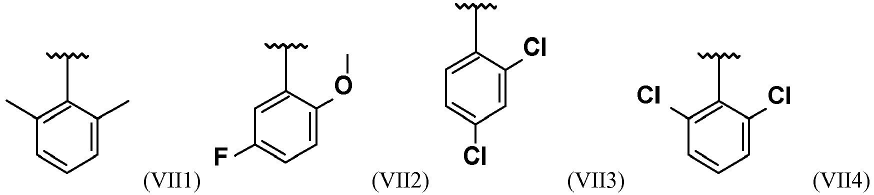

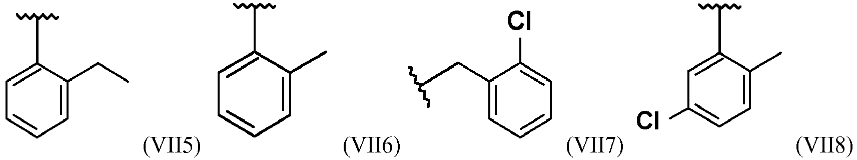

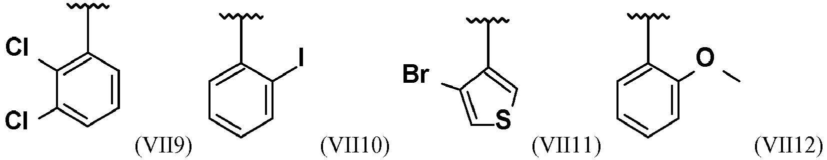

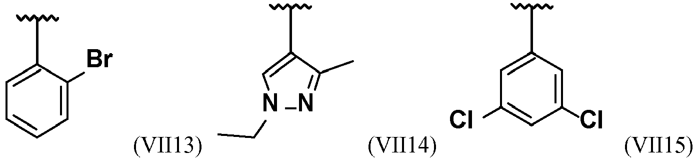

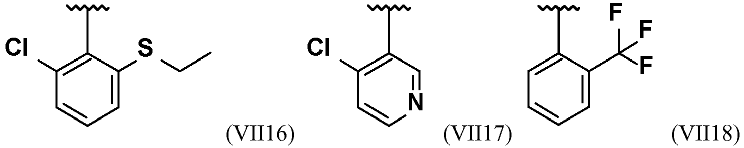

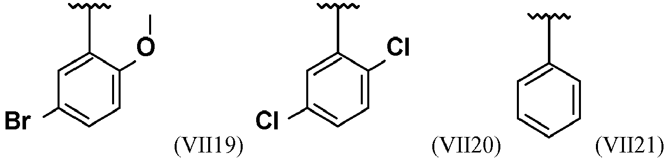

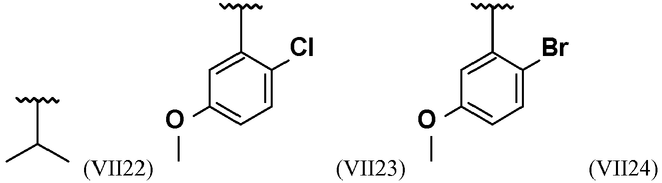

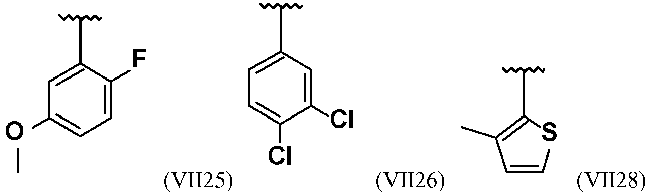

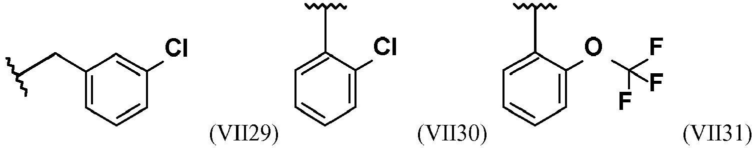

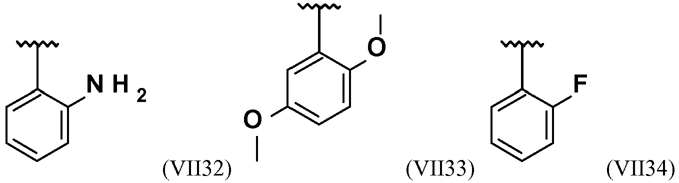

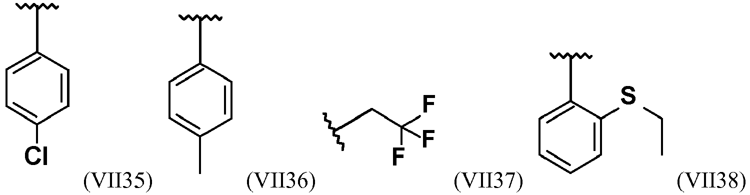

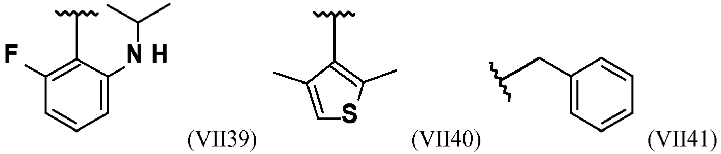

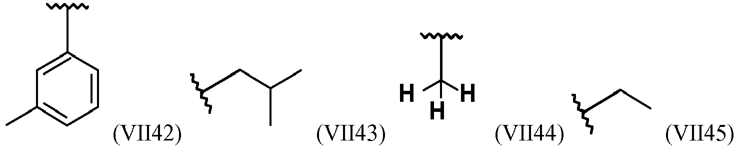

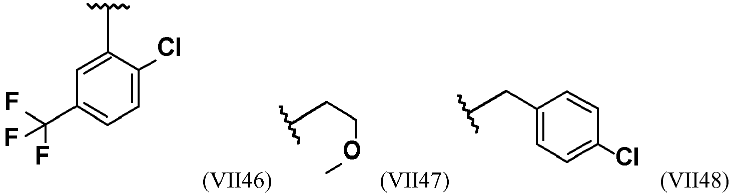

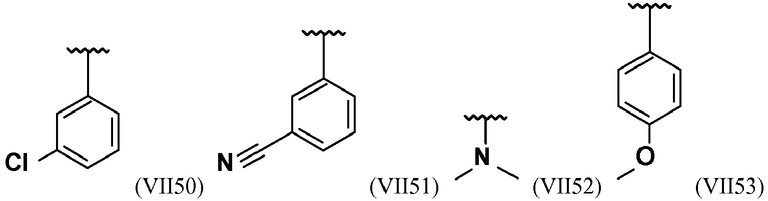

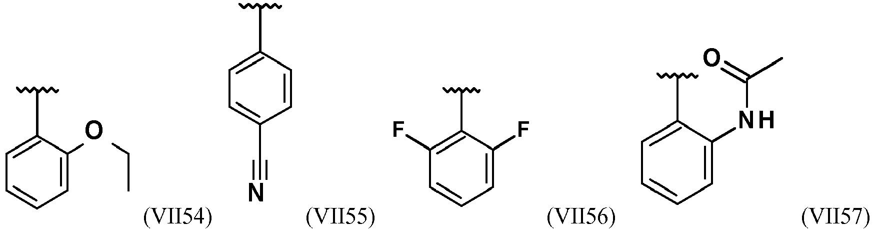

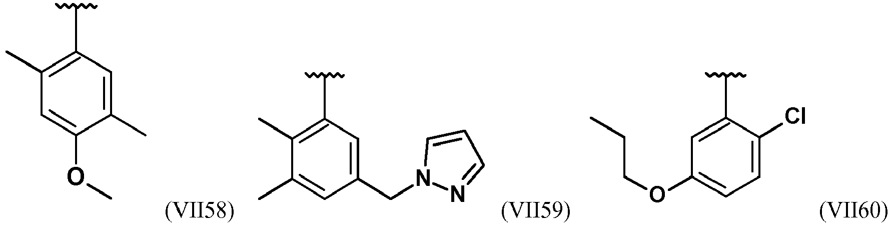

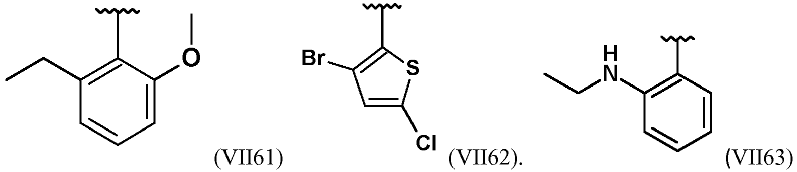

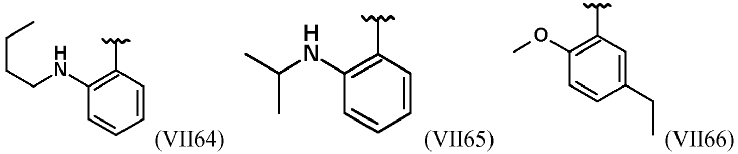

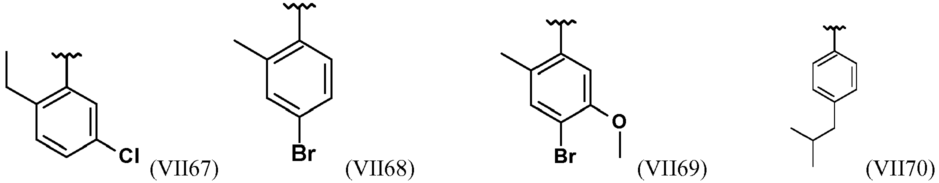

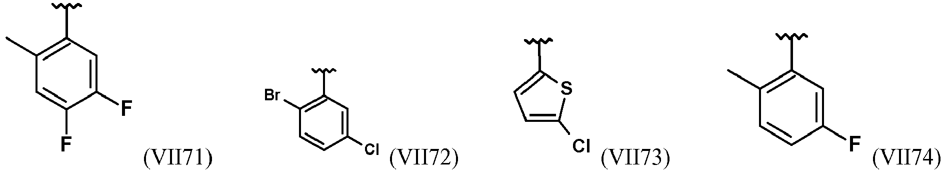

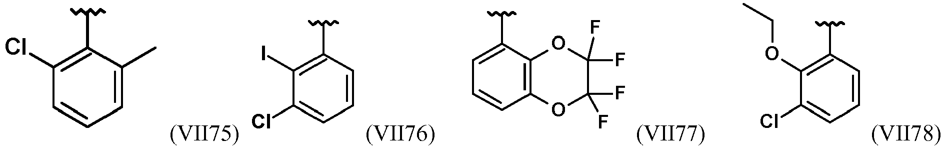

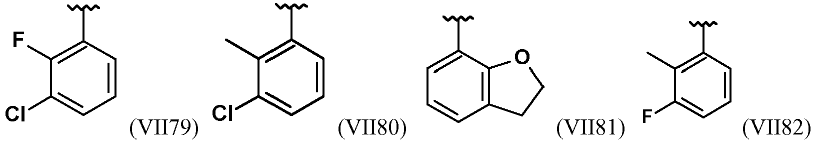

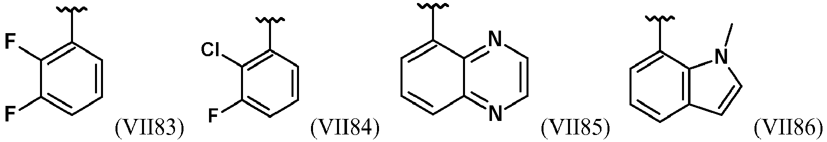

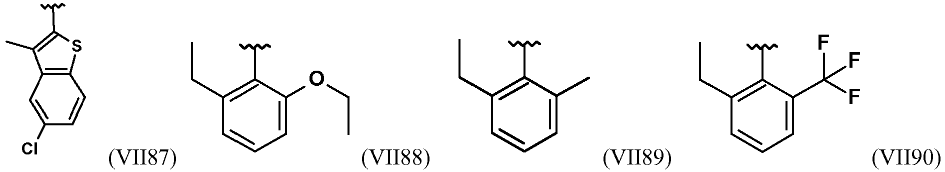

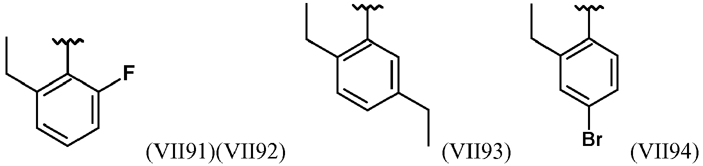

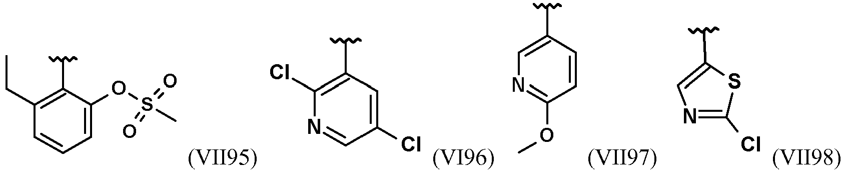

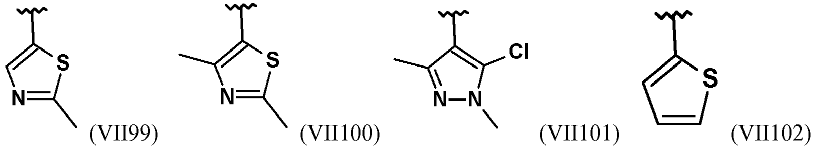

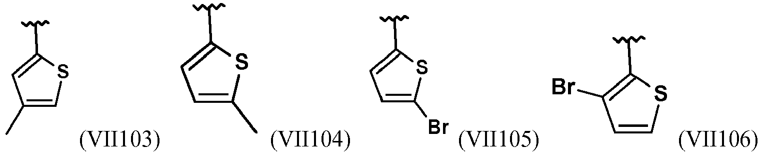

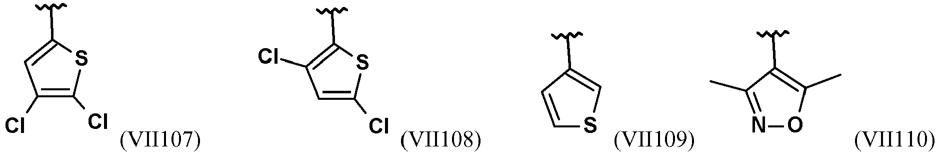

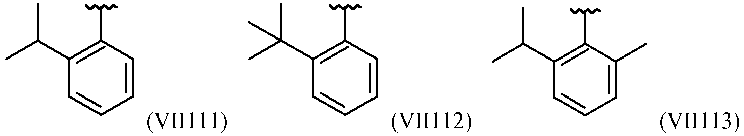

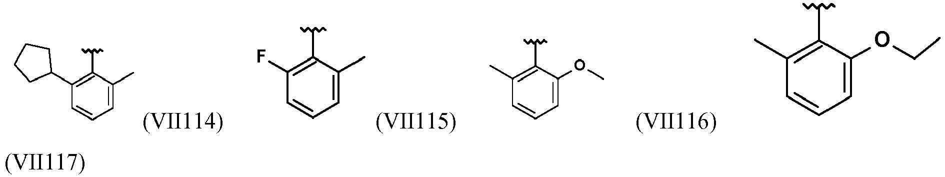

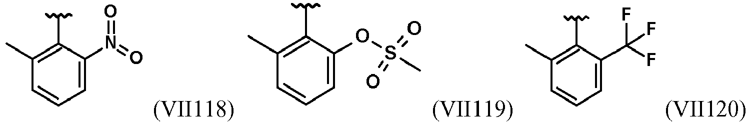

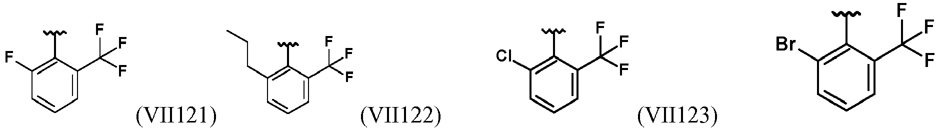

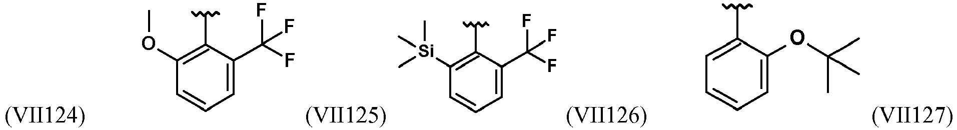

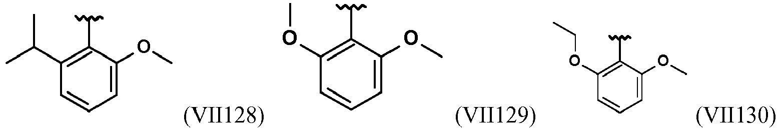

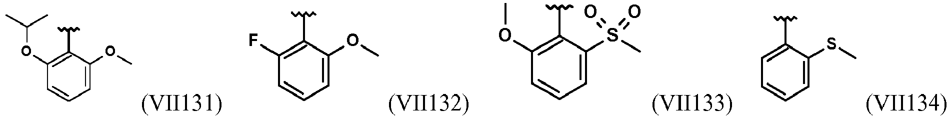

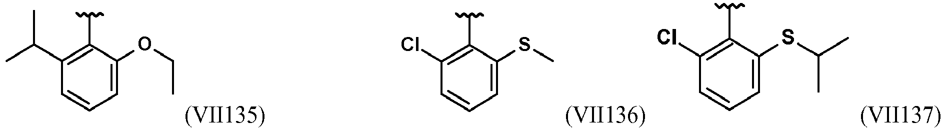

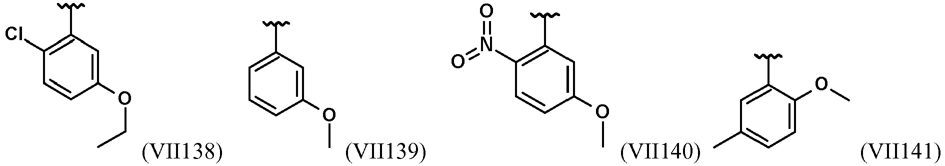

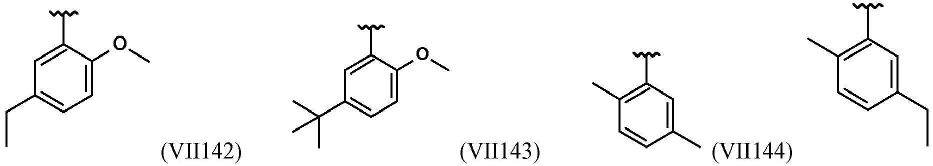

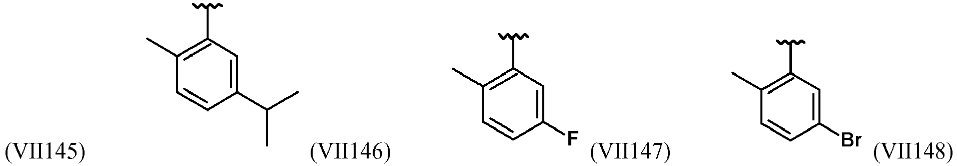

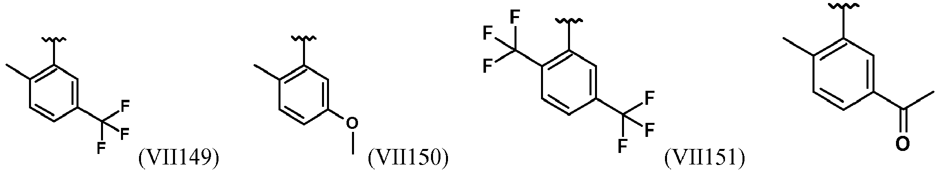

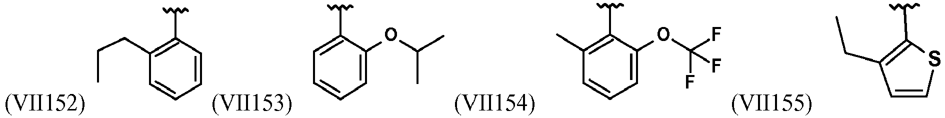

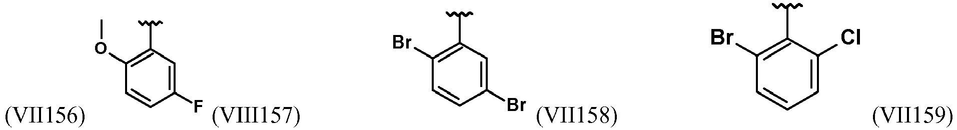

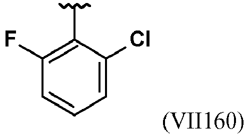

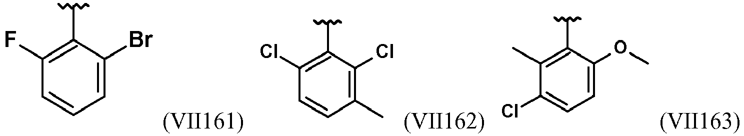

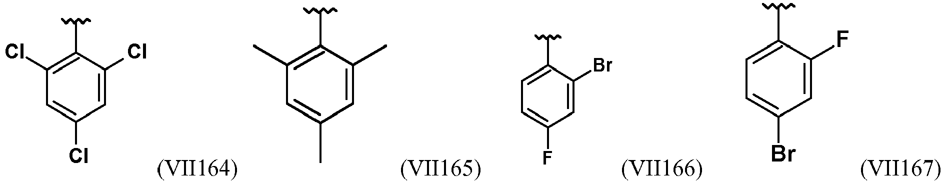

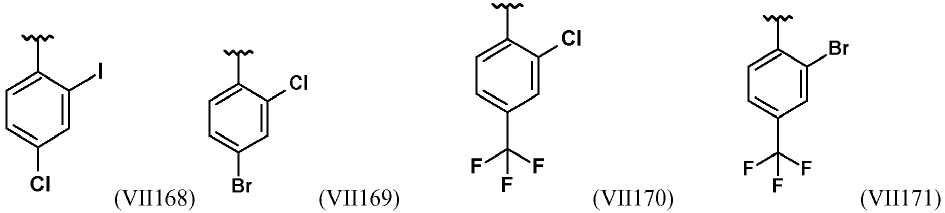

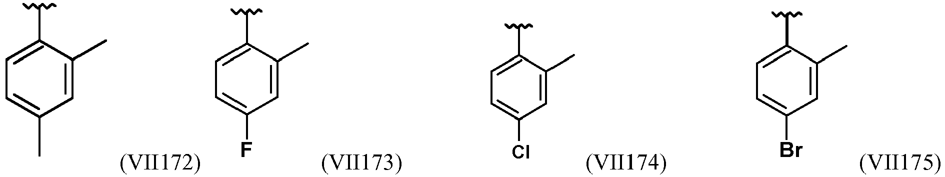

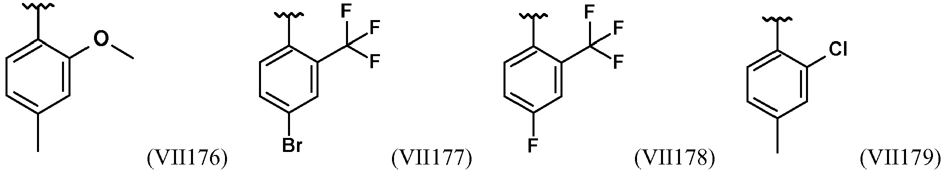

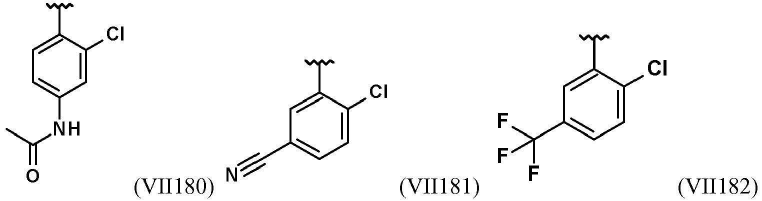

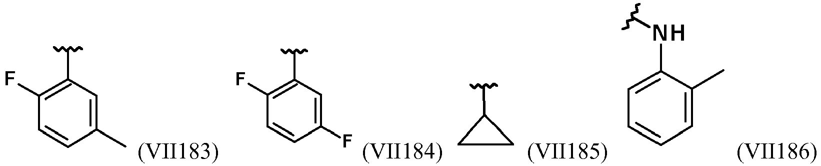

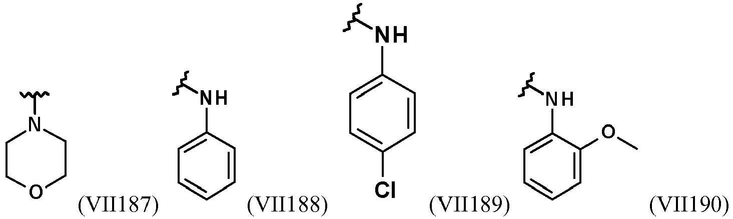

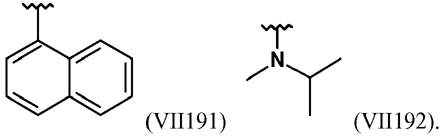

22. Zwischenprodukte der Formeln XIa - XIq

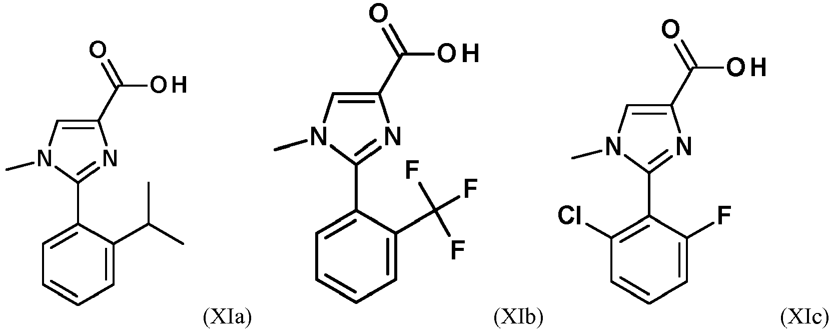

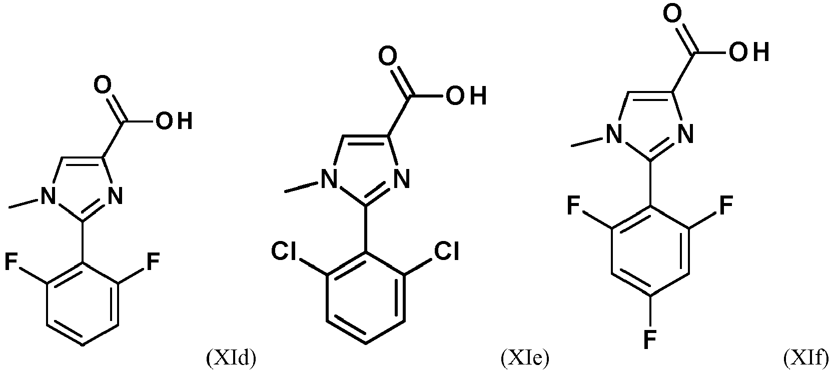

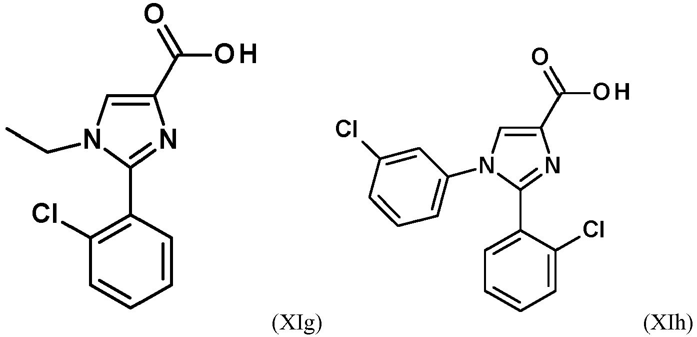

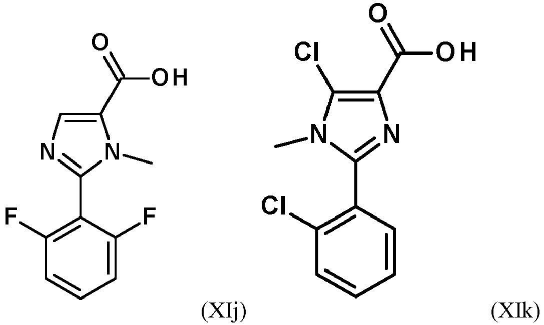

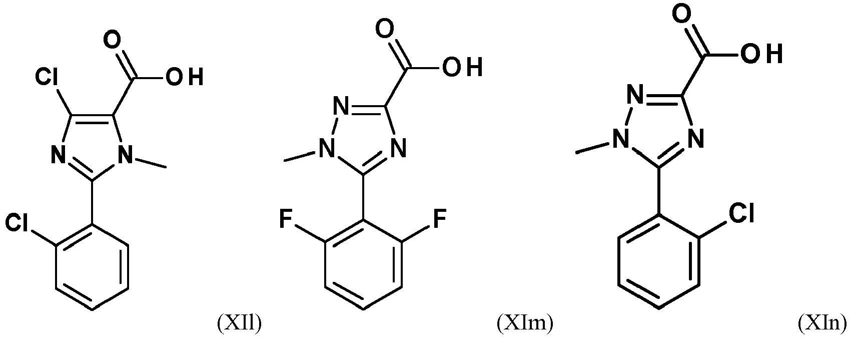

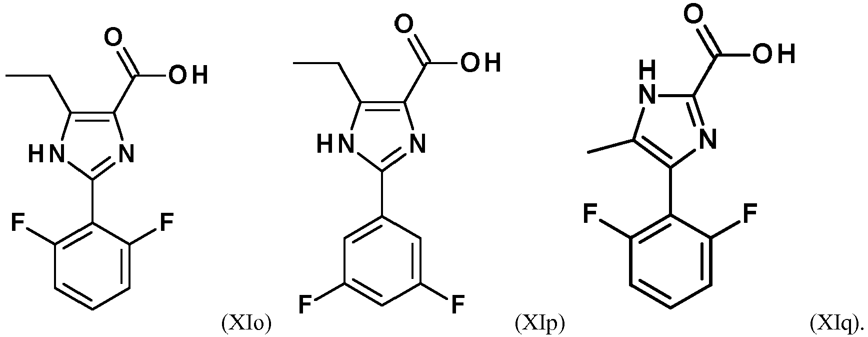

## Claims

1. Use of a compound of the formula (I) 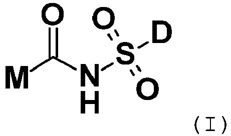 in which
M represents a radical selected from the formulae (IIa-IIf): 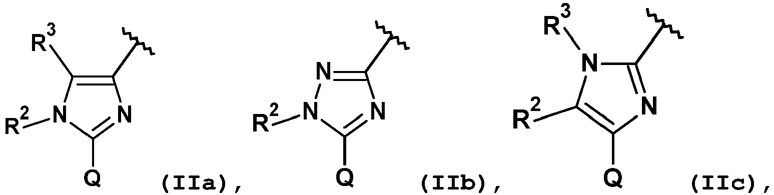 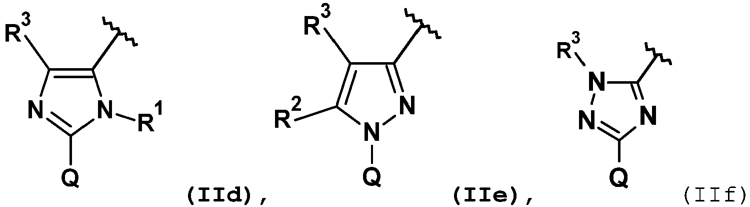 where
R¹, R², R³ each independently of one another represent H or a substituted or unsubstituted alkyl, cycloalkyl, alkenyl, cycloalkenyl, cycloheteroalkyl, aryl or heteroaryl radical, where in cases (IIc) and (IIe) R² may additionally represent a halogen radical or an alkoxy radical, and in cases (IIa), (IId) and (IIe) R³ may additionally represent a halogen radical;
Q represents a substituted or unsubstituted aryl or heteroaryl radical, but, in case (IIe), does not represent 2-pyrimidinyl;
D represents a substituted or unsubstituted alkyl, heteroalkyl, optionally partially unsaturated cycloalkyl, cycloheteroalkyl, heteroaryl, aryl or phenylalkyl radical or a substituted or unsubstituted nitrogen radical,
for controlling animal pests, where the surgical, therapeutic or diagnostic treatment of the human or animal body is excluded.

2. Use according to Claim 1 where
M represents a radical selected from formulae (IIa-IIf), where R¹, R², R³ each independently of one another represent H or a substituted or unsubstituted (C₁-C₄)-alkyl, (C₃-C₆)-cycloalkyl, (C₂-C₄)-alkenyl, pyridyl or phenyl radical, where in cases (IIc) and (IIe) R² may additionally represent a halogen radical or an alkoxy radical and where in cases (IIa), (IId) and (IIe) R³ may additionally represent a halogen radical;
Q represents a substituted or unsubstituted phenyl, naphthyl or heteroaryl radical which may contain one to three heteroatoms from the group consisting of oxygen, sulphur, nitrogen; but, in case (IIe), does not represent 2-pyrimidinyl;
D represents a substituted or unsubstituted alkyl, heteroalkyl, cycloalkyl, heteroaryl, aryl or phenyl-(C₁-C₈)-alkyl radical or a substituted or unsubstituted nitrogen radical.

3. Use according to Claim 1 or 2 where
M represents a radical selected from the formulae (IIa-IIf), where R¹, R², R³ are defined as in Claim 1 or 2 and
Q represents a phenyl, naphthyl, pyridyl, pyrimidinyl, thiophene, benzothiophene, isoquinoline, benzo-dioxole or pyrazole radical which is unsubstituted or substituted by one or more radicals R⁴, but, in case (IIe), does not represent 2-pyrimidinyl,
where the substituent(s) R⁴ is/are each independently of one another selected from:
cyano, halogen, nitro, acetyl, hydroxy, carboxy, amino, SCN, tri-(C₁-C₆)-alkylsilyl, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl- (C₁-C₆)-alkoxy, (C₁-C₆)-alkoxycarbonyl-(C₁-C₆)-alkyl, (C₁-C₆)-alkoxy- (C₁-C₆)-alkyl, (C₂-C₆)-alkenyl, (C₂-C₆)-haloalkenyl, (C₂-C₆)-cyanoal-kenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl- (C₁-C₆)-alkoxy, (C₁-C₆)-alkoxy-(C₁-C₆)-alkoxy, (C₁-C₆)-al-kylhydroxyimino, (C₁-C₆)-alkoxyimino, (C₁-C₆)-alkyl-(C₁-C₆)-alkoxyimino, (C₁-C₆)-haloalkyl-(C₁-C₆)-alkox-yimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkyl-sulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy- (C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-al-kylaminothiocarbonyl, di-(C₁-C₆)-alkylaminocarbonyl, di-(C₁-C₆)-alkylaminothiocarbonyl, (C₂-C₆)-alkenyla-minocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloal-kylamino, NHCO-(C₁-C₆)-alkyl((C₁-C₆)-alkylcarbonylamino) and/or
aryl, aryloxy or hetaryl, optionally mono- or polysubstituted by identical or different substituents, where (in the case of hetaryl) optionally at least one carbonyl group may be present and where possible substituents in each case are as follows: cyano, carboxyl, halogen, nitro, acetyl, hydroxy, amino, SCN, tri-(C₁-C₆)-alkylsilyl, (C₃-C₈)-cycloalkyl, (C₃-C₈)-cycloalkyl- (C₃-C₈)-cycloalkyl, (C₁-C₆)-alkyl- (C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloal-koxy, (C₁-C₆)-cyanoalkoxy, (C₁-C₆)-alkoxycarbonyl-(C₁-C₆)-alkoxy, (C₁-C₆)-alkoxy- (C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloal-kylthio, (C₁-C₆)-alkoxy-(C₁-C₆)-alkylthio, (C₁-C₆)-al-kylthio- (C₁-C₆)-alkyl, (C₁-C₆)-alkylsulphinyl, (C₁-C₆)-haloalkylsulphinyl, (C₁-C₆)-alkoxy- (C₁-C₆)-alkylsulphinyl, (C₁-C₆)-alkylsulphinyl- (C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, di-(C₁-C₆)-alkylaminocarbonyl, (C₂-C₆)-alkenylaminocarbonyl, di-(C₂-C₆)-alkenyla-minocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkyl-aminothiocarbonyl, di-(C₁-C₆)-alkylaminothiocar-bonyl, (C₃-C₈)-cycloalkylamino and (C₁-C₆)-alkylcarbonylamino and
D represents a radical, unsubstituted or substituted by one or more radicals R⁵, from the group consisting of (C₁-C₆)-alkyl, phenyl, phenyl-(C₁-C₂)-alkyl and heteroaryl which may contain one to three heteroatoms from the group consisting of oxygen, sulphur, nitrogen, or represents an NR⁶R⁷ radical,
where the substituent(s) R⁵ is/are each independently of one another selected from:
cyano, halogen, nitro, acetyl, hydroxy, carboxy, amino, SCN, tri-(C₁-C₆)-alkylsilyl, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl- (C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoal-kenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-al-kylhydroxyimino, (C₁-C₆)-alkoxyimino, (C₁-C₆)-alkyl-(C₁-C₆)-alkoxyimino, (C₁-C₆)-haloalkyl- (C₁-C₆)-alkox-yimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkyl-sulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy- (C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-al-kylaminothiocarbonyl, di-(C₁-C₆)-alkylaminocarbonyl, di-(C₁-C₆)-alkylaminothiocarbonyl, (C₂-C₆)-alkenyla-minocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloal-kylamino, (C₁-C₆)-alkylcarbonylamino) and (1-pyrazolyl)-(C₁-C₃)-alkyl
and/or
aryl or hetaryl, mono- or polysubstituted by identical or different substituents, where (in the case of hetaryl) optionally at least one carbonyl group may be present and where possible substituents in each case are as follows: cyano, carboxyl, halogen, nitro, acetyl, hydroxy, amino, SCN, tri-(C₁-C₆)-alkylsilyl, (C₃-C₈)-cycloalkyl, (C₃-C₈)-cycloalkyl-(C₃-C₈)-cycloalkyl, (C₁-C₆)-alkyl- (C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl- (C₁-C₆)-alkyl, (C₁-C₆)-alkoxy- (C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl- (C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl- (C₁-C₆)-alkoxy-imino, (C₁-C₆)-haloalkyl- (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy- (C₁-C₆)-alkylsulphonyl, (C₁-C₆)-alkylsulphonyl- (C₁-C₆)-alkyl, (C₁-C₆)-alkylsulphonyloxy, (C₁-C₆)-alkylcarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, di-(C₁-C₆)-alkylaminocarbonyl, (C₂-C₆)-alkenylaminocarbonyl, di-(C₂-C₆)-alkenylaminocarbonyl, (C₃-C₈)-cycloalkyla-minocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-al-kylaminothiocarbonyl, (C₃-C₈)-cycloalkylamino and (C₁-C₆)-alkylcarbonylamino and where R⁶ and R⁷ each independently of one another represent H, (C₁-C₆)-alkyl or a substituted or unsubstituted phenyl radical or R⁶ and R⁷ together may form an unsubstituted or substituted 4- to 8-membered saturated or optionally fully or partially unsaturated ring which may be interrupted by 1 to 3 heteroatoms from the group consisting of oxygen, sulphur, nitrogen and which may be provided by one or more substitutions corresponding to one of the definitions of R⁵.

4. Use according to any of Claims 1 to 3 where
M represents a radical selected from the formulae (IIa-IIf), where R¹, R², R³ are defined as in Claim 1 or 2 and
Q represents a phenyl, naphthyl, pyridyl, pyrimidinyl, thiophene, benzothiophene, isoquinoline, benzodioxole or pyrazole radical which is unsubstituted or substituted by one or more radicals R⁴, but, in case (IIe), does not represent 2-pyrimidinyl,
where the substituent(s) R⁴ is/are each independently of one another selected from:
cyano, halogen, nitro, acetyl, hydroxy, carboxy, amino, tri-(C₁-C₆)-alkylsilyl, (C₃-C₈)-cycloalkyl, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy- (C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino, phenyl, halophenyl, phenoxy or halophenoxy and
D represents a radical, unsubstituted or substituted by one or more radicals R⁵, from the group consisting of (C₁-C₆)-alkyl, phenyl, phenyl-(C₁-C₂)-alkyl and heteroaryl which may contain one to three heteroatoms from the group consisting of oxygen, sulphur and nitrogen, or represents an NR⁶R⁷ radical,
where the substituent(s) R⁵ is/are each independently of one another selected from:
cyano, halogen, nitro, acetyl, hydroxy, carboxy, amino, tri-(C₁-C₆)-alkylsilyl, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino or (1-pyrazolyl)-(C₁-C₃)-alkyl and
R⁶ and R⁷ each independently of one another represent H, a (C₁-C₆)-alkyl, (C₁-C₆)-haloalkyl, (C₁-C₆)-alkoxy-(C₁-C₆)-alkyl radical or an unsubstituted phenyl radical or a phenyl radical substituted by halogen, (C₁-C₆)-alkyl, (C₁-C₆)-alkoxy, (C₁-C₆)-haloalkyl, (C₁-C₆)-alkoxy-(C₁-C₆)-alkyl
or
R⁶ and R⁷ together may form an unsubstituted or substituted 5- to 6-membered saturated or optionally fully or partially unsaturated ring which may be interrupted by 1 to 3 heteroatoms from the group consisting of oxygen, sulphur and nitrogen and which may be provided by one or more substitutions corresponding to one of the definitions of R⁵.

5. Use according to any of Claims 1 to 4 where
M represents a radical selected from the formulae (IIa-IIf), where
in case (IIa) R², R³ each independently of one another represent H or a substituted or unsubstituted (C₁-C₄)-alkyl, (C₃-C₆)-cycloalkyl or phenyl radical, where R³ may additionally represent a halogen radical,
in case (IIb) R² represents H or a substituted or unsubstituted (C₁-C₄)-alkyl or (C₃-C₆)-cycloalkyl radical,
in case (IIc) R², R³ each independently of one another represent H or a substituted or unsubstituted (C₁-C₄)-alkyl, (C₃-C₆)-cycloalkyl or phenyl radical, where R² may additionally represent a halogen or (C₁-C₄)-alkoxy radical,
in case (IId) R¹, R³ each independently of one another represent H or a substituted or unsubstituted (C₁-C₄)-alkyl or (C₃-C₆)-cycloalkyl radical, where R³ may additionally represent a halogen radical,
in case (IIe) R², R³ each independently of one another represent H, halogen or a substituted or unsubstituted (C₁-C₄)-alkyl, (C₃-C₆)-cycloalkyl, pyridyl or phenyl radical, and R² may additionally represent a (C₁-C₄)-alkoxy radical and
in case (IIf) R³ represents H or a substituted or unsubstituted (C₁-C₄)-alkyl or (C₃-C₆)-cycloalkyl radical
and Q is defined as in any of Claims 1 to 4 and
D represents a (C₁-C₆)-alkyl, phenyl, naphth-2-yl, pyridine, pyrimidine, pyrazole, triazole, thiazole, oxazole, thiadiazole, oxadiazole, thiophene, pyrrole, furan, tetrahydrofuran, dioxane, isoxazole, benzyl, 2,3-dihydro-1,4-benzodioxin-5-yl, 2,3-dihydro-1-benzofuran-7-yl, quinoxalin-5-yl or indol-7-yl radical which is unsubstituted or substituted by one or more radicals R⁵ or represents an NR⁶R⁷ radical,
where the substituent(s) R⁵ is/are each independently of one another selected from:
cyano, halogen, nitro, acetyl, hydroxy, carboxy, amino, tri-(C₁-C₆)-alkylsilyl, (C₃-C₈)-cycloalkyl, (C₃-C₈)-cycloalkyloxy, (C₃-C₈)-cycloalkyl-(C₃-C₈)-cycloalkyl, (C₁-C₆)-alkyl- (C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino or (1-pyrazolyl)-(C₁-C₃)-alkyl and
R⁶ and R⁷ each independently of one another represent H, a (C₁-C₆)-alkyl, (C₁-C₆)-haloalkyl, (C₁-C₆)-alkoxy-(C₁-C₆)-alkyl radical or an unsubstituted phenyl radical or a phenyl radical substituted by halogen, (C₁-C₆)-alkyl, (C₁-C₆)-alkoxy, (C₁-C₆)-haloalkyl or (C₁-C₆)-alkoxy-(C₁-C₆)-alkyl,
or form a ring from the group pyrrolidine, morpholine, piperidine.

6. Use according to any of Claims 1 to 5 where
M represents a radical selected from the radicals of the formulae (IIa) to (IIf) where in case (IIa) R² represents H, methyl or ethyl or optionally halogen-substituted phenyl and R³ represents H, methyl, ethyl, isopropyl or halogen,
in case (IIb) R² represents H, methyl or ethyl,
in case (IIc) R² represents H or methyl and R³ represents H, methyl or ethyl,
in case (IId) R¹ represents H or methyl and R³ represents H or halogen,
in case (IIe) R² represents H, methyl, methoxy, halogen-substituted phenyl or halogen-substituted pyridyl and R³ represents H,
in case (IIf) R³ represents H, methyl or ethyl
and Q is defined as in any of Claims 1 to 4.

7. Use according to any of Claims 1 to 6 where
M is selected from one of the formulae (IIa) to (IIf) or (IVa) to (IVf), where R¹ to R³ are as defined in any of Claims 1, 2, 5 or 6 and
Q represents a phenyl, naphth-1-yl, pyridyl, pyrimidinyl, thiophen-2-yl, benzothiophen-2-yl, benzothiophen-3-yl, isoquinolin-1-yl, benzodioxol-4-yl or pyrazol-5-yl radical which is unsubstituted or substituted by one or more radicals R⁴, but, in case (IVe), does not represent 2-pyrimidinyl,
where the substituent(s) R⁴ independently of one another represent(s):
cyano, halogen, nitro, acetyl, (C₃-C₆)-cycloalkyl, (C₃-C₆)-cycloalkyloxy, (C₃-C₆)-cycloalkyl-(C₃-C₆)-cycloalkyl, (C₁-C₆)-alkyl-(C₃-C₈)-cycloalkyl, halo-(C₃-C₆)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-alkynyl, (C₁-C₆)-alkoxy, (C₁-C₆)-haloalkoxy, (C₁-C₆)-alkylthio, (C₁-C₆)-haloalkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkylsulphonyl, (C₁-C₆)-haloalkylsulphonyl, (C₁-C₆)-alkylsulphonyloxy, (C₁-C₆)-alkylcarbonyl, (C₁-C₆)-alkylamino, di-(C₁-C₆)-alkylamino, (C₁-C₆)-alkylcarbonylamino, phenyl, halophenyl, phenoxy or halophenoxy and
D represents a (C₁-C₆)-alkyl, phenyl, naphth-2-yl, pyridine, pyrimidine, pyrazole, triazole, thiazole, oxazole, thiadiazole, oxadiazole, thiophene, pyrrole, furan, tetrahydrofuran, dioxane, isoxazole, benzyl, 2,3-dihydro-1,4-benzodioxin-5-yl, 2,3-dihydro-1-benzofuran-7-yl, quinoxalin-5-yl or indol-7-yl radical which is unsubstituted or substituted by one or more radicals R⁵ or represents an NR⁶R⁷ radical,
where the substituent(s) R⁵ independently of one another represent(s):
cyano, halogen, nitro, acetyl, hydroxy, carboxy, amino, (C₃-C₆)-cycloalkyl, (C₃-C₆)-cycloalkyloxy, (C₃-C₆)-cycloalkyl-(C₃-C₆)-cycloalkyl, (C₁-C₆)-alkyl-(C₃-C₈)-cycloalkyl, halo-(C₃-C₆)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-alkynyl, (C₁-C₆)-alkoxy, (C₁-C₆)-haloalkoxy, (C₁-C₆)-alkylthio, (C₁-C₆)-haloalkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkylsulphonyl, (C₁-C₆)-haloalkylsulphonyl, (C₁-C₆)-alkylsulphonyloxy, (C₁-C₆)-alkylcarbonyl, (C₁-C₆)-alkylamino, di-(C₁-C₆)-alkylamino, (C₁-C₆)-alkylcarbonylamino or (1-pyrazolyl)-(C₁-C₃)-alkyl and
R⁶ and R⁷ each independently of one another represent H, (C₁-C₆)-alkyl, phenyl, alkoxyphenyl or halophenyl or form a ring from the group pyrrolidine, morpholine, piperidine.

8. Use according to any of Claims 1 to 7 where
M represents a radical selected from the radicals of the formulae (Va-Vz): 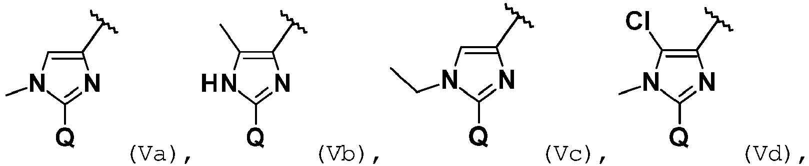 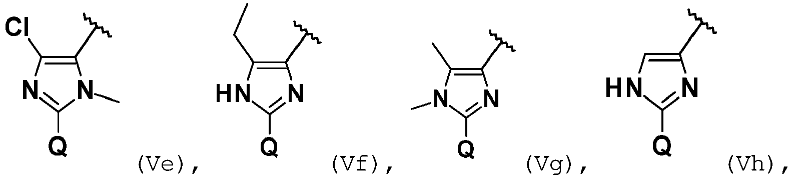 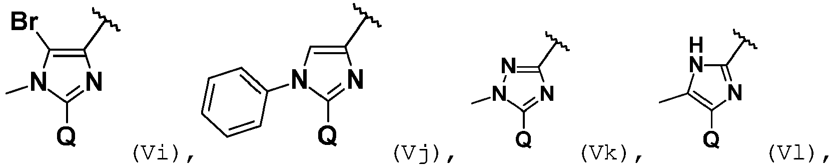 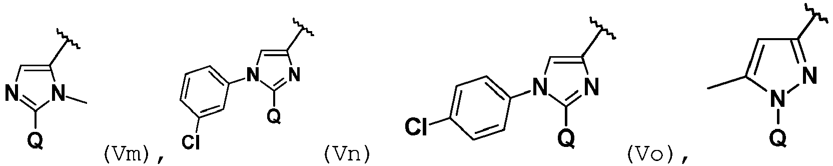 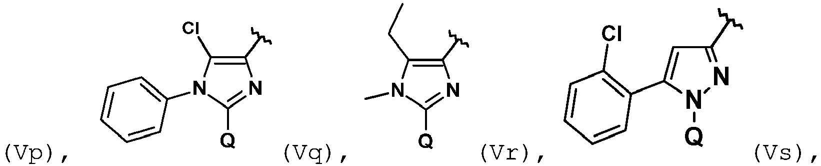 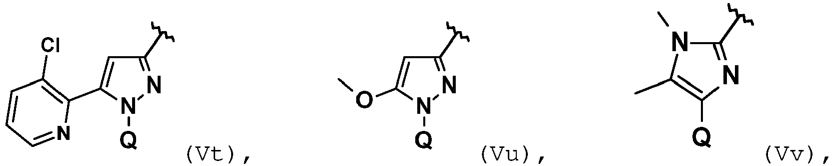 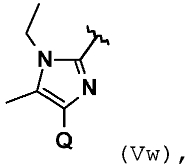 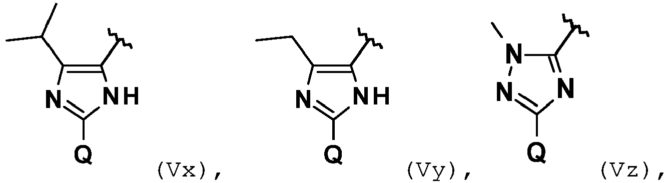 Q represents a radical selected from the radicals of the formulae (VIa-VIz and VIal - VIa30): 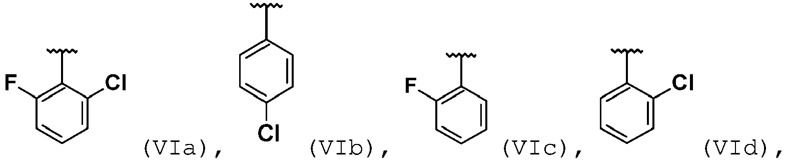 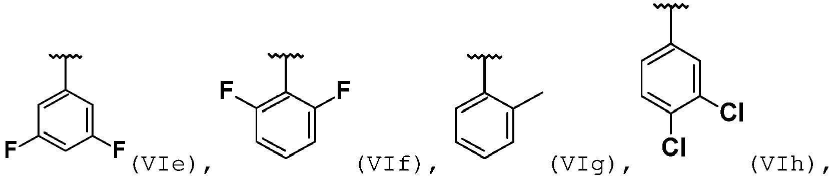 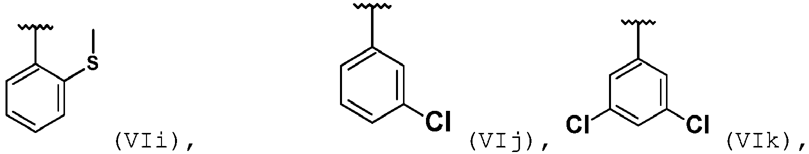 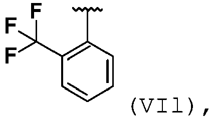 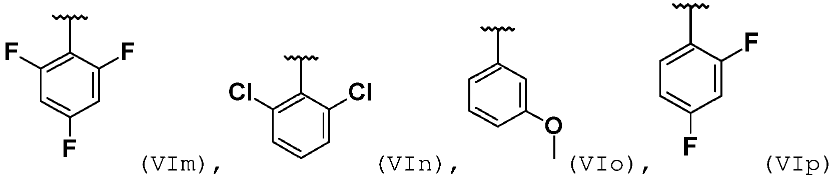 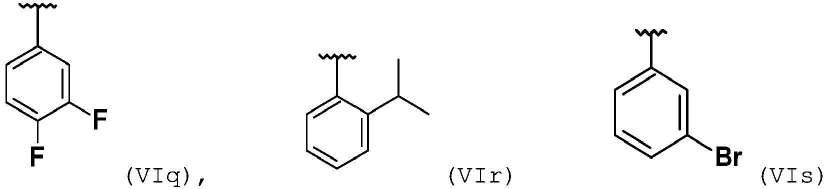 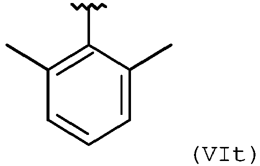 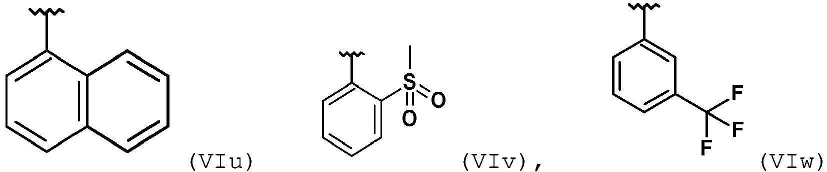 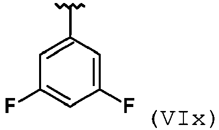 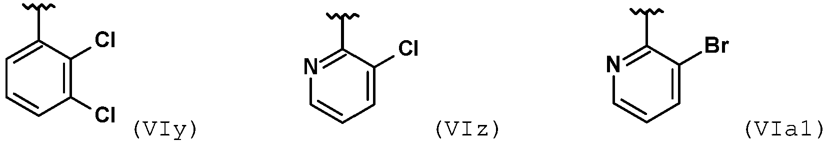 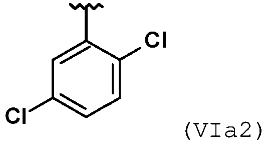 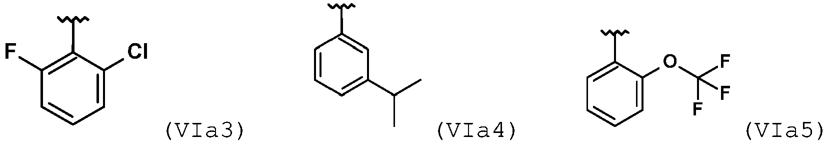 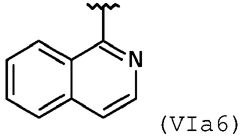 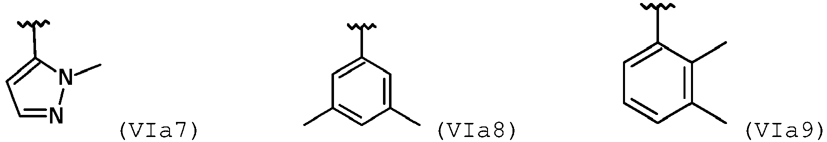 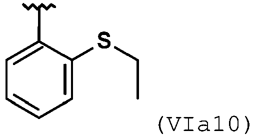 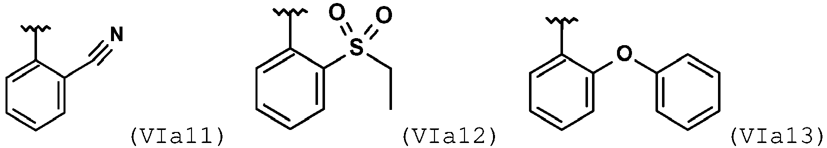 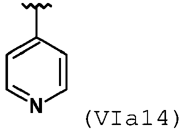 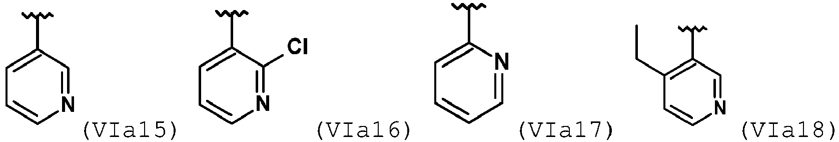 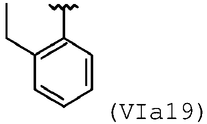 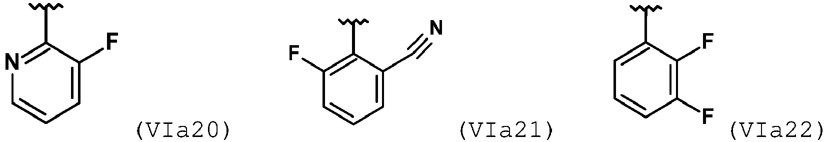 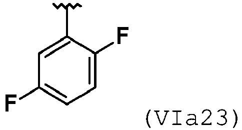 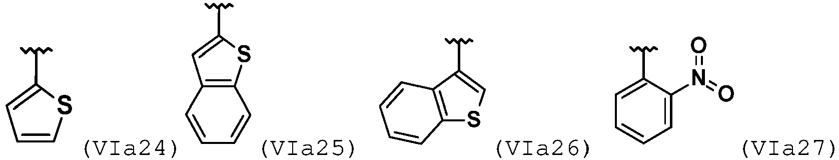 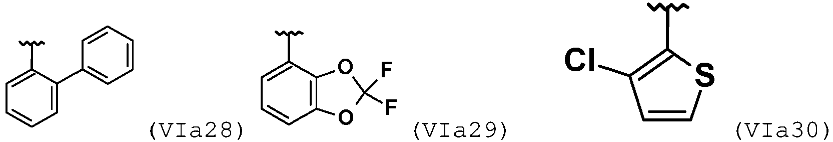 and
D represents a radical selected from the radicals of the formulae (VII1-VII192)

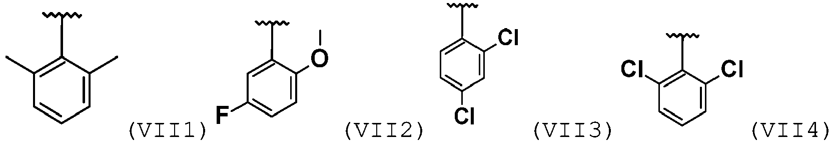

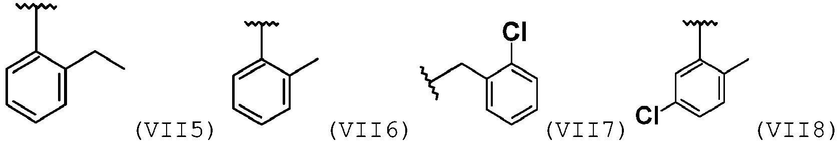

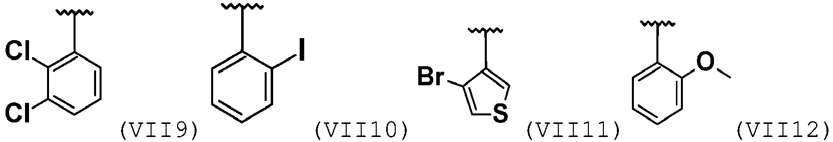

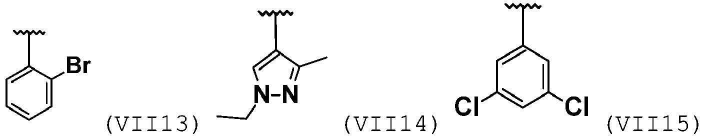

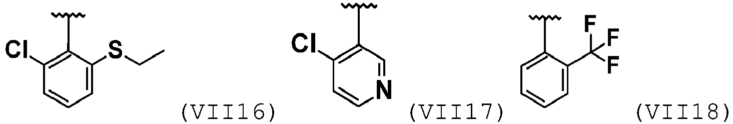

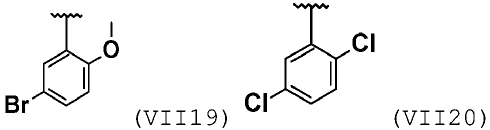

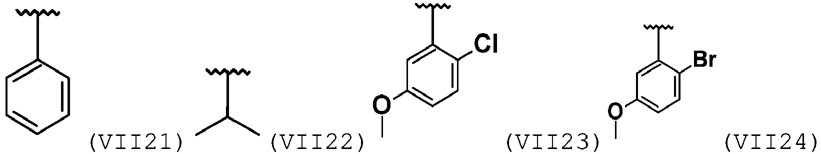

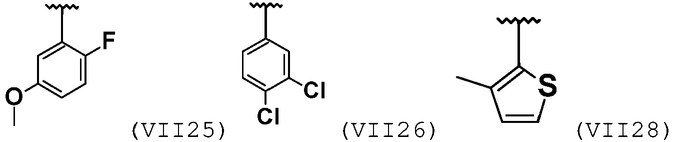

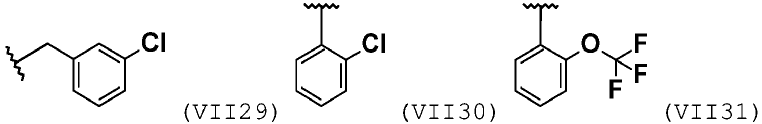

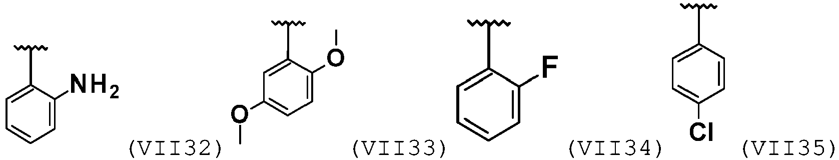

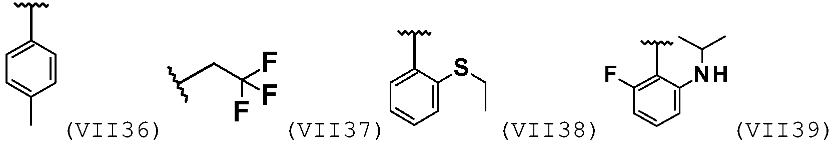

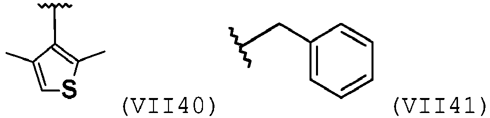

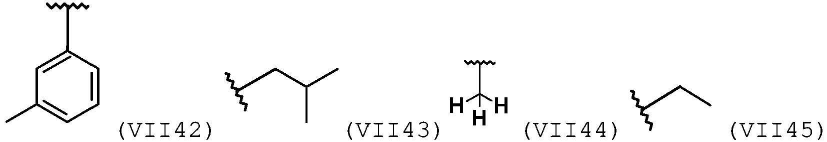

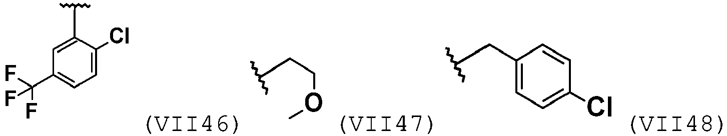

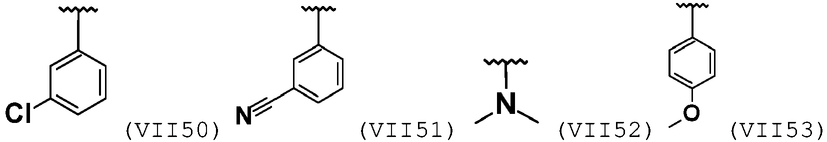

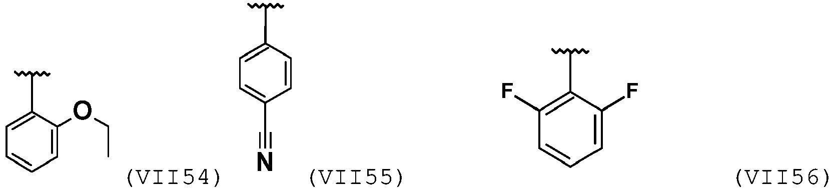

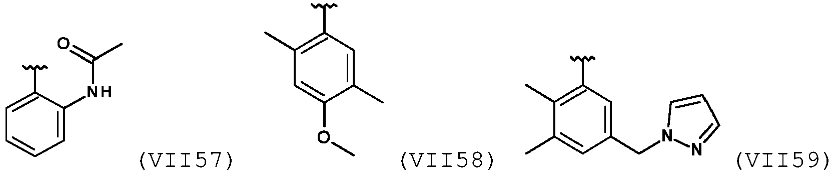

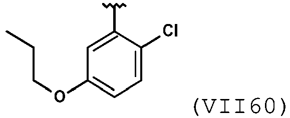

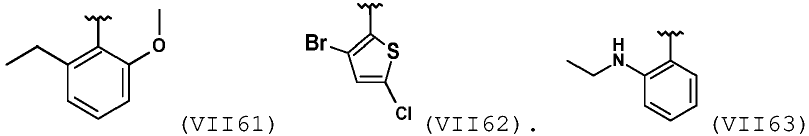

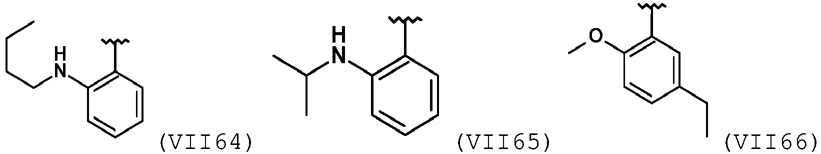

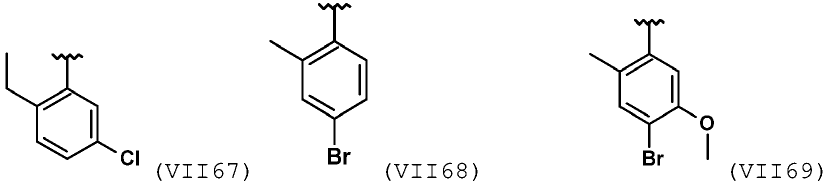

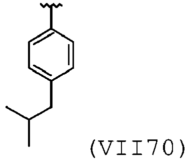

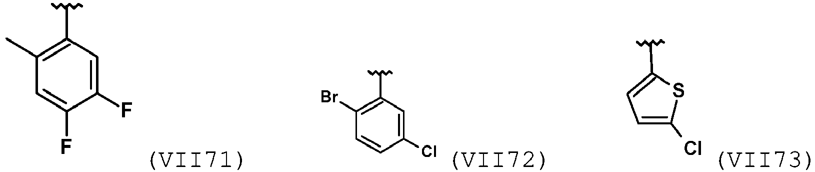

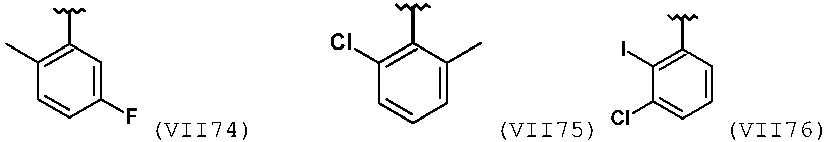

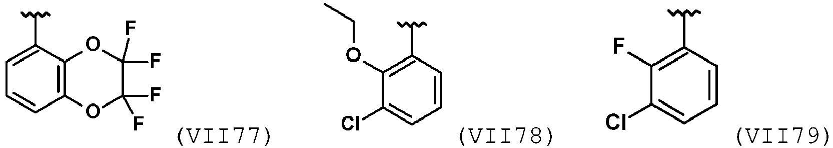

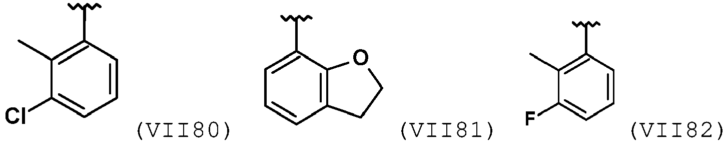

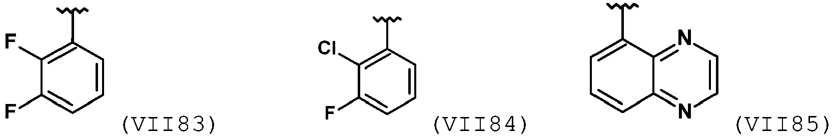

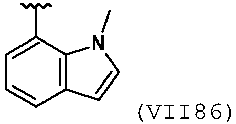

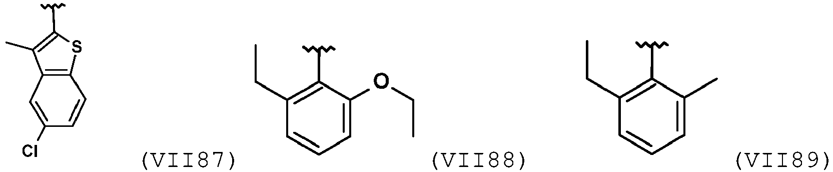

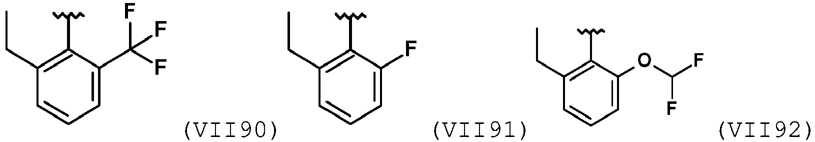

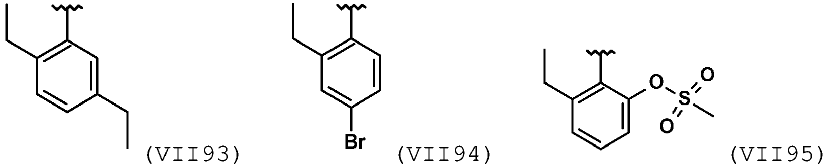

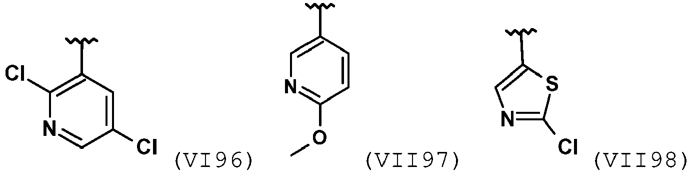

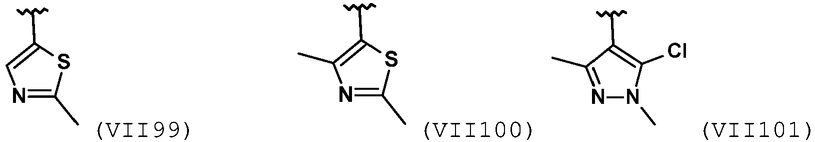

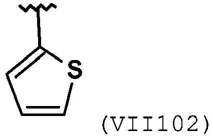

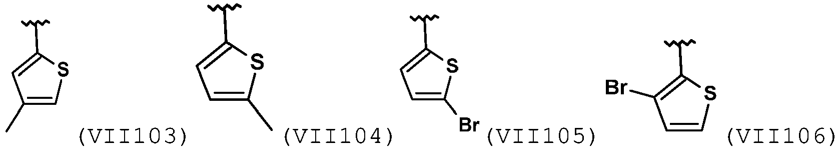

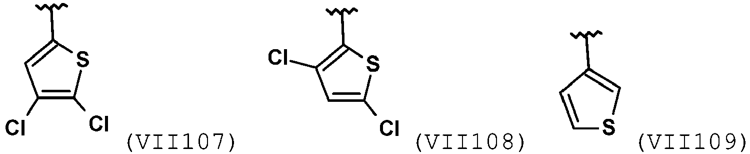

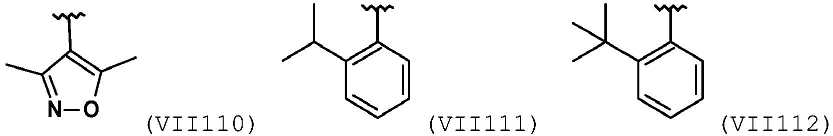

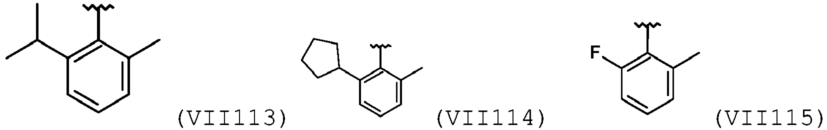

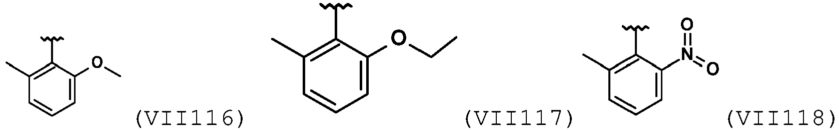

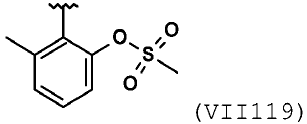

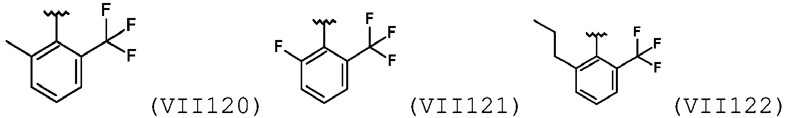

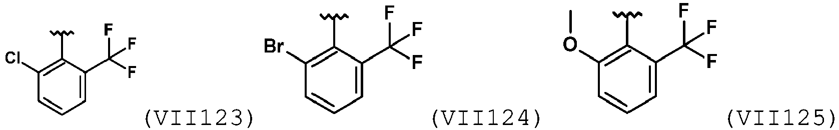

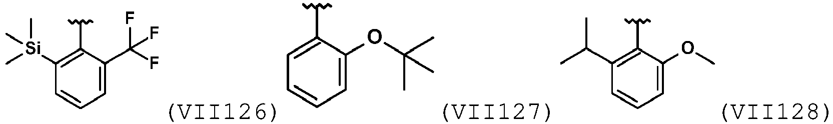

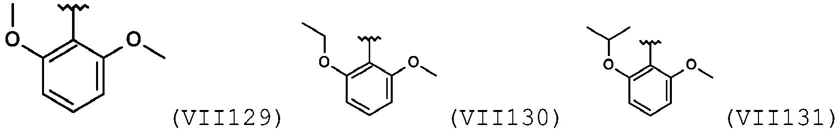

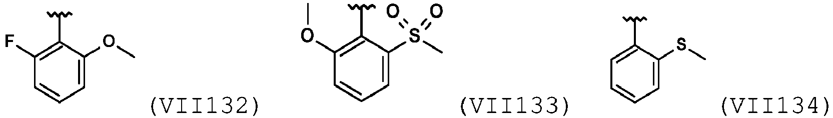

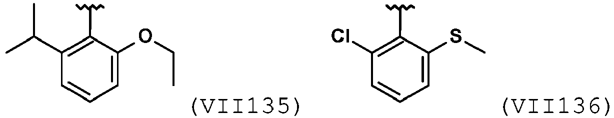

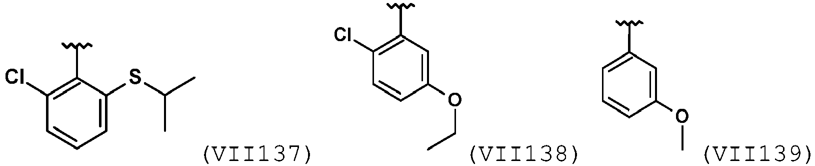

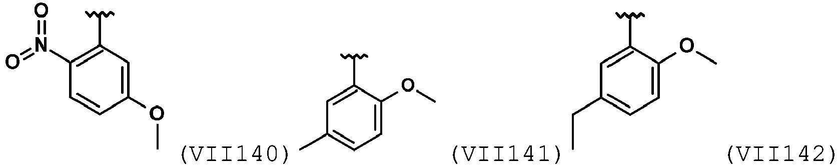

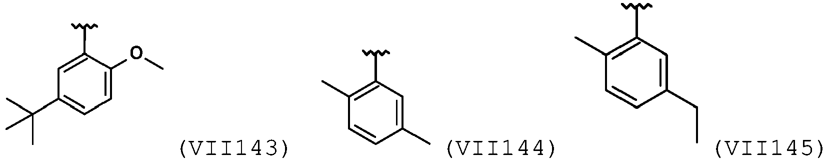

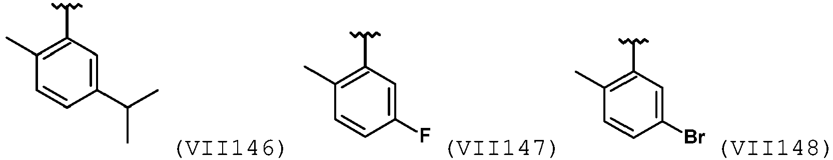

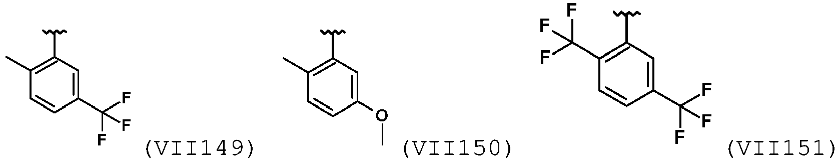

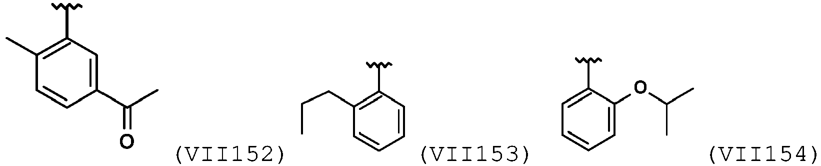

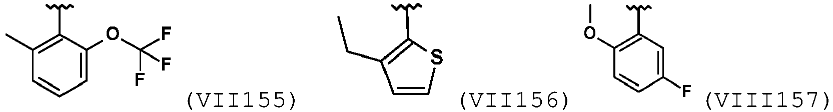

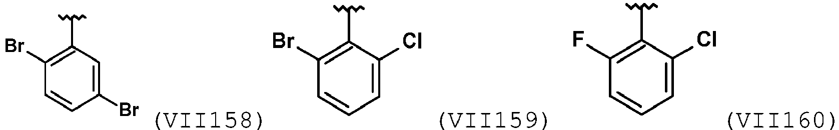

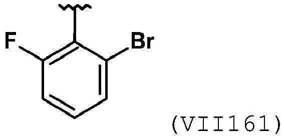

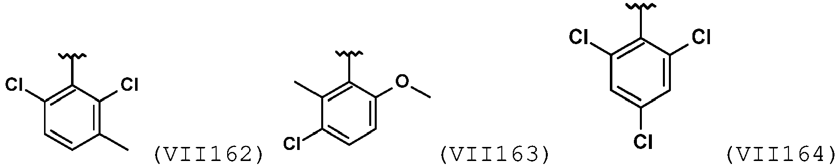

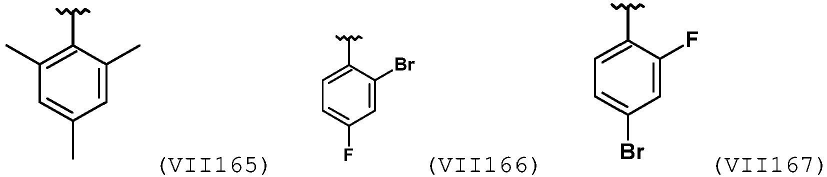

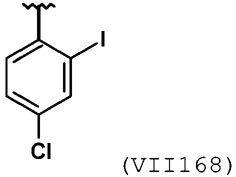

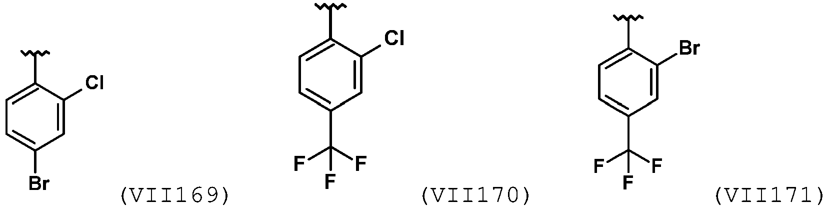

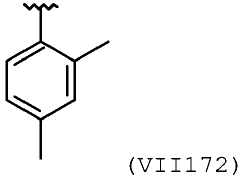

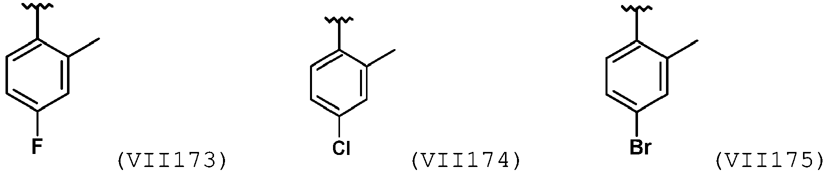

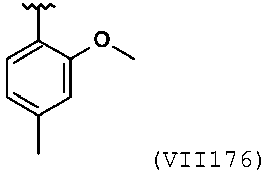

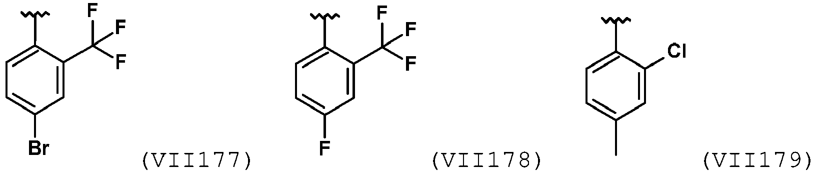

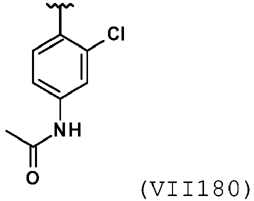

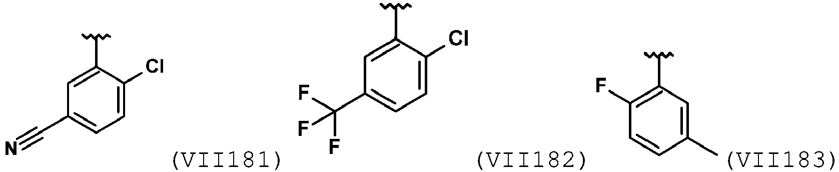

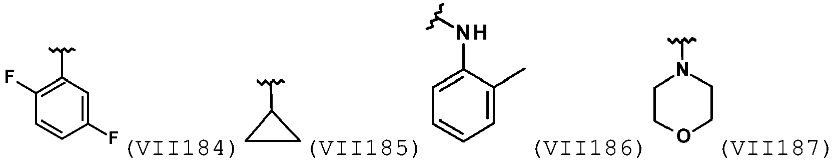

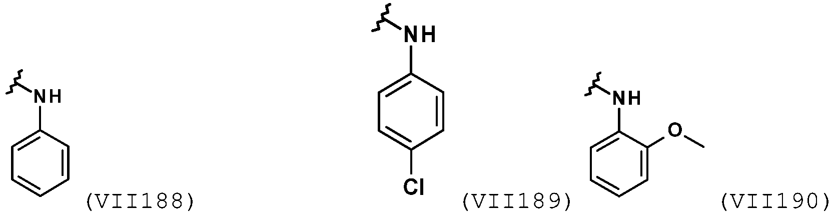

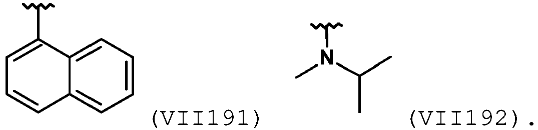

9. Use according to any of Claims 1 to 8 for protecting the propagation material of plants.

10. Composition, comprising at least one compound of the formula (I) according to any of Claims 1 to 8 and customary extenders and/or surfactants in particular for controlling animal pests.

11. Method for controlling animal pests, where at least one compound of the formula (I) according to any of Claims 1 to 8 or a composition according to Claim 10 is allowed to act on the animal pests and/or their habitat, where the surgical, therapeutic and diagnostic treatment of the human or animal body is excluded.

12. Agrochemical formulation comprising at least one compound of the formula (I) according to any of Claims 1 to 8 in biologically effective amounts of from 0.00000001 to 98% by weight based on the weight of the agrochemical formulation, and also extenders and/or surfactants.

13. Agrochemical formulation according to Claim 12, additionally comprising at least one further agrochemically active compound.

14. Compounds of the formula (VIII) 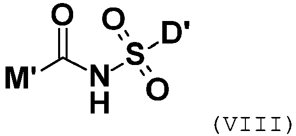 in which
M' represents a radical of the formula (II) selected from: 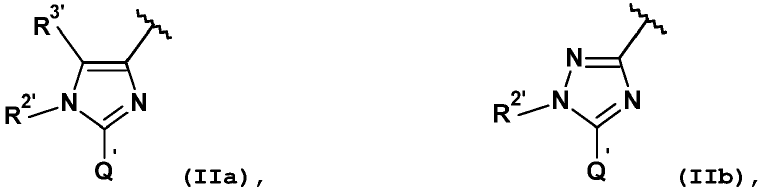 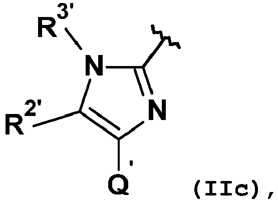 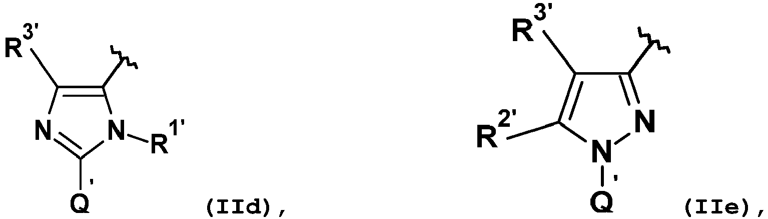 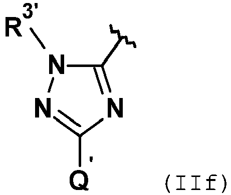 in which
R^{1'}, R^{2'}, R^{3'} each independently of one another represent H or a substituted or unsubstituted alkyl, cycloalkyl, alkenyl, cycloalkenyl, cycloheteroalkyl, aryl or heteroaryl radical, where in cases (IIc) and (IIe) R² may additionally represent a halogen radical or an alkoxy radical, in cases (IIa) and (IIb) R^{2'} may only represent H or a substituted or unsubstituted alkyl or cycloalkyl radical and in cases (IIa), (IId) and (IIe) R^{3'} may additionally represent a halogen radical,
Q' represents a substituted or unsubstituted aryl or heteroaryl radical, but in cases (IIa), (IId) in case R^{1'} = H and R^{3'} = methyl does not represent 3-methoxyphenyl, in case (IId) does not represent 3-pyridyl, in case (IIe) does not represent 2-pyrimidinyl, nor unsubstituted phenyl, nor 3,4-dichlorophenyl, nor 3,5-bis-tert-butyl;
D' represents a substituted or unsubstituted alkyl, heteroalkyl, optionally partially unsaturated cycloalkyl, cycloheteroalkyl, heteroaryl, aryl or phenylalkyl radical or in the case that Q' carries at least one substituent in the 2-position represents a substituted or unsubstituted nitrogen radical.

15. Compounds according to Claim 14 in which
M' represents a radical of one of the formulae (IIa) to (IIf) and
in case (IIa) R^{2'}, R^{3'} each independently of one another represent H or a substituted or unsubstituted (C₁-C₄)-alkyl, (C₃-C₆)-cycloalkyl or phenyl radical, where R^{3'} may additionally represent a halogen radical;
in case (IIb) R^{2'} represents H or a substituted or unsubstituted (C₁-C₄)-alkyl or (C₃-C₆)-cycloalkyl radical,
in case (IIc) R^{2'}, R^{3'} each independently of one another represent H or a substituted or unsubstituted (C₁-C₄)-alkyl, (C₃-C₆)-cycloalkyl or phenyl radical, where R^{2'} may additionally represent a halogen radical or a (C₁-C₄)-alkoxy radical,
in case (IId) R^{1'}, R^{3'} each independently of one another represent H or a substituted or unsubstituted (C₁-C₄)-alkyl or (C₃-C₆)-cycloalkyl radical and R^{3'} may additionally represent a halogen radical,
in case (IIe) R^{2'}, R^{3'} each independently represent H, halogen or a substituted or unsubstituted (C₁-C₄)-alkyl, (C₃-C₆)-cycloalkyl, pyridyl or phenyl radical, and R² may additionally represent a (C₁-C₄)-alkoxy radical and
in case (IIf) R³ represents H or a substituted or unsubstituted (C₁-C₄)-alkyl or (C₃-C₆)-cycloalkyl radical,
Q' represents a substituted or unsubstituted phenyl, naphthyl or heteroaryl radical which may contain one to three heteroatoms from the group of oxygen, sulphur and nitrogen, but in cases (IIa), (IId) in case R^{1'} = H and R^{3'} = methyl does not represent 3-methoxyphenyl, in case (IId) does not represent 3-pyridyl, in case (IIe) does not represent 2-pyrimidinyl, nor unsubstituted phenyl, nor 3,4-dichlorophenyl,
D' represents a substituted or unsubstituted alkyl, heteroalkyl, cycloalkyl, heteroaryl, aryl or phenyl-(C₁-C₈)-alkyl radical or, in the case that Q' carries at least one substituent in the 2-position, represents a substituted or unsubstituted nitrogen radical.

16. Compounds according to Claim 14 or 15 where
M' represents a radical selected from the formulae (IIa-IIf), where R^{1'}, R^{2'}, R^{3'} are defined as in Claim 14 or 15 and
Q' represents a phenyl, naphthyl, pyridyl, pyrimidinyl, thiophene, benzothiophene, isoquinoline, benzodioxole or pyrazole radical which is unsubstituted or substituted by one or more radicals R^{4'}, but in cases (IIa), (IId) in the case R^{1'} = H and R^{3'} = methyl does not represent 3-methoxyphenyl, in case (IId) does not represent 3-pyridyl, in case (IIe) does not represent 2-pyrimidinyl, nor unsubstituted phenyl, nor 3,4-dichlorophenyl and
where the substituent(s) R^{4'} is/are each independently of one another selected from:
cyano, halogen, nitro, acetyl, hydroxy, carboxy, amino, SCN, tri-(C₁-C₆)-alkylsilyl, (C₃-C₈)-cycloalkyl, (C₃-C₈)-cycloalkyloxy, (C₃-C₈)-cycloalkyl- (C₃-C₈)-cycloalkyl, (C₁-C₆)-alkyl- (C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino; and/or
aryl, aryloxy or hetaryl, optionally mono- or polysubstituted by identical or different substituents, where (in the case of hetaryl) optionally at least one carbonyl group may be present and where possible substituents in each case are as follows: cyano, carboxyl, halogen, nitro, acetyl, hydroxy, amino, SCN, tri-(C₁-C₆)-alkylsilyl, (C₃-C₈)-cycloalkyl, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, di-(C₁-C₆)-alkylaminocarbonyl, (C₂-C₆)-alkenylaminocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloalkylamino and (C₁-C₆)-alkylcarbonylamino and
D' represents a radical, unsubstituted or substituted by one or more radicals R^{5'}, from the group consisting of (C₁-C₆)-alkyl, phenyl and heteroaryl which may contain one to three heteroatoms from the group consisting of oxygen, sulphur and nitrogen, or, in the case that Q' carries at least one substituent in the 2-position, represents an NR^{6'}R^{7'} radical,
where the substituent(s) R^{5'} is/are each independently of one another selected from:
cyano, halogen, nitro, acetyl, hydroxy, carboxy, amino, SCN, tri-(C₁-C₆)-alkylsilyl, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio- (C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino and (1-pyrazolyl)-(C₁-C₃)-alkyl,
and/or
aryl or hetaryl, mono- or polysubstituted by identical or different substituents, where (in the case of hetaryl) optionally at least one carbonyl group may be present and where possible substituents in each case are as follows: cyano, carboxyl, halogen, nitro, acetyl, hydroxy, amino, SCN, tri-(C₁-C₆)-alkylsilyl, (C₃-C₈)-cycloalkyl, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy- (C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, di-(C₁-C₆)-alkylaminocarbonyl, (C₂-C₆)-alkenylaminocarbonyl, di-(C₂-C₆)-alkenylaminocarbonyl, (C₃-C₈)-cycloalkylaminocarbonyl, (C₁-C₆)-alkylsulphonylamino, (C₁-C₆)-alkylamino, di-(C₁-C₆)-alkylamino, aminosulphonyl, (C₁-C₆)-alkylaminosulphonyl, di-(C₁-C₆)-alkylaminosulphonyl, (C₁-C₆)-alkylsulphoximino, aminothiocarbonyl, (C₁-C₆)-alkylaminothiocarbonyl, di-(C₁-C₆)-alkylaminothiocarbonyl, (C₃-C₈)-cycloalkylamino and (C₁-C₆)-alkylcarbonylamino,
and where R^{6'} and R^{7'} each independently of one another represent H, a (C₁-C₆)-alkyl, (C₁-C₆)-haloalkyl, (C₁-C₆)-alkoxy-(C₁-C₆)-alkyl radical or an unsubstituted phenyl radical or a phenyl radical substituted by halogen, (C₁-C₆)-alkyl, (C₁-C₆)-alkoxy, (C₁-C₆)-haloalkyl, (C₁-C₆)-alkoxy-(C₁-C₆)-alkyl, or
R^{6'} and R^{7'} together may form an unsubstituted or substituted 5- to 6-membered saturated or optionally fully or partially unsaturated ring which may be interrupted by 1 to 3 heteroatoms from the group consisting of oxygen, sulphur, nitrogen and which may be provided by one or more substitutions corresponding to one of the definitions of R^{5'}.

17. Compounds according to any of Claims 14 to 16 where M' represents a radical selected from the formulae (IIa-IIf), where R^{1'}, R^{2'}, R^{3'} are defined as in Claim 14 or 15 and
Q' represents a phenyl, naphthyl, pyridyl, pyrimidinyl, thiophene, benzothiophene, isoquinoline, benzodioxole or pyrazole radical which is unsubstituted or substituted by one or more radicals R^{4'}, but in cases (IIa), (IId) in the case R^{1'} = H and R^{3'} = methyl does not represent 3-methoxyphenyl, in case (IId) does not represent 3-pyridyl, in case (IIe) does not represent 2-pyrimidinyl, nor unsubstituted phenyl, nor 3,4-dichlorophenyl,
and the substituent(s) R^{4'} each independently of one another are selected from: cyano, halogen, nitro, acetyl, hydroxy, carboxy, amino, tri-(C₁-C₆)-alkylsilyl, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkoxy-(C₁-C₆)-alkylsulphinyl, (C₁-C₆)-alkylsulphinyl-(C₁-C₆)-alkyl, (C₁-C₆)-alkylsulphonyl, (C₁-C₆)-haloalkylsulphonyl, (C₁-C₆)-alkoxy-(C₁-C₆)-alkylsulphonyl, (C₁-C₆)-alkylsulphonyl-(C₁-C₆)-alkyl, (C₁-C₆)-alkylsulphonyloxy, (C₁-C₆)-alkylcarbonyl, (C₁-C₆)-alkylthiocarbonyl, (C₁-C₆) -haloalkylcarbonyl, (C₁-C₆) -alkylcarbonyloxy, (C₁-C₆) -alkoxycarbonyl, (C₁-C₆) -haloalkoxycarbonyl, aminocarbonyl, (C₁-C₆) -alkylaminocarbonyl, (C₁-C₆)-alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylaminocar-bonyl, (C₃-C₈)-cycloalkylaminocarbonyl, (C₁-C₆) -alkylsulphonylamino, (C₁-C₆) -alkylamino, di-(C₁-C₆) -alkylamino, aminosulphonyl, (C₁-C₆)-alkylaminosulphonyl, di-(C₁-C₆) -alkylaminosulphonyl, (C₁-C₆) -alkyl-sulphoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino, phenyl, halophenyl, phenoxy or halophenoxy and
D' represents a phenyl, pyridine, pyrimidine, pyrazole, triazole, thiazole, oxazole, thiadiazole, oxadiazole, thiophene, pyrrole, furan, tetrahydrofuran, dioxane or (C₁-C₆)-alkyl radical which is unsubstituted or substituted by one or more radicals R^{5'}, or represents an NR^{6'}R^{7'} radical,
where the substituent(s) R^{5'} is/are each independently of one another selected from:
cyano, halogen, nitro, acetyl, hydroxy, carboxy, amino, tri-(C₁-C₆) -alkylsilyl, (C₃-C₈)-cycloalkyl, (C₃-C₈)-cycloalkyloxy, (C₃-C₈)-cycloalkyl- (C₃-C₈)-cycloalkyl, (C₁-C₆) -alkyl- (C₃-C₈)-cycloalkyl, halo- (C₃-C₈) -cycloalkyl, (C₁-C₆) -alkyl, (C₁-C₆) -haloalkyl, (C₁-C₆)-cyanoalkyl, (C₁-C₆) -hydroxyalkyl, hydroxycarbonyl-(C₁-C₆) -alkoxy, (C₁-C₆) -alkoxycarbonyl- (C₁-C₆) - alkyl, (C₁-C₆) -alkoxy- (C₁-C₆) -alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆) -alkoxy, (C₁-C₆) -haloalkoxy, (C₁-C₆) -cyanoalkoxy, (C₁-C₆) -alkoxycarbonyl- (C₁-C₆) -alkoxy, (C₁-C₆)-alkoxy- (C₁-C₆) -alkoxy, (C₁-C₆) -alkylhydroxyimino, (C₁-C₆) -alkoxyimino, (C₁-C₆) -alkyl- (C₁-C₆) -alkoxyimino, (C₁-C₆) -haloalkyl- (C₁-C₆) -alkoxyimino, (C₁-C₆)-alkylthio, (C₁-C₆) -haloalkylthio, (C₁-C₆) -alkoxy- (C₁-C₆)-alkylthio, (C₁-C₆) -alkylthio- (C₁-C₆) -alkyl, (C₁-C₆)-alkylsulphinyl, (C₁-C₆) -haloalkylsulphinyl, (C₁-C₆) -alkoxy- (C₁-C₆) -alkylsulphinyl, (C₁-C₆) -alkylsulphinyl- (C₁-C₆) -alkyl, (C₁-C₆) -alkylsulphonyl, (C₁-C₆) -haloalkylsulphonyl, (C₁-C₆) -alkoxy- (C₁-C₆) -alkylsulphonyl, (C₁-C₆) -alkylsulphonyl- (C₁-C₆) -alkyl, (C₁-C₆)-alkylsulphonyloxy, (C₁-C₆) -alkylcarbonyl, (C₁-C₆)-alkylthiocarbonyl, (C₁-C₆)-haloalkylcarbonyl, (C₁-C₆) -alkylcarbonyloxy, (C₁-C₆) -alkoxycarbonyl, (C₁-C₆) -haloalkoxycarbonyl, aminocarbonyl, (C₁-C₆)-alkylaminocarbonyl, (C₁-C₆)-alkylaminothiocarbonyl, di-(C₁-C₆) -alkylaminocarbonyl, di-(C₁-C₆) -alkylaminothiocarbonyl, (C₂-C₆)-alkenylaminocarbonyl, di-(C₂-C₆)-alkenylaminocarbonyl, (C₃-C₈)-cycloalkyla-minocarbonyl, (C₁-C₆) -alkylsulphonylamino, (C₁-C₆)-alkylamino, di-(C₁-C₆)-alkylamino, aminosulphonyl, (C₁-C₆) -alkylaminosulphonyl, di-(C₁-C₆) -alkylaminosulphonyl, (C₁-C₆)-alkylsulphoximino, aminothiocarbonyl, (C₁-C₆) -alkylaminothiocarbonyl, di-(C₁-C₆) -alkylaminothiocarbonyl, (C₃-C₈)-cycloalkylamino, (C₁-C₆) -alkylcarbonylamino and (1-pyrazolyl)-(C₁-C₃)-alkyl,
and where R^{6'} and R^{7'} each independently of one another represent H, a (C₁-C₄)-alkyl, (C₁-C₄)-haloalkyl, (C₁-C₄)-alkoxy-(C₁-C₄)-alkyl radical or an unsubstituted phenyl radical or a phenyl radical substituted by halogen, (C₁-C₄)-alkyl, (C₁-C₄)-alkoxy, (C₁-C₄)-haloalkyl, (C₁-C₄)-alkoxy- (C₁-C₄)-alkyl, or
R^{6'} and R^{7'} together may form an unsubstituted or substituted 5- to 6-membered saturated or optionally fully or partially unsaturated ring which may be interrupted by 1 to 3 heteroatoms from the group consisting of oxygen, sulphur, nitrogen and which may be provided by one or more substitutions corresponding to one of the definitions of R^{5'}.

18. Compounds according to any of Claims 14 to 17 where
D' represents a (C₁-C₆)-alkyl radical, phenyl radical, pyridine, pyrimidine, pyrazole, triazole, thiazole, oxazole, thiadiazole, oxadiazole, thiophene, pyrrole, furan, tetrahydrofuran, dioxane, isoxazole, benzyl, 2,3-dihydro-1,4-benzodioxin-5-yl, 2,3-dihy-dro-1-benzofuran-7-yl, quinoxalin-5-yl or indol-7-yl radical which is unsubstituted or substituted by one or more radicals R^{5'} or represents an NR^{6'} R^{7'} radical,
where the substituent(s) R^{5'} is/are each independently of one another selected from:
cyano, halogen, nitro, acetyl, hydroxy, carboxy, amino, tri-(C₁-C₆)-alkylsilyl, (C₃-C₈)-cycloalkyl, (C₃-C₈)-cycloalkyloxy, (C₃-C₈)-cycloalkyl- (C₃-C₈)-cycloalkyl, (C₁-C₆)-alkyl- (C₃-C₈)-cycloalkyl, halo- (C₃-C₈) -cycloalkyl, (C₁-C₆) -alkyl, (C₁-C₆) -haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl- (C₁-C₆)-alkyl, (C₁-C₆)-alkoxy- (C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl- (C₁-C₆)-alkoxy, (C₁-C₆)-alkoxy- (C₁-C₆)-alkoxy, (C₁-C₆)-alkylhydroxyimino, (C₁-C₆)-alkoxyimino, (C₁-C₆)-alkyl- (C₁-C₆)-alkoxyimino, (C₁-C₆)-haloalkyl- (C₁-C₆)-alkoxyimino, (C₁-C₆)-alkylthio, (C₁-C₆)-haloalkylthio, (C₁-C₆)-alkoxy- (C₁-C₆)-alkylthio, (C₁-C₆)-alkylthio- (C₁-C₆)-alkyl, (C₁-C₆)-alkylsulphinyl, (C₁-C₆)-haloalkylsulphinyl, (C₁-C₆)-alkoxy- (C₁-C₆)-alkylsulphinyl, (C₁-C₆)-alkylsulphinyl- (C₁-C₆)-alkyl, (C₁-C₆)-alkylsulphonyl, (C₁-C₆) -haloalkylsulphonyl, (C₁-C₆) -alkoxy- (C₁-C₆) -alkylsulphonyl, (C₁-C₆) -alkylsulphonyl- (C₁-C₆) -alkyl, (C₁-C₆)-alkylsulphonyloxy, (C₁-C₆) -alkylcarbonyl, (C₁-C₆)-alkylthiocarbonyl, (C₁-C₆)-haloalkylcarbonyl, (C₁-C₆) -alkylcarbonyloxy, (C₁-C₆) -alkoxycarbonyl, (C₁-C₆) -haloalkoxycarbonyl, aminocarbonyl, (C₁-C₆)-alkylaminocarbonyl, (C₁-C₆)-alkylaminothiocarbonyl, di-(C₁-C₆) -alkylaminocarbonyl, di-(C₁-C₆) -alkylaminothiocarbonyl, (C₂-C₆)-alkenylaminocarbonyl, di-(C₂-C₆)-alkenylaminocarbonyl, (C₃-C₈)-cycloalkyla-minocarbonyl, (C₁-C₆) -alkylsulphonylamino, (C₁-C₆)-alkylamino, di-(C₁-C₆)-alkylamino, aminosulphonyl, (C₁-C₆) -alkylaminosulphonyl, di-(C₁-C₆) -alkylaminosulphonyl, (C₁-C₆)-alkylsulphoximino, aminothiocarbonyl, (C₁-C₆) -alkylaminothiocarbonyl, di-(C₁-C₆) -alkylaminothiocarbonyl, (C₃-C₈)-cycloalkylamino, (C₁-C₆) -alkylcarbonylamino and (1-pyrazolyl)-(C₁-C₃)-alkyl and
R^{6'} and R^{7'} each independently of one another represent H, a (C₁-C₆) -alkyl, (C₁-C₆) -haloalkyl, (C₁-C₆) -alkoxy-(C₁-C₆) -alkyl radical or unsubstituted phenyl or halogen-, (C₁-C₆) -alkyl-, (C₁-C₆) -haloalkyl- or (C₁-C₆)-alkoxy-(C₁-C₆)-alkyl-substituted phenyl radical
or form a ring from the group pyrrolidine, morpholine, piperidine.

19. Compounds according to any of Claims 14 to 18 where
M' represents a radical selected from the radicals of the formulae (IIa) to (IIf) where
in case (IIa) R^{2'} represents H, methyl or ethyl or optionally halogen-substituted phenyl and R^{3'} represents H, methyl, ethyl, isopropyl or halogen,
in case (IIb) R^{2'} represents H, methyl or ethyl, in case (IIc) R^{2'} represents H or methyl and R^{3'} represents H, methyl or ethyl,
in case (IId) R^{1'} represents H or methyl and R^{3'} represents H or halogen,
in case (IIe) R^{2'} represents H, methyl, methoxy, halogen-substituted phenyl or halogen-substituted pyridyl and R^{3'} represents H,
in case (IIf) R^{3'} represents H, methyl or ethyl and
Q' represents a phenyl, naphth-1-yl, pyridyl, pyrimidinyl, thiophen-2-yl, benzothiophen-2-yl, benzothi-ophen-3-yl, isoquinolin-1-yl, benzodioxol-4-yl or pyrazol-5-yl radical which is unsubstituted or substituted by one or more radicals R^{4'}, but in cases (IIa), (IId) in the case R^{1'} = H and R^{3'} = methyl does not represent 3-methoxyphenyl, in case (IId) does not represent 3-pyridyl, in case (IIe) does not represent 2-pyrimidinyl, nor unsubstituted phenyl, nor 3,4-dichlorophenyl and
where the substituents R^{4'} are as defined in Claim 17 or 18.

20. Compounds according to any of Claims 14 to 19 where
Q' represents a phenyl, naphth-1-yl, pyridyl, pyrimidinyl, thiophen-2-yl, benzothiophen-2-yl, benzothi-ophen-3-yl, isoquinolin-1-yl, benzodioxol-4-yl or pyrazol-5-yl radical which is unsubstituted or substituted by one or more radicals R^{4'}, but in cases (IIa), (IId) in the case R^{1'} = H and R^{3'} = methyl does not represent 3-methoxyphenyl, in case (IId) does not represent 3-pyridyl, in case (IIe) does not represent 2-pyrimidinyl, nor unsubstituted phenyl, nor 3,4-dichlorophenyl and where the substituent(s) R^{4'} is/are independently of one another selected from:
cyano, halogen, nitro, acetyl, (C₃-C₆)-cycloalkyl, (C₃-C₆)-cycloalkyloxy, (C₃-C₆)-cycloalkyl- (C₃-C₆)-cycloalkyl, (C₁-C₆)-alkyl- (C₃-C₈)-cycloalkyl, halo- (C₃-C₆)-cycloalkyl, (C₁-C₆) -alkyl, (C₁-C₆) -haloalkyl, (C₁-C₆)-cyanoalkyl, (C₁-C₆)-hydroxyalkyl, (C₁-C₆)-alkoxycarbonyl- (C₁-C₆)-alkyl, (C₁-C₆)-alkoxy- (C₁-C₆)-alkyl, (C₂-C₆)-alkenyl, (C₂-C₆)-haloalkenyl, (C₂-C₆)-alkynyl, (C₁-C₆) -alkoxy, (C₁-C₆) -haloalkoxy, (C₁-C₆)-alkylthio, (C₁-C₆)-haloalkylthio, (C₁-C₆)-alkylthio-(C₁-C₆)-alkyl, (C₁-C₆)-alkylsulphinyl, (C₁-C₆)-haloalkylsulphinyl, (C₁-C₆)-alkylsulphonyl, (C₁-C₆)-haloalkylsulphonyl, (C₁-C₆)-alkylsulphonyloxy, (C₁-C₆)-alkylcarbonyl, (C₁-C₆)-alkylamino, di-(C₁-C₆)-alkylamino, (C₁-C₆)-alkylcarbonylamino, phenyl, halophenyl, phenoxy or halophenoxy;
D' represents a (C₁-C₆)-alkyl, phenyl, pyridine, pyrimidine, pyrazole, triazole, thiazole, oxazole, thiadiazole, oxadiazole, thiophene, pyrrole, furan, tetrahydrofuran or dioxane radical which is unsubstituted or substituted by one or more radicals R^{5'},
where the substituent(s) R^{5'} is/are independently of one another selected from:
cyano, halogen, nitro, acetyl, hydroxy, carboxy, amino, (C₃-C₆)-cycloalkyl, (C₃-C₆)-cycloalkyloxy, (C₃-C₆)-cycloalkyl- (C₃-C₆)-cycloalkyl, (C₁-C₆)-alkyl- (C₃-C₈)-cycloalkyl, halo-(C₃-C₆)-cycloalkyl, (C₁-C₆)-alkyl, (C₁-C₆) -haloalkyl, (C₁-C₆) -cyanoalkyl, (C₁-C₆)-hydroxyalkyl, (C₁-C₆)-alkoxycarbonyl- (C₁-C₆)-alkyl, (C₁-C₆)-alkoxy- (C₁-C₆)-alkyl, (C₂-C₆)-alkenyl, (C₂-C₆) -haloalkenyl, (C₂-C₆)-alkynyl, (C₁-C₆) -alkoxy, (C₁-C₆)-haloalkoxy, (C₁-C₆)-alkylthio, (C₁-C₆)-haloalkylthio, (C₁-C₆)-alkylthio- (C₁-C₆)-alkyl, (C₁-C₆)-alkylsulphinyl, (C₁-C₆) -haloalkylsulphinyl, (C₁-C₆) -alkylsulphonyl, (C₁-C₆) -haloalkylsulphonyl, (C₁-C₆) -al-kylsulphonyloxy, (C₁-C₆) -alkylcarbonyl, (C₁-C₆) -alkylamino, di-(C₁-C₆) -alkylamino, (C₁-C₆) -alkylcarbonylamino or (1-pyrazolyl) (C₁-C₃)-alkyl and
R^{6'} and R^{7'} each independently of one another represent H, a (C₁-C₆) -alkyl, phenyl, alkoxyphenyl or halophenyl or form a ring from the group pyrrolidine, morpholine, piperidine.

21. Compounds according to any of Claims 14 to 20 where
M' represents a radical selected from the radicals of the formulae (Va-Vz): 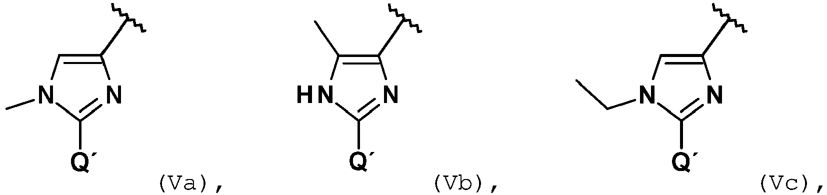 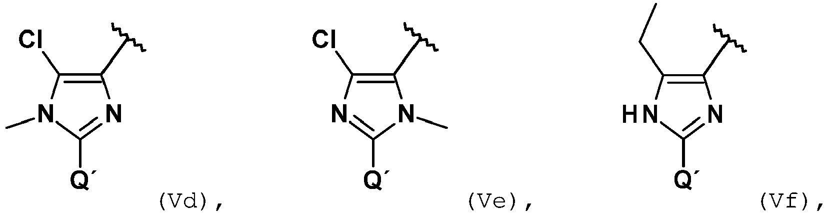 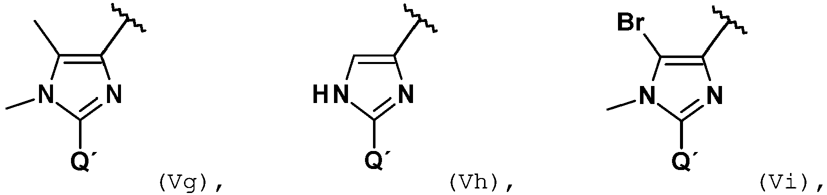 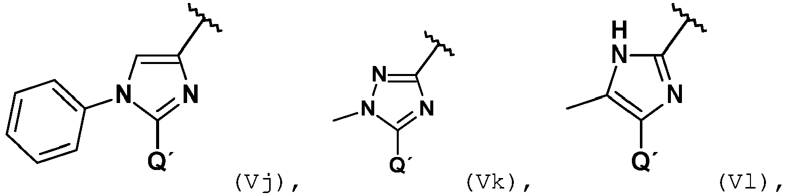 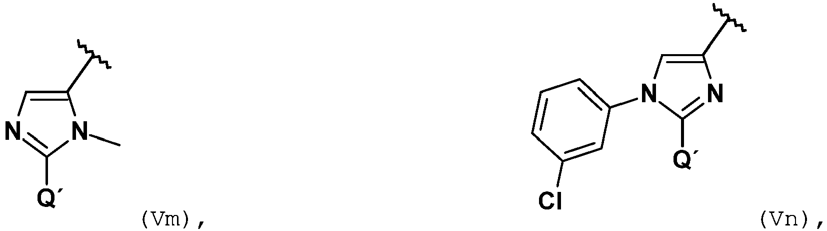 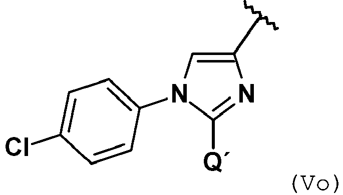 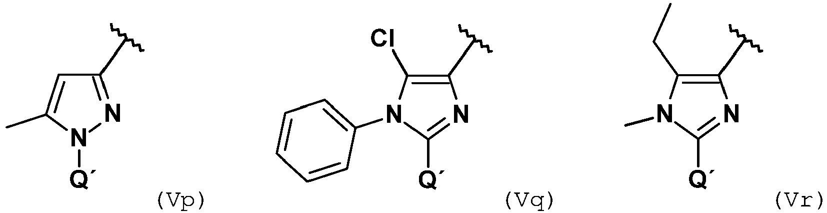 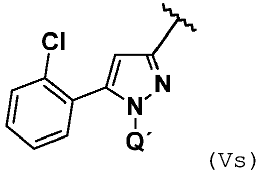 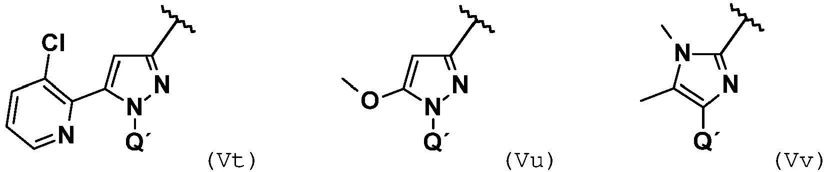 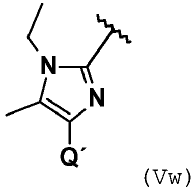 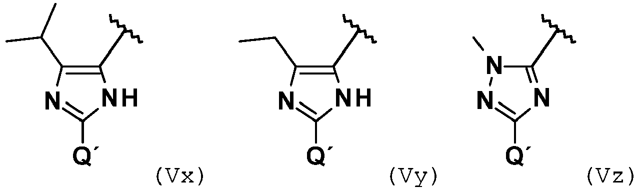 Q' represents a radical selected from the radicals of the formulae (VIa-VIz and VIa1-VIa30) : 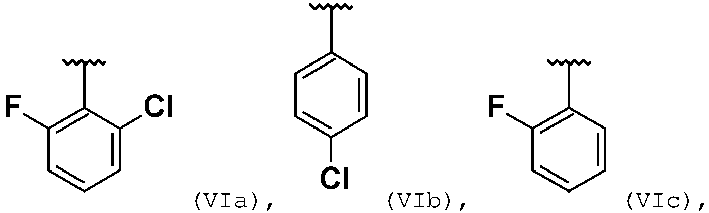 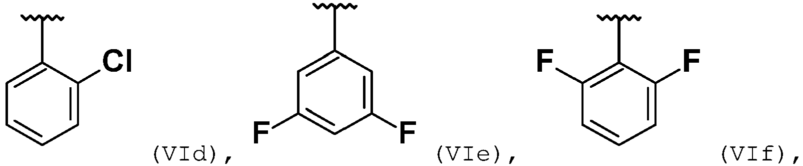 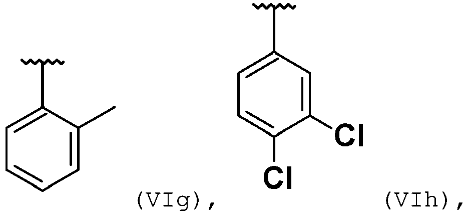 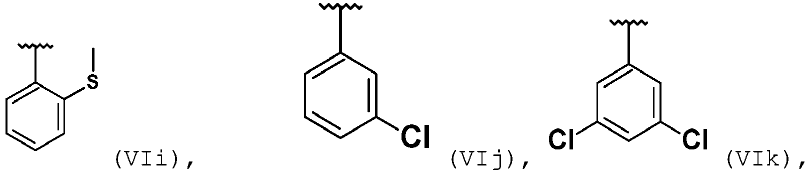 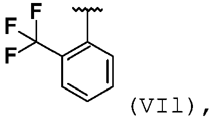 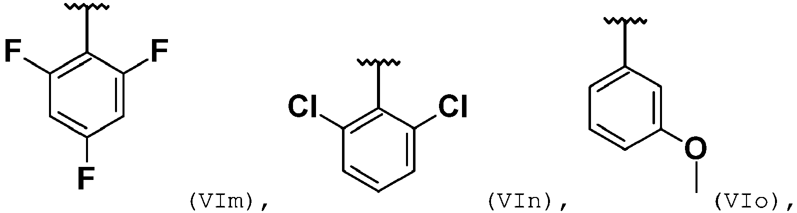 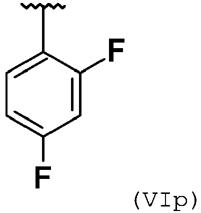 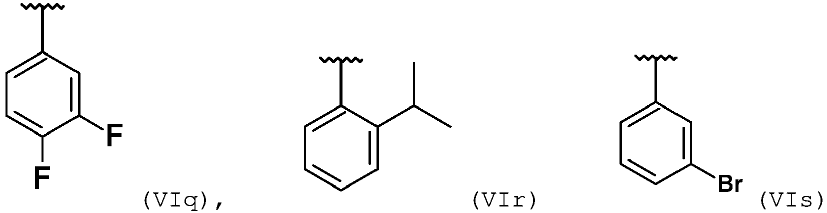 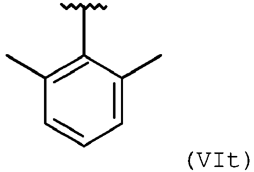 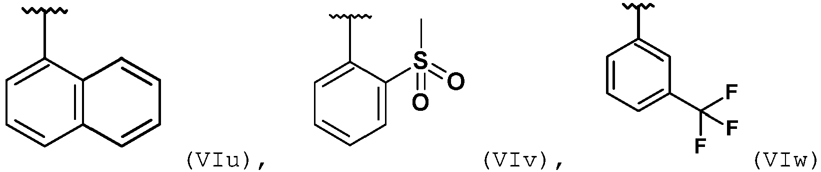 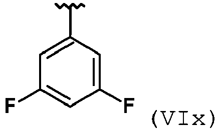 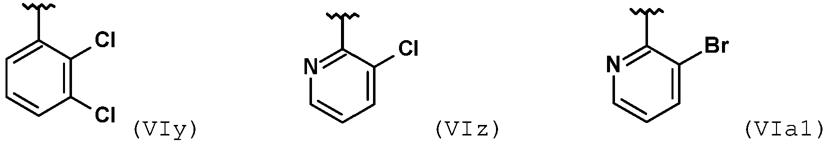 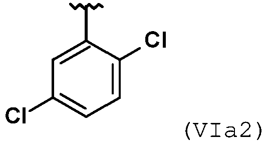 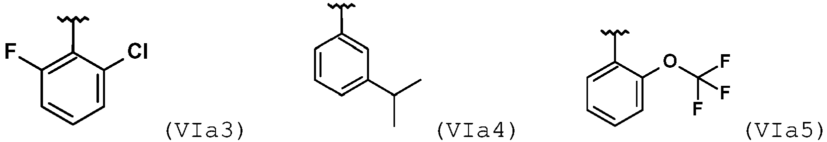 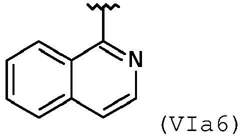 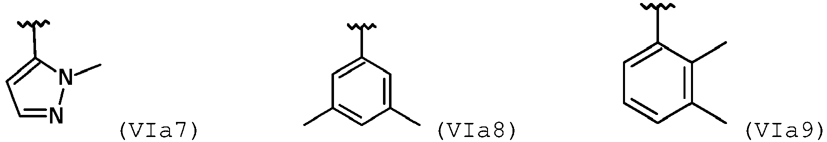 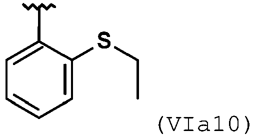 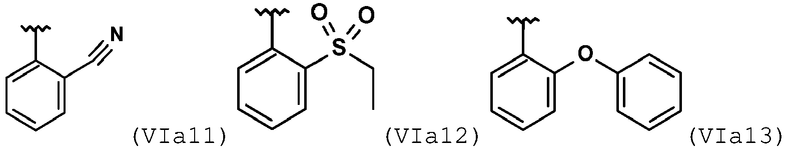 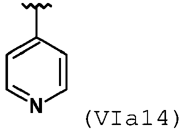 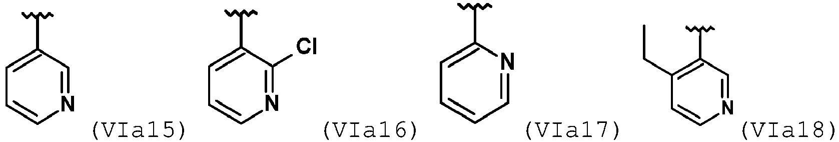 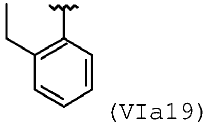 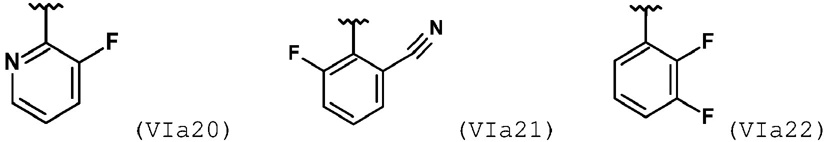 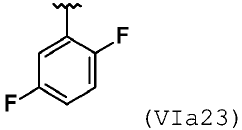 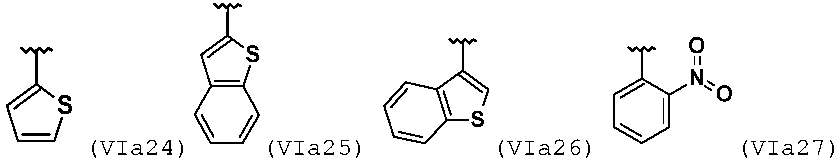 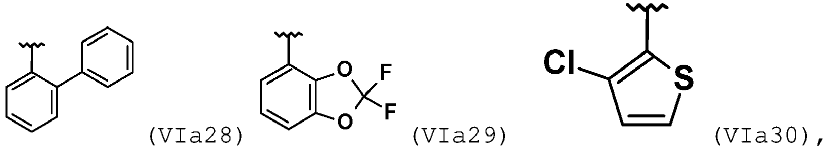 D' represents a radical selected from the radicals of the formulae (VII1-VII192)

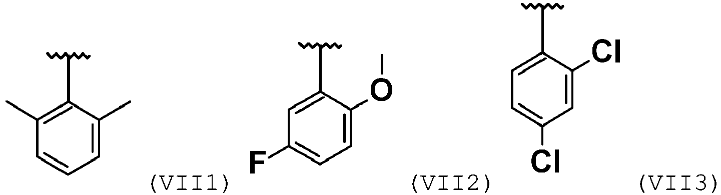

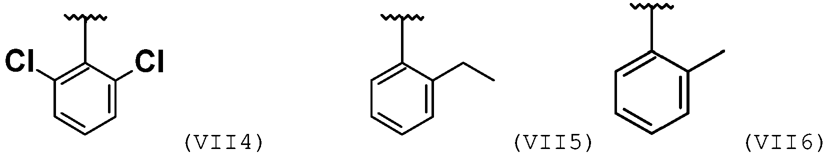

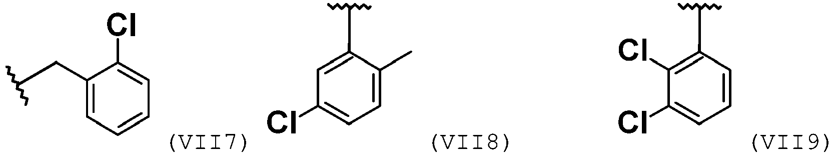

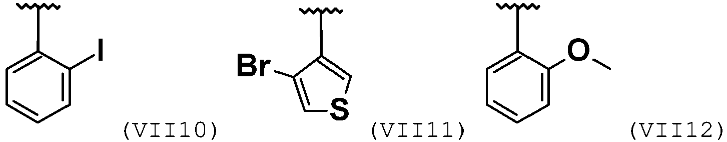

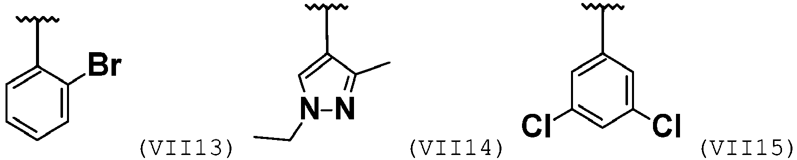

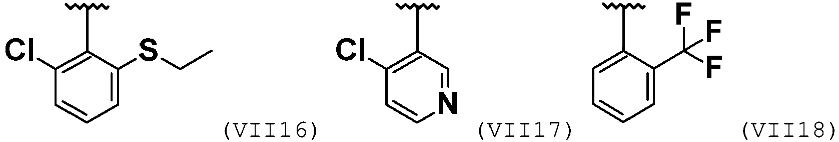

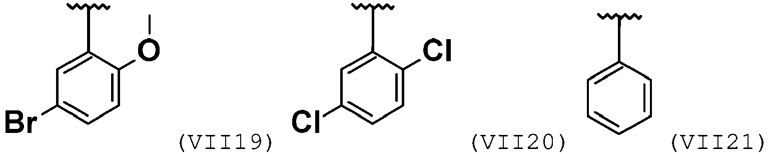

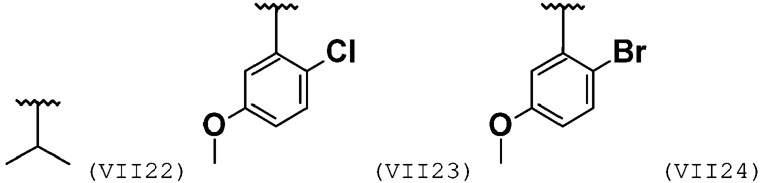

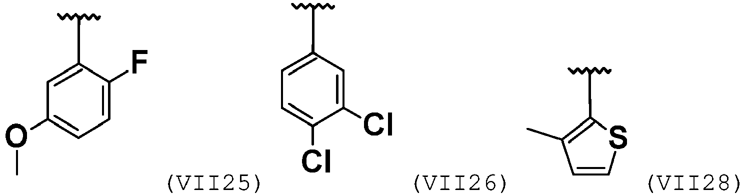

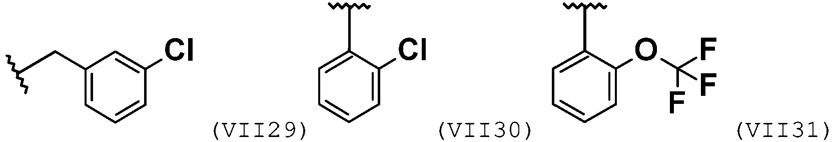

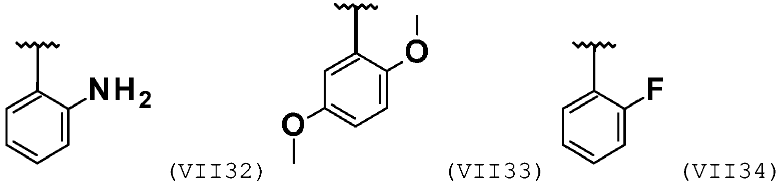

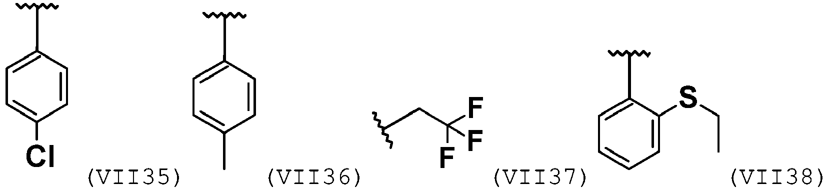

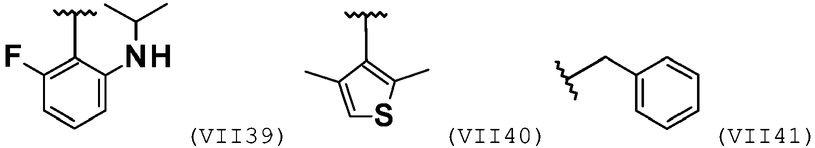

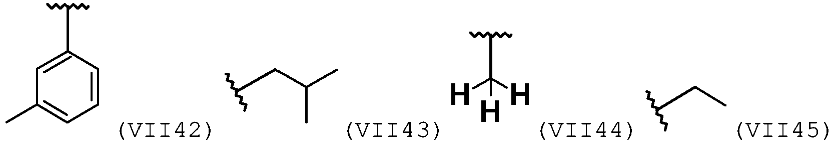

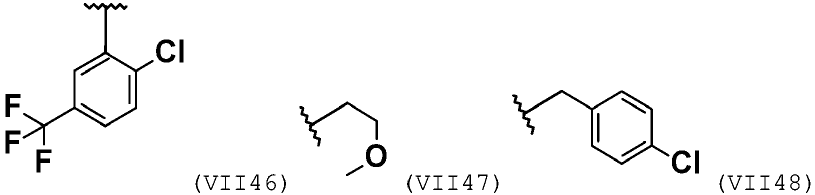

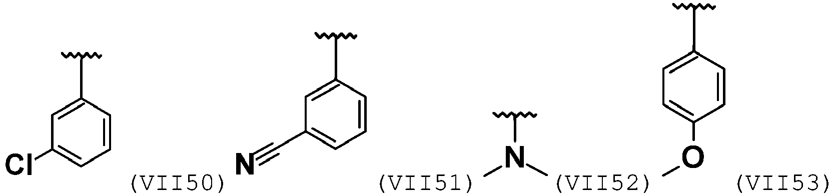

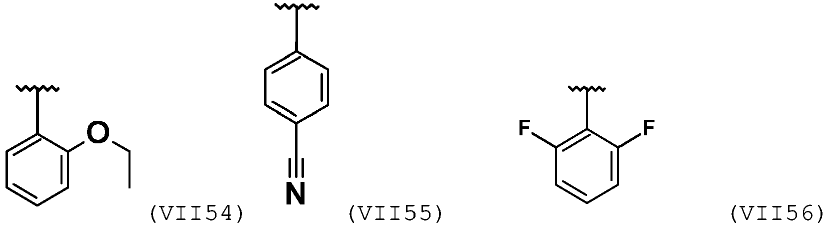

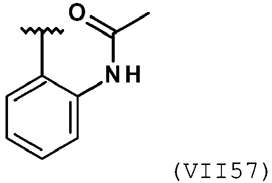

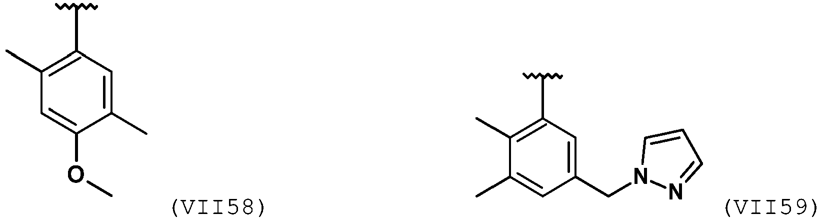

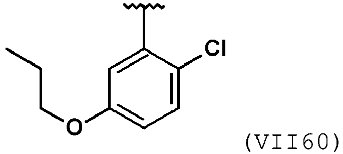

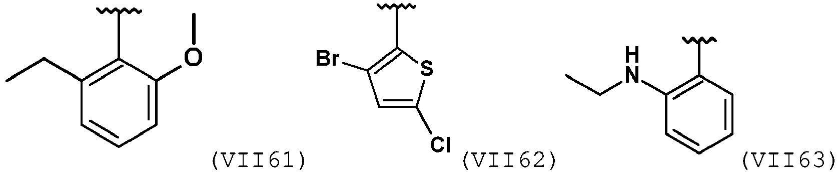

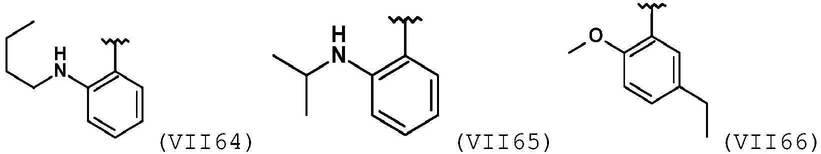

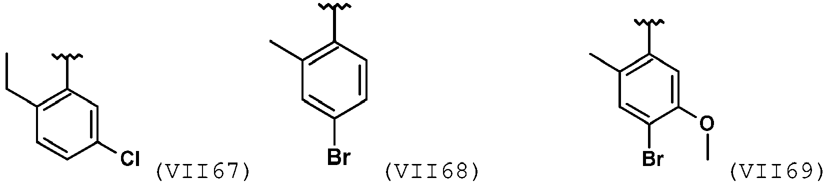

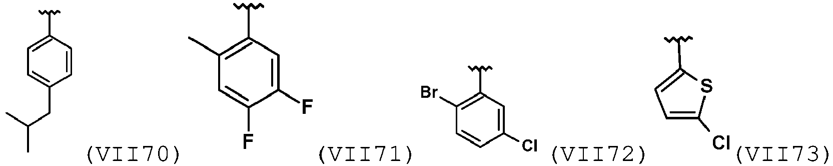

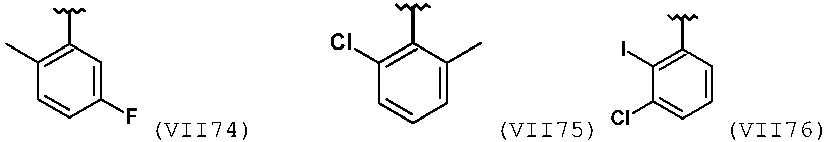

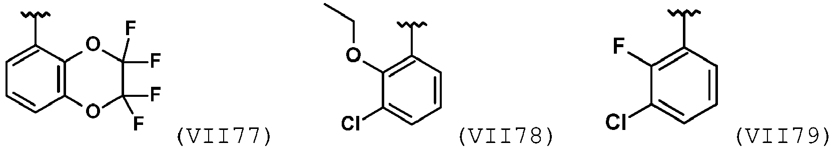

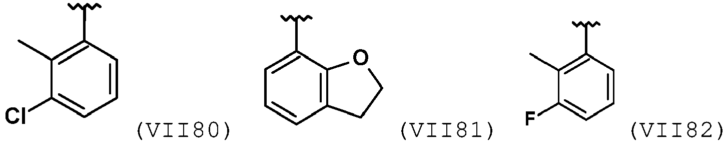

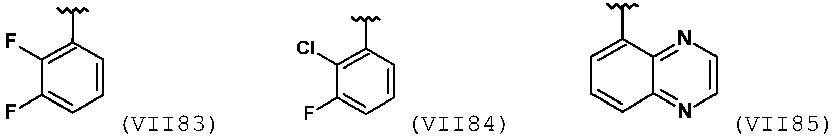

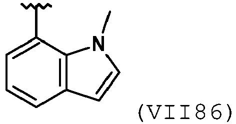

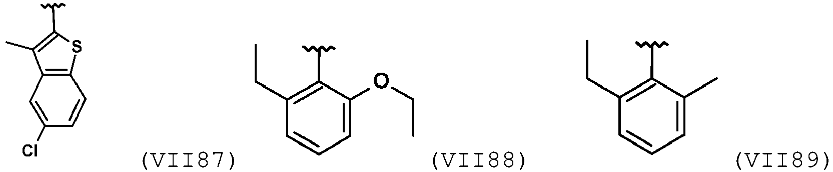

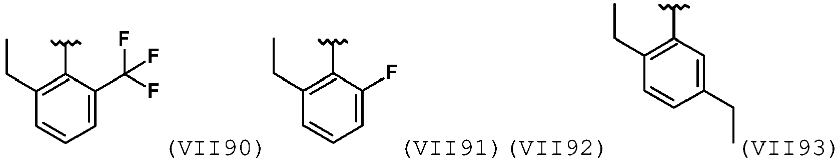

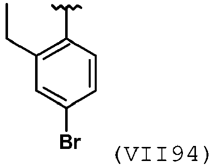

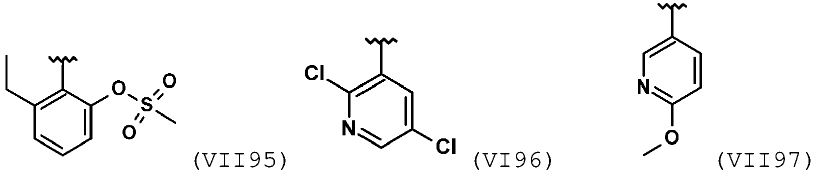

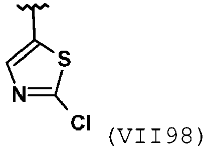

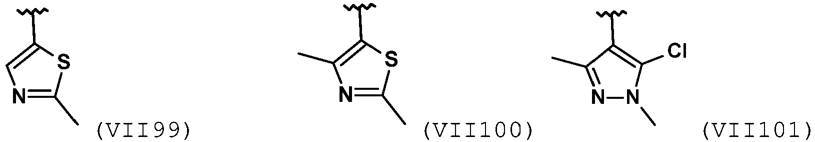

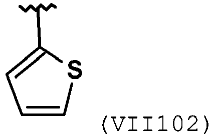

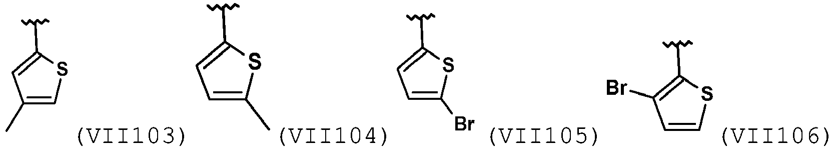

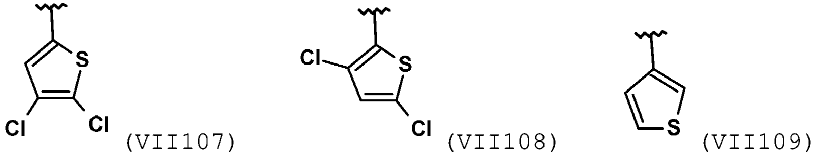

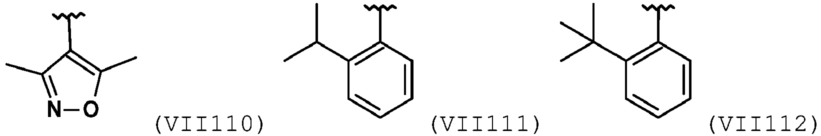

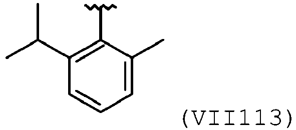

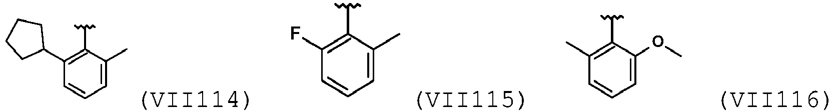

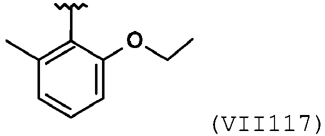

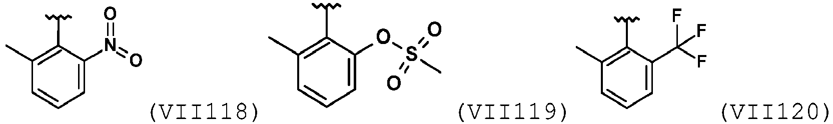

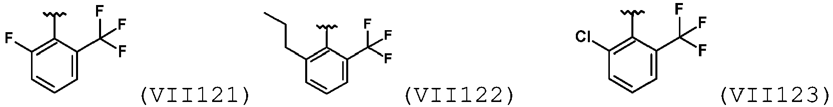

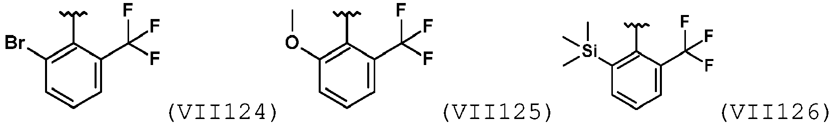

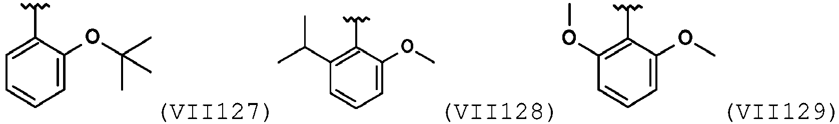

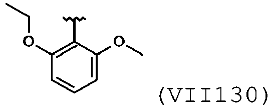

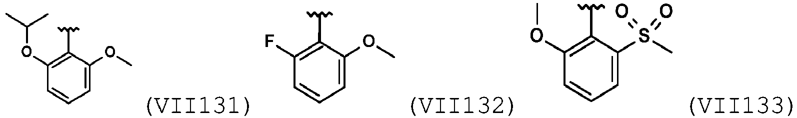

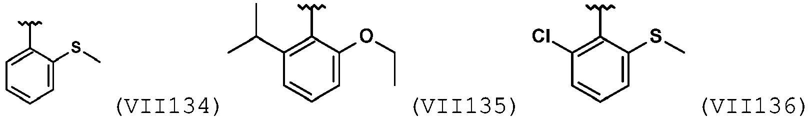

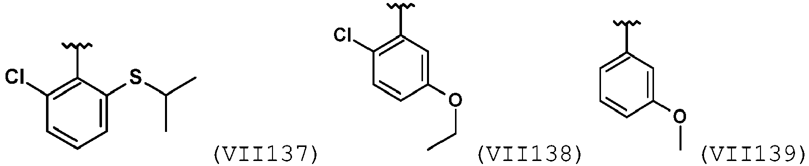

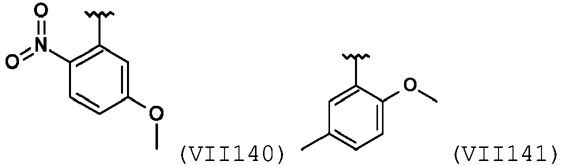

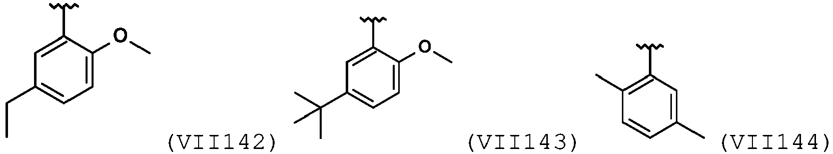

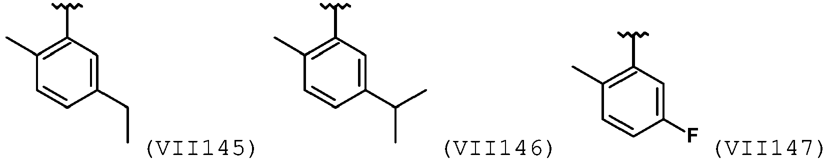

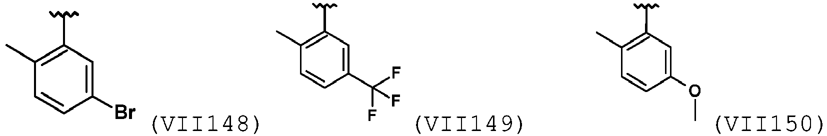

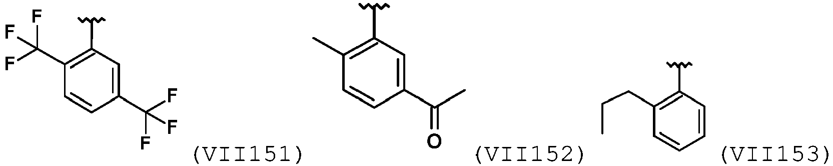

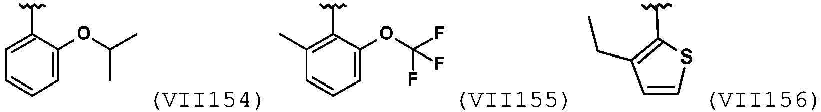

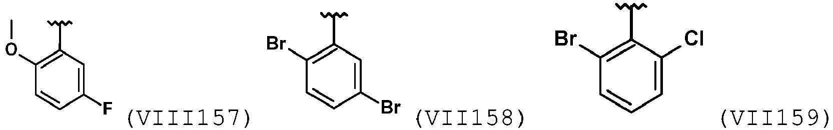

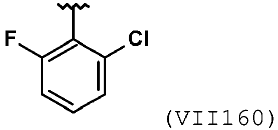

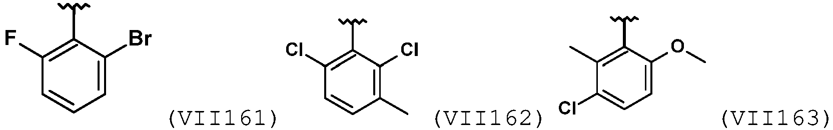

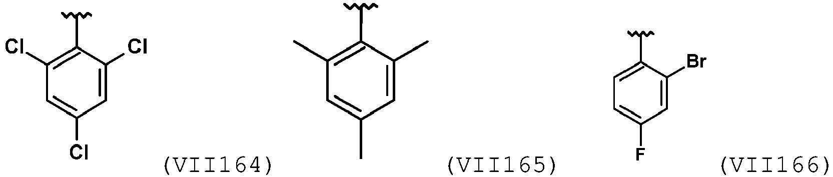

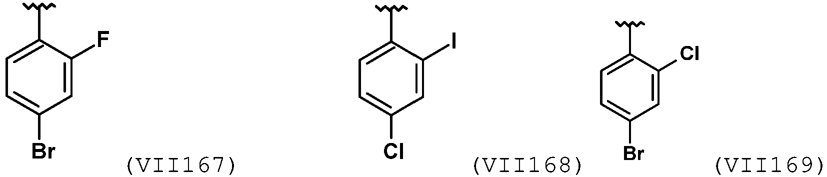

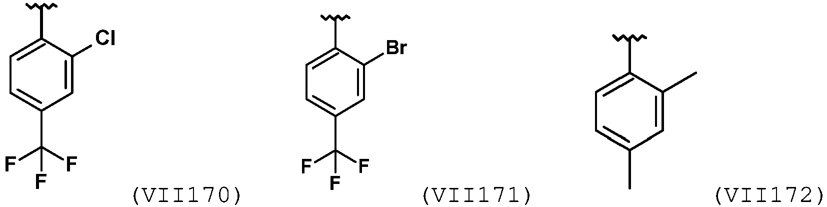

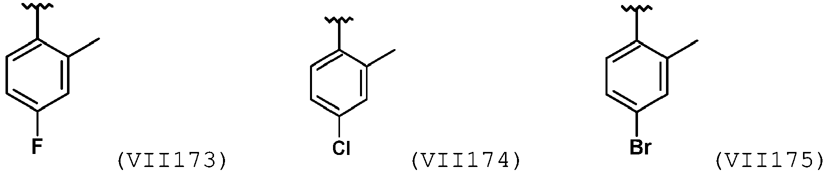

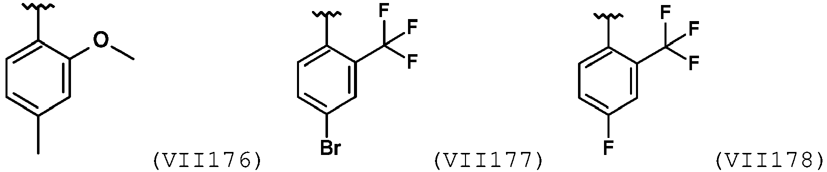

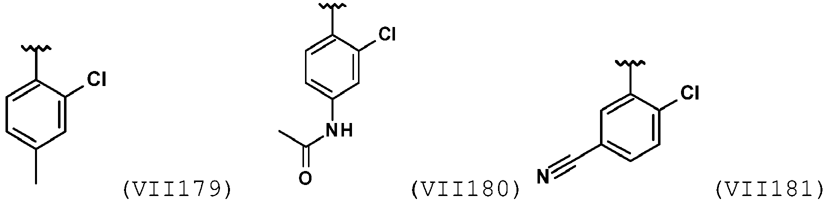

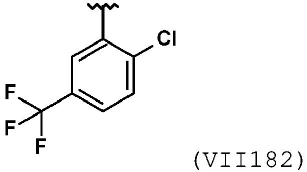

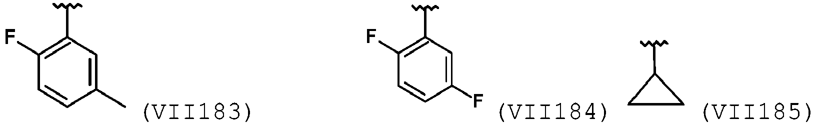

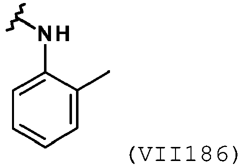

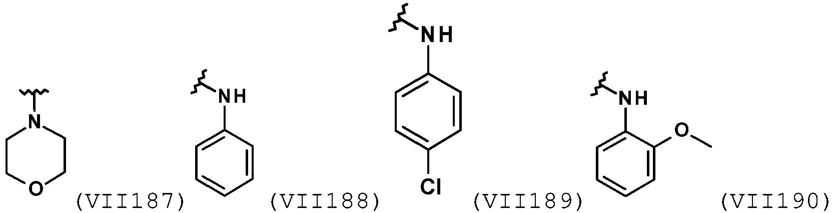

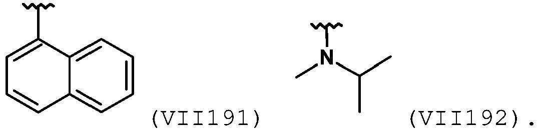

22. Intermediates of the formulae XIa - XIq

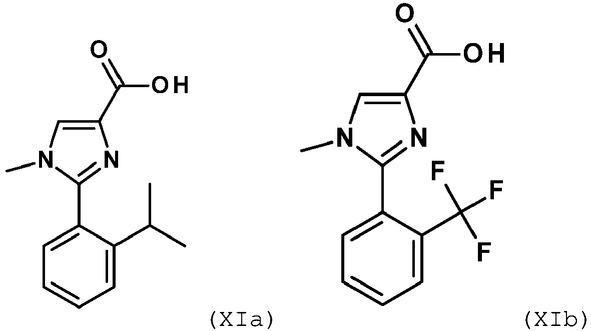

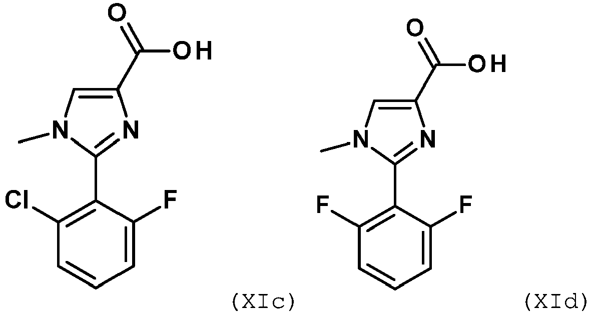

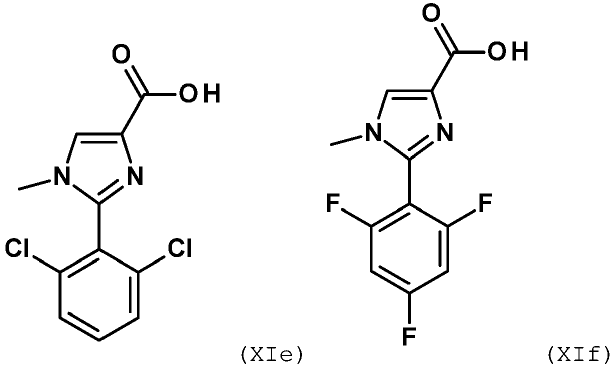

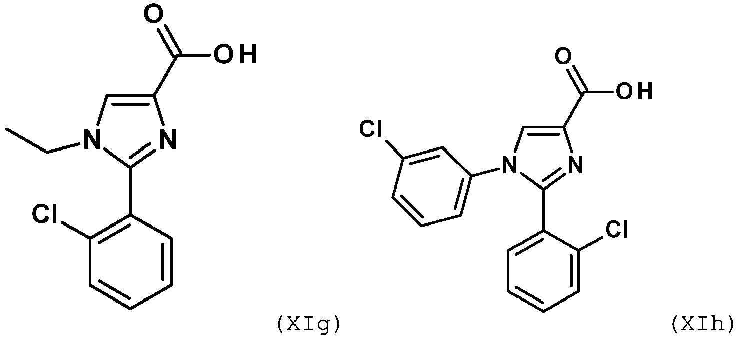

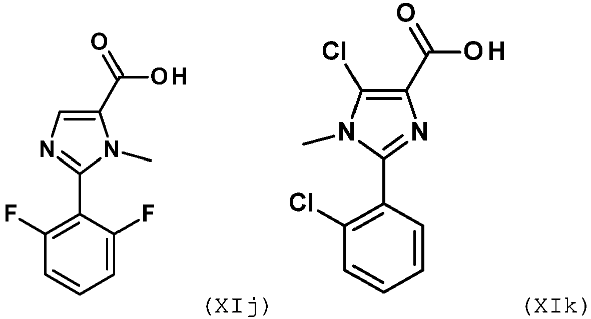

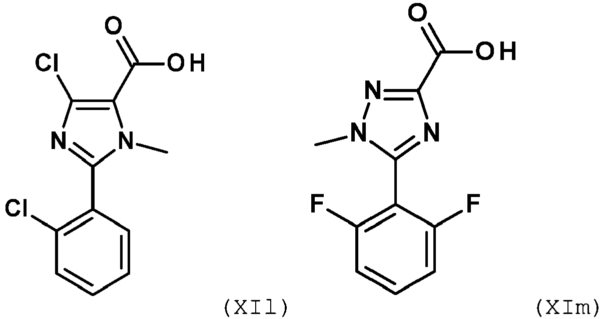

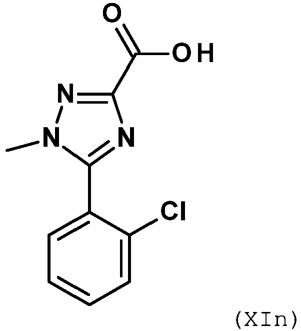

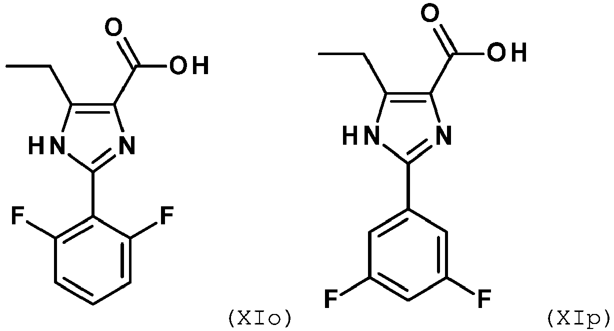

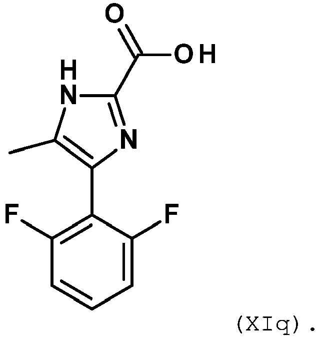

## Revendications

1. Utilisation d'un composé de formule (I) 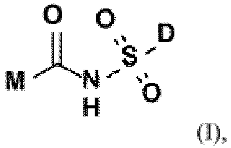 dans laquelle
M représente un radical choisi parmi les formules (IIa à IIf) : 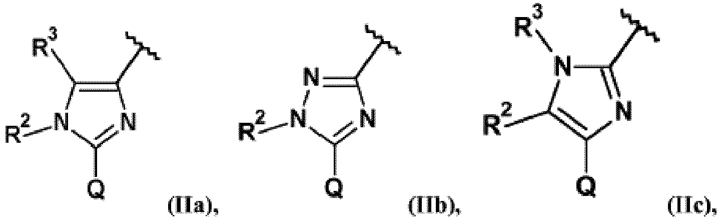 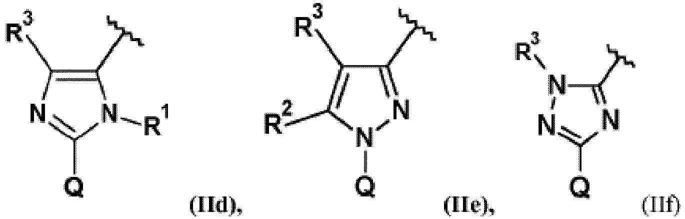 R¹, R², R³ à chaque fois indépendamment les uns des autres étant H ou un radical alkyle, cycloalkyle, alcényle, cycloalcényle, cyclohétéroalkyle, aryle ou hétéroaryle substitué ou non substitué, R² dans le cas de (IIc) et (IIe) pouvant de plus être un radical halogène ou un radical alcoxy, et R³ pouvant de plus être un radical halogène dans le cas de (IIa), (IId) et (IIe) ;
Q étant un radical aryle ou hétéroaryle substitué ou non substitué, mais n'étant pas 2-pyrimidinyle dans le cas de (IIe) ;
D étant un radical alkyle, hétéroalkyle, cycloalkyle éventuellement partiellement insaturé, cyclohétéroalkyle, hétéroaryle, aryle ou phénylalkyle substitué ou non substitué ou un radical azoté substitué ou non substitué,
pour la lutte contre des parasites animaux, le traitement chirurgical, thérapeutique ou diagnostique du corps humain ou animal étant exclu.

2. Utilisation selon la revendication 1, dans laquelle
M représente un radical choisi parmi les formules (IIa à IIf), R¹' R², R³ à chaque fois indépendamment les uns des autres étant H ou un radical (C₁-C₄)-alkyle, (C₃-C₆)-cycloalkyle, (C₂-C₄)-alcényle, pyridinyle ou phényle substitué ou non substitué, R² dans le cas de (IIc) et (IIe) pouvant de plus être un radical halogène ou un radical alcoxy, et R³ pouvant de plus être un radical halogène dans le cas de (IIa), (IId) et (IIe) ;
Q est un radical phényle, naphtyle ou hétéroaryle substitué ou non substitué, qui peut contenir 1 à 3 hétéroatome(s) de la série oxygène, soufre, azote ; mais n'est pas 2-pyrimidinyle dans le cas de (IIe) ;
D est un radical alkyle, hétéroalkyle, cycloalkyle, hétéroaryle, aryle ou phényl-(C₁-C₈)-alkyle substitué ou non substitué ou un radical azoté substitué ou non substitué,

3. Utilisation selon la revendication 1 ou 2, dans laquelle
M représente un radical choisi parmi les formules (IIa à IIf), R¹' R², R³ étant définis tel que dans la revendication 1 ou 2 et
Q est un radical phényle, naphtyle, pyridinyle, pyrimidinyle, thiophène, benzothiophène, isoquinoléine, benzodioxole ou pyrazole non substitué ou substitué par un ou plusieurs radicaux R⁴, mais n'est pas 2-pyrimidinyle dans le cas de (IIe),
le ou les substituant (s) R⁴ à chaque fois indépendamment les uns des autres étant choisi(s) parmi :
cyano, halogène, nitro, acétyle, hydroxy, carboxy, amino, SCN, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl- (C₃-C₈)-cycloalkyle, halogéno-(C₃-C₈)-cycloalkyle, (C₁-C₆) -alkyle, (C₁-C₆)-halogénoalkyle, (C₁-C₆)-cyanoalkyle, (C₁-C₆)-hydroxyalkyle, hydroxycarbonyl- (C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio- (C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆) -alkylsulfinyl- (C₁-C₆) - alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆) -alkylsulfonyl-(C₁-C₆) - alkyle, (C₁-C₆)-alkylsulfonyloxy, (C₁-C₆)-alkylcarbonyle, (C₁-C₆) -alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, NHCO-(C₁-C₆)-alkyle, ((C₁-C₆)-alkylcarbonylamino) et/ou aryle, aryloxy ou hétaryle éventuellement substitué une ou plusieurs fois, de manière identique ou différente, éventuellement au moins un groupe carbonyle pouvant être contenu (dans le cas d'hétaryle) et les substituants pouvant être à chaque fois : cyano, carboxy, halogène, nitro, acétyle, hydroxy, amino, SCN, tri-(C₁-C₆)-alkylsilyle, (C₃-C₈)-cycloalkyle, (C₃-C₈)-cycloalkyl-(C₃-C₈)-cycloalkyle, (C₁-C₆)-alkyl- (C₃-C₈)-cycloalkyle, halogéno-(C₃-C₈)-cycloalkyle, (C₁-C₆)-alkyle, (C₁-C₆) -halogénoalkyle, (C₁-C₆)-cyanoalkyle, (C₁-C₆)-hydroxyalkyle, hydroxycarbonyl- (C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆) -cyanoalcoxy, (C₁-C₆)-alcoxycarbonyl-(C₁-C₆) -alcoxy, (C₁-C₆) -alcoxy-(C₁-C₆)-alcoxy, (C₁-C₆) -alkylhydroxyimino, (C₁-C₆)-alcoxyimino, (C₁-C₆) -alkyl-(C₁-C₆) -alcoxyimino, (C₁-C₆)-halogénoalkyl-(C₁-C₆) -alcoxyimino, (C₁-C₆)-alkylthio, (C₁-C₆) -halogénoalkylthio, (C₁-C₆)-alcoxy-(C₁-C₆) -alkylthio, (C₁-C₆) -alkylthio- (C₁-C₆)-alkyle, (C₁-C₆) -alkylsulfinyle, (C₁-C₆)-halogénoalkylsulfinyle, (C₁-C₆) -alcoxy-(C₁-C₆) - alkylsulfinyle, (C₁-C₆) -alkylsulfinyl-(C₁-C₆) - alkyle, (C₁-C₆) -alkylsulfonyle, (C₁-C₆)-halogénoalkylsulfonyle, (C₁-C₆) -alcoxy-(C₁-C₆) - alkylsulfonyle, (C₁-C₆) -alkylsulfonyl-(C₁-C₆) - alkyle, (C₁-C₆) -alkylsulfonyloxy, (C₁-C₆)-alkylcarbonyle, (C₁-C₆)-halogénoalkylcarbonyle, (C₁-C₆) -alkylcarbonyloxy, (C₁-C₆) -alcoxycarbonyle, (C₁-C₆)-halogénoalcoxycarbonyle, aminocarbonyle, (C₁-C₆)-alkylaminocarbonyle, di-(C₁-C₆)-alkylaminocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino et D est un radical (C₁-C₆)-alkyle, phényle, phényl-(C₁-C₂)-alkyle ou hétéroaryle non substitué ou substitué par un ou plusieurs radicaux R⁵, qui peut contenir 1 à 3 hétéroatome(s) de la série oxygène, soufre, azote ou est un radical NR⁶R⁷, le ou les substituant (s) R⁵ à chaque fois indépendamment les uns des autres étant choisi(s) parmi :
cyano, halogène, nitro, acétyle, hydroxy, carboxy, amino, SCN, tri-(C₁-C₆) -alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆) -alkyl- (C₃-C₈)-cycloalkyle, halogéno-(C₃-C₈)-cycloalkyle, (C₁-C₆) -alkyle, (C₁-C₆) -halogénoalkyle, (C₁-C₆)-cyanoalkyle, (C₁-C₆)-hydroxyalkyle, hydroxycarbonyl- (C₁-C₆) -alcoxy, (C₁-C₆)-alcoxycarbonyl-(C₁-C₆) -alkyle, (C₁-C₆) -alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆) -alcoxy, (C₁-C₆)-halogénoalcoxy, (C₁-C₆) -cyanoalcoxy, (C₁-C₆)-alcoxycarbonyl-(C₁-C₆) -alcoxy, (C₁-C₆) -alcoxy-(C₁-C₆)-alcoxy, (C₁-C₆) -alkylhydroxyimino, (C₁-C₆)-alcoxyimino, (C₁-C₆) -alkyl- (C₁-C₆) -alcoxyimino, (C₁-C₆)-halogénoalkyl-(C₁-C₆) -alcoxyimino, (C₁-C₆)-alkylthio, (C₁-C₆) -halogénoalkylthio, (C₁-C₆)-alcoxy-(C₁-C₆) -alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆) - alkylsulfinyle, (C₁-C₆) -alkylsulfinyl-(C₁-C₆) - alkyle, (C₁-C₆) -alkylsulfonyle, (C₁-C₆)-halogénoalkylsulfonyle, (C₁-C₆) -alcoxy-(C₁-C₆) - alkylsulfonyle, (C₁-C₆) -alkylsulfonyl-(C₁-C₆) - alkyle, (C₁-C₆) -alkylsulfonyloxy, (C₁-C₆)-alkylcarbonyle, (C₁-C₆) -alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆) -alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino) et (1-pyrazolyl) - (C₁-C₃)-alkyle
et/ou
aryle ou hétaryle substitué une ou plusieurs fois, de manière identique ou différente, éventuellement au moins un groupe carbonyle pouvant être contenu (dans le cas d'hétaryle) et les substituants pouvant être à chaque fois : cyano, carboxyle, halogène, nitro, acétyle, hydroxy, amino, SCN, tri-(C₁-C₆) -alkylsilyle, (C₃-C₈)-cycloalkyle, (C₃-C₈)-cycloalkyl-(C₃-C₈)-cycloalkyle, (C₁-C₆) -alkyl-(C₃-C₈)-cycloalkyle, halogéno-(C₃-C₈)-cycloalkyle, (C₁-C₆) -alkyle, (C₁-C₆)-halogénoalkyle, (C₁-C₆)-cyanoalkyle, (C₁-C₆)-hydroxyalkyle, hydroxycarbonyl- (C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆) -halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)- alkylsulfinyle, (C₁-C₆)-alkylsulfinyl-(C₁-C₆)- alkyle, (C₁-C₆)-alkylsulfonyle, (C₁-C₆)-halogénoalkylsulfonyle, (C₁-C₆)-alcoxy-(C₁-C₆)- alkylsulfonyle, (C₁-C₆)-alkylsulfonyl-(C₁-C₆)- alkyle, (C₁-C₆)-alkylsulfonyloxy, (C₁-C₆)-alkylcarbonyle, (C₁-C₆)-halogénoalkylcarbonyle, (C₁-C₆) -alkylcarbonyloxy, (C₁-C₆) -alcoxycarbonyle, (C₁-C₆)-halogénoalcoxycarbonyle, aminocarbonyle, (C₁-C₆)-alkylaminocarbonyle, di-(C₁-C₆)- alkylaminocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino et (C₁-C₆)-alkylcarbonylamino
et R⁶ et R⁷ à chaque fois indépendamment l'un de l'autre étant H, (C₁-C₆)-alkyle ou un radical phényle substitué ou non substitué ou R⁶ et R⁷ pouvant former ensemble un cycle non substitué ou substitué à 4 à 8 chaînons, saturé ou éventuellement entièrement ou partiellement insaturé qui peut être interrompu par 1 à 3 hétéroatome(s) de la série oxygène, soufre, azote et qui peut être doté une ou plusieurs fois par une substitution correspondant à la définition de R5.

4. Utilisation selon l'une quelconque des revendications 1 à 3, dans laquelle
M représente un radical choisi parmi les formules (IIa à IIf), R¹, R², R³ étant définis tel que dans la revendication 1 ou 2 et
Q est un radical phényle, naphtyle, pyridinyle, pyrimidinyle, thiophène, benzothiophène, isoquinoléine, benzodioxole ou pyrazole non substitué ou substitué par un ou plusieurs radicaux R⁴, mais n'est pas 2-pyrimidinyle dans le cas de (IIe),
le ou les substituant (s) R⁴ à chaque fois indépendamment les uns des autres étant choisi(s) parmi :
cyano, halogène, nitro, acétyle, hydroxy, carboxy, amino, tri-(C₁-C₆)-alkylsilyle, (C₃-C₈)-cycloalkyle, (C₃-C₈)-cycloalkyl- (C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)- alkylsulfinyle, (C₁-C₆)-alkylsulfinyl-(C₁-C₆)- alkyle, (C₁-C₆)-alkylsulfonyle, (C₁-C₆)-halogénoalkylsulfonyle, (C₁-C₆)-alcoxy-(C₁-C₆)- alkylsulfonyle, (C₁-C₆)-alkylsulfonyl-(C₁-C₆)- alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino, phényle, halogénophényle, phénoxy ou halogénophénoxy et D est un radical (C₁-C₆) -alkyle, phényle, phényl-(C₁-C₂) -alkyle ou hétéroaryle non substitué ou substitué par un ou plusieurs radicaux R⁵, qui peut contenir 1 à 3 hétéroatome(s) de la série oxygène, soufre, azote ou est un radical NR⁶R⁷, le ou les substituant (s) R⁵ à chaque fois indépendamment les uns des autres étant choisi(s) parmi :
cyano, halogène, nitro, acétyle, hydroxy, carboxy, amino, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)- alkylsulfinyle, (C₁-C₆)-alkylsulfinyl-(C₁-C₆)- alkyle, (C₁-C₆)-alkylsulfonyle, (C₁-C₆)-halogénoalkylsulfonyle, (C₁-C₆)-alcoxy-(C₁-C₆)- alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino ou (1-pyrazolyl)-(C₁-C₃)-alkyle et
R⁶ et R⁷ étant à chaque fois indépendamment l'un de l'autre H, un radical (C₁-C₆)-alkyle, (C₁-C₆)-halogénoalkyle, (C₁-C₆)-alcoxy-(C₁-C₆)-alkyle ou un radical phényle non substitué ou un radical phényle substitué par halogène, (C₁-C₆)-alkyle, (C₁-C₆) -alcoxy, (C₁-C₆) -halogénoalkyle, (C₁-C₆)-alcoxy-(C₁-C₆)-alkyle
ou R⁶ et R⁷ ensemble pouvant former un cycle non substitué ou substitué à 5 à 6 chaînons, saturé ou éventuellement entièrement ou partiellement insaturé qui peut être interrompu par 1 à 3 hétéroatome(s) de la série oxygène, soufre, azote et qui peut être doté une ou plusieurs fois par une substitution correspondant à la définition de R⁵.

5. Utilisation selon l'une quelconque des revendications 1 à 4, dans laquelle
M représente un radical choisi parmi les formules (IIa à IIf),
dans le cas de (IIa), R², R³ à chaque fois indépendamment l'un de l'autre étant H ou un radical (C₁-C₄) -alkyle, (C₃-C₆) -cycloalkyle ou phényle substitué ou non substitué, R³ pouvant de plus être un radical halogène,
dans le cas de (IIb), R² étant H ou un radical (C₁-C₄) -alkyle ou (C₃-C₆) -cycloalkyle substitué ou non substitué,
dans le cas de (IIc), R², R³ à chaque fois indépendamment l'un de l'autre étant H ou un radical (C₁-C₄) -alkyle, (C₃-C₆) -cycloalkyle ou phényle substitué ou non substitué et R² pouvant de plus être un radical halogène ou (C₁-C₄)-alcoxy, dans le cas de (IId), R¹, R³ à chaque fois indépendamment l'un de l'autre étant H ou un radical (C₁-C₄) -alkyle ou (C₃-C₆) -cycloalkyle substitué ou non substitué, R³ pouvant de plus être un radical halogène,
dans le cas de (IIe), R², R³ à chaque fois indépendamment l'un de l'autre étant H, halogène ou un radical (C₁-C₄) -alkyle, (C₃-C₆) -cycloalkyle, pyridinyle ou phényle substitué ou non substitué et R² pouvant de plus être un radical (C₁-C₄)-alcoxy et
dans le cas de (IIf) R³ étant H ou un radical (C₁-C₄)-alkyle ou (C₃-C₆) -cycloalkyle substitué ou non substitué
et Q étant défini tel que dans l'une quelconque des revendications 1 à 4 et
D étant un radical (C₁-C₆)-alkyle, phényle, napht-2-yle, pyridine, pyrimidine, pyrazole, triazole, thiazole, oxazole, thiadiazole, oxadiazole, thiophène, pyrrole, furanne, tétrahydrofuranne, dioxanne, isoxazole, benzyle, 2,3-dihydro-1,4-benzodioxin-5-yle, 2,3-dihydro-1-benzofuran-7-yle, quinoxalin-5-yle ou indol-7-yle non substitué ou substitué par un ou plusieurs radicaux R⁵, ou un radical NR⁶R⁷,
le ou les substituant (s) R⁵ à chaque fois indépendamment les uns des autres étant choisi(s) parmi :
cyano, halogène, nitro, acétyle, hydroxy, carboxy, amino, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl- (C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino ou (1-pyrazolyl)-(C₁-C₃)-alkyle et
R⁶ et R⁷ étant à chaque fois indépendamment l'un de l'autre H, un radical (C₁-C₆)-alkyle, (C₁-C₆)-halogénoalkyle, (C₁-C₆)-alcoxy-(C₁-C₆)-alkyle ou un radical phényle non substitué ou un radical phényle substitué par halogène, (C₁-C₆)-alkyle, (C₁-C₆)-alcoxy, (C₁-C₆)-halogénoalkyle, (C₁-C₆)-alcoxy-(C₁-C₆)-alkyle,
ou formant un cycle de la série pyrrolidine, morpholine, pipéridine.

6. Utilisation selon l'une quelconque des revendications 1 à 5, dans laquelle
M est un radical choisi parmi les radicaux des formules (IIa à IIf),
dans le cas de (IIa), R² étant H, méthyle ou éthyle ou phényle éventuellement substitué par halogène, et R³ étant H, méthyle, éthyle, iso-propyle ou halogène,
dans le cas de (IIb), R² étant H, méthyle ou éthyle,
dans le cas de (IIc), R² étant H ou méthyle et R³ étant H, méthyle ou éthyle,
dans le cas de (IId), R¹ étant H ou méthyle et R³ étant H ou halogène,
dans le cas de (IIe) R² étant H, méthyle, méthoxy, phényle substitué par halogène ou pyridinyle substitué par halogène et R³ étant H,
dans le cas de (IIf), R³ étant H, méthyle ou éthyle et Q étant défini tel que dans l'une quelconque des revendications 1 à 4.

7. Utilisation selon l'une quelconque des revendications 1 à 6, dans laquelle
M est choisi parmi une des formules (IIa à IIf) ou (IVa) à (IVf), R¹ à R³ étant définis tel que dans l'une quelconque des revendications 1, 2, 5 ou 6 et
Q est un radical phényle, napht-1-yle, pyridinyle, pyrimidinyle, thiophén-2-yle, benzothiophén-2-yle, benzothiophén-3-yle, isoquinoléin-1-yle, benzodioxol-4-yle ou pyrazol-5-yle non substitué ou substitué par un ou plusieurs radicaux R⁴, mais n'est pas 2-pyrimidinyle dans le cas de (IVe),
le ou les substituant(s) R⁴ étant indépendamment les uns des autres :
cyano, halogène, nitro, acétyle, (C₃-C₆)-cycloalkyle, (C₃-C₆) -cycloalkyloxy, (C₃-C₆)-cycloalkyl-(C₃-C₆)-cycloalkyle, (C₁-C₆)-alkyl- (C₃-C₈)-cycloalkyle, halogéno-(C₃-C₆)-cycloalkyle, (C₁-C₆)-alkyle, (C₁-C₆)-halogénoalkyle, (C₁-C₆)-cyanoalkyle, (C₁-C₆)-hydroxyalkyle, (C₁-C₆)-alcoxycarbonyl-(C₁-C₆)-alkyle, (C₁-C₆)-alcoxy- (C₁-C₆)-alkyle, (C₂-C₆)-alcényle, (C₂-C₆)-halogénoalcényle, (C₂-C₆)-alcynyle, (C₁-C₆)-alcoxy, (C₁-C₆)-halogénoalcoxy, (C₁-C₆)-alkylthio, (C₁-C₆)-halogénoalkylthio, (C₁-C₆)-alkylthio- (C₁-C₆)-alkyle, (C₁-C₆)-alkylsulfinyle, (C₁-C₆)-halogénoalkylsulfinyle, (C₁-C₆)-alkylsulfonyle, (C₁-C₆)-halogénoalkylsulfonyle, (C₁-C₆)-alkylsulfonyloxy, (C₁-C₆)-alkylcarbonyle, (C₁-C₆)-alkylamino, di-(C₁-C₆)-alkylamino, (C₁-C₆)-alkylcarbonylamino, phényle, halogénophényle, phénoxy ou halogénophénoxy
et
D étant un radical (C₁-C₆)-alkyle, phényle, napht-2-yle, pyridine, pyrimidine, pyrazole, triazole, thiazole, oxazole, thiadiazole, oxadiazole, thiophène, pyrrole, furanne, tétrahydrofuranne, dioxanne, isoxazole, benzyle, 2,3-dihydro-1,4-benzodioxin-5-yle, 2,3-dihydro-1-benzofuran-7-yle, quinoxalin-5-yle ou indol-7-yle non substitué ou substitué par un ou plusieurs radicaux R⁵ ou un radical NR⁶R⁷,
le ou les substituant(s) R⁵ à chaque fois indépendamment les uns des autres étant choisi(s) parmi :
cyano, halogène, nitro, acétyle, hydroxy, carboxy, amino, (C₃-C₆)-cycloalkyle, (C₃-C₆)-cycloalkyloxy, (C₃-C₆)-cycloalkyl-(C₃-C₆)-cycloalkyle, (C₁-C₆)-alkyl-(C₃-C₈)-cycloalkyle, halogéno-(C₃-C₆)-cycloalkyle, (C₁-C₆)-alkyle, (C₁-C₆)-halogénoalkyle, (C₁-C₆)-cyanoalkyle, (C₁-C₆)-hydroxyalkyle, (C₁-C₆)-alcoxycarbonyl-(C₁-C₆)-alkyle, (C₁-C₆)-alcoxy- (C₁-C₆)-alkyle, (C₂-C₆)-alcényle, (C₂-C₆)-halogénoalcényle, (C₂-C₆)-alcynyle, (C₁-C₆)-alcoxy, (C₁-C₆)-halogénoalcoxy, (C₁-C₆)-alkylthio, (C₁-C₆)-halogénoalkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alkylsulfonyle, (C₁-C₆)-halogénoalkylsulfonyle, (C₁-C₆)-alkylsulfonyloxy, (C₁-C₆)-alkylcarbonyle, (C₁-C₆)-alkylamino, di-(C₁-C₆)-alkylamino, (C₁-C₆)-alkylcarbonylamino ou (1-pyrazolyl)-(C₁-C₃)-alkyle et
R⁶ et R⁷ étant à chaque fois indépendamment l'un de l'autre H, un radical (C₁-C₆)-alkyle, phényle, alcoxyphényle ou halogénophényle ou formant un cycle de la série pyrrolidine, morpholine, pipéridine.

8. Utilisation selon l'une quelconque des revendications 1 à 7, dans laquelle
M est un radical choisi parmi les radicaux des formules (Va à Vz) : 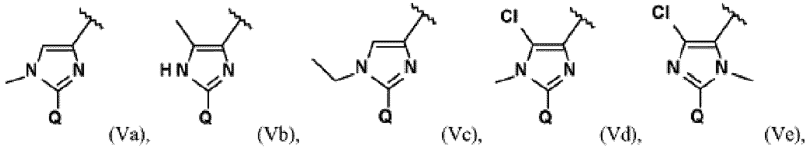 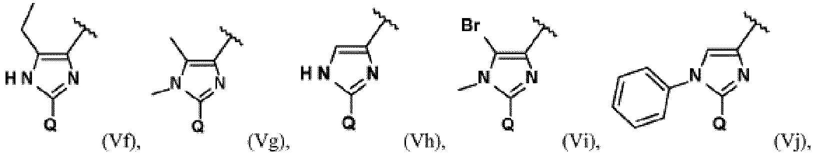 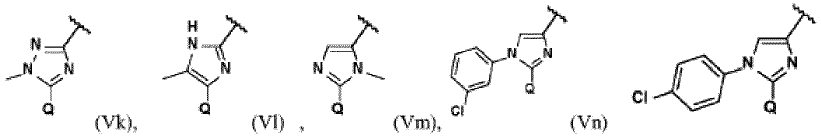 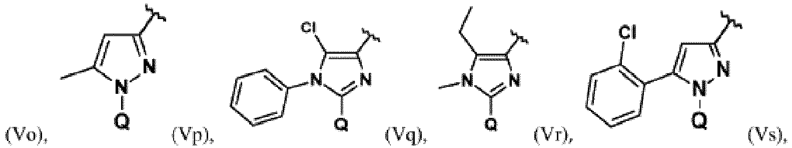 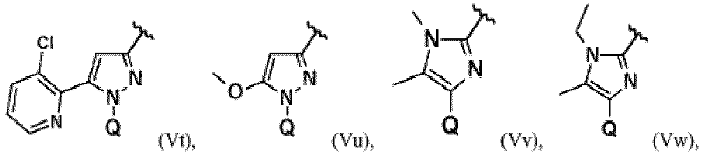 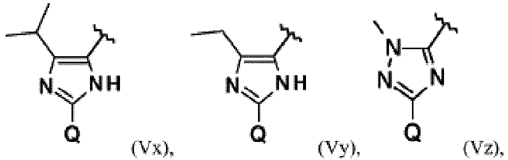 Q est un radical choisi parmi les radicaux des formules (VIa à VIz et VIa1 à VIa30) : 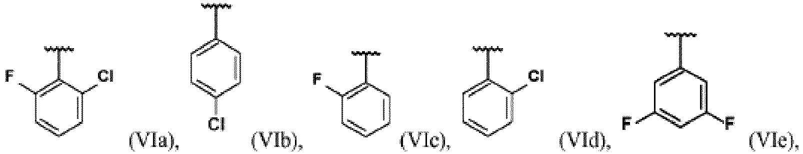 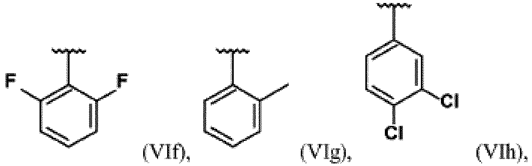 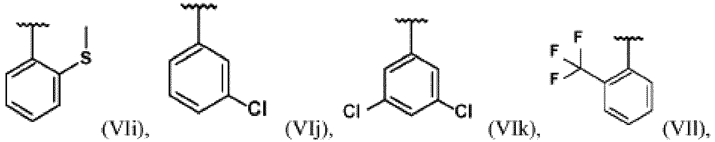 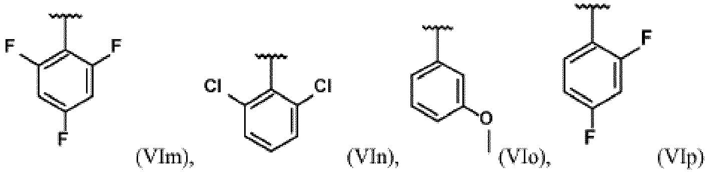 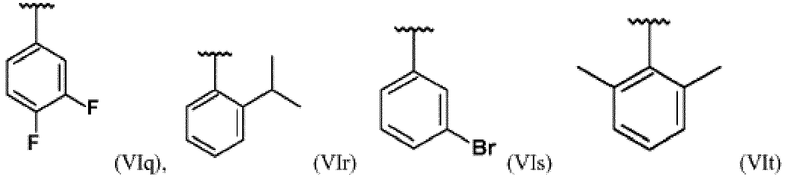 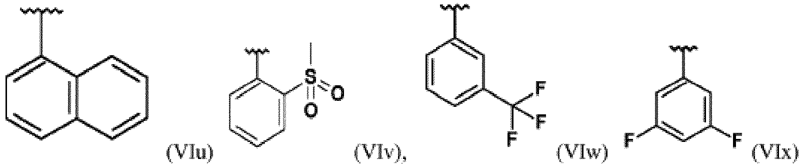 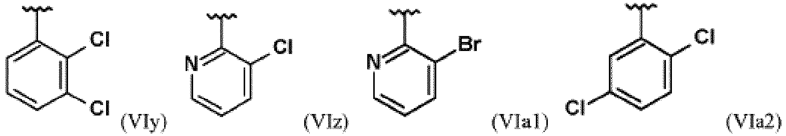 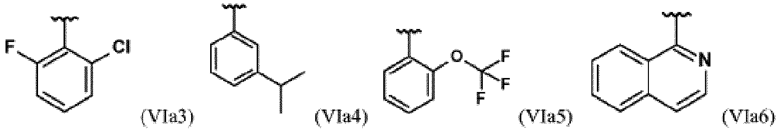 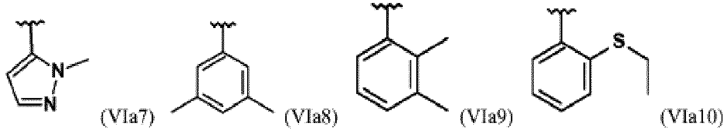 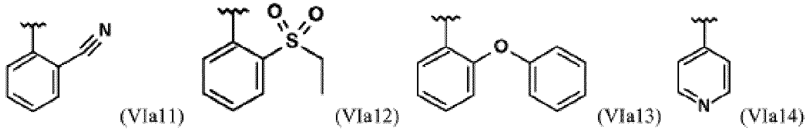 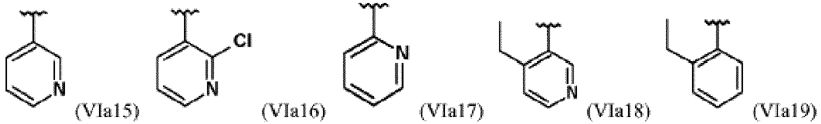 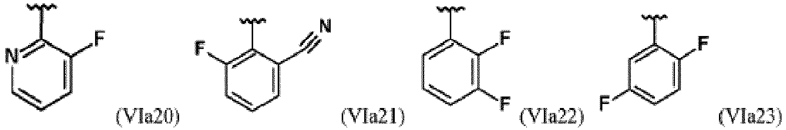 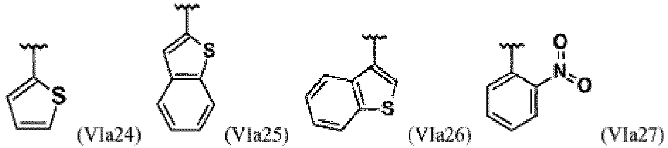 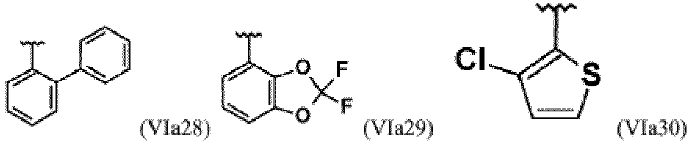 et
D est un radical choisi parmi les radicaux des formules (VII1 à VII192)

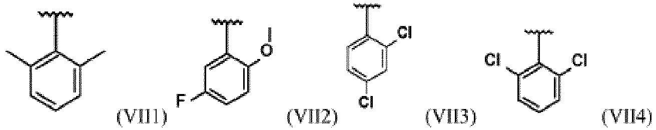

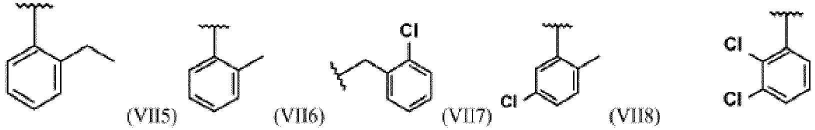

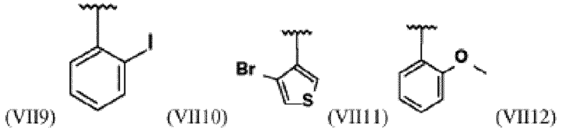

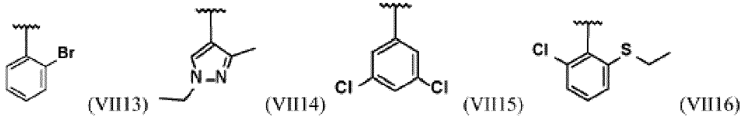

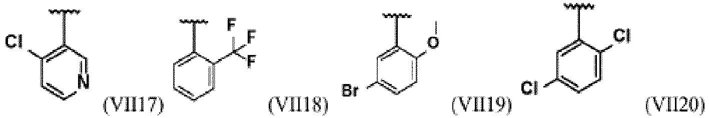

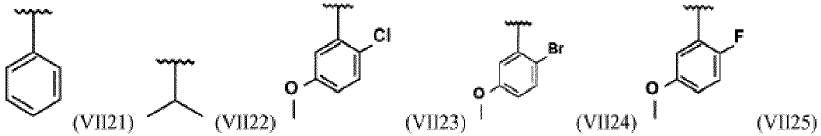

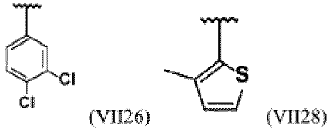

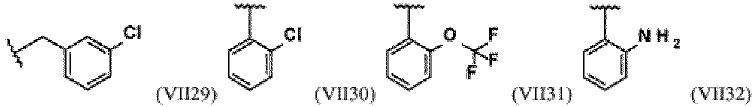

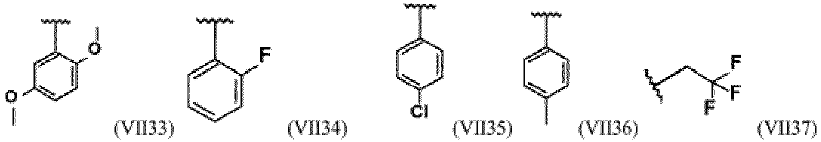

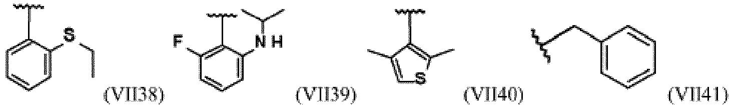

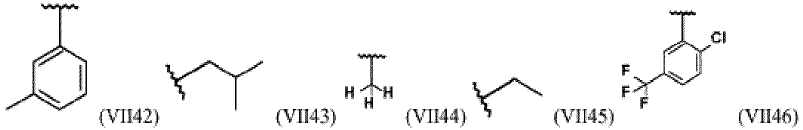

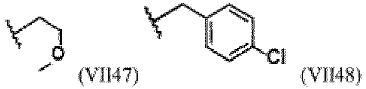

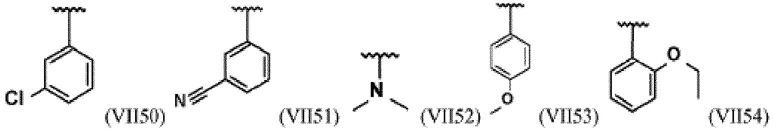

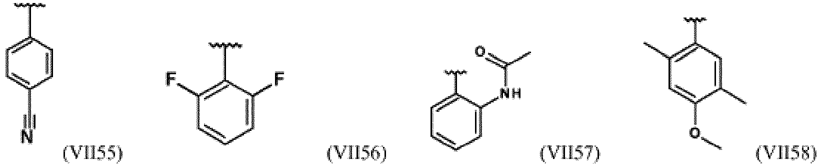

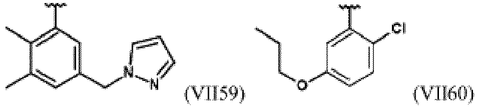

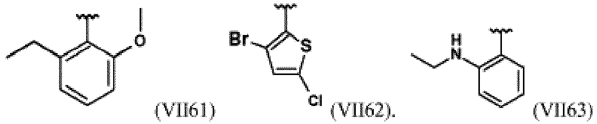

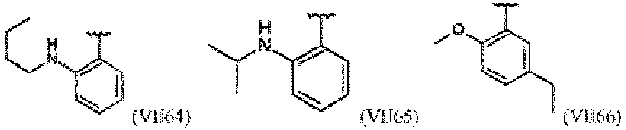

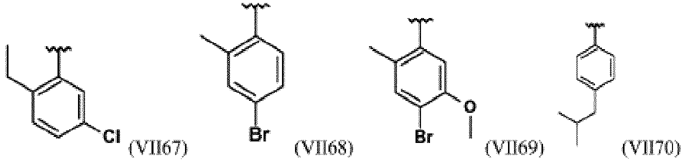

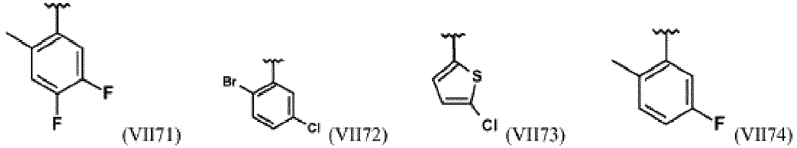

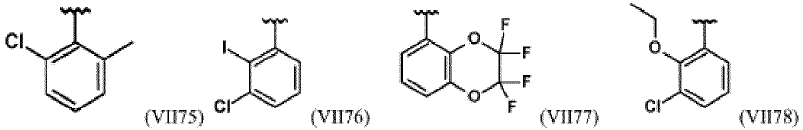

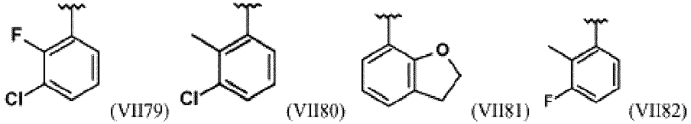

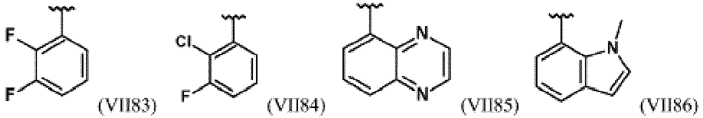

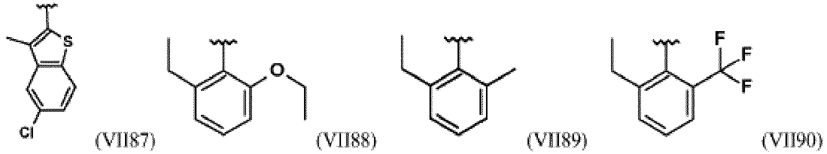

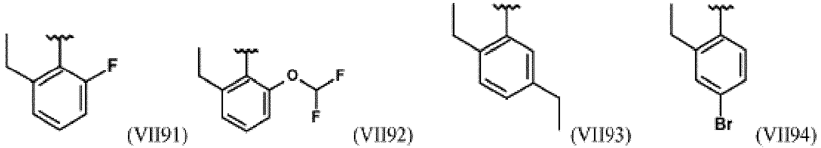

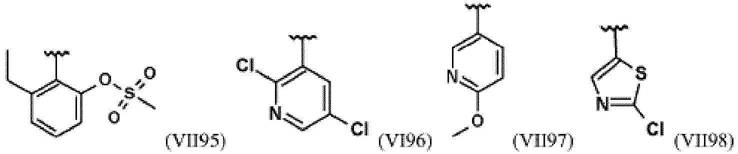

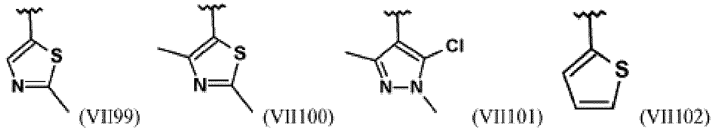

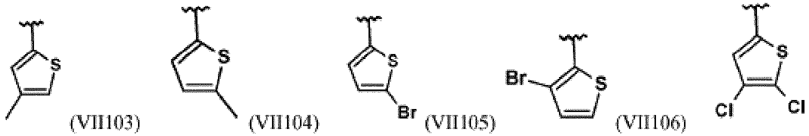

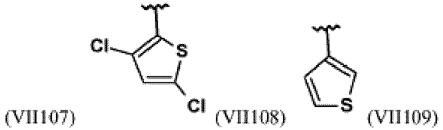

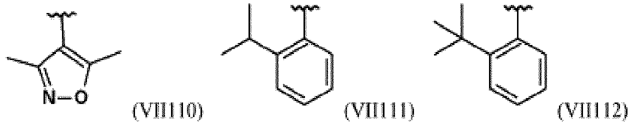

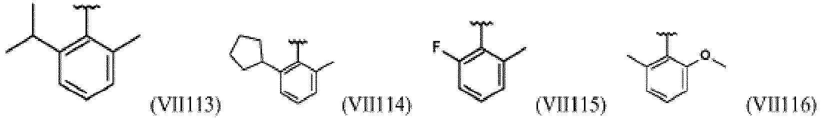

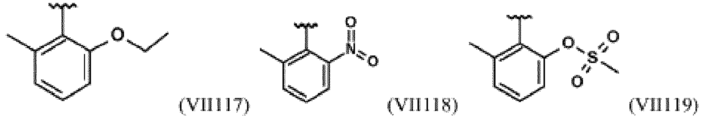

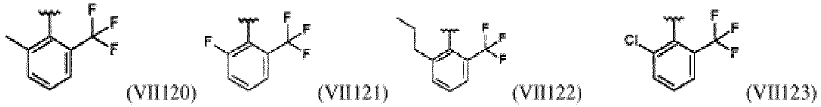

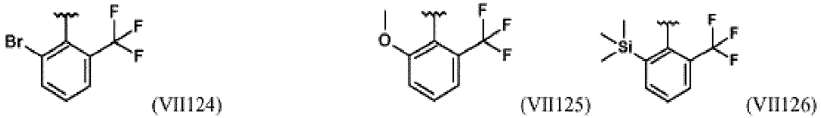

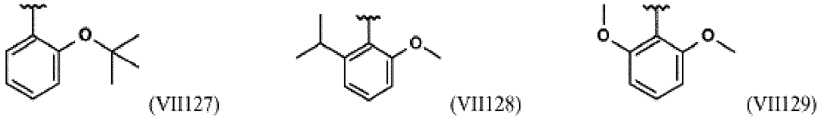

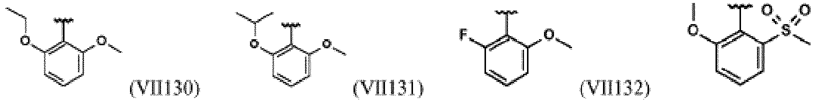

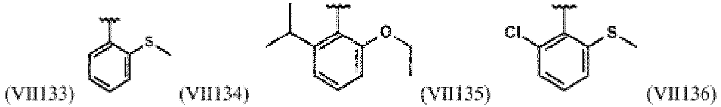

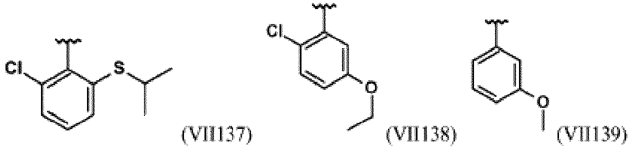

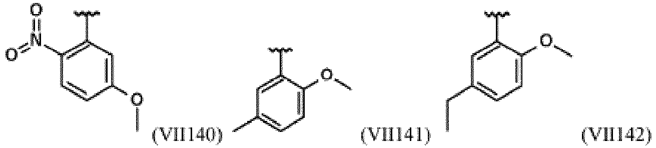

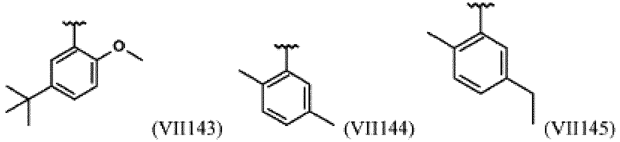

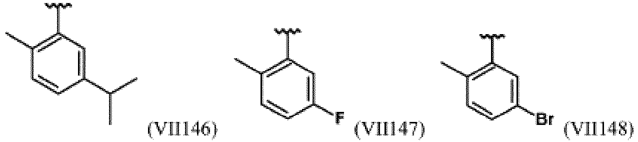

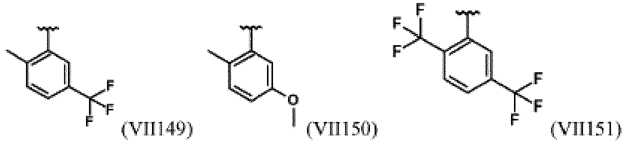

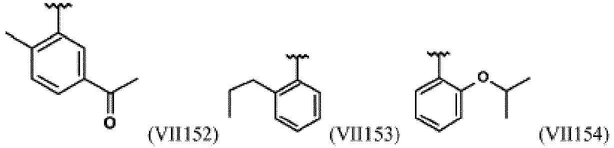

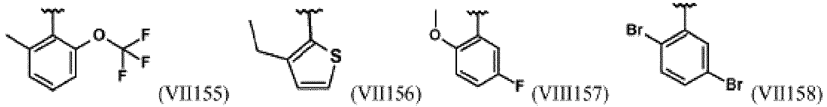

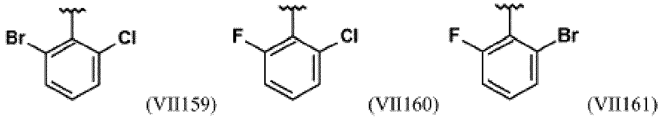

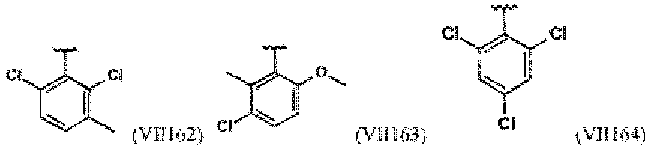

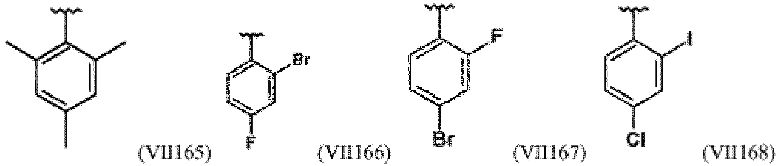

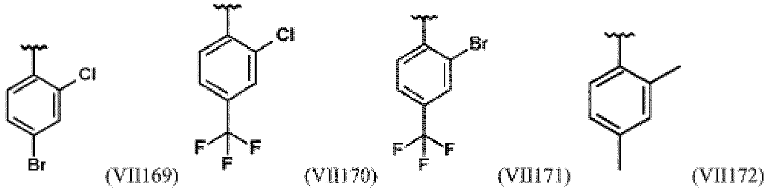

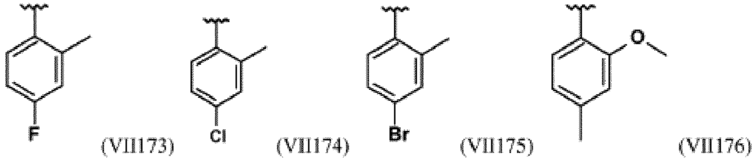

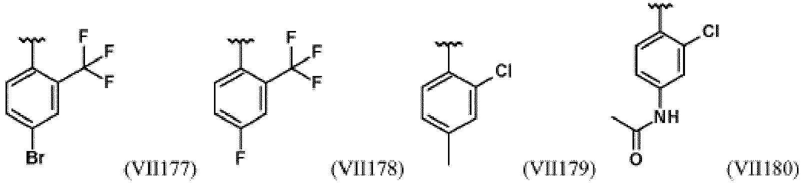

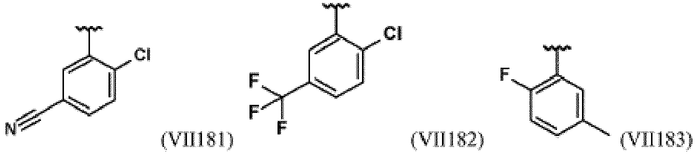

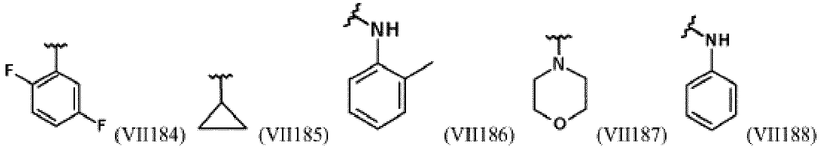

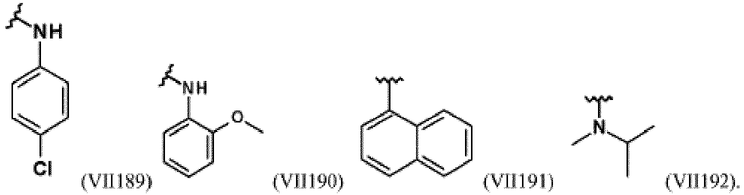

9. Utilisation selon l'une quelconque des revendications 1 à 8 pour la protection du matériel de propagation de végétaux.

10. Produit, comportant une teneur d'au moins un composé selon la formule (I) selon l'une quelconque des revendications 1 à 8 et des diluants habituels et/ou des substances tensioactives en particulier pour la lutte contre des parasites animaux.

11. Procédé pour la lutte contre des parasites animaux, dans lequel on laisse agir au moins un composé selon la formule (I) selon l'une quelconque des revendications 1 à 8 ou un produit selon la revendication 10 sur les parasites animaux et/ou sur leur lieu de vie, dans lequel le traitement chirurgical, thérapeutique et diagnostique du corps humain ou animal est exclu.

12. Formulation agrochimique contenant au moins un composé selon la formule (I) selon l'une quelconque des revendications 1 à 8 en des teneurs biologiquement efficaces comprises entre 0,00000001 et 98 % en poids par rapport au poids de la formulation agrochimique, ainsi que des diluants et/ou des matières tensioactives.

13. Formulation agrochimique selon la revendication 12 contenant de plus au moins un autre principe actif agrochimique.

14. Composés de formule 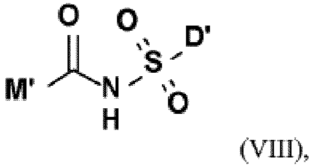 M' représentant un radical de formule (II), choisi parmi : 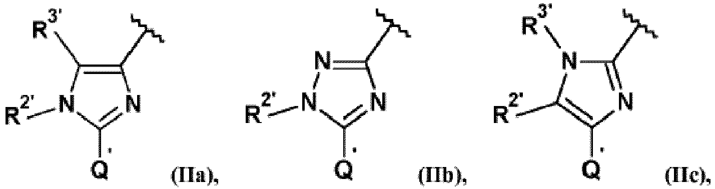 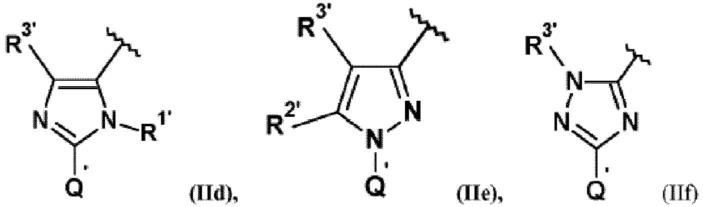 dans laquelle
R^{1'}, R^{2'}, R^{3'} à chaque fois indépendamment les uns des autres sont H ou un radical alkyle, cycloalkyle, alcényle, cycloalcényle, cyclohétéroalkyle, aryle ou hétéroaryle substitué ou non substitué, R^{2'} dans le cas de (IIc) et (IIe) pouvant de plus être un radical halogène ou un radical alcoxy, et R^{2'} dans le cas de (IIa) et (IIb) pouvant être seulement H ou un radical alkyle ou cycloalkyle substitué ou non substitué, R^{3'} pouvant de plus être un radical halogène dans le cas de (IIa), (IId) et (IIe),
Q' est un radical aryle ou hétéroaryle substitué ou non substitué, mais n'est pas 3-méthoxyphényle dans le cas de (IIa), de (IId) dans le cas où R^{1'} = H et R^{3'} = méthyle, n'est pas 3-pyridinyle dans le cas de (IId), n'est pas 2-pyrimidinyle dans le cas de (IIe), n'est pas phényle non substitué, n'est pas 3,4-dichlorophényle et n'est pas 3,5-bis-tert-butyle ;
D' est un radical alkyle, hétéroalkyle, cycloalkyle éventuellement partiellement insaturé, cyclohétéroalkyle, hétéroaryle, aryle ou phénylalkyle ou dans le cas où Q' porte au moins un substituant en position 2, est un radical azoté substitué ou non substitué.

15. Composés selon la revendication 14, dans lesquels M' représente un radical choisi parmi les formules (IIa) à (IIf), et
dans le cas de (IIa), R^{2'}, R^{3'} à chaque fois indépendamment l'un de l'autre étant H ou un radical (C₁-C₄)-alkyle, (C₃-C₆)-cycloalkyle ou phényle substitué ou non substitué, R^{3'} pouvant de plus être un radical halogène,
dans le cas de (IIb), R^{2'} étant H ou un radical (C₁-C₄)-alkyle ou (C₃-C₆)-cycloalkyle substitué ou non substitué,
dans le cas de (IIc), R^{2'}, R^{3'} à chaque fois indépendamment l'un de l'autre étant H ou un radical (C₁-C₄)-alkyle, (C₃-C₆)-cycloalkyle ou phényle substitué ou non substitué et R^{2'} pouvant de plus être un radical halogène ou (C₁-C₄)-alcoxy, dans le cas de (IId), R^{1'}, R^{3'} à chaque fois indépendamment l'un de l'autre étant H ou un radical (C₁-C₄)-alkyle ou (C₃-C₆)-cycloalkyle substitué ou non substitué, R^{3'} pouvant de plus être un radical halogène ;
dans le cas de (IIc), R^{2'}, R^{3'} à chaque fois indépendamment l'un de l'autre étant H, halogène ou un radical (C₁-C₄)-alkyle, (C₃-C₆)-cycloalkyle, pyridinyle ou phényle substitué ou non substitué et R² pouvant de plus être un radical (C₁-C₄)-alcoxy et
dans le cas de (IIf) R³ étant H ou un radical (C₁-C₄)-alkyle ou (C₃-C₆)-cycloalkyle substitué ou non substitué,
Q' est un radical phényle, naphtyle ou hétéroaryle substitué ou non substitué, qui peut contenir 1 à 3 hétéroatome(s) de la série oxygène, soufre, azote, mais n'est pas 3-méthoxyphényle dans le cas de (IIa), de (IId) dans le cas où R^{1'} = H et R^{3'} = méthyle, n'est pas 3-pyridinyle dans le cas de (IId), n'est pas 2-pyrimidinyle dans le cas de (IIe), n'est pas phényle non substitué et n'est pas 3,4-dichlorophényle,
D' est un radical alkyle, hétéroalkyle, cycloalkyle, hétéroaryle, aryle ou phényl-(C₁-C₈)-alkyle substitué ou non substitué ou dans le cas où Q' porte au moins un substituant en position 2, est un radical azoté substitué ou non substitué.

16. Composés selon la revendication 14 ou 15, dans lesquels,
M' représente un radical choisi parmi les formules (IIa à IIf), R^{1'}, R^{2'}, R^{3'} étant définis tel que dans la revendication 14 ou 15 et
Q' est un radical phényle, naphtyle, pyridinyle, pyrimidinyle, thiophène, benzothiophène, isoquinoléine, benzodioxole ou pyrazole non substitué ou substitué par un ou plusieurs radicaux R^{4'}, mais n'est pas 3-méthoxyphényle dans le cas de (IIa), de (IId) dans le cas où R^{1'} = H et R^{3'} = méthyle, n'est pas 3-pyridinyle dans le cas de (IId), n'est pas 2-pyrimidinyle dans le cas de (IIe), n'est pas phényle non substitué et n'est pas 3,4-dichlorophényle et
le ou les substituant (s) R^{4'} à chaque fois indépendamment les uns des autres étant choisi(s) parmi :
cyano, halogène, nitro, acétyle, hydroxy, carboxy, amino, SCN, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl- (C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy- (C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino) et/ou
aryle, aryloxy ou hétaryle éventuellement substitué une ou plusieurs fois, de manière identique ou différente, éventuellement au moins un groupe carbonyle pouvant être contenu (dans le cas d'hétaryle) et les substituants pouvant être à chaque fois : cyano, carboxy, halogène, nitro, acétyle, hydroxy, amino, SCN, tri-(C₁-C₆)-alkylsilyle, (C₃-C₈)-cycloalkyle, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy- (C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy- (C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, di-(C₁-C₆)-alkylaminocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino et
D' est un radical (C₁-C₆)-alkyle, phényle ou hétéroaryle non substitué ou substitué par un ou plusieurs radicaux R^{5'}, qui peut contenir 1 à 3 hétéroatome(s) de la série oxygène, soufre, azote ou, dans le cas où Q' porte au moins un substituant en position 2, est un radical NR^{6'}R^{7'}, le ou les substituant (s) R^{5'} à chaque fois indépendamment les uns des autres étant choisi(s) parmi :
cyano, halogène, nitro, acétyle, hydroxy, carboxy, amino, SCN, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy- (C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino et (1-pyrazolyl)-(C₁-C₃)-alkyle
et/ou
aryle ou hétaryle substitué une ou plusieurs fois, de manière identique ou différente, éventuellement au moins un groupe carbonyle pouvant être contenu (dans le cas d'hétaryle) et les substituants pouvant être à chaque fois : cyano, carboxyle, halogène, nitro, acétyle, hydroxy, amino, SCN, tri-(C₁-C₆)-alkylsilyle, (C₃-C₈)-cycloalkyle, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy- (C₁-C₆)-alcoxy, (C₁-C₆)-alkylhydroxyimino, (C₁-C₆)-alcoxyimino, (C₁-C₆)-alkyl- (C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, di-(C₁-C₆)-alkylaminocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino et (C₁-C₆)-alkylcarbonylamino,
et R^{6'} et R^{7'} étant à chaque fois indépendamment l'un de l'autre H, un radical (C₁-C₆)-alkyle, (C₁-C₆)-halogénoalkyle, (C₁-C₆)-alcoxy-(C₁-C₆)-alkyle ou un radical phényle non substitué ou un radical phényle substitué par halogène, (C₁-C₆)-alkyle, (C₁-C₆)-alcoxy, (C₁-C₆)-halogénoalkyle, (C₁-C₆)-alcoxy-(C₁-C₆)-alkyle ou
R^{6'} et R^{7'} ensemble pouvant former un cycle non substitué ou substitué à 5 à 6 chaînons, saturé ou éventuellement entièrement ou partiellement insaturé qui peut être interrompu par 1 à 3 hétéroatome(s) de la série oxygène, soufre, azote et qui peut être doté une ou plusieurs fois par une substitution correspondant à la définition de R^{5'}.

17. Composés selon l'une quelconque des revendications 14 à 16, dans lesquels
M' représente un radical choisi parmi les formules (IIa à IIf), R^{1'}, R^{2'}, R^{3'} étant définis tel que dans la revendication 14 ou 15 et
Q' est un radical phényle, naphtyle, pyridinyle, pyrimidinyle, thiophène, benzothiophène, isoquinoléine, benzodioxole ou pyrazole non substitué ou substitué par un ou plusieurs radicaux R^{4'}, mais n'est pas 3-méthoxyphényle dans le cas de (IIa), de (IId) dans le cas où R^{1'} = H et R^{3'} = méthyle, n'est pas 3-pyridinyle dans le cas de (IId), n'est pas 2-pyrimidinyle dans le cas de (IIe), n'est pas phényle non substitué et n'est pas 3,4-dichlorophényle
et le ou les substituant (s) R^{4'} à chaque fois indépendamment les uns des autres étant choisi(s) parmi :
cyano, halogène, nitro, acétyle, hydroxy, carboxy, amino, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy- (C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy- (C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino, phényle, halogénophényle, phénoxy ou halogénophénoxy et D' est un radical phényle, pyridine, pyrimidine, pyrazole, triazole, thiazole, oxazole, thiadiazole, oxadiazole, thiophène, pyrrole, furanne, tétrahydrofuranne, dioxanne ou (C₁-C₆)-alkyle non substitué ou substitué par un ou plusieurs radicaux R^{5'}, ou est un radical NR^{6'}R^{7'}, le ou les substituant (s) R^{5'} à chaque fois indépendamment les uns des autres étant choisi(s) parmi :
cyano, halogène, nitro, acétyle, hydroxy, carboxy, amino, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy-(C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino ou (1-pyrazolyl)-(C₁-C₃)-alkyle,
et R^{6'} et R^{7'} étant à chaque fois indépendamment l'un de l'autre H, un radical (C₁-C₄)-alkyle, (C₁-C₄)-halogénoalkyle, (C₁-C₄)-alcoxy-(C₁-C₄)-alkyle ou un radical phényle non substitué ou un radical phényle substitué par halogène, (C₁-C₄)-alkyle, (C₁-C₄)-alcoxy, (C₁-C₄)-halogénoalkyle, (C₁-C₄)-alcoxy-(C₁-C₄)-alkyle ou
R^{6'} et R^{7'} ensemble pouvant former un cycle non substitué ou substitué à 5 à 6 chaînons, saturé ou éventuellement entièrement ou partiellement insaturé qui peut être interrompu par 1 à 3 hétéroatome(s) de la série oxygène, soufre, azote et qui peut être doté une ou plusieurs fois par une substitution correspondant à la définition de R^{5'}.

18. Composés selon l'une quelconque des revendications 14 à 17, dans lesquels
D' est un radical (C₁-C₆)-alkyle, un radical phényle, un radical pyridine, pyrimidine, pyrazole, triazole, thiazole, oxazole, thiadiazole, oxadiazole, thiophène, pyrrole, furanne, tétrahydrofuranne, dioxanne, isoxazole, benzyle, 2,3-dihydro-1,4-benzodioxin-5-yle, 2,3-dihydro-1-benzofuran-7-yle, quinoxalin-5-yle ou indol-7-yle non substitué ou substitué par un ou plusieurs radicaux R^{5'}, ou un radical NR^{6'}R^{7'},
le ou les substituant (s) R^{5'} à chaque fois indépendamment les uns des autres étant choisi(s) parmi :
cyano, halogène, nitro, acétyle, hydroxy, carboxy, amino, tri-(C₁-C₆)-alkylsilyle, (C₃-C₈)-cycloalkyle, (C₃-C₈)-cycloalkyloxy, (C₃-C₈)-cycloalkyl-(C₃-C₈)-cycloalkyle, (C₁-C₆)-alkyl-(C₃-C₈)-cycloalkyle, halogéno-(C₃-C₈)-cycloalkyle, (C₁-C₆)-alkyle, (C₁-C₆)-halogénoalkyle, (C₁-C₆)-cyanoalkyle, (C₁-C₆)-hydroxyalkyle, hydroxycarbonyl-(C₁-C₆)-alcoxy, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-cyanoalcényle, (C₂-C₆)-alcynyle, (C₂-C₆)-halogénoalcynyle, (C₂-C₆)-cyanoalcynyle, (C₁-C₆)-alcoxy, (C₁-C₆)-halogénoalcoxy, (C₁-C₆)-cyanoalcoxy, (C₁-C₆)-alcoxycarbonyl-(C₁-C₆)-alcoxy, (C₁-C₆)-alcoxy- (C₁-C₆)-alcoxy, (C₁-C₆)-alkylhydroxyimino, (C₁-C₆)-alcoxyimino, (C₁-C₆)-alkyl-(C₁-C₆)-alcoxyimino, (C₁-C₆)-halogénoalkyl-(C₁-C₆)-alcoxyimino, (C₁-C₆)-alkylthio, (C₁-C₆)-halogénoalkylthio, (C₁-C₆)-alcoxy-(C₁-C₆)-alkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alcoxy-(C₁-C₆)-alkylsulfinyle, (C₁-C₆)-alkylsulfinyl-(C₁-C₆)-alkyle, (C₁-C₆)-alkylsulfonyle, (C₁-C₆)-halogénoalkylsulfonyle, (C₁-C₆)-alcoxy-(C₁-C₆)-alkylsulfonyle, (C₁-C₆)-alkylsulfonyl-(C₁-C₆)-alkyle, (C₁-C₆)-alkylsulfonyloxy, (C₁-C₆)-alkylcarbonyle, (C₁-C₆)-alkylthiocarbonyle, (C₁-C₆)-halogénoalkylcarbonyle, (C₁-C₆)-alkylcarbonyloxy, (C₁-C₆)-alcoxycarbonyle, (C₁-C₆)-halogénoalcoxycarbonyle, aminocarbonyle, (C₁-C₆)-alkylaminocarbonyle, (C₁-C₆)-alkylaminothiocarbonyle, di-(C₁-C₆)-alkylaminocarbonyle, di-(C₁-C₆)-alkylaminothiocarbonyle, (C₂-C₆)-alcénylaminocarbonyle, di-(C₂-C₆)-alcénylaminocarbonyle, (C₃-C₈)-cycloalkylaminocarbonyle, (C₁-C₆)-alkylsulfonylamino, (C₁-C₆)-alkylamino, di-(C₁-C₆)-alkylamino, aminosulfonyle, (C₁-C₆)-alkylaminosulfonyle, di-(C₁-C₆)-alkylaminosulfonyle, (C₁-C₆)-alkylsulfoximino, aminothiocarbonyle, (C₁-C₆)-alkylaminothiocarbonyle, di-(C₁-C₆)-alkylaminothiocarbonyle, (C₃-C₈)-cycloalkylamino, (C₁-C₆)-alkylcarbonylamino ou (1-pyrazolyl)-(C₁-C₃)-alkyle et
R^{6'} et R^{7'} étant à chaque fois indépendamment l'un de l'autre H, un radical (C₁-C₆)-alkyle, (C₁-C₆)-halogénoalkyle, (C₁-C₆)-alcoxy-(C₁-C₆)-alkyle ou un radical phényle non substitué ou un radical phényle substitué par halogène, (C₁-C₆)-alkyle, (C₁-C₆)-halogénoalkyle ou (C₁-C₆)-alcoxy-(C₁-C₆)-alkyle
ou formant un cycle de la série pyrrolidine, morpholine, pipéridine.

19. Composés selon l'une quelconque des revendications 14 à 18, dans lesquels
M' est un radical choisi parmi les radicaux des formules (IIa) à (IIf),
dans le cas de (IIa), R^{2'} étant H, méthyle ou éthyle ou phényle éventuellement substitué par halogène, et R^{3'} étant H, méthyle, éthyle, iso-propyle ou halogène,
dans le cas de (IIb), R^{2'} étant H, méthyle ou éthyle,
dans le cas de (IIc), R^{2'} étant H ou méthyle et R^{3'} étant H, méthyle ou éthyle,
dans le cas de (IId), R^{1'} étant H ou méthyle et R^{3'} étant H ou halogène,
dans le cas de (IIe) R^{2'} étant H, méthyle, méthoxy, phényle substitué par halogène ou pyridinyle substitué par halogène et R^{3'} étant H,
dans le cas de (IIf), R^{3'} étant H, méthyle ou éthyle
et
Q' est un radical phényle, napht-1-yle, pyridinyle, pyrimidinyle, thiophén-2-yle, benzothiophén-2-yle, benzothiophén-3-yle, isoquinoléin-1-yle, benzodioxol-4-yle ou pyrazol-5-yle non substitué ou substitué par un ou plusieurs radicaux R^{4'}, mais n'est pas 3-méthoxyphényle dans le cas de (IIa), de (IId) dans le cas où R^{1'} = H et R^{3'} = méthyle, n'est pas 3-pyridinyle dans le cas de (IId), n'est pas 2-pyrimidinyle dans le cas de (IIe), n'est pas phényle non substitué et n'est pas 3,4-dichlorophényle, et
les substituants R^{4'} étant définis tel que dans la revendication 17 ou 18.

20. Composés selon l'une quelconque des revendications 14 à 19, dans lesquels
Q' est un radical phényle, napht-1-yle, pyridinyle, pyrimidinyle, thiophén-2-yle, benzothiophén-2-yle, benzothiophén-3-yle, isoquinoléin-1-yle, benzodioxol-4-yle ou pyrazol-5-yle non substitué ou substitué par un ou plusieurs radicaux R^{4'}, mais n'est pas 3-méthoxyphényle dans le cas de (IIa), de (IId) dans le cas où R^{1'} = H et R^{3'} = méthyle, n'est pas 3-pyridinyle dans le cas de (IId), n'est pas 2-pyrimidinyle dans le cas de (IIe), n'est pas phényle non substitué et n'est pas 3,4-dichlorophényle et
le ou les substituants R^{4'} étant choisis indépendamment les uns des autres parmi :
cyano, halogène, nitro, acétyle, (C₃-C₆)-cycloalkyle, (C₃-C₆)-cycloalkyloxy, (C₃-C₆)-cycloalkyl-(C₃-C₆)-cycloalkyle, (C₁-C₆)-alkyl-(C₃-C₈)-cycloalkyle, halogéno-(C₃-C₆)-cycloalkyle, (C₁-C₆)-alkyle, (C₁-C₆)-halogénoalkyle, (C₁-C₆)-cyanoalkyle, (C₁-C₆)-hydroxyalkyle, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-alcynyle, (C₁-C₆)-alcoxy, (C₁-C₆)-halogénoalcoxy, (C₁-C₆)-alkylthio, (C₁-C₆)-halogénoalkylthio, (C₁-C₆)-alkylthio- (C₁-C₆)-alkyle, (C₁-C₆)-alkylsulfinyle, (C₁-C₆)-halogénoalkylsulfinyle, (C₁-C₆)-alkylsulfonyle, (C₁-C₆)-halogénoalkylsulfonyle, (C₁-C₆)-alkylsulfonyloxy, (C₁-C₆)-alkylcarbonyle, (C₁-C₆)-alkylamino, di-(C₁-C₆)-alkylamino, (C₁-C₆)-alkylcarbonylamino, phényle, halogénophényle, phénoxy ou halogénophénoxy ;
D' étant un radical (C₁-C₆)-alkyle, phényle, pyridine, pyrimidine, pyrazole, triazole, thiazole, oxazole, thiadiazole, oxadiazole, thiophène, pyrrole, furanne, tétrahydrofuranne ou dioxanne non substitué ou substitué par un ou plusieurs radicaux R^{5'},
le ou les substituant(s) R⁵ à chaque fois indépendamment les uns des autres étant choisi(s) parmi :
cyano, halogène, nitro, acétyle, hydroxy, carboxy, amino, (C₃-C₆)-cycloalkyle, (C₃-C₆)-cycloalkyloxy, (C₃-C₆)-cycloalkyl-(C₃-C₆)-cycloalkyle, (C₁-C₆)-alkyl-(C₃-C₈)-cycloalkyle, halogéno-(C₃-C₆)-cycloalkyle, (C₁-C₆)-alkyle, (C₁-C₆)-halogénoalkyle, (C₁-C₆)-cyanoalkyle, (C₁-C₆)-hydroxyalkyle, (C₁-C₆)-alcoxycarbonyl-(C₁-C₆)-alkyle, (C₁-C₆)-alcoxy-(C₁-C₆)-alkyle, (C₂-C₆)-alcényle, (C₂-C₆)-halogénoalcényle, (C₂-C₆)-alcynyle, (C₁-C₆)-alcoxy, (C₁-C₆)-halogénoalcoxy, (C₁-C₆)-alkylthio, (C₁-C₆)-halogénoalkylthio, (C₁-C₆)-alkylthio-(C₁-C₆)-alkyle, (C₁-C₆)-alkylsulfinyle, (C₁-C₆)-halogénoalkylsulfinyle, (C₁-C₆)-alkylsulfonyle, (C₁-C₆)-halogénoalkylsulfonyle, (C₁-C₆)-alkylsulfonyloxy, (C₁-C₆)-alkylcarbonyle, (C₁-C₆)-alkylamino, di-(C₁-C₆)-alkylamino, (C₁-C₆)-alkylcarbonylamino ou (1-pyrazolyl)-(C₁-C₃)-alkyle et
R⁶ et R⁷ étant à chaque fois indépendamment l'un de l'autre H, un radical (C₁-C₆)-alkyle, phényle, alcoxyphényle ou halogénophényle ou formant un cycle de la série pyrrolidine, morpholine, pipéridine.

21. Composés selon l'une quelconque des revendications 14 à 20, dans lesquels
M' est un radical choisi parmi les radicaux des formules (Va) à (Vz) : 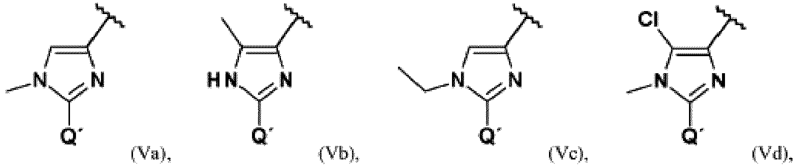 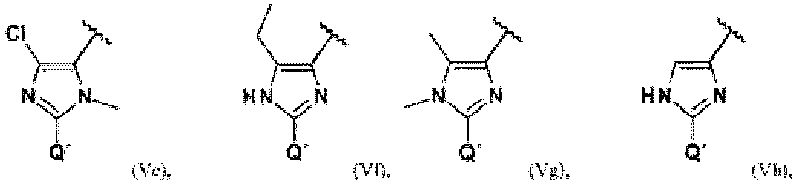 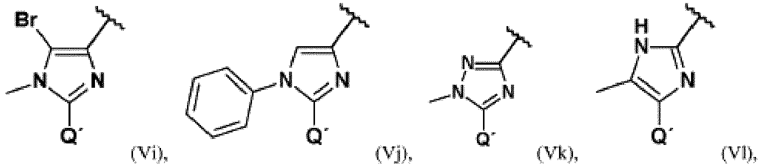 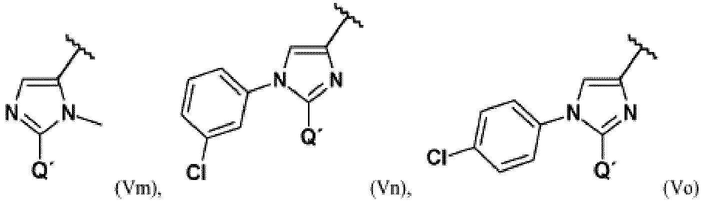 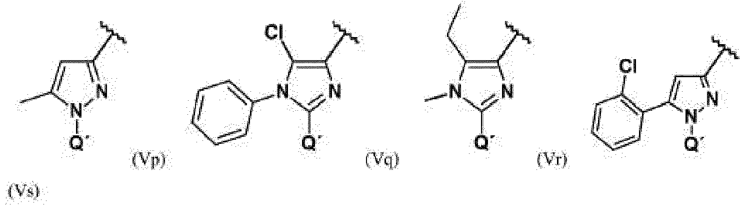 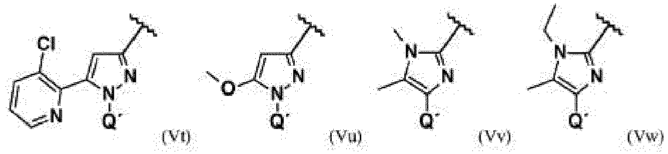 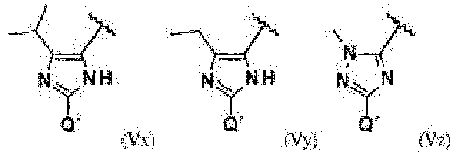 Q' est un radical choisi parmi les radicaux des formules (VIa à VIz) et (VIa1 à VIa30) : 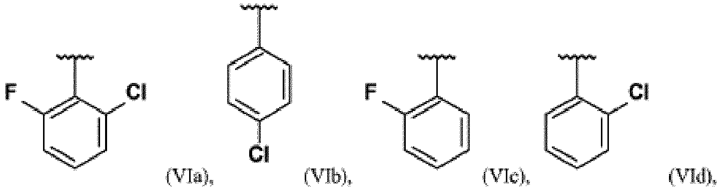 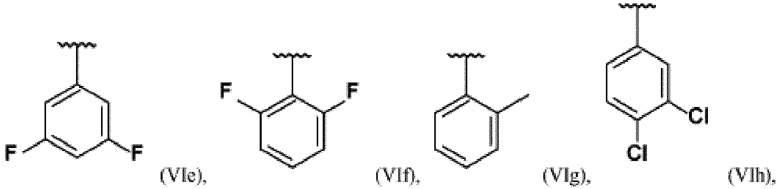 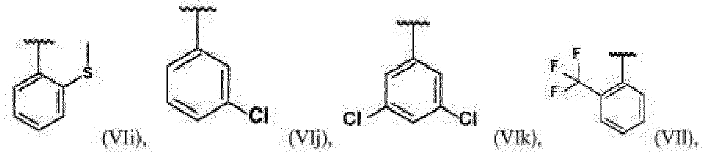 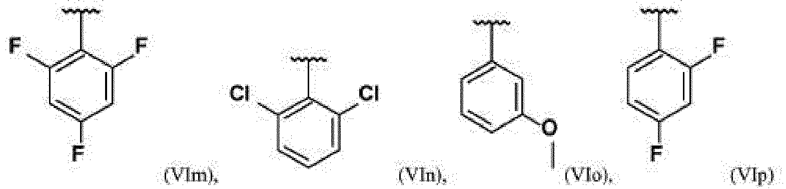 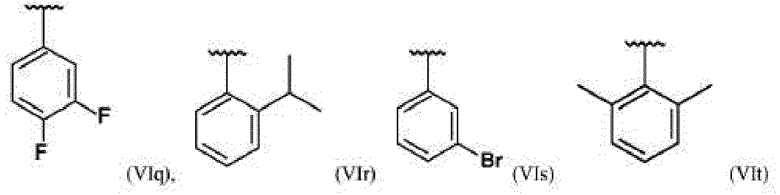 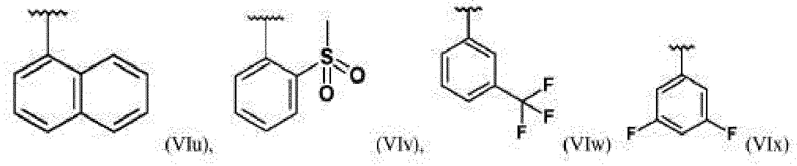 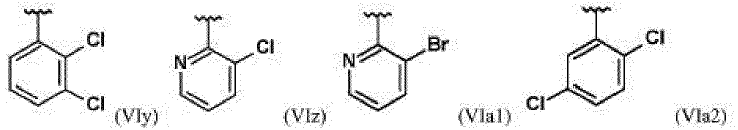 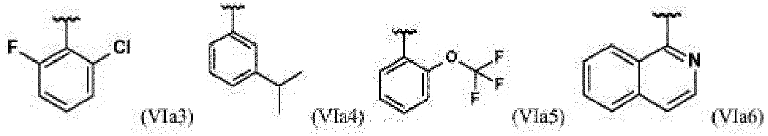 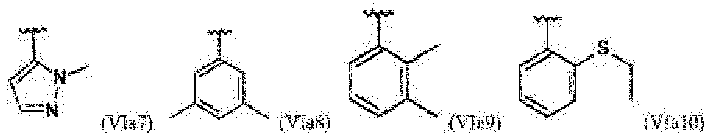 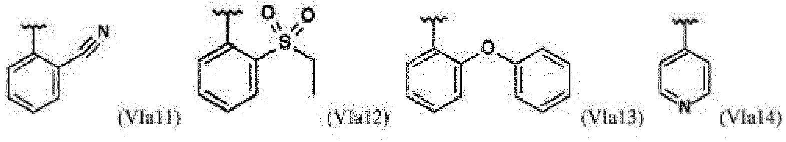 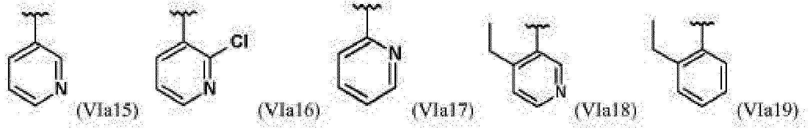 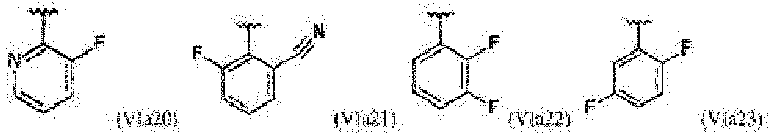 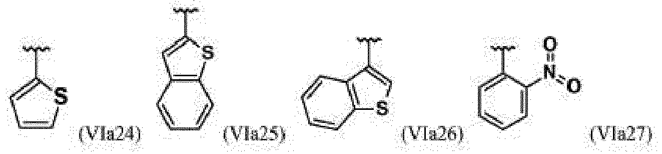 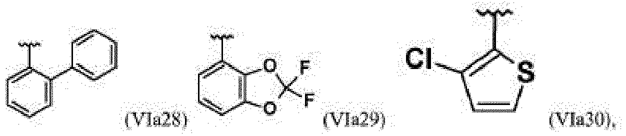 D' est un radical choisi parmi les radicaux des formules (VII1 à VII192)

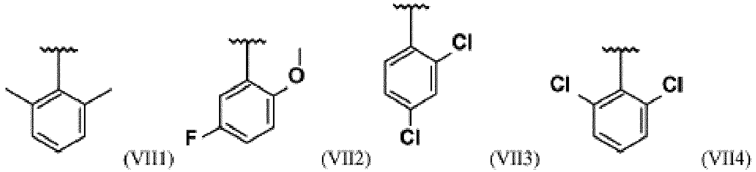

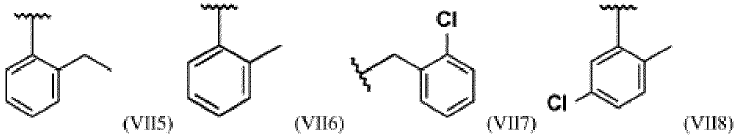

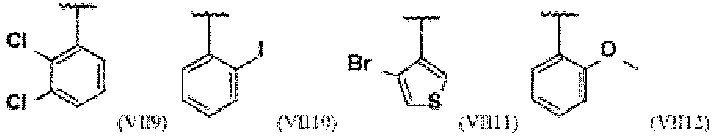

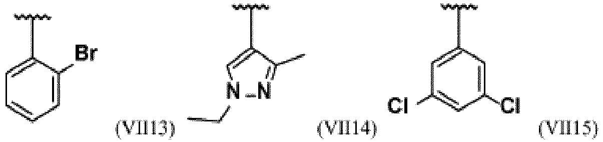

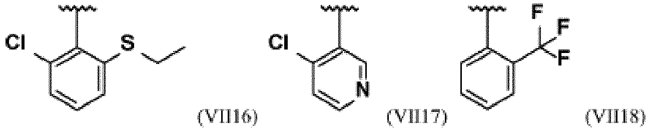

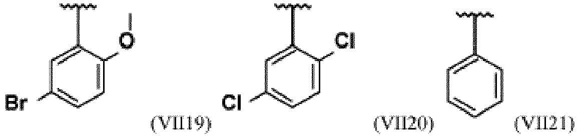

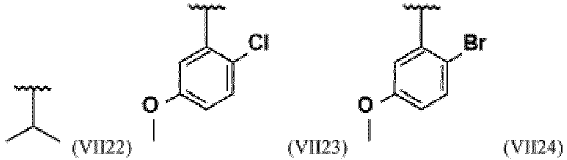

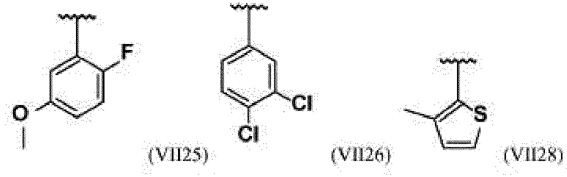

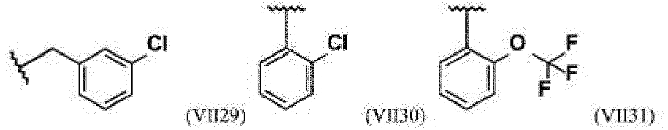

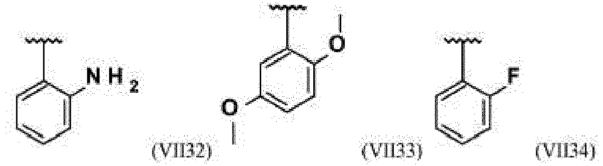

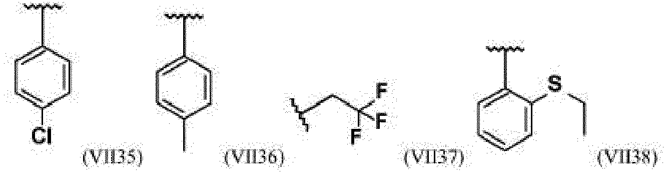

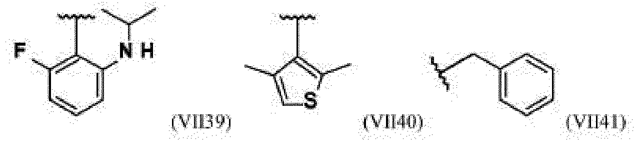

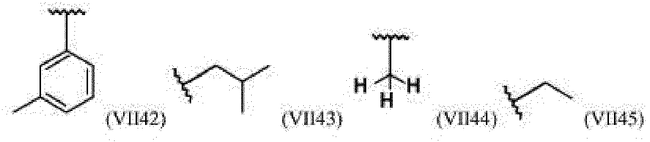

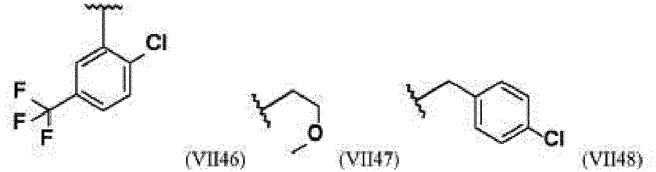

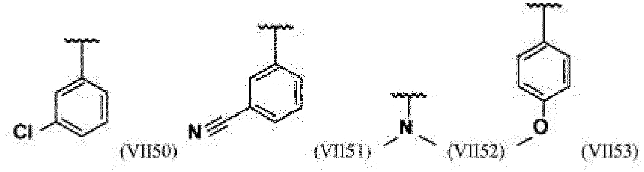

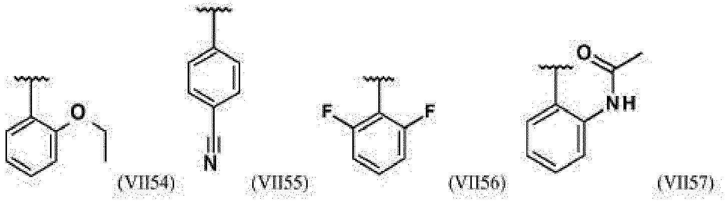

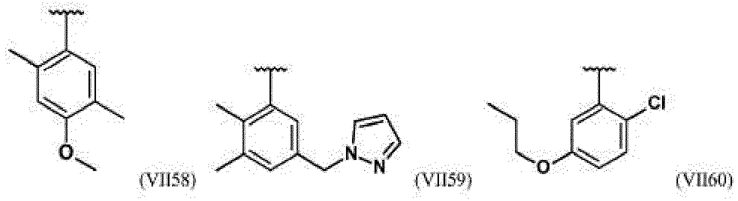

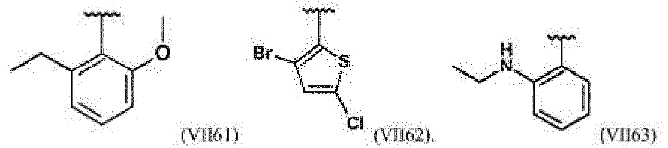

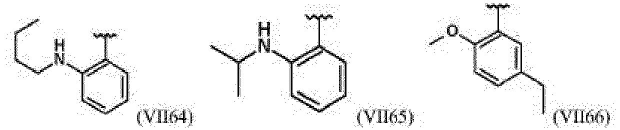

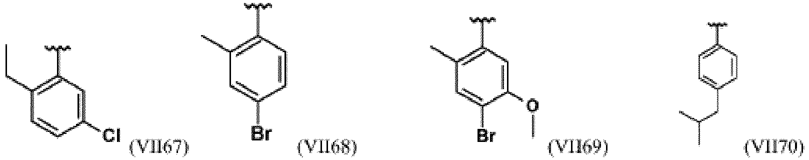

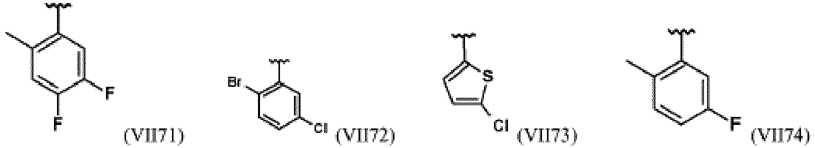

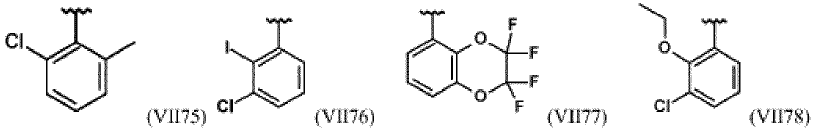

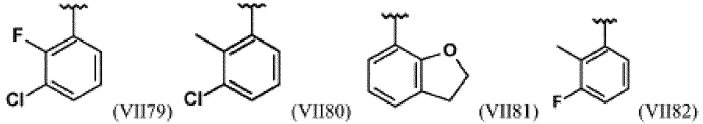

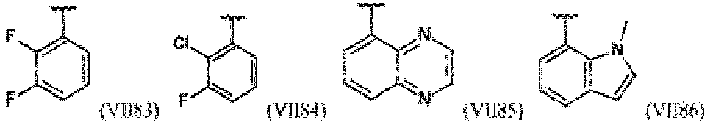

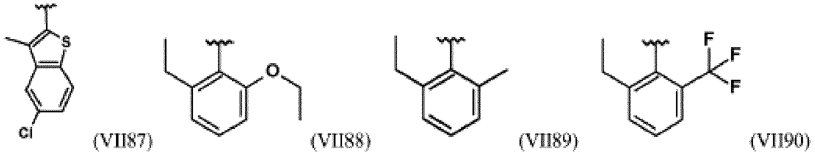

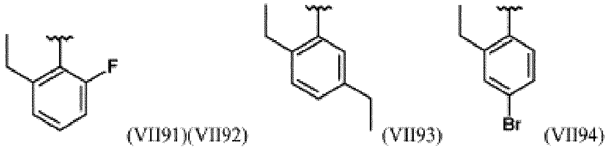

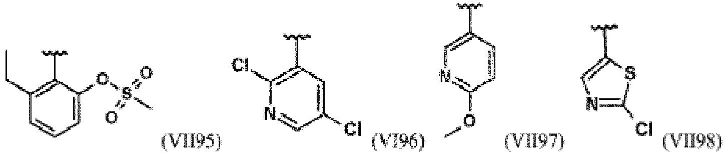

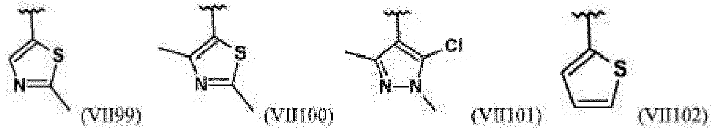

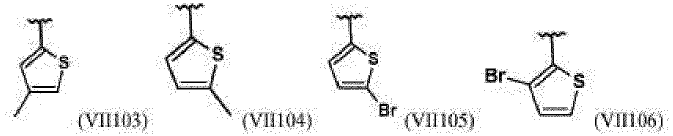

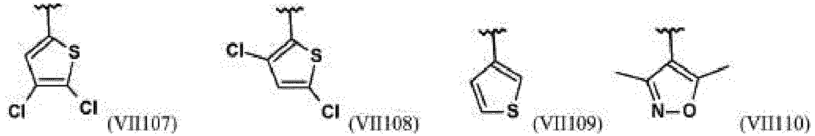

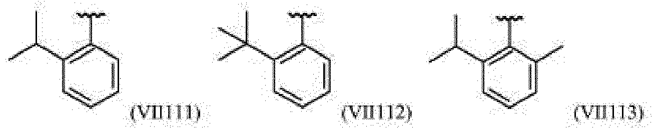

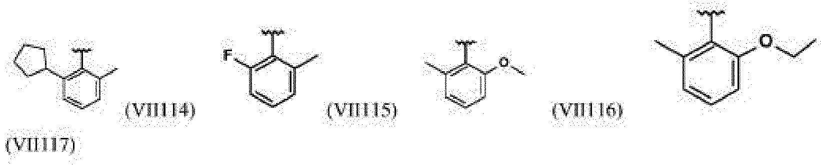

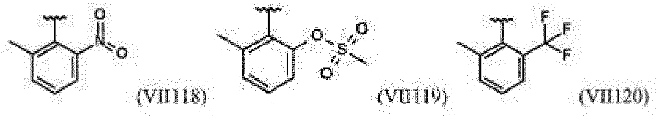

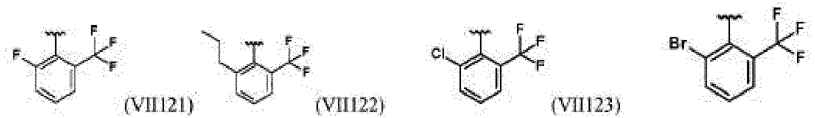

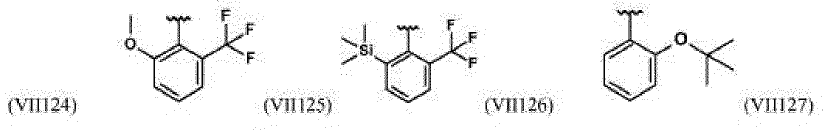

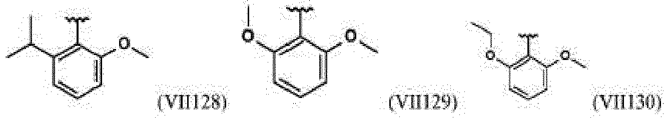

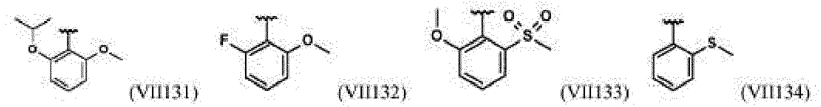

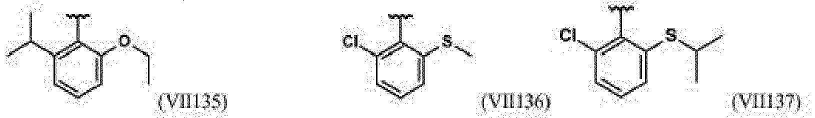

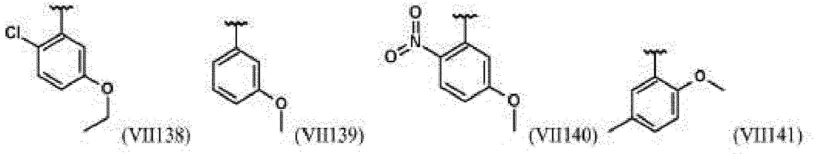

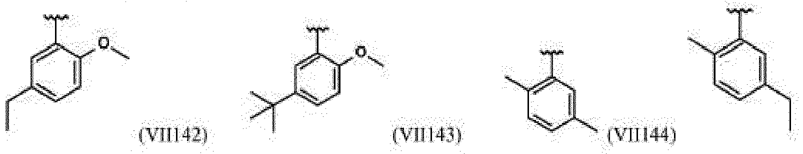

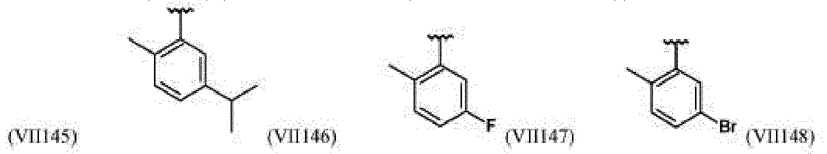

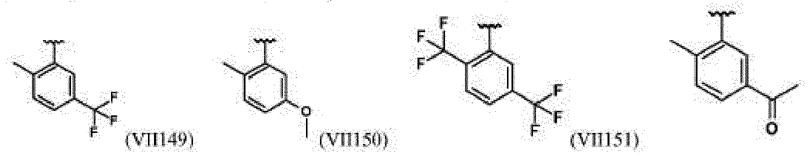

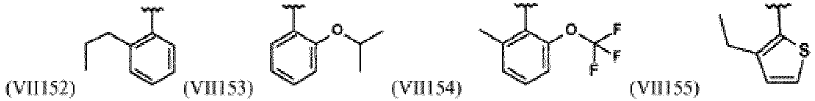

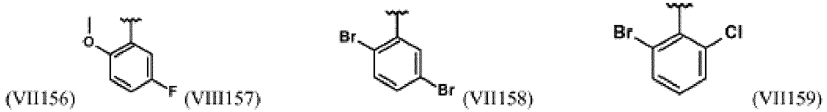

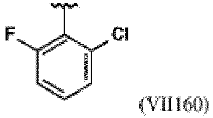

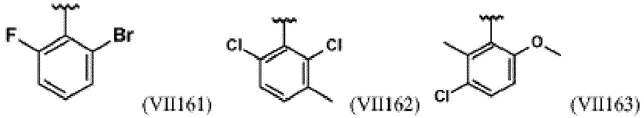

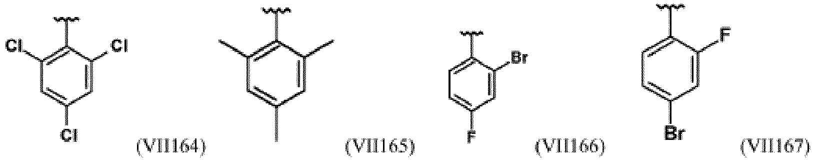

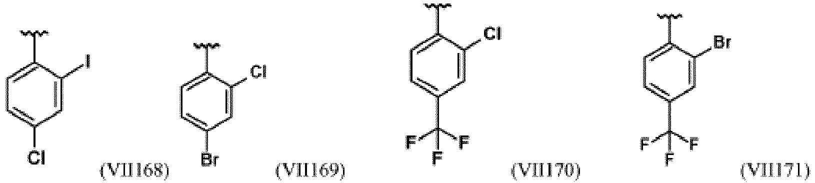

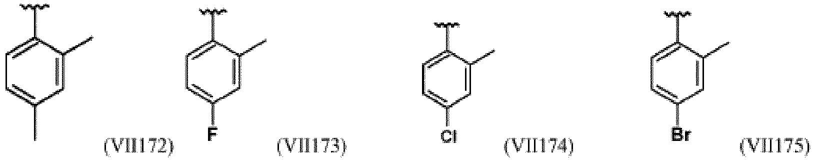

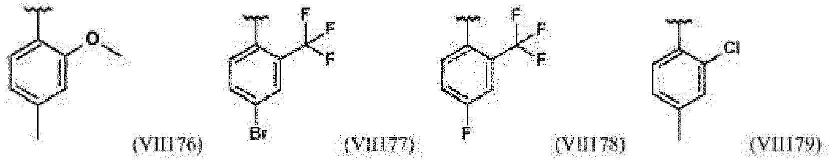

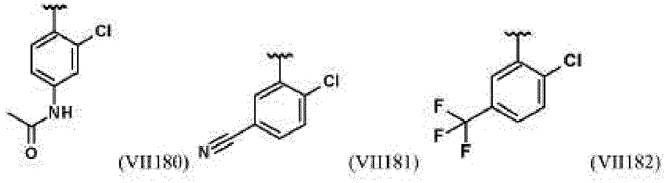

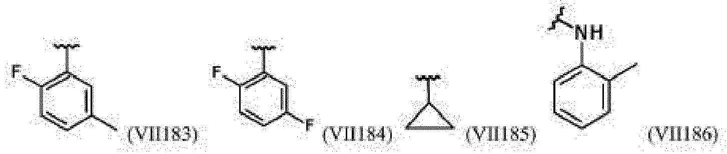

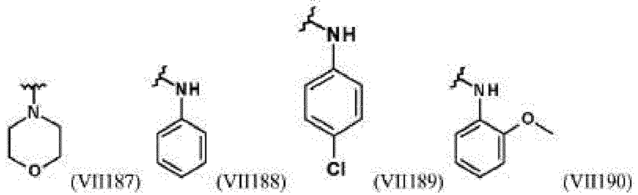

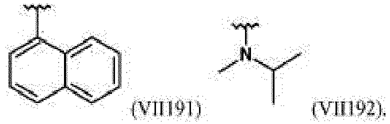

22. Produits intermédiaires des formules XIa à XIq
